# EUROPEAN PATENT APPLICATION

(11) **EP 4 368 614 A1**
(43) Date of publication of application: **15.05.2024**
(21) Application number: 22836982.3
(22) Date of filing: 06.07.2022
(51) Int. Cl.: C07D 239/48, C07D 241/26, C07D 249/14, A61P 35/00

(54) **SYNTHESIS AND APPLICATION OF PHOSPHATASE DEGRADER**

(30) Priority: 07.07.2021 CN 202110769589
(71) Applicant: Hinova Pharmaceuticals Inc., Chengdu, Sichuan 610041 (CN)
(72) Inventor: FAN, Lei, Chengdu, Sichuan 610041 (CN); YU, Hua, Chengdu, Sichuan 610041 (CN); WANG, Fei, Chengdu, Sichuan 610041 (CN); AI, Chaowu, Chengdu, Sichuan 610041 (CN); XU, Kexin, Chengdu, Sichuan 610041 (CN); LIU, Xingtai, Chengdu, Sichuan 610041 (CN); DU, Jing, Chengdu, Sichuan 610041 (CN); PENG, Ying, Chengdu, Sichuan 610041 (CN); LUO, Tongchuan, Chengdu, Sichuan 610041 (CN); PENG, Shiming, Chengdu, Sichuan 610041 (CN); TAN, Bin, Chengdu, Sichuan 610041 (CN); XIAO, Daibiao, Chengdu, Sichuan 610041 (CN); HUO, Yongxu, Chengdu, Sichuan 610041 (CN); LIU, Chengcheng, Chengdu, Sichuan 610041 (CN); LI, Xinghai, Chengdu, Sichuan 610041 (CN); CHEN, Yuanwei, Chengdu, Sichuan 610041 (CN)
(74) Representative: Altmann Stößel Dick Patentanwälte PartG mbB
(86) International application number: PCT/CN2022/104221
(87) International publication number: WO 2023/280237

(57) **Abstract**

A synthesis and an application of a phosphatase degrader, belonging to the field of chemical medicine. The phosphatase degrader is a compound represented by formula I, or a salt thereof, or a deuterated compound thereof, or a stereoisomer thereof, or a solvate thereof, or a hydrate thereof, or a prodrug thereof. The compound can be used as a phosphatase degrader, especially as an SHP2 protein degrader, can treat malignant diseases such as tumors, and has good application prospects.

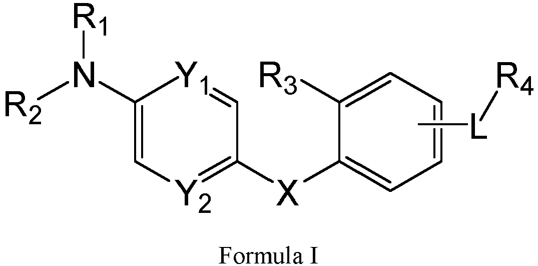

## Description

### Technical field

The present invention belongs to the chemistry-medicinal field, and specifically relates to the synthesis and application of a phosphatase degrader.

### Background technology

SHP2 (the Src homology-2 domain) is a non-receptor tyrosine phosphatase encoded by the PTPN11 gene, and contains a conserved tyrosine phosphatase domain, two N-terminal SH2 domains, and a C-terminal tail. The two SH2 domains determine the subcellular localization and functional regulation of SHP2. In the inactive state, the N-terminal SH2 domain will bind to the PTP domain and cause it to lose activity. When the SH2 domain binds to receptors or specific tyrosine residues in adapter proteins, the PTP domain is released. For example, the exposure of catalytic sites by the stimulation of cytokines and growth factors leads to the activation of SHP2.

SHP2 is widely expressed and participates in various cell signaling pathways, such as Ras-Erk, PI3K-Akt, Jak-Stat, Met, FGFR, EGFR, as well as insulin receptor and NF-kB pathways, thereby playing important roles in cell proliferation, differentiation, migration, and cell cycles. Superactivation of SHP2 caused by germline or somatic mutations has been found in Noonan syndrome, Leopard syndrome, juvenile myelomonocytic leukemia, myelodysplastic syndrome, B-cell acute lymphoblastic leukemia, and acute myeloid leukemia. In addition, activation and mutations of PTPN11 have also been found in solid tumors such as lung cancer, colon cancer, melanoma, neuroblastoma, and liver cancer. Therefore, activated SHP2 or upregulated SHP2 protein in human tumors or other diseases has become new therapeutic targets.

SHP2 represents a promising target for many cancers, such as triple negative and HER2+ breast cancers, as well as cancers caused by abnormal activation of receptor protein tyrosine kinase (PTK). Therefore, discovering and searching for SHP2 protein degraders with good druggability have gradually become a hot research field in the industrial and academic circles.

### Summary of the invention

The present invention is to provide the synthesis and application of a phosphatase degrader.

The present invention provides a compound represented by formula I, or a salt thereof, or a deuterated compound thereof, or a stereoisomer thereof, or a solvate thereof, or a hydrate thereof, or a prodrug thereof: 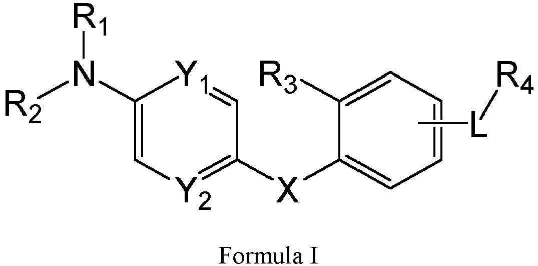 wherein,
R₁ and R₂, together with the N atom to which they are attached, form a 5-10 membered heterocyclic group substituted with 0-5 R₅;
each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
each R₆ is independently selected from the group consisting of hydroxyl, amino, halogen, carboxyl, nitro, cyano, C₁-C₈ alkoxy, and -N(H)R₇;
R₇ is selected from the group consisting of C₁-C₈ alkyl substituted with 0-5 R₉, C₁-C₈ alkoxy, and tert-butoxycarbonyl;
R₈ is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy;
Y₁ and Y₂ are each independently selected from -N- or -CH-; and at least one of Y₁ and Y₂ is selected from -N-;
X is selected from -S- or absence;
R₃ is selected from the group consisting of hydrogen, C₁-C₈ alkyl, C₁-C₈ alkoxy, halogen, hydroxyl, carboxyl, amino, nitro, and cyano;

R₄ is selected from the group consisting of

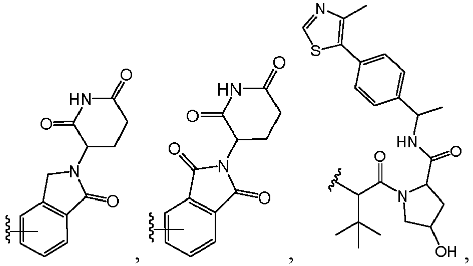

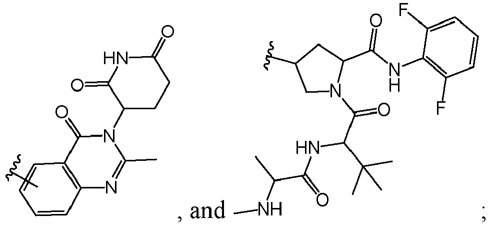

L is connected to phenyl ring at any position, and selected from the group consisting of 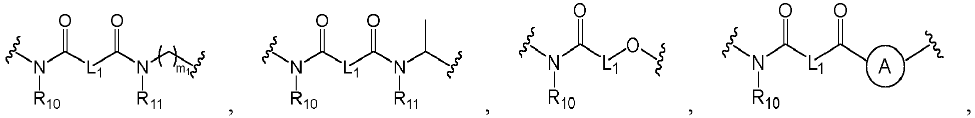 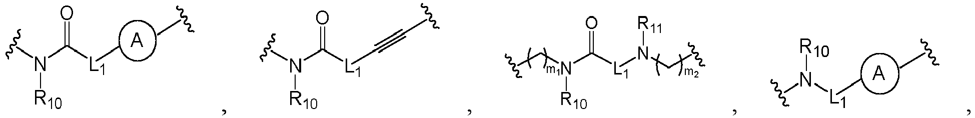 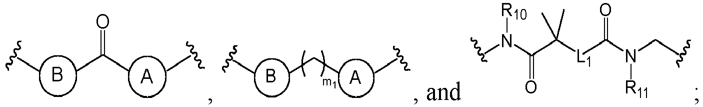
m₁ is an integer from 0 to 15;
m₂ is an integer from 0 to 15;
R₁₀ and R₁₁ are each independently selected from Hand C₁-C₈ alkyl;
L₁ is selected from the group consisting of 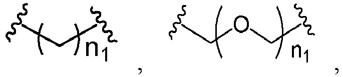 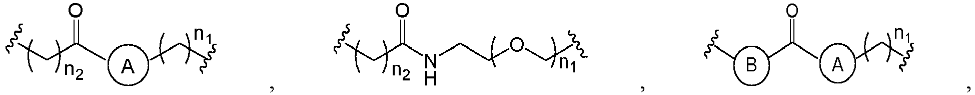 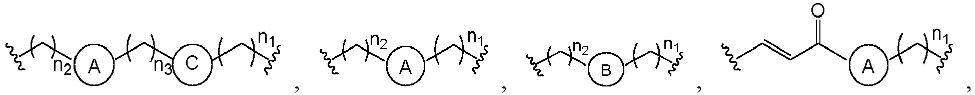 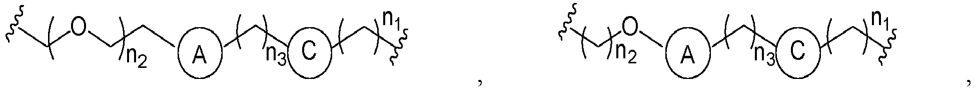 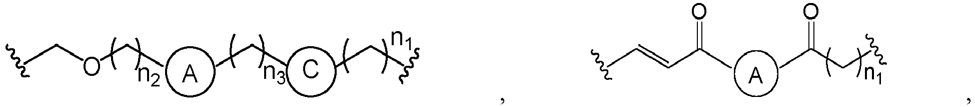 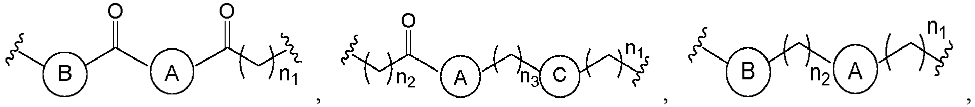 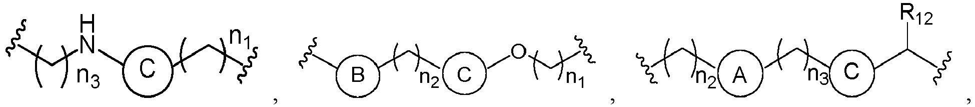 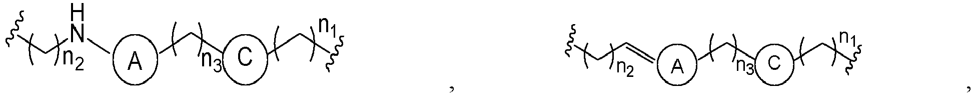 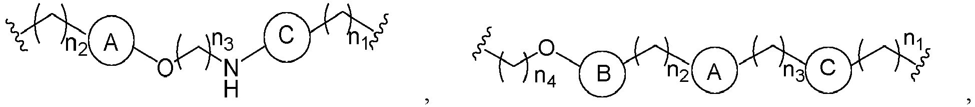 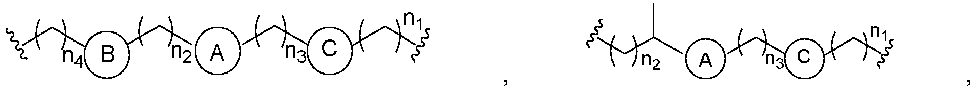 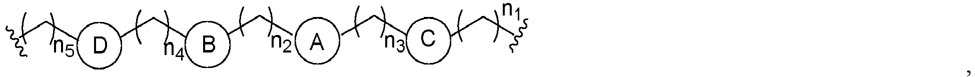 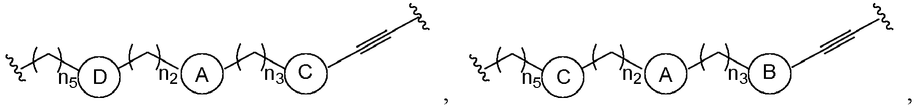 and 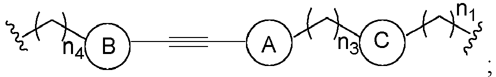
n₁ is an integer from 0 to 15;
n₂ is an integer from 0 to 15;
n₃ is an integer from 0 to 15;
n₄ is an integer from 0 to 15;
n₅ is an integer from 0 to 15;
R₁₂ is selected from C₁-C₈ alkyl and trifluoromethyl.
ring A is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring B is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring C is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring D is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino.

Further, R₁ and R₂, together with the N atom to which they are attached, form piperidyl substituted with 0-2 R₅, 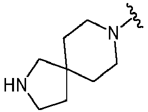 substituted with 0-2 R₅, 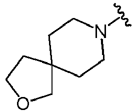 substituted with 0-2 R₅, 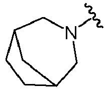 substituted with 0-2 R₅, 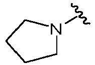 substituted with 0-2 R₅, and 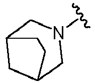 substituted with 0-2 R₅;
each R₅ is independently selected from the group consisting of C₁-C₈ alkyl substituted with 0-2 R₆, C₁-C₈ alkoxy, carboxyl, nitro, cyano, amino, halogen, hydroxyl, -N(H)C(O)R₇, -N(H)R₇, and -C(O)Rs;
each R₆ is independently selected from the group consisting of hydroxyl, amino, halogen, carboxyl, nitro, cyano, C₁-C₈ alkoxy, and -N(H)R₇;
R₇ is selected from the group consisting of C₁-C₈ alkyl substituted with 0-5 R₉, C₁-C₈ alkoxy, and tert-butoxycarbonyl;
R₈ is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and Ci-Cs alkoxy;
Y₁ and Y₂ are each independently selected from -N- or -CH-; and at least one of Y₁ and Y₂ is selected from -N-;
X is selected from -S- or absence;
R₃ is selected from the group consisting of hydrogen, C₁-C₈ alkyl, C₁-C₈ alkoxy, halogen, hydroxyl, carboxyl, amino, nitro, and cyano;
R₄ is selected from the group consisting of 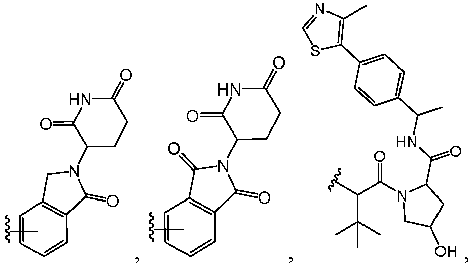 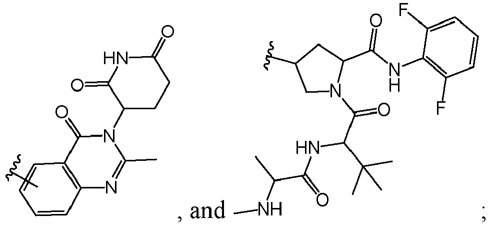 L is connected to phenyl ring at any position, and selected from the group consisting of 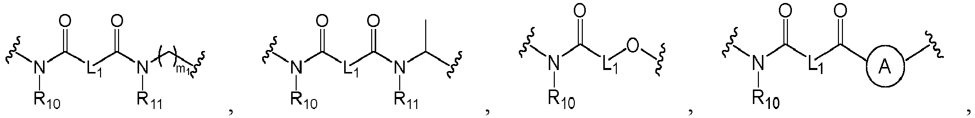 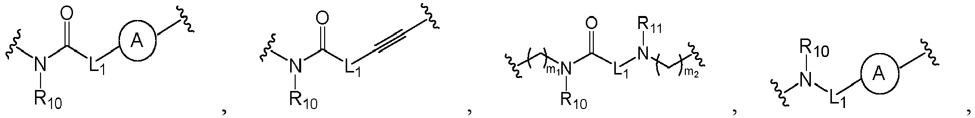 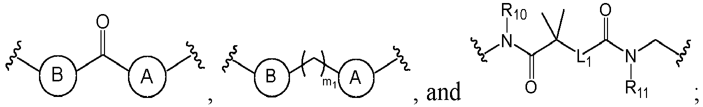
m₁ is an integer from 0 to 15;
m₂ is an integer from 0 to 15;
R₁₀ and R₁₁ are each independently selected from Hand C₁-C₈ alkyl;
L₁ is selected from the group consisting of 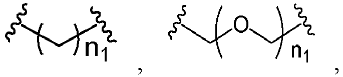 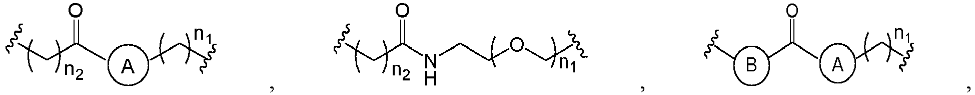 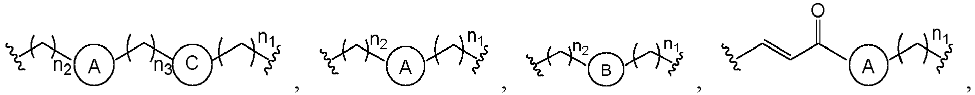 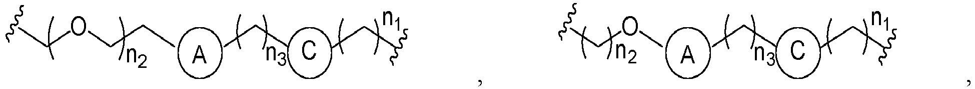 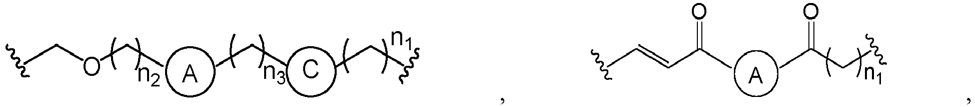 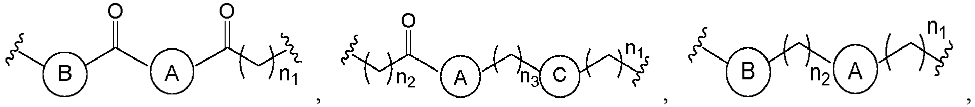 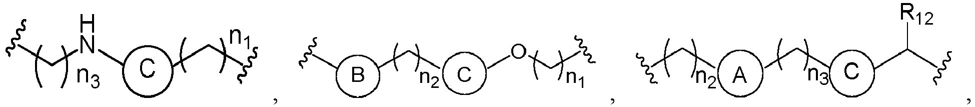 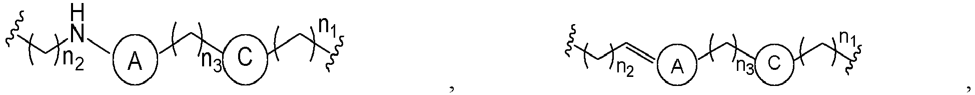 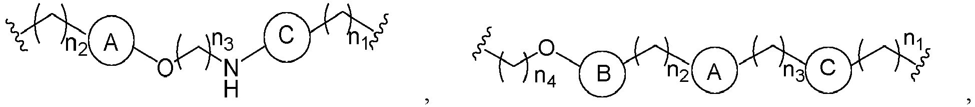 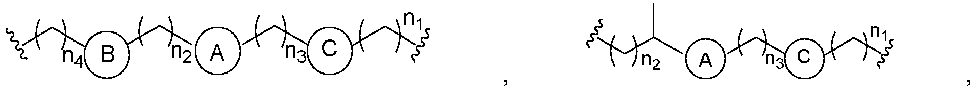 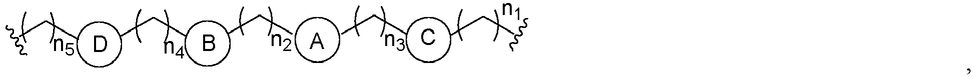 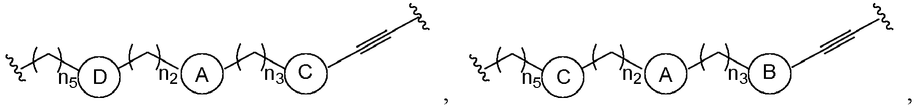 and 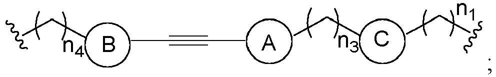
n₁ is an integer from 0 to 15;
n₂ is an integer from 0 to 15;
n₃ is an integer from 0 to 15;
n₄ is an integer from 0 to 15;
n₅ is an integer from 0 to 15;
R₁₂ is selected from C₁-C₈ alkyl and trifluoromethyl;
each ring A is independently selected from the group consisting of 3-6 membered cycloalkyl substituted with 0-3 R₁₃, piperazinyl substituted with 0-3 R₁₃, piperidyl substituted with 0-3 R₁₃, azetidinyl substituted with 0-3 R₁₃, pyrrolidinyl substituted with 0-3 R₁₃, 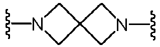 substituted with 0-3 R₁₃, 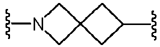 substituted with 0-3 R₁₃, 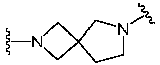 substituted with 0-3 R₁₃, 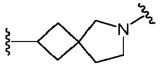 substituted with 0-3 R₁₃, 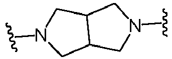 substituted with 0-3 R₁₃, 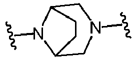 substituted with 0-3 R₁₃, 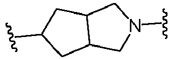 substituted with 0-3 R₁₃, 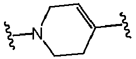 substituted with 0-3 R₁₃, 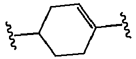 substituted with 0-3 R₁₃, phenyl substituted with 0-3 R₁₃, 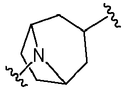 substituted with 0-3 R₁₃, 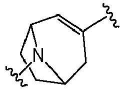 substituted with 0-3 R₁₃, 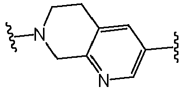 substituted with 0-3 R₁₃, 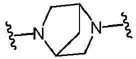 substituted with 0-3 R₁₃, 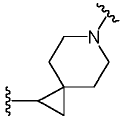 substituted with 0-3 R13, 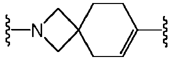 substituted with 0-3 R₁₃, 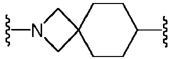 substituted with 0-3 R₁₃, 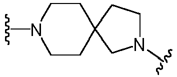 substituted with 0-3 R₁₃, and 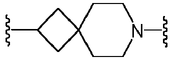 substituted with 0-3 R₁₃;
each ring B is independently selected from the group consisting of 3-6 membered cycloalkyl substituted with 0-3 R₁₃, phenyl substituted with 0-3 R₁₃, piperidyl substituted with 0-3 R₁₃, pyrrolidinyl substituted with 0-3 R₁₃, piperazinyl substituted with 0-3 R₁₃, 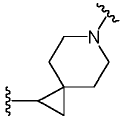 substituted with 0-3 R₁₃, 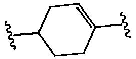 substituted with 0-3 R₁₃, 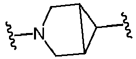 substituted with 0-3 R₁₃, 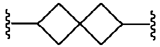 substituted with 0-3 R₁₃, 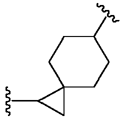 substituted with 0-3 R₁₃, and 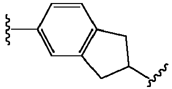 substituted with 0-3 R₁₃;
each ring C is independently selected from the group consisting of phenyl substituted with 0-3 R₁₃, pyrimidinyl substituted with 0-3 R₁₃, pyridazinyl substituted with 0-3 R₁₃, pyrazolyl substituted with 0-3 R₁₃, and pyrazinyl substituted with 0-3 R₁₃;
each ring D is independently selected from the group consisting of phenyl substituted with 0-3 R₁₃, thienyl substituted with 0-3 R₁₃, cycloalkyl substituted with 0-3 R₁₃, pyridyl substituted with 0-3 R₁₃, pyridazinyl substituted with 0-3 R₁₃, 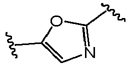 substituted with 0-3 R₁₃, 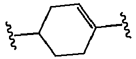 substituted with 0-3 R₁₃, and 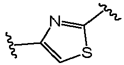 substituted with 0-3 R₁₃;
each R₁₃ is independently selected from the group consisting of C₁-C₈ alkyl substituted with 0-3 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino.

Further, the compound is as represented by formula II: 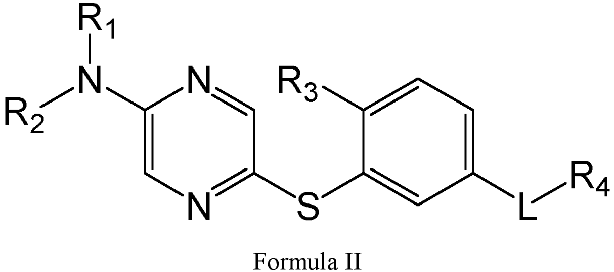 wherein,
R₁ and R₂, together with the N atom to which they are attached, form a 5-10 membered heterocyclic group substituted with 0-5 R₅;
each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
each R₆ is independently selected from the group consisting of hydroxyl, amino, halogen, carboxyl, nitro, cyano, C₁-C₈ alkoxy, and -N(H)R₇;
R₇ is independently selected from the group consisting of C₁-C₈ alkyl substituted with 0-5 R₉, C₁-C₈ alkoxy, and tert-butoxycarbonyl;
R₈ is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy;
R₃ is selected from the group consisting of hydrogen, C₁-C₈ alkyl, C₁-C₈ alkoxy, halogen, hydroxyl, carboxyl, amino, nitro, and cyano;
R₄ is selected from the group consisting of 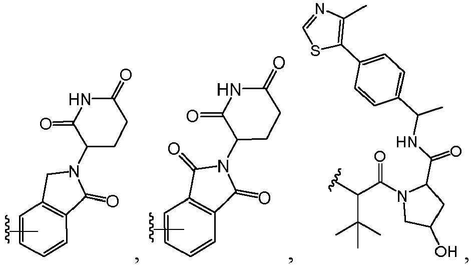 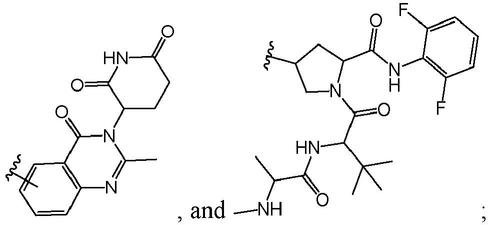
L is connected to phenyl ring at any position, and selected from the group consisting of 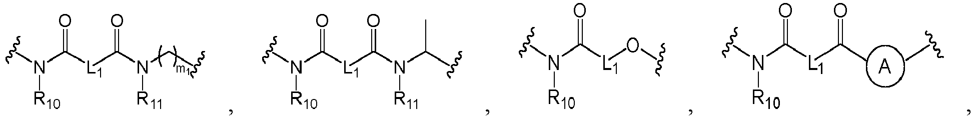 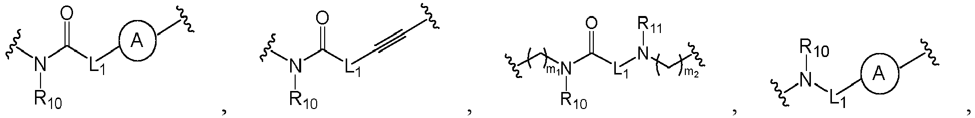 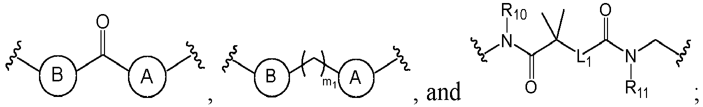
m₁ is an integer from 0 to 15;
m₂ is an integer from 0 to 15;
R₁₀ and R₁₁ are each independently selected from Hand C₁-C₈ alkyl;
L₁ is selected from the group consisting of 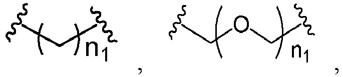 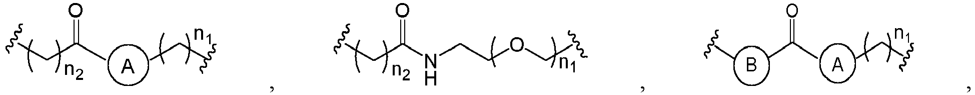 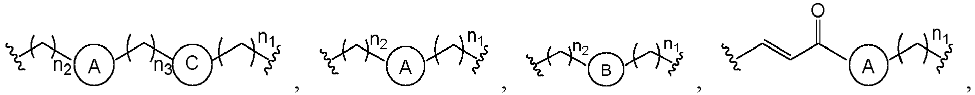 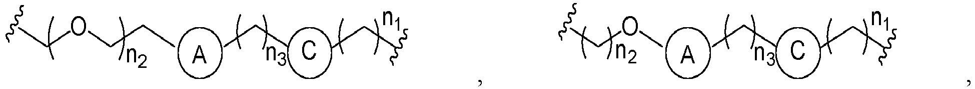 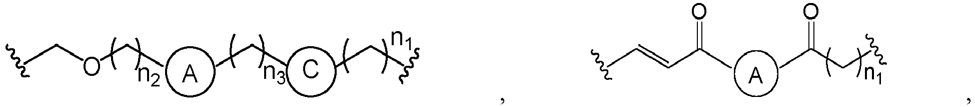 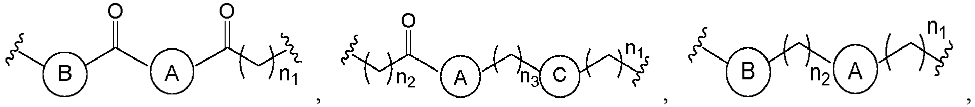 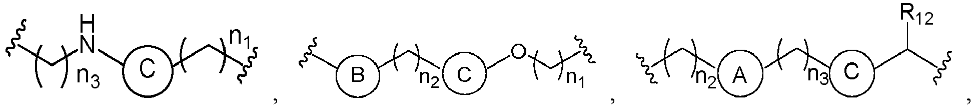 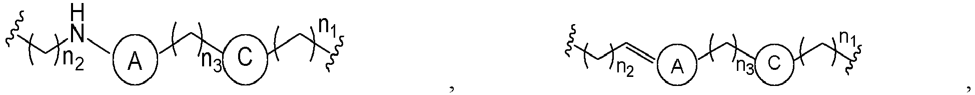 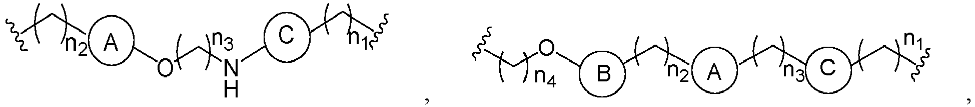 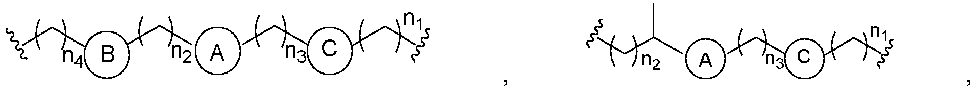 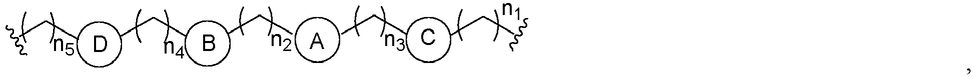 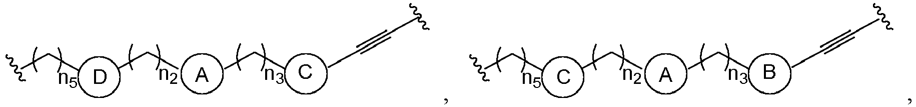 and 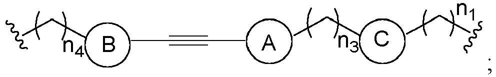
n₁ is an integer from 0 to 15;
n₂ is an integer from 0 to 15;
n₃ is an integer from 0 to 15;
n₄ is an integer from 0 to 15;
n₅ is an integer from 0 to 15;
R₁₂ is selected from C₁-C₈ alkyl and trifluoromethyl;
ring A is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring B is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring C is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring D is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino;
preferably,
R₁, R₂, R₃, R₄, and L are as defined in the above.

Further, the compound is as represented by formula III: 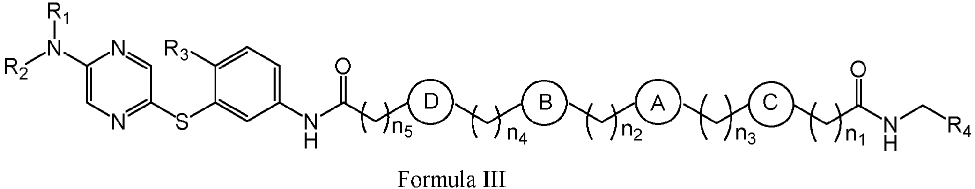 wherein,
R₁ and R₂, together with the N atom to which they are attached, form a 5-10 membered heterocyclic group substituted with 0-5 R₅;
each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
each R₆ is independently selected from the group consisting of hydroxyl, amino, halogen, carboxyl, nitro, cyano, C₁-C₈ alkoxy, and -N(H)R₇;
R₇ is independently selected from the group consisting of C₁-C₈ alkyl substituted with 0-5 R₉, C₁-C₈ alkoxy, and tert-butoxycarbonyl;
R₈ is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and Ci-Cs alkoxy;
R₃ is selected from the group consisting of hydrogen, C₁-C₈ alkyl, C₁-C₈ alkoxy, halogen, hydroxyl, carboxyl, amino, nitro, and cyano;
R₄ is selected from the group consisting of 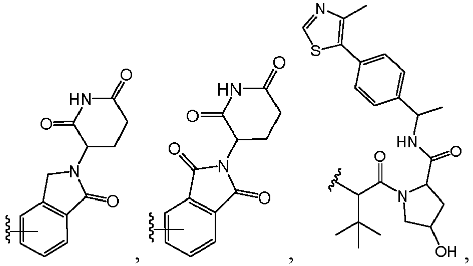 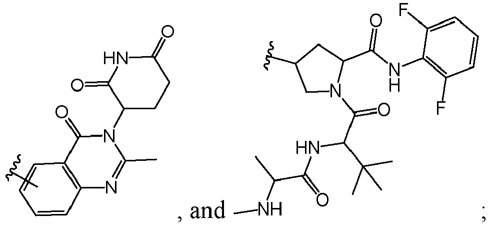
n₁ is an integer from 0 to 15;
n₂ is an integer from 0 to 15;
n₃ is an integer from 0 to 15;
n₄ is an integer from 0 to 15;
n₅ is an integer from 0 to 15;
ring A is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring B is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring C is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring D is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino;
preferably,
R₁, R₂, R₃, R₄, n₁, n₂, n₃, n₄, n₅, ring A, ring B, ring C, and ring D are as defined in the above. Further, the compound is as represented by formula IV: 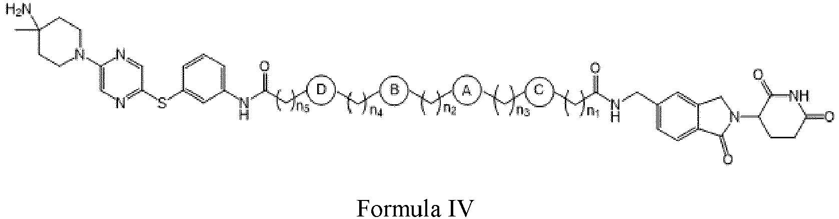 wherein,
   n₁ is an integer from 0 to 15;
   n₂ is an integer from 0 to 10;
   n₃ is an integer from 0 to 10;
   n₄ is an integer from 0 to 10;
   n₅ is an integer from 0 to 10;
   ring A is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
   ring B is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
   ring C is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
   ring D is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
   each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
   each R₁₄ is independently selected from the group consisting of hydroxyl, carboxyl, halogen, and amino;
   preferably,
   n₁, n₂, n₃, n₄, n₅, ring A, ring B, ring C, and ring D are as defined in the above.

Further, the compound is as represented by formula V: 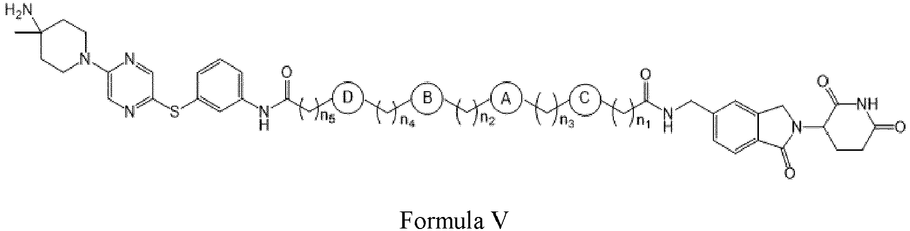 wherein,
ring A is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring B is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring C is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring D is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino;
preferably,
ring A, ring B, ring C, and ring D are as defined in the above.

Further,
rings A and B are selected from 4-10 membered heterocyclic group substituted with 0-5 R₁₃, and 3-10 membered cycloalkyl substituted with 0-5 R₁₃;
rings C and D are selected from 5-10 membered aryl substituted with 0-5 R₁₃, and 5-10 membered heteroaryl substituted wtih 0-5 R₁₃;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino;
preferably,
each group is as defined in the above.

Further, the compound is as represented by formula VI: 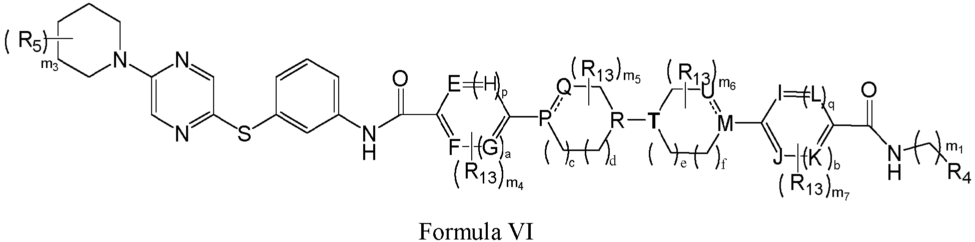 wherein,
R₅ is a substituent in ring at any position, and each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
m₃ is an integer from 0 to 5;
each R₆ is independently selected from the group consisting of hydroxyl, amino, halogen, carboxyl, nitro, cyano, C₁-C₈ alkoxy, and -N(H)R₇;
R₇ is selected from the group consisting of C₁-C₈ alkyl substituted with 0-5 R₉, C₁-C₈ alkoxy, and tert-butoxycarbonyl;
R₈ is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy; R₄ is selected from the group consisting of 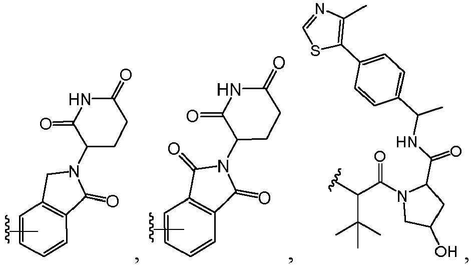 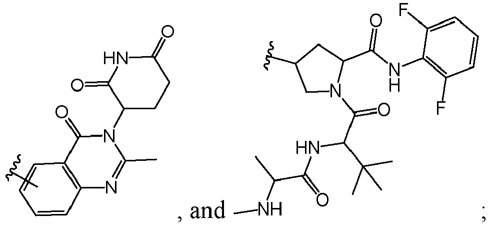
E, F, G, H, I, J, K, L, M, U, T, P, Q, and Rare C or N atom;
the bond between U and M is a single or double bond;
the bond between Q and P is a single or double bond;
a, b, c, d, e, f, p, and q are each independently selected from an integer of 0 to 1;
m₄ is an integer from 0 to 5;
m₅ is an integer from 0 to 5;
m₆ is an integer from 0 to 5;
m₇ is an integer from 0 to 5;
m₁ is an integer from 0 to 15;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino.

Further, the compound is as represented by formula VII: 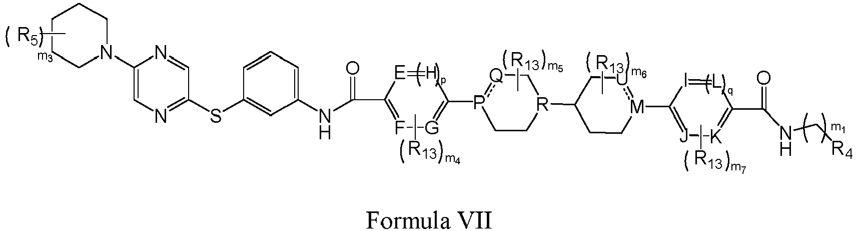 wherein,
R₅ is a substituent in ring at any position, and each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs; m₃ is an integer from 0 to 5;
each R₆ is independently selected from the group consisting of hydroxyl, amino, halogen, carboxyl, nitro, cyano, C₁-C₈ alkoxy, and -N(H)R₇;
R₇ is independently selected from the group consisting of C₁-C₈ alkyl substituted with 0-5 R₉, C₁-C₈ alkoxy, and tert-butoxycarbonyl;
R₈ is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy; R₄ is selected from the group consisting of 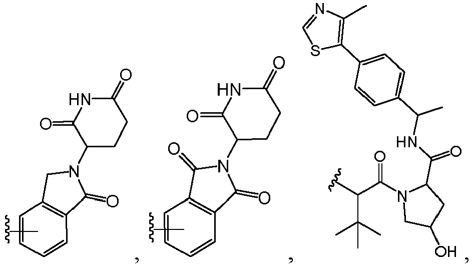 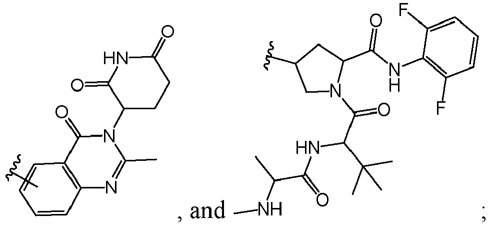
E, F, G, H, I, J, K, L, M, U, P, Q, and Rare C or N atom;
the bond between O and M is a single or double bond;
the bond between Q and P is a single or double bond;
p and q are each independently selected from an integer of 0 to 1;
m₄ is an integer from 0 to 5;
m₅ is an integer from 0 to 5;
m₆ is an integer from 0 to 5;
m₇ is an integer from 0 to 5;
m₁ is an integer from 0 to 15;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino.

Further, the compound is as represented by formula VIII: 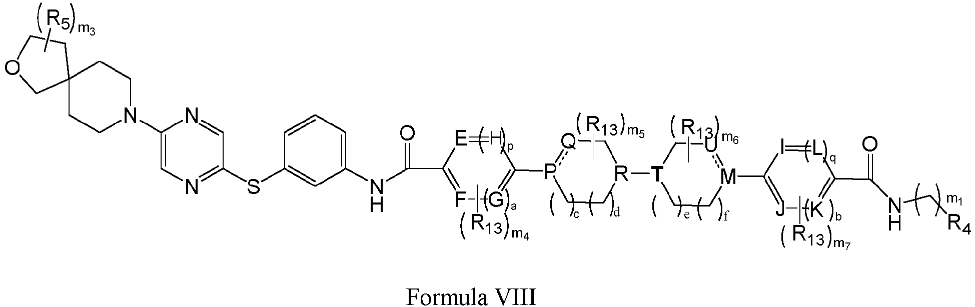 wherein,
R₅ is a substituent in ring at any position, and each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
m₃ is an integer from 0 to 2;
each R₆ is independently selected from the group consisting of hydroxyl, amino, halogen, carboxyl, nitro, cyano, C₁-C₈ alkoxy, and -N(H)R₇;
R₇ is independently selected from the group consisting of C₁-C₈ alkyl substituted with 0-5 R₉, C₁-C₈ alkoxy, and tert-butoxycarbonyl;
R₈ is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy;
R₄ is selected from the group consisting of 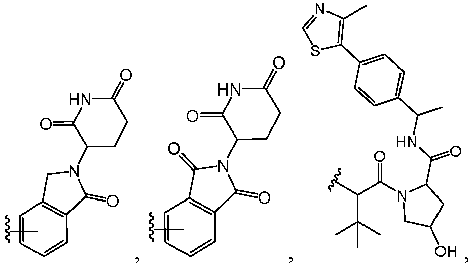 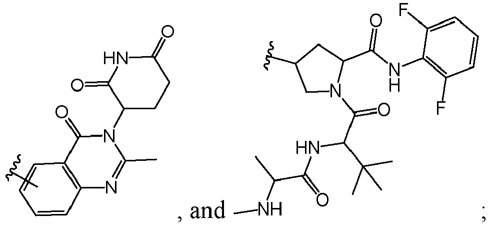
E, F, G, H, I, J, K, L, M, U, T, P, Q, and Rare C or N atom;
the bond between U and M is a single or double bond;
the bond between Q and P is a single or double bond;
a, b, c, d, e, f, p and q are each independently selected from an integer of 0 to 1;
m₄ is an integer from 0 to 5;
m₅ is an integer from 0 to 5;
m₆ is an integer from 0 to 5;
m₇ is an integer from 0 to 5;
m₁ is an integer from 0 to 15;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino.

Further, the compound is as represented by formula IX: 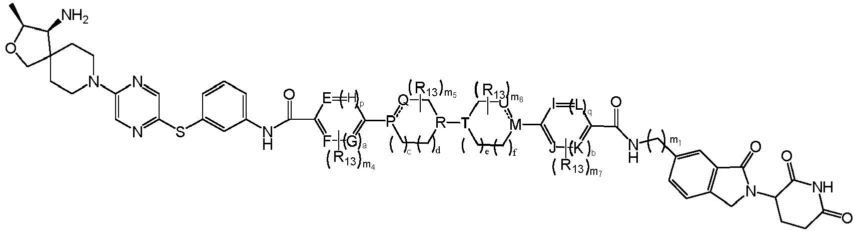 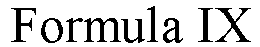 wherein,
E, F, G, H, I, J, K, L, M, U, T, P, Q, and Rare C or N atom;
the bond between U and M is a single or double bond;
the bond between Q and P is a single or double bond;
a, b, c, d, e, f, p and q are each independently selected from an integer of 0 to 1;
m₄ is an integer from 0 to 5;
m₅ is an integer from 0 to 5;
m₆ is an integer from 0 to 5;
m₇ is an integer from 0 to 5;
m₁ is an integer from 0 to 15;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino.

Further, the compound is selected from the group consisting of:

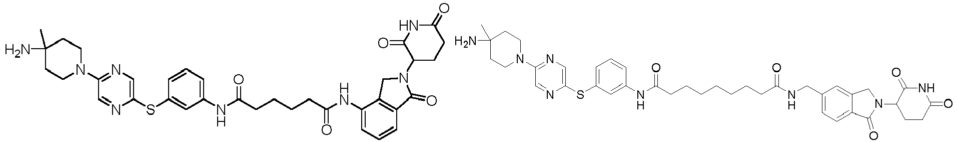

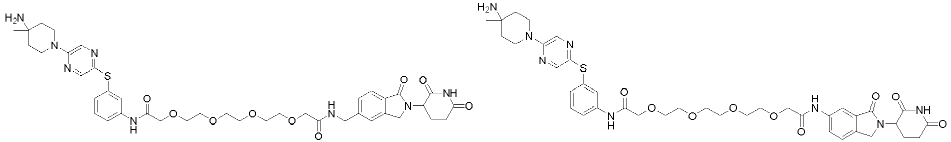

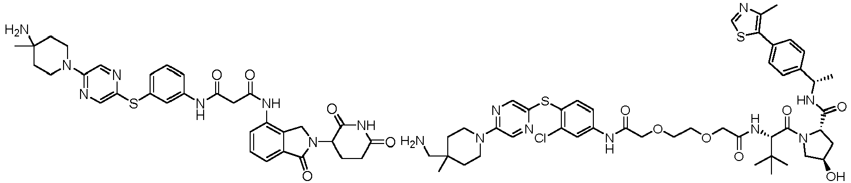

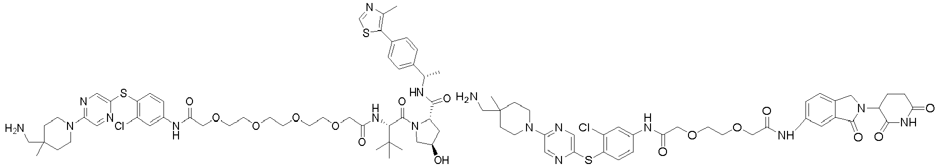

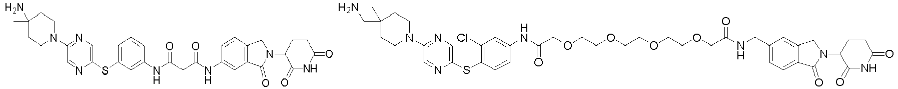

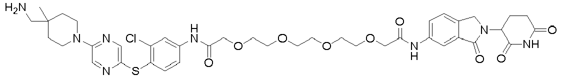

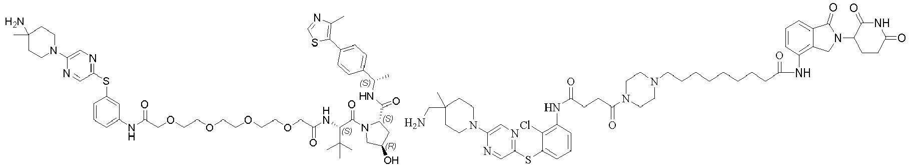

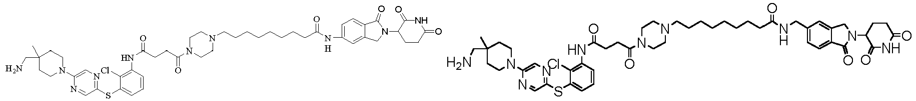

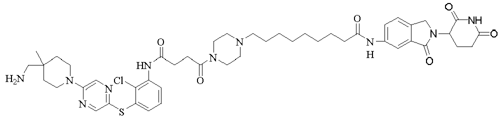

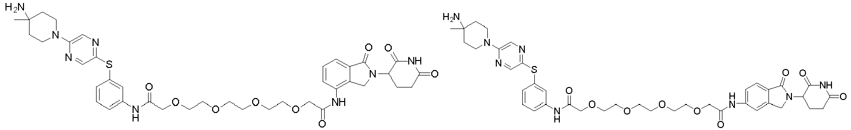

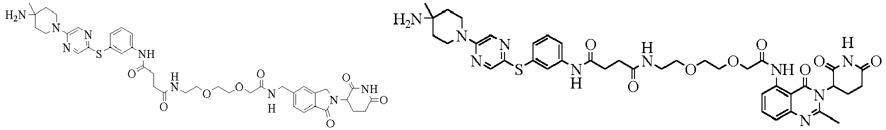

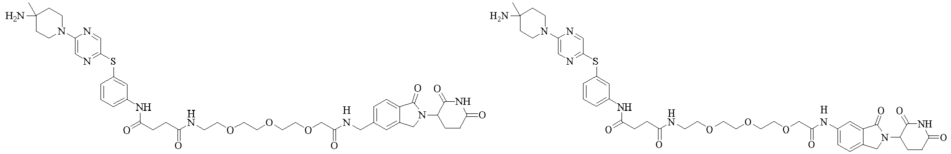

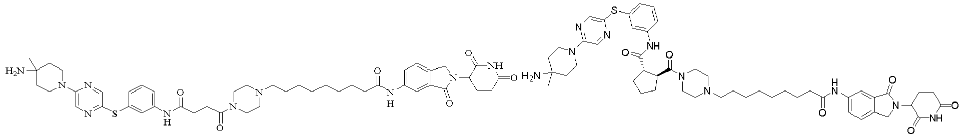

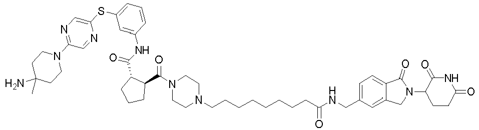

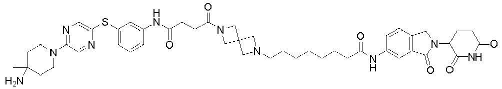

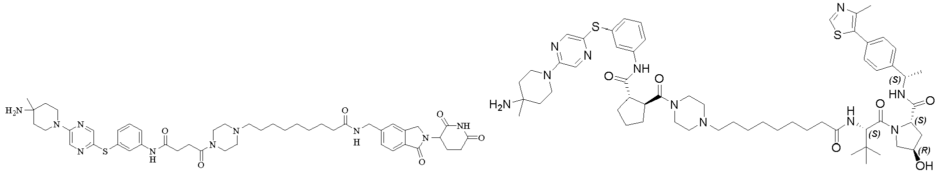

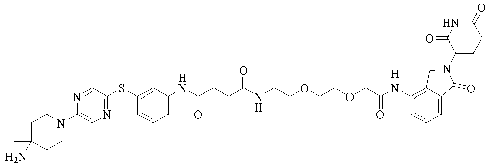

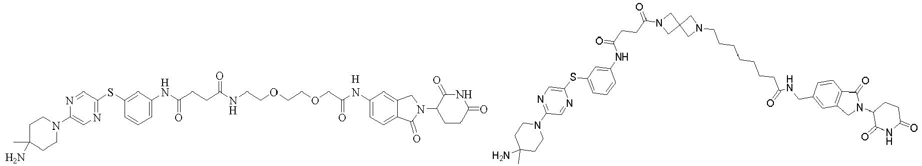

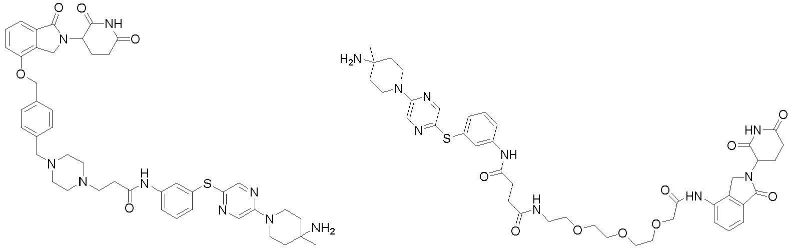

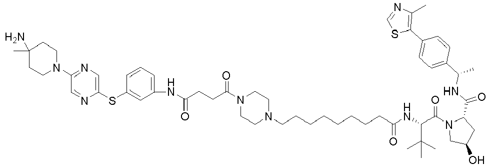

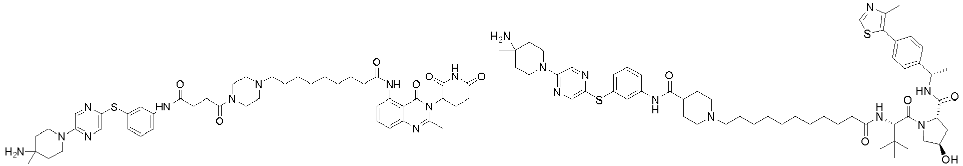

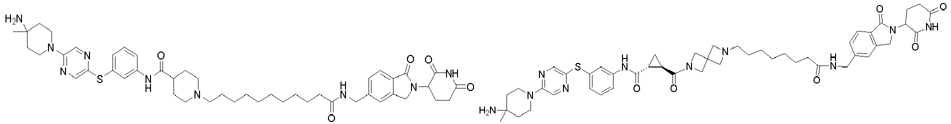

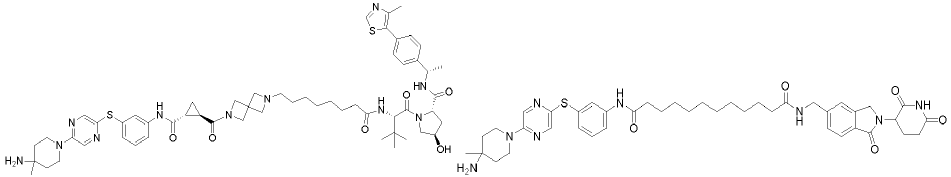

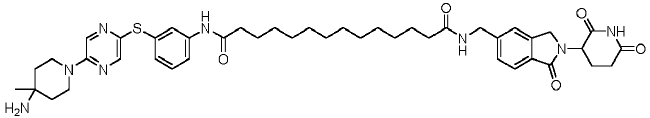

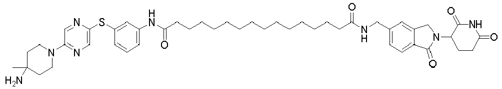

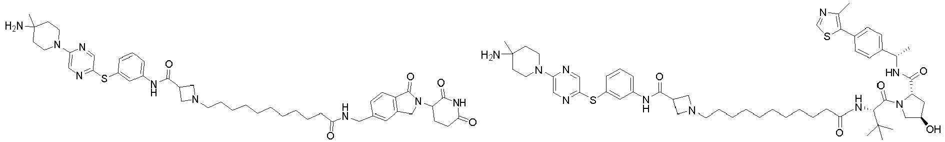

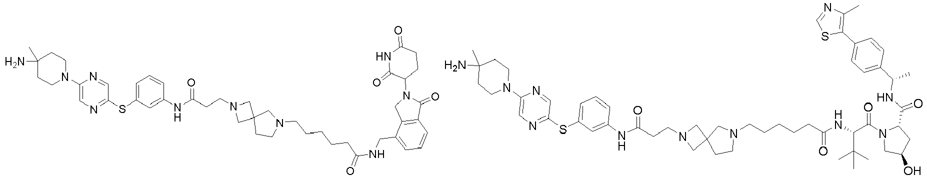

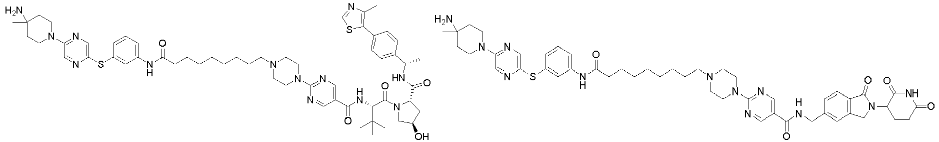

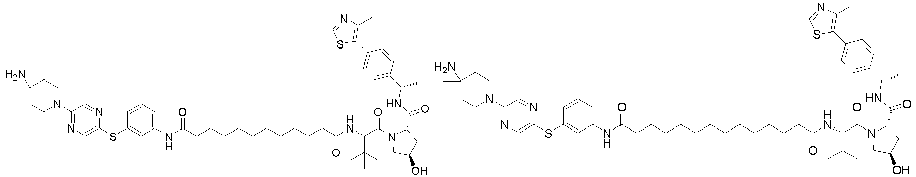

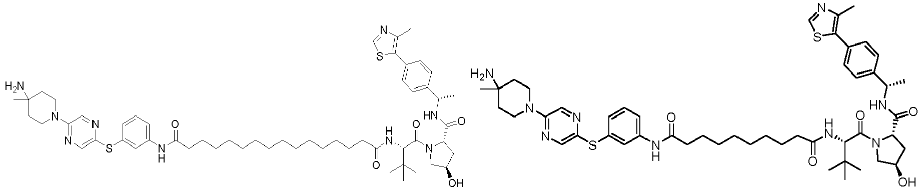

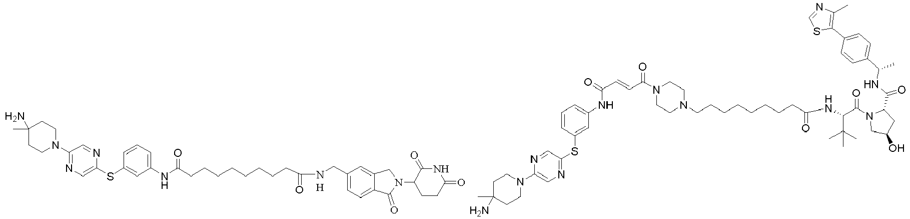

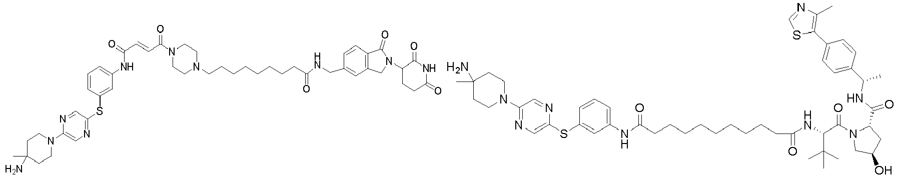

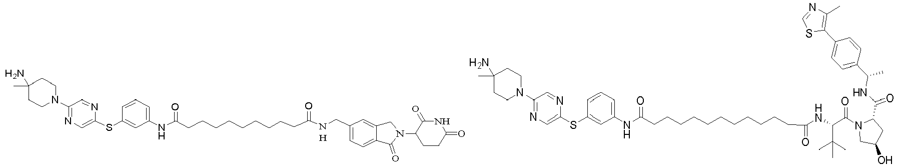

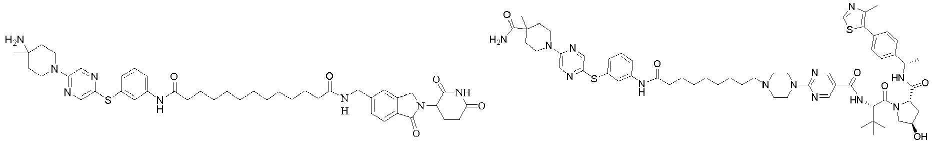

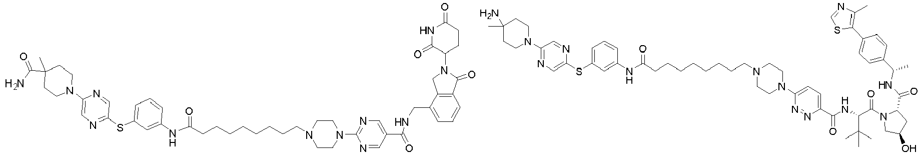

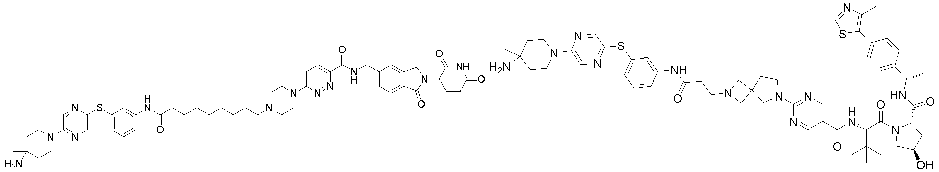

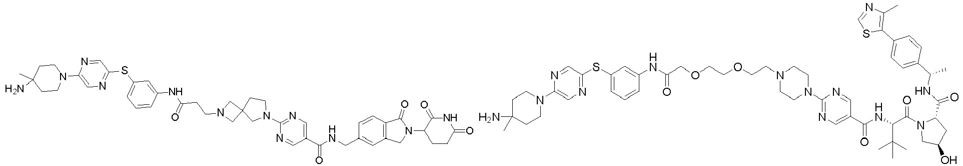

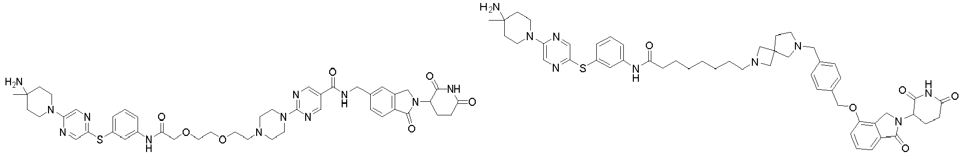

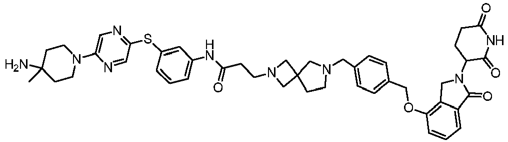

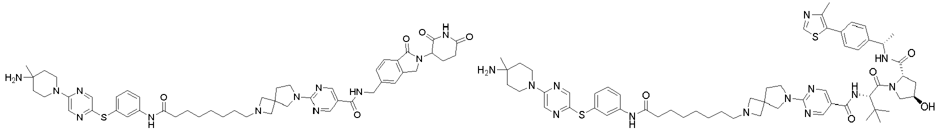

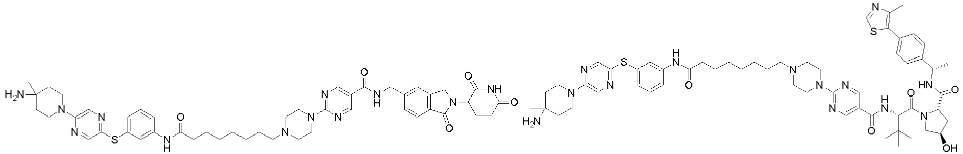

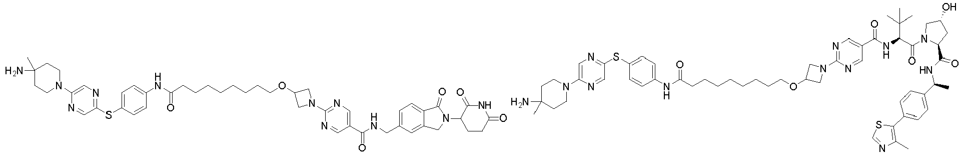

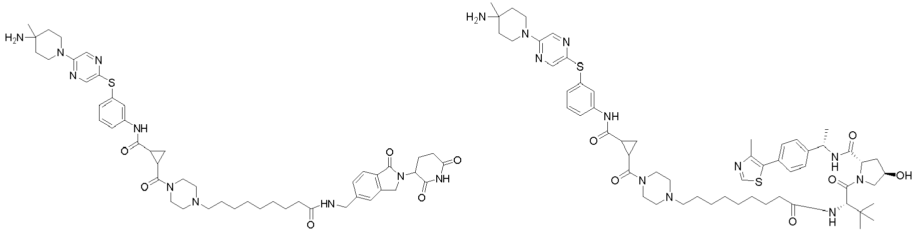

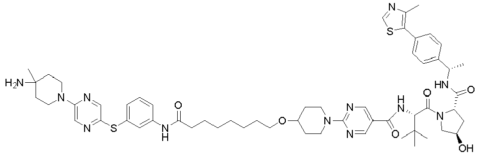

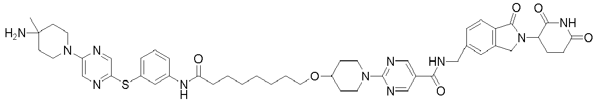

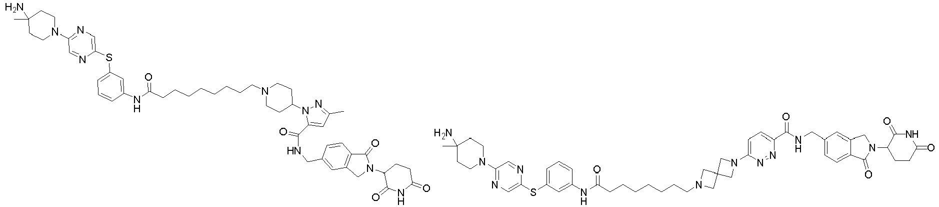

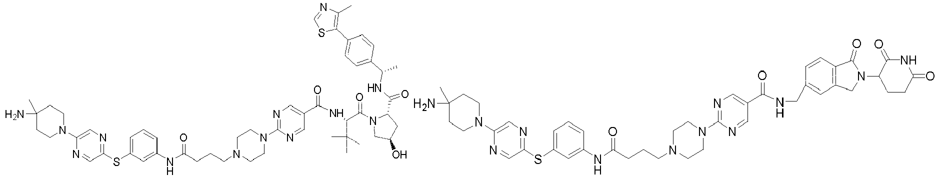

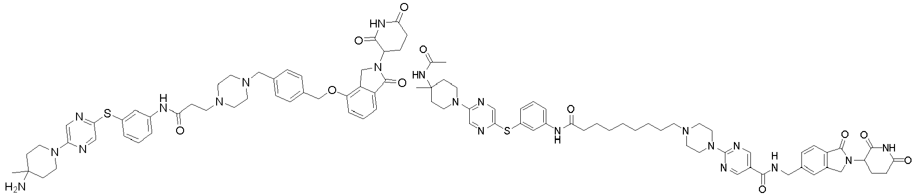

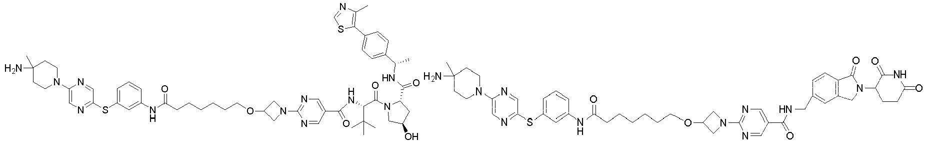

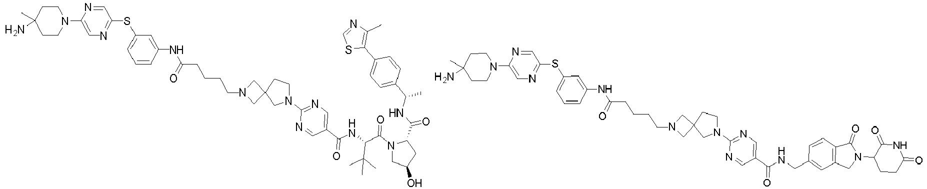

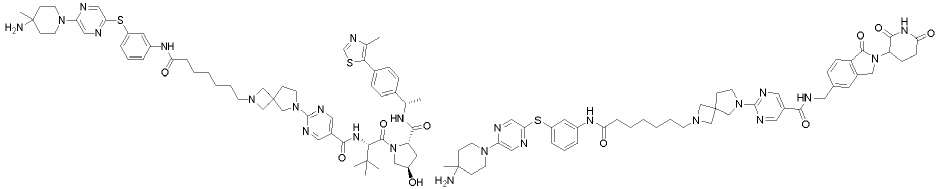

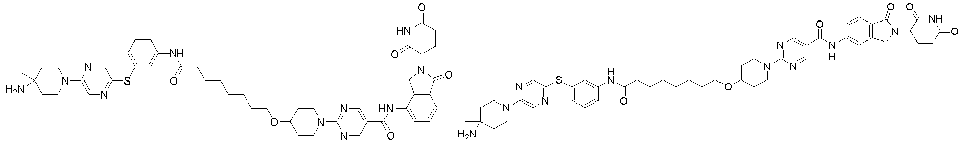

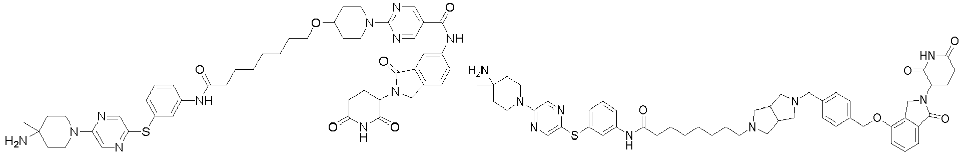

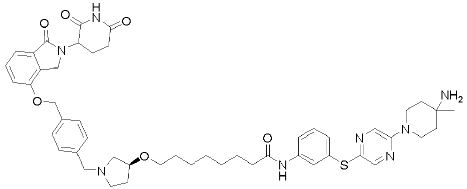

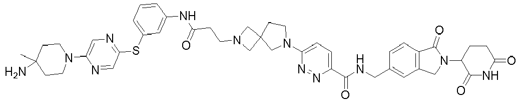

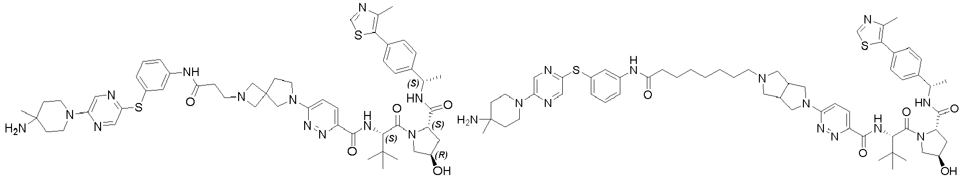

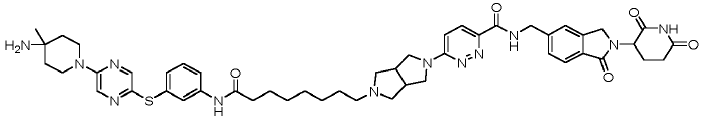

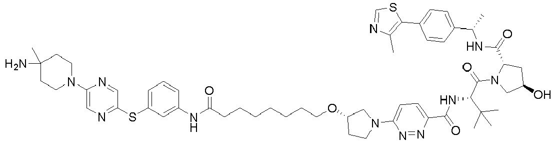

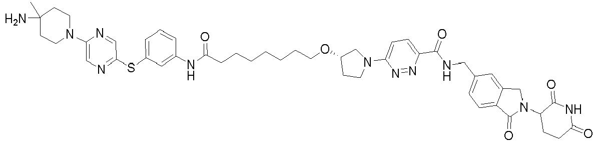

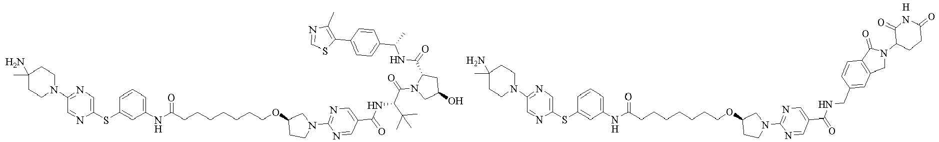

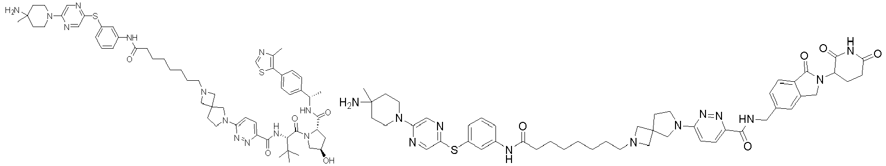

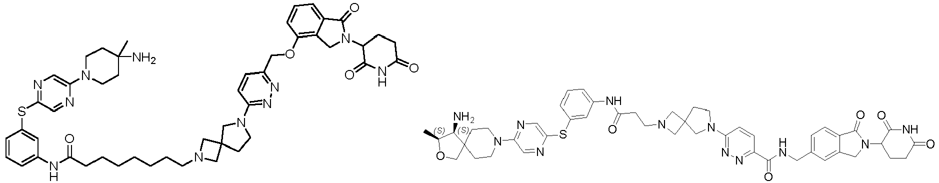

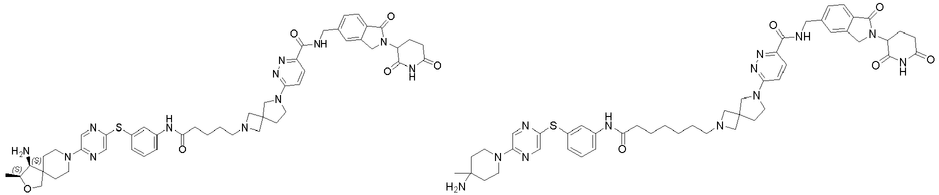

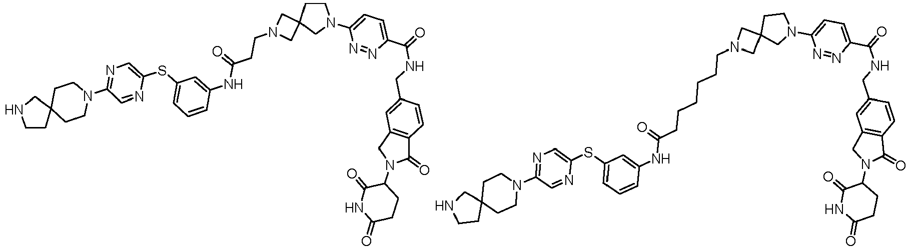

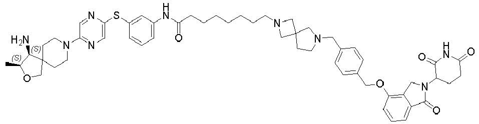

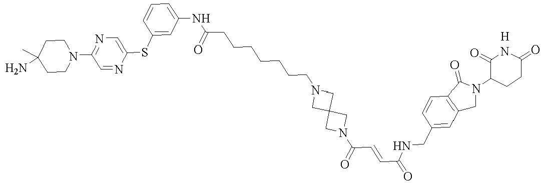

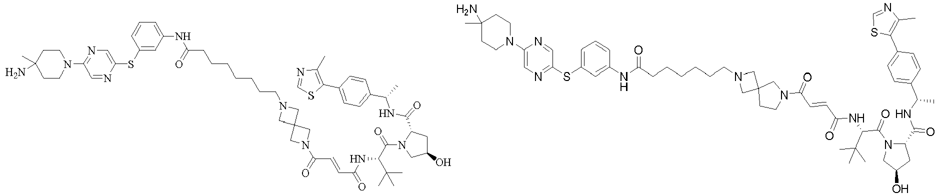

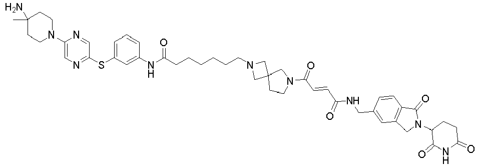

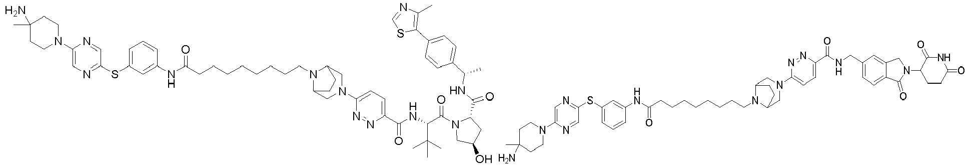

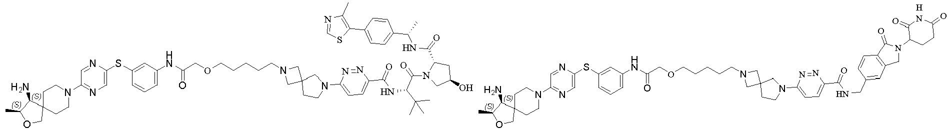

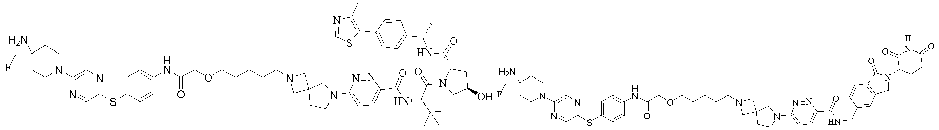

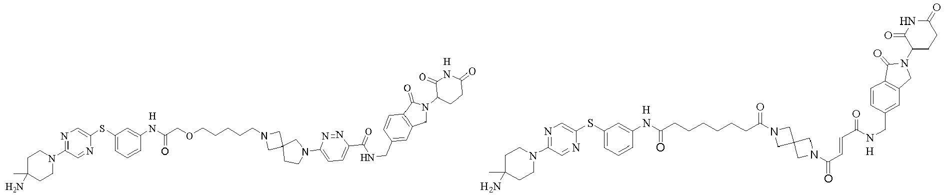

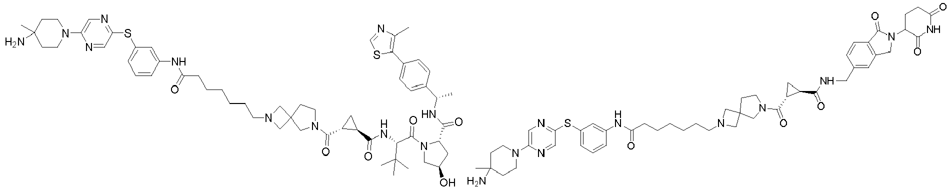

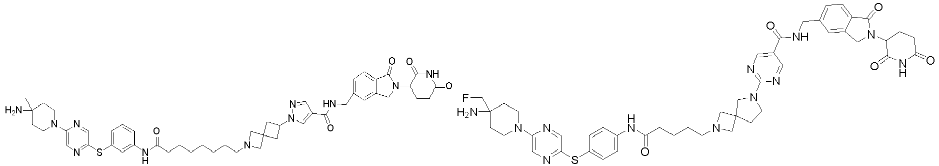

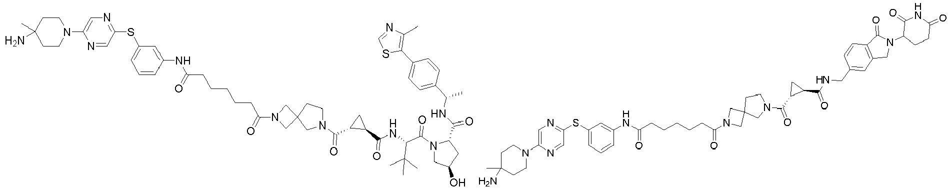

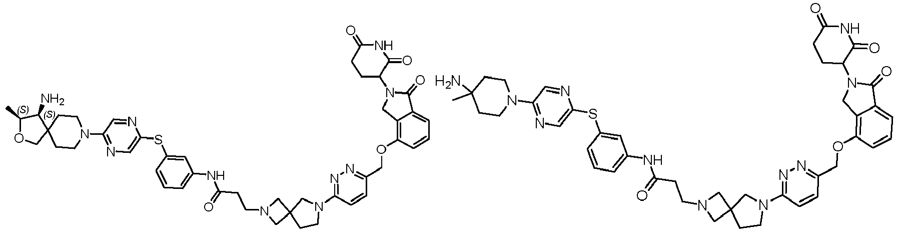

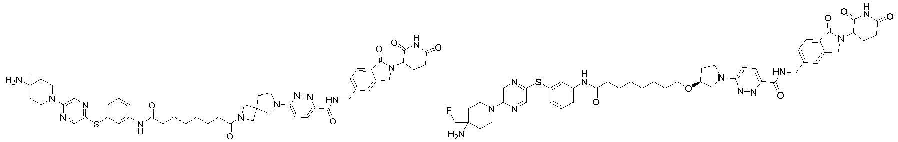

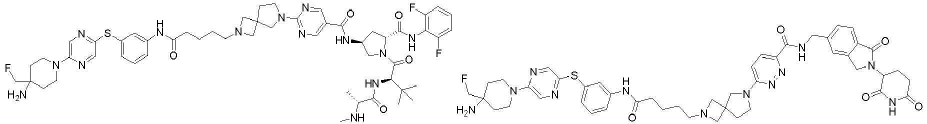

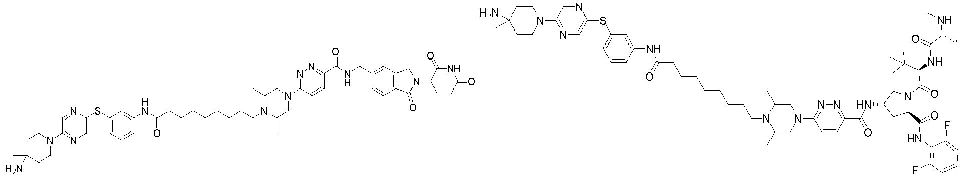

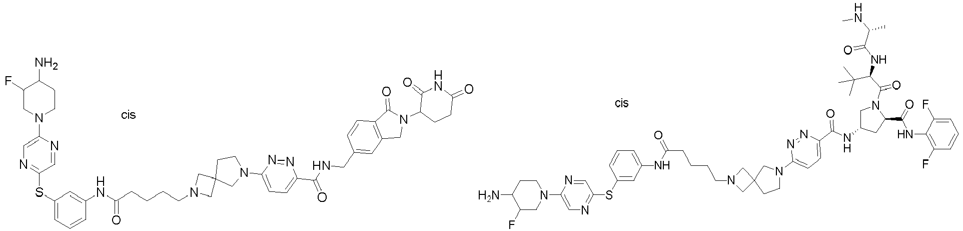

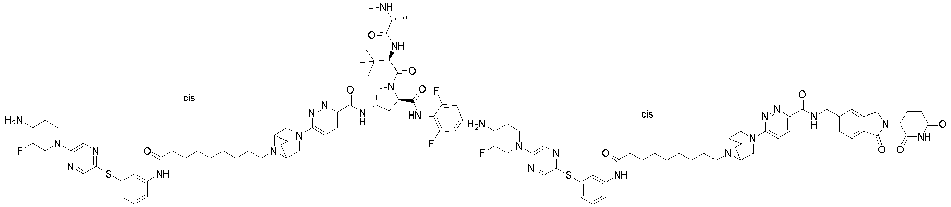

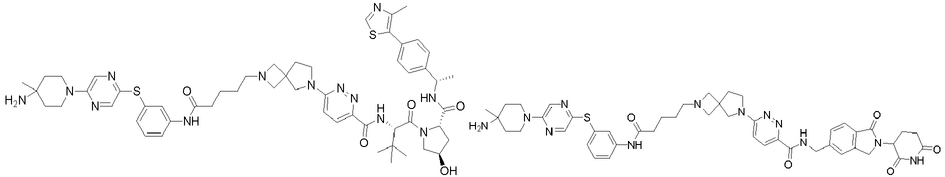

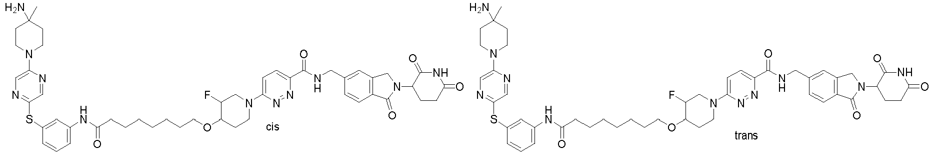

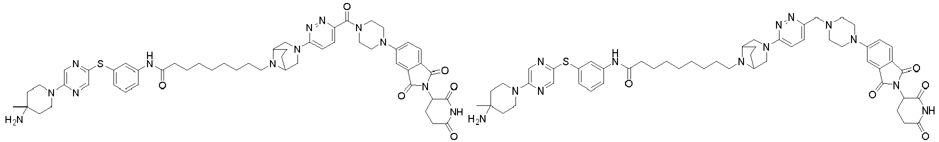

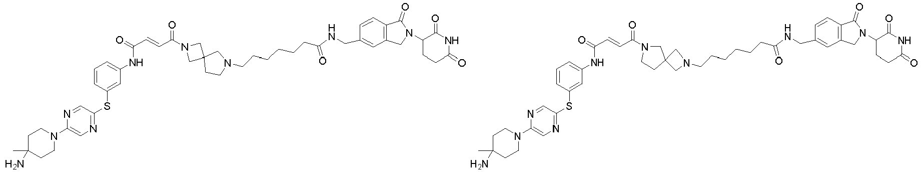

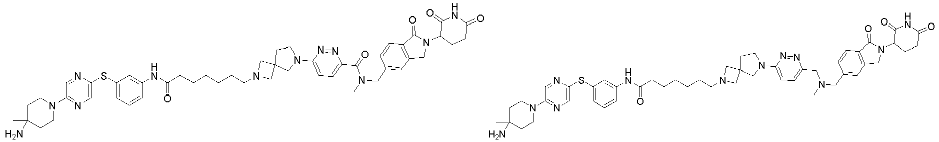

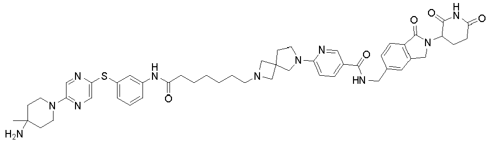

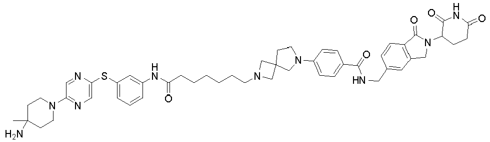

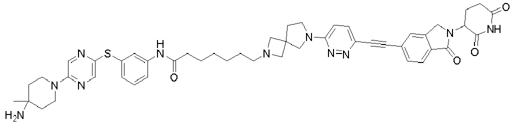

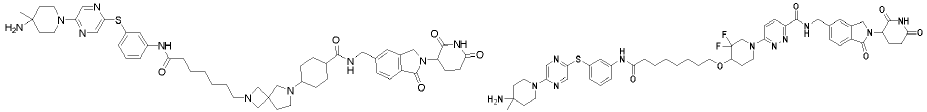

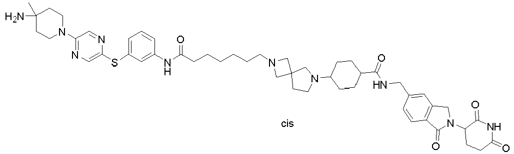

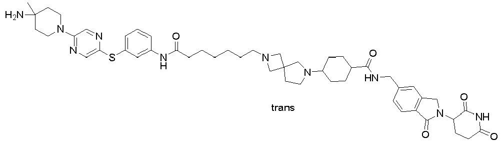

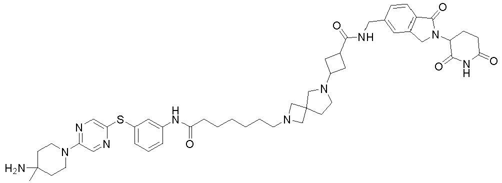

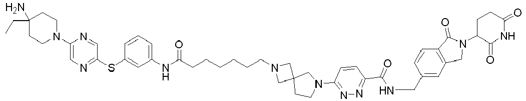

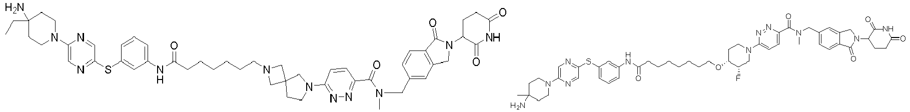

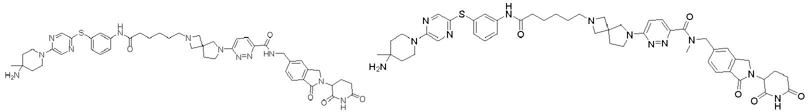

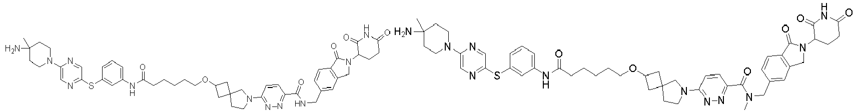

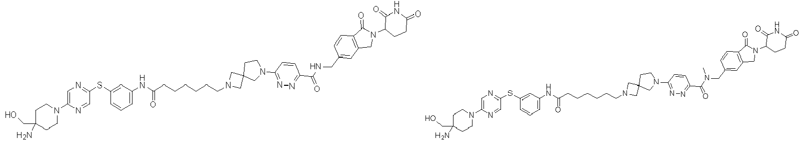

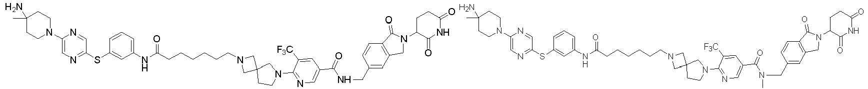

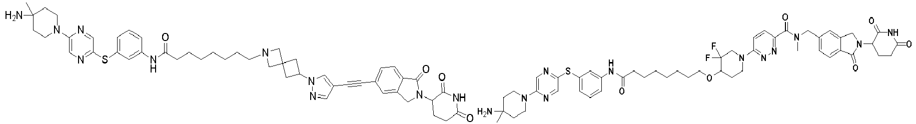

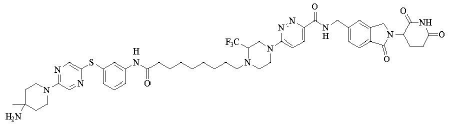

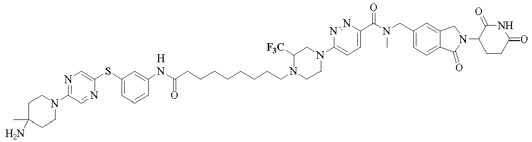

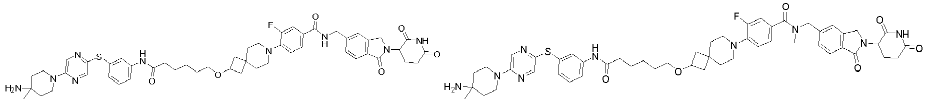

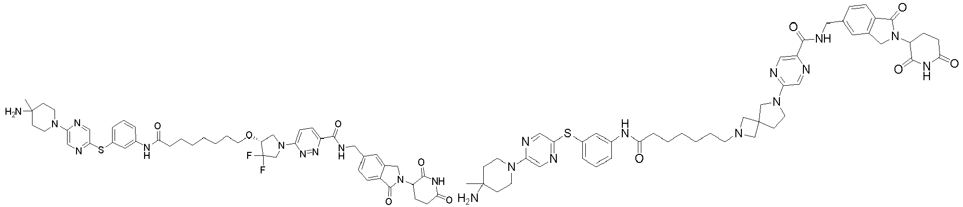

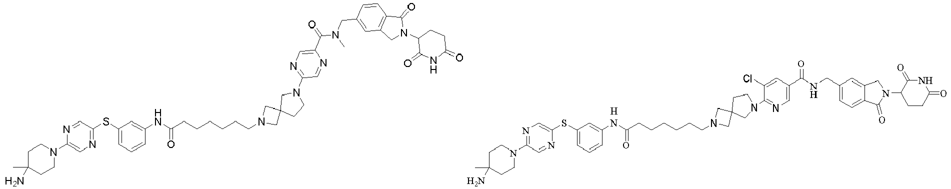

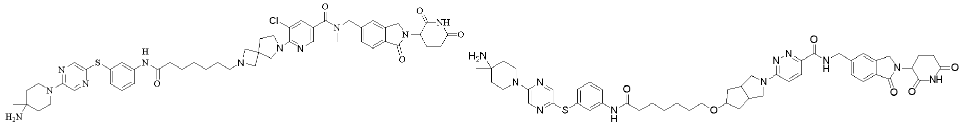

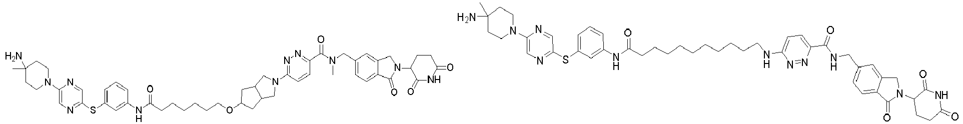

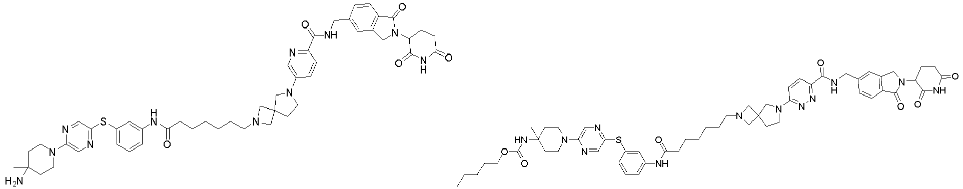

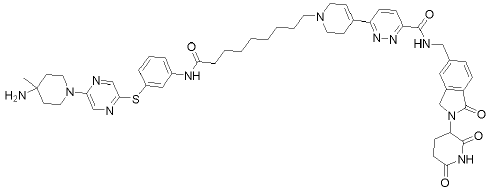

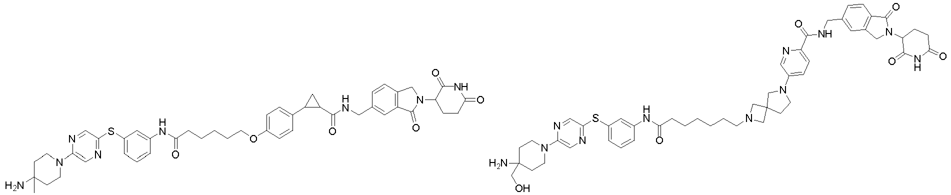

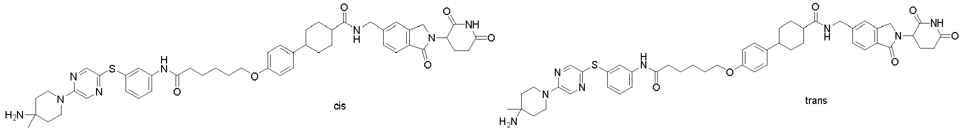

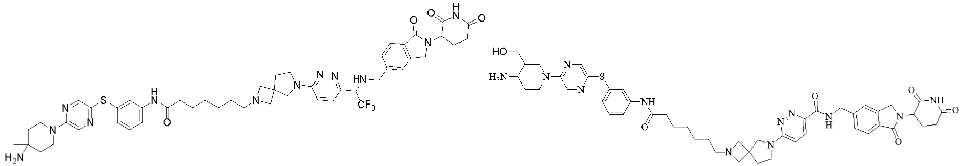

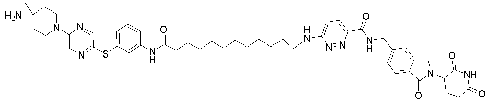

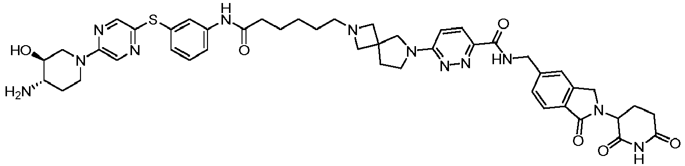

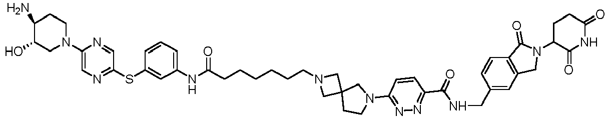

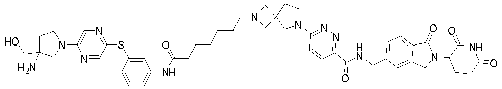

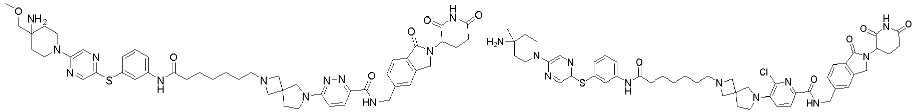

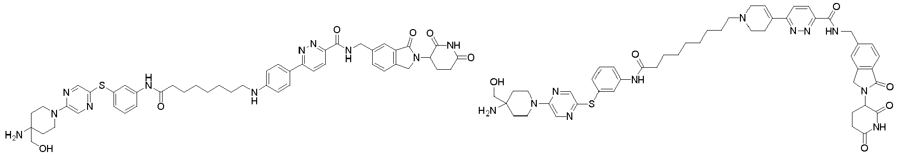

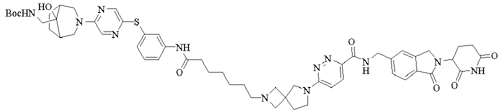

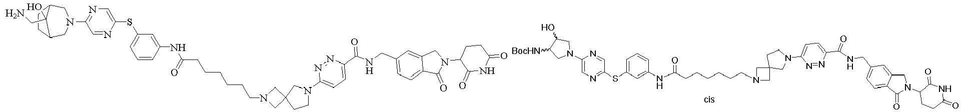

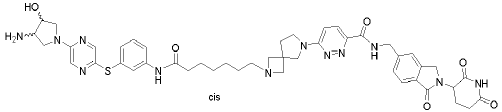

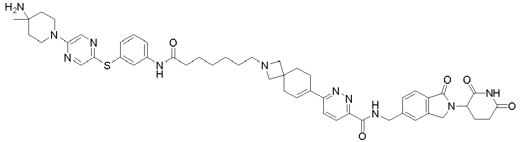

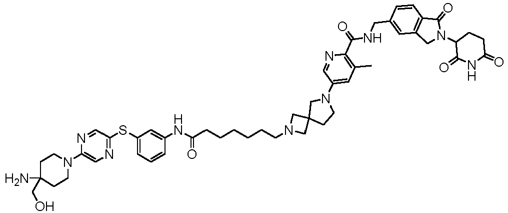

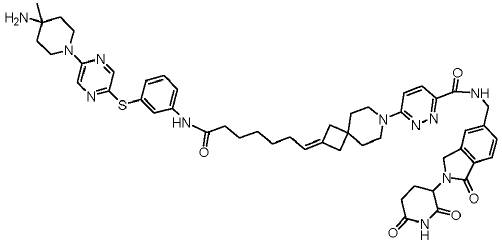

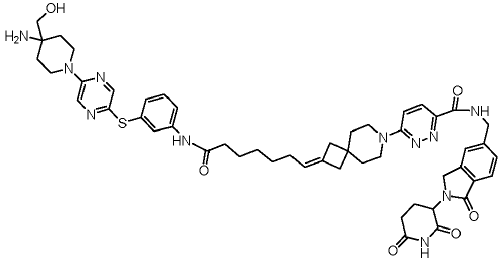

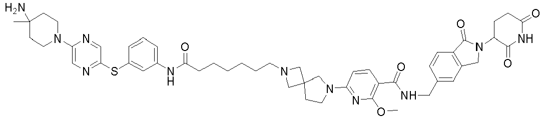

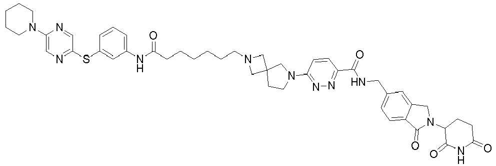

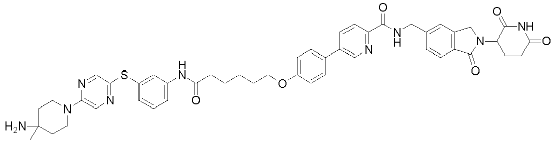

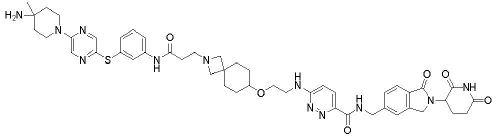

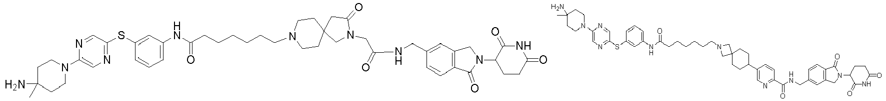

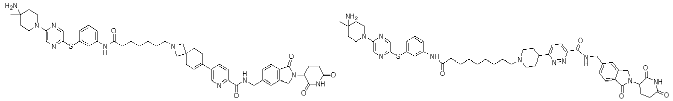

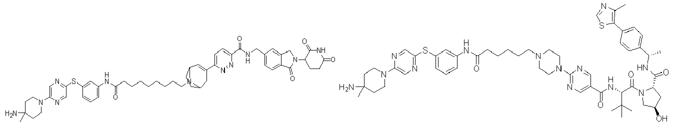

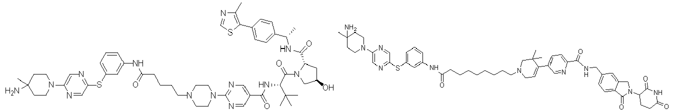

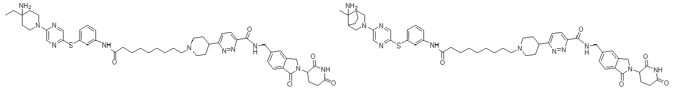

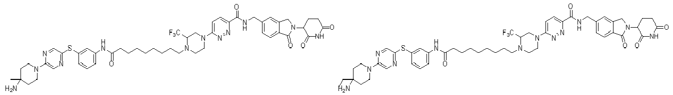

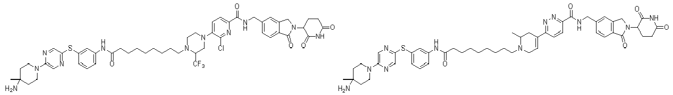

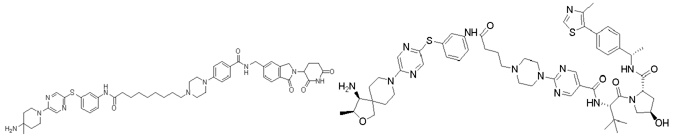

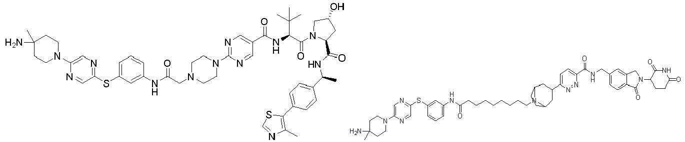

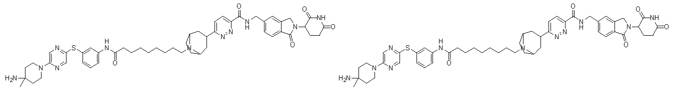

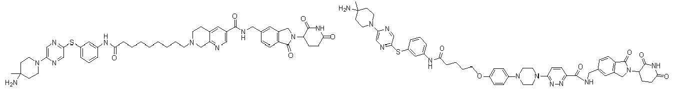

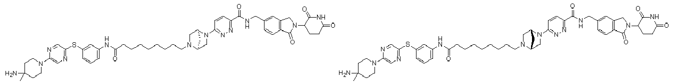

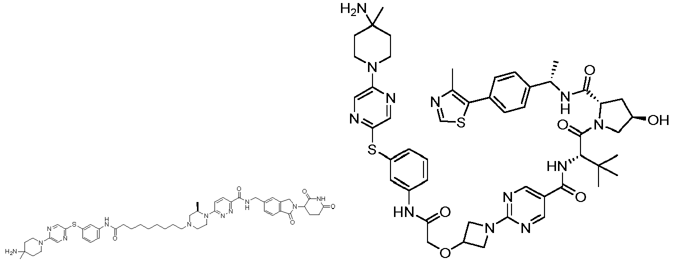

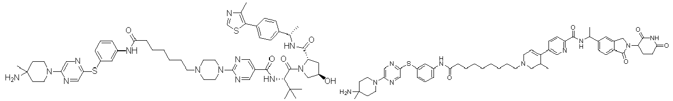

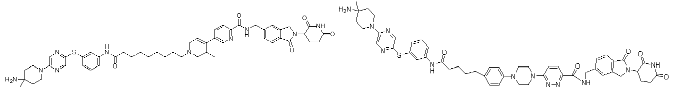

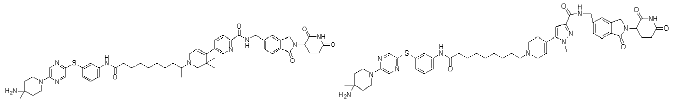

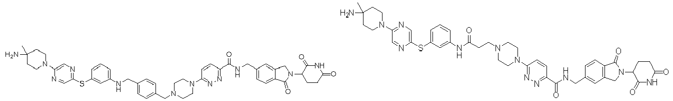

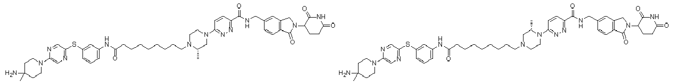

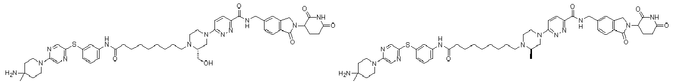

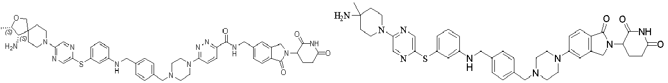

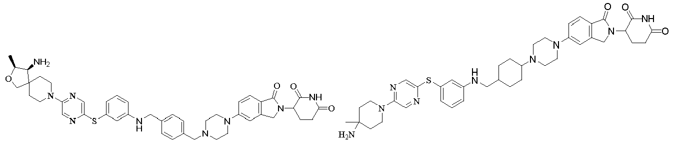

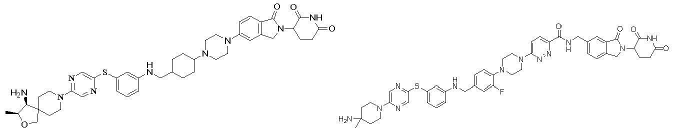

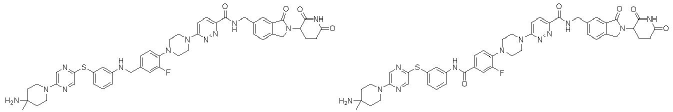

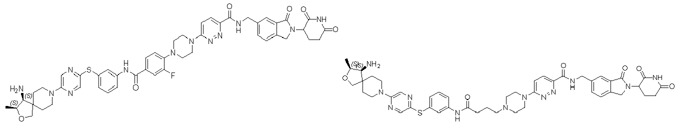

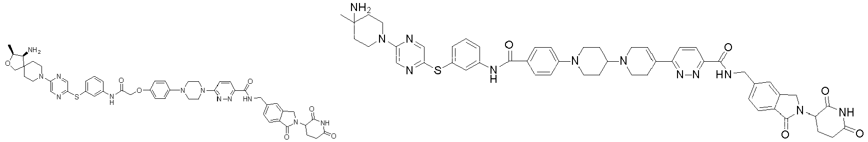

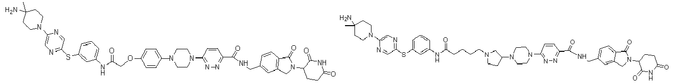

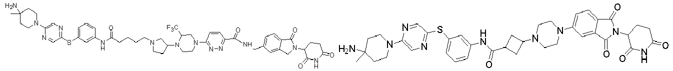

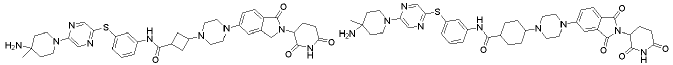

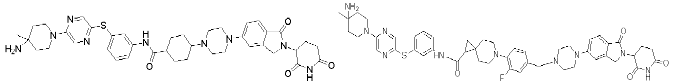

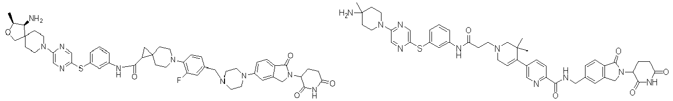

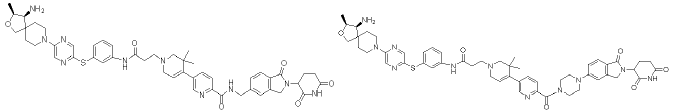

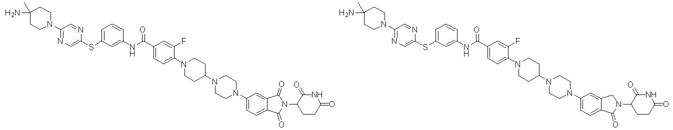

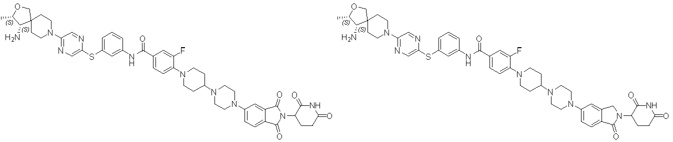

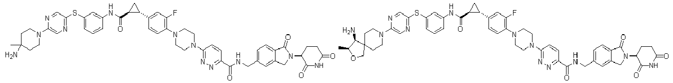

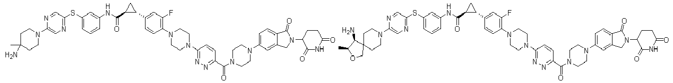

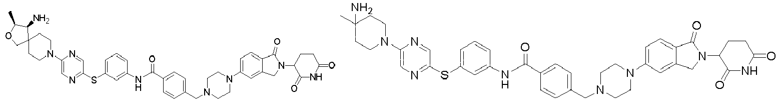

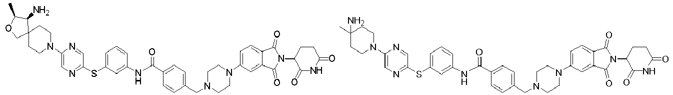

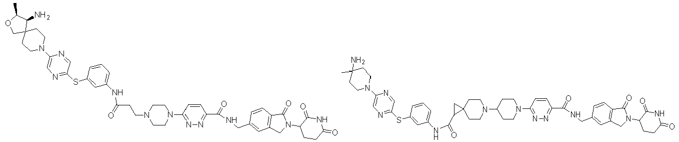

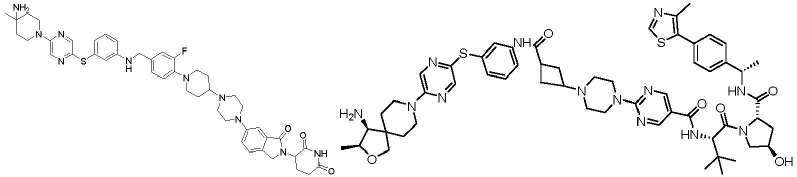

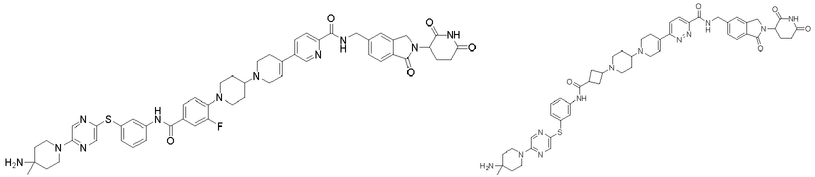

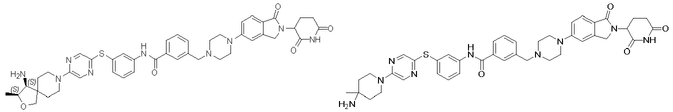

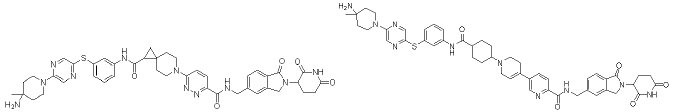

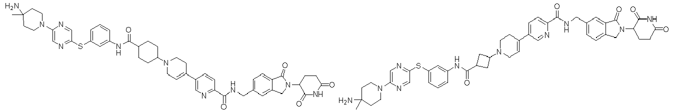

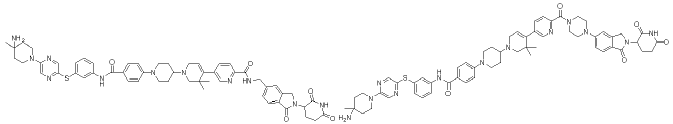

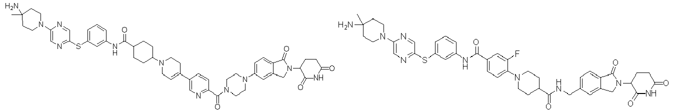

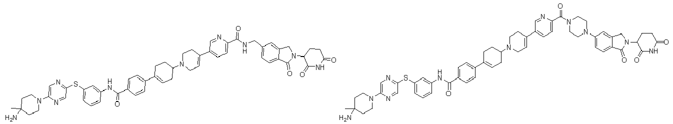

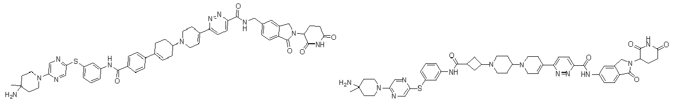

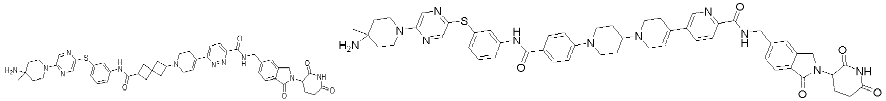

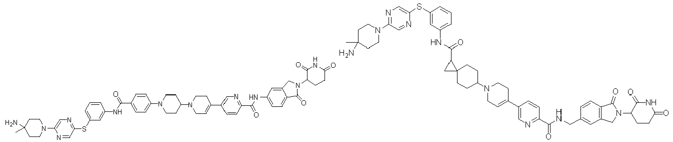

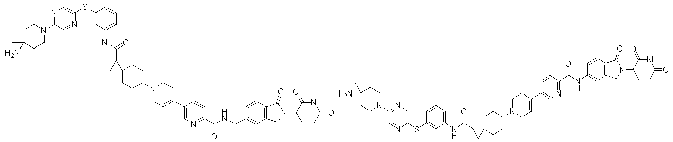

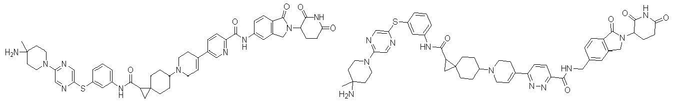

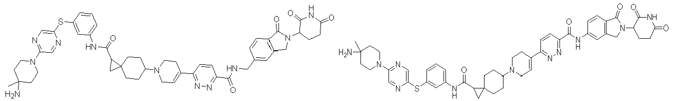

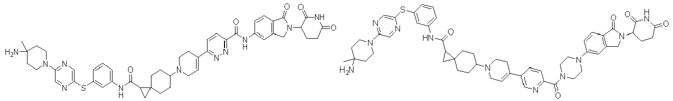

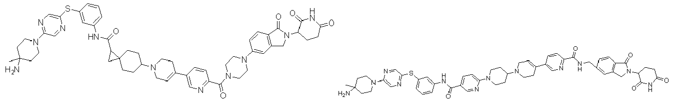

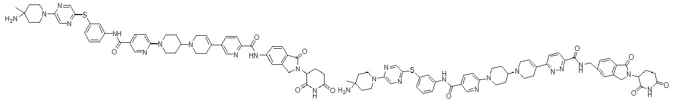

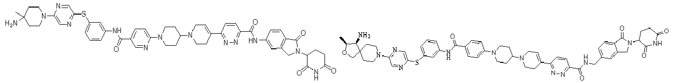

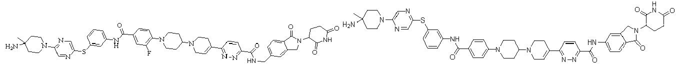

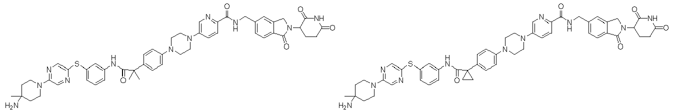

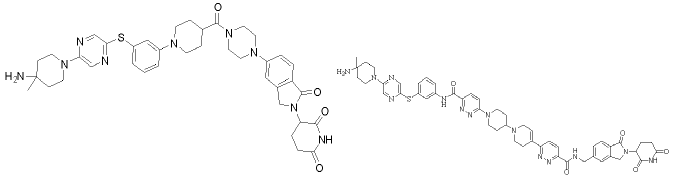

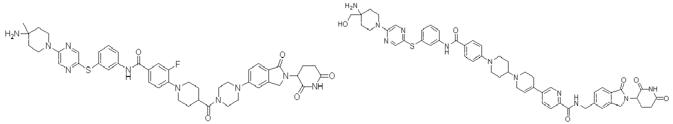

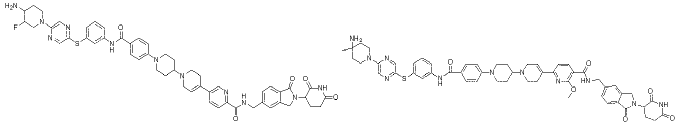

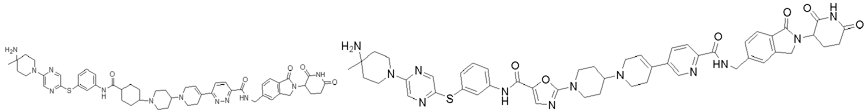

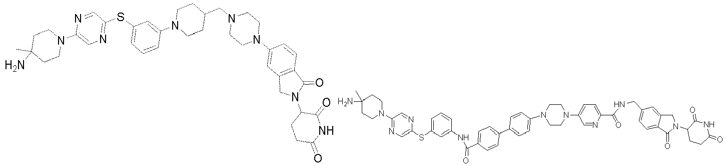

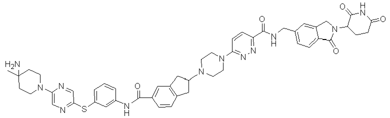

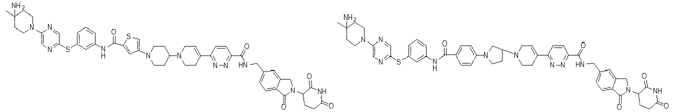

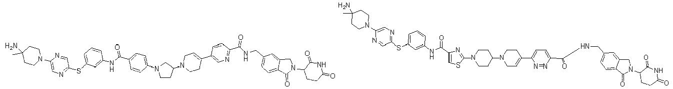

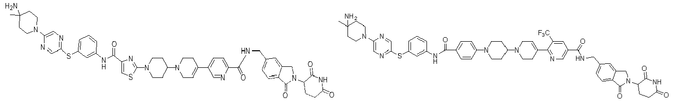

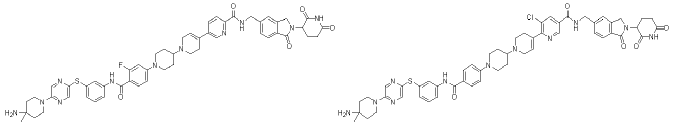

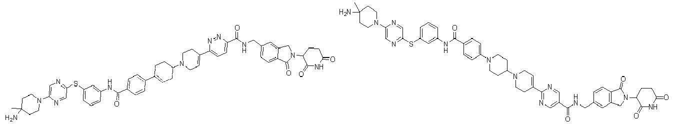

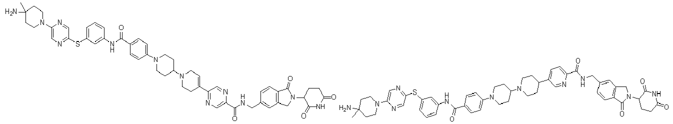

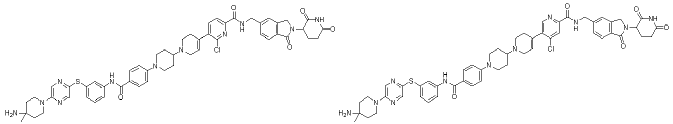

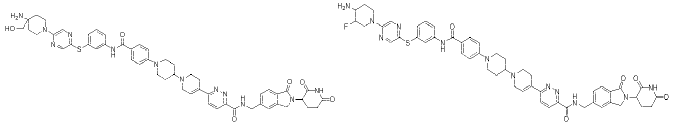

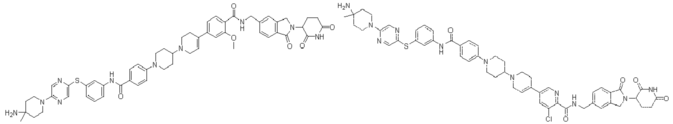

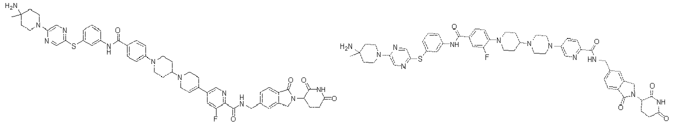

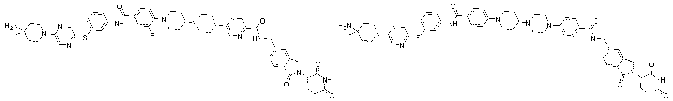

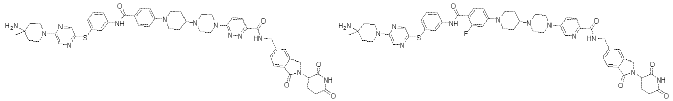

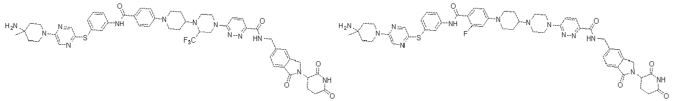

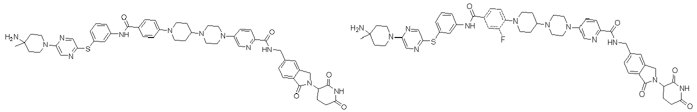

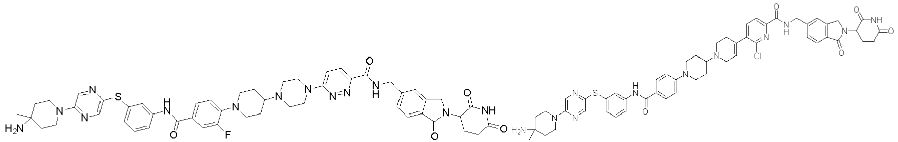

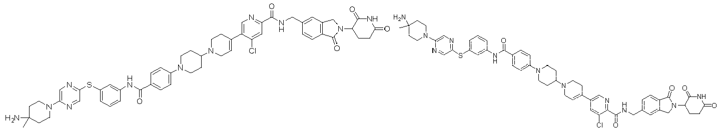

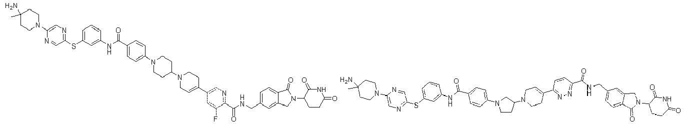

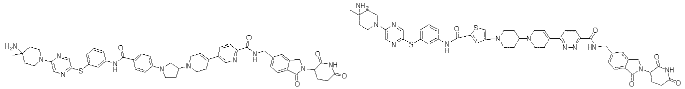

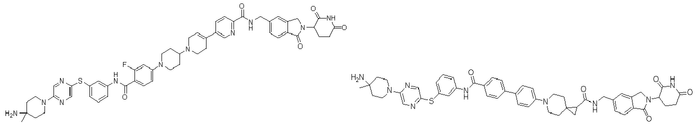

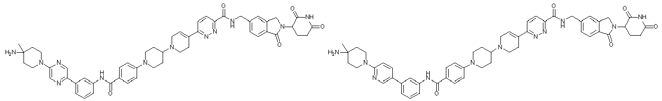

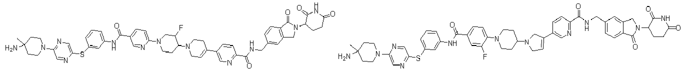

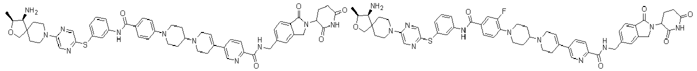

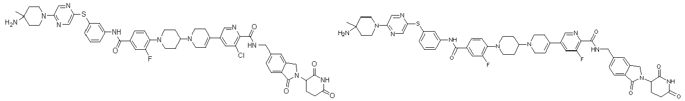

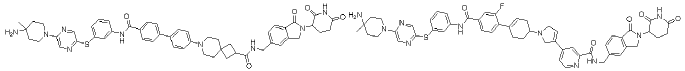

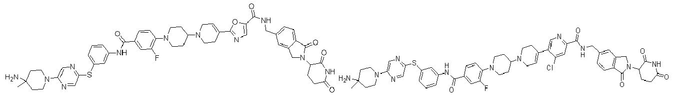

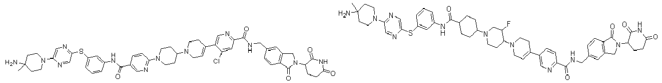

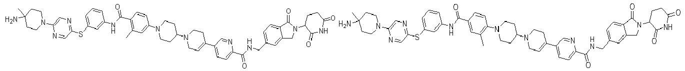

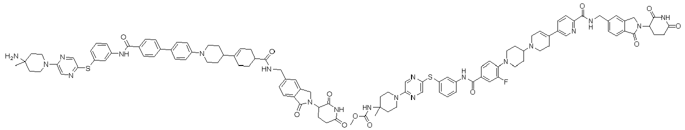

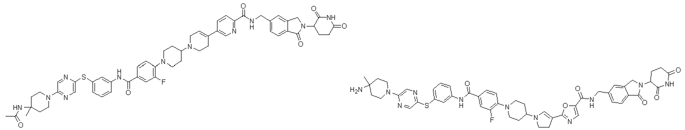

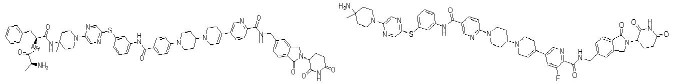

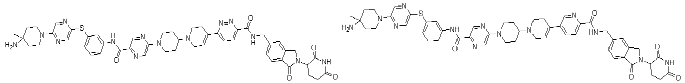

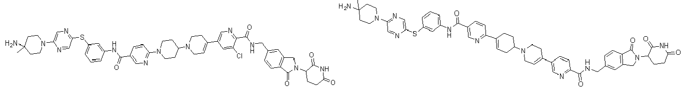

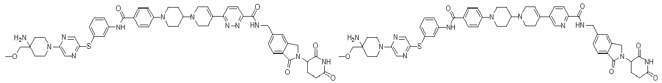

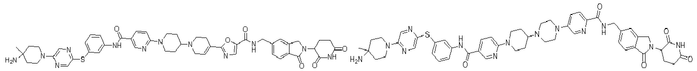

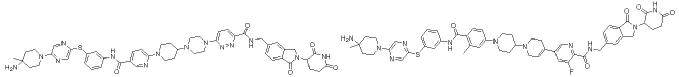

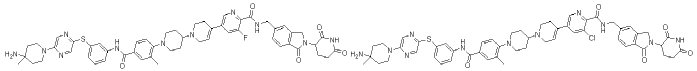

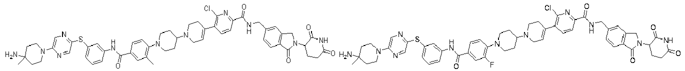

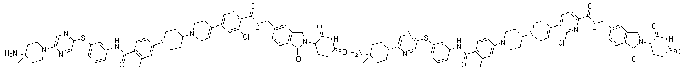

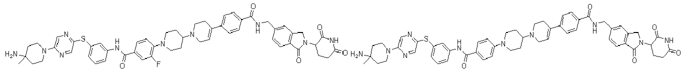

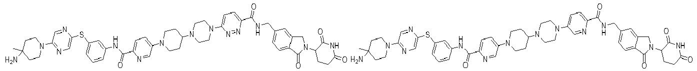

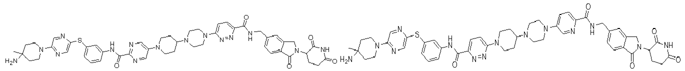

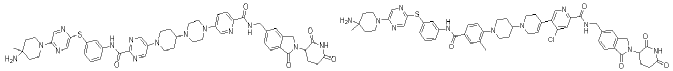

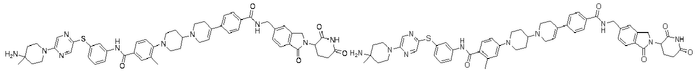

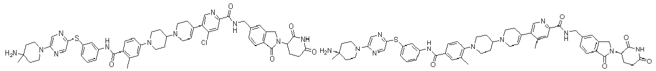

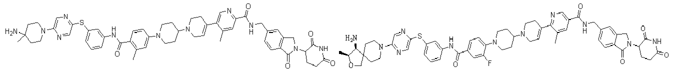

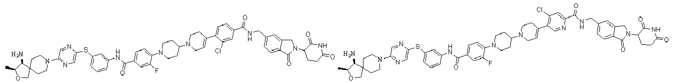

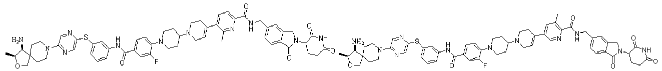

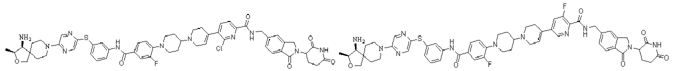

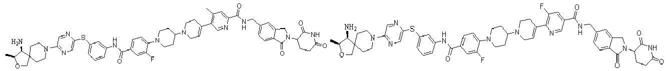

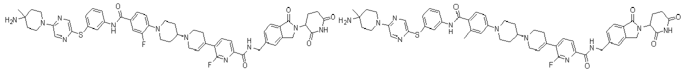

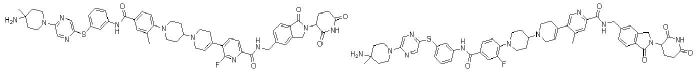

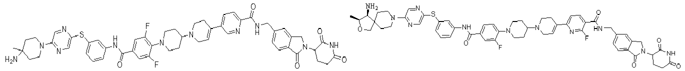

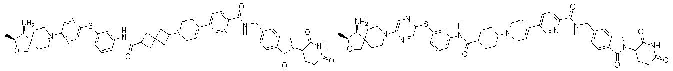

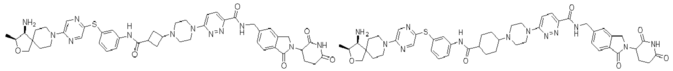

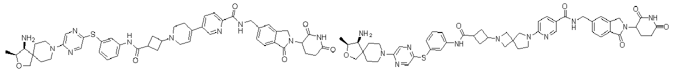

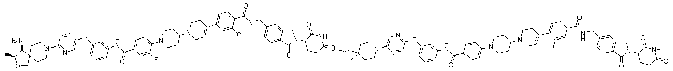

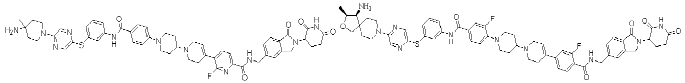

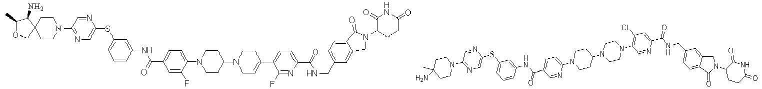

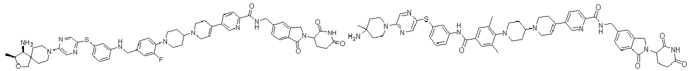

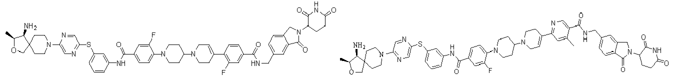

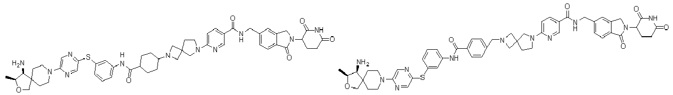

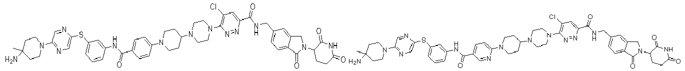

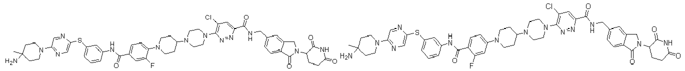

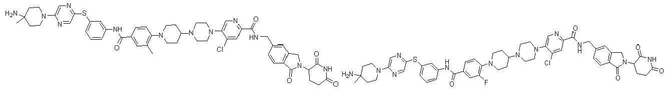

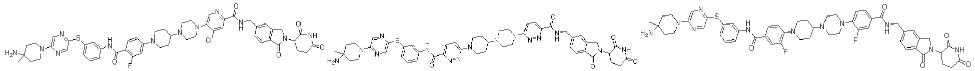

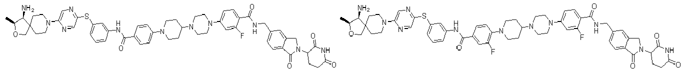

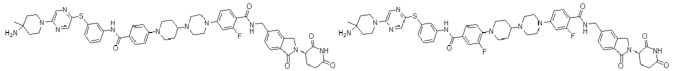

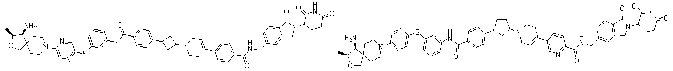

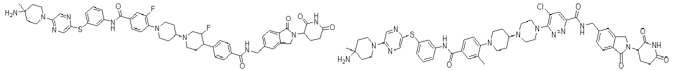

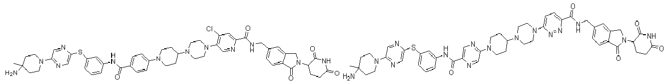

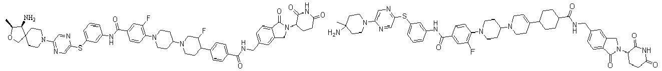

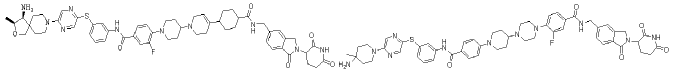

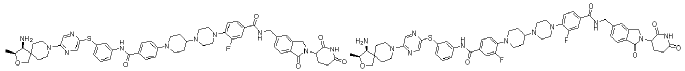

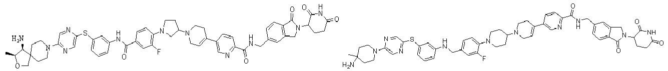

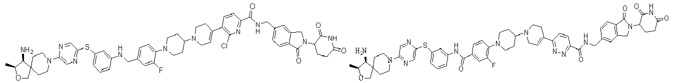

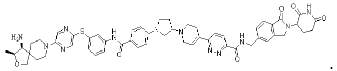

The present invention further provides the use of the compound mentioned above, or a salt thereof, or a deuterated compound thereof, or a stereoisomer thereof, or a solvate thereof, or a hydrate thereof, or a prodrug thereof in the manufacturer of phosphatase degraders.

The present invention further provides the use of the compound mentioned above, or a salt thereof, or a deuterated compound thereof, or a stereoisomer thereof, or a solvate thereof, or a hydrate thereof, or a prodrug thereof in the manufacturer of SHP2 protein degraders.

The present invention further provides the use of the compound mentioned above, or a salt thereof, or a deuterated compound thereof, or a stereoisomer thereof, or a solvate thereof, or a hydrate thereof, or a prodrug thereof in the manufacturer of medicaments for treatment of cancer, Noonan syndrome, Leopard syndrome, juvenile myelomonocytic leukemia, and myelodysplastic syndrome.

Further, the medicament is used to treat lung cancer, colon cancer, rectal cancer, melanoma, neuroblastoma, pancreatic cancer, liver cancer, esophageal cancer, prostate cancer, breast cancer, bile duct cancer, hematoma, and acute leukemia.

The present invention further provides a medicament, which is a preparation formed by the compound mentioned above, or a salt thereof, or a deuterated compound thereof, or a stereoisomer thereof, or a solvate thereof, or a hydrate thereof, or a prodrug thereof as active ingredient, in combination with pharmaceutically acceptable excipients or adjuvant ingredients.

The present invention further provides a drug combination, which comprises the compound mentioned above, or a salt thereof, or a deuterated compound thereof, or a stereoisomer thereof, or a solvate thereof, or a hydrate thereof, or a prodrug thereof and other anti-tumor drugs at the same or different specifications, which are administered simultaneously or separately, in combination with pharmaceutically acceptable carriers.

The compounds and derivatives provided in the present invention can be named according to IUPAC (International Union of Pure and Applied Chemistry) or CAS (Chemical Abstracting Service, Columbus, OH) naming system.

For the definition of terms used in the present invention: unless defined otherwise, the initial definition provided for the group or term herein applies to the group or term of the whole specification; for the terms that are not specifically defined herein, they should have the same meaning as commonly understood by one of ordinary skill in the art to which the disclosure belongs.

"Substitution" means that the hydrogen in a molecule is substituted with other different atoms or molecules.

"Alkyl" refers to an aliphatic hydrocarbon group, that is, a saturated hydrocarbon group. The alkyl moiety can be either a straight alkyl or a branched alkyl. Typical alkyls include but are not limited to methyl, ethyl, propyl, isopropyl, butyl, isobutyl, tert-butyl, pentanyl, hexanyl, and so on.

The C₁-Cₙ used in the present invention includes C₁-C₂, C₁-C₃...C₁-Cₙ, where n is an integer of >1; as a prefix for a substituent, n represents the minimum and maximum number of carbons in the substituent. For example, "C₁-C₈ alkyl" refers to a straight or branched alkyl containing 1-8 carbons.

The "ring" in the present invention can be a single ring or a multi-ring, and can also be a fused ring, a spiral ring, or a bridged ring.

"Cycloalkyl" refers to a saturated or unsaturated cyclic hydrocarbon substituent, for example, "3-10 membered cycloalkyl" denotes a cyclic alkyl comprising 3-6 carbons; "cycloalkyl" includes but is not limited to 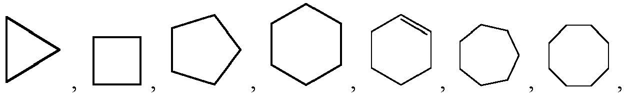 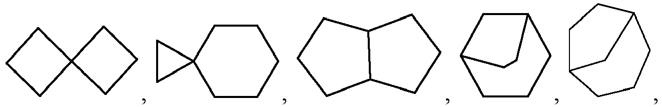 and the same.

"Heterocyclic group" refers to a cycloalkyl containing at least one heteroatom in the ring skeleton. Heteroatoms include but are not limited to O, S, N, P, Si, etc. "Heterocyclic groups" include but are not limited to 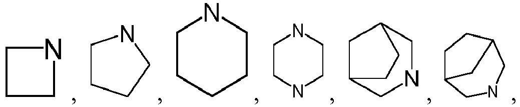 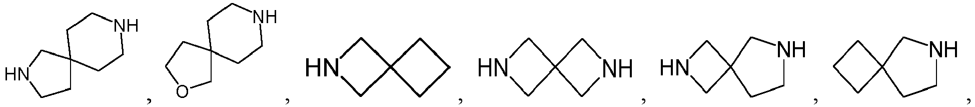 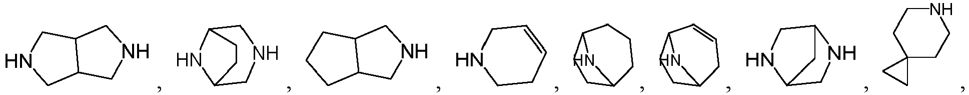 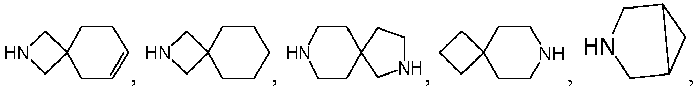 and the same.

"Aryl" refers to a planar ring which has a delocalized π-electron system and contains 4n+2 π electrons, where n is an integer. Aryl ring can be composed of five, six, seven, eight, nine or more atoms. Aryl includes but is not limited to phenyl, naphthyl, phenanthryl, anthracyl, fluorenyl, and indenyl. The aryl of the present invention also includes but is not limited to 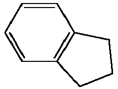 and the same.

"Heteroaryl" refers to an aryl in which a carbon is substituted with an atom other than carbon, such as N, O, S, etc. "Heteroaryl" includes but is not limited to pyrimidinyl, pyridazinyl, pyrazolyl, pyridyl, pyrazinyl, pyrazolyl, thienyl, furyl, 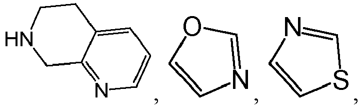 and the same.

"Halogen" or "halo" refers to fluorine, chlorine, bromine, or iodine.

In the present invention, *cis* refers to the compound being a *cis* isomer, while *trans* refers to the compound being a *trans* isomer.

The compound of the present invention has a good inhibitory effect on both hematomas and solid tumor cell lines. It has strong inhibitory effects on the proliferation of acute leukemia, esophageal cancer, KRAS mutant non-small cell lung cancer and pancreatic cancer cell lines. Moreover, when it is combined with other anti-tumor medicaments, a significant synergistic effect is found. In addition, the compound of the present invention has a rather different mechanism of action compared to traditional small-molecule targeting drugs or macromolecular drugs such as antibodies, and has good application prospects. The compound of the present invention can be used as a phosphatase degrader, especially as a SHP2 protein degrader, so that it can be used in the manufacturer of medicaments for treating diseases such as cancer, and has good application prospects.

The compound of the present invention can be used as a phosphatase degrader, especially as a SHP2 protein degrader, so that it can be used in the manufacturer of medicaments for treating cancer, Noonan syndrome, Leopard syndrome, juvenile myelomonocytic leukemia, and myelodysplastic syndrome, and has good application prospects.

Obviously, based on the above content of the present invention, according to the common technical knowledge and the conventional means in the field, other various modifications, alternations, or changes can further be made, without department from the above basic technical spirits.

With reference to the following specific examples, the above content of the present invention is further illustrated. But it should not be construed that the scope of the above subject matter of the present invention is limited to the following examples. The techniques realized based on the above content of the present invention are all within the scope of the present invention.

### Examples

The starting materials and equipment used in the specific examples of the present invention are all known products obtained by purchasing those commercially available.

### Synthesis of general intermediates

### Synthesis of tert-butyl (1-(5-((3-aminophenyl)thio)pyrazin-2-yl)-4-methylpyridin-4-yl)carbamate (HWH-1)

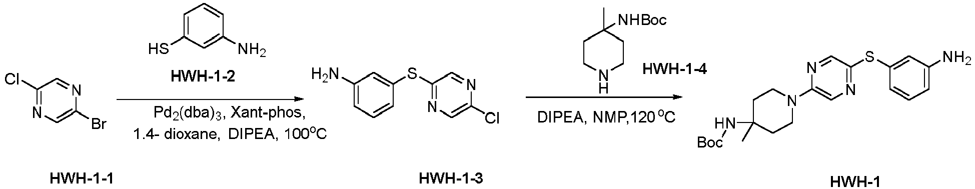

### Step 1: Synthesis of compound 3-((5-chloropyrazin-2-yl)thio)aniline (HWH-1-3)

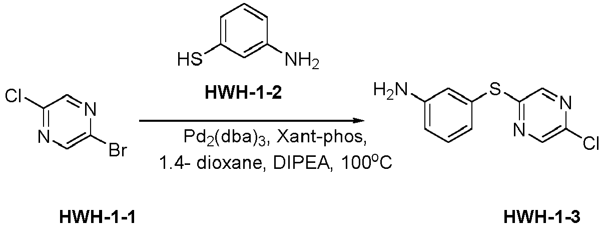

Under nitrogen protection, to a solution of **HWH-1-1** (10.0 g, 52.11 mmol, 1.0 eq) and **HWH-1-2** (6.5 g, 52.11 mmol, 1.0 eq) in dioxane (100 mL), were added diisopropylethylamine (13.3 g, 104.22 mmol, 2.0 eq), Xantphos (3.0 g, 5.21 mmol, 0.1 eq.), and Pd₂(dba)₃ (2.4 g, 2.60 mmol, 0.05 eq.), and then the resultant mixture was allowed to react at 100 °C. After completion of the reaction, the solvent was removed by evaporation, and the residue was purified by silica gel column chromatography, to provide the intermediate **HWH-1-3** (10.7 g, 45.15 mmol). MS (M+1): *m*/*z* 237.9.

### Step 2: Synthesis of tert-butyl (1-(5-((3-aminophenyl)thio)pyrazin-2-yl)-4-methylpyridin-4-yl)carbamate (HWH-1)

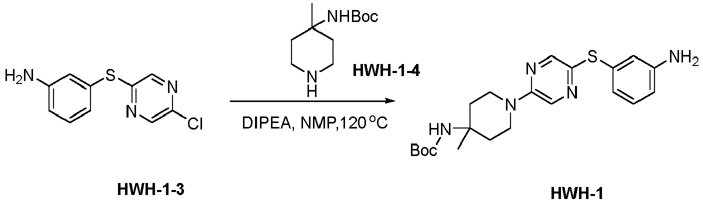

To a solution of **HWH-1-3** (10.7 g, 45.15 mmol, 1.0 eq.) and **HWH-1-4** (12.6 g, 58.88 mmol, 1.3 eq.) in NMP (15 mL), was added diisopropylethylamine (60 mL, 344.65 mmol, 7.6 eq). The mixture was allowed to react at about 120 °C for about 10 h. After completion of the reaction, the mixture was poured into water (400 mL) and then extracted with EA (2 * 100 mL). The organic layer was washed with saturated brine (2*400 mL) and then dried with anhydrous Na₂SO₄. After rotatory evaporation, the residue was purified by silica gel column chromatography to obtain **HWH-1** (17.7 g, yield 94.5%). MS(M+1): *m*/*z* 416.1.

### Synthesis of intermediate tert-butyl ((3S,4S)-8-(5-((3-aminophenyl)thio)pyrazin-2-yl)-3-methyl-2-oxo-8-azaspiro[4.5]decan-4-yl)carbamate (HWH-2)

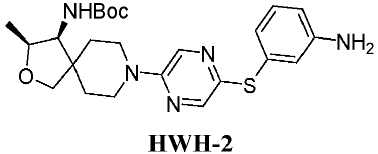

Using a similar method to the synthesis of **HWH-1**, the intermediate **HWH-2** can be prepared by replacing **HWH-1-4** used in the synthesis of **HWH-1** with tert-butyl ((3S,4S)-3-methyl-2-oxa-8-azaspiro[4.5]decan-4-yl)carbamate.

### Synthesis of intermediate 3-(5-(aminomethyl)-1-oxoisoindolin-2-yl)piperidin-2,6-dione hydrochloride (TC)

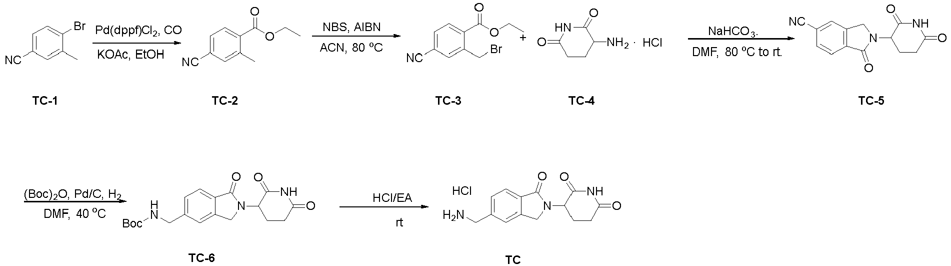

### Step 1: Synthesis of ethyl 4-cyano-2-methylbenzoate (TC-2)

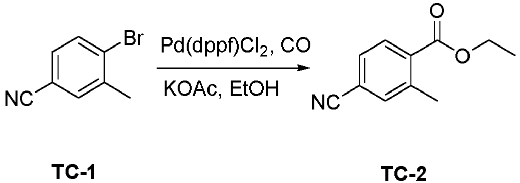

Under nitrogen protection, to a solution of compound **TC-1** (1.0 g, 5.13 mmol, 1.0 eq) and KOAc (1.5 g, 15.39 mmol, 3.0 eq) in ethanol (10 mL), was added Pd(dppf)Cl₂ (373 mg, 0.51 mmol, 0.1 eq.), and the reaction mixture was allowed to react at about 70°C under CO atmosphere. After completion of the reaction, the reaction solution was diluted by adding EA (20 mL), and then filtered to remove insoluble solids. The filtrate was concentrated, and the residue was purified by silica gel column chromatography, to obtain the target product **TC-2** (0.9 g, 4.76 mmol, yield 92.3%).

### Step 2: Synthesis of ethyl 2-(bromomethyl)-4-cyanobenzoate (TC-3)

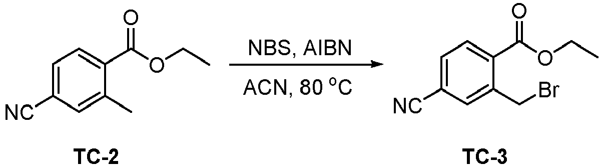

To a solution of **TC-2** ( 8.5 g, 50.5 mmoL, 1.0 eq) and NBS (17.8 g, 100.0 mmoL, 2.0 eq) in acetonitrile (100 mL), was added AIBN (820 mg, 5.0 mmol, 0.1 eq), and then the mixture was allowed to react at about 80°C under nitrogen protection. After completion of the reaction, the solvent was removed. The residue was diluted with EA (100 mL), and the ethyl acetate layer was washed with saturated NaHSO₃ solution (100 mL) and brine (100 mL), then dried with anhydrous Na₂SO₄. After rotatory evaporation of the solvent, the residue was purified by silica gel column chromatography to obtain **TC-3** (9.0 g, 33.71 mmol, yield 75.8%).

### Step 3: Synthesis of 2-(2,6-dioxopiperidin-3-yl)-1-oxoisoindolin-5-nitrile (TC-5)

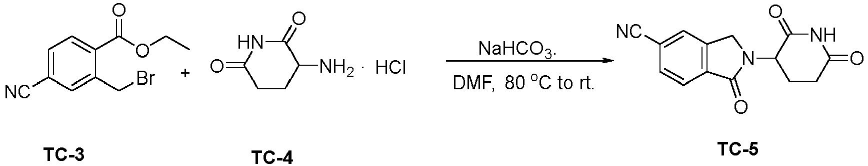

To a solution of **TC-3** (35.7 g, 133.15 mmol, 1.0 eq.) and **TC-4** (43.8 g, 266.30 mmol, 2.0 eq.) in DMF (400 mL), was added NaHCO₃ (22.4 g, 266.30 mmol, 2.0 eq.), and then the mixture reacted at about 80 °C for 3 h, followed by reaction at room temperature. After the reaction was completed, water (2 L) was added. After precipitation and filtration, **TC-5** (25.1 g, 93.24 mmol, yield 70.0%) was obtained by beating with ethanol. MS (M+H⁺): *m*/*z* 270.1.

### Step 4: Synthesis of tert-butyl ((2-(2,6-dioxopiperidin-3-yl)-1-oxoisoindolin-5-yl)methyl)carbamate (TC-6)

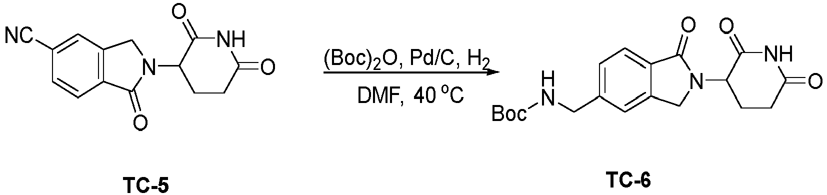

To a solution of compound **TC-5** (19.0 g, 70.63 mmol, 1.0 eq.) and (Boc)₂O (22.8 g, 218.25 mmol, 1.5 eq.) in DMF (2 L), was added wet Pd/C (4.0 g, 10%, c.a.55% water), and then the mixture reacted at 40 °C under hydrogen atmosphere. After completion of the reaction, the catalyst was removed by filtering, and the filtrate was concentrated. The residue was triturated with methanol to obtain the target product **TC-6** (15.1 g, 40.48 mmol, yield 57.3%). MS (M+H⁺): *m*/*z* 374.1.

### Step 5: Synthesis of 3-(5-(aminomethyl)-1-oxoisoindolin-2-yl)piperidin-2,6-dione hydrochloride (TC)

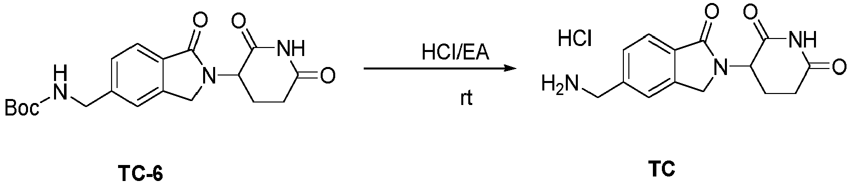

A solution of compound **TC-6** (3.7 g, 10.0 mmol, 1.0 eq) in HCl/EA (3 M, 35 mL, 10.5 eq.) was stirred at room temperature. After completion of the reaction, the solvent was removed to obtain the target product **TC** (3.1 g, 10.0 mmol, yiled 100% for crude product). MS (M+H⁺): *m*/*z* 274.2.

### Synthesis of intermediate (2S,4R)-1-((S)-2-amino-3,3-dimethylbutyryl)-4-hydroxyl-N-((S)-1-(4-(4-methylthiazol-5-yl)phenyl)ethyl)pyrrolin-2-formamide trifluoroacetate (TV)

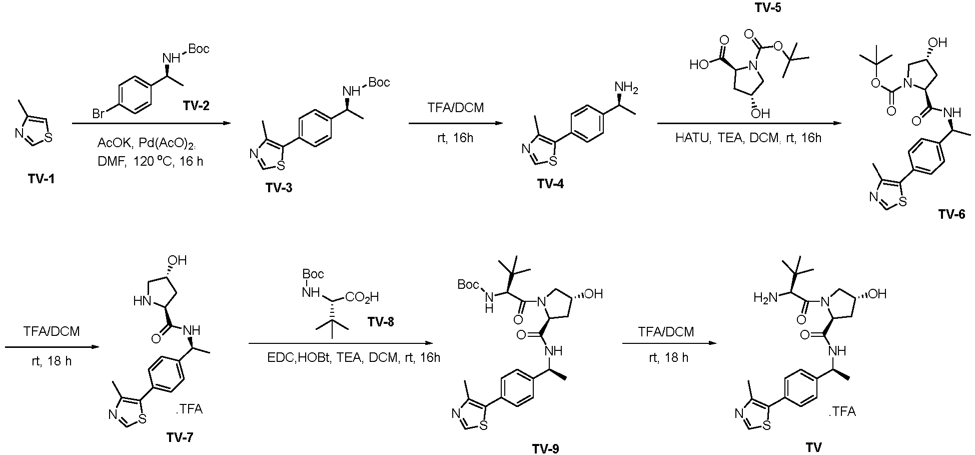

### Step 1: Synthesis of intermediate tert-butyl (S)-(1-(4-(4-methylthiazol-5-yl)phenyl)ethyl)formamide (TV-3)

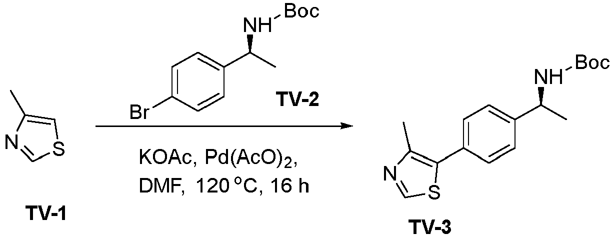

To a solution of **TV-2** (10.0 g, 33.31 mmol, 1.0 eq.) in DMF (150 mL), were added **TV-1** (6.61 g, 66.62 mmol, 2.0 eq.), KOAc (6.53 g, 66.62 mmol, 2.0 eq.), and Pd(OAc)₂ (305.05 mg, 0.33 mmol, 0.01 eq.), and then the reaction was allowed to react at about 120 °C under nitrogen protection. After completion of the reaction, the reaction solution was concentrated, and the residue was purified by silica gel column chromatography, to obtain the target product **TV-3** (10 g, 31.40 mmol, yield 94.3%). LCMS [M+H]: *m*/*z* 319.1.

### Step 2: Synthesis of (S)-1-(4-(4-methylthiazol-5-yl)phenyl)ethyl-1-amine (TV-4)

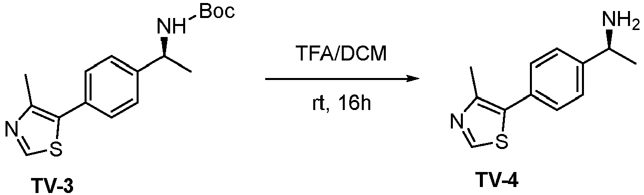

Compound **TV-3** (10.0 g, 31.40 mmol, 1.0 eq.) and TFA (20.0 mL, 261.36 mmol, 8.3 eq.) were stirred in DCM (20 mL), until the reaction was completed. After concentration, trifluoroacetate of **TV-4** (10.7 g, 31.40 mmol, crude product, ca. 100%) was obtained, which was directly used in the next step. LCMS [M+H]⁺: *m*/*z* 219.1.

### Step 3: Synthesis of tert-butyl (2S,4R)-4-hydroxyl-2-(((S)-1-(4-(4-methylthiazol-5-yl)phenyl)ethyl)formyl)pyrrolin-1-carboxylate (TV-6)

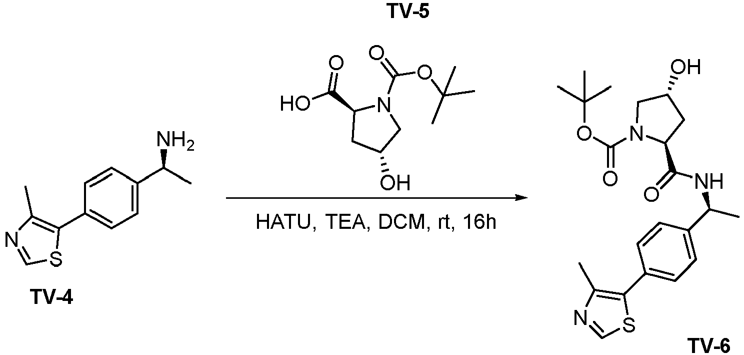

At room temperature, to a solution of **TV-4** (10.0 g, 30.09 mmol, 1.0 eq.) and **TV-5** (6.96 g, 30.09 mmol, 1.0 eq.) in DCM (500 mL), were added triethylamine (16.02 mL, 90.27 mmol, 3.0 eq.) and HATU (17.15 g, 45.13 mmol, 1.5 eq.). The mixture was stirred at room temperature, until the reaction was completed. After concentration, the residue was purified by silica gel column chromatography, to obtain the target compound TV-6 (11.0 g, 25.49 mmol, yield 84.7%). LCMS [M+H]: *m*/*z* 432.4.

### Step 4: Synthesis of (2S,4R)-4-hydroxyl-N-((S)-1-(4-(4-methylthiazol-5-yl)phenyl)ethyl)pyrrolin-2-formamide trifluoroacetate (TV-7)

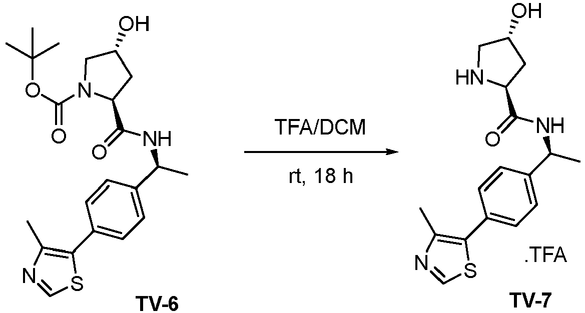

Compound **TV-6** (1.0 g, 2.32 mmol, 1.0 eq.) and TFA (5.0 mL, 65.34 mmol, 28.1 eq.) were stirred in DCM (10 mL) at room temperature, until the reaction was completed. After the reaction solution was concentrated to remove the solvent, **TV-7** (1.03 g, 2.32 mmol, crude product, ca.100%) was obtained, which was directly used in the next step. LCMS [M+H]: *m*/*z* 332.2.

### Step 5: Synthesis of tert-butyl ((S)-1-((2S,4R)-4-hydroxyl-2-(((S)-1-(4-(4-methylthiazol-5-yl)phenyl)ethyl)formyl)pyrrolin-1-yl)-3,3-dimethyl-1-oxobutanone-2-yl)formamide (TV-9)

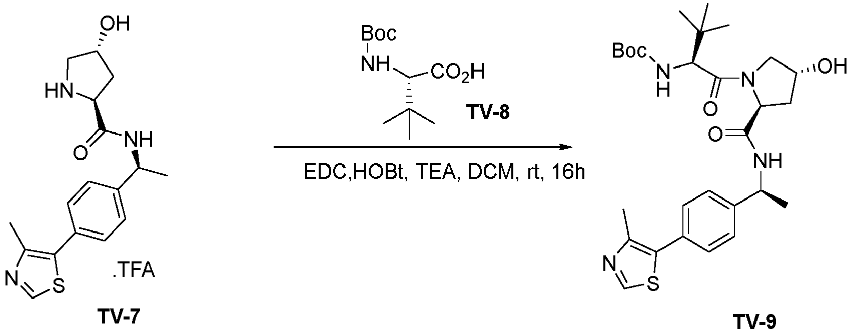

To a solution of compounds **TV-7** (2.0 g, 4.04 mmol, 1.0 eq.) and TV-8 (934.6 mg, 4.04 mmol, 1.0 eq.) in DCM (40 mL), were added TEA (3.59 mL, 20.2 mmol, 5.0 eq.), HOBt (659.6 mg, 4.85 mmol, 1.2 eq.), and EDC (931.2 mg, 4.85 mmol, 1.2 eq.). The mixture was reacted until completion of the reaction. The mixture was washed with brine (2*20 mL), and then the mixed solution was separated. The organic layer was dried over Na₂SO₄, followed by rotatory evaporation. The residue was chromatographed over silica gel column, to obtain TV-9 (1.7 g, 3.12 mmol, yield 77.2%). LCMS [M+H]: *m*/*z* 545.5.

### Step 6. Synthesis of (2S,4R)-1-((S)-2-amino-3,3-dimethylbutyryl)-4-hydroxyl-N-((S)-1-(4-(4-methylthiazol-5-yl)phenyl)ethyl)pyrrolin-2-formamide trifluoroacetate (TV)

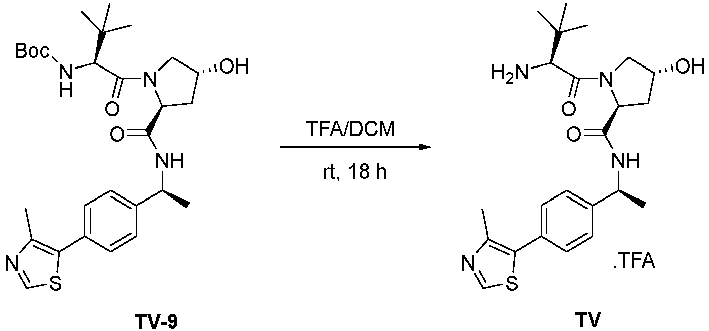

Compound **TV-9** (1.7 g, 3.12 mmol, 1.0 eq.) and TFA (5.0 mL, 65.34 mmol, 20.9 eq.) were stirred in DCM (10 mL) at room temperature, until the reaction was completed. After the reaction solution was concentrated to remove the solvent, **TV** (1.74 g, 3.12 mmol, crude product, ca. 100%) was obtained, which was directly used in the next step. LCMS [M+H]: *m*/*z* 445.2.

### Example 1 Synthesis of compound N-(3-((5-(4-amino-4-methylpyridin-1-yl)pyrazin-2-yl)thio)phenyl)-8-(6-(4-((((2-(2,6-dioxopiperidin-3-yl)-1-oxoisoindol-4-yl)oxy)methyl)benzyl)-2,6-diazaspiro[3.4]octan-2-yl)octylamide (68):

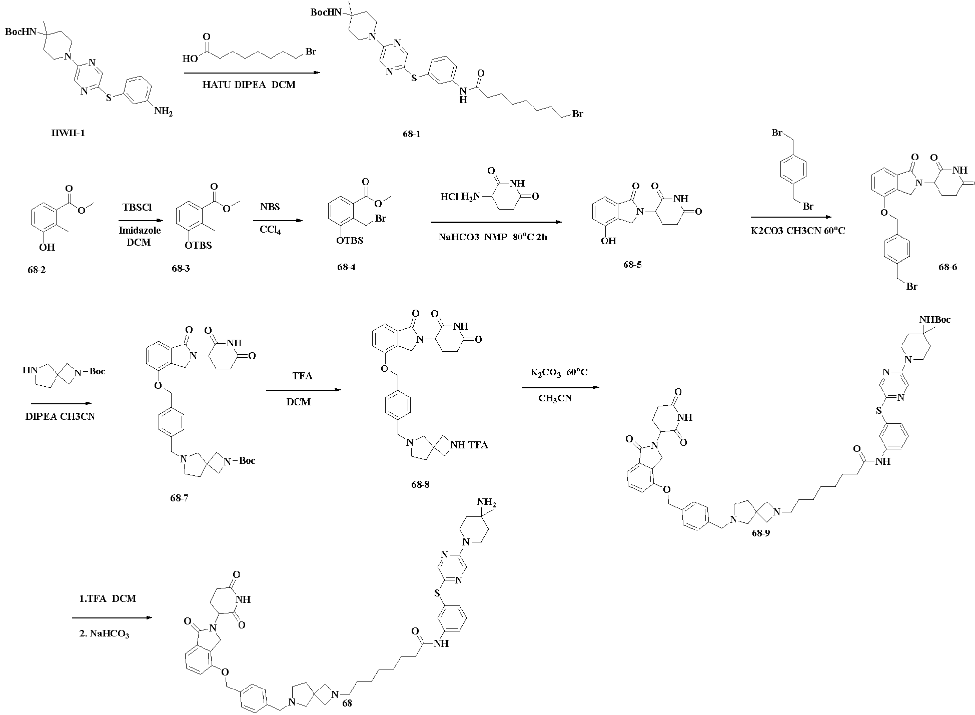

### Synthesis of intermediate tert-butyl (1-(5-((3-(8-bromooctylamino)phenyl)thio)pyrazin-2-yl)-4-methylpyridin-4-yl)carbamate (68-1):

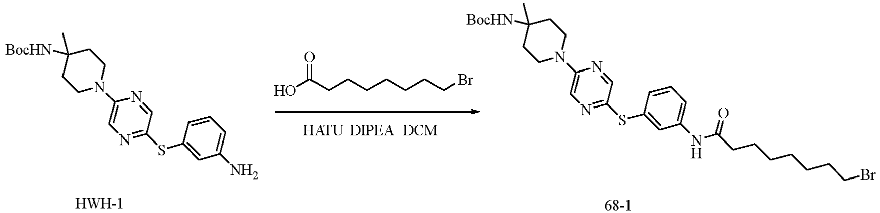

Intermediate tert-butyl (1-(5-((3-aminophenyl)thio)pyrazin-2-yl)4-methylpyridin-4-yl)carbamate (200 mg, 0.48 mmol), 8-bromooctanoic acid (108 mg, 0.48 mmol), 2-(7-azobenzotriazole)-N,N,N',N'-tetramethylurea hexafluorophosphate (274 mg, 0.72 mmol) and *N*,*N*-diisopropylethylamine (124 mg, 0.96 mmol) were dissolved in dichloromethane (5 mL); and then the mixture was reacted under stirring at room temperature for 2 h. The reaction solution was washed once with water, saturated NaHCOs aqueous solution, and saturated brine, respectively, and then the reaction solution was separated. The organic layer was dried over anhydrous Na₂SO₄, and then concentrated, to obtain 215 mg of product (**68-1**), with a yield of 71.4%. MS: *m*/*z* 620 (M+H⁺); 622 (M+2+H⁺).

### Synthesis of intermediate methyl 3-((tert-butyldimethylsilyl)oxy)-2-methylbenzoate (68-3):

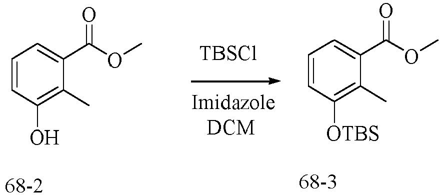

**68-2** (2.9 g, 17.5 mmol) and imidazole (2.4 g, 35 mmol) were added to dichloromethane (40 mL), to which was then added tert-butyldimethylsilyl chloride (3.1 g, 20 mmol) dropwise in an ice bath. After addition, the mixture was stirred at room temperature for 1 h. After the reaction was completed, the reaction solution was directly poured into water. The organic phase was washed with water, dried with anhydrous Na₂SO₄, and rotatory evaporated to obtain the intermediate **68-3** (5.2 g, yield: 100%). MS: *m*/*z* 281 [M+H]⁺.

### Synthesis of intermediate methyl 2-(bromomethyl)-3-((tert-butyl dimethylsilyl)oxy)benzoate (68-4):

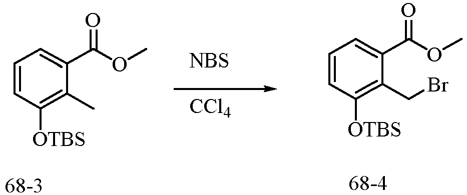

Compound **68-3** (5.2 g, 17.5 mmol) was dissolved in tetrachloromethane, to which was added N-bromosuccinimide (3.3 g, 18.4 mmol), and then the mixture was reacted at 80 °C for 2h. After completion of the reaction, the intermediate **68-4** (5.62 g, yield 89.5%) was obtained by purification via column chromatography. MS: *m*/*z* 359 [M+H]⁺, 361 [M+2+H]⁺.

### Synthesis of intermediate 3-(4-hydroxyl-1-oxoisoindol-2-yl)piperidin-2,6-dione (68-5):

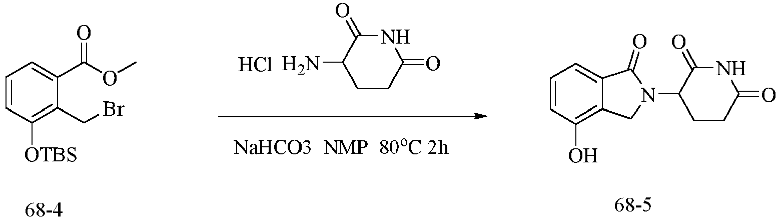

**68-4** (3.6 g, 10 mmol), 3-aminopiperidin-2,6-dionehydrochloride (1.65 g, 10 mmol), and NaHCO₃ (1.68 g, 20 mmol) were added into NMP (20 mL), and then the mixture was stirred at 80 °C for 2h. After that, the reaction solution was stirred overnight at room temperature. Once completion of the reaction, the reaction solution was concentrated, and the residue was purified by column chromatography, to obtain the intermediate **68-5** (1.7 g, yield 65.3%). MS: *m*/*z* 261 [M+H]⁺.

### Synthesis of intermediate 3-(4-((4-(bromomethyl)benzyl)oxy)-1-oxoisoindol-2-yl)piperidin-2,6-dione (68-6)

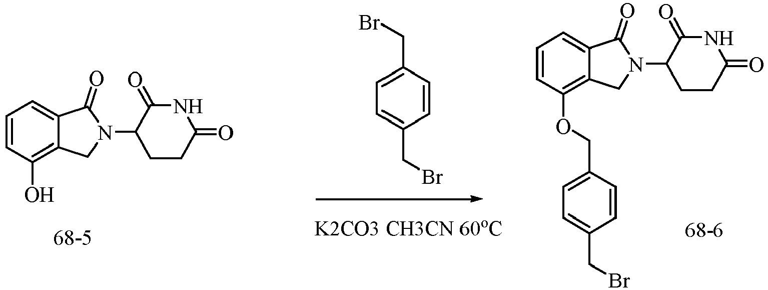

**68-5** (1.7 g, 6.5 mmol), 1,4-bis(bromomethyl)benzene (1.72 g, 6.5 mmol), and K₂CO₃ (1.79 g, 13 mmol) were added into acetonitrile (50 mL), and then the mixture was stirred at 60°C for 2h. After completion of the reaction, the reaction solution was concentrated, and the residue was purified by column chromatography, to obtain the intermediate **68-6** (1.1 g, yiled 38.3%). MS: *m*/*z* 443 [M+H]⁺, 445 [M+2+H]⁺.

### Synthesis of intermediate tert-butyl 6-(4-((((2-(2,6-dioxopiperidin-3-yl)-1-oxoisoindol-4-yloxy)methyl)benzyl)-2,6-diazaspiro[3.4]octan-2-carboxylate (68-7)

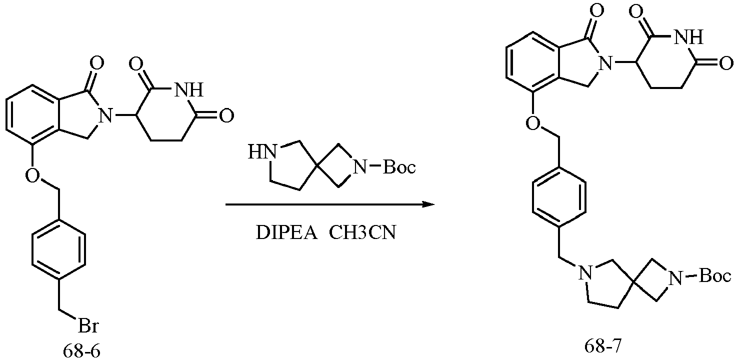

Intermediate **68-6** (200 mg, 0.45 mmol), tert-butyl 2,6-diazaspiro[3.4]octan-2-carboxylate (96 mg, 0.45 mmol), diisopropylethylamine (116 mg, 0.9 mmol) and acetonitrile (5 mL) were mixed, and then heated to 40 °C, and allowed to react for 1h. After completion of the reaction, the reaction was directly purified by column chromatography, to obtain the intermediate **68-7** (210 mg, yield 81.4%). MS: *m*/*z* 575 [M+H]⁺.

### Synthesis of intermediate 3-(4-((4-(((2,6-diazaspiro[3.4]octan-6-yl)methyl)benzyl]oxy)-1-oxoisoindol-2-yl)piperidin-2,6-dione trifluoroacetate (68-8)

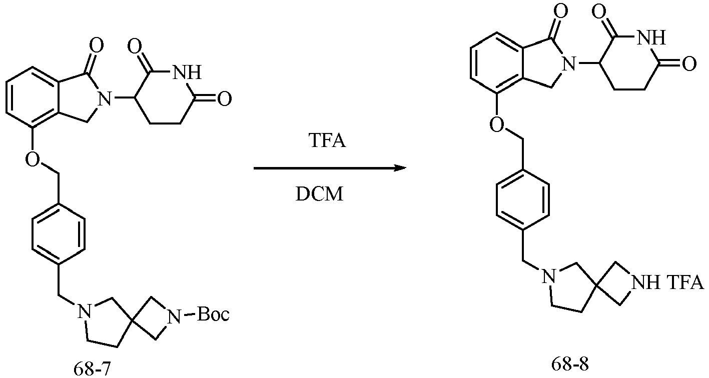

**68-7** (210 mg, 0.36 mmol) and trifluoroacetic acid (1 ml) were added in dichloromethane (2 ml), and then allowed to react for 0.5 h. After completion of the reaction, the solvent was removed by rotatory evaporation, and compound (**68-8**) (220 mg, yield 100%) was obtained. MS: **m/z** 475 [M+H]⁺.

### Synthesis of intermediate tert-butyl (1-(5-((3-(8-(6-(4-((((2-(2,6-dioxopiperidin-3-yl)-1-oxoisoindol-4-yl)oxy))benzyl)-2,6-diazaspiro[3.4]octan-2-yl)octylamido)phenyl) thiopyrazin-2-yl)-4-methylpyridin-4-yl)carbamate (68-9)

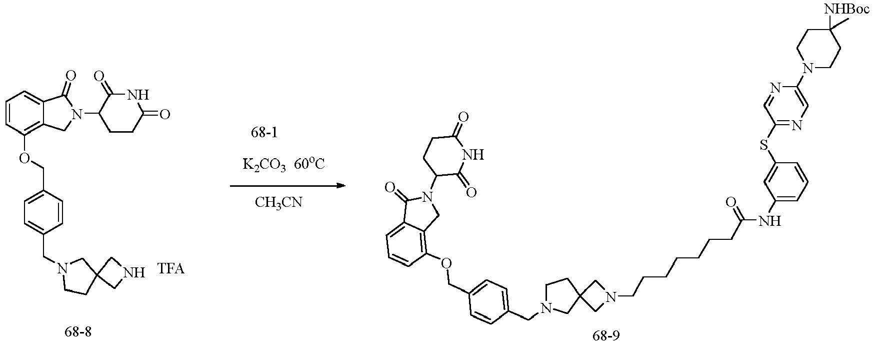

Intermediate **68-1** (100 mg, 0.17 mmol), intermediate **68-8** (109 mg, 0.17 mmol), K₂CO₃ (49 mg, 0.35 mmol) and acetonitrile (5 mL) were mixed, and then heated to 60 °C, and allowed to react for 2h. After completion of the reaction, the reaction solution was directly subjected to suction filtration, and washed with ethyl acetate (5 mL × 3). The organic layers were combined, dried, concentrated, and purified by column chromatography, to obtain the intermediate **68-9** (110 mg, yield 62.1%). MS: *m*/*z* 1014 [M+H]⁺.

### Synthesis of compound N-(3-((5-(4-amino-4-methylpyridin-1-yl)pyrazin-2-yl)thio)phenyl)-8-(6-(4-((((2-(2,6-dioxopiperidin-3-yl)-1-oxoisoindol-4-yl)oxy)methyl) benzyl)-2,6-diazaspiro[3.4]octan-2-yl)octylamide (68)

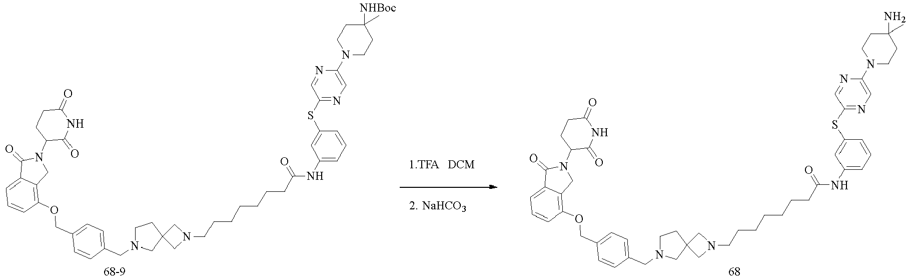

**68-9** (110 mg, 0.11 mmol) and trifluoroacetic acid (0.5 mL) were added into dichloromethane (1 mL), and allowed to react for 0.5 h. After completion of the reaction, the solvent was removed by rotatory evaporation, and then methanol (3 mL) was added. The resultant solution was adjusted to be pH ~7 with NaHCO₃ solid, filtered, concentrated, and then dissolved by adding dichloromethane (3 mL) and methanol (0.3 mL), followed by filtration and concentration, to obtain compound (**68**) (81 mg, yield 82%). MS: *m*/*z* 914 [M+H]⁺. ¹H NMR (400 MHz, DMSO-d₆) *δ* 9.91 (s, 2H), 9.80(m, 2H), 8.52-8.01 (m, 3H), 7.52-7.30 (m, 10H), 5.14 (s, 2H), 4.42-4.20 (m, 3H), 3.66 (s, 2H), 3.50-3.11 (m, 10H), 2.50-2.11 (m, 10H), 1.78-1.50 (m, 6H), 1.50-1.27 (m, 10H), 1.23 (s, 3H).

### Example 2 Synthesis of compound 1-(1-(9-((3-((5-(4-amino-4-methylpyridin-1-yl)pyrazin-2-yl)thio)phenyl)amino)-9-oxocarbonyl)piperidin-4-yl)-N-(((2-(2,6-dioxopiperidin-3-yl)-1-oxoisoindol-5-yl)methyl)-3-methyl-1H-pyrazol-5-formamide (80)

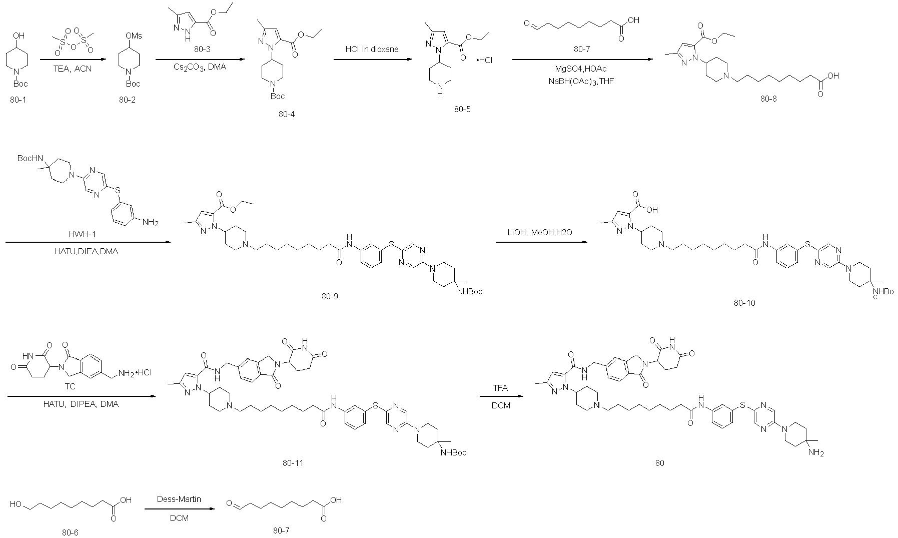

### Synthesis of intermediate tert-butyl 4-((methylsulfonyl)oxy)piperidin-1-carboxylate (80-2)

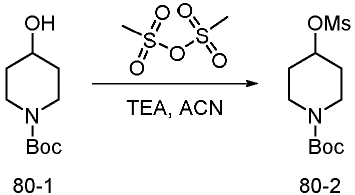

The starting material N-Boc-4-hydroxypiperidine (**80-1**) (2 g, 10 mmol) and trimethylamine (2 g, 20 mmol) were dissolved in 20 mL of acetonitrile, to which was added methanesulfonic anhydride (1.74 g, 10 mmol) dropwise in an ice water bath, and then the mixture was reacted at room temperature for 5 h. The reaction solution was poured into 20 mL of water, and extracted with 50 mL of ethyl acetate. The organic layer was washed once with 20 mL of saturated NaHCOs aqueous solution and 20 mL of saturated brine, respectively, dried with anhydrous sodium sulfate, and concentrated to obtain 2.79 g of crude product (**80-2**), with a yield of 99%. MS: *m*/*z* 280 [M+H]⁺.

### Synthesis of intermediate tert-butyl 4-(5-(ethoxycarbonyl)-3-methyl-1H-pyrazol-1-yl)piperidin-1-carboxylate (80-4)

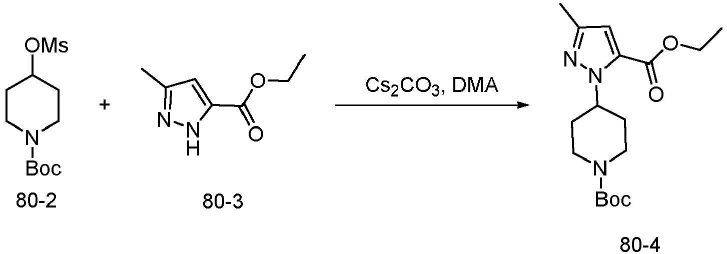

Intermediate **80-2** (2.79 g, 10 mmol) and the starting material ethyl 3-methyl-1H-pyrazol-5-carboxylate (**80-3**) (1.54 g, 10 mmol) were dissolved in 20 mL of N,N-dimethylacetamide, to which was added cesium carbonate (9.9 g, 30 mmol), and then the reaction system was heated to 90 °C and reacted for 15 h. The reaction solution was poured into 20 mL of water, extracted with 50 mL of ethyl acetate, dried over anhydrous sodium sulfate, concentrated, and subjected to column chromatography, to obtain 1.35 g of product (**80-4**) with a yield of 40%. MS: *m*/*z* 338 [M+H]⁺.

### Synthesis of intermediate ethyl 3-methyl-1-(piperidin-4-yl)-1H-pyrazol-5-carboxylate hydrochloride (80-5)

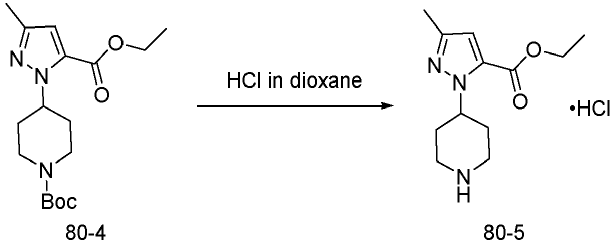

To a reaction flask, was added intermediate **80-4** (1.35 g, 4 mmol), followed by addition of 20 mL of HCl in dioxane (4 mol/L), and then the mixture was allowed to react at room temperature for 2 h. The reaction solution was concentrated to obtain 1.1 g of crude product (**80-5**), with a yield of 98%. MS: 238 [M+H]⁺.

### Synthesis of intermediate 9-oxynonanoic acid (80-7)

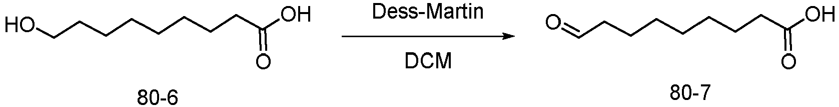

The starting material 9-hydroxylnonanoic acid (**80-6**) (1.74 g, 10 mmol) was dissolved in 35 mL of dichloromethane, to which was added Dess-Martin periodinane (4.7 g, 11 mmol), and the mixture was allowed to react for 1 h. The reaction solution was filtered over diatomaceous earth, and the filter cake was washed with 50 mL of dichloromethane. The filtrate was combined and concentrated, to obtain the crude product (**80-7**), which was directly used in the next step_{∘}

### Synthesis of intermediate 9-(4-(5-(ethoxycarbonyl)-3-methyl-1H-pyrazol-1-yl)piperidin-1-yl)nonanoic acid (80-8)

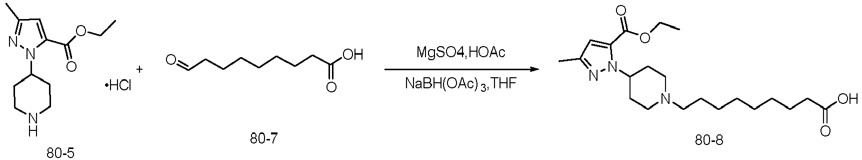

Intermediate **80-5** (474 mg, 2 mmol) and intermediate **80-7** (344 mg, 4 mmol) were dissolved in 10 mL of tetrahydrofuran, to which were added two drops of acetic acid and 1 g of MgSO₄, and then the mixture was stirred at room temperature for 1 h. Then, sodium triacetoxyborohydride (2.1 g, 10 mmol) was added, and then the mixture was allowed to react for 3 h. The reaction solution was filtered over diatomaceous earth. The filter cake was washed with 30 mL mixed solvent of dichloromethane/methanol (v/v = 10:1). The filtrate was combined, concentrated, and purified over reversed-phase column, to obtain 300 mg of product (**80-8**), with a yield of 40%. MS: *m*/*z* 394 [M+H]⁺.

### Synthesis of intermediate ethyl 1-(1-(9-((3-((5-(4-((tert-butoxycarbonyl)amino)-4-methylpyridin-1-yl)pyrazin-2-yl)thio)phenyl)amino)-9-oxocarbonyl)piperidin-4-yl)-3-methyl-1H-pyrazol-5-carboxylate (80-9)

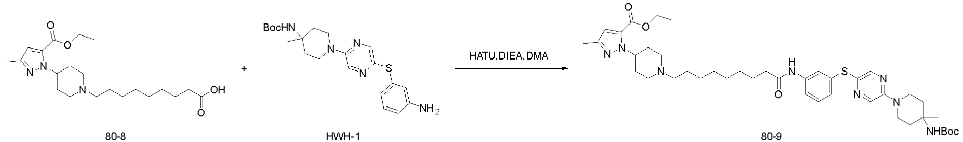

Intermediate **80-8** (393 mg, 1 mmol), tert-butyl (1-(5-((3-aminophenyl)thio)pyrazin-2-yl)-4-methylpyridin-4-yl)carbamate (**HWH-1**) (415 mg, 1 mmol), HATU (380 mg, 1 mmol), and DIEA (390 mg, 3 mmol) were dissolved in 5 mL ofN,N-dimethylacetamide, and the mixture was allowed to react at room temperature for 2 h. The reaction solution was poured into 10 mL of water, and extracted with 30 mL of ethyl acetate. The organic layer was dried with anhydrous sodium sulfate, concentrated, and purified by column chromatography to obtain 395 mg of product (80-9), with a yield of 50%. MS: *m*/*z* 791 [M+H]⁺.

### Synthesis of intermediate 1-(1-(9-((3-((5-(4-((tert-butoxycarbonyl)amino)-4-methylpyridin-1-yl)pyrazin-2-yl)thio)phenyl)amino)-9-oxocarbonyl)piperidin-4-yl)-3-methyl-1H-pyrazol-5-carboxylic acid (80-10)

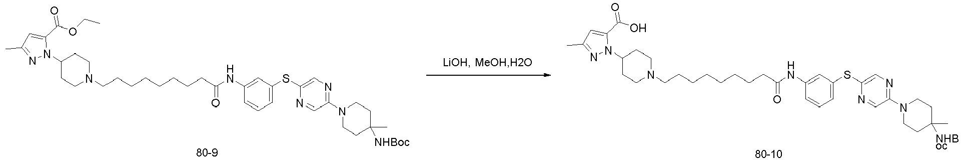

Intermediate **80-9** (395 mg, 0.5 mmol) was dissolved in a mixed solvent of 10 mL methanol and 2 mL water, to which was added lithium hydroxide monohydrate (210 mg, 5 mmol), and the mixture was reacted at room temperature for 2 h. The pH of the reaction solution was adjusted to 6 with 0.5 N of dilute hydrochloric acid, and then the solution was extracted with 30 mL of dichloromethane. The organic phase was dried with anhydrous sodium sulfate, and concentrated to obtain 350 mg of product (**80-10**), with a yield of 90%. MS: *m*/*z* 763 [M+H]⁺.

### Synthesis of intermediate tert-butyl (1-(5-((3-(9-(4-(5-(((2-(2,6-dioxopiperidin-3-yl)-1-oxoisoindol-5-yl)methyl)formamido)-3-methyl-1H-pyrazol-1-yl)piperidin-1-yl)nonylamido)phenyl)thiopyrazin-2-yl)-4-methylpyridin-4-yl)carbamate (80-11)

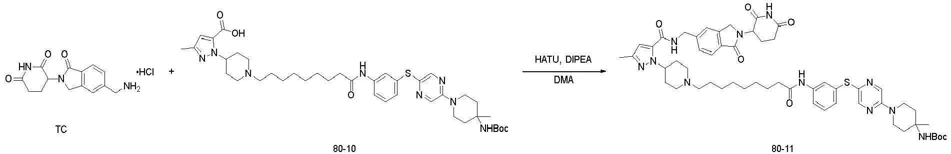

Intermediate **80-10** (762 mg, 1 mmol), intermediate 3-(5-(aminomethyl)-1-oxoisoindol-2-yl)piperidin-2,6-dione hydrochloride (**TC**) (273 mg, 1 mmol), HATU (380 mg, 1 mmol), and DIPEA (390 mg, 3 mmol) were dissolved in 6 mL of N,N-dimethylacetamide, and the mixture was reacted at room temperature for 2 h. The reaction was purified by column chromatography, to obtain 509 mg of product (**80-11**), with a yield of 50%. MS: *m*/*z* 459 (M-100+H⁺)/2.

### Synthesis of compound 1-(1-(9-((3-((5-(4-amino-4-methylpyridin-1-yl)pyrazin-2-yl)thio)phenyl)amino)-9-oxocarbonyl)piperidin-4-yl)-N-(((2-(2,6-dioxopiperidin-3-yl)-1-oxoisoindol-5-yl)methyl)-3-methyl-1H-pyrazol-5-formamide (80)

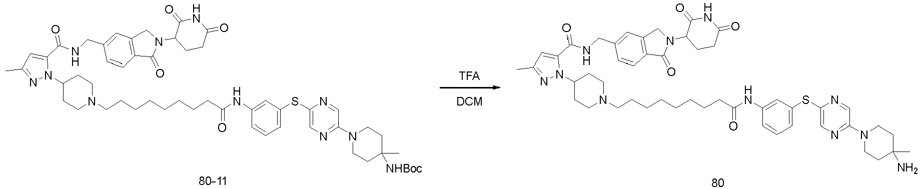

Intermediate **80-11** (509 mg, 0.5 mmol) was dissolved in 10 mL of dichloromethane, to which was added 1 mL of trifluoroacetic acid, and then the mixture was reacted at room temperature for 2h. Dichloromethane was removed by rotatory evaporation, and then 10 mL of dichloromethane was added, followed by rotatory evaporation to dry again, that was repeated twice. The residue was dissolved in 5 mL of methanol, and NaHCOs solid was added to adjust the pH to be 7-8. The resultant solution was filtered, and the filtrate was rotatory evaporated to dry. The residue was dissolved in a mixed solvent of dichloromethane/methanol (V/V = 10:1), and then the resultant solution was filtered. The filtrate was rotatory evaporated to dry, and the residue was dissolve in a mixed solvent of dichloromethane/methanol (V/V = 10:1), followed by filtration. The filtrate was rotatory evaporated to dry, to obtain 430 mg of compound **80** with a yield of 95%. MS: *m*/*z* 459 [M+H]⁺/2.

### Example 3 Synthesis of compound 6-(1-(9-((3-((5-(4-amino-4-methylpyridin-1-yl)pyrazin-2-yl)thio)phenyl)amino)-9-oxocarbonyl)-1,2,3,6-tetrahydropyridin-4-yl)-N-(((2-(2,6-dioxopiperidin-3-yl)-1-oxoisoindol-5-yl)methyl)pyridazin-3-formamide(188):

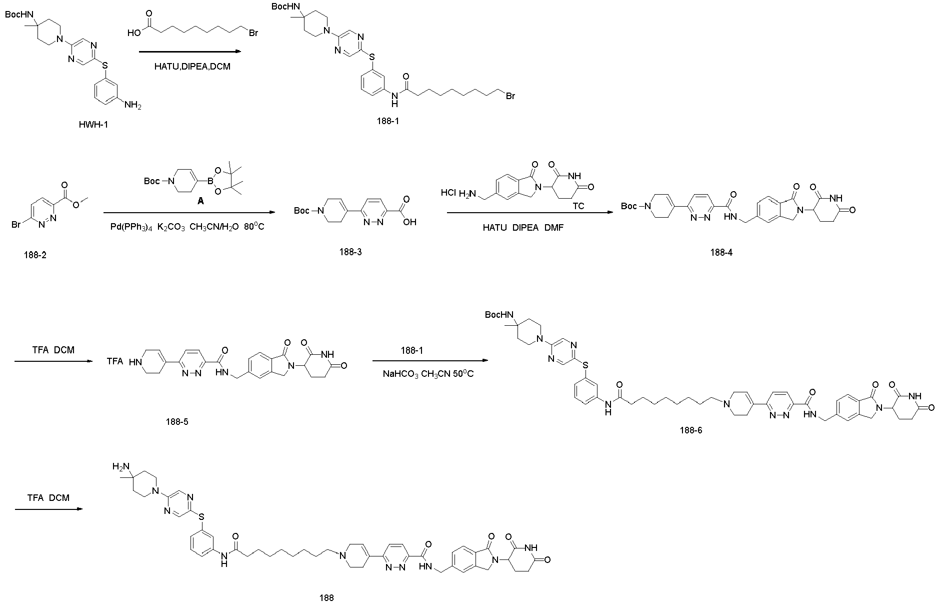

### Synthesis of intermediate tert-butyl (1-(5-((3-(9-Bromoaminoformylamino)phenyl) thio)pyrazin-2-yl)-4-methylpyridin-4-yl)carbamate (188-1):

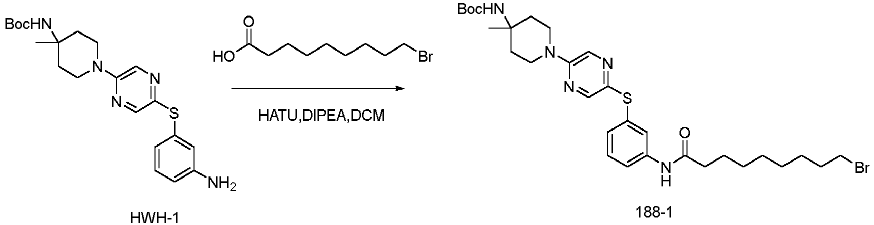

Intermediate tert-butyl (1-(5-((3-aminophenyl)thio)pyrazin-2-yl)4-methylpyridin-4-yl)carbamate (200 mg, 0.48 mmol), 9-bromononanoic acid (114 mg, 0.48 mmol), 2-(7-azobenzotriazole)-N,N,N',N'-tetramethylurea hexafluorophosphate (274 mg, 0.72 mmol), and *N*,*N*-diisopropylethylamine (124 mg, 0.96 mmol) were dissolved in dichloromethane (5 ml); and then the mixture was reacted under stirring at room temperature for 2 h. The reaction solution was successively washed once with water, saturated NaHCOs aqueous solution, and saturated brine, and then the reaction solution was separated. The organic layer was dried over anhydrous Na₂SO₄, and then concentrated, to obtain 230 mg of product (**188-1**), with a yield of 76%. MS: *m*/*z* 634 [M+H]⁺; 636 [M+2+H]⁺.

### Synthesis of intermediate 6-(1-(1-tert-butoxycarbonyl)-1,2,3,6-tetrahydropyridin-4-yl)pyridazin-3-carboxylic acid (188-3):

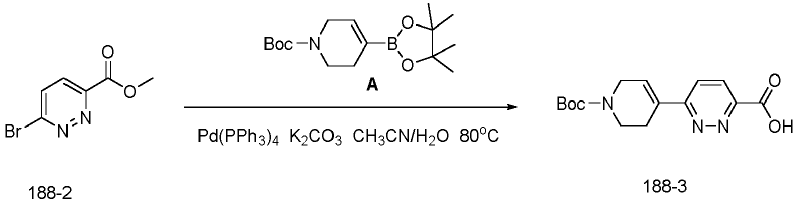

**188-2** (216 mg, 1 mmol), tert-butyl 4-(4,4,5,5-tetramethyl-1,3,2-dioxaborinan-2-yl)-3,6-dihydropyridin-1(2H)-carboxylate (371 mg, 1.2 mmol), tetrakis(triphenylphosphine)palladium (57 mg, 0.05 mmol), and K₂CO₃ (276 mg, 2 mmol) were dissolved in 9 mL of acetonitrile and 1 mL of water, and then the reaction system was purged with nitrogen thrice. The reaction solution was stirred at 80 °C for 10 h, cooled, and then extracted three times with ethyl acetate. The organic layer was dried with anhydrous sodium sulfate and concentrated. The residue was purified by column chromatography to obtain 240 mg of solid (**188-3**), with a yield of 78.7%.

### Synthesis of intermediate tert-butyl 4-(6-(((2-(2,6-dioxopiperidin-3-yl)-1-oxoisoindol-5-yl)methyl)aminoformyl)pyridazin-3-yl)-3,6-dihydropyridine 1(2H)-carboxylate (188-4):

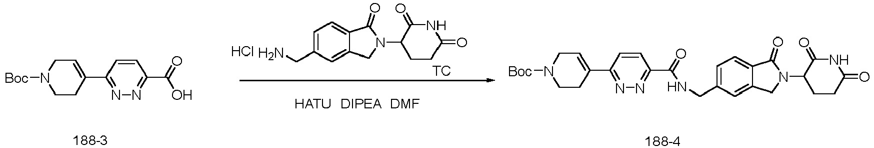

Compound **188-3** (100 mg, 0.32 mmol), 3-(5-(aminomethyl)-1-oxoisoindol-2-yl)piperidin-2,6-dione hydrochloride (102 mg, 0.32 mmol), 2-(7-azobenzotriazole)-N,N,N',N'-tetramethylurea hexafluorophosphate (183 mg, 0.48 mmol), N,N-diisopropylethylamine (83 mg, 0.64 mmol) and N,N-dimethylacetamide (2 ml) were allowed to react overnight. After completion of the reaction, the intermediate **88-4** (81 mg, yield 55.1%) was obtained by purification via column chromatography. MS: *m*/*z* 461 [M-100+H]⁺.

### Synthesis of intermediate N-((2-(2,6-dioxopiperidin-3-yl)-1-oxoisoindol-5-yl)methyl)-6-(1,2,3,6-tetrahydropyridin-4-yl)pyridazin-3-amide trifluoroacetate (188-5)

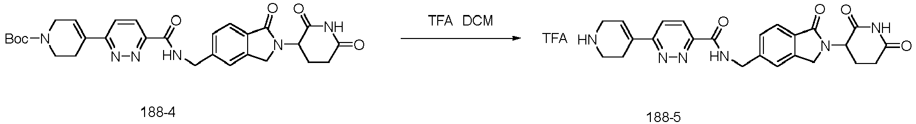

**188-4** (81 mg, 0.17 mmol), trifluoroacetic acid (0.5 ml) and dichloromethane (1 mL) were allowed to react for 0.5 h. After completion of the reaction, the reaction solution was concentrated to obtain **188-5** (80 mg, yield 100 %). MS: *m*/*z* 461 [M+H]⁺.

### Synthesis of intermediate tert-butyl (1-(5-((3-(9-(4-(6-(((2-(2,6-dioxopiperidin-3-yl)-1-oxoisoindol-5-yl)methyl)aminoformyl)pyridazin-3-yl)-3,6-dihydropyridin-1(2H)-yl)nonylamido)phenyl)thiopyrazin-2-yl)-4-methylpyridin-4-yl)carbamate (188-6)

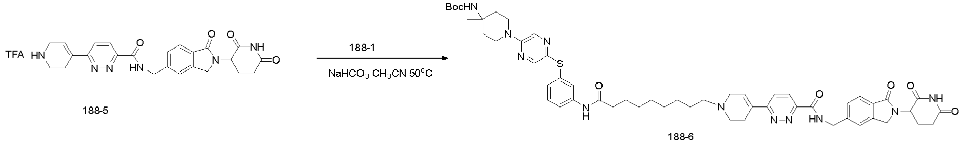

Intermediate **188-1** (91 mg, 0.14 mmol), intermediate 188-5 (80 mg, 0.14 mmol), NaHCOs (36 mg, 0.42 mmol), and acetonitrile (3 mL) were mixed, and then heated to 50 °C, and allowed to react overnight. After completion of the reaction, the reaction solution was directly subjected to suction filtration, and washed with ethyl acetate (5 mL × 3). The organic layers were combined, dried, concentrated, and purified by column chromatography, to obtain the intermediate **188-6** (61 mg, yield 42%). MS: *m*/*z* 1014 [M+H]⁺.

### Synthesis of compound 6-(1-(9-((3-((5-(4-amino-4-methylpyridin-1-yl)pyrazin-2-yl)thio)phenyl)amino)-9-oxocarbonyl)-1,2,3,6-tetrahydropyridin-4-yl)-N-(((2-(2,6-dioxopiperidin-3-yl)-1-oxoisoindol-5-yl)methyl)pyridazin-3-formamide (188)

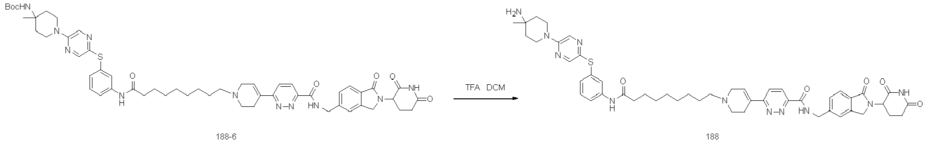

**188-6** (61 mg, 0.06 mmol) and trifluoroacetic acid (0.5 mL) were added into dichloromethane (1 mL), and allowed to react for 0.5 h. After completion of the reaction, the solvent was removed by rotatory evaporation, and then methanol (3 mL) was added. The resultant solution was adjusted to be pH ~7 with NaHCOs solid, filtered, concentrated, and then dissolved by adding dichloromethane (3 mL) and methanol (0.3 mL), followed by filtration and concentration, to obtain compound (**188**) (31 mg, yield 52.5%). MS: *m*/*z* 914 [M+H]⁺.

¹H NMR (400 MHz, DMSO) *δ* 11.01 (s, 1H), 9.98-9.90(m, 2H), 8.45-8.11 (m, 5H), 7.86-7.82 (m, 1H), 7.60-7.62 (m, 2H), 7.55-7.40 (m, 2H), 7.25-7.20 (m, 1H), 5.20-5.10 (m, 1H), 4.60 (s, 2H), 4.41-4.20 (m, 3H), 3.30-2.50 (m, 10H), 2.40 -1.91 (m, 6H), 1.80-1.70 (m, 2H), 1.60-1.49 (m, 2H), 1.40-1.20 (m, 10H), 1.23 (s, 3H).

### Example 4 Synthesis of compound N-(3-((5-(4-amino-4-methylpyridin-1-yl)pyrazin-2-yl)thio)phenyl)-7-(6-(6-(1-(((2-( 2,6-dioxopiperidin-3-yl)-1-oxoisoindol-5-yl)methyl)amino)-2,2,2-trifluoroethyl)pyridazin-3-yl)-2,6-diazaspiro[3.4]octan-2-yl)heptanamide (193):

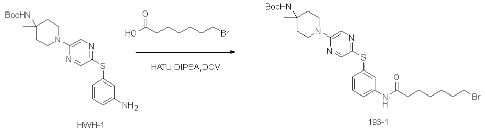

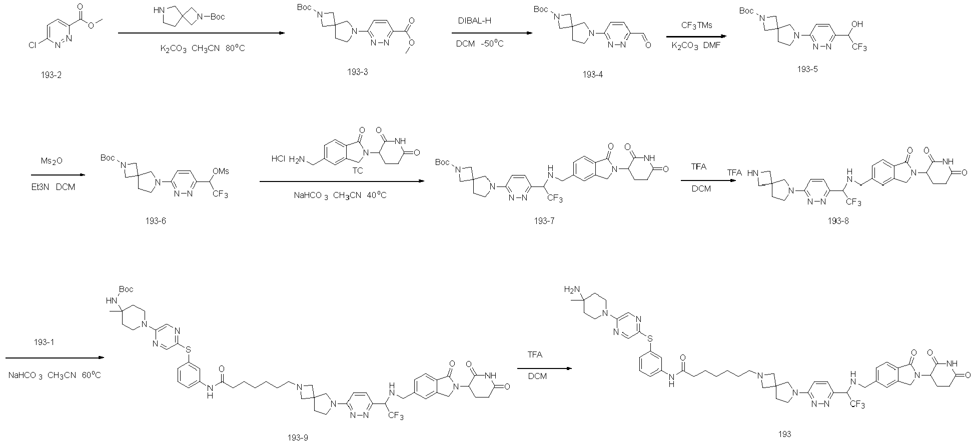

### Synthesis of intermediate tert-butyl (1-(5-((3-(7-bromoheptanamido)phenyl)thio]pyrazin-2-yl)-4-methylpyridin-4-yl)carbamate (193-1):

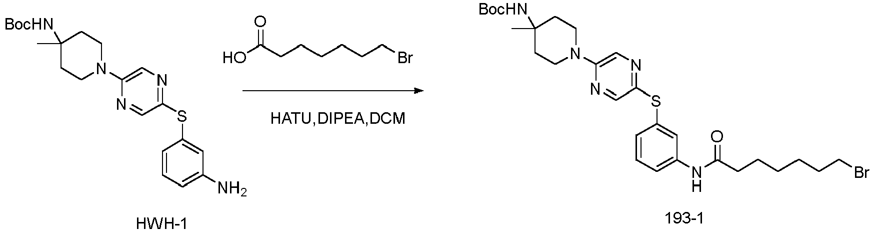

Intermediate tert-butyl (1-(5-((3-aminophenyl)thio)pyrazin-2-yl)4-methylpyridin-4-yl)carbamate (200 mg, 0.48 mmol), 7-bromohexanoic acid (100 mg, 0.48 mmol), 2-(7-azobenzotriazole)-N,N,N',N'-tetramethylurea hexafluorophosphate (274 mg, 0.72 mmol) and *N*,*N*-diisopropylethylamine (124 mg, 0.96 mmol) were dissolved in dichloromethane (5 ml); and then the mixture was reacted under stirring at room temperature for 2 h. The reaction solution was successively washed once with water, saturated NaHCOs aqueous solution, and saturated brine, and then the reaction solution was separated. The organic layer was dried over anhydrous Na₂SO₄, and then concentrated, to obtain 210 mg of product (**193-1**), with a yield of 72.4%. MS: *m*/*z* 606 [M+H]⁺; 608 [M+2+H]⁺.

### Synthesis of intermediate tert-butyl 6-(6-(6-(methoxycarbonyl)pyrazin-3-yl)-2,6-diazaspiro[3.4]octan-2-carboxylate (193-3):

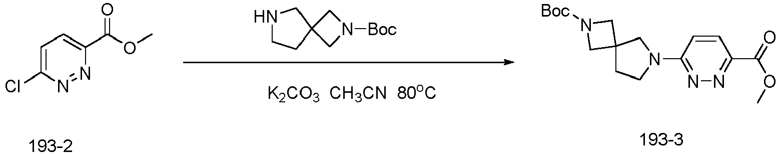

**193-2** (344 mg, 2 mmol), tert-butyl 2,6-diazaspiro[3.4]octan-2-carboxylate (425 mg, 2 mmol), and K₂CO₃ (552 mg, 4 mmol) were added to acetonitrile (10 ml), and then the mixture was stirred at 80°C for 5 h. After completion of the reaction, the intermediate **193-3** (520 mg, yield 75.1%) was obtained by purification via column chromatography. MS: *m*/*z* 349 [M+H]⁺.

### Synthesis of intermediate tert-butyl 6-(6-formylpyrazin-3-yl)-2,6-diazaspiro[3.4]octan-2-carboxylate( 193-4):

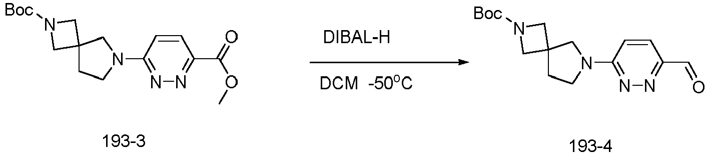

Compound **193-3** (520 mg, 1.49 mmol) was dissolved in dichloromethane, and then cooled to -50 °C. Then, DIBAL-H (1.64 mL, 1.64 mmol) was added dropwise, and the reaction was detected by LCMS. After completion of the reaction, the intermediate **193-4** (402 mg, yield 84.8%) was obtained by purification via column chromatography. MS: 319 [M+H]⁺.

### Synthesis of intermediate tert-butyl 6-(6-(6-(2,2,2-trifluoro-1-hydroxyethyl)pyridazin-3-yl)-2,6-diazaspiro[3.4]octane-2-carboxylate (193-5)

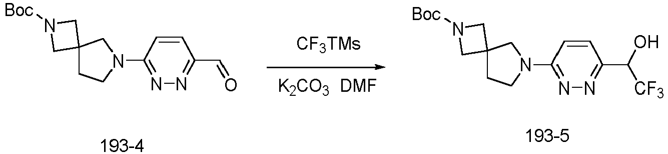

**193-4** (242 mg, 0.76 mmol) and K₂CO₃ (210 mg, 1.52 mmol) were added into DMF (3 ml), and then cooled to 0 °C, to which was then added trifluoromethyltrimethylsilane (119 mg, 0.84 mmol) dropwise. After addition, the mixture was allowed to react for 0.5 h. After completion of the reaction, the reaction solution was concentrated, and the residue was purified by column chromatography, to obtain intermediate **193-5** (223 mg, yield 75.6%). MS: *m*/*z* 389 [M+H]⁺.

### Synthesis of intermediate tert-butyl 6-(6-(6-(2,2,2-trifluoro-1-((methylsulfonyl)oxy)ethyl)pyridazin-3-yl)-2,6-diazaspiro[3.4]octan-2-carboxylate (193-6)

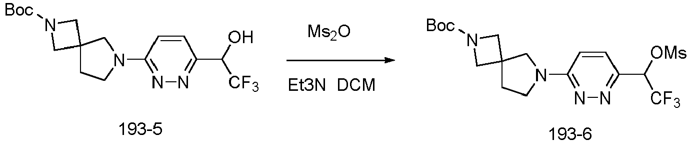

**193-6** (116 mg, 0.3 mmol) and triethylamine (61 mg, 0.6 mmol) were dissolved in dichloromethane, to which was then added methanesulfonic anhydride (58 mg, 0.33 mmol), and the mixture was stirred at room temperature for 10 min. LCMS indicated completion of the reaction. Then, the reaction solution was poured into water, and extracted with dichloromethane. The organic phase was washed with 1N hydrochloric acid, dried, and rotatory evaporated, to obtain **193-6** (128 mg, yield 92%). MS: *m*/*z* 467 [M+H]⁺.

### Synthesis of intermediate tert-butyl 6-(6-(1-((((2-(2,6-dioxopiperidin-3-yl)-1-oxoisoindol-5-yl)methyl)amino)-2,2,2-trifluoroethyl)pyridazin-3-yl)-2,6-diazaspiro[3.4]octan-2-carboxylate (193-7)

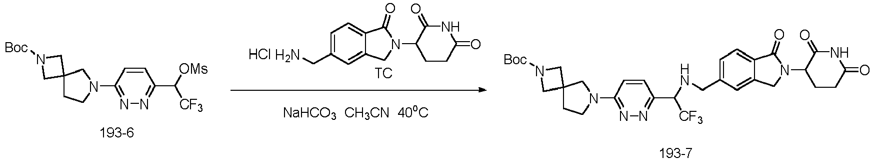

Intermediate 193-6 (128 mg, 0.27 mmol), 3-(5-(aminomethyl)-1-oxoisoindol-2-yl)piperidin-2,6-dionehydrochloride (93 mg, 0.3 mmol), NaHCOs (46 mg, 0.54 mmol), and acetonitrile (5 mL) were mixed, and then heated to 40 °C, and allowed to react overnight. After completion of the reaction, the reaction solution was directly subjected to suction filtration, and washed with ethyl acetate (5 mL × 3). The organic layers were combined, dried, concentrated, and purified by column chromatography, to obtain the intermediate **193-7** (65 mg, yield 37.6%). MS: *m*/*z* 644 [M+H]⁺.

### Synthesis of intermediate 3-(5-(((1-(6-(2,6-diazaspiro[3.4]octan-6-yl)pyridazin-3-yl)-2,2,2-trifluoroethyl)amino)methyl)-1-oxoisoindolin-2-yl)piperidin-2,6-dione trifluoroacetate (193-8)

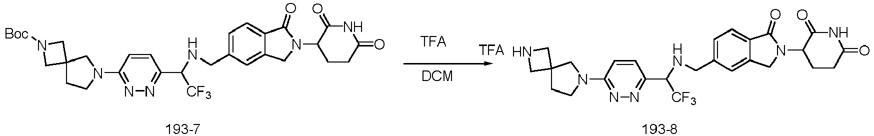

**193-7** (65 mg, 0.1 mmol) and trifluoroacetic acid (0.5 ml) were added into dichloromethane (1 ml), and allowed to react for 0.5 h. After completion of the reaction, the solvent was removed by rotatory evaporation, to obtain compound (**193-8**) (60 mg, yield 100%). MS: *m*/*z* 544 [M+H]⁺.

### Synthesis of intermediate tert-butyl (1-(5-((3-(7-(6-(6-(1-((((2-(2,6-dioxopiperidin-3-yl))-1-oxoisoindol-5-yl)methyl)amino)-2,2,2-trifluoroethyl)pyridazin-3-yl)-2,6-diazaspiro[3.4]octan-2-yl)heptanamido)phenyl)thiopyrazin-2-yl)-4-methylpiperidin-4-ylcarbamate (193-9)

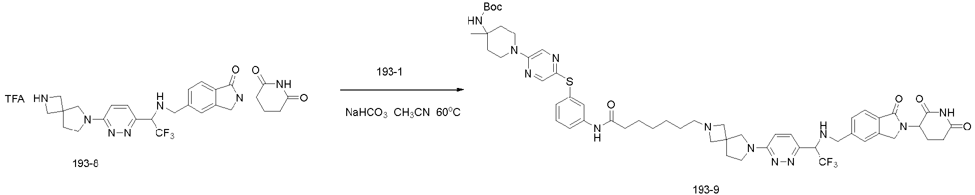

Intermediate **193-8** (60 mg, 0.09 mmol), intermediate **193-1** (57 mg, 0.09 mmol), NaHCOs (16 mg, 0.18 mmol) and acetonitrile (3mL) were mixed, and then heated to 60 °C, and allowed to react overnight. After completion of the reaction, the reaction solution was directly subjected to suction filtration, and washed with ethyl acetate (5 mL × 3). The organic layers were combined, dried, concentrated, and purified by column chromatography, to obtain the intermediate **193-9** (27 mg, yield 27%). MS: *m*/*z* 1069 [M+H]⁺.

### Synthesis of compound N-(3-((5-(4-amino-4-methylpyridin-1-yl)pyrazin-2-yl)thio)phenyl)-7-(6-(6-(1-(((2-(2,6-dioxopiperidin-3-yl)-1-oxoisoindol-5-yl)methyl)amino)-2,2,2-trifluoroethyl)pyridazin-3-yl)-2,6-diazaspiro[3.4]octan-2-yl)heptanamide (193)

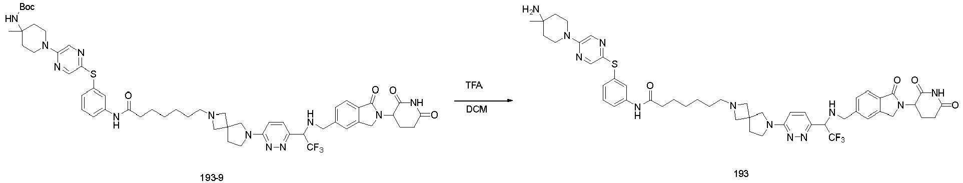

**193-9** (27 mg, 0.025 mmol) and trifluoroacetic acid (0.5 ml) were added into dichloromethane (1 mL), and allowed to react for 0.5 h. After completion of the reaction, the solvent was removed by rotatory evaporation, and then methanol (3 mL) was added. The resultant solution was adjusted to be pH ~7 with NaHCOs solid, filtered, concentrated, and then dissolved by adding dichloromethane (3 mL) and methanol (0.3 mL), followed by filtration and concentration, to obtain compound (**193**) (21 mg, yield 86%). MS: *m*/*z* 969 [M+H]⁺.

¹H NMR (400 MHz, DMSO-d₆) *δ* 11.01 (s, 1H), 10.03(s, 1H), 8.36 (s, 2H), 8.23-6.82 (m, 12H), 5.05-5.02 (m, 1H), 4.42-3.20 (m, 19H), 2.60-1.10 (m, 20H),1.21 (s, 3H).

### Example 5 Synthesis of compound N¹-(3-((5-(4-amino-4-methylpyridin-1-yl)pyrazin-2-yl)thio)phenyl)-N¹⁴-((2-(2,6-dioxopiperidin-3-yl)-1-oxoisoindol-5-yl)methyl)-3,6,9,12-tetraoxadecanediamide (3):

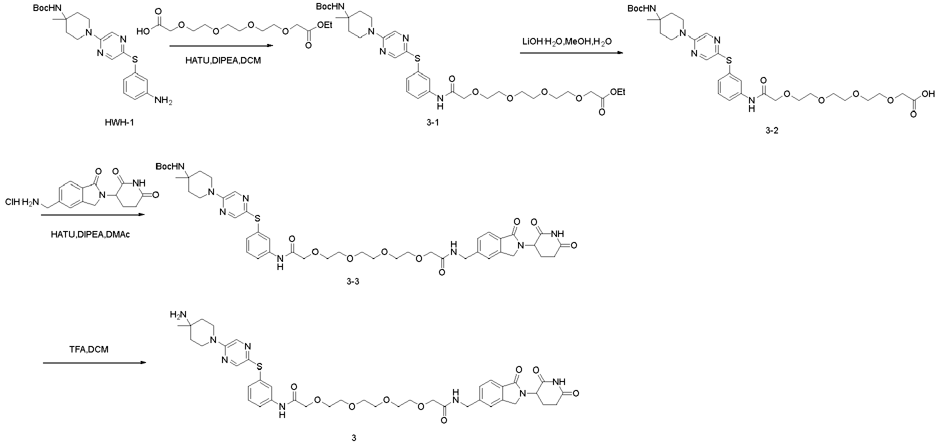

### Synthesis of intermediate ethyl-14-((3-((5-(4-((tert-butoxycarbonyl)amino)-4-methylpyridin-1-yl)pyrazin-2-yl)thio)phenyl)amino)-14-oxo-3,6,9,12-tetraoxalate (3-1):

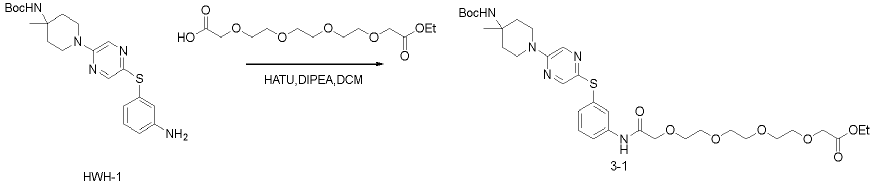

To a 25 mL single-necked flask, were added compound tert-butyl (1-(5-(((3-aminophenyl)thio)pyrazin-2-yl)-4-methylpyridin-4-yl)carbamate (HWH-1, 100 mg, 0.24 mmol), 14-oxo-3,6,9,12,15-pentaoxaheptadecanoic acid (85 mg, 0.29 mmol), 2-(7-azobenzotriazole)-*N*,*N*,*N*',*N*'-tetramethylurea hexafluorophosphate(137mg,0.36mmol), *N*,*N*-diisopropylethylamine (62 mg, 0.48 mmol) and dichloromethane (5 mL), and then the mixture was stirred overnight at room temperature. After completion of the reaction, water (5 mL) was added to the reaction solution, which was separated. The organic layer was dried, concentrated, and then purified by column chromatography, to obtain intermediate (**3-1**, 135mg, yield 81%). MS: m/z 692 [M+H]⁺.

### Synthesis of intermediate 14-((3-((5-(4-((tert-butoxycarbonyl)amino)-4-methylpyridin-1-yl)pyrazin-2-yl)thio)phenyl)amino)-14-oxo-3,6,9,12-tetraoxatetradecanoic acid (3-2):

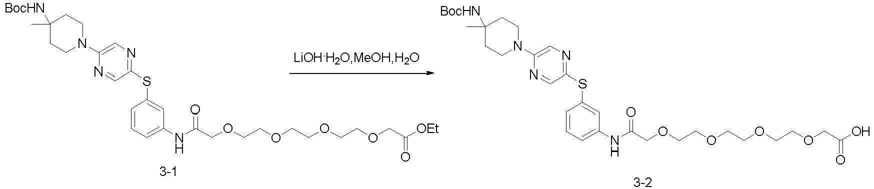

To a 25 mL single-necked flask, were added compound **3-1** (135 mg, 0.2 mmol), lithium hydroxide monohydrate (25 mg, 0.6 mmol), methanol (3 mL) and water (1 mL), and then the mixture was stirred at room temperature for 3-4 h. After completion of the reaction, the pH of the reaction solution was adjusted to 4-5 with 1 N of hydrochloric acid, and then the solution was extracted with dichloromethane (3 mL × 3). The organic phase was combined, dried, and concentrated to obtain intermediate **3-2** (100 mg, yield 77%). MS: *m*/*z* 664 [M+H]⁺.

### Synthesis of intermediate tert-butyl (1-(5-((3-(1-(2-(2,6-dioxopiperidin-3-yl)-1-oxoisoindol-5-yl)-3-oxo-5,8,11,14-tetraoxy-2-azahexadecane-16-amino)phenyl)thio) pyrazin-2-yl)-4-methylpyridin-4-yl)carbamate (3-3):

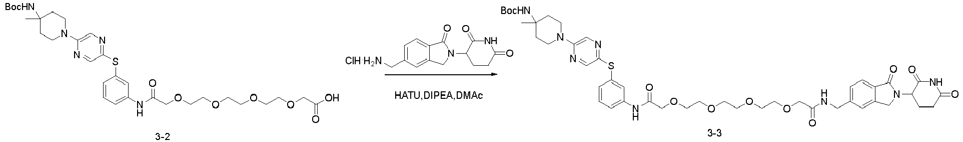

To a 25 mL single-necked flask, were added compound **3-2** (50 mg, 0.075 mmol), 3-(5-(aminomethyl)-1-oxoisoindol-2-yl)piperidin-2,6-dionehydrochloride (23 mg, 0.075 mmol), 2-(7-azobenzotriazole)-N,N,N',N'-tetramethylurea hexafluorophosphate (38 mg, 0.1 mmol), N,N-diisopropylethylamine (29 mg, 0.23 mmol), and N,N-dimethylacetamide(1 mL), and the mixture was allowed to react overnight. After completion of the reaction, the reaction was purified by pre-TLC, to obtain intermediate **3-3** (35mg, yield 51%). MS: *m*/*z* 919 [M+H]⁺.

### Synthesis of compound N¹-(3-((5-(4-amino-4-methylpyridin-1-yl)pyrazin-2-yl)thio)phenyl)-N¹⁴-((2-(2,6-dioxopiperidin-3-yl)-1-oxoisoindol-5-yl)methyl)-3,6,9,12-tetraoxadecanediamide (compound 3):

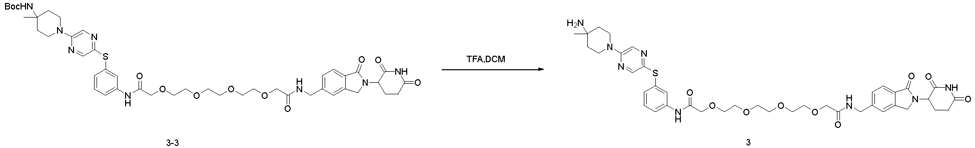

To a 25 mL single-necked flask, were added **3-3** (35 mg, 0.036 mmol), trifluoroacetic acid (1 mL), and dichloromethane (1 mL), and the mixture was allowed to react for 1-2 h. After completion of the reaction, the solvent was removed by rotatory evaporation, and then methanol (3 mL) was added. The resultant solution was adjusted to be pH ~7 with NaHCOs solid, filtered, concentrated, and then dissolved by adding dichloromethane (3 mL) and methanol (0.3 mL), followed by filtration and concentration, to obtain compound **3** (14 mg, yield 44%). MS: *m*/*z* 819 [M+H]⁺.

¹H NMR (400 MHz, DMSO-*d*₆) *δ* 1.00 (s, 1H),9.93 (s, 1H), 8.34 (d, *J* = 1.5 Hz, 1H), 8.23 (s, 1H), 8.15 (d, *J* = 1.4 Hz, 1H), 7.82-7.17 (m, 8H), 6.91-6.83 (m, 1H), 4.36-4.06 (m, 9H), 3.61-3.52(m, 12H), 3.03-2.85(m, 4H), 2.24 -2.12(m, 4H), 1.56-1.40 (m, 4H), 1.25-1.15 (m, 3H).

### Example 6 Synthesis of compound 2-(4-((8-((3-((5-(4-amino-4-methylpyridin-1-yl)pyrazin-2-yl)thio)phenyl)amino)-8-oxyoctyl)oxy)piperidin-1-yl)-N-(((2-(2,6-dioxopiperidin-3-yl)-1-oxoisoindol-5-yl)methyl)pyrimidine-5-formamide (79)

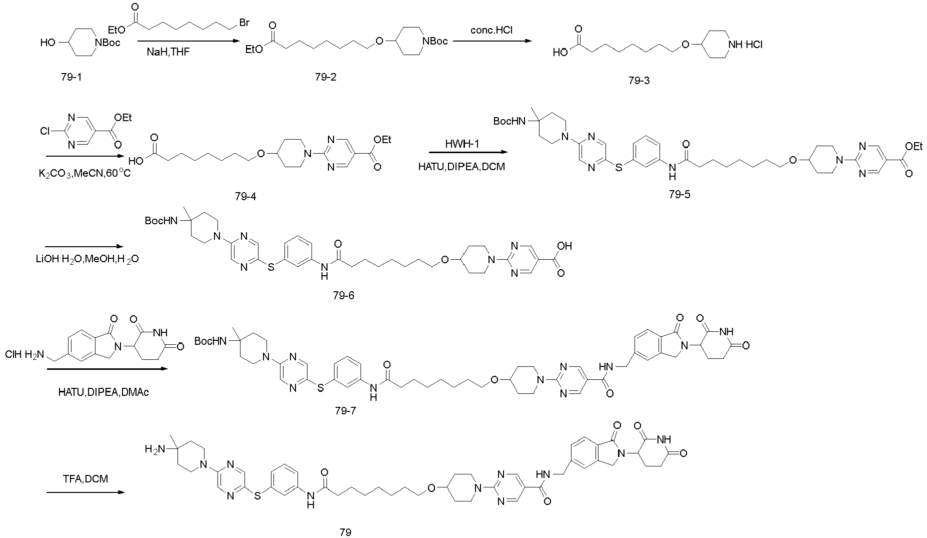

### Synthesis of intermediate tert-butyl 4-((8-ethoxy-8-oxyoctyl)oxy)piperidin-1-formate (79-2)

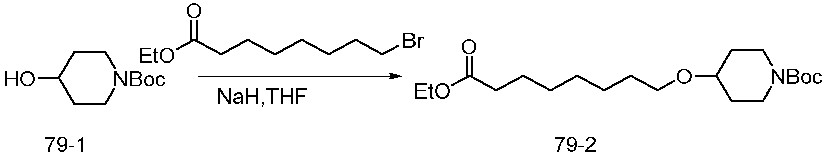

To a 100 mL single-necked flask, were added tert-butyl 4-hydroxylpiperidin-1-carboxylate (**79-1**, 1g, 5 mmol) and tetrahydrofuran (15 mL), and then sodium hydride (300 mg, 7.5 mmol) was added in batches in an ice bath. After addition, the ice bath was removed, and the reaction mixture was stirred at room temperature for 0.5 h. Ethyl 8-bromooctanoate (1.9 g, 7.5 mmol) was added, and the reaction was further stirred at room temperature for 4 h, followed by addition of ammonium chloride aqueous solution (20 mL). The resultant solution was extracted with ethyl acetate (20 mL×3). After extraction, the organic layers were combined, dried, concentrated, and purified by column chromatography, to obtain intermediate **79-2** (1 g, yiled 54%). MS: *m*/*z* 372 [M+H]⁺.

### Synthesis of intermediate 8-(piperidin-4-yloxy)octanoic acid hydrochloride (79-3)

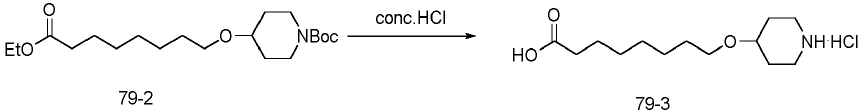

To a 50 mL single-necked flask, were added intermediate **79-2** (1 g, 2.7 mmol) and concentrated hydrochloric acid (10 mL), and then allowed to react for 48 h. After completion of the reaction, the reaction solution was rotatory evaporated to remove water and obtain intermediate **79-3** (250 mg, yield 33%). MS: *m*/*z* 244 [M+H]⁺.

### Synthesis of intermediate 8-((1-(5-(ethoxycarbonyl)pyrimidine-2-yl)piperidin-4-yl)oxy)octanoic acid (79-4)

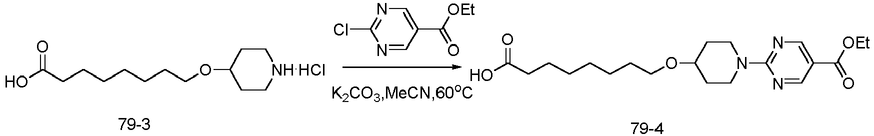

To a 50 mL single-necked flask, were added **79-3** (250 mg, 0.9 mmol), ethyl 2-chloropyrimidine-5-carboxylate (168 mg, 0.9 mmol), K₂CO₃ (500 mg, 3.6 mmol), and acetonitrile (3 mL), and then the mixture was heated to 60 °C, and allowed to react overnight. After completion of the reaction, the reaction solution was diluted with water, and extracted with ethyl acetate (5 mL × 3). The organic layers were combined, dried, concentrated, and purified by column chromatography, to obtain the intermediate **79-4** (136 mg, yield 39%). MS: *m*/*z* 394 [M+H]⁺.

### Synthesis of intermediate ethyl-2-(4-(8-(3-((5-(4-(tert-butoxycarbonyl)amino)-4-methylpyridin-1-yl)pyrazin-2-yl)thio)phenyl)amino)ethyl-8-oxyoctyl)oxy)piperidin-1-yl)pyrimidine-5-carboxylate (79-5)

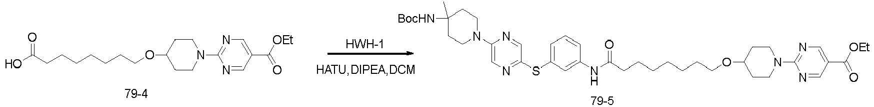

To a 50 mL single-necked flask, were added intermediate **79-4** (136 mg, 0.35 mmol), HWH-1 (145 mg, 0.35 mmol), 2-(7-azobenzotriazole)-N,N,N',N'-tetramethylurea hexafluorophosphate (173 mg, 0.46 mmol), N,N-diisopropylethylamine (136 mg, 1.1 mmol), and dichloromethane (2 mL), and the mixture was allowed to react overnight. After completion of the reaction, purification by pTLC provided intermediate **79-5** (230 mg, yield 83%). MS: *m*/*z* 791 [M+H]⁺.

### Synthesis of intermediate 2-(4-((8-((3-((5-(4-((tert-butoxycarbonyl)amino)-4-methylpyridin-1 -yl)pyrazin-2-yl)thio)phenyl)amino)-8-oxyoctyl)oxy)piperidin-1 -yl)pyrimidine-5-carboxylic acid (79-6)

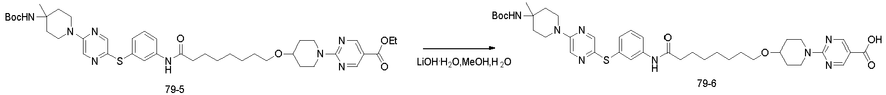

To a 25 mL single-necked flask, were added intermediate **79-5** (230 mg, 0.29 mmol), lithium hydroxide monohydrate (122 mg, 2.9 mmol), methanol (3 mL) and water (1 mL), and then the mixture was stirred at room temperature for 3-4 h. After completion of the reaction, the pH of the reaction solution was adjusted to 4-5 with 1 N of hydrochloric acid, and then the solution was extracted with dichloromethane (3 mL × 3). The organic phase was combined, dried, and concentrated to obtain intermediate **79-6** (210 mg, yield 95%). MS: *m*/*z* 763 [M+H]⁺.

### Synthesis of intermediate tert-butyl (1-(5-((3-(8-((1-(5-(((2-(2,6-dioxopiperidin-3-yl)-1-oxoisoindol-5-yl)methylaminoformyl)pyrimidine-2-ylpiperidin-4-yl)oxy)octylamido) phenyl)thiopyrazin-2-yl)-4-methylpyridin-4-ylcarbamate (79-7)

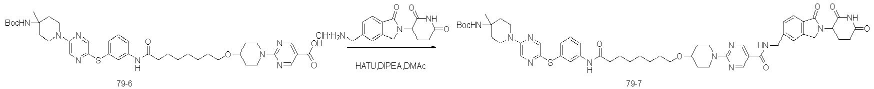

To a 25 mL single-necked flask, were added intermediate **79-6** (50 mg, 0.07 mmol), 3-(5-(aminomethyl)-1-oxoisoindol-2-yl)piperidin-2,6-dionehydrochloride (23 mg, 0.075 mmol), 2-(7-azobenzotriazole)-N,N,N',N'-tetramethylurea hexafluorophosphate (38 mg, 0.1 mmol), N,N-diisopropylethylamine (29 mg, 0.23 mmol), and N,N-dimethylacetamide (1 mL), and the mixture was allowed to react overnight. After completion of the reaction, the reaction mixture was directly purified by pTLC, to obtain intermediate **79-7** (28 mg, yield 42%). MS: *m*/*z* 1018 [M+H]⁺.

### Synthesis of compound 2-(4-((8-((3-((5-(4-amino-4-methylpyridin-1-yl)pyrazin-2-yl)thio)phenyl)amino)-8-oxyoctyl)oxy)piperidin-1-yl)-N-(((2-(2,6-dioxopiperidin-3-yl)-1-oxoisoindol-5-yl)methyl)pyrimidine-5-formamide (79)

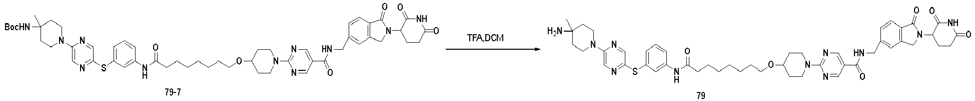

To a 25 mL single-necked flask, were added **79-7** (28 mg, 0.028 mmol), trifluoroacetic acid (1 mL), and dichloromethane (3 mL), and the mixture was allowed to react for 1-2 h. After completion of the reaction, the solvent was removed by rotatory evaporation, and then methanol (3 mL) was added. The resultant solution was adjusted to be pH ~7 with NaHCOs solid, filtered, concentrated, and then dissolved by adding dichloromethane (3 mL) and methanol (0.3 mL), followed by filtration and concentration, to obtain compound **79** (15 mg, yield 60%). MS: *m*/*z* 918 [M+H]⁺.

¹H NMR (400 MHz, DMSO-*d*₆) *δ* 11.00 (s, 1H), 9.94 (s, 1H), 9.02 (t, *J* = 6.0 Hz, 1H), 8.81 (s, 2H), 8.38 (d, *J* = 1.5 Hz, 1H), 8.18 (d, *J* = 1.4 Hz, 1H), 8.02 (d, *J* = 43.3 Hz, 2H), 7.69 (d, *J* = 7.8 Hz, 1H), 7.64-7.50 (m, 2H), 7.45 (d, *J* = 8.1 Hz, 2H), 7.23 (t, *J* = 8.0 Hz, 1H), 6.90 (dd, *J* = 7.8, 1.7 Hz, 1H), 5.11 (dd, *J* = 13.3, 5.1 Hz, 1H), 4.57 (d, *J* = 5.9 Hz, 2H), 4.48-3.96 (m, 6H), 3.43-3.37 (m, 7H), 2.98-2.83 (m, 1H), 2.62-2.57 (m, 1H), 2.38-2.22 (m, 2H), 2.06-1.63 (m, 7H), 1.58-1.25 (m, 16H).

### Example 7 Synthesis of compound N-(3-((5-(4-amino-4-methylpyridin-1-yl)pyrazin-2-yl)thio)phenyl)-7-(6-(6-((2-(2,6-dioxopiperidin-3-yl)-1-oxoisoindol-5-yl)ethynyl) pyridazin-3-yl)-2,6-diazaspiro[3.4]octan-2-yl)heptanamide (157):

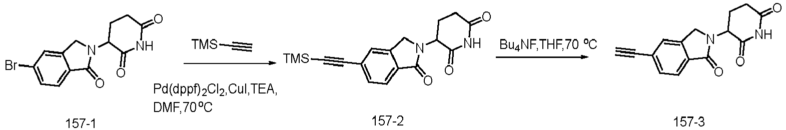

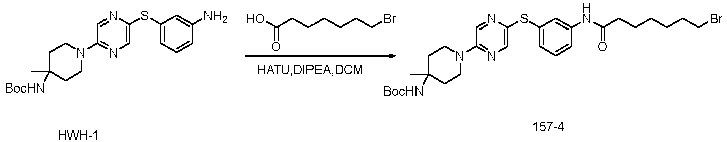

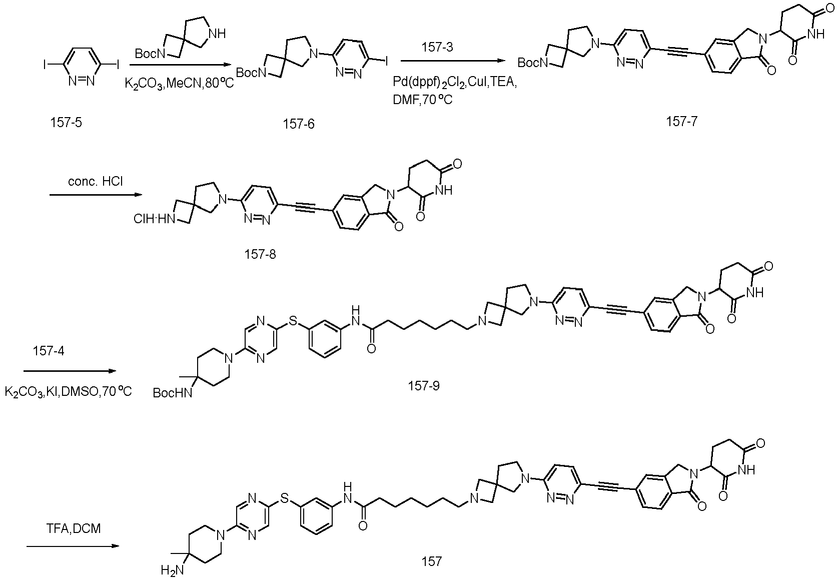

### Synthesis of intermediate 3-(1-oxo-5-((trimethylsilyl)ethynyl)isoindol-2-yl)piperidin-2,6-dione (157-2)

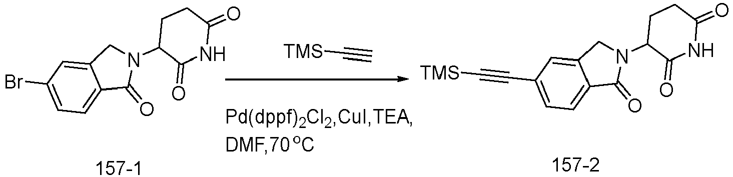

To a 100 mL single-necked flask, were added 3-(5-bromo-1-oxoisoindol-2-yl)piperidin-2,6-dione (**157-1**, 5 g, 15.5 mmol), ethynyltrimethylsilane (7.6 g, 77.6 mmol), [1,1'-bis(diphenylphosphino)ferrocene]dichloropalladium (2.3 g, 3.1 mmol), CuI (589 mg, 3.1 mmol), trimethylamine (10 mL), and N,N-dimethylformamide (15 mL), and then the mixture was heated to 70 °C and reacted overnight under nitrogen protection. After completion of the reaction, the reaction solution was diluted with water (20 mL), and extracted with dichloromethane (20 mL × 3). The organic layers were combined, dried, and purified by column chromatography, to obtain intermediate **157-2** (2.5 g, yield 47%). MS: *m*/*z* 341 [M+H]⁺.

### Synthesis of intermediate 3-(5-ethynyl-1-oxoisoindol-2-yl)piperidin-2,6-dione (157-3)

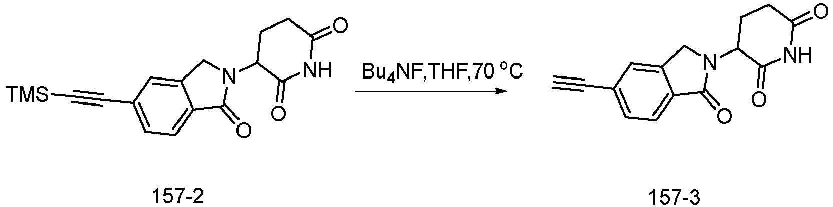

To a 100 mL single-necked flask, were added **157-2** (4.4 mmol, 1.5 g), tetrabutylammonium fluoride (4.6 g, 17.6 mmol), and tetrahydrofuran (200 mL), and then the reaction solution was heated to 70 °C for about 2 h. After completion of the reaction, the solvent was removed by rotatory evaporation, and then the residue was triturated with tetrahydrofuran (3 mL), followed by filtration, to obtain intermediate **157-3** (700 mg, yield 59%). MS: *m*/*z* 269 [M+H]⁺.

### Synthesis of intermediate tert-butyl (1-(5-((3-(7-bromoheptanamido)phenyl)thio)pyrazin-2-yl)-4-methylpyridin-4-yl)carbamate (157-4)

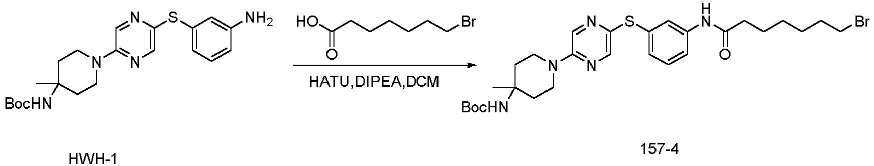

To a 100 mL single-necked flask, were added **HWH-1** (200 mg, 0.48 mmol), 7-bromohexanoic acid (120 mmol, 0.58 mmol), 2-(7-azobenzotriazole)-N,N,N',N'-tetramethylurea hexafluorophosphate (237 mg, 0.62 mmol), N,N-diisopropylethylamine (124 mg, 0.96 mmol), and dichloromethane (5 mL), and then the mixture was allowed to react overnight. After completion of the reaction, the reaction solution was diluted with water (5 mL), and extracted with dichloromethane (5 mL × 3). The organic layers were combined, dried, and purified by column chromatography, to obtain intermediate **157-4** (250 mg, yield 86%). MS: *m*/*z* 606, 608 [M+H]⁺.

### Synthesis of intermediate tert-butyl 6-(6-iodopyridazin-3-yl)-2,6-diazaspiro[3.4]octan-2-carboxylate (157-6)

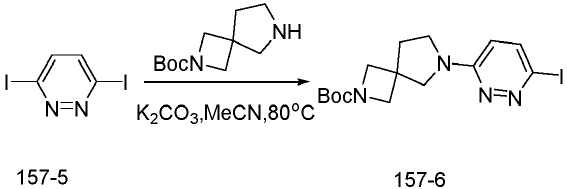

To a 100 mL single-necked flask, were added 3,6-diiodopyridazine (**157-5**, 3 g, 9 mmol), tert-butyl 2,6-diazaspiro[3.4]octan-2-carboxylate (1.9 g, 9 mmol), K₂CO₃ (3.7 g, 27 mmol) and acetonitrile (30 mL), and the mixed solution was heated to 80 °C and reacted overnight, followed by addition of water (10 mL). The resultant solution was extracted with ethyl acetate (20 mL×3) thrice. The organic layers were combined, dried, and purified by column chromatography, to obtain intermediate **157-6** (3 g, yield 80%). MS: *m*/*z* 417 [M+H]⁺.

### Synthesis of intermediate tert-butyl 6-(6-((2-(2,6-dioxopiperidin-3-yl)-1-oxoisoindolin-5-yl)ethynyl)pyridazin-3-yl)-2,6-diazaspiro[3.4]octan-2-carboxylate (157-7)

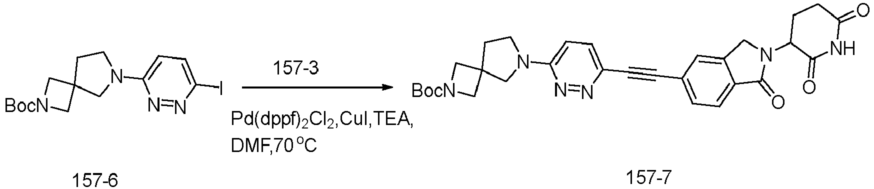

To a 25 mL single-necked flask, were added **157-6** (300 mg, 0.72 mmol), **157-3** (193 mg, 0.72 mmol), [1,1'-bis(diphenylphosphino)ferrocene]dichloropalladium (110 mg, 0.15 mmol), CuI (29 mg, 0.15 mmol), trimethylamine (2 mL), and N,N-dimethylformamide (2 mL), and then the mixture was heated to 70 °C and reacted overnight under nitrogen protection. After completion of the reaction, the solution was cooled to room temperature and filtered. The filter cake was washed with acetonitrile (3 mL) and dried, to obtain intermediate **157-7** (270 mg, yield 68%). MS: *m*/*z* 557 [M+H]⁺.

### Synthesis of intermediate 3-(5-((6-(2,6-diazaspiro[3.4]octan-6-yl)pyridazin-3-yl)ethynyl)-1-oxoisoindol-2-yl)piperidin-2,6-dione hydrochloride (157-8)

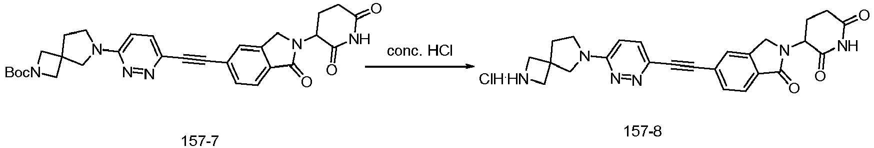

To a 25 mL single-necked flask, were added **157-7**(100 mg, 0.18 mmol) and concentrated hydrochloric acid (3 mL), and the mixture was reacted for about 1 h. After completion of the reaction, the solution was concentrated to obtain intermediate **157-8** (88 mg, yield 99%). MS: *m*/*z* 457 [M+H]⁺.

### Synthesis of intermediate tert-butyl (1-(5-((3-(7-(6-(6-((2-(2,6-dioxopiperidin-3-yl)-1-oxoisoindolin-5-yl)ethynyl)pyridazin-3-(ethynyl)-2,6-diazaspiro[3.4]octan-2-yl) heptanamido)phenyl)thiopyrazin-2-yl)-4-methylpyridin-4-yl)carbamate (157-9)

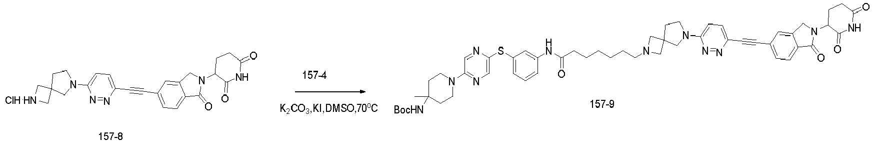

To a 25 mL single-necked flask, were added **157-8** (88 mg, 0.18 mmol), **157-4** (109 mg, 0.18 mmol), K₂CO₃ (100 mg, 0.72 mmol), KI (30 mg, 0.18 mmol), and dimethylsulfoxide (2 mL), and then the reaction solution was heated to 70 °C for about 3 h. After completion of the reaction, the reaction solution was diluted with water (3 mL), and extracted with dichloromethane (5 mL × 3). The organic layers were combined, dried, and purified by pTLC, to obtain intermediate **157-9** (14 mg, yield 8%). MS: *m*/*z* 982 [M+H]⁺.

### Synthesis of compound N (3-((5-(4-amino-4-methylpyridin-1-yl)pyrazin-2-yl)thio)phenyl)-7-(6-(6-((2-(2,6-dioxopiperidin-3-yl)-1-oxoisoindol-5-yl)ethynyl)pyridazin-3-yl)-2,6-diazaspiro[3.4]octan2-yl)heptanamide (157)

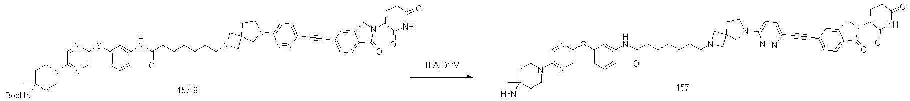

To a 25 mL single-necked flask, were added **157-9** (14 mg, 0.014 mmol), trifluoroacetic acid (1 mL), and dichloromethane (3 mL), and the mixture was allowed to react for 1-2 h. After completion of the reaction, the solvent was removed by rotatory evaporation, and then methanol (3 mL) was added. The resultant solution was adjusted to be pH ~7 with NaHCO₃ solid, filtered, concentrated, and then dissolved by adding dichloromethane (3 mL) and methanol (0.3 mL), followed by filtration and concentration, to obtain compound **157** (12 mg, yield 95%). MS: *m*/*z* 882 [M+H]⁺.

¹H NMR (400 MHz, DMSO-*d*₆) *δ* 11.00 (s, 1H),9.95 (s, 1H), 8.38 (*d*, *J* = 1.5 Hz, 1H), 8.18 (d, *J* = 1.4 Hz, 1H), 7.84-7.60 (m, 7H), 7.24 (t, *J* = 8.0 Hz, 2H), 6.90 (dd, *J* = 8.3, 2.8 Hz, 2H), 5.32 (t, *J* = 4.9 Hz, 1H), 4.76 (dd, *J* = 10.3, 5.0 Hz, 1H), 4.71-4.45 (m, 3H), 4.05 (d, *J* = 14.0 Hz, 3H), 3.69-3.39 (m, 9H), 2.34-2.24 (m, 6H), 1.72-1.62 (m, 4H), 1.44-1.26 (m, 13H).

### Example 8 Synthesis of compound N-(3-((5-(4-amino-4-methylpyridin-1-yl)pyrazin-2-yl)thio)phenyl)-8-(6-(4-((2-(2,6-dioxopiperidin-3-yl)-1-oxoisoindol-5-yl)ethynyl)-1H-pyrazol-1-yl)-2-azaspiro[3.3]heptan-2-yl)octylamide (172)

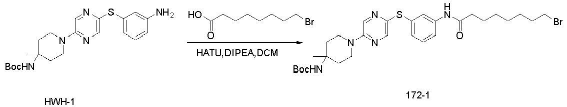

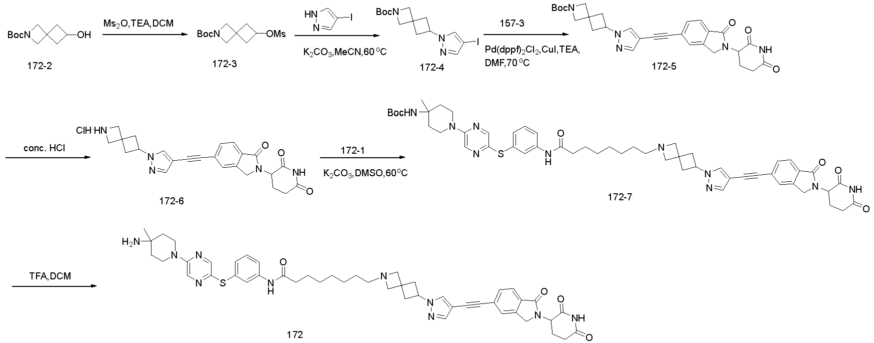

### Synthesis of intermediate tert-butyl (1-(5-((3-(8-bromooctylamino)phenyl)thio)pyrazin-2-yl)-4-methylpyridin-4-yl)carbamate (172-1)

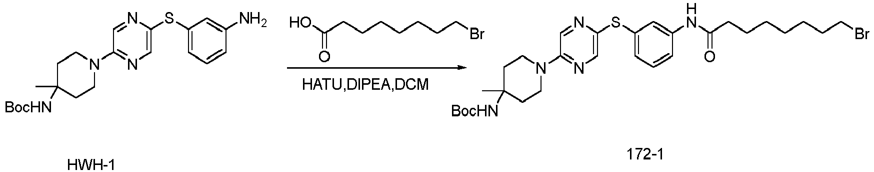

To a 100 mL single-necked flask, were added **HWH-1** (400 mg, 0.96 mmol), 8-bromooctanoic acid (258 mmol, 1.16 mmol), 2-(7-azobenzotriazole)-N,N,N',N'-tetramethylurea hexafluorophosphate (471 mg, 1.24 mmol), N,N-diisopropylethylamine (248 mg, 1.92 mmol), and dichloromethane (10 mL), and the mixture was allowed to react overnight. After completion of the reaction, the reaction solution was diluted with water (10 mL), and extracted with dichloromethane (10 mL × 3). The organic layers were combined, dried, and purified by column chromatography, to obtain intermediate **172-1** (520 mg, yield 87%). MS: *m*/*z* 620, 622 [M+H]⁺.

### Synthesis of intermediate tert-butyl 6-(((methylsulfonyl)oxy)-2-azaspiro[3.3]heptan-2-carboxylate (172-3)

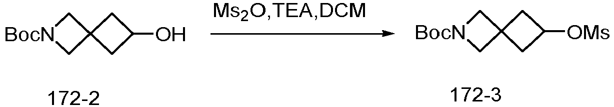

To a 100 mL single-necked flask, were added tert-butyl 6-hydroxyl-2-azaspiro[3.3]heptan-2-carboxylate (**172-2**, 1 g, 4.7 mmol), methanesulfonic anhydride (900 mg, 5.2 mmol), trimethylamine (950 mg, 9.4 mmol) and dichloromethane (20 mL), and the mixture was allowed to react overnight. After completion of the reaction, the reaction solution was diluted with 1 N of dilute HCl aqueous solution (10 mL), and then separated. The organic layer was dried and concentrated, to obtain intermediate **172-3** (1.36 g, 100%). MS: *m*/*z* 292 [M+H]⁺.

### Synthesis of intermediate tert-butyl 6-(4-iodo-1H-pyrazol-1-yl)-2-azaspiro[3.3]heptan-2-carboxylate (172-4)

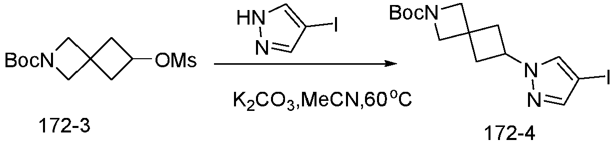

To a 100 mL single-necked flask, were added **172-3** (1.36 g, 4.7 mmol), 4-iodo-1*H*-pyrazole (1.8 g, 9.4 mmol), K₂CO₃ (1.3 g, 9.4 mmol) and acetonitrile (20 mL), and then the reaction solution was heated to 60 °C overnight. After completion of the reaction, the reaction solution was diluted with water (10 mL) and then extracted with ethyl acetate (20 mL×3). The organic layers were combined, dried, and purified by column chromatography, to obtain intermediate **HC-172-4** (1.5 g, 82%). MS: *m*/*z* 390 [M+H]⁺.

### Synthesis of intermediate tert-butyl -6-(4-((2-(2,6-dioxopiperidin-3-yl)-1-oxoisoindolin-5-yl)ethynyl)-1H-pyrazol-1-yl)-2-azaspiro[3.3]heptan-2-carboxylate (172-5)

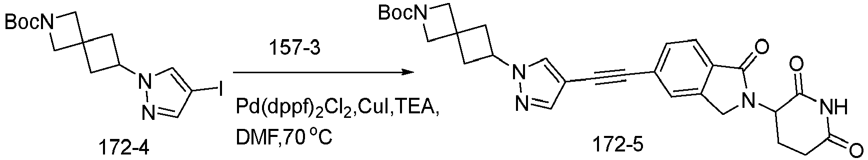

To a 100 mL single-necked flask, were added **172-4** (389 mg, 1 mmol), **157-3** (322 mg, 1.2 mmol), [1,1'-bis(diphenylphosphino)ferrocene]dichloropalladium (146 mg, 0.2 mmol), CuI (38 mg, 0.2 mmol), trimethylamine (2 mL), and N,N-dimethylformamide (2 mL), and then the mixture was heated to 70 °C and reacted overnight under nitrogen protection. After completion of the reaction, the reaction solution was cooled to room temperature, diluted with water (4 mL), and then extracted with dichloromethane (5 mL×3). The organic layers were combined, dried, and purified by column chromatography, to obtain intermediate **172-5** (300 mg, yield 82%). MS: *m*/*z* 530 [M+H]⁺.

### Synthesis of intermediate 3-(5-((1-(2-(azaspiro[3.3]heptan-6-yl)-1H-pyrazol-4-yl-ethynyl)-1-oxoisoindol-2-yl)piperidin-2,6-dionehydrochloride (172-6)

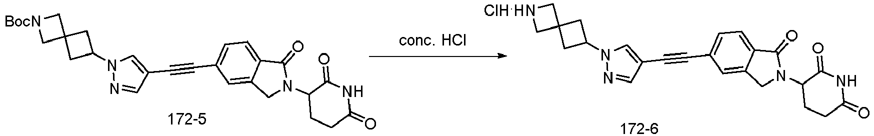

To a 25 mL single-necked flask, were added **172-5** (300 mg, 0.57 mmol) and concentrated hydrochloric acid (5 mL), and the mixture was reacted for about 1 h. After completion of the reaction, the solution was concentrated to obtain intermediate **172-6** (264 mg, yield 100%). MS: *m*/*z* 430 [M+H]⁺.

### Synthesis of intermediate tert-butyl (1-(5-((3-(8-(6-(4-((2-(2,6-dioxopiperidin-3-yl)-1-oxoisoindol-5-yl)ethynyl)-1H-pyrazol-1-yl)-2-azaspiro[3.3]heptan-2-yl)octanoylamino) phenyl)thiopyrazin-2-yl)-4-methylpyridin-4-ylcarbamate (172-7)

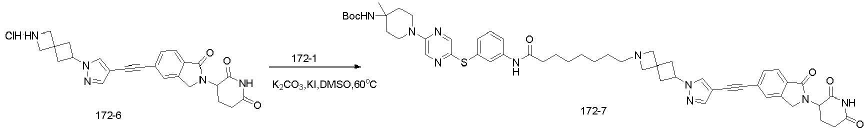

To a 25 mL single-necked flask, were added **172-6** (264 mg, 0.57 mmol), **172-1** (353 mg, 0.57 mmol), K₂CO₃ (315 mg, 2.28 mmol), KI (95 mg, 0.57 mmol), and dimethylsulfoxide (4 mL), and then the reaction solution was heated to 70 °C for about 3 h. After completion of the reaction, the reaction solution was diluted with water (3 mL), and extracted with dichloromethane (5 mL × 3). The organic layers were combined, dried, and purified by pTLC, to obtain intermediate **172-7** (60 mg, yield 11%). MS: *m*/*z* 969 [M+H]⁺.

### Synthesis of N-(3-((5-(4-amino-4-methylpyridin-1-yl)pyrazin-2-yl)thio)phenyl)-8-(6-(4-((2-(2,6-dioxopiperidin-3-yl)-1-oxoisoindol-5-yl)ethynyl)-1H-pyrazol-1-yl)-2-azaspiro[3.3]heptan-2-yl)octylamide (172)

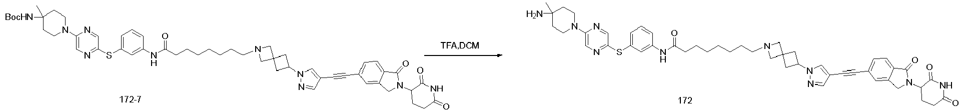

To a 25 mL single-necked flask, were added **172-7** (20 mg, 0.02 mmol), trifluoroacetic acid (1 mL), and dichloromethane (3 mL), and the mixture was allowed to react for 1-2 h. After completion of the reaction, the solvent was removed by rotatory evaporation, and then methanol (3 mL) was added. The resultant solution was adjusted to be pH 7 with NaHCO₃ solid, filtered, concentrated, and then dissolved by adding dichloromethane (3 mL) and methanol (0.3 mL), followed by filtration and concentration, to obtain compound **172** (15 mg, yield 83%). MS: *m*/*z* 869 [M+H]⁺.

¹H NMR (400 MHz, DMSO-*d*₆) *δ* 11.00 (s, 1H),9.93 (s, 1H), 8.34 (d, *J* = 1.5 Hz, 1H), 8.23 (s, 1H), 8.15 (d, *J* = 1.4 Hz, 1H), 7.82-7.17 (m, 9H), 6.92-6.82 (m, 1H), 5.36-5.05 (m, 1H), 4.83-4.16 (m, 4H), 3.76-3.58 (m, 4H), 3.16-3.01 (m, 5H), 2.54 (d, *J* = 8.0 Hz, 3H), 2.26 (q, *J=* 7.8 Hz, 5H), 2.07-1.92 (m, 2H), 1.56-1.40 (m, 7H), 1.20-1.05 (m, 10H).

### Example 9 Synthesis of compound 2-(4-((6-((3-((5-(4-amino-4-methylpyridin-1-yl)pyrazin-2-yl)thio)phenyl)amino)-6-oxohexyl)oxy)phenyl)-N-((2-(2,6-dioxopiperidin-3-yl)-3-oxoisoindol-5-yl)methyl)cyclopropane-1-formamide (189):

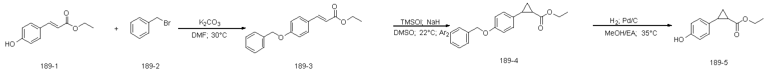

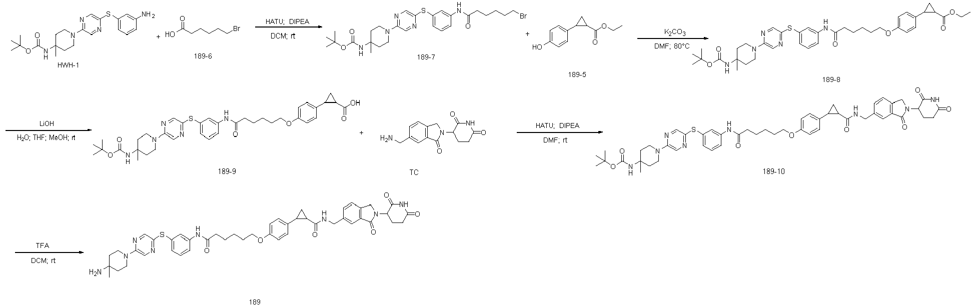

### Synthesis of ethyl (E)-3-(4-(benzyloxy)phenyl)acrylate (189-3):

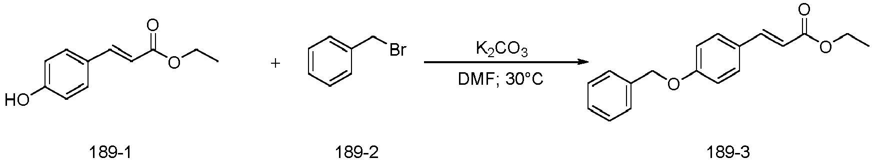

(E)-ethyl p-hydroxycinnamate (1.92 g, 10.00 mmol) and (bromomethyl)benzene (1.88 g, 11.00 mmol) were dissolved in 60 mL of N,N-dimethylformamide, to which was added K₂CO₃ (3.73 g, 27.00 mmol), and then the mixture was allowed to react for 2 h at 30 °C. The reaction solution was diluted with 50 mL of water, stirred, and extracted with 50 mL of ethyl acetate. The organic layer was dried with anhydrous Na₂SO₄, filtered, concentrated, and purified by column chromatography, to obtain 2.68 g of solids (**189-3**), with a yield of 95%. MS: *m*/*z* 283 [M+H]⁺.

### Synthesis of ethyl 2-(4-(benzyloxy)phenyl)cyclopropane-1-carboxylate (189-4):

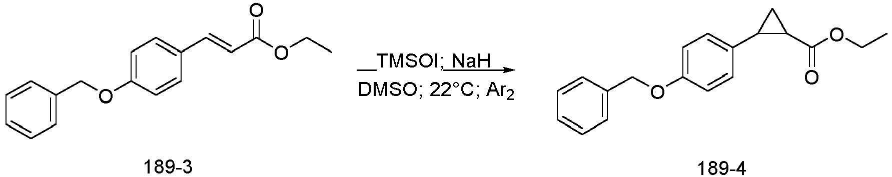

Trimethylsulfoxonium iodide (440 mg, 2.00 mmol) was dissolved in 5 mL dimethylsulfoxide, and then the reaction system was purged with argon thrice, to which was added NaH (60 mg, 0.50 mmol) in portions. The mixture was allowed to react at 22 °C for 0.5 h, followed by addition of **189-3** (282 mg, 1.00 mmol), and then the reaction was further stirred for 1h. 10 mL of water was added for quenching reaction, and then the resultant solution was extracted with 10 mL of ethyl acetate. The organic layer was dried with anhydrous sodium sulfate, filtered, concentrated, and purified with column chromatography to obtain 137 mg of solid (**189-4**), with a yield of 46%. MS: *m*/*z* 297 [M+H]⁺.

### Synthesis of ethyl 2-(4-hydroxylphenyl)cyclopropane-1-carboxylate (189-5):

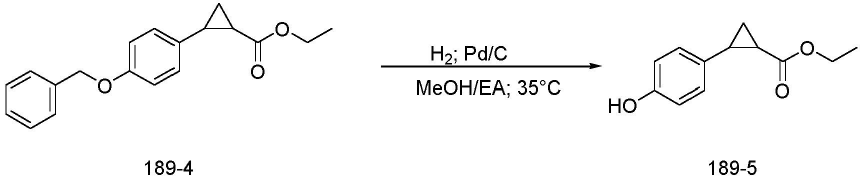

**189-4** (96 mg, 0.32 mmol) and Pd/C (24 mg, 0.03 mmol) were dissolved in 3 mL mixed solvent of methanol/ethyl acetate (1:1), and then the system was purged with hydrogen for three times. The mixture was allowed to react at 35 °C for 3h. The reaction solution was filtered, concentrated, and purified by column chromatography, to obtain 64 mg of solid (**189-5**), with a yield of 91%. MS: *m*/*z* 207 [M+H]⁺.

### Synthesis of tert-butyl (1-(5-((3-(6-bromohexanoylamino)phenyl)thio)pyrazin-2-yl)-4-methylpyridin-4-yl)carbamate:

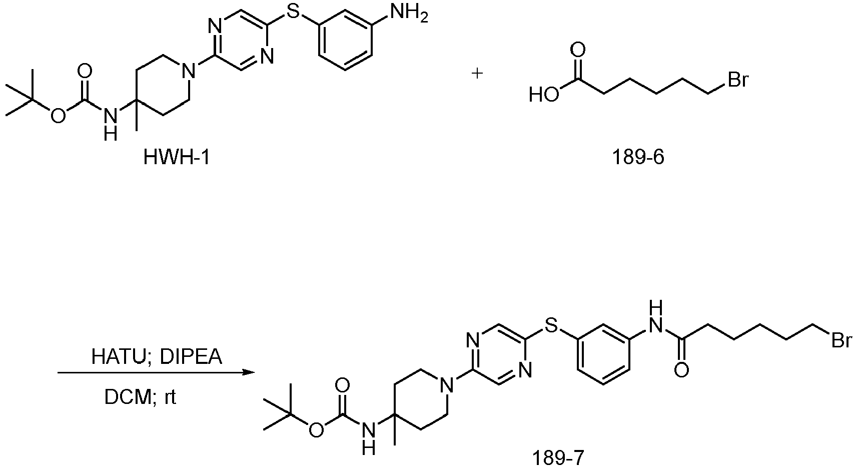

**HWH-1** (830 mg, 2.00 mmol), 6-bromohexanoic acid (390 mg, 2.00 mmol), HATU (837 mg, 2.20 mmol) and DIPEA (517 mg, 4.00 mmol) were dissolved in 15 mL of dichloromethane, and then the mixture was allowed to react overnight at room temperature. The reaction solution was successively washed with 10 mL of water, 10 mL of HCl solution (0.5 mol/L), 10 mL of saturated NaHCOs solution, and 10 mL of saturated brine, and then separated. The water layer was re-extracted with 10 mL of dichloromethane. The organic layers were combined, dried with anhydrous sodium sulfate, filtered, concentrated, and purified by column chromatography to obtain 1.03 g of solid (**189-7**), with a yield of 87%. MS: *m*/*z* 592 [M+H]⁺.

### Synthesis of ethyl 2-(4-((6-((3-((5-(4-((tert-butoxycarbonyl)amino)-4-methylpyridin-1-yl)pyrazin-2-yl)thio)phenyl)amino)ethyl-6-oxohexyl)oxy)phenyl)cyclopropane-1-carboxylate (189-9):

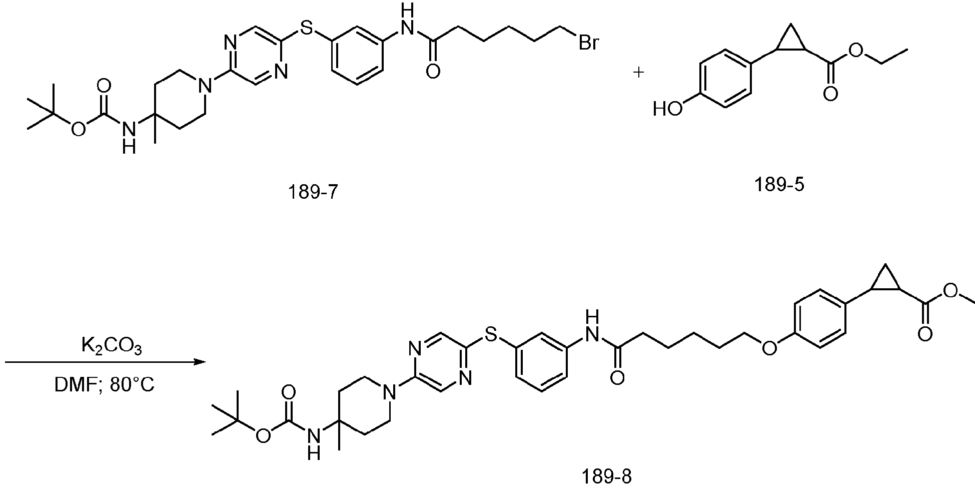

**189-7** (120 mg, 0.20 mmol) and **189-5** (64 mg, 0.2 mmol) were dissolved in 1 mL of N,N-dimethylformamide, to which was added K₂CO₃ (56 mg, 0.40 mmol), and then the mixture was allowed to react overnight at 80 °C. The reaction solution was cooled to room temperature and concentrated. The residue was purified by column chromatography to obtain 90 mg of solids (**189-8**), with a yield of 62%. MS: *m*/*z* 618 [M-100+H]⁺

### Synthesis of 2-(4-((6-((3-((5-(4-((tert-butoxycarbonyl)amino)-4-methylpyridin-1-yl)pyrazin-2-yl)thio)phenyl)amino)-6-oxohexyl)oxy)phenyl)cyclopropane-1-carboxylic acid (189-9):

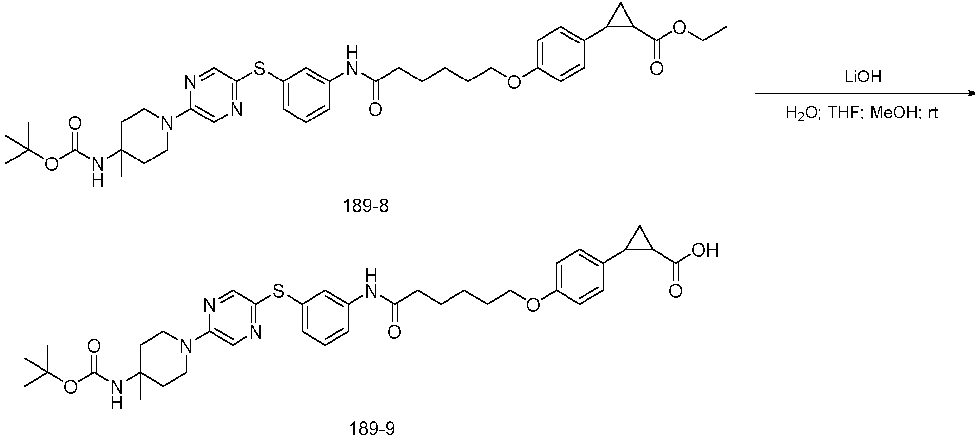

**189-8** (90 mg, 0.13 mmol) was dissolved in a mixed solvent of tetrahydrofuran/methanol/water (1:1:1, 3 mL), to which was added lithium hydroxide monohydrate (82 mg, 1.95 mmol), and the mixture was reacted at room temperature for 0.5 h. The pH of the reaction solution was adjusted to 6-7 with 0.5 mol/L of dilute hydrochloric acid, and then the solution was extracted with 10 mL of dichloromethane. The organic phase was dried with anhydrous sodium sulfate, filtered, and concentrated to obtain 70 mg of crude product (**189-9**), with a yield of 81%. MS: *m*/*z* 329 ((M-100)/2+H⁺).

### Synthesis of tert-butyl (1-(5-((3-(6-(4-(2-(((2-(2,6-dioxopiperidin-3-yl)-3-oxoisoindol-5-yl)methyl)aminoformyl)cyclopropyl)phenoxy)6-amino)phenyl)thio)pyrazin-2-yl)-4-methylpyridin-4-yl)carbamate (189-10):

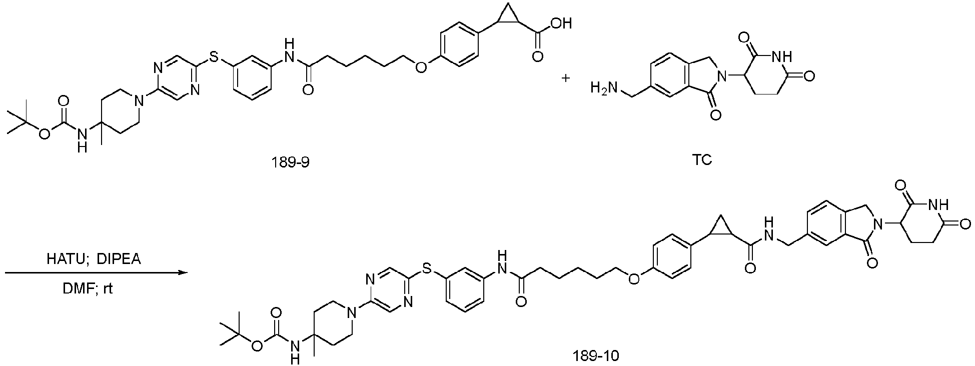

**189-9** (70 mg, 0.10 mmol), TC (29 mg, 0.11 mmol), HATU (46 mg, 0.12 mmol), and DIPEA (33 mg, 0.25 mmol) were dissolved in 0.5 mL of N,N-dimethylformamide, and then the mixture was allowed to react at room temperature overnight. Purification by column chromatography provided 40 mg of solids (**189-10**), with a yield of 42%. MS: *m*/*z* 945 [M+H]⁺.

### Synthesis of 2-(4-((6-((3-((5-(4-amino-4-methylpyridin-1-yl)pyrazin-2-yl)thio)phenyl)amino)-6-oxohexyl)oxy)phenyl)-N-((2-(2,6-dioxopiperidin-3-yl)-3-oxoisoindol-5-yl)methyl)cyclopropane-1-formamide (189):

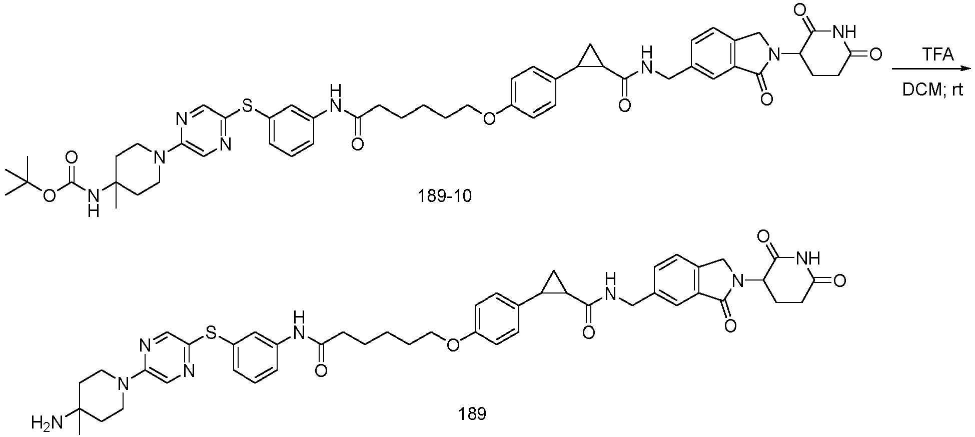

**189-10** (40 mg, 0.42 mmol) was dissolved in 3 mL of dichloromethane, to which was added 1 mL of trifluoroacetic acid, and then the mixture was reacted at room temperature for 0.5 h. The reaction solution was concentrated to dry, and the residue was dissolved in 10 mL of dichloromethane, followed by concentration to remove trifluoroacetic acid, that was repeated twice. To the residue, was added 5 mL of methanol, and then the pH value was adjusted to be 7-8 with NaHCO₃, followed by filtration and concentration. The residue was dissolved in 5 mL of dichloromethane/methanol (10:1), and the resultant solution was filtered and concentrated, to obtain 29 mg of product (**189**), with a yield of 81%. MS: *m*/*z* 845 [M+H]⁺.

¹H NMR (400 MHz, DMSO-*d*₆) *δ* 9.95 (s, 1H), 8.36 (d, *J* = 1.5 Hz, 1H), 8.16 (d, *J* = 1.4 Hz, 1H), 7.66 (ddd, *J* = 15.3, 8.0, 5.0 Hz, 1H), 7.55 (t, *J* = 1.9 Hz, 1H), 7.48 - 7.43 (m, 2H), 7.37 (q, *J* = 8.3, 7.1 Hz, 1H), 7.25 - 7.18 (m, 2H), 7.05 (dd, *J* = 14.0, 8.4 Hz, 2H), 6.89 (d, *J* = 7.8 Hz, 1H), 6.84 - 6.79 (m, 2H), 4.49 - 4.33 (m, 3H), 3.95 - 3.79 (m, 4H), 3.58 - 3.46 (m, 4H), 2.33 - 2.22 (m, 3H), 2.15 (t, *J* = 7.4 Hz, 1H), 2.08 - 1.96 (m, 3H), 1.78 (t, *J* = 7.5 Hz, 1H), 1.73 - 1.68 (m, 2H), 1.64 - 1.58 (m, 4H), 1.48 - 1.38 (m, 4H), 1.30 - 1.20 (m, 7H).

### Example 10 Synthesis of compound 9-(4-(4-((3-((5-(4-(aminomethyl)-4-methylpyridin-1-yl)pyrazin-2-yl)thio)-2-chlorophenyl)amino)-4-oxobutyryl)piperazin-1-yl)-N ((2-(2,6-dioxopiperidin-3-yl)-1-oxoisoindol-5-yl)methyl)nonanamide (15)

### Synthesis of intermediate methyl 9-(4-(4-((3-((5-(4-((tert-butoxycarbonyl)amino)methyl)-4-methylpyridin-1-yl)pyrazin-2-yl)thio)-2-chlorophenyl)amino)-4-oxobutyryl)piperazin-1-yl)nonanoate (15-1):

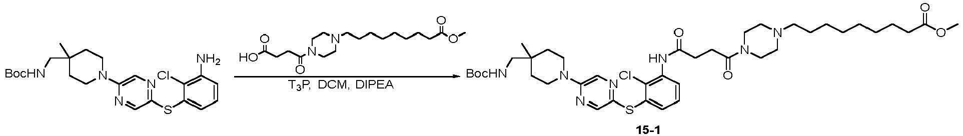

Tert-butyl (1-(5-((3-amino-2-chlorophenyl)thio)pyrazin-2-yl)-4-methylpyridin-4-yl)carbamate (92.8 mg, 0.2 mmol) and 4-(4-(9-methoxy-9-oxocarbonyl)piperazin-1-yl)-4-oxobutyric acid (71.3 mg, 0.2 mmol) were dissolved in 5 mL of DCM, to which were added T₃P (318.2 mg, 1 mmol) and DIPEA (154.8 mg, 1.2 mmol), respectively. The mixture was stirred at room temperature for 12 h. After the reaction was completed, to the reaction solution, was added saturated brine (5 mL), and the resultant solution was rested to separate the organic layer. The water layer was extracted with dichloromethane. The organic layers were combined, dried with anhydrous sodium sulfate, and concentrated. The residue was purified by TLC to obtain 121 mg of intermediate **15-1**, with a yield of 75%. MS: *m*/*z* 802 [M+H]⁺.

### Synthesis of intermediate 9-(4-(4-((3-((5-(4-((tert-butoxycarbonyl)amino)methyl)-4-methylpyridin-1-yl)pyrazin-2-yl)thio)-2-chlorophenyl)amino)-4-oxobutyryl)piperazin-1-yl)nonanoic acid (15-2):

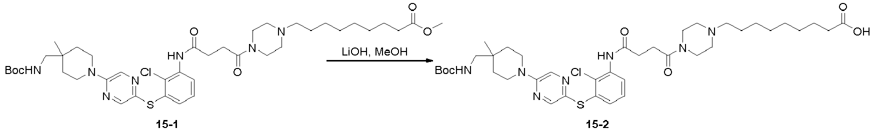

Methyl 9-(4-(4-((3-((5-(4-((tert-butoxycarbonyl)amino)methyl)-4-methylpyridin-1-yl)pyrazin-2-yl)thio)-2-chlorophenyl)amino)-4-oxobutyryl)piperazin-1-yl)nonanoate (intermediate **15-1**) (96.3 mg, 0.12 mmol) was dissolved in methanol (3 mL), to which was added lithium hydroxide monohydrate (100.7 mg, 2.4 mmol), and the mixture was stirred at room temperature for 3 h. After completion of the reaction, the pH of the reaction solution was adjusted to about 6 with 0.5 N of hydrochloric acid, and then the solution was diluted with dichloromethane. After standing, the organic layer was separated, and the water layer was extracted with dichloromethane. The organic layers were combined, dried with anhydrous sodium sulfate, and concentrated. The residue was purified by TLC to obtain 80 mg of intermediate **15-2**, with a yield of 85%. MS: *m*/*z* 788 [M+H]⁺.

### Synthsis of intermediate tert-butyl ((1-(5-((2-chloro-3-(4-(4-((2-(2,6-dioxopiperidin-3-yl)-1-oxoisoquinolin-5-yl)methyl)amino)-9-oxononyl)piperazin-1-yl)-4-oxobutylamido)phenyl)thio)pyrazin-2-yl)-4-methylpyridin-4-yl)carbamate (15-3):

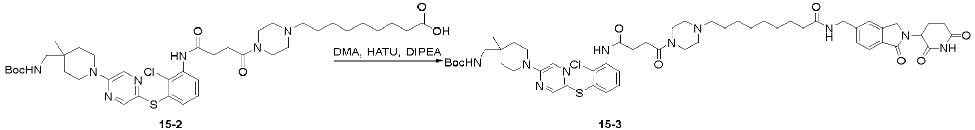

9-(4-(4-((3-((5-(4-((tert-butoxycarbonyl)amino)methyl)-4-methylpyridin-1-yl)pyrazin-2-yl)thio)-2-chlorophenyl)amino)-4-oxobutyryl)piperazin-1-yl)nonanoic acid (intermediate **15-2**) (39.4 mg, 0.05 mmol) and 3-(5-(aminomethyl)-1-oxoisoindol-2-yl)piperidin-2,6-dione (13.7 mg, 0.05 mmol) were dissolved in 0.5 mL of DMA, to which were added HATU (28.5 mg, 0.075 mmol) and DIPEA (12.9 mg, 0.1 mmol), respectively. The mixture was stirred at room temperature for 12 h. After completion of the reaction, purification by TLC provided 35 mg of intermediate **15-3**, with a yield of 67%. MS: *m*/*z* 1043 [M+H]⁺.

### Synthesis of compound 9-(4-(4-((3-((5-(4-(aminomethyl)-4-methylpyridin-1-yl)pyrazin-2-yl)thio)-2-chlorophenyl)amino)-4-oxobutyryl)piperazin-1-yl)-N-((2-(2,6-dioxopiperidin-3-yl)-1-oxoisoindol-5-yl)methyl)nonanamide (15):

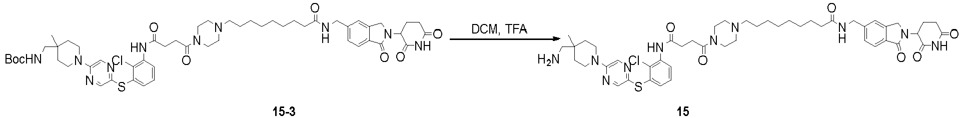

Tert-butyl (1-(5-((2-chloro-3 -(4-(4-((2-(2,6-dioxopiperidin-3-yl)-1-oxoisoquinolin-5-yl)methyl)amino)-9-oxononyl)piperazin-1-yl)-4-oxobutylamido)phenyl)thio)pyrazin-2-yl)-4-methylpyridin-4-yl)carbamate (intermediate **15-3**) (31.3 mg, 0.03 mmol) was dissolved in a mixed solvent of 3 mL DCM and 1 mL TFA, and then the mixture was reacted at room temperature unde stirring for 3 h. After the reaction was completed, the solvent was removed by evaporation under reduced pressure. 3 mL of methanol was added, and the pH of the resultant solution was adjusted to about 8 with NaHCOs. The insoluble substances were filtered out. After methanol was removed by evaporation under reduced pressure, the obtained solid was dissolved in a solution of dichloromethane/methanol (10:1, 5 mL). The insoluble substances were removed by filtration, and the solvent was removed by evaporation under reduced pressure, to obtain 25 mg of compound **15**, with a yield of 88%. MS: *m*/*z* 943 [M+H]⁺.

### Example 11 Synthesis of compound 2-(4-(9-((3-((5-(4-amino-4-methylpyridin-1-yl)pyrazin-2-yl)thio)phenyl)amino)-9-oxynonyl)piperazin-1-yl)-N-((2-(2,6-dioxopiperidin-3-yl)-1-oxoisoindol-5-yl)methyl)pyrimidine-5-formamide (48):

### Synthesis of intermediate ethyl 2-(4-(tert-butoxycarbonyl)piperazin-1-yl)pyrimidine-5-carboxylate (48-1):

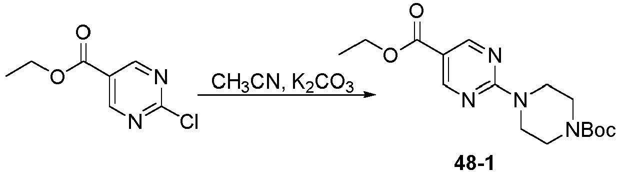

Ethyl 2-chloropyrimidine-5-carboxylate (1.87 g, 10 mmol) and tert-butyl piperazin-1-carboxylate (1.86 g, 10 mmol) were dissolved in 20 mL of acetonitrile, to which was added K₂CO₃ (2.76 g, 20 mmol). The mixture was stirred at 80 °C for 12 h. The reaction solution was cooled, diluted with 30 mL of water, and extracted three times with ethyl acetate. The organic layer was dried with anhydrous sodium sulfate and concentrated. The crude product was recrystallized in ethyl acetate to obtain 3.1 g of intermediate **48-1**, with a yield of 92%. MS: *m*/*z* 337 [M+H]⁺.

### Synthesis of intermediate ethyl 2-(piperazin-1-yl)pyrimidine-5-carboxylate (48-2):

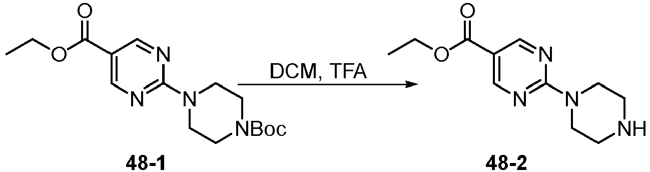

Ethyl 2-(4-(tert-butoxycarbonyl)piperazin-1-yl)pyrimidine-5-carboxylate (intermediate **48-1**) (1.68 g, 5 mmol) was dissolved in a mixed solvent of 10 mL DCM and 5 mL TFA, and then the solution was stirred at room temperature for 3 h. After completion of the reaction, the solvent was removed by evaporation under reduced pressure, and to residue, was added saturated NaHCO₃ solution. The resultant solution was extracted three times with dichloromethane. The organic layer was dried with anhydrous sodium sulfate, and concentrated, to obtain 1.1 g of intermediate **48-2**, with a yield of 92%. MS: *m*/*z* 337 [M+H]⁺.

### Synthesis of intermediate 9-(4-(5-(ethoxycarbonyl)pyrimidine-2-yl)piperazin-1-yl)nonanoic acid (48-3):

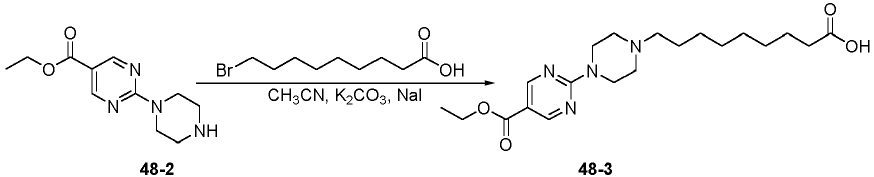

Ethyl 2-(piperazin-1-yl)pyrimidine-5-carboxylate (intermediate **48-2**) (236 mg, 1 mmol) and 9-bromononanoic acid (237 mg, 1 mmol) were dissolved in 10 mL of acetonitrile, to which were added K₂CO₃ (0.69 g, 5 mmol) and NaI (15 mg, 0.1 mmol). The mixture was stirred at 80 °C for 12 h, and then cooled. The pH of the resultant solution was adjusted to around 6 with 0.5 N hydrochloric acid. The solution was extracted three times with ethyl acetate. The organic layer was dried with anhydrous sodium sulfate and concentrated. Purification by column chromatography provided 300 mg of intermediate **48-3**, with a yield of 76.4%. MS: *m*/*z* 393 [M+H]⁺.

### Synthesis of intermediate ethyl 2-(4-(9-((3-((5-(4-((tert-butoxycarbonyl)amino)-4-methylpyridin-1-yl)pyrazin-2-yl)thio)phenyl)amino)-9-oxononyl)piperazin-1-yl)pyrimidine-5-carboxylate (48-4):

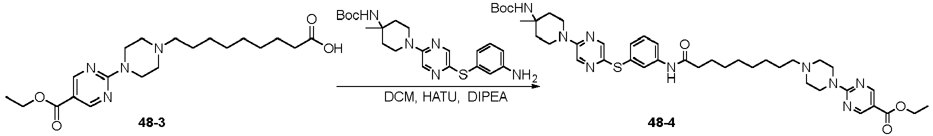

9-(4-(5-(ethoxycarbonyl)pyrimidine-2-yl)piperazin-1-yl)nonanoic acid (intermediate **48-3**) (118 mg, 0.3 mmol) and tert-butyl (1-(5-((3-aminophenyl)thio)pyrazin-2-yl)-4-methylpyridin-4-yl)carbamate (125 mg, 0.3 mmol) were dissolved in 5 mL of dichloromethane, to which were added HATU (171 mg, 0.45 mmol) and DIPEA (77 mg, 0.6 mmol). The mixture was stirred at room temperature for 12 h. After completion of the reaction, purification by TLC provided 160 mg of intermediate **48-4**, with a yield of 67.6%. MS: *m*/*z* 790 [M+H]⁺.

### Synthesis of intermediate 2-(4-(9-((3-((5-(4-((tert-butoxycarbonyl)amino)-4-methylpyridin-1-yl)pyrazin-2-yl)thio)phenyl)amino)-9-oxononyl)piperazin-1-yl)pyrimidine-5-carboxylic acid (48-5):

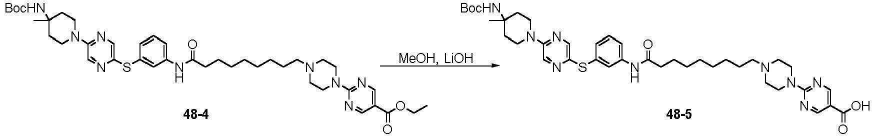

Ethyl 2-(4-(9-((3-((5-(4-((tert-butoxycarbonyl)amino)-4-methylpyridin-1-yl)pyrazin-2-yl)thio)phenyl)amino)-9-oxononyl)piperazin-1-yl)pyrimidine-5-carboxylate (intermediate **48-4**) (158 mg, 0.2 mmol) was dissolved in methanol (3 mL), to which was added lithium hydroxide monohydrate (168 mg, 4 mmol), and the mixture was stirred at room temperature for 3 h. After completion of the reaction, the pH of the reaction solution was adjusted to about 6 with 0.5 N of hydrochloric acid, and then the solution was diluted with dichloromethane. After standing, the organic layer was separated, and the water layer was extracted with dichloromethane. The organic layers were combined, dried with anhydrous sodium sulfate, and concentrated. The residue was purified by TLC to obtain 145 mg of intermediate **48-5**, with a yield of 95%. MS: *m*/*z* 762 [M+H]⁺.

### Synthesis of intermediate tert-butyl (1-(5-((3-(9-(4-(5-((2-(2,6-dioxopiperidin-3-yl)-1-oxoisoindol-5-yl)methyl)carbamoyl)pyrimidine-2-yl)piperazin-1-yl)oxononylamino)phenyl)thio)pyrazin-2-yl)-4-methylpyridin-4-yl)carbamate (48-6):

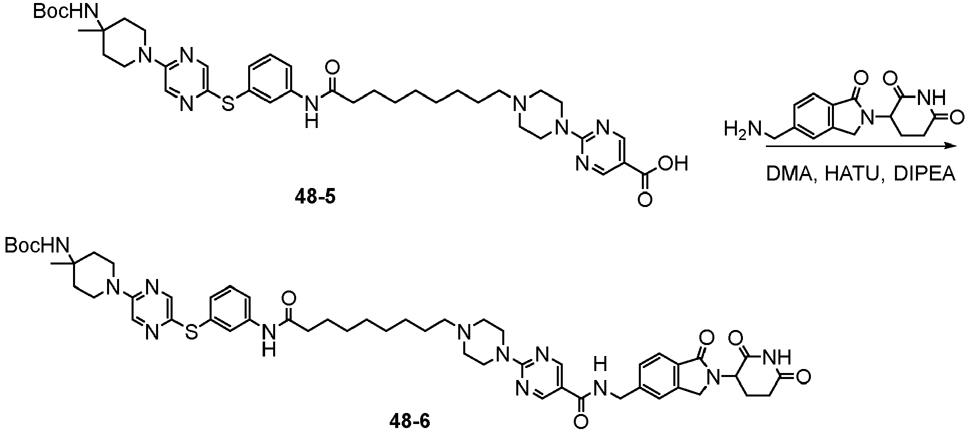

2-(4-(9-((3-((5-(4-((tert-butoxycarbonyl)amino)-4-methylpyridin-1-yl)pyrazin-2-yl)thio)phenyl)amino)-9-oxononyl)piperazin-1-yl)pyrimidine-5-carboxylic acid (intermediate **48-5**) (38 mg, 0.05 mmol) and 3-(5-(aminomethyl)-1-oxoisoindol-2-yl)piperidin-2,6-dione (13.7 mg, 0.05 mmol) were dissolved in 0.5 mL of DMA, to which were added HATU (28.5 mg, 0.075 mmol) and DIPEA (12.9 mg, 0.1 mmol). The mixture was stirred at room temperature for 12 h. After completion of the reaction, purification by TLC provided 40 mg of intermediate **48-6**, with a yield of 78.6%. MS: *m*/*z* 1017 [M+H]⁺.

### Synthesis of compound 2-(4-(9-((3-((5-(4-amino-4-methylpyridin-1-yl)pyrazin-2-yl)thio)phenyl)amino)-9-oxononyl)piperazin-1-yl)-N-((2-(2,6-dioxopiperidin-3-yl)-1-oxoisoindol-5-yl)methyl)pyrimidine-5-formamide (48):

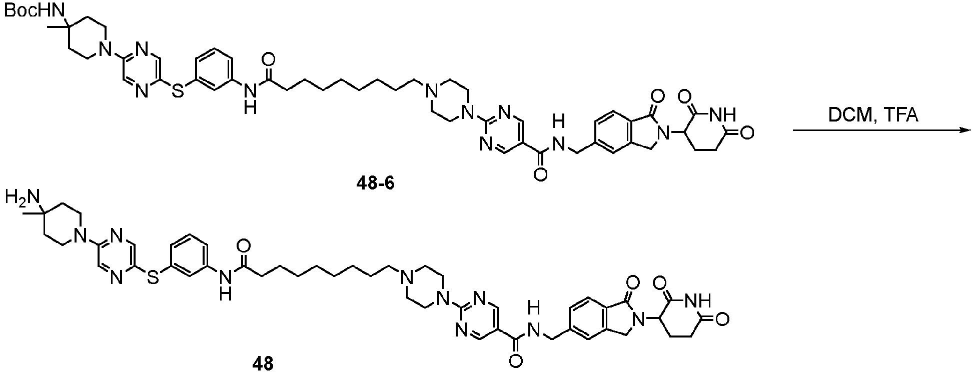

Tert-butyl (1-(5-((3-(9-(4-(5-((2-(2,6-dioxopiperidin-3-yl)-1-oxoisoindol-5-yl)methyl)carbamoyl)pyrimidine-2-yl)piperazin-1-yl)oxononylamino)phenyl)thio)pyrazin-2-yl)-4-methylpyridin-4-yl)carbamate (**48-6**) (30.3 mg, 0.03 mmol) was dissolved in 3 mL DCM and 1 mL TFA, and then the solution was stirred and reacted at room temperature for 3 h. After completion of the reaction, the solvent was removed by evaporation under reduced pressure, and to the residue, was added 3 mL of methanol. The pH of the resultant solution was adjusted to about 8 with NaHCOs solution. The insoluble substances were filtered out. After methanol was removed by evaporation under reduced pressure, the obtained solid was dissolved in a solution of dichloromethane/methanol (10:1, 5 mL). The insoluble substances were removed by filtration, and the solvent was evaporated under reduced pressure, to obtain 21 mg of compound **48**, with a yield of 76.3%. MS: *m*/*z* 917 [M+H]⁺.

### Example 12 Synthesis of compound (E)-N-(3-((5-(4-amino-4-methylpyridin-1-yl)pyrazin-2-yl)thio)phenyl)-8-(6-(4-((2-(2,6-dioxopiperidin-3-yl)-1-oxoisoindol-5-yl)methyl)amino)-4-oxobutyryl-2-ene)-2,6-diazaspiro[3.3]heptan-2-yl)octylamide (114):

### Synthesis of intermediate tert-butyl (E)-6-(4-methoxy-4-oxobutyryl-2-ene)-2,6-diazaspiro[3.3]heptan-2-carboxylate (114-1):

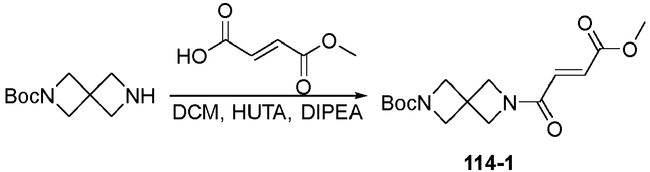

Tert-butyl 2,6-diazaspiro[3.3]heptan-2-carboxylate (198.3 mg, 1 mmol) and (E)-4-methoxy-4-oxobutyl-2-en-1-carboxylic acid (130.1 mg, 1 mmol) were dissolved in 5 mL of dichloromethane, to which were added HATU (570 mg, 1.5 mmol) and DIPEA (258 mg, 2 mmol). The mixture was stirred at room temperature for 12 h. After completion of the reaction, purification by TLC provided 250 mg of intermediate **114-1**, with a yield of 80.6%. MS: *m*/*z* 311 [M+H]⁺.

### Synthsis of intermediate methyl (E)-4-oxo-4-(2,6-diazaspiro[3.3]heptan-2-yl)butan-2-en-1-carboxylate (114-2):

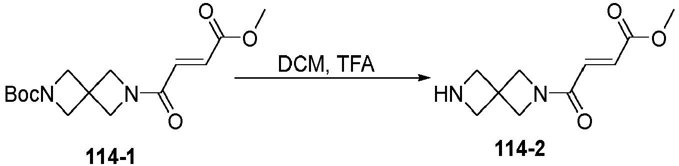

Tert-butyl (E)-6-(4-methoxy-4-oxobutan-2-ene)-2,6-diazaspiro[3.3]heptan-2-carboxylate (intermediate **114-1)** (248 mg, 0.8 mmol) were dissolved in 10 mL DCM and 5 mL TFA, and then the mixture was stirred and reacted at room temperature for 3h. After completion, to the reaction solution, was addd saturated NaHCOs aqueous solution, and the resultant solution was extracted with dichloromethane. The organic phase was dried with anhydrous sodium sulfate, and evaporated under reduced pressure to remove the solvent, and obtain 145 mg of intermediate **114-2**, with a yield of 86.2%. MS: *m*/*z* 211 [M+H]⁺.

### Synthesis of intermediate tert-butyl (1-(5-((3-(8-bromooctamide)phenyl)thio)pyrazin-2-yl)-4-methylpyridin-4-yl)carbamate (114-3):

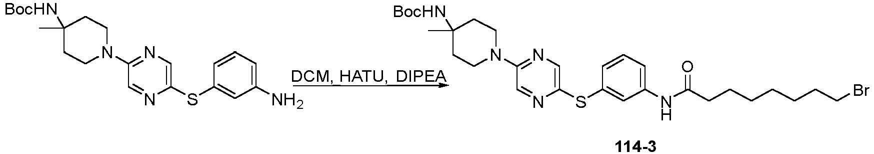

8-bromooctanoic acid (111.5 mg, 0.5 mmol) and tert-butyl (1-(5-((3-aminophenyl)thio)pyrazin-2-yl)-4-methylpyridin-4-yl)carbamate (207.8 mg, 0.5 mmol) were dissolved in 5 mL of dichloromethane, to which were added HATU (285 mg, 0.75 mmol) and DIPEA (129 mg, 1 mmol). The mixture was stirred at room temperature for 12 h. After completion of the reaction, purification by TLC provided 210 mg of intermediate **114-3**, with a yield of 67.7%. MS: *m*/*z* 620 [M+H]⁺.

### Synthesis of intermediate methyl (E)-4-(6-(8-((3-((5-(4-((tert-butoxycarbonyl)amino)-4-methylpyridin-1-yl)pyrazin-2-yl)thio)phenyl)amino)-8-oxyoctyl)-2,6-diazaspiro[3.3]heptan-2-yl)-4-oxo-2-en-1-carboxylate (114-4):

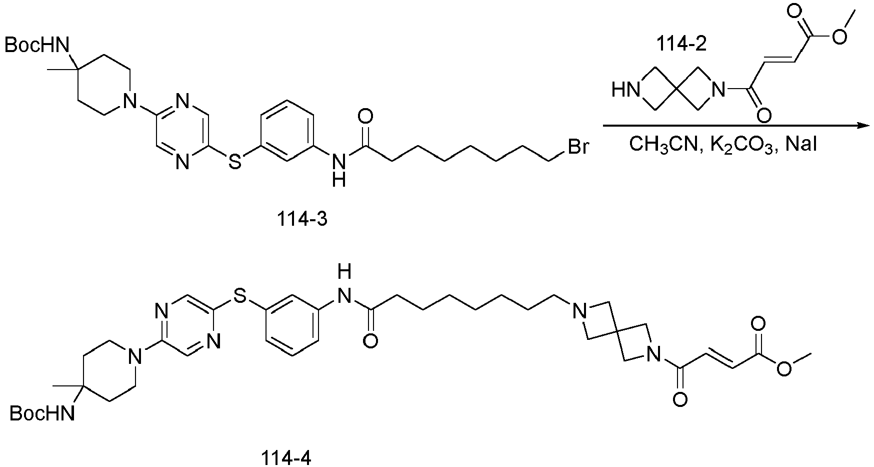

Tert-butyl (1-(5-((3-(8-bromooctamide)phenyl)thio)pyrazin-2-yl)-4-methylpyridin-4-yl)carbamate (intermediate **114-3**) (124 mg, 0.2 mmol) and methyl (E)-4-oxo-4-(2,6-diazaspiro[3.3]heptan-2-yl)butan-2-en-1-carboxylate (intermediate **114-2**) (42 mg, 0.2 mmol) were dissolved in 10 mL of acetonitrile, to which were added K₂CO₃ (138 mg, 1 mmol) and NaI (3 mg, 0.02 mmol). The mixture was stirred at 80 °C for 12 h, and then cooled. The pH of the resultant solution was adjusted to around 6 with 0.5 N hydrochloric acid. The solution was extracted three times with ethyl acetate. The organic layer was dried with anhydrous sodium sulfate and concentrated. Purification by column chromatography provided 115 mg of intermediate **114-4**, with a yield of 76.7%. MS: *m*/*z* 750 [M+H]⁺.

**Sythesis of intermediate (E)-4-(6-(8-((3-((5-(4-((tert-butoxycarbonyl)amino)-4-methylpyridin-1-yl)pyrazin-2-yl)thio)phenyl)amino)-8-oxyoctyl)-2,6-diazaspiro[3.3]heptan-2-yl)-4-oxobutan-2-en-1-carboxylic acid (intermediate 114-5):**

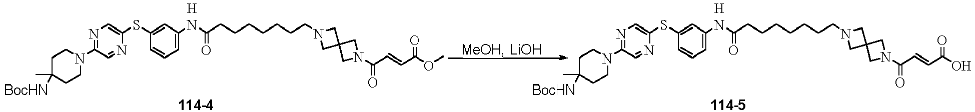

Methyl (E)-4-(6-(8-((3-((5-(4-((tert-butoxycarbonyl)amino)-4-methylpyridin-1-yl)pyrazin-2-yl)thio)phenyl)amino)-8-oxyoctyl)-2,6-diazaspiro[3.3]heptan-2-yl)-4-oxobutan-2-en-1-carboxylate (intermediate **114-4**) (112 mg, 0.15 mmol) was dissolved in methanol (3 mL), to which was added lithium hydroxide monohydrate (126 mg, 3 mmol), and the mixture was stirred at room temperature for 3 h. After completion of the reaction, the pH of the reaction solution was adjusted to about 6 with 0.5 N of hydrochloric acid, and then the solution was diluted with dichloromethane. After standing, the organic layer was separated, and the water layer was extracted with dichloromethane. The organic layers were combined, dried with anhydrous sodium sulfate, and concentrated. The residue was purified by TLC to obtain 95 mg of intermediate **114-5**, with a yield of 86%. MS: *m*/*z* 736 [M+H]⁺.

### Synthesis of intermediate tert-butyl (E)-(1-(5-((3-(8-(6-(4-((2-(2,6-dioxopiperidin-3-yl)-1-oxoisoquinolin-5-yl)methyl)amino)-4-oxobutan-2-en-1-carbonyl)-2,6-diazaspiro[3.3]heptan-2-yl)octamido)phenyl)thio)pyrazin-2-yl)-4-methylpyridin-4-yl)carbamate (114-6):

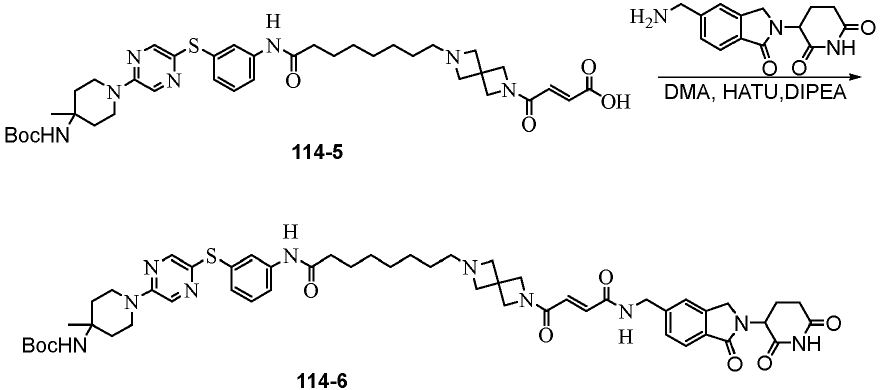

(E)-4-(6-(8-((3-((5-(4-((tert-butoxycarbonyl)amino)-4-methylpyridin-1-yl)pyrazin-2-yl)thio)phenyl)amino)-8-oxyoctyl)-2,6-diazaspiro[3.3]heptan-2-yl)-4-oxo-2-en-1-carboxylic acid (intermediate **114-5**) (38 mg, 0.05 mmol) and 3-(5-(aminomethyl)-1-oxoisoindol-2-yl)piperidin-2,6-dione (13.7 mg, 0.05 mmol) were dissolved in 0.5 mL of DMA, to which were added HATU (28.5 mg, 0.075 mmol) and DIPEA(12.9 mg, 0.1 mmol). The mixture was stirred at room temperature for 12 h. After completion of the reaction, purification by TLC provided 37 mg of intermediate **114-6**, with a yield of 74.7%. MS: *m*/*z* 991 [M+H]⁺.

### Synthesis of compound (E)-N-(3-((5-(4-amino-4-methylpyridin-1-yl)pyrazin-2-yl)thio)phenyl)-8-(6-(4-((2-(2,6-dioxopiperidin-3-yl)-1-oxoisoindol-5-yl)methyl)amino)-4-oxobutan-2-en-1-carbonyl)-2,6-diazaspiro[3.3]heptan-2-yl)octylamide (compound 114):

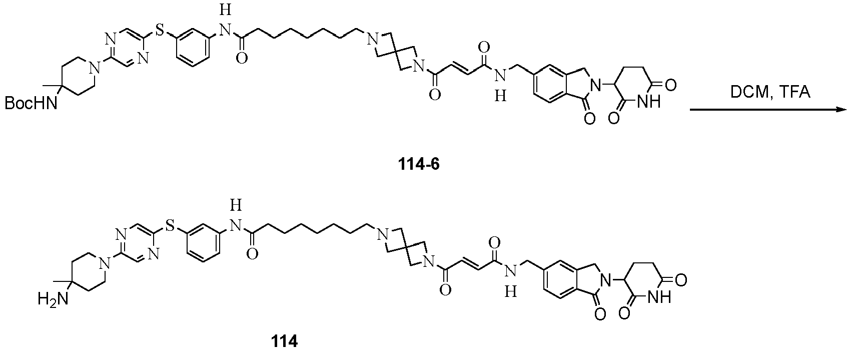

Tert-butyl ((E)-(1-(5-((3-(8-(6-(4-((2-(2,6-dioxopiperidin-3-yl)-1-oxoisoquinolin-5-yl)methyl)amino)-4-oxobutan-2-en-1-carbonyl)-2,6-diazaspiro[3.3]heptan-2-yl)octamido)phenyl)thio)pyrazin-2-yl)-4-methylpyridin-4-yl)carbamate (intermediate **114-6**) (29.7 mg, 0.03 mmol) was dissolved in 3 mL DCM and 1 mL TFA, and then the solution was stirred and reacted at room temperature for 3 h. After completion of the reaction, the solvent was removed by evaporation under reduced pressure, and to the residue, was added 3 mL of methanol. The pH of the resultant solution was adjusted to about 8 with NaHCOs solution. The insoluble substances were filtered out. After methanol was removed by evaporation under reduced pressure, the obtained solid was dissolved in a solution of dichloromethane/methanol (10:1, 5 mL). The insoluble substances were removed by filtration, and the solvent was evaporated under reduced pressure, to obtain 22 mg of compound **114**, with a yield of 82.3%. MS: *m*/*z* 891 [M+H]⁺.

### Example 13 Synthesis of compound 4-(2-(7-((3-((5-(4-amino-4-methylpyridin-1-yl)pyrazin-2-yl)thio)phenyl)amino)-7-oxoheptyl)-2,6-diazaspiro[3.4]octan6-yl)-N-((2-(2,6-dioxopiperidin-3-yl)-1-oxoisoindol-5-yl)methyl)cyclohexane-1-formamide (158):

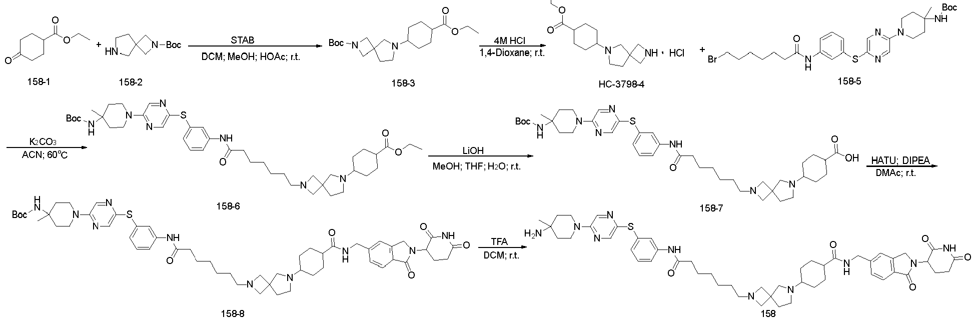

### Synthesis of intermediate tert-butyl-6-(4-(ethoxycarbonyl)cyclohexyl)-2,6-diazaspiro[3.4]octan-2-formic acid ethyl ester (158-3):

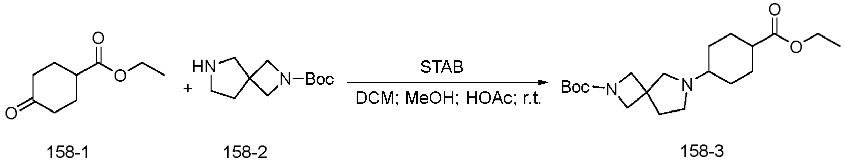

Ethyl 4-oxocyclohexane-1-formate (200 mg, 1.176 mmol) and tert-butyl 2,6-diazaspiro[3.4]octan-6-formate (274 mg, 1.294 mmol) were dissolved in DCM/MeOH (10:1, 5 ml), to which was added one drop of acetic acid, and then the mixture was stirred and reacted at room temperature for 1 h. Then, sodium triacetoxyborohydride (498 mg, 2.353 mmol) was added, and then the mixture was stirred for 3 h. The reaction solution was sequentially washed with hydrochloric acid (0.5 N, 10 ml) and saturated brine. Then, the organic phase was separated, dried with anhydrous sodium sulfate, and concentrated to obtain 400 mg of crude product (158-3), with a yield of 92.8%. MS: *m*/*z* 367 [M+H]⁺.

### Synthesis of intermediate ethyl-4-(2,6-diazaspiro[3.4]octan-6-yl)cyclohexane-1-formate hydrochloride (158-4):

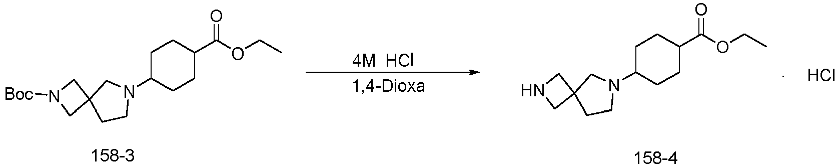

**158-3** (400 mg, 1.092 mmol) was dissolved in a solution of HCl in 1,4-dioxane (4 M, 10 ml), and then the mixture was allowed to react at room temperature for 1h. The reaction solution was concentrated to obtain 315 mg of product (**158-4**), with a yield of 95.45%. MS: *m*/*z* 267 [M+H]⁺.

### Synthesis of intermediate tert-butyl (1-(5-((3-(7-bromoheptanamide)phenyl)thio) pyrazin-2-yl)4-methylpyridin-4-yl)carbamate (158-5)

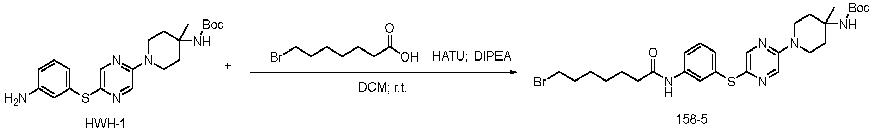

Intermediate tert-butyl (1-(5-((3-aminophenyl)thio)pyrazin-2-yl)-4-methylpyridin-4-yl)carbamate (2 g, 4.81 mmol), 7-bromohexanoic acid (838 mg, 4.01 mmol), HATU (1.83 g, 4.816 mmol) and *N*,*N*-diisopropylethylamine (1.04 g, 8.06 mmol) were dissolved in dichloromethane (20 ml); and then the mixture was reacted under stirring at room temperature for 2 h. The reaction solution was successively washed once with water, saturated NaHCOs aqueous solution, and saturated brine, and then the reaction solution was separated. The organic layer was dried over anhydrous Na₂SO₄, and then concentrated, to obtain 2.3 g of product (**158-5**), with a yield of 79.3%. MS: *m*/*z* 606 [M+H]⁺; 608 [M+2+H]⁺.

### Synthesis of intermediate ethyl-4-(2-(7-((3-((5-(4-((3-T oxy)amino)-4-methylpyridin-1-yl)pyrazin-2-yl)thio)phenyl)amino)-7-oxoheptyl)-2,6-diazaspiro [3.4] octan-6-yl) cyclohexane-1-formate (158-6):

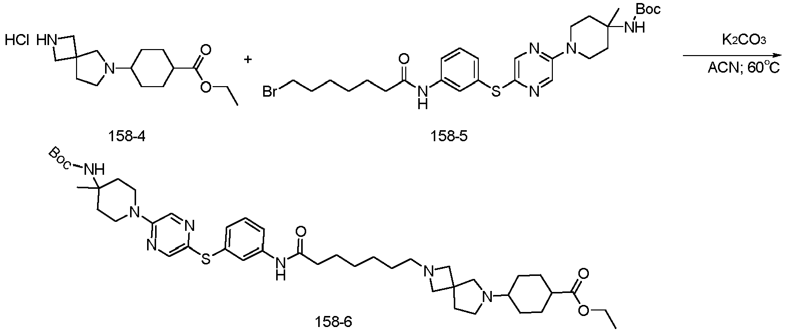

**158-4** (120 mg, 0.397 mmol), **158-5** (264 mg, 0.436 mmol), and K₂CO₃ (164 mg, 1.192 mmol) were added into acetonitrile (5 ml), and then the mixture was stirred overnight at 60 °C. After cooling to room temperature, the reaction solution was poured into water, extracted with ethyl acetate. The organic phase was separated, successively washed with hydrochloric acid (1N) and saturated brine, dried with anhydrous sodium sulfate, and concentrated. The residue was purified by column chromatography to obtain 77 mg of product (intermediate for **158-6** and isomer **159**), with a yield of 24.5%. MS: *m*/*z* 792 [M+H]⁺.

### Synthesis of intermediate 4-(2-(7-((3-((5-(4-((tert-butoxycarbonyl)amino)-4-methylpyridin-1-yl)pyrazin-2-yl)thio)phenyl)amino)-7-oxoheptyl)-2,6-diazaspiro[3.4]octan-6-yl)cyclohexane-1-formic acid (158-7):

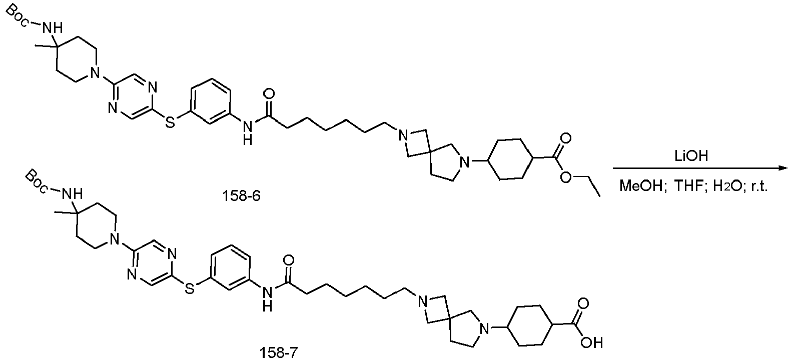

**158-6** (77 mg, 0.101 mmol) and lithium hydroxide monohydrate (17 mg, 0.404 mmol) were added into a mixed solvent of tetrahydrofuran (4 ml), methanol (1 ml) and water (2 ml), and then the mixture was allowed to react overnight at room temperature. The pH of the reaction solution was adjusted to 3-4 with hydrochloric acid (0.5 N). The resultant solution was extracted with ethyl acetate, washed with saturated brine, dried with anhydrous sodium sulfate, and concentrated, to obtain 70 mg of crude product (**158-7**), with a yield of 94.2%. MS: *m*/*z* 764 [M+H]⁺.

### Synthesis of intermediate tert-butyl (1-(5-((3-(7-(6-(4-(((2-(2,6-dioxopiperidin-3-yl)-1-oxoisoindol-5-yl)methyl)aminoformyl)cyclohexyl)-2,6-diazaspiro[3.4]octan-2-yl)heptanamido)phenyl)thio)pyrazin-2-yl)-4-methylpyridin-4-yl)carbamate (158-8):

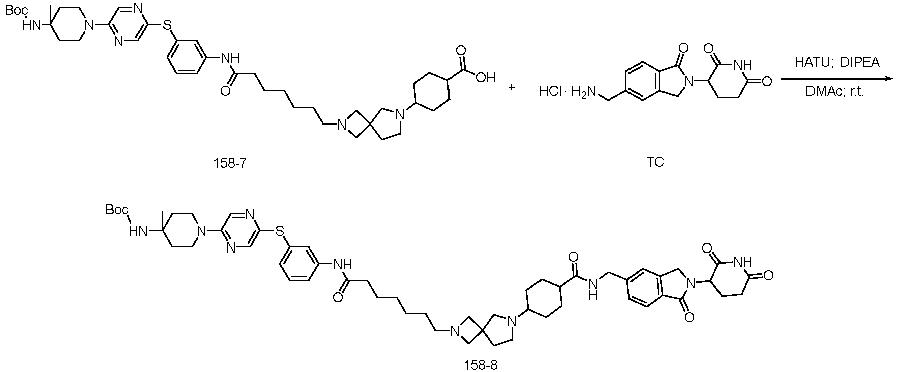

Intermediate **158-7** (70 mg, 0.0917 mmol), 3-(5-(aminomethyl)-1-oxoisoindol-2-yl)piperidin-2,6-dionehydrochloride (28 mg, 0.0917 mmol), HATU (42 mg, 0.11 mmol), and *N,N*-diisopropylethylamine (23 mg, 0.185 mmol) were dissolved in *N*,*N*-dimethylacetamide (1 ml), and then the mixture was stirred overnight at room temperature. Purification by pre-TLC afforded 30 mg of product (**158-8**), with a yield of 32.13%. MS: *m*/*z* 460.7 ((M-100)/2+H⁺).

### Synthesis of compound 4-(2-(7-((3-((5-(4-amino-4-methylpyridin-1-yl)pyrazin-2-yl)thio)phenyl)amino)-7-oxoheptyl)-2,6-diazaspiro[3.4]octan6-yl)-N-((2-(2,6-dioxopiperidin-3-yl)-1-oxoisoindol-5-yl)methyl)cyclohexane-1-formamide (158):

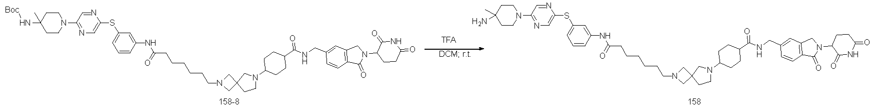

**158-8** (30 mg, 0.029 mmol) was dissolved in dichloromethane (3 mL), to which was added trifluoroacetic acid (1 ml), and then the mixture was stirred 1h at room temperature. The reaction solution was rotatory evaporated, and then the residue was dissolved in dichloromethane, followed by evaporation, that was repeated twice. To the residue, was added anhydrous methanol (4 ml), and the pH was adjusted to 7-8 with NaHCOs. The resultant solution was filtered and concentrated, to obtain 25 mg of product (**158**), with a yield of 92.6%. MS: *m*/*z* 460.7(M/2+H⁺). ¹H NMR (400 MHz, DMSO-*d*₆) *δ* 11.00 (s, 1H), 10.05 (s, 1H), 8.62 - 8.02 (m, 5H), 7.65 (s, 1H), 7.57 (s, 1H), 7.44 (d, *J* = 13.6 Hz, 2H), 7.34 (d, *J* = 8.2 Hz, 1H), 7.25 - 7.17 (m, 1H), 6.88 (d, *J* = 7.8 Hz, 1H), 5.35 - 5.05 (m, 1H), 4.89 - 4.69 (m, 1H), 4.57 (d, *J* = 17.7 Hz, 1H), 4.46 - 4.39 (m, 1H), 4.35 (d, *J* = 5.8 Hz, 2H), 4.19 (d, *J* = 6.8 Hz, 2H), 4.09 - 3.81 (m, 6H), 3.08 - 2.95 (m, 3H), 2.88 (s, 1H), 2.31 - 2.22 (m, 4H), 2.21 - 2.14 (m, 1H), 2.04 - 1.95 (m, 2H), 1.87 (d, *J* = 9.2 Hz, 2H), 1.75 (dd, *J* = 10.4, 5.0 Hz, 7H), 1.52 (d, *J* = 6.9 Hz, 4H), 1.37 (s, 8H), 1.25 (d, *J* = 5.9 Hz, 6H).

### Example 14 Synthesis of compound 6-(1-(1-(4-((3-((5-(4-amino-4-methylpyridin-1-yl)pyrazin-2-yl)thio)phenyl)carbamoyl)phenyl)piperidin-4-yl)-1,2,3,6-tetrahydropyridin-4-yl)-N-((2-(2,6-dioxopiperidin-3-yl)-1-oxoisoquinolin-5-yl)methyl)pyridazin-3-formamide (264):

### Synthesis of intermediate tert-butyl (4-methyl-1-(5-((3-(4-(4-oxopiperidin-1-yl)benzamido)phenyl)thio)pyrazin-2-yl)piperidin-4-yl)carbamate (264-2):

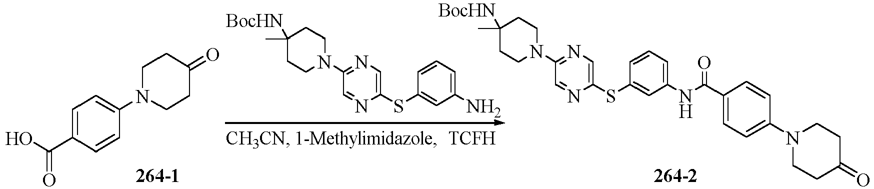

4-(4-oxopiperidin-1-yl)benzoic acid (intermediate **264-1**) (658 mg, 3 mmol) and tert-butyl (1-(5-((3-aminophenyl)thio)pyrazin-2-yl)-4-methylpyridin-4-yl)carbamate (1.25 g, 3 mmol) were dissolved in 30 mL of acetonitrile, to which were added N-methylimidazole (739 mg, 9 mmol) and TCFH (1.09 g, 3.9 mmol), and then the mixture was stirred 3h at room temperature. After the reaction was completed, the solvent was removed by evaporation under reduced pressure, and then 300 mL of dichloromethane was added, followed by washing twice with saturated brine. The organic phase was evaporated under reduced pressure to remove the solvent, and the residue was purified by column chromatography (mobile phase: PE:EA=1:1) to obtain 1.57 g of intermediate **264-2**, with a yield of 85%. MS: *m*/*z* 617 [M+H]⁺.

### Synthesis of intermediate ethyl 6-(1-(1-(4-((3-((5-(4-((tert-butoxycarbonyl)amino)-4-methylpyridin-1-yl)pyrazin-2-yl)thio)phenyl)carbamoyl)phenyl)piperidin-4-yl)-1,2,3,6-tetrahydropyridin-4-yl)pyridazin-3-carboxylate (264-3):

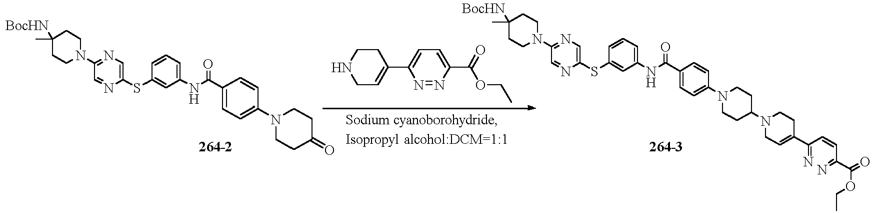

Tert-butyl (4-methyl-1-(5-((3-(4-(4-oxopiperidin-1-yl)benzamido)phenyl)thio)pyrazin-2-yl)piperidin-4-yl)carbamate (**264-2**) (1.54 g, 2.5 mmol) and ethyl 6-(1,2,3,6-tetrahydropyridin-4-yl)pyridazin-3-carboxylate (0.54 g, 2.5 mmol) were dissolved in a mixed solvent of isopropanol and dichloromethane (v:v = 1: 1). The pH value of the solution was adjusted to about 5 with acetic acid, to which was added sodium cyanoborohydride (0.39 g, 6.25 mmol), and then the solution was stirred at room temperature for 12 h. Once completion of the reaction, dichloromethane was added, and after standing, the organic layer was separated. The water layer was extracted with dichloromethane. The organic layers were combined, dried with anhydrous sodium sulfate, and concentrated. The residue was subjected to column chromatography (mobile phase DCM: MeOH = 20:1), to obtain 1.5 g of intermediate **264-3**, with a yield of 72%. MS: *m*/*z* 834 [M+H]⁺.

### Synthsis of intermediate 6-(1-(1-(4-((3-((5-(4-((tert-butoxycarbonyl)amino)-4-methylpyridin-1-yl)pyrazin-2-yl)thio)phenyl)carbamoyl)phenyl)piperidin-4-yl)-1,2,3,6-tetrahydropyridin-4-yl)pyridazin-3-carboxylic acid (264-4):

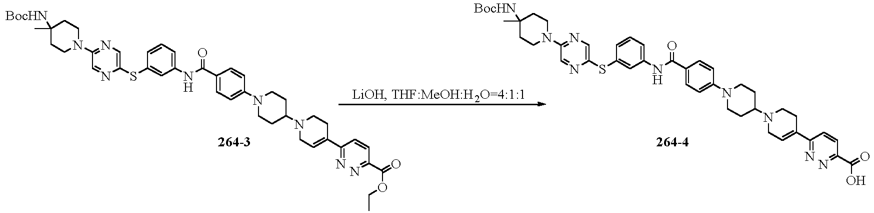

Ethyl (6-(1 -(1 -(4-((3 -((5-(4-((tert-butoxycarbonyl)amino)-4-methylpyridin-1-yl)pyrazin-2-yl)thio)phenyl)carbamoyl)phenyl)piperidin-4-yl)-1,2,3,6-tetrahydropyridin-4-yl)pyridazin-3-carboxylate (intermediate **264-3**) (1.5 g, 1.8 mmol) was dissolved in a mixed solvent of tetrahydrofuran, methanol and water (v:v:v =4:1:1), to which was added lithium hydroxide monohydrate (1.5 g, 36 mmol), and the mixture was stirred at room temperature for 3 h. After completion of the reaction, the pH of the reaction solution was adjusted to about 4 with 0.5 N of hydrochloric acid, and white solid precipitated, which was filtered to remove solvent. Then, the solid was dried, to obtain 1.25 g of intermediate **264-4**, with a yield of 86%. MS: *m*/*z* 806 [M+H]⁺.

### Synthesis of intermediate tert-butyl (1-(5-((3-(4-(4-(4-(6-((2-(2,6-dioxopiperidin-3-yl)-1-oxoisoindol-5-yl)methyl)carbamoyl)pyridazin-3-yl)-3,6-dihydropyridin-1(2H)-yl)piperidin-1-yl)benzamido)phenyl)thio)pyrazin-2-yl)-4-methylpyridin-4-yl)carbamate (264-5):

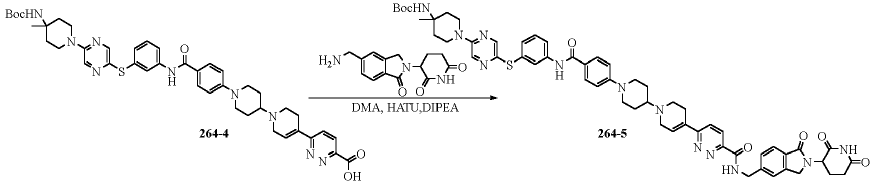

6-(1-(1-(4-((3-((5-(4-((tert-butoxycarbonyl)amino)-4-methylpyridin-1-yl)pyrazin-2-yl)thio)phenyl)carbamoyl)phenyl)piperidin-4-yl)-1,2,3,6-tetrahydropyridin-4-yl)pyridazin-3-carboxylic acid (intermediate **264-4**) (1.21 g, 1.5 mmol) and 3-(5-(aminomethyl)-1-oxoisoindol-2-yl)piperidin-2,6-dione (0.41 g, 1.5 mmol) were dissolved in 10 mL of DMA, to which were added HATU (0.86 g, 2.25 mmol) and DIPEA (0.39 g, 3 mmol), and then the mixture was stirred at room temperature for 12 h. After completion of the reaction, purification by column chromatography (mobile phase DCM:MeOH = 20: 1) afforded 1.15 g of intermediate **264-5**, with a yield of 72%. MS: *m*/*z* 1061 [M+H]⁺.

### Synthesis of compound 6-(1-(1-(4-((3-((5-(4-amino-4-methylpyridin-1-yl)pyrazin-2-yl)thio)phenyl)carbamoyl)phenyl)piperidin-4-yl)-1,2,3,6-tetrahydropyridin-4-yl)-N-((2-(2,6-dioxopiperidin-3-yl)-1-oxoisoquinolin-5-yl)methyl)pyridazin-3-formamide (compound 264):

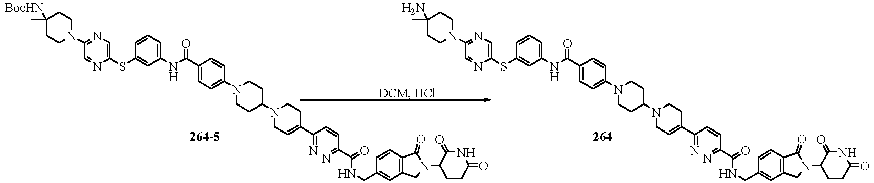

Tert-butyl (1-(5-((3-(4-(4-(4-(6-((2-(2,6-dioxopiperidin-3-yl)-1-oxoisoindol-5-yl)methyl)carbamoyl)pyridazin-3-yl)-3,6-dihydropyridin-1(2H)-yl)piperidin-1-yl)benzamido)phenyl)thio)pyrazin-2-yl)-4-methylpyridin-4-yl)carbamate (intermediate **264-5**) (600 mg, 0.57 mmol) was dissolved in 800 mL of DCM, and under stirring, dry HCl gas was introduced, and then the mixture was stirred for 1 h. After the reaction was completed, the solvent was removed by evaporation under reduced pressure, to obtain 520 mg of compound **264** with a yield of 95.8%. MS: *m*/*z* 962 [M+H]⁺.

¹H NMR (400 MHz, DMSO-*d*₆) *δ* 11.45 (s, 1H), 10.98 (s, 1H), 10.11 - 9.90 (m, 2H), 8.49 - 8.15 (m, 5H), 7.98 - 7.47 (m, 7H), 7.42 - 6.87 (m, 5H), 5.10 (dd, *J* = 13.4, 5.1 Hz, 1H), 4.62 (d, *J* = 6.3 Hz, 2H), 4.48 - 4.26 (m, 3H), 3.80 (s, 2H), 3.68 (d, *J* = 11.3 Hz, 2H), 3.39 (s, 1H), 3.24 (s, 1H), 2.99 (s, 1H), 2.94 - 2.74 (m, 4H), 2.59 (d, *J* = 17.0 Hz, 2H), 2.41 - 2.31 (m, 2H), 2.23 (s, 1H), 1.98 (s, 3H), 1.77 (d, *J* = 19.0 Hz, 4H), 1.37 (s, 3H), 1.20 (d, *J* = 22.9 Hz, 3H).

### Example 15 Synthesis of compound 1'-(4-((3-((5-(4-amino-4-methylpyridin-1-yl)pyrazin-2-yl)thio)phenyl)carbamoyl)phenyl)piperidin-4-yl)-N-((2-(2,6-dioxopiperidin-3-yl)-1-oxoisoindol-5-yl)methyl)-1',2',3',6'-tetrahydro-[3,4'-bipyridine]-6-formamide (312):

### Synthesis of intermediate methyl 1'-(1-(4-((3-((5-(4-((tert-butoxycarbonyl)amino)-4-methylpyridin-1-yl)pyrazin-2-yl)thio)phenyl)carbamoyl)phenyl)piperidin-4-yl)-1¹,2¹,3¹,6¹-tetrahydro-[3,4'-bipyridine]-6-carboxylate (312-1):

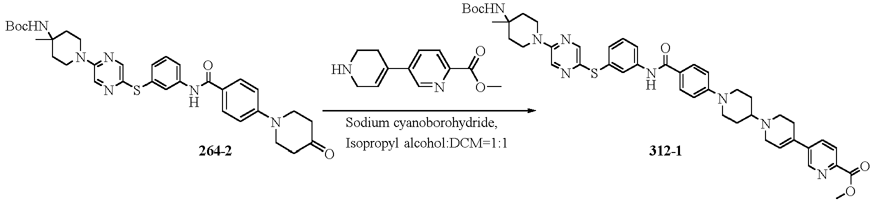

Tert-butyl (4-methyl-1-(5-((3-(4-(4-oxopiperidin-1-yl)benzamido)phenyl)thio)pyrazin-2-yl)piperidin-4-yl)carbamate (**264-2**) (1.54 g, 2.5 mmol) and methyl 1',2',3',6'-tetrahydro-[3,4'-bipyridine]-6-carboxylate (0.55 g, 2.5 mmol) were dissolved in a mixed solvent of isopropanol and dichloromethane (v:v=1:1). The pH value of the solution was adjusted to about 5 with acetic acid, to which was added sodium cyanoborohydride (0.39 g, 6.25 mmol), and then the solution was stirred at room temperature for 12 h. Once completion of the reaction, dichloromethane was added, and after standing, the organic layer was separated. The water layer was extracted with dichloromethane. The organic layers were combined, dried with anhydrous sodium sulfate, and concentrated. The residue was subjected to column chromatography (mobile phase DCM: MeOH = 20:1), to obtain 1.38 g of intermediate **312-1**, with a yield of 67%. MS: *m*/*z* 819 [M+H]⁺.

### Synthesis of intermediate 1'-(1-(4-((3-((5-(4-((tert-butoxycarbonyl)amino)-4-methylpyridin-1-yl)pyrazin-2-yl)thio)phenyl)carbamoyl)phenyl)piperidin-4-yl)-1',2',3',6'-tetrahydro-[3,4'-bipyridine]-6-carboxylic acid (312-2):

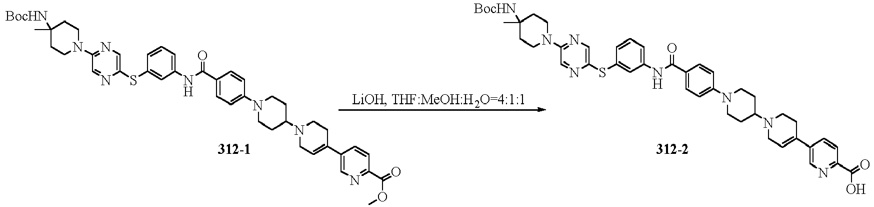

Methyl 1'-(1-(4-((3-((5-(4-((tert-butoxycarbonyl)amino)-4-methylpyridin-1-yl)pyrazin-2-yl)thio)phenyl)carbamoyl)phenyl)pipeiidin-4-yl)-1',2',3',6'-tetrahydro-[3,4'-bipyridine]-6-carboxylate (**312-1**) (1.3 g, 1.6 mmol) was dissolved in a mixed solvent of tetrahydrofuran, methanol and water (v:v:v = 4:1:1), to which was added lithium hydroxide monohydrate (1.3 g, 32 mmol), and the mixture was stirred at room temperature for 3 h. After completion of the reaction, the pH of the reaction solution was adjusted to about 4 with 0.5 N of hydrochloric acid, and then the solution was extracted with a mixed solvent of dichloromethane and methanol (v:v=10:1). The organic phase was dried with anhydrous Na₂SO₄, and then, concentrated under reduced pressure, to obtain 1.1 g of intermediate **312-2**, with a yield of 85%. MS: *m*/*z* 805 [M+H]⁺.

### Synthesis of intermediate tert-butyl (1-(5-((3-(4-(4-(6-((2-(2,6-dioxopiperidin-3-yl)-1-oxoisoindol-5-yl)methyl)carbamoyl)-3',6'-dihydro-[3,4'-bipyridine]-1'(2'H)-yl)piperidin-1-yl)benzamido)phenyl)thio)pyrazin-2-yl)-4-methylpyridin-4-yl)carbamate (312-3):

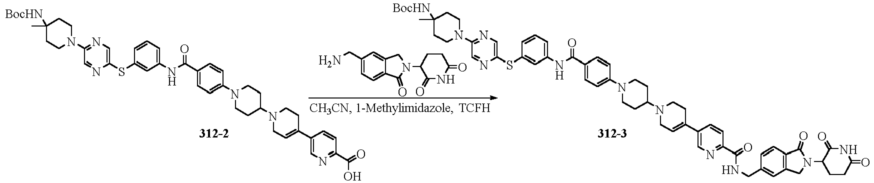

1'-(1-(4-((3-((5-(4-((tert-butoxycarbonyl)amino)-4-methylpyridin-1-yl)pyrazin-2-yl)thio)phenyl)carbamoyl)phenyl)piperidin-4-yl)-1',2',3',6'-tetrahydro-[3,4'-bipyridine]-6-carboxylic acid (intermediate **312-2**) (1.05 g, 1.3 mmol) and 3-(5-(aminomethyl)-1-oxoisoindol-2-yl)piperidin-2,6-dione (0.36 g, 1.3 mmol) were dissolved in 10 mL of DMA, to which were added N-methylimidazole (320 mg, 3.9 mmol) and TCFH (477 mg, 1.7 mmol), and the mixture was stirred at room temperature for 3 h. After the reaction was completed, the reaction solution was dropped into water, and solid was precipitated, which was filtered. The obtained solid was purified by column chromatography (mobile phase DCM:MeOH = 10:1) to obtain 1.45 g of intermediate **312-3**, with a yield of 81%. MS: *m*/*z* 1060 [M+H]⁺.

### Synthesis of 1'-(4-((3-((5-(4-amino-4-methylpyridin-1-yl)pyrazin-2-yl)thio)phenyl) carbamoyl)phenyl)piperidin-4-yl)-N-((2-(2,6-dioxopiperidin-3-yl)-1-oxoisoindol-5-yl) methyl)-1',2',3',6'-tetrahydro-[3,4'-bipyridine]-6-formamide(312):

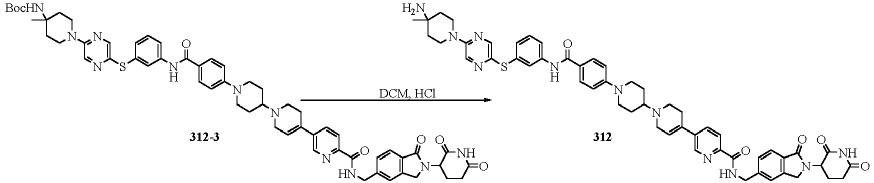

Tert-butyl (1-(5-((3-(4-(4-(6-((2-(2,6-dioxopiperidin-3-yl)-1-oxoisoindol-5-yl)methyl) carbamoyl)-3',6'-dihydro-[3,4'-bipyridine]-1'(2'H)-yl)piperidin-1-yl)benzamido)phenyl)thio) pyrazin-2-yl)-4-methylpyridin-4-yl)carbamate (intermediate **312-3**) (600 mg, 0.57 mmol) was dissolved in 800 mL of DCM, and under stirring, dry HCl gas was introduced, and then the mixture was stirred for 1 h. After the reaction was completed, the solvent was removed by evaporation under reduced pressure, to obtain 510 mg of compound **312**, with a yield of 94%. MS: *m*/*z* 960 [M+H]⁺.

¹H NMR (400 MHz, DMSO-*d*₆) *δ* 11.35 (s, 1H), 10.96 (s, 1H), 10.15 - 9.95 (m, 2H), 8.46 - 8.14 (m, 6H), 7.96 - 7.45 (m, 7H), 7.41 - 6.89 (m, 5H), 5.10 (dd, *J* = 13.4, 5.1 Hz, 1H), 4.62 (d, *J* = 6.3 Hz, 2H), 4.48 - 4.26 (m, 3H), 3.80 (s, 2H), 3.68 (d, *J* = 11.3 Hz, 2H), 3.39 (s, 1H), 3.24 (s, 1H), 2.99 (s, 1H), 2.94 - 2.74 (m, 4H), 2.59 (d, *J* = 17.0 Hz, 2H), 2.41 - 2.31 (m, 2H), 2.23 (s, 1H), 1.98 (s, 3H), 1.77 (d, *J* = 19.0 Hz, 4H), 1.37 (s, 3H), 1.20 (d, *J* = 22.9 Hz, 3H).

### Example 16 Synthesis of compound 1'-(1-(4-((3-((5-(4-amino-4-methylpyridin-1-yl)pyrazin-2-yl)thio)phenyl)aminoformyl)-2-fluorophenyl)piperidin-4-yl)-N-((2-(2,6-dioxopiperidin-3-yl)-1-oxoisoindolin-5-yl)methyl)-1',2',3',6'-tetrahydro-[3,4'-bipyridine]-6-formamide hydrochloride (295)

### Synthesis of intermediate ethyl 3-fluoro-4-(1,4-dioxa-8-azaspiro[4.5]decan-8-yl)benzoate (295-3)

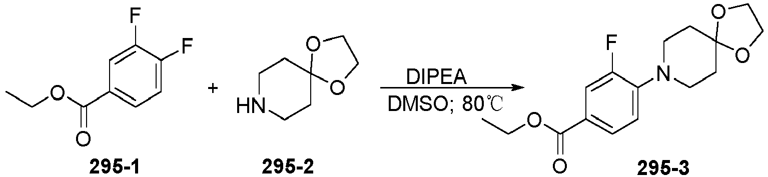

Intermediates **295-1** (930 mg, 5mmol), **295-2** (787 mg, 5.5mmol), and DIPEA(1290 mg, 10 mmol) were successively added to a single-necked flask containing DMSO (10 mL), and then the mixture was stirred at 80 °C for 12 h. After completion of the reaction, the solution was cooled and then added into water dropwise. The resultant solution was extracted with ethyl acetate (10 mL × 3). The organic phase was washed with saturated brine, dried with anhydrous sodium sulfate, and rotatory evaporated, to obtain 1 g of intermediate compound **295-3.**

**intermediate 3-fluoro-4-(1,4-dioxa-8-azaspiro[4.5]decan-8-yl)benzoic acid (295-4)**

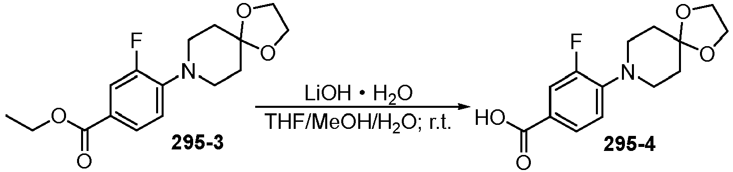 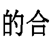

The intermediate compound obtained in the previous step was dissolved in THF/MeOH/H₂O (12 mL/3 mL/3 mL), to which was added LiOH (840 mg, 20 mmol), and then the mixture was stirred at room temperature for 1h. The reaction was detected by TLC. After completion of the reaction, the pH value of the solution was adjusted to 2 with HCl (1N). The resultant solution was extracted with ethyl acetate. The organic phase was dried with anhydrous sodium sulfate, and then rotatory evaporated, to obtain 920 mg of intermediate compound **295-4.**

### Synthesis of intermediate 3-fluoro-4-(4-oxopiperidin-1-yl)benzoic acid (295-5)

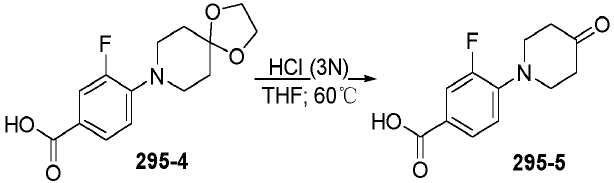

The intermediate obtained in the previous step was dissolved in THF (10 mL), to which was added HCl (3N, 10 mL), and then the mixture was stirred at 60 °C for 12 h. The reaction solution was extracted with ethyl acetate. Separation by column chromatography provided 820 mg of intermediate compound **295-5**, with a three-step yield of 69.2%. MS: *m*/*z* 238 [M+H]⁺.

### Synthesis of intermediate tert-butyl (1-(5-((3-(3-fluoro-4-(4-oxopiperidin-1-yl)benzamido)phenyl)thio)pyrazin-2-yl)-4-methylpyridin-4-yl)carbamate (295-6)

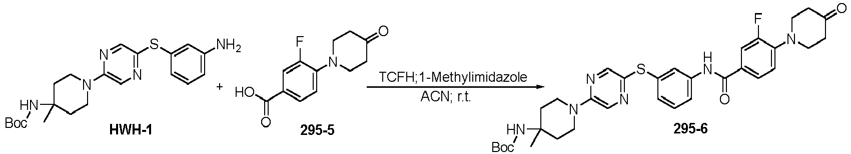

**HWH-1** (1 g, 2.4 mmol), **295-5** (569 mg, 2.4 mmol), and 1-methylimidazole (590 mg, 7.2 mmol) were added into acetonitrile (10 mL), to which was added TCFH (875 mg, 3.12 mmol), and then the mixture was stirred at room temperature for 2 h. The reaction solution was rotatory evaporated to remove the solvent. The residue was separated by column chromatography, to obtain 1.4 g of intermediate **295-6**, with a yield of 92%. MS: *m*/*z* 635 [M+H]⁺.

### Synthesis of intermediate methyl 1'-(1-(4-((3-((5-(4-((tert-butoxycarbonyl)amino)-4-methylpyridin-1-yl)pyrazin-2-yl)thio)phenyl)aminoformyl)-2-fluorophenyl)piperidin-4-yl)-1',2',3',6'-tetrahydro-[3,4'-bipyridine]-6-carboxylate (295-7)

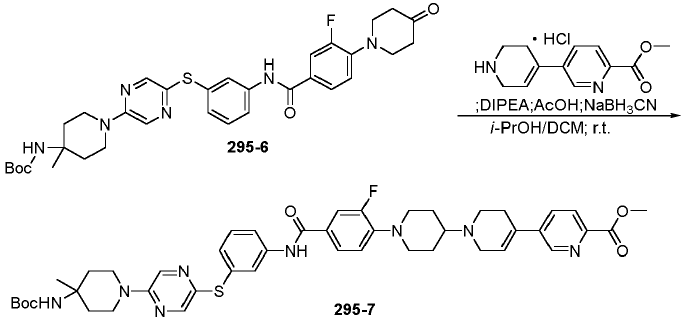

Intermediate **295-6** (1.4 g, 2.2 mmol) and methyl 1',2',3',6'-tetrahydro-[3,4'-bipyridine]-6-carboxylate hydrochloride (456 mg, 1.8mmol) were added into a mixed solvent of isopropanol (10 mL) and dichloromethane (10 mL), and then the pH value of the reaction solution was adjusted to 7-8 with DIPEA, and then adjusted to 5-6 with AcOH. Finally, sodium cyanoborohydride (227 mg, 3.6 mmol) was added to the reaction solution, and then the solution was stirred at room temperature. TLC was used to detect the progress of the reaction. After completion of the reaction, to the solution, was added 20 mL of saturated NaHCOs aqueous solution, and then extracted with dichloromethane. Separation by column chromatography afforded 840 mg of intermediate compound **295-7**, with a yield of 56%; MS: *m*/*z* 837 [M+H]⁺.

### Synthesis of intermediate 1'-(1-(4-((3-((5-(4-((tert-butoxycarbonyl)amino)-4-methylpyridin-1-yl)pyrazin-2-yl)thio)phenyl)aminoformyl)-2-fluorophenyl)piperidin-4-yl)-1',2',3',6'-tetrahydro-[3,4'-bipyridine]-6-carboxylic acid (295-8)

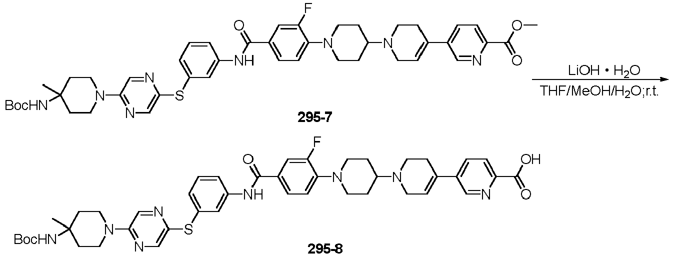

Intermediate **295-7** (840 mg, 1 mmol) was dissolved in THF/MeOH/H₂O (8 mL/2 mL/2 mL), to which was added lithium hydroxide monohydrate (168 mg, 4 mmol), and then the mixture was stirred at room temperature for 1 h. The pH value of the solution was adjusted to 2 with HCl (1N). The reaction solution was extracted with DCM/MeOH (10/1). The organic phase was dried with anhydrous sodium sulfate, and rotatory evaporated to obtain 863 mg of intermediate **295-8** as crude product, with a yield of 100%. MS: *m*/*z* 823 [M+H]⁺.

### Synthesis of intermediate tert-butyl (1-(5-((3-(4-(4-(6-(((2-(2,6-dioxopiperidin-3-yl)-1-oxoisoindolin-5-yl)methyl)aminoformyl)-3',6'-dihydro-[3,4'-bipyridine]-1'(2'H)-yl)piperidin-1-yl)-3-fluorobenzamido)phenyl)thio)pyrazin-2-yl)-4-methylpyridin-4-yl)carbamate (295-9)

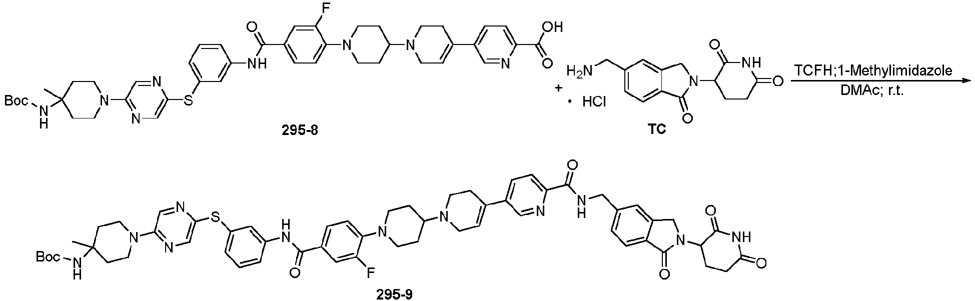

Intermediate **295-8** (863 mg, 1 mmol), TC (310 mg, 1 mmol), and 1-methylimidazole (246 mg, 3 mmol) were dissolved in DMAc (10 mL), to which was added TCFH (364 mg, 1.3 mmol), and then the mixture was stirred for 1h. The reaction solution was added to water dropwise, and then extracted with dichloromethane. Purification by TLC afforded 600 mg of intermediate **295-9**, with a yield of 56%. MS: *m*/*z* 539 [1/2M+H]⁺.

### Synthesis of compound 1'-(1-(4-((3-((5-(4-amino-4-methylpyridin-1-yl)pyrazin-2-yl)thio)phenyl)aminoformyl)-2-fluorophenyl)piperidin-4-yl)-N-((2-(2,6-dioxopiperidin-3-yl)-1-oxoisoindolin-5-yl)methyl)-1',2',3',6'-tetrahydro-[3,4'-bipyridine]-6-formamide hydrochloride (295)

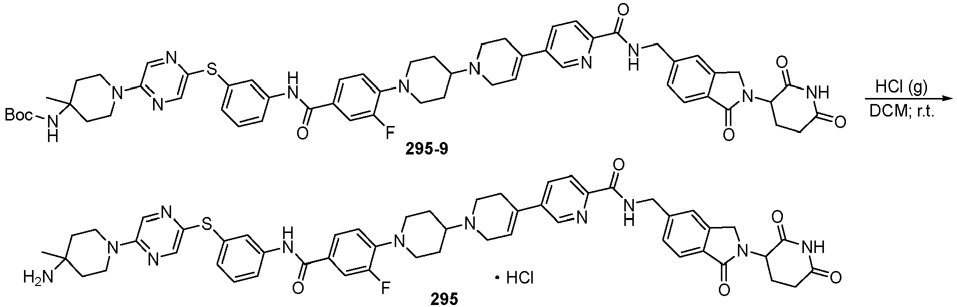

**295-9** (600 mg) was dissolved in dichloromethane (20 mL), to which was continuously introduced HCl gas. The reaction was monitored by TLC. After completion of the reaction, the reaction solution was rotatory evaporated, and the residue was beaten in methyl tert-butyl ether, followed by filtration, to obtain 600 mg of compound **295**, with a yield of 100%, MS: m/z 489 [1/2M+H]⁺. ¹H NMR (400 MHz, DMSO-*d*₆) *δ* 10.98 (s, 1H), 10.19 (s, 1H), 9.49 (s, 1H), 8.81 (d, *J* = 2.0 Hz, 1H), 8.39 (s, 1H), 8.24 (s, 2H), 8.20 (s, 1H), 8.16- 8.10 (m, 1H), 8.07 (d, *J* = 8.1 Hz, 1H), 7.78 (d, *J* = 12.8 Hz, 3H), 7.68 (d, *J* = 7.8 Hz, 2H), 7.54 (s, 1H), 7.47 (d, *J* = 8.0 Hz, 1H), 7.30 (t, *J* = 8.0 Hz, 1H), 7.17 (t, *J* = 8.8 Hz, 1H), 6.99 (d, *J* = 7.9 Hz, 1H), 6.52 (s, 1H), 5.10 (dd, *J* = 13.4, 5.1 Hz, 1H), 4.62 (d, *J* = 6.3 Hz, 2H), 4.48-4.26 (m, 3H), 3.80 (s, 2H), 3.68 (d, *J* = 11.3 Hz, 2H), 3.39 (s, 1H), 3.24 (s, 1H), 2.99 (s, 1H), 2.94-2.74 (m, 4H), 2.59 (d, *J* = 17.0 Hz, 2H), 2.41-2.31 (m, 2H), 2.23 (s, 1H), 1.98 (s, 3H), 1.77 (d, *J* = 19.0 Hz, 4H), 1.37 (s, 3H), 1.20 (d, *J* = 22.9 Hz, 3H).

### Example 17 Synthesis of compound 6-(4-(5-((3-((5-(4-amino-4-methylpyridin-1-yl)pyrazin-2-yl)thio)phenyl)carbamoyl)-2,3-dihydro-1H-inden-2-yl)piperazin-1-yl)-N-((2-(2,6-dioxapiperazin-3-yl)-1-oxoisoindolin-5-yl)methyl)pyridazin-3-formamide hydrochloride (compound 344)

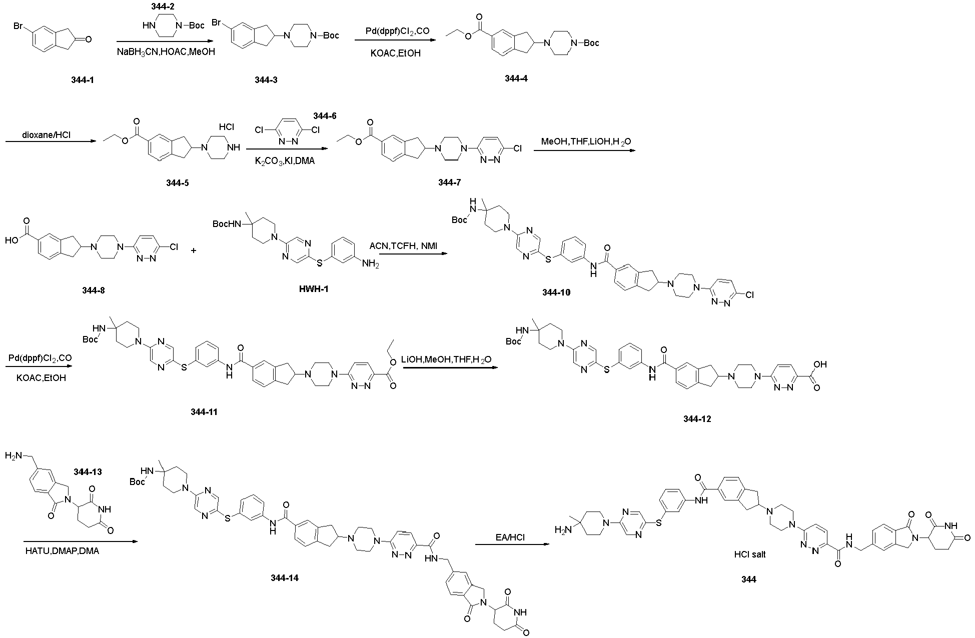

### Step 1: Synthesis of tert-butyl 4-(5-bromo-2,3-dihydro-1H-inden-2-yl)piperazin-1-carbonate (344-3)

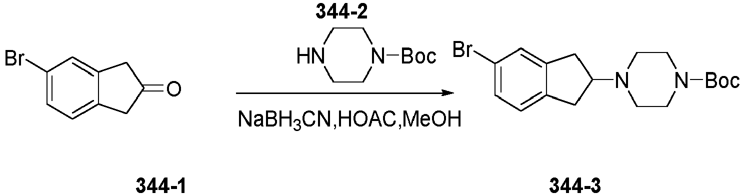

To a solution of compounds **344-1** (400 mg, 1.89 mmol, 1.0 eq) and **344-2** (706 mg, 3.79 mmol, 2.0 eq) in methanol, were added HOAC (two drops) and NaBH₃CN (238 mg, 3.79 mmol, 2.0 eq). The mixture was stirred at room temperature until the reaction was completed. After completion of the reaction, the reaction solution was quenched with 1N HCl, adjusted to pH 7-8 with sodium carbonate, and then extracted with EA(2*100 ml). The organic layer was dried and rotatory evaporated. The residue was purified by silica gel column chromatography to obtain **344-3** (600 mg, yield 83.03%). MS: *m*/*z* 383.2 [M+H]⁺.

### Step 2. Synthesis of compound tert-butyl 4-(5-(ethoxycarbonyl)-2,3-dihydro-1H-inden-2-yl)piperazin-1-carbonate (344-4)

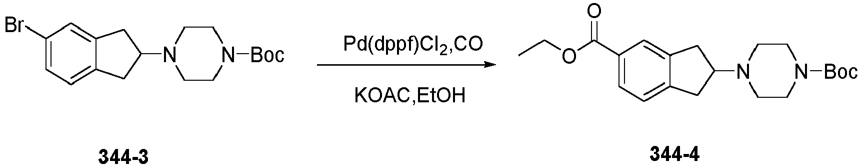

To a solution of compound **344-3** (600 mg, 1.57 mmol, 1.0 eq) in EtOH (8 mL), were added KOAC (462 mg, 4.72 mmol, 3.0 eq) and Pd(dppf)Cl₂ (58 mg, 0.079 mmol, 0.05 eq). After mixed and degassed, the mixture was allowed to react at about 80 °C under CO atmosphere. After the reaction was completed, the solid was filtered out, and the solvent was removed by rotatory evaporation. The residue was purified by silica gel column chromatography to obtain **344-4** (530 mg, yield 89.9%). MS: *m*/*z* 375.2 [M+H]⁺.

### Step 3: Synthesis of compound ethyl 2-(piperazin-1-yl)-2,3-dihydro-1H-inden-5-carboxylate hydrochloride (344-5)

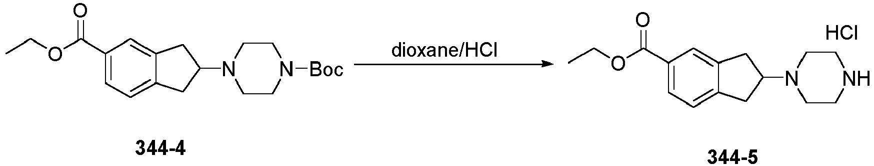

Compound **344-4** (530 mg, 1.41 mmol, 1.0 eq) was added to a solution of dioxane/HCl (4 M, 6 mL), and the resulting mixture was stirred at room temperature until the reaction was completed. The solvent was evaporated to obtain the target product **344-5** (437 mg, crude, yield 100%), which was directly used in the next step. MS: *m*/*z* 275.3 [M+H]⁺.

### Step 4. Synthesis of compound ethyl 2-(4-(6-chloropyridazin-3-yl)piperidin-1-yl)-2,3-dihydro-1H-inden-5-carboxylate (344-7)

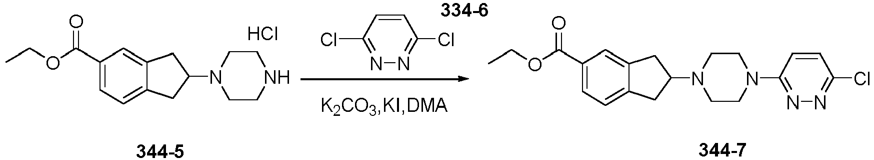

To a solution of compounds **344-5** (437 mg, 1.41 mmol, 1.0 eq) and **344-6** (230 mg, 1.55 mmol, 1.1 eq) in DMA (10 mL), were added K₂CO₃ (584 mg, 4.23 mmol, 3.0 eq) and KI (468 mg, 2.82 mmol, 2.0 eq). The mixture was stirred at about 120 °C until the reaction was completed. The mixture was poured into saturated brine (50 mL) and extracted with EA (2 * 50 mL). The organic layer was dried and rotatory evaporated. The residue was purified by silica gel column chromatography to obtain **344-7** (350 mg, yield 64.4%). MS: *m*/*z* 387.3 [M+H]⁺.

### Step 5: Synthesis of compound 2-(4-(6-chloropyridazin-3-yl)piperazin-1-yl)-2,3-dihydro-1H-inden-5-carboxylic acid (344-8)

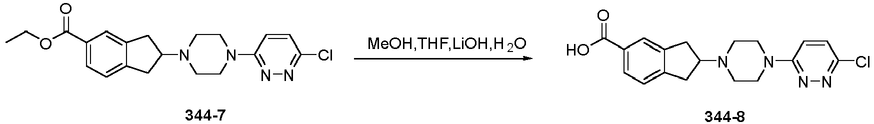

To a solution of compound **344-7** (350 mg, 0.91 mmol, 1.0 eq) in MeOH (3 mL), THF (3 mL), and H₂O (3 mL), was added LiOH (109 mg, 4.55 mmol, 5.0 eq). The obtained mixture was stirred at room temperature until the reaction was completed, and then the solvent was concentrated. The residue was diluted with water, and the pH value was adjusted to 5-6 with 1 N hydrochloric acid. The precipitated solid was filtered and dried, to obtain **344-8** (300 mg, yield 92.4%). MS: *m*/*z* 359.1 [M+H]⁺.

### Step 6. Synthesis of compound tert-butyl (1-(5-((3-(2-(4-(6-chloropyridazin-3-yl)piperazin-1-yl)-2,3-dihydro-1H-inden-5-carbonylamino)phenyl)thio)pyrazin-2-yl)-4-methylpyridin-4-yl)carbamate (344-10)

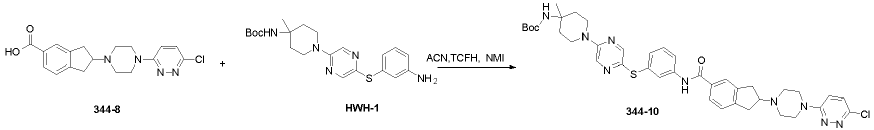

To a solution of compounds **344-8** (280 mg, 0.78 mmol, 1.0 eq) and **HWH-1** (324 mg, 0.78 mmol, 1.0 eq) in ACN (10 mL), were added N-methylimidazole (192 mg, 2.34 mmol, 3.0 eq) and TCFH (262 mg, 0.94 mmol, 1.2 eq). The mixture was stirred at room temperature until the reaction was completed, and then the solvent was evaporated. The residue was purified by silica gel column chromatography to obtain **344-10** (85 mg, yield 14.4%). MS: *m*/*z* 756.5 [M+H]⁺.

### Step 7. Synthesis of compound ethyl 6-(4-(5-((3-((5-(4-((tert-butoxycarbonyl)amino)-4-methylpyridin-1-yl)pyrazin-2-yl)thio)phenyl)carbamoyl)-2,3-dihydro-1H-inden-2-yl)piperazin-1-yl)pyridazin-3-carboxylate (344-11)

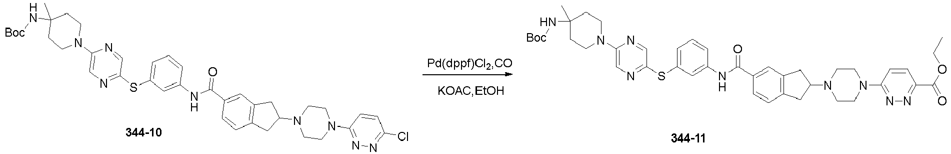

To a solution of compound **344-10** (85 mg, 0.11 mmol, 1.0 eq) in EtOH (5 mL), were added KOAC (33 mg, 0.33 mmol, 3.0 eq) and Pd(dppf)Cl₂ (24 mg, 0.033 mmol, 0.3 eq). After degassed, the mixture was stirred and reacted at 70 °C under CO atmosphere. After completion of the reaction, the reaction solution was concentrated. The residue was purified by silica gel column chromatography to obtain **344-11** (70 mg, yield 78.5%). MS: *m*/*z* 794.6 [M+H]⁺.

### Step 8: Synthesis of compound 6-(4-(5-((3-((5-(4-((tert-butoxycarbonyl)amino)-4-methylpyridin-1-yl)pyrazin-2-yl)thio)phenyl)carbamoyl)-2,3-dihydro-1H-inden-2-yl)piperazin-1-yl)pyridazin-3-carboxylic acid (344-12)

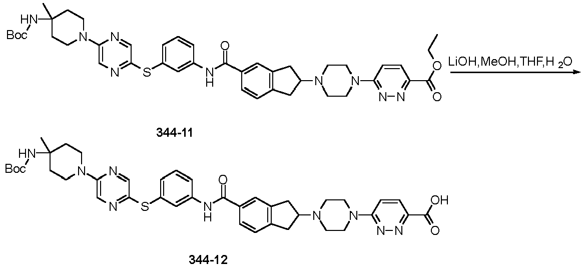

To a solution of compound **344-11** (70 mg, 0.088 mmol, 1.0 eq) in a mixed solvent of MeOH (2 mL)/THF (2 mL)/H₂O (2 mL), was added LiOH (21 mg, 0.88 mmol, 10.0 eq). The mixture was stirred at room temperature. After completion of the reaction, the solvent was evaporated, and the residue was diluted with water. The pH value of the obtained mixture was adjusted to 5-6 using 1N HCl. The precipitated solid was filtered and dried to obtain **344-12** (70 mg, crude, yield ca. 100%). MS: *m*/*z* 766.5 [M+H]⁺.

### Step 9: Synthesis of compound tert-butyl (1-(5-((3-(2-(4-(6-(((2-(2,6-dioxopiperidin-3-yl)-1-oxoisoindolin-5-yl)methyl)carbamoyl)pyridazin-3-yl)piperazin-1-yl)-2,3-dihydro-1H-inden-5-formamido)phenyl)thio)pyrazin-2-yl)-4-methylpyridin-4-yl)carbamate (344-14)

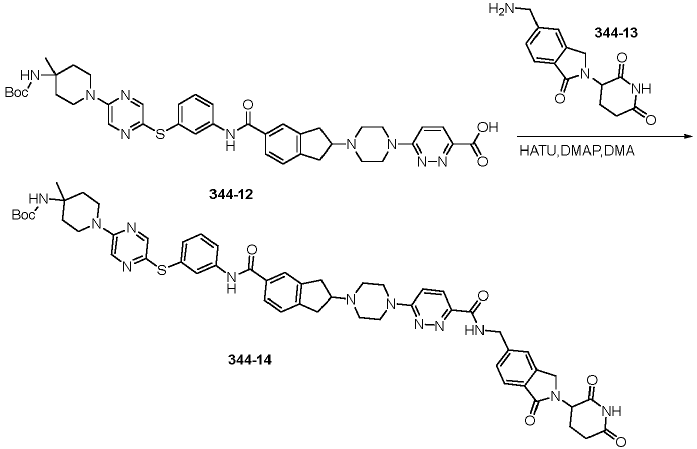

To a solution of compounds **344-12** (70 mg, 0.091 mmol, 1.0 eq), **344-13** (30 mg, 0.11 mmol, 1.2 eq) and HATU (52 mg, 0.14 mmol, 1.5 eq) in DMA (3 mL), was added DMAP (17 mg, 0.14 mmol, 1.5 eq). The obtained mixture was stirred at room temperature until the reaction was completed. The crude product obtained after conventional treatment was purified by reversed-phase column (acetonitrile/water), to obtain **344-14** (75 mg, yield 80.6%). MS: *m*/*z* 1021.3 [M+H]⁺.

### Step 10. Synthesis of compound 6-(4-(5-((3-((5-(4-amino-4-methylpyridin-1-yl)pyrazin-2-yl)thio)phenyl)carbamoyl)-2,3-dihydro-1H-inden-2-yl)piperazin-1-yl)-N-((2-(2,6-dioxapiperazin-3-yl)-1-oxoisoindolin-5-yl)methyl)piperazin-3-formamide hydrochloride (compound 344)

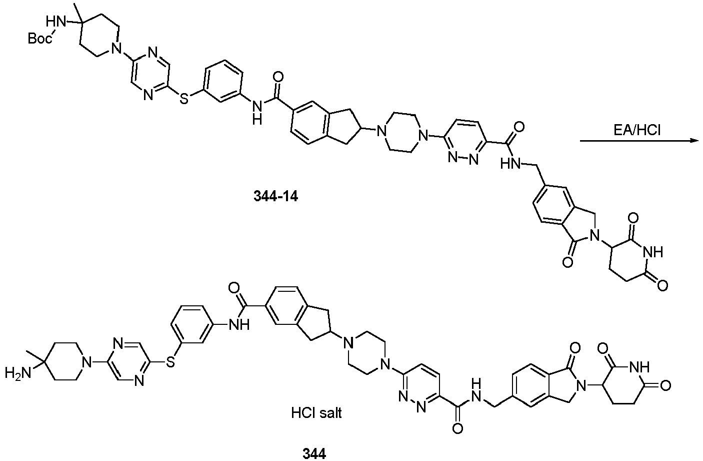

Compound **344-14** (75 mg, 0.073 mmol, 1.0 eq) was added into HCl/EA (3 M, 5 mL), and the resultant mixture was stirred at room temperature until the reaction was completed. The crude product was purified by reversed-phase column (acetonitrile/0.1% HCl), to obtain compound **344** (62.5 mg, yield 92.4%). MS: *m*/*z* 921.3 [M+H]⁺.

¹H NMR (400 MHz, DMSO-*d*₆): *δ* 11.93 (s, 1 H), 10.97 (s, 1 H), 10.27 (s, 1 H),9.59 (t, *J* = 6.2 Hz, 1 H), 8.39(d, *J* =1.2 Hz, 1 H), 8.20-8.17 (m, 4 H), 7.97-7.95 (m, 1 H), 7.84-7.77 (m, 3 H), 7.69 (d, *J* =8.0 Hz, 2 H), 7.54-7.41 (m, 4 H),7.30 (t, *J* =8.0 Hz, 1 H), 6.98 (d, *J* =8.0 Hz, 1 H), 5.10-5.07 (m, 1 H), 4.69-4.61(m, 4 H), 4.45-4.41 (m, 1 H), 4.32-4.27 (m , 1 H), 4.17-4.08 (m, 2H), 3.63-3.56 (m, 11 H),3.24-3.21(m, 2 H), 2.95-2.86 (m, 1 H),2.67-2.61 (m, 2 H), 2.39-2.32(m, 1 H), 2.00-1.97 (m, 1 H), 1.78-1.74 (m, 4 H), 1.37 (s, 3 H).

### Example 18 Synthesis of compound 5-(4-(4'-((3-((5-(4-amino-4-methylpyridin-1-yl)pyrazin-2-yl)thio)phenyl)carbamoyl)-[1,1'-diphenyl]-4-yl)piperazin-1-yl)-N-((2-(2,6-dioxopiperidin-3-yl)-1-oxoisoindolin-5-yl)methyl)pyridinecarboxamide (343)

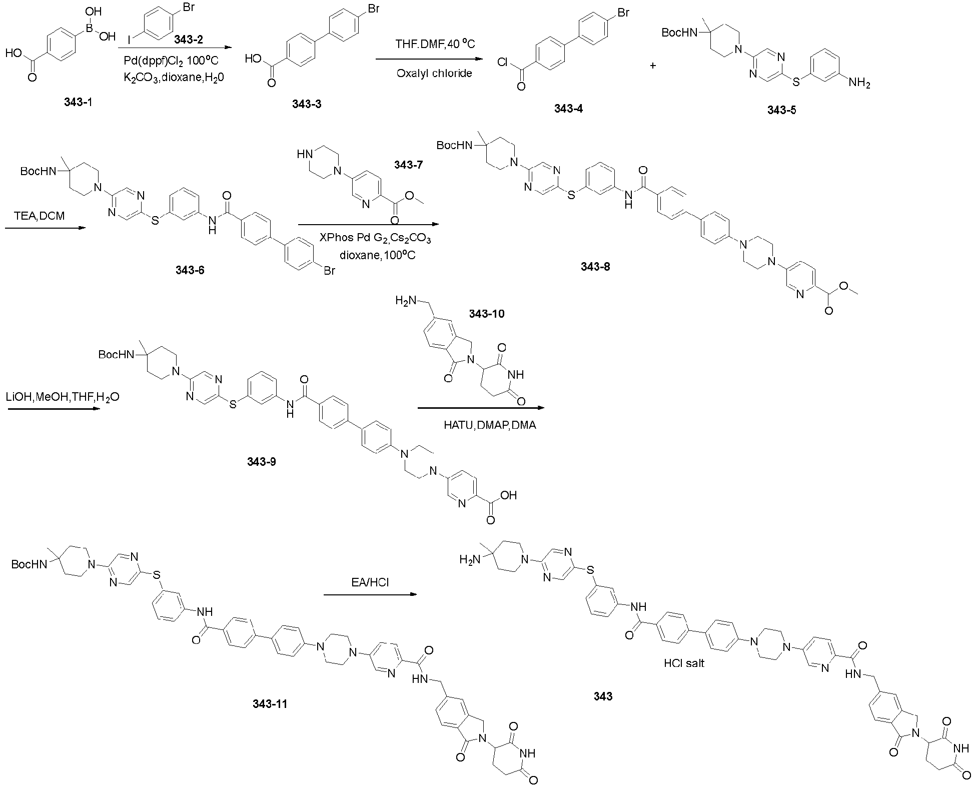

### Step 1: Synthesis of intermediate 4'-bromo-[1,1'-diphenyl]-4-carboxylic acid (343-3)

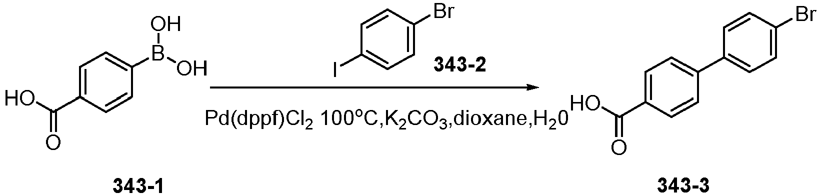

To a solution of compounds **343-1** (1.00 g, 6.06 mmol, 1.0 eq) and **343-2** (1.71 g, 6.06 mmol, 1.0 eq) in dioxane (20 mL) and water (5 mL), were added K₂CO₃ (2.51 g, 18.18 mmol, 3.0 eq) and Pd(dppf)Cl₂ (222 mg, 0.30 mmol, 0.05 eq). The mixture was allowed to react at about 100 °C. After completion of the reaction, the mixture was diluted with saturated brine (100 mL), and then extracted with ethyl acetate (2 * 100 mL). The organic layer was dried and rotary evaporated, to obtain crude product **343-3** (700 mg, yield 41.9%). MS(M-2): *m*/*z* 275.0.

### Step 2. Synthesis of intermediate 4'-bromo-[1,1'-diphenyl]-4-formyl chloride (343-4)

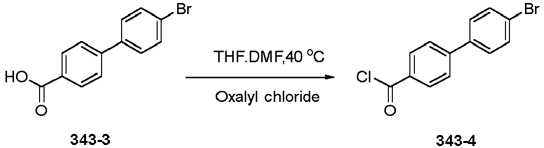

Compound **343-3** (600 mg, 2.16 mmol, 1.0 eq) was dissolved in THF (10 mL), to which was added oxalyl chloride (1.37 g, 10.83 mmol, 5.0 eq) dropwise, together with catalytic amount of DMF. The mixture was reacted at 40 °C until the reaction was completed. The reaction solution was concentrated, to obtain the crude product **343-4** (640 mg, yield 100%), which was directly used in the next step.

### Step 3: Synthesis of intermediate tert-butyl (1-(5-((3-(4'-bromo-[1,1'-diphenyl]-4-formamido)phenyl)thio)pyrazin-2-yl)-4-methylpyridin-4-yl)carbamate (343-6)

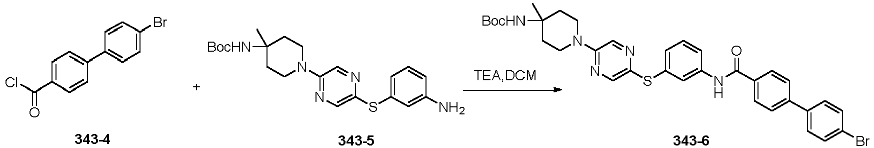

To a solution of compounds **343-4** (640 mg, 2.16 mmol, 1.0 eq) and **343-5** (720 mg, 1.73 mmol, 0.8 eq) in dichloromethane (10 mL), was added TEA (873 mg, 8.64 mmol, 4.0 eq), and the mixture was stirred at room temperature until the reaction was completed. The mixture was poured into saturated brine (50 mL) and extracted with EA (2 * 50 mL). The organic layer was dried and rotatory evaporated, and then the residue was purified by silica gel column chromatography (EA/PE = 3/1), to obtain **343-6** (960 mg, yield 82.1%). MS: *m*/*z* 674.5 [M+H]⁺.

### Step 4: Synthesis of intermediate methyl 5-(4-(4'-((3-((5-(4-((tert-butoxycarbonyl)amino)-4-methylpyridin-1-yl)pyrazin-2-yl)thio)phenyl)carbamoyl)-[1,1'-diphenyl]-4-yl)piperazin-1-yl)picolinate (343-8)

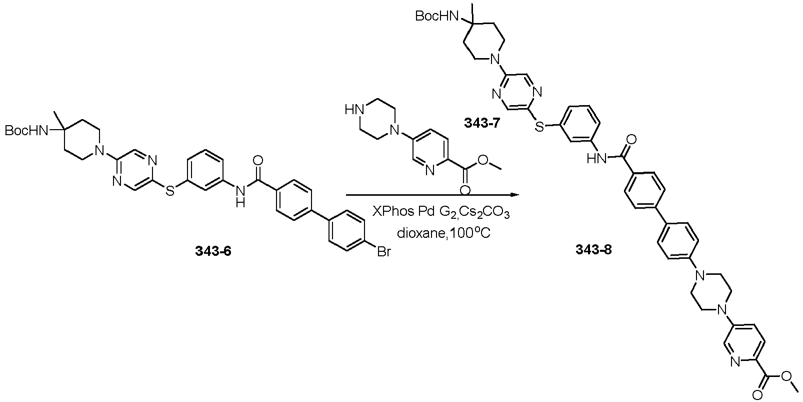

To a solution of compounds **343-6** (150 mg, 0.22 mmol, 1.0 eq) and **343-7** (98 mg, 0.44 mmol, 2.0 eq) in dioxane (5 mL), were added Cs₂CO₃(215 mg, 0.66 mmol, 3.0 eq) and XPhos Pd G₂ (22 mg, 0.022 mmol, 0.1 eq). The mixture was stirred at 100 °C until the reaction was completed. The mixture was diluted with ethyl acetate, and insoluble substances were filtered out. After the solvent was evaporated, the residue was purified by reversed-phase column chromatography (acetonitrile/water), to obtain **343-8** (50 mg, yield 27.5%). MS: *m*/*z* 815.4 [M+H]⁺.

### Step 5: Synthesis of intermediate 5-(4-(4'-((3-((5-(4-((tert-butoxycarbonyl)amino)-4-methylpyridin-1-yl)pyrazin-2-yl)thio)phenyl)carbamoyl)-[1,1'-diphenyl]-4-yl)piperazin-1-yl)picolinic acid (343-9)

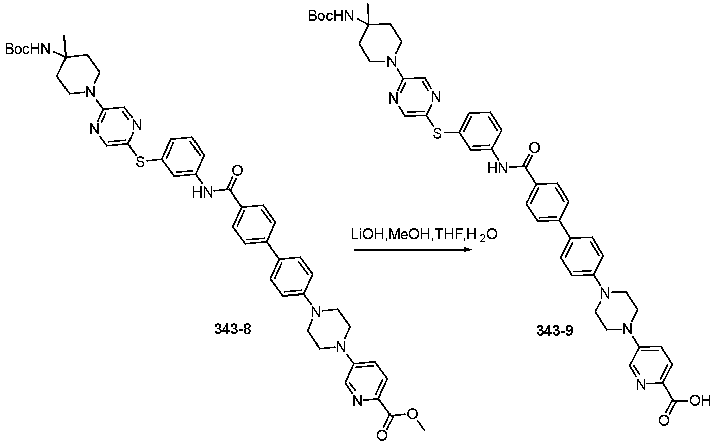

To a solution of compound **343-8** (50 mg, 0.061 mmol, 1.0 eq) in a mixed solvent of MeOH (2 mL)/THF (2 mL)/H₂O (2 mL), was added LiOH (15 mg, 0.61 mmol, 10.0 eq). The mixture was stirred at room temperature. After completion of the reaction, the solvent was evaporated, and the residue was diluted with water. The pH value of the obtained mixture was adjusted to 5-6 using 1N HCl. The precipitated solid was filtered and dried to obtain the target compound **343-9** (50 mg, crude, yield ca. 100%).

### Step 6: Synthesis of intermediate tert-butyl (1-(5-((3-(4'-(4-(6-(((2-(2,6-dioxopiperidin-3-yl)-1-oxoisoindolin-5-yl)methyl)formamido)piperidin-3-yl)piperazin-1-yl)-[1,1'-diphenyl]-4-formamido)phenyl)thio)pyrazin-2-yl)-4-methylpyridin-4-yl)carbamate (343-11)

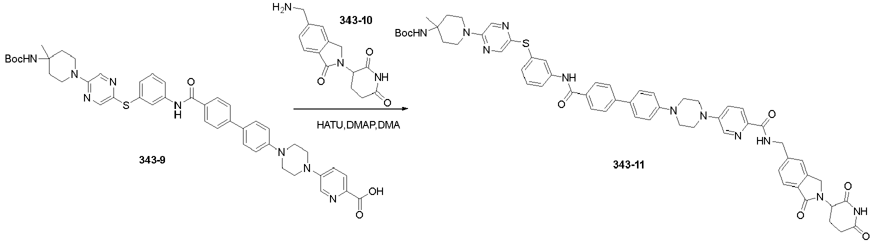

To a solution of compounds **343-9** (50 mg, 0.062 mmol, 1.0 eq), **343-10** (20 mg, 0.074 mmol, 1.2 eq), and HATU (36 mg, 0.094 mmol, 1.5 eq) in DMA (2 mL), was added DMAP (12 mg, 0.094 mmol, 1.5 eq). The mixture was stirred at room temperature until the reaction was completed. Purification by reversed-phase column (acetonitrile/water) provided the target compound **343-11** (20 mg, yield 30.3%). MS: *m*/*z* 1056.3 [M+H]⁺.

### Step 7: Synthesis of compound 5-(4-(4'-((3-((5-(4-amino-4-methylpyridin-1-yl)pyrazin-2-yl)thio)phenyl)carbamoyl[1,1'-diphenyl]-4-yl)piperazin-1-yl)-N-((2-(2,6-dioxopiperidine)-1-oxoisoindolin-5-yl)methyl)pyridineformamide (compound 343)

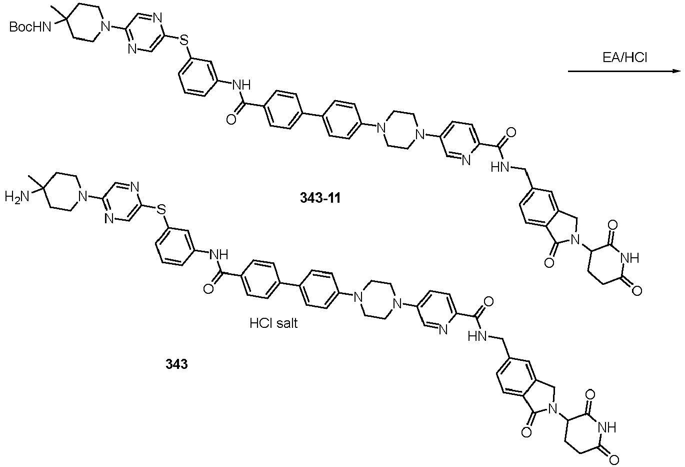

Compound **343-11** (20 mg, 0.019 mmol, 1.0 eq) was added into HCl/EA (3M/L, 3 mL), and the resultant mixture was stirred at room temperature until the reaction was completed. The crude product was purified by reversed-phase column (acetonitrile/0.1% HCl), to obtain compound **343** (7.25 mg, yield 40.3%). MS: *m*/*z* 956.3 [M+H]⁺.

¹H NMR (400 MHz, DMSO-*d₆*): *δ* 10.97 (s, 1 H),10.29-10.20 (m, 1 H), 9.19 (t, *J* = 6.2 Hz, 1 H), 8.41-8.37 (m, 2 H), 8.20-8.15 (m, 4 H), 8.09-7.92 (m, 3 H), 7.83-7.69 (m, 6 H), 7.59-7.53 (m, 2 H),7.51-7.47 (m, 1 H), 7.39-7.32 (m, 2 H), 7.30-7.22 (m, 1 H), 7.19-6.99 (m, 1 H), 5.12-5.07 (m, 1 H), 4.59-4.58 (d, *J* = 6.0 Hz, 2 H), 4.45-4.41 (m, 1 H), 4.37-4.21 (m, 1 H),4.08-4.03 (m, 2 H), 3.43-3.35 (m, 10 H),2.94-2.86 (m,2 H), 2.67-2.56 (m, 2 H), 2.39-2.32 (m, 1 H), 2.02-1.95 (m, 3 H), 1.82-1.74 (m, 4 H), 1.38 (d, *J* = 4.4 Hz, 3 H), 1.29-1.98 (m, 4 H).

### Example 19 Synthesis of compound 2-(4-(3-((3-((5-((3S,4S)-4-amino-3-methyl-2-oxa-8-azaspiro [4.5] decan-8-yl)pyrazin-2-yl)thio)phenyl)carbamoyl)cyclobutyl)piperazin-1-yl)-N-((S)-1-((2S,4R)-4-hydroxyl-2-(((S)-1-(4-(4-methylthiazol-5-yl)phenyl)ethyl)carbamoyl) pyrrolin-1-yl)-3,3-dimethyl-1-oxobutane-2-yl)pyrimidine-5-formamide hydrochloride (compound 294)

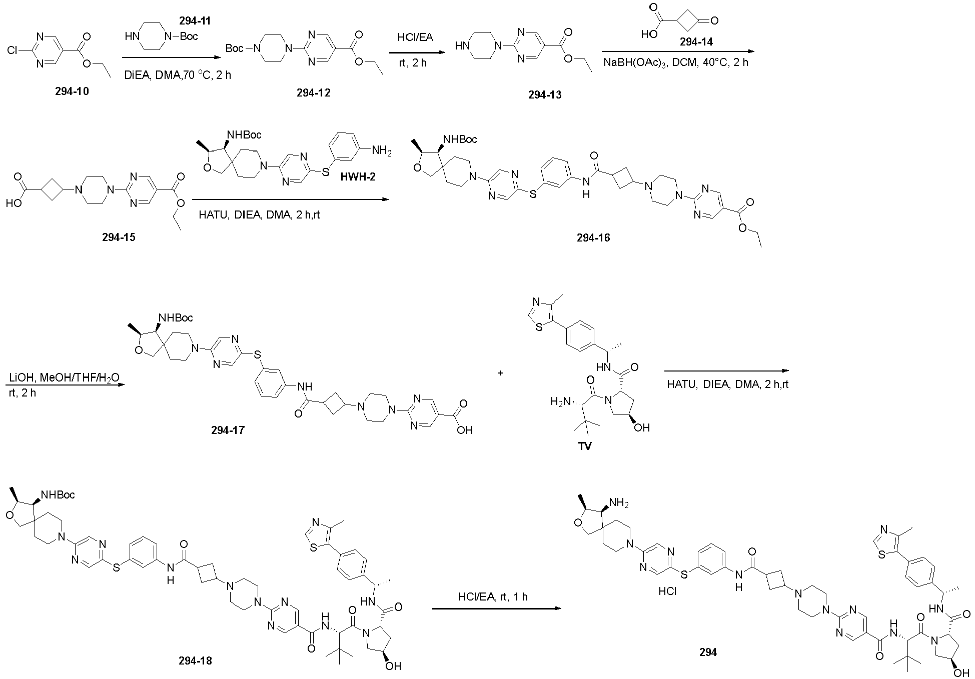

### Step 1: Synthesis of intermediate ethyl 2-(4-(tert-butoxycarbonyl)piperazin-1-yl)pyrimidine-5-carboxylate (294-12)

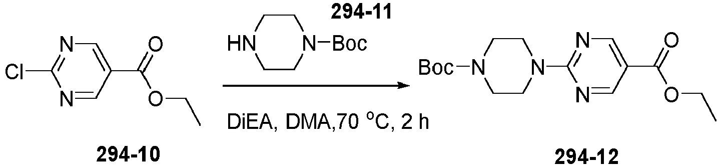

To a solution of compound **294-10** (1.9 g, 10.0 mmol, 1.0 eq) and compound **294-11** (1.9 g, 10.0 mmol, 1.0 eq) in DMA (20 mL), was added diisopropylethylamine (2.6 g, 20.0 mmol, 2.0 eq). The obtained mixture was allowed to react at about 70 °C. After completion of the reaction, the reaction solution was poured into water (100 mL). The precipitated solid was filtered, collected, and dried, to obtain the target product **294-12** (3.4 g, crude product, 10.0 mmol, yield ca. 100%). MS: *m*/*z* 337.1 [M+H]⁺.

### Step 2: Synthesis of intermediate ethyl 2-(piperazin-1-1-yl)pyrimidine-5-carboxylate (294-13)

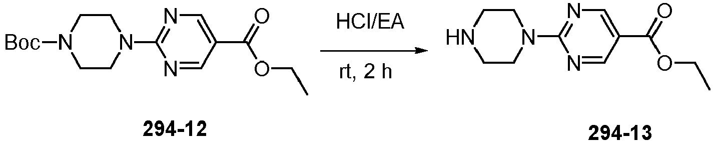

Compound **294-12** (3.4 g, 10.0 mmol, 1.0 eq) was added into HCl/EA (35 mL, 10.5 eq., 3M), and the mixture was stirred at room temperature. After completion of the reaction, the solvent was evaporated, to obtain **294-13**-hydrochloride (2.4 g, 8.82 mmol, yield 88.2%), which was directly used in the next step.

### Step 3: Synthesis of intermediate 3-(4-(5-(ethoxycarbonyl)pyrimidine-2-yl)piperazin-1-yl)cyclobutyl-1-carboxylic acid (294-15)

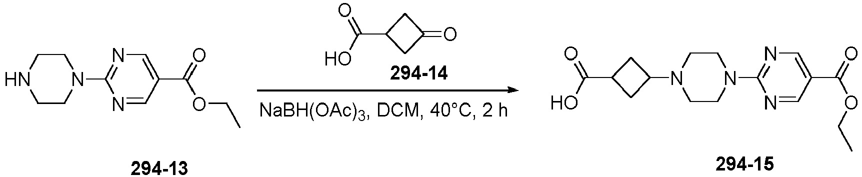

To a solution of compounds **294-13** (200 mg, 1.75 mmol, 1.0 eq) and **294-14** (414 mg, 1.75 mmol, 1.0 eq) in DCM (10 mL), was added NaBH(OAc)₃ (740 mg, 3.51 mmol, 2.0 eq). The obtained mixture was stirred at about 40 °C until the reaction was completed. The reaction was quenched with 1N HCl (50 mL). After routine working-up, **294-15** (2.6 g, crude product) was obtained. MS: *m*/*z* 335.1 [M+H]⁺.

### Step 4: Synthesis of intermediate ethyl 2-(4-(3-((3-((5-((3S,4S)-4-((tert-butoxycarbonyl)amino)-3-methyl-2-oxo-8-azaspiro[4.5]decan-8-yl)pyrazin-2-yl)thio) phenyl)carbamoyl)cyclobutyl)piperazin-1-yl)pyrimidine-5-carboxylate (294-16)

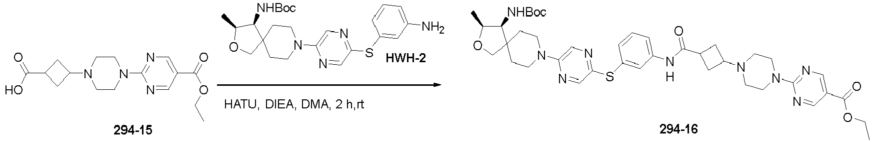

To a solution of compound **294-15** (30 mg, 0.09 mmol, 1.0 eq) and **HWH-2** (42 mg, 0.09 mmol, 1.0 eq) in DMA (5 mL), were added HATU (41 mg, 0.11 mmol, 1.2 eq) and diisopropylethylamine (29 mg, 0.22 mmol, 2.5 eq), and the resultant mixture was allowed to react at room temperature. After completion of the reaction, the mixture was diluted with water (30 mL), and then extracted with EA (30 mL*2). The organic layer was washed with saturated brine (50 mL*3), dried with Na₂SO₄, and rotatory evaporated. The residue was purified by reversed-phase column, to obtain **294-16** (40 mg, yield 56.5%). MS: *m*/*z* 788.2 [M+H]⁺.

### Step 5: Synthesis of intermediate 2-(4-(3-((3-((5-((3S,4S)-4-((tert-butoxycarbonyl) amino)-3-methyl-2-oxo-8-azaspiro[4.5]decan-8-yl)pyrazin-2-yl)thio)phenyl)carbamoyl) cyclobutyl)piperazin-1-yl)pyrimidine-5-carboxylic acid (294-17)

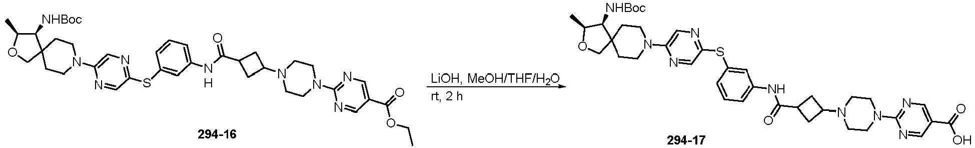

To a solution of compound **294-16** (40 mg, 0.05 mmol, 1.0 eq) in MeOH/THF/H₂O (1 mL/1 mL/1 mL), was added LiOH (4.9 mg, 0.20 mmol, 4.0 eq), and the resultant mixture was stirred at room temperature. After completion of the reaction, the solvent was evaporated, and the residue was diluted with water. The pH value of the reaction solution was adjusted to 5 using 0.5 N HCl, and then extracted with EA (5 mL*2). The solvent was evaporated, to obtain the crude product **294-17** (20 mg, yield 51.8%), which was directly used in the next step. MS: *m*/*z* 760.3 [M+H]⁺.

### Step 6: Synthesis of intermediate tert-butyl ((3S,4S)-8-(5-((3-(3-(4-(5-(((S)-1-((2S,4R)-4-hydroxyl-2-(((S)-1-(4-(4-methylthiazol-5-yl)phenyl)ethyl)formamido)pyrrolin-1-yl)-3,3-dimethyl-1-oxobutan-2-yl)carbamoyl)pyrimidine-2-yl)piperazin-1-yl)cyclobutyl-1-formamido)phenyl)thio)pyrazin-2-yl)-3-methyl-2-oxa-8-azaspiro[4.5]decan-4-yl)carbamate (294-18)

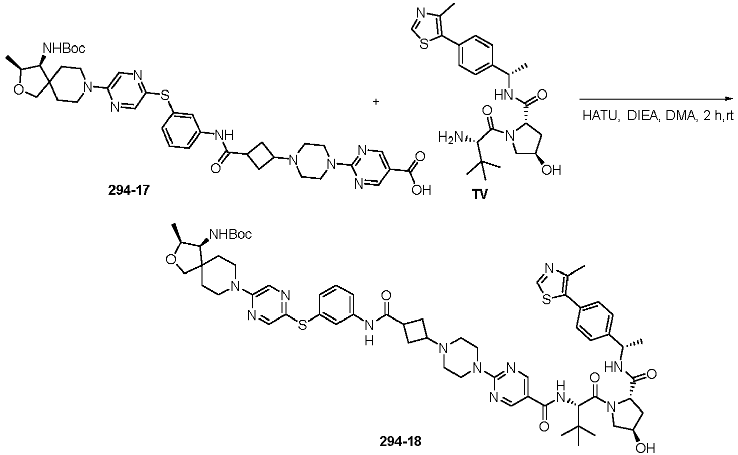

To a solution of compound **294-17** (20 mg, 0.026 mmol, 1.0 eq) and **TV** (12 mg, 0.026 mmol, 1.0 eq) in DMA (2 mL), were added HATU (12 mg, 0.031 mmol, 1.2 eq) and diisopropylethylamine (8 mg, 0.066 mmol, 2.5 eq), and the obtained mixture was stirred at room temperature until the reaction was completed. The reaction was diluted with water (5 mL) and then extracted with EA (5 mL * 2). The organic layer was subjected to the routine processing, and then the residue was purified by reversed-phase column (water), to obtain **294-18** (15 mg, yield 48.1%). MS: *m*/*z* 1186.5 [M+H]⁺.

### Step 7: Synthesis of compound 2-(4-(3-((3-((5-((3S,4S)-4-amino-3-methyl-2-oxa-8-azaspiro[4.5]decan-8-yl)pyrazin-2-yl)thio)phenyl)carbamoyl)cyclobutyl)piperazin-1-yl)-N-((S)-1-((2S,4R)-4-hydroxyl-2-(((S)-1-(4-(4-methylthiazol-5-yl)phenyl)ethyl)carbamoyl) pyrrolin-1-yl)-3,3-dimethyl-1-oxobutane-2-yl)pyrimidine-5-formamide hydrochloride (compound 294)

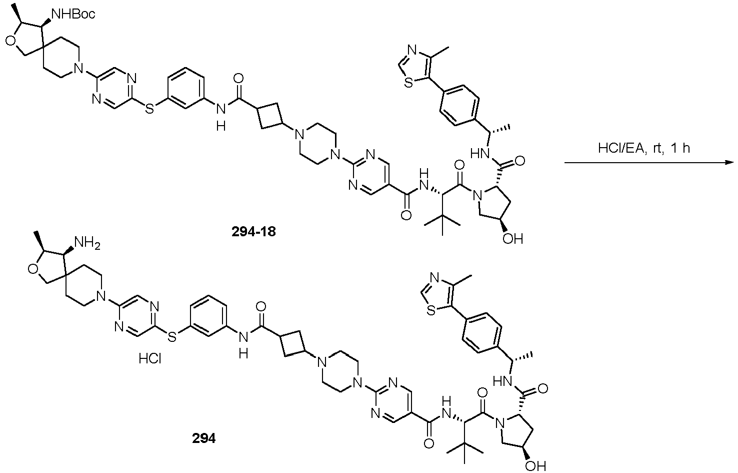

Compound **294-18** (15 mg, 0.013 mmol, 1.0 eq) was added into HCl/EA (2 mL), and then the resultant mixture was stirred at room temperature until the reaction was completed. The solvent was evaporated. The crude product was purified by Prep-HPLC(0.1% HCl), to obtain compound **294** (9.88 mg, yield 69.6%). MS: *m*/*z* 1086.5 [M+H]⁺.

¹H NMR (400 MHz, MeOD): *δ* 9.73 (d, *J*=2.4 Hz, 1 H), 8.85-8.84 (m, 2 H), 8.22-8.16 (m, 2 H), 7.67-7.64 (m, 1 H), 7.55-7.49 (m, 4 H), 7.45 (d, *J*=8.0 Hz, 1 H), 7.24 (t, *J*=8.0 Hz, 1 H), 6.98 (d, *J*=7.2 Hz, 1 H), 5.10-5.00 (m, 4 H), 4.60 (t, *J*=8.4 Hz 1 H), 4.46 (s, 1 H), 4.33-4.18 (m, 3 H), 4.00-3.80 (m, 5 H), 3.64-3.61 (m, 2 H), 3.48-3.38 (m, 4 H), 3.24-2.93 (m, 5 H), 2.77-2.61 (m, 4 H), 2.58 (s, 4 H), 2.25-2.20 (m, 1 H), 2.03-1.78 (m, 6 H), 1.52 (d, *J*=7.2 Hz, 3 H), 1.31(d, *J*=6.8 Hz, 6H), 1.11 (s, 9H).

### Example 20 Synthesis of compound N-{[2-(2,6-oxohexahydropyridin-3-yl)-1-oxo-2,3-dihydro-1H-isoindol-5-yl]methyl}-5-(1-{1-[2-fluoro-4-({[3-({5-[(3S,4S)-4-amino-3-methyl-8-aza-2-oxaspiro[4.5]decan-8-yl]pyrazin-2-yl}thio)phenyl]amino}carbonyl)phenyl] hexahydropyridine-4-yl}-1,2,3,6-tetrahydropyridin-4-yl)-6-methylpyridine-2-formamide hydrochloride (compound 461)

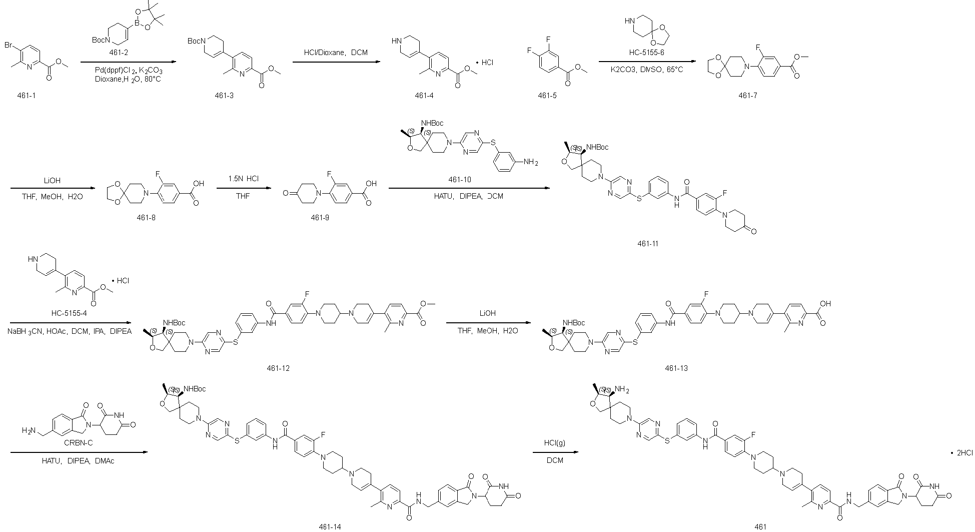

### Step 1: Synthesis of compound 4-[6-(methoxycarbonyl)-2-methylpyridine-3-yl]-1,2,3,6-tetrahydropyridin-1-formic acid-2-methylpropyl-2-yl ester (461-3)

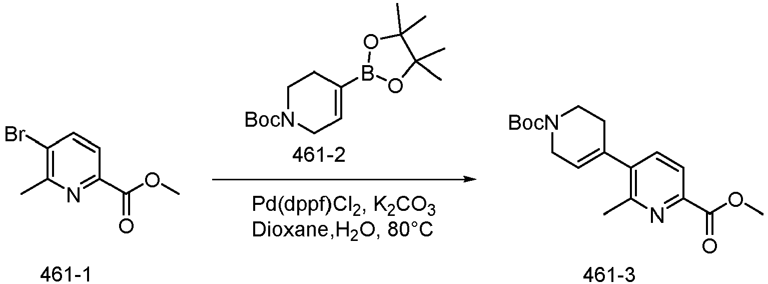

Methyl 5-bromo-6-methylpyridine-2-formate (500 mg, 2.17 mmol), 4-(4,4,5,5-tetramethyl-1,3,2-dioxaborolane-2-yl)-1,2,3,6-tetrahydropyridin-1-formic acid-2-methylpropyl-2-yl ester (739 mg, 2.39 mmol), and K₂CO₃ (601 mg, 4.35 mmol) were successively added into a mixed solvent of 1,4-dioxane (9 mL) and water (0.6 mL), and then the system was purged with argon for three times. Under argon protection, [1,1'-bis(diphenylphosphino)ferrocene]dichloropalladium (159 mg, 0.22 mmol) was added to the reaction solution, and then the reaction system was heated to 80 °C and reacted for 16 h. The reaction solution was cooled to room temperature, and filtered over diatomaceous earth. The filtrate was concentrated under reduced pressure, and the residue was purified by column chromatography, to obtain 661 mg of product (intermediate **461-3),** with a yield of 91.6%. MS: *m*/*z* 333.1 [M+H]⁺.

### Step 2. Synthesis of compound methyl 6-methyl-5-(1,2,3,6-tetrahydropyridin-4-yl)pyridine-2-formate hydrochloride (461-4)

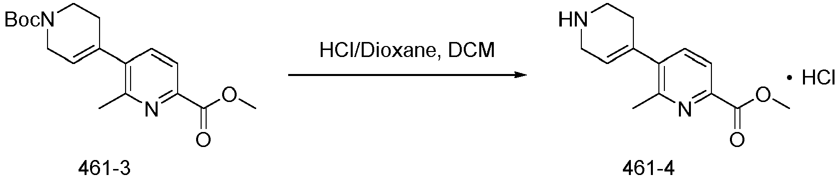

Intermediate **461-3** (660 mg, 1.98 mmol) was dissolved in 15 mL of dichloromethane, to which was added 15 mL of HCl-dioxane solution (4 mol/L), and then the mixture was allowed to react for 2h at room temperature. The reaction solution was concentrated, and the residue was triturated with 10 mL of dichloromethane, followed by filtration. The filter cake was successively washed with 5 mL of dichloromethane and 5 mL of methyl tert-butyl ether, and then dried, to obtain 520 mg of product (intermediate **461-4),** with a yield of 97.4%. MS: *m*/*z* 233.1 [M+H]⁺.

### Step 3. Synthesis of compound methyl 3-fluoro-4-(8-aza-1,4-dioxaspiro[4.5]decan-8-yl)benzoate (461-7)

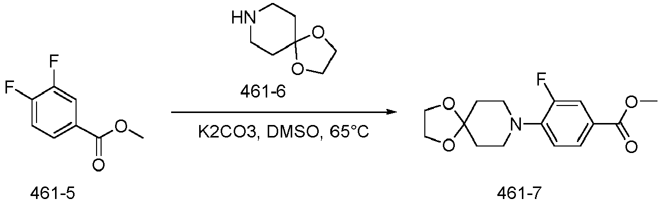

Methyl 3,4-difluorobenzoate (1.72 g,10 mmol), 8-aza-1,4-dioxaspiro[4.5]decane (1.43 g, 10 mmol), and K₂CO₃ (1.81 g, 13 mmol) were successively added into 35 mL of dimethylsulfoxide, and then the reaction solution was heated to 65 °C and reacted for 16 h. The reaction solution was slowly poured into 100 mL of ice water after cooling to room temperature. The resultant solution was stirred for 1 h, and filtered. The solid was triturated with 40 mL mixed solvent of petroleum ether/ethyl acetate (10:1), and then filtered. The filter cake was dried, to obtain 2.73 g of product (intermediate **461-7)**, with a yield of 92.4%. MS: *m*/*z* 296.1 [M+H]⁺.

### Step 4. Synthesis of compound 3-fluoro-4-(8-aza-1,4-dioxaspiro[4.5]decan-8-yl)benzoic acid (461-8)

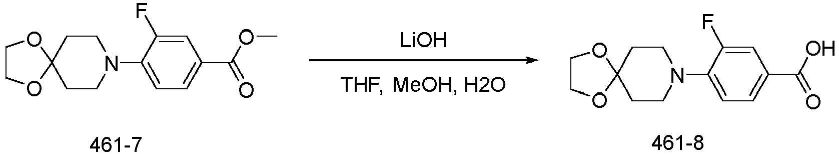

Intermediate **461-7** (2.73 g, 9.24 mmol) and lithium hydroxide monohydrate (1.94 g, 46.22 mmol) were successively added into a mixed solvent of tetrahydrofuran (50 mL), methanol (25 mL), and water (25 mL), and then the reaction system was heated to 40 °C and reacted for 4 h. The pH value of the reaction system was adjusted to 7 with 1N HCl aqueous solution. The organic solvent in the system was concentrated under reduced pressure, and then the pH vale of the reaction system was adjusted to 2-3 with 1N HCl aqueous solution, to which was added 25 mL of water. The reaction solution was stirred for 0.5 h, and filtered. The solid was dried to obtain 2.4 g of product (intermediate **461-8),** with a yield of 92.3%. MS: *m*/*z* 282.1 [M+H]⁺.

### Step 5. Synthesis of compound 3-fluoro-4-(4-oxohexahydropyridin-1-yl)benzoic acid (461-9)

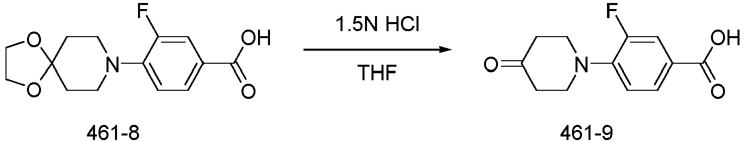

Intermediate **461-8** (2.4 g, 8.53 mmol) was dispersed in 50 mL of tetrahydrofuran, to which was added 50 mL of HCl aqueous solution (1.5 N), and then the system was heated to 70 °C and reacted for 16 h. The reaction solution was cooled to room temperature, and the pH value of the system was adjusted to 4 with saturated NaHCOs aqueous solution. The reaction solution was concentrated under reduced pressure to remove organic solvents, followed by adding 50 mL of water. The resultant solution was stirred for 0.5 h, and filtered. The filter cake was washed with 10 mL of water, and dried, to obtain 1.87 g of product (intermediate **461-9),** with a yield of 92.4%. MS: *m*/*z* 238.1 [M+H]⁺.

### Step 6. Synthesis of compound {[(3S,4S)-8-(5-{[3-({[3-fluoro-4-(4-oxohexahydropyridine-1-yl)phenyl]carbonyl}amino)phenyl]thio}pyrazin-2-yl)-3-methyl-8-aza-2-oxaspiro[4.5]decan-4-yl]amino}formic acid-2-methylpropyl-2-yl ester (461-11)

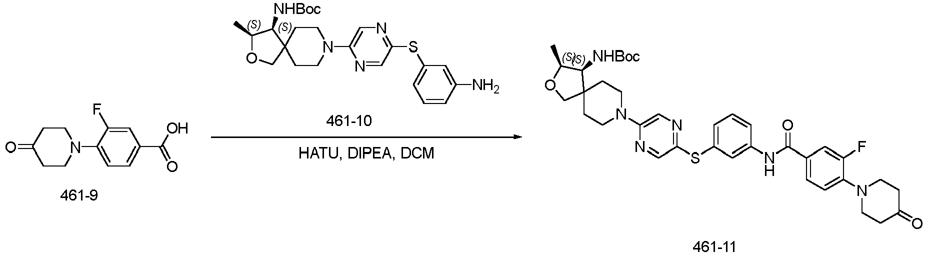

Intermediate **461-9** (1.87 g, 7.88 mmol), {[(3S,4S)-8-{5-[(3-aminophenyl)thio]pyrazin-2-yl}-3-methyl-8-aza-2-oxaspiro[4.5]decan-4-yl]amino}formic acid-2-methylpropyl-2-yl ester (3.53 g, 7.49 mmol), HATU(3.13 g, 8.24 mmol), and N,N-diisopropylethylamine (1.94 g, 14.98 mmol) were successively added into 40 mL of dichloromethane, and then the mixture was allowed to react at room temperature for 16 h. The reaction solution was diluted with 60 mL of dichloromethane, and then washed sequentially with 50 mL of water, 50 mL of 1N HCl aqueous solution, 50 mL of saturated NaHCOs aqueous solution, and 50 mL of saturated brine. The organic layer was dried with anhydrous sodium sulfate, filtered, and then concentrated under reduced pressure. The residue was purified by column chromatography to obtain 3.61 g of product (intermediate **461-11),** with a yield of 69.8%. MS: *m*/*z* 691.1 [M+H]⁺.

### Step 7. Synthesis of compound methyl 5-(1-{1-[2-fluoro-4-({[3-({5-[(3S,4S)-3-methyl-4-({[(2-methylpropan-2-yl)oxy]carbonyl}amino)-8-aza-2-oxaspiro[4.5]decan-8-yl]pyrazin-2-yl}thio)phenyl]amino}carbonyl)phenyl]hexahydropyridine-4-yl}-1,2,3,6-tetrahydropyridin-4-yl)-6-methylpyridine-2-formate (461-12)

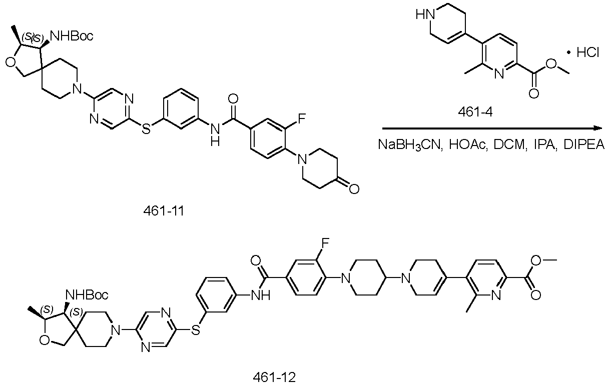

Intermediate **461-11** (500 mg, 0.72 mmol) and intermediate **461-4** (233 mg, 0.87 mmol) were successively added into a mixed solvent of dichloromethane (10 mL) and isopropanol (10 mL), and then the pH value of the system was adjusted to 7-8 with N,N-diisopropylethylamine. Subsequently, the pH was adjusted to 6 with acetic acid, followed by addition of sodium cyanodorohydride (91 mg, 1.45 mmol). The mixture was allowed to react at room temperature for 16 h. The reaction solution was quenched with 20 mL of saturated NaHCOs aqueous solution, and extracted with 50 mL of dichloromethane. The organic layer was dried with anhydrous sodium sulfate, filtered, and concentrated under reduced pressure. The residue was purified by column chromatography to obtain 360 mg of product (intermediate **461-12),** with a yield of 54.8%. MS: *m*/*z* 907.3 [M+H]⁺.

### Step 8. Synthesis of compound 5-(1-{1-[2-fluoro-4-({[3-({5-[(3S,4S)-3-methyl-4-({[(2-methylpropan-2-yl)oxy]carbonyl}amino)-8-aza-2-oxaspiro[4.5]decan-8-yl]pyrazin-2-yl}thio) phenyl]amino}carbonyl)phenyl]hexahydropyridine-4-yl}-1,2,3,6-tetrahydropyridin-4-yl)-6-methylpyridine-2-formic acid (461-13)

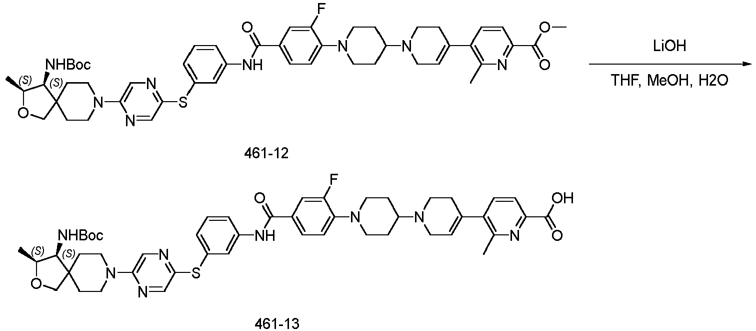

Intermediate **461-12** (360 mg, 0.40 mmol) and lithium hydroxide monohydrate (166 mg, 3.97 mmol) were successively added into a mixed solvent of tetrahydrofuran (4 mL), methanol (2 mL), and water (2 mL), and then the mixture was reacted at room temperature for 1 h. The pH value of the reaction system was adjusted to 4 with 1N HCl aqueous solution, and extracted with 30 mL of dichloromethane. The organic layer was dried with anhydrous sodium sulfate, filtered, and concentrated under reduced pressure to obtain 342 mg of product (intermediate **461-13),** with a yield of 96.5%. MS: *m*/*z* 893.2 [M+H]⁺.

Step 9. Synthesis of compound {[(3S,4S)-8-[5-({3-[({4-[4-(4-{6-[({[2-(2,6-oxohexahydropyridine-3-yl)-1-oxo-2,3-dihydro-1H-isoindol-5-yl]methyl}amino)carbonyl]-2-methylpyridine-3-yl}-1,2,3,6-tetrahydropyridin-1-yl)hexahydropyridine-1-yl]-3- fluorophenyl}carbonyl)amino]phenyl}thio)pyrazin-2-yl]-3-methyl-8-aza-2-oxaspiro[4.5]decan-4-yl]amino}formic acid-2-methylpropyl-2-yl ester **(461-14)**

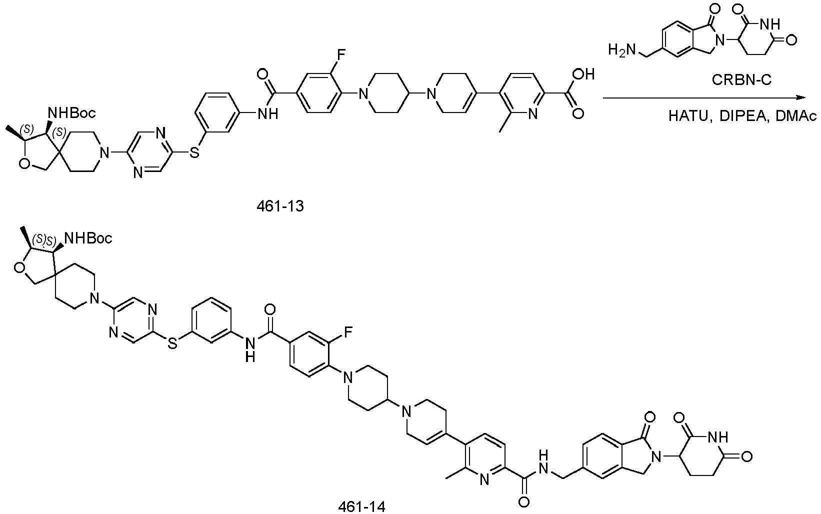

Intermediate **461-13** (342 mg, 0.38 mmol), 3-[5-(aminomethyl)-1-oxo-2,3-dihydro-1H-isoindol-2-yl]hexahydropyridine-2,6-dione (105 mg, 0.38 mmol), HATU (175 mg, 0.46 mmol), and N,N-diisopropylethylamine (124 mg, 0.96 mmol) were successively added into 4 mL of N,N-dimethylacetamide, and then the mixture was allowed to react at room temperature for 16 h. The reaction solution was slowly added into 40 mL of water dropwise, and filtered. The solid was purified by column chromatography to obtain 230 mg of product (intermediate **461-14)** with a yield of 52.3%. MS: *m*/*z* 574.8 [M/2+H]⁺.

### Step 10. Synthesis of compound N-{[2-(2,6-oxohexahydropyridine-3-yl)-1-oxo-2,3-dihydro-1H-isoindol-5-yl]methyl}-5-(1-{1-[2-fluoro-4-({[3-({5-[(3S,4S)-4-amino-3-methyl-8-aza-2-oxaspiro[4.5]decan-8-yl]pyrazin-2-yl}thio)phenyl]amino}carbonyl)phenyl] hexahydropyridine-4-yl } -1 ,2,3, 6-tetrahydropyridin-4-yl)-6- methylpyridine-2- formamide hydrochloride (461)

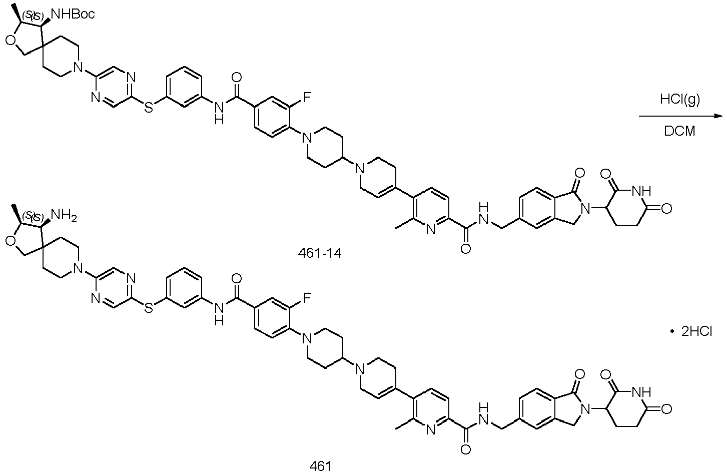

Intermediate **461-14** (230 mg, 0.20 mmol) was dissolved in 25 mL of dichloromethane, to which was continuously introduced dry HCl gas at room temperature, and TLC indicated completion of the reaction. The reaction solution was concentrated under reduced pressure. The residue was triturated with 25 mL of dichloromethane, and filtered. The filter cake was sequentially washed with 5 mL of dichloromethane and 5 mL of methyl tert-butyl ether. The solid was dried to obtain 220 mg of product (compound **461),** with a yield of 98.0%. MS: *m*/*z* 524.7 [M/2+H]⁺.

¹H NMR (400 MHz, DMSO-*d*₆) *δ* 11.81 (s, 1H), 10.99 (s, 1H), 10.27 (s, 1H), 9.49 - 9.42 (m, 1H), 8.41 - 8.29 (m, 4H), 8.18 (d, *J=* 1.4 Hz, 1H), 7.94 (d, *J=* 7.9 Hz, 1H), 7.83 - 7.66 (m, 6H), 7.54 (s, 1H), 7.47 (d, *J* = 7.6 Hz, 1H), 7.29 (t, *J* = 8.0 Hz, 1H), 7.17 (t, *J* = 8.7 Hz, 1H), 7.00 - 6.95 (m, 1H), 5.79 (s, 1H), 5.10 (dd, *J=* 13.3, 5.1 Hz, 1H), 4.62 (d, *J* = 6.4 Hz, 2H), 4.47 - 4.26 (m, 2H), 4.26 - 4.11 (m, 3H), 3.96 - 3.85 (m, 3H), 3.77 - 3.61 (m, 4H), 3.49 (s, 1H), 3.34 (t, *J=* 5.6 Hz, 1H), 3.28 - 3.17 (m, 1H), 3.12 - 2.98 (m, 4H), 2.96 - 2.78 (m, 3H), 2.66 - 2.54 (m, 4H), 2.44 - 2.23 (m, 3H), 2.06 - 1.91 (m, 3H), 1.86 - 1.75 (m, 2H), 1.70 - 1.56 (m, 2H), 1.24 (d, *J=* 6.5 Hz, 3H).

### Example 21 Synthesis of compound N-{[2-(2,6-oxohexahydropyridine-3-yl)-1-oxo-2,3-dihydro-1H-isoindol-5-yl]methyl}-6-(1-{1-[2-fluoro-4-({[3-({5-[(3S,4S)-4-amino-3-methyl-8-aza-2-oxaspiro[4.5]decan-8-yl]pyrazin-2-yl}thio)phenyl]amino}carbonyl)phenyl] hexahydropyridine-4-yl}-1,2,3,6-tetrahydropyridin-4-yl)-1,2-diazacyclohexane-3-formamide hydrochloride (compound 519)

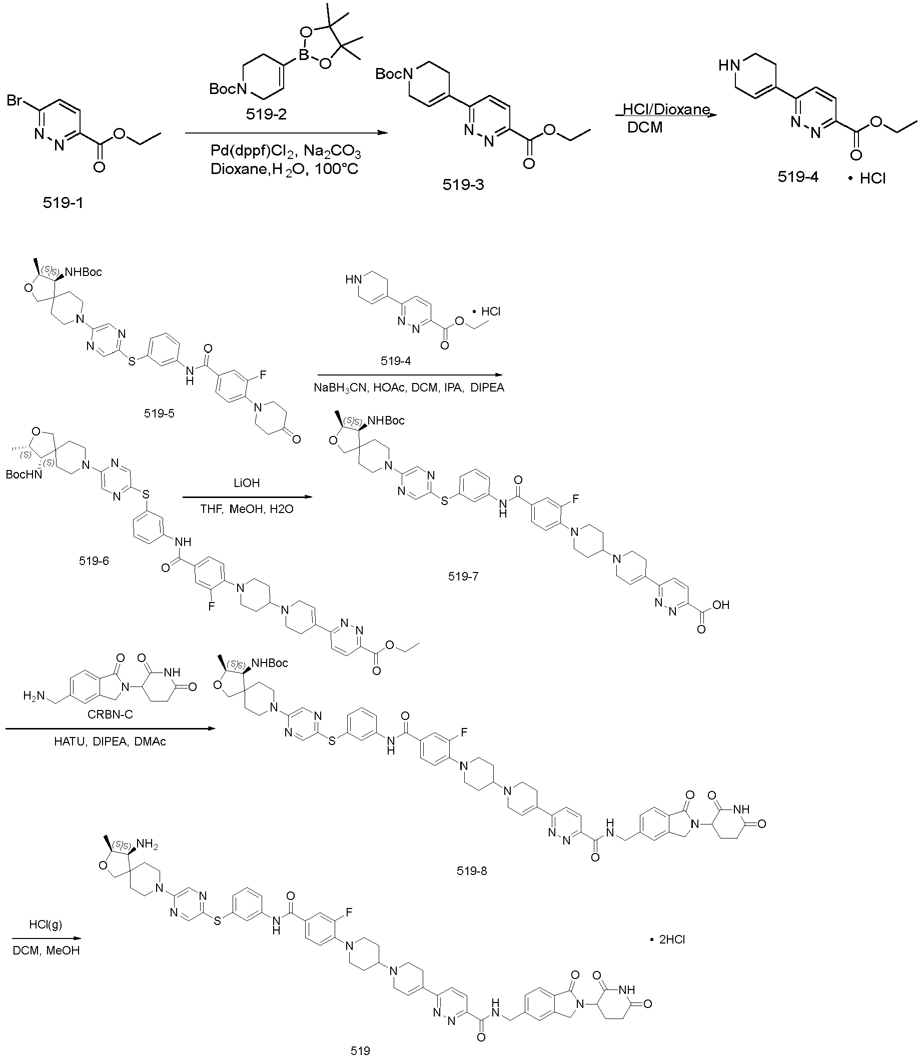

### Step 1: Synthesis of compound intermediate 4-[6-(ethoxycarbonyl)-1,2-diazacyclohexane-3-yl]-1,2,3,6-tetrahydropyridin-1-formic acid-2-methylpropyl-2-yl ester (519-3)

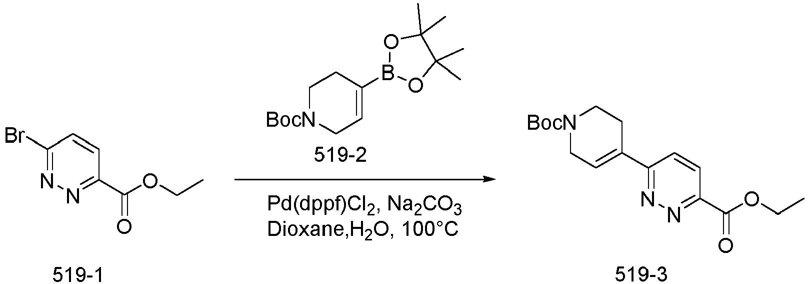

Ethyl 6-bromo-1,2-diazacyclohexane-3-formate (20.0 g, 0.11 mol), 4-(4,4,5,5-tetramethyl-1,3,2-dioxaborolane-2-yl)-1,2,3,6-tetrahydropyridin-1-formic acid-2-methylpropyl-2-yl ester (36.4 g, 0.12 mol), and Na₂CO₃ (22.8 g, 0.21 mol) were added into a mixed solvent of 1,4-dioxane (200 mL) and water (13 mL), and then the system was purged with argon three times, followed by addition of [1,1'-bis(diphenylphosphino)ferrocene]dichloropalladium (7.8 g, 0.01 mmol) under argon protection. The reaction system was heated to 100 °C and reacted for 12 h, and then the reaction solution was cooled to room temperature, and filtered over diatomaceous earth. The filtrate was concentrated under reduced pressure. The residue was purified by column chromatography to provide 18.4 g of the product (intermediate **519-3),** with a yield of 63.7%. MS: *m*/*z* 334.2 [M+H]⁺.

### Step 2. Synthesis of compound intermediate ethyl 6-(1,2,3,6-tetrahydropyridin-4-yl)-1,2-diazacyclohexane-3-formate hydrochloride (519-4)

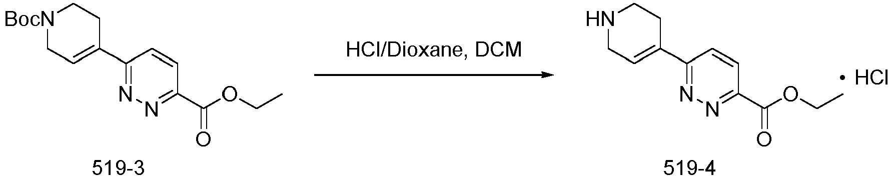

Intermediate **519-3** (18.4 g, 0.05 mol) was dissolved in 60 mL of dichloromethane, and then added into 100 mL of HCl-dioxane solution (4 mol/L). The mixture was allowed to react at room temperature for 3 h. The reaction solution was concentrated, and the residue was triturated with 50 mL of dichloromethane and filtered. The filter cake was sequentially rinsed once with 20 mL of dichloromethane and 20 mL of methyl tert-butyl ether, and then dried, to obtain 14.5 g of product (intermediate **519-4),** with a yield of 97.4%. MS: *m*/*z* 234.1 [M+H]⁺.

### Step 3. Synthesis of compound 519-5

The procedures are the same as that of intermediate **461-11.**

### Step 4. Synthesis of compound ethyl 6-(1-{1-[2-fluoro-4-({[3-({5-[(3S,4S)-3-methyl-4-({[(2-methylpropan-2-yl)oxy]carbonyl}amino)-8-aza-2-oxaspiro[4.5]decan-8-yl]pyrazin-2-yl}thio)phenyl]amino}carbonyl)phenyl]hexahydropyridine-4-yl}-1,2,3,6-tetrahydropyridin-4-yl)-1,2-diazacyclohexane-3-formate (519-6)

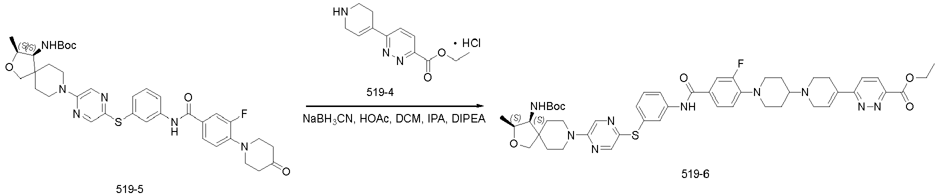

Intermediate **519-4** (222 mg, 0.87 mmol) and intermediate **519-5** (500 mg, 0.72 mmol) were successively added into a mixed solvent of dichloromethane (10 mL) and isopropanol (10 mL), and then the pH value of the system was adjusted to 7-8 with N,N-diisopropylethylamine. Subsequently, the pH was adjusted to 6 with acetic acid, followed by addition of sodium cyanodorohydride (91 mg, 1.45 mmol). The mixture was allowed to react at room temperature for 16 h. The reaction solution was quenched with 20 mL of saturated NaHCOs aqueous solution, and extracted with 50 mL of dichloromethane. The organic layer was dried with anhydrous sodium sulfate, filtered, and concentrated under reduced pressure. The residue was purified by column chromatography to obtain 370 mg of product (intermediate **519-6),** with a yield of 56.3%. MS: *m*/*z* 454.8 [M/2+H]⁺.

### Step 5. Synthesis of compound 6-(1-{1-[2-fluoro-4-({[3-({5-[(3S,4S)-3-methyl-4-({[(2-methylpropan-2-yl)oxy]carbonyl}amino)-8-aza-2-oxaspiro[4.5]decan-8-yl]pyrazin-2-yl}thio)phenyl]amino}carbonyl)phenyl]hexahydropyridine-4-yl}-1,2,3,6-tetrahydropyridin-4-yl)-1,2-diazacyclohexane-3-formic acid (519-7)

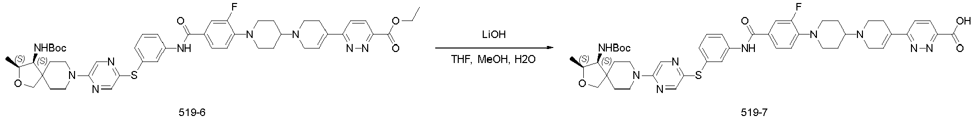

Intermediate **519-6** (370 mg, 0.41 mmol) and lithium hydroxide monohydrate (174 mg, 4.14 mmol) were successively added into a mixed solvent of tetrahydrofuran (4 mL), methanol (2 mL), and water (2 mL), and then the mixture was reacted at room temperature for 1 h. The pH value of the reaction system was adjusted to 5 with 1N HCl aqueous solution, and extracted with 30 mL of dichloromethane. The organic layer was dried with anhydrous sodium sulfate, filtered, and concentrated under reduced pressure to obtain 347 mg of product (intermediate **519-7),** with a yield of 96.8%. MS: *m*/*z* 440.8 [M/2+H]⁺.

Step 6. Synthesis of compound {[(3S,4S)-8-[5-({3-[({4-[4-(4-{6-[({[2-(2,6-oxohexahydropyridine-3-yl)-1-oxo-2,3-dihydro-1H-isoindol-5-yl]methyl}amino)carbonyl]-1,2-diazacyclohexane-3-yl}-1,2,3,6-tetrahydropyridin-1-yl)hexahydropyridine-1-yl]-3-fluorophenyl}carbonyl)amino]phenyl}thio)pyrazin-2-yl]-3-methyl-8-aza-2-oxaspiro[4.5]decan-4-yl]amino}formic acid-2-methylpropyl-2-yl ester **(519-8)**

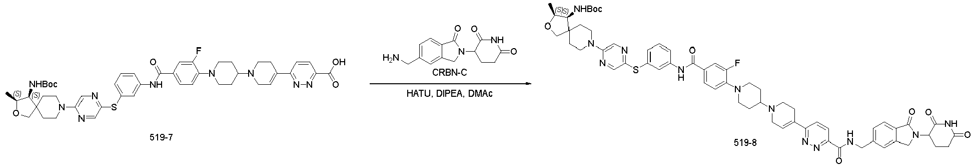

Intermediate **519-7** (347 mg, 0.39 mmol), 3-[5-(aminomethyl)-1-oxo-2,3-dihydro-1H-isoindol-2-yl]hexahydropyridine-2,6-dione (134 mg, 0.43 mmol), HATU (180 mg, 0.47 mmol), and N,N-diisopropylethylamine (127 mg, 0.98 mmol) were successively added into 4 mL of N,N-dimethylacetamide, and then the mixture was allowed to react at room temperature for 16 h. The reaction solution was slowly added into 40 mL of water dropwise, and filtered. The solid was purified by column chromatography to obtain 200 mg of product (intermediate **519-8)** with a yield of 44.7%. MS: *m*/*z* 568.4 [M/2+H]⁺.

### Step 7. Synthesis of compound N-{ [2-(2,6-oxohexahydropyridine-3-yl)-1-oxo-2,3-dihydro-1H-isoindol-5-yl]methyl}-6-(1-{1-[2-fluoro-4-({[3-({5-[(3S,4S)-4-amino-3-methyl-8-aza-2-oxaspiro[4.5]decan-8-yl]pyrazin-2-yl}thio)phenyl]amino}carbonyl)phenyl] hexahydropyridine-4-yl}-1,2,3,6-tetrahydropyridin-4-yl)-1,2-diazacyclohexane-3-formamide hydrochloride (compound 519)

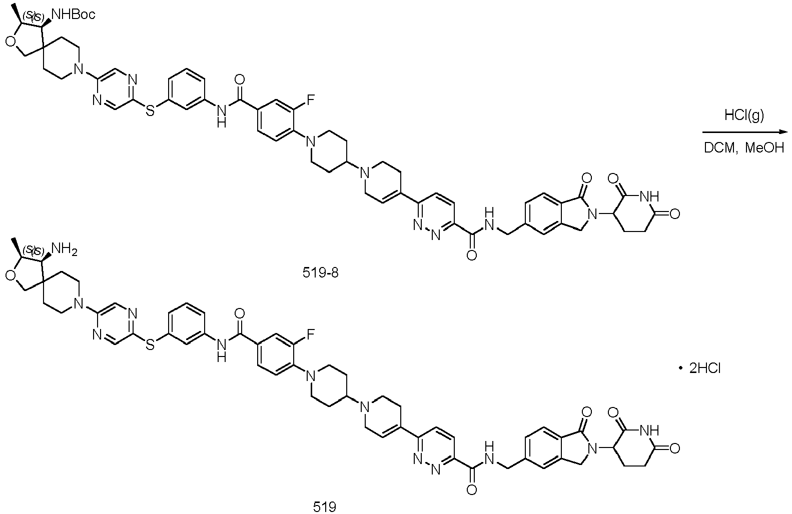

Intermediate **519-8** (200 mg, 0.18 mmol) was dissolved in a mixed solvent of dichloromethane (20 mL) and methanol (1 mL), to which was continuously introduced dry HCl gas at room temperature, and TLC indicated completion of the reaction. The reaction solution was concentrated under reduced pressure. The residue was triturated with 20 mL of dichloromethane, and filtered. The filter cake was sequentially rinsed with 5 mL of dichloromethane and 5 mL of methyl tert-butyl ether. The solid was dried to obtain 180 mg of product (compound **519),** with a yield of 92.2%. MS: *m*/*z* 518.3 [M/2+H]⁺.

¹H NMR (400 MHz, DMSO-*d*₆) *δ* 11.73 (s, 1H), 10.99 (s, 1H), 10.28 (s, 1H), 9.98 (t, *J*= 6.3 Hz, 1H), 8.43 - 8.30 (m, 4H), 8.27 - 8.17 (m, 3H), 7.84 - 7.66 (m, 5H), 7.57 (s, 1H), 7.53 - 7.48 (m, 1H), 7.29 (t, *J* = 7.9 Hz, 1H), 7.16 (t, *J* = 8.7 Hz, 1H), 7.01 - 6.95 (m, 2H), 5.10 (dd, *J* = 13.3, 5.1 Hz, 1H), 4.66 (d, *J* = 6.3 Hz, 2H), 4.47 - 4.26 (m, 2H), 4.26 - 4.02 (m, 5H), 3.93 (d, *J* = 9.0 Hz, 1H), 3.88 - 3.79 (m, 1H), 3.71 - 3.61 (m, 3H), 3.55 - 3.45 (m, 1H), 3.34 (t, *J* = 5.6 Hz, 1H), 3.29 - 3.18 (m, 1H), 3.17 - 3.02 (m, 4H), 2.96 - 2.77 (m, 3H), 2.63 - 2.54 (m, 1H), 2.44 - 2.22 (m, 3H), 2.07 - 1.91 (m, 3H), 1.87 - 1.74 (m, 2H), 1.70 - 1.56 (m, 2H), 1.24 (d, *J* = 6.5 Hz, 3H).

### Example 22 Synthesis of compound 1'-(1-(4-((3-((5-((3S,4S)-4-amino-3-methyl-2-oxa-8-azaspiro[4.5]decan-8-yl)pyrazin-2-yl)thio)phenyl)aminoformyl)-2-fluorophenyl) piperidin-4-yl)-N-((2-(2,6-dioxopiperidin-3-yl)-1-oxoisoindol-5-yl)methyl)-2-fluoro-1',2',3',6'-tetrahydro-[3,4'-bipyridine]-6-formamide hydrochloride (483)

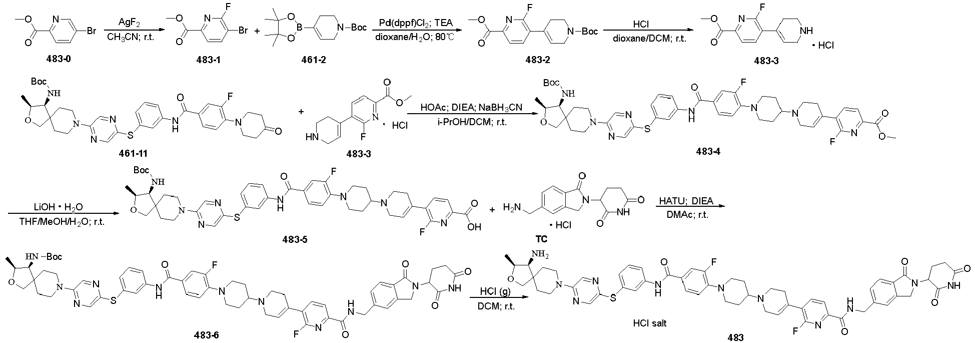

### Step 1. Synthesis of intermediate methyl 5-bromo-6-fluoropicolinate (483-1)

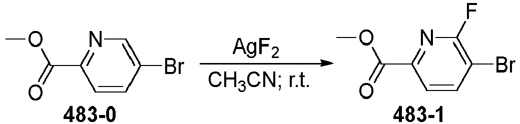

Methyl 5-bromopicolinate (2.15 g, 10 mmol) was added into a 100 ml single-necked flask, to which was added acetonitrile (22 ml), and then AgF2 (5.83 g, 40 mmol) was added under stirring at room temperature. After addition, the reaction solution was stirred for additional 16 h, and filtered over diatomaceous earth. The filter cake was rinsed with acetonitrile (22 mL), and then subjected to column chromatography, to obtain the target intermediate **483-1** (2.3 g). MS: *m*/*z* 234/236 [M+H]⁺.

### Step 2. Synthsis of intermediate 4-[2-fluoro-6-(methoxycarbonyl)pyridine-3-yl]-1,2,3,6-tetrahydropyridin-1-formic acid-2-methylpropyl-2-yl ester (483-2)

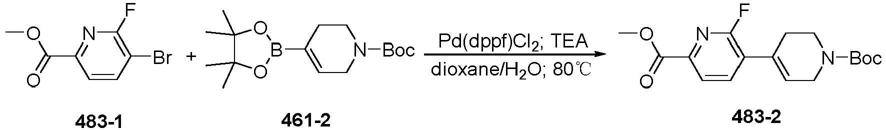

**483-1** (2 g, 8.5 mmol), **461-2** (2.9 g, 9.4 mmol), Pd(dppf)Cl₂ (0.31 g, 0.43 mmol), and TEA (1.72 g, 17 mmol) were added into dioxane/H₂O (10/1, 30 mL), and then under nitrogen protection, the mixture was stirred at 80 °C. The progress of the reaction was detectd with TLC. After completion of the reaction, the reaction solution was diluted with water (30 mL), and then extracted with EA (30 mL × 3). Purification by column chromatography provided the target compound **483-2** (2.4 g). MS: *m*/*z* 337 [M+H]⁺.

### Step 3. Synthesis of intermediate methyl 6-fluoro-5-(1,2,3,6-tetrahydropyridin-4-yl)pyridine-2-formate hydrochloride (483-3)

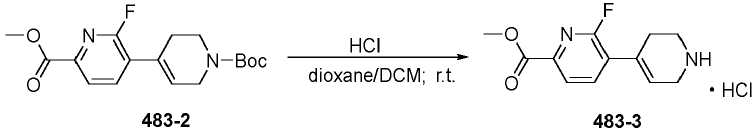

**483-2** (2.4 g, 7.1 mmol) was dissolved in dichloromethane (24 mL), to which was added the solution of HCl in dioxane (24 mL), and then the mixture was stirred for 2 h at room temperature. The reaction solution was rotatory evaporated, to obtain the target compound **483-3** (2.2 g). MS: *m*/*z* 237 [M+H]⁺.

### Step 4. Synthesis of intermediate methyl 1'-(1-(4-((3-((5-((3S,4S)-4-((tert-butoxycarbonyl)amino)-3-methyl-2-oxa-8-azaspiro[4.5]decan-8-yl)pyrazin-2-yl)thio)phenyl) aminoformyl)-2-fluorophenyl)piperidin-4-yl)-2-fluoro-1',2',3',6'-tetrahydro-[3,4'-bipyridine]-6-formate (483-4)

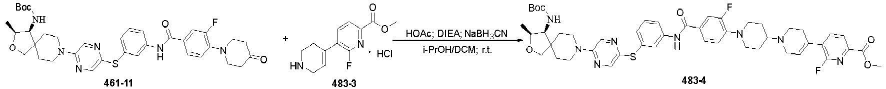

**461-11** (2 g, 2.9 mmol) and **483-3** (0.95 g, 3.5 mmol) were added into a mixed solvent of isopropanol (20 mL) and dichloromethane (20 mL), to which were successively added DIEA (0.57 g, 4.4 mmol), HOAc (1.04 g, 17.4 mmol), and sodium cyanoborohydride (0.73 g, 11.6 mmol) at room temperature. After addition, the mixture was continually stirred, and the reaction was detected with TLC. After completion of the reaction, saturated NaHCOs aqueous solution (40 mL) was added, and the resultant solution was extracted with dichloromethane (40 mL×2). Purification by column chromatography afforded the target compound **483-4** (1.6 g). MS: *m*/*z* 911 [M+H]⁺.

### Step 5. Synthesis of intermediate 1'-(1-(4-((3-((5-((3 S,4S)-4-((tert-butoxycarbonyl)amino)-3-methyl-2-oxa-8-azaspiro[4.5]decan-8-yl)pyrazin-2-yl)thio)phenyl)aminoformyl)-2-fluorophenyl)piperidin-4-yl)-2-fluoro-1_{'},2',3',6'-tetrahydro-[3,4'-bipyridine]-6-formic acid (483-5)

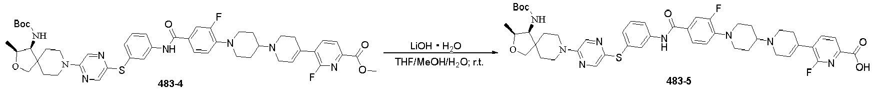

**483-4** (1.6 g, 1.76 mmol) was placed in a 50 mL single-necked flask, to which were added tetrahydrofuran (10 mL), methanol (2.5 mL), water (2.5 mL), and lithium hydroxide monohydrate (0.59 g, 14.07 mmol), and then the mixture was stirred at room temperature for 2 h. The pH of the reaction solution was adjusted to neutral with HCl (1N). The organic solvent was removed by rotatory evaporation. To the residue, was added 10 mL of water, and then the pH was adjusted to 2 with HCl (1N), followed by filtration. The filter cake was washed with water, and air dried, to obtain the target compound **483-5** (1.28 g). MS: *m*/*z* 897 [M+H]⁺.

### Step 6. Synthesis of intermediate tert-butyl ((3S,4S)-8-(5-((3-(4-(4-(6-(((2-(2,6-dioxopiperidin-3-yl)-1-oxoisoindol-5-yl)methyl)aminoformyl)-2-fluoro-3',6'-dihydro-[3,4'-bipiperidine]-1'(2'H)-yl)piperidin-1-yl)-3-fluorobenzamido)phenyl)thio)pyrazin-2-yl)-3-methyl-2-oxa-8-azaspiro[4.5]decan-4-yl)carbamate (486-6)

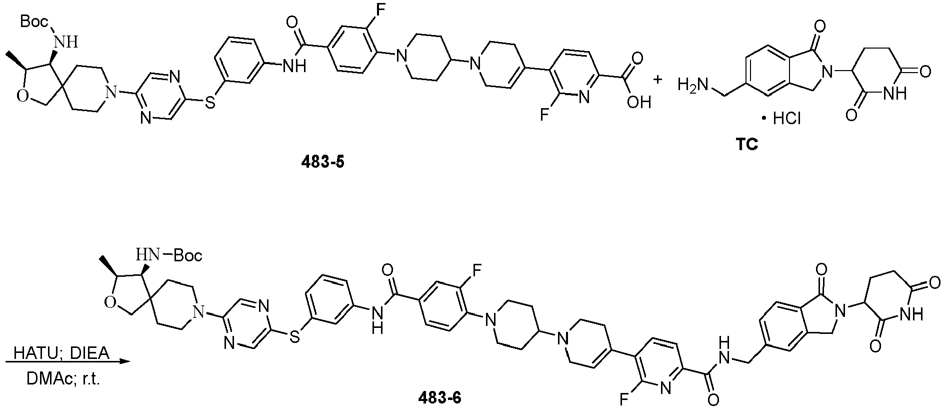

**483-5** (1.28 g, 1.4 mmol), compound TC (0.5 g, 1.6 mmol), HATU (0.64 g, 1.7 mmol) and DIEA (0.45 g, 3.5 mmol) were added into DMAc (15 mL), and then the mixture was stirred at room temperature. The reaction was detected with TLC. After completion of the reaction, the reaction solution was added to water (60 mL) dropwise, and filtered. The filter cake was rinsed with water (10 mL), and subjected to column chromatography, to obtain the target compound **486-6** (1 g). MS: *m*/*z* 1152 [M+H]⁺.

### Step 7. Synthesis of compound 1'-(1-(4-((3-((5-((3 S,4S)-4-amino-3-methyl-2-oxa-8-azaspiro[4.5]decan-8-yl)pyrazin-2-yl)thio)phenyl)aminoformyl)-2-fluorophenyl)piperidin-4-yl)-N-((2-(2,6-dioxopiperidin-3-yl)-1-oxoisoindol-5-yl)methyl)-2-fluoro-1',2',3',6'-tetrahydro-[3,4'-bipyridine]-6-formamide hydrochloride (483)

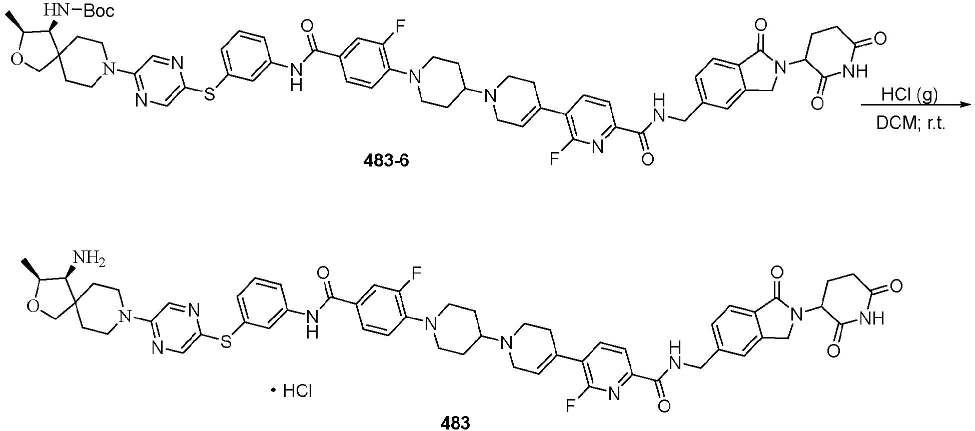

Compound **483-6** (1 g, 0.86 mmol) was dissolved in DCM (20 mL), to which was continuously introduced HCl gas at room temperature under stirring. The reaction was monitored by TLC. After completion of the reaction, the reaction solution was rotatory evaporated, and then the residue was dissolved in DCM, followed by rotatory evaporation, that was repeated once, to obtain compound **483** (1 g). MS: *m*/*z* 1052 [M+H]⁺.

¹H NMR: (400 MHz, DMSO-*d*₆) *δ* 11.61 (s, 1H), 10.99 (s, 1H), 10.27 (s, 1H), 9.45 (t, *J* = 6.3 Hz, 1H), 8.39 (d, *J* = 1.4 Hz, 1H), 8.37 - 8.22 (m, 3H), 8.21 - 8.11 (m, 2H), 8.01 (dd, *J* = 7.7, 1.8 Hz, 1H), 7.84 - 7.77 (m, 2H), 7.77 - 7.70 (m, 2H), 7.68 (d, *J* = 7.9 Hz, 1H), 7.53 (s, 1H), 7.50 - 7.41 (m, 1H), 7.29 (t, *J* = 8.0 Hz, 1H), 7.16 (t, *J* = 8.7 Hz, 1H), 7.01 - 6.92 (m, 1H), 6.33 (s, 1H), 5.10 (dd, *J* = 13.3, 5.1 Hz, 1H), 4.58 (d, *J* = 6.3 Hz, 2H), 4.43 (d, *J* = 17.4 Hz, 1H), 4.29 (d, *J* = 17.5 Hz, 1H), 4.25 - 4.04 (m, 3H), 3.93 (d, *J* = 9.0 Hz, 2H), 3.75 (s, 1H), 3.65 (t, *J* = 11.1 Hz, 3H), 3.48 (s, 1H), 3.35 (t, *J* = 5.6 Hz, 1H), 3.19 (d, *J* = 23.2 Hz, 1H), 3.06 (s, 4H), 2.86 (dt, *J* = 32.3, 12.6 Hz, 3H), 2.69 (d, *J* = 15.9 Hz, 1H), 2.59 (d, *J* = 16.9 Hz, 1H), 2.44 - 2.19 (m, 3H), 1.99 (d, *J* = 12.3 Hz, 3H), 1.80 (t, *J* = 12.2 Hz, 2H), 1.72 - 1.52 (m, 2H), 1.24 (d, *J* = 6.6 Hz, 3H).

### Example 23 Synthesis of compound 4-(1-(1-(4-((3-((3S,4S)-4-amino-3-methyl-2-oxo-8-azaspiro[4.5]decan-8-yl)pyrazin-2-yl)thio)phenyl)carbamoyl)-2-fluorophenyl)piperidin-4-yl)piperazin-1-yl)-N-((2-(2,6-dioxopiperidin-3-yl)-1-oxoisoquinolin-5-yl)methyl)-2-fluorobenzamide hydrochloride (501)

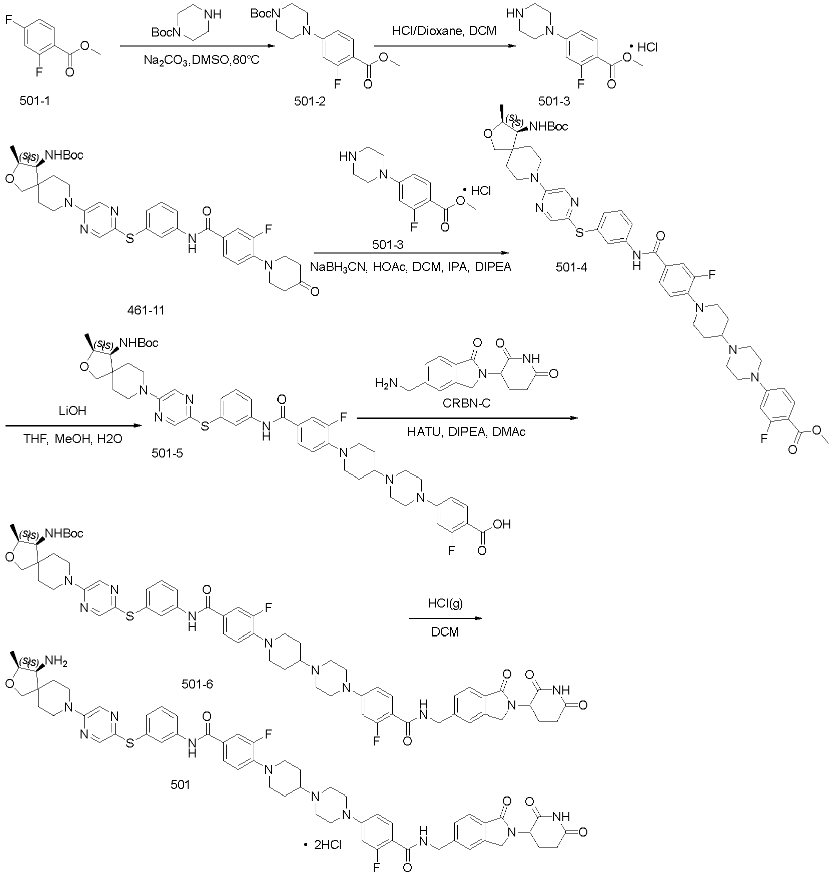

### Step 1. Synthesis of intermediate tert-butyl 4-(3-fluoro-4-(methoxycarbonyl)phenyl)piperazin-1-carboxylate (501-2)

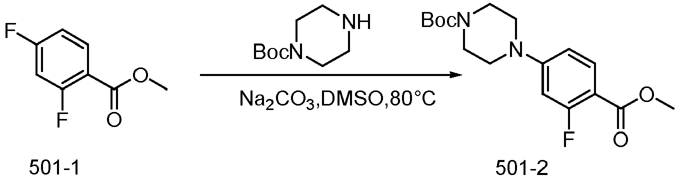

Methyl 2,4-difluorobenzoate (1.72 g, 10 mmol), 1-Boc-piperazine (1.86 g,10 mmol), and Na₂CO₃ (2.12 g, 20 mmol) were successively added into 15 mL of dimethylsulfoxide, and then the reaction system was heated to 80 °C and reacted for 16 h. The reaction solution was cooled to room temperature, and poured into 100 mL of water, followed by extraction with ethyl acetate. The obtained organic phase was dried with anhydrous sodium sulfate. After the solvent was evaporated under reduced pressure, the crude product was recrystallized in ethyl acetate, and filtered to remove the filtrate. The filter cake was dried to obtain 1.62 g of product (intermediate **501-2),** with a yield of 48%. MS: *m*/*z* 339.1 [M+H]⁺.

### Step 2. Synthesis of intermediate methyl 2-fluoro-4-(piperazin-1-yl)benzoate hydrochloride (501-3)

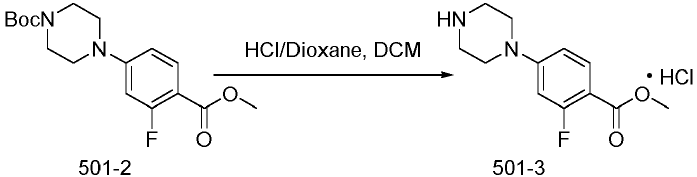

Intermediate **501-2** (1.02 g, 3 mmol) was dissolved in 30 mL of dichloromethane, to which was added 30 mL of HCl-dioxane solution (4 mol/L), and the mixture was allowed to react at room temperature for 2 h. The reaction solution was concentrated, and the residue was triturated with 10 mL of dichloromethane and filtered. The filter cake was sequentially rinsed with 10 mL of dichloromethane and 10 mL of methyl tert-butyl ether, and then dried, to obtain 783 mg of product (intermediate **501-3),** with a yield of 95%. MS: *m*/*z* 239.1 [M+H]⁺.

### Step 3. Synthesis of intermediate methyl 4-(4-(1-(4-((3-((5-((3S,4S)-4-((tert-butoxycarbonyl)amino)-3-methyl-2-oxa-8-azaspiro[4.5]decan-8-yl)pyrazin-2-yl)thio)phenyl) carbamoyl)-2-fluorophenyl)piperidin-4-yl)piperazin-1-yl)-2-fluorobenzoate (501-4)

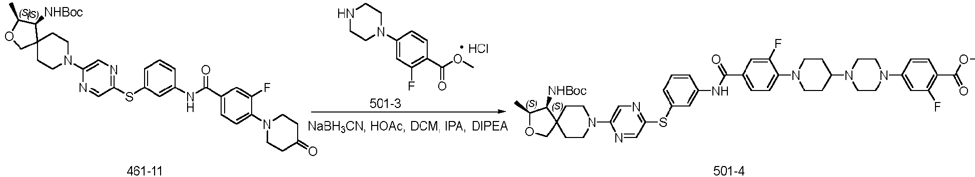

Intermediate **461-11** (500 mg, 0.72 mmol) and intermediate **501-3** (239 mg, 0.87 mmol) were successively added into a mixed solvent of dichloromethane (10 mL) and isopropanol (10 mL), and then the pH value of the system was adjusted to 7-8 with N,N-diisopropylethylamine. Subsequently, the pH was adjusted to 6 with acetic acid, followed by addition of sodium cyanodorohydride (91 mg, 1.45 mmol). The mixture was allowed to react at room temperature for 16 h. The reaction solution was quenched with 20 mL of saturated NaHCOs aqueous solution, and extracted with 50 mL of dichloromethane. The organic layer was dried with anhydrous sodium sulfate, filtered, and concentrated under reduced pressure. The residue was purified by column chromatography to obtain 437 mg of product (intermediate **501-4),** with a yield of 55%. MS: *m*/*z* 913.4 [M+H]⁺.

### Step 4. Synthesis of intermediate methyl 4-(4-(1-(4-((3-((5-((3S,4S)-4-((tert-butoxycarbonyl)amino)-3-methyl-2-oxa-8-azaspiro[4.5]decan-8-yl)pyrazin-2-yl)thio)phenyl) carbamoyl)-2-fluorophenyl)piperidin-4-yl)piperazin-1-yl)-2-fluorobenzoate (501-5)

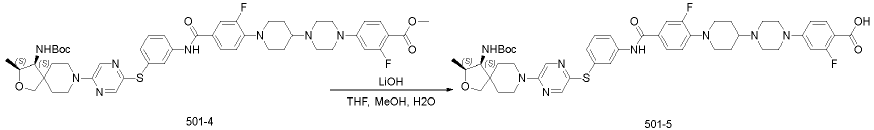

Intermediate **501-4** (365 mg, 0.40 mmol) and lithium hydroxide monohydrate (166 mg, 3.97 mmol) were successively added into a mixed solvent of tetrahydrofuran (4 mL), methanol (2 mL), and water (2 mL), and then the mixture was reacted at room temperature for 1 h. The pH value of the reaction system was adjusted to 4 with 1N HCl aqueous solution, and extracted with 30 mL of dichloromethane. The organic layer was dried with anhydrous sodium sulfate, filtered, and concentrated under reduced pressure to obtain 360 mg of product (intermediate **501-5,** with a yield of 95%. MS: *m*/*z* 899.4 [M+H]⁺.

### Step 5. Synthesis of intermediate tert-butyl ((3S,4S)-8-(5-(3-(4-(4-(4-(4-(4-(3-yl)-1-oxoisoquinolin-5-yl)methyl)carbamoyl)-3-fluorophenyl)piperazin-1-yl)piperidin-1-yl)-3-fluorobenzamido)phenyl)thio)pyrazin-2-yl)-3-methyl-2-oxo-8-azaspiro[4.5]decan-4-yl)carbamate (501-6)

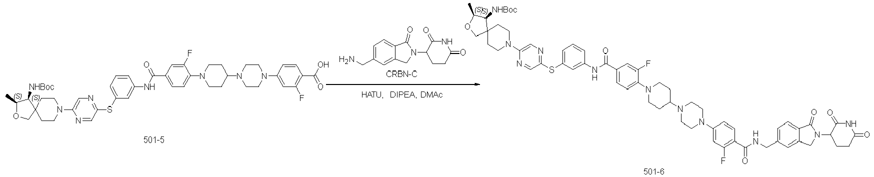

Intermediate **501-5** (342 mg, 0.38 mmol), 3-[5-(aminomethyl)-1-oxo-2,3-dihydro-1H-isoindol-2-yl]hexahydropyridine-2,6-dione (105 mg, 0.38 mmol), HATU (175 mg, 0.46 mmol), and N,N-diisopropylethylamine (124 mg, 0.96 mmol) were successively added into 4 mL of N, N-dimethylacetamide, and then the mixture was allowed to react at room temperature for 16 h. The reaction solution was slowly added into 40 mL of water dropwise, and filtered. The solid was purified by column chromatography to obtain 232 mg of product (intermediate **501-6**), with a yield of 53%. MS: *m*/*z* 574.8 [M/2+H]⁺.

### Step 6. Synthesis of compound 4-(1-(1-(4-((3-((3S, 4S)-4-amino-3-methyl-2-oxo-8-azaspiro[4.5]decan-8-yl)pyrazin-2-yl)thio)phenyl)carbamoyl)-2-fluorophenyl)piperidin-4-yl)piperazin-1-yl)-N-((2-(2,6-dioxopiperidin-3-yl)-1-oxoisoquinolin-5-yl)methyl)-2-fluorobenzamide hydrochloride(501)

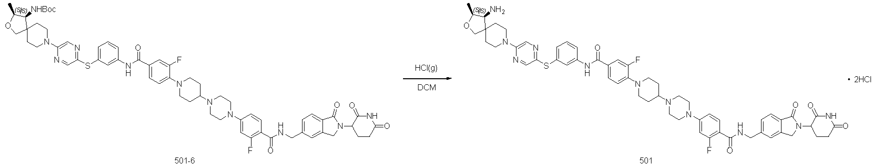

Intermediate **501-6** (231 mg, 0.20 mmol) was dissolved in 25 mL of dichloromethane, to which was continuously introduced dry HCl gas at room temperature, and TLC indicated completion of the reaction. The reaction solution was concentrated under reduced pressure. The residue was triturated with 25 mL of dichloromethane, and filtered. The filter cake was sequentially rinsed with 5 mL of dichloromethane and 5 mL of methyl tert-butyl ether. The solid was dried to obtain 220 mg of product (compound **501),** with a yield of 98.0%. MS: *m*/*z* 1054.5 [M+H]⁺.

¹H NMR (400 MHz, DMSO-*d*₆) *δ* 11.78 (s, 1H), 11.00 (s, 1H), 10.24 (s, 1H), 8.63 (q, *J* = 5.8 Hz, 1H), 8.45-8.14 (m, 5H), 7.87-7.64 (m, 6H), 7.59 -7.41 (m, 2H), 7.23 (dt, *J* = 53.8, 8.4 Hz, 2H), 7.02-6.85 (m, 3H), 5.11 (dd, *J* = 13.3, 5.1 Hz, 1H), 4.56 (d, *J* = 5.9 Hz, 2H), 4.44 (d, J= 17.4 Hz, 1H), 4.36-4.11 (m, 4H), 4.03 (d, *J* = 12.8 Hz, 2H), 3.92 (d, *J* = 9.1 Hz, 1H), 3.72 -3.57 (m, 5H), 3.48-3.31 (m, 4H), 3.23-3.02 (m, 4H), 2.98-2.73 (m, 3H), 2.64-2.54 (m, 1H), 2.46-2.23 (m, 3H), 1.97 (dt, *J=* 16.9, 6.8 Hz, 3H), 1.87 -1.74 (m, 2H), 1.71-1.56 (m, 2H), 1.24 (d, *J=* 6.6 Hz, 3H).

By using a synthesis method similar to the above examples, the compounds listed in Table 1 could be synthesized by selecting appropriate reactants, reagents, and reaction conditions.

**Table 1. List of compounds.**

| ID | Structure | LC-MS (M+1) | ¹H NMR |
|---|---|---|---|
| **1** | 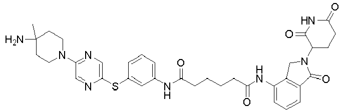 | 685 | |
| **2** | 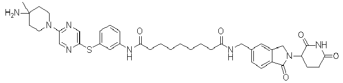 | 741 | |
| **4** | 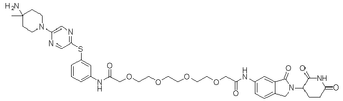 | 805 | |
| **5** | 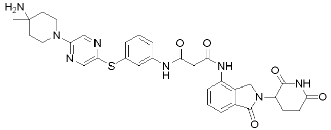 | 643 | |
| **6** | 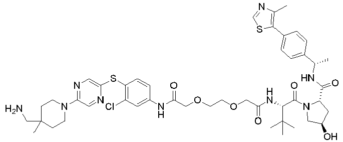 | 950 | |
| **7** | 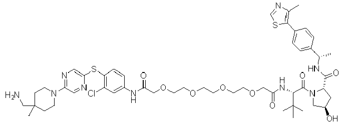 | 1038 | |
| **8** | 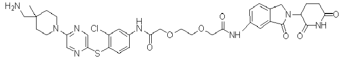 | 765 | |
| **9** | 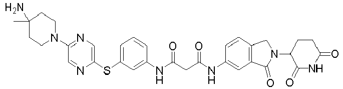 | 643 | |
| **10** | 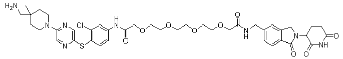 | 853 | |
| 11 | 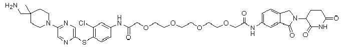 | 853 | |
| **999** | 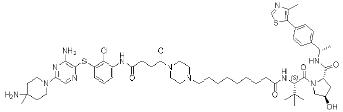 | 1115 | |
| **12** | 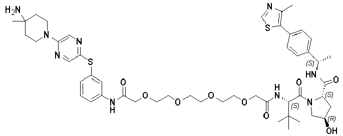 | 990 | |
| **13** | 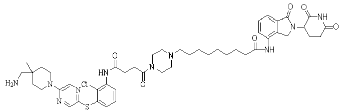 | 929 | |
| **14** | 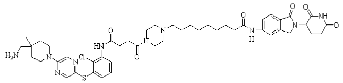 | 929 | |
| **16** | 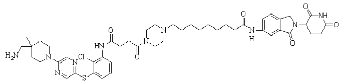 | 929 | |
| **17** | 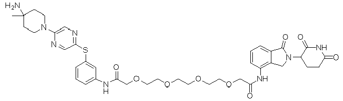 | 805 | |
| **18** | 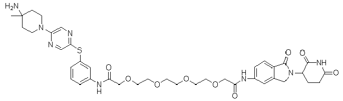 | 805 | |
| **19** | 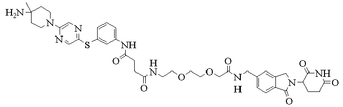 | 816 | |
| **20** | 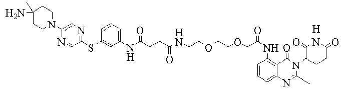 | 829 | |
| **21** | 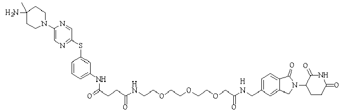 | 860 | |
| **22** | 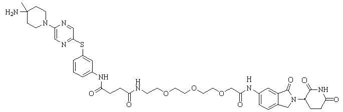 | 846 | |
| **23** | 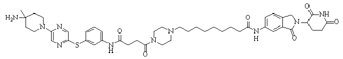 | 881 | |
| **24** | 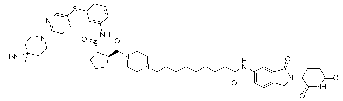 | 921 | ¹H NMR (400 MHz, DMSO-*d*₆) δ 10.99 (s, 1H), 10.14 (d, *J* = 2.7 Hz, 1H), 10.06 (s, 1H), 8.38 (d, *J* = 1.5 Hz, 1H), 8.16 (d, *J* = 1.4 Hz, 1H), 8.11 (d, *J* = 1.9 Hz, 1H), 7.88 (s, 2H), 7.70 (dd, *J* = 8.3, 2.0 Hz, 1H), 7.60 (t, *J* = 1.9 Hz, 1H), 7.54 - 7.46 (m, 2H), 7.24 (t, *J* = 8.0 Hz, 1H), 6.92 (m, 1H), 5.10 (dd, *J* = 13.3, 5.1 Hz, 1H), 4.44 - 4.21 (m, 2H), 4.03 (m, 2H), 3.49 - 3.25 (m, 5H), 3.13 (q, *J* = 8.4 Hz, 1H), 2.91 (m, 1H), 2.60 (m, 1H), 2.45 - 2.16 (m, 9H), 2.08 - 1.92 (m, 3H), 1.66 (m, 10H), 1.41 - 1.21 (m, 15H). |
| **25** | 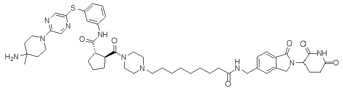 | 935 | ¹H NMR (400 MHz, DMSO-*d*₆) δ 10.05 (s, 1H), 8.42 (t, *J* = 6.0 Hz, 1H), 8.38 (d, *J* = 1.4 Hz, 1H), 8.16 (d, *J* = 1.4 Hz, 1H), 7.67 (d, *J* = 7.8 Hz, 1H), 7.59 (t, *J* = 2.0 Hz, 1H), 7.48 (dd, *J* = 7.7, 2.0 Hz, 1H), 7.45 (s, 1H), 7.38 (dd, *J* = 7.9, 1.4 Hz, 1H), 7.23 (t, *J =* 8.0 Hz, 1H), 6.91 (dt, *J* = 8.0, 1.3 Hz, 1H), 5.11 (dd, *J* = 13.3, 5.1 Hz, 1H), 4.47 - 4.25 (m, 4H), 3.97 (m, 2H), 3.52 - 3.25 (m, 5H), 3.13 (q, *J* = 8.5 Hz, 1H), 2.92 (m, 1H), 2.60 (m, 1H), 2.45 - 2.31 (m, 1H), 2.31 - 2.09 (m, 8H), 2.06 - 1.92 (m, 3H), 1.77 - 1.44 (m, 10H), 1.41 - 1.18 (m, 15H). |
| **26** | 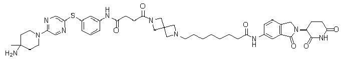 | 879 | |
| **27** | 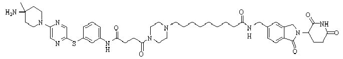 | 895 | |
| **28** | 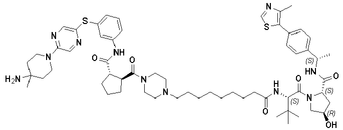 | 1106 | ¹H NMR (400 MHz, DMSO-*d*₆) δ 10.03 (s, 1H), 8.98 (s, 1H), 8.39 (d, *J* = 7.8 Hz, 1H), 8.33 (d, *J* = 1.5 Hz, 1H), 8.13 (d, *J* = 1.4 Hz, 1H), 7.80 (d, *J* = 9.3 Hz, 1H), 7.52 (t, *J* = 2.0 Hz, 1H), 7.50 - 7.31 (m, 5H), 7.21 (t, *J* = 8.0 Hz, 1H), 6.89 (dt, *J* = 7.8, 1.3 Hz, 1H), 5.11 (s, 1H), 4.91 (p, *J* = 7.1 Hz, 1H), 4.51 (d, *J* = 9.3 Hz, 1H), 4.41 (t, *J* = 8.0 Hz, 1H), 4.27 (d, *J* = 4.0 Hz, 1H), 3.78 - 3.66 (m, 2H), 3.65 - 3.52 (m, 4H), 3.48 - 3.39 (m, 4H), 3.11 (q, *J* = 8.4 Hz, 2H), 2.45 (s, 3H), 2.28 - 1.95 (m, 12H), 1.83 - 1.69 (m, 2H), 1.66 - 1.54 (m, 3H), 1.48 - 1.41 (m, 5H), 1.39 - 1.30 (d, *J* = 7.0 Hz, 5H), 1.27 -1.18 (m, 10H), 1.11 (s, 3H), 0.93 (s, 9H). |
| **29** | 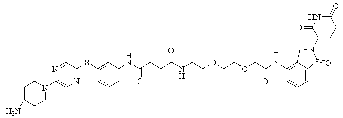 | 802 | |
| **30** | 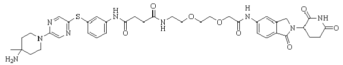 | 802 | |
| **31** | 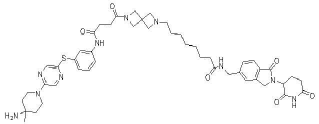 | 893 | |
| **32** | 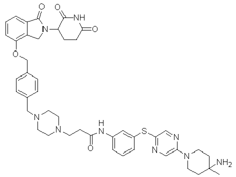 | 818 | |
| **33** | 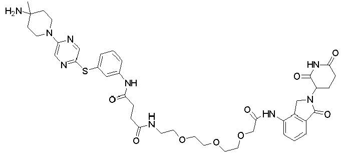 | 846 | |
| **34** | 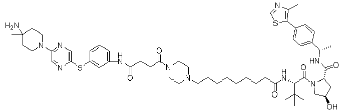 | 1066 | |
| **35** | 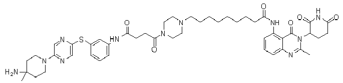 | 908 | |
| **36** | 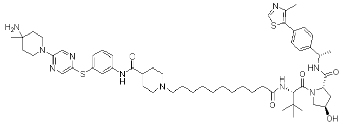 | 1037 | ¹H NMR (400 MHz, DMSO-*d*₆) δ 10.03 (s, 1H), 8.98 (s, 1H), 8.39 (d, *J* = 7.8 Hz, 1H), 8.33 (d, *J* = 1.5 Hz, 1H), 8.13 (d, *J* = 1.4 Hz, 1H), 7.80 (d, *J* = 9.3 Hz, 1H), 7.52 (t, *J* = 2.0 Hz, 1H), 7.50 - 7.31 (m, 5H), 7.21 (t, *J* = 8.0 Hz, 1H), 6.89 (dt, *J* = 7.8, 1.3 Hz, 1H), 5.11 (s, 1H), 4.91 (p, *J* = 7.1 Hz, 1H), 4.51 (d, *J=* 9.3 Hz, 1H), 4.41 (t, *J* = 8.0 Hz, 1H), 4.27 (d, *J* **=** 4.0 Hz, 1H), 3.78 - 3.66 (m, 2H), 3.65 - 3.52 (m, 2H), 3.48 - 3.39 (m, 2H), 3.11 (q, *J* = 8.4 Hz, 2H), 2.45 (s, 3H), 2.28 - 1.95 (m, 8H), 1.83 - 1.69 (m, 2H), 1.66 - 1.54 (m, 3H), 1.48 - 1.41 (m, 4H), 1.39 -1.30 (d, *J* = 7.0 Hz, 5H), 1.27 -1.18 (m, 16H), 1.11 (s, 3H), 0.93 (s, 9H). |
| **37** | 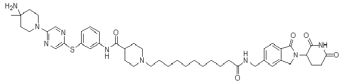 | 866 | ¹H NMR (400 MHz, DMSO-*d*₆) δ 11.01 (s, 1H), 10.04 (s, 1H), 8.44 (t, *J* = 6.1 Hz, 1H), 8.38 (s, 1H), 8.18 (s, 1H), 7.67 (d, *J* = 7.8 Hz, 1H), 7.59 (d, *J* = 2.0 Hz, 1H), 7.48 (d, *J* = 8.1 Hz, 1H), 7.45 (s, 1H), 7.38 (d, *J* = 7.9 Hz, 1H), 7.24 (t, *J* = 8.0 Hz, 1H), 6.91 (d, *J* = 7.9 Hz, 1H), 5.11 (dd, *J* = 13.3, 5.1 Hz, 1H), 4.48 - 4.26 (m, 4H), 4.12 - 4.00 (m, 2H), 3.51 - 3.35 (m, 2H), 3.12 (s, 2H), 2.98 - 2.85 (m, 1H), 2.65 - 2.54 (m, 2H), 2.42 - 2.35 (m, 2H), 2.14 (t, *J* = 7.3 Hz, 3H), 2.05 - 1.94 (m, 2H), 1.87 - 1.66 (m, 6H), 1.58 - 1.43 (m, 4H), 1.37 (s, 3H), 1.24 (s, 17H). |
| **38** | 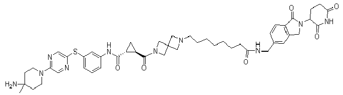 | 905 | |
| **39** | 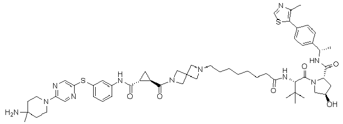 | 1076 | |
| **40** | 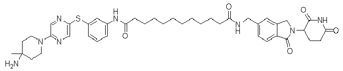 | 783 | |
| **41** | 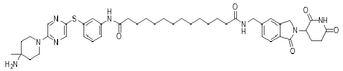 | 811 | |
| **42** | 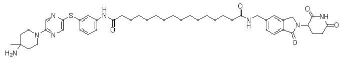 | 839 | |
| **43** | 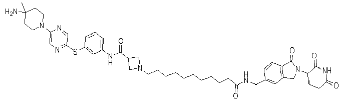 | 838 | |
| **44** | 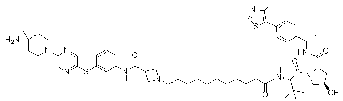 | 1009 | |
| **45** | 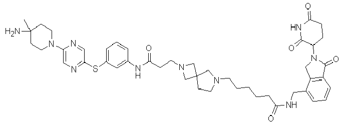 | 851 | |
| **46** | 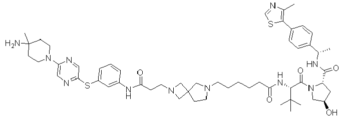 | 1022 | |
| **47** | 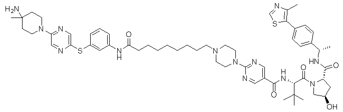 | 1088 | |
| **49** | 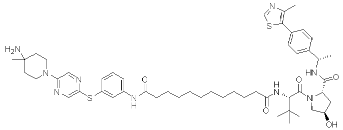 | 954 | |
| **50** | 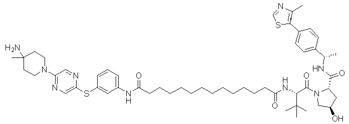 | 982 | |
| **51** | 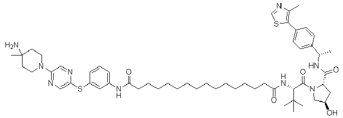 | 1010 | |
| **52** | 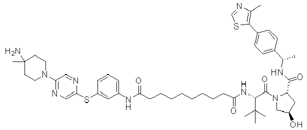 | 926 | |
| **53** | 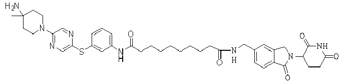 | 755 | |
| **54** | 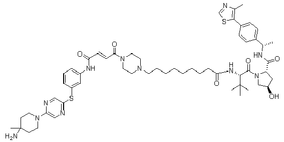 | 1064 | |
| **55** | 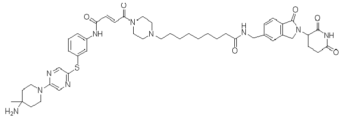 | 893 | |
| **56** | 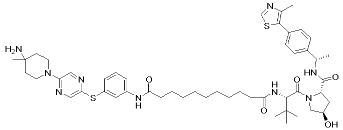 | 470.8 (M/2+ 1) | |
| **57** | 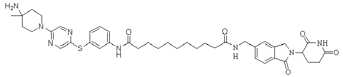 | 769.3 | |
| **58** | 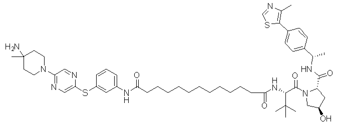 | 484.9( M/2+ 1) | |
| **59** | 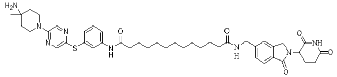 | 770 | |
| **60** | 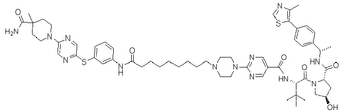 | 1116 | |
| **61** | 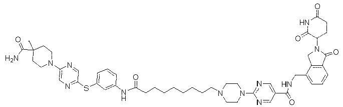 | 945 | |
| **62** | 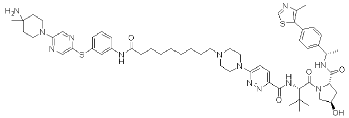 | 1088 | ¹H NMR (400 MHz, DMSO-*d*₆) δ 9.97 (s, 1H), 8.98 (s, 1H), 8.49 (d, *J* = 7.7 Hz, 1H), 8.38 (s, 1H), 8.34 (d, *J* = 9.7 Hz, 1H), 8.17 (s, 1H), 8.14 (s, 2H), 7.85 (d, *J* = 9.5 Hz, 1H), 7.58 (s, 1H), 7.48 - 7.32 (m, 6H), 7.23 (t, *J* = 8.0 Hz, 1H), 6.90 (d, *J* = 7.8 Hz, 1H), 5.18 (s, 1H), 4.91 (t, *J* = 7.3 Hz, 1H), 4.71 (d, *J* = 9.8 Hz, 1H), 4.46 (t, *J* = 8.2 Hz, 1H), 4.30 (s, 1H), 4.07-4.01 (m, 2H), 3.76-3.61 (m, 6H), 2.45 (s, 3H), 2.35 - 2.23 (m, 4H), 2.10 - 2.05 (m, 1H), 1.82 - 1.68 (m, 5H), 1.59 - 1.42 (m, 5H), 1.40 - 1.31 (m, 7H), 1.28 - 1.23 (m, 12H), 1.00 (s, 9H). |
| **63** | 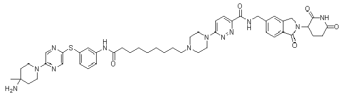 | 917 | ¹H NMR (400 MHz, DMSO-*d*₆) δ 10.98 (s, 1H), 9.96 (s, 1H), 9.55 (t, *J* = 6.4 Hz, 1H), 8.38 (d, *J* = 1.5 Hz, 1H), 8.17 (d, *J* = 1.3 Hz, 1H), 8.14 (s, 2H), 7.85 (d, *J* = 9.5 Hz, 1H), 7.68 (d, *J* = 7.8 Hz, 1H), 7.57 (d, *J* = 2.0 Hz, 1H), 7.53 (s, 1H), 7.47 (dd, *J* = 8.3, 2.3 Hz, 2H), 7.36 (d, *J* = 9.6 Hz, 1H), 7.23 (t, *J* = 8.0 Hz, 1H), 6.90 (dd, *J* = 7.8, 1.9 Hz, 1H), 5.10 (dd, *J* = 13.3, 5.1 Hz, 1H), 4.60 (d, *J* = 6.3 Hz, 2H), 4.47 - 4.25 (m, 2H), 4.09 - 4.01 (m, 2H), 3.71 (s, 4H), 2.96 - 2.85 (m, 1H), 2.65 - 2.53 (m, 3H), 2.44 - 2.31 (m, 1H), 2.27 (t, *J* = 7.4 Hz, 2H), 2.03 - 1.94 (m, 2H), 1.81 - 1.68 (m, 4H), 1.60 - 1.42 (m, 5H), 1.37 (s, 3H), 1.35 - 1.24 (m, 12H). |
| **64** | 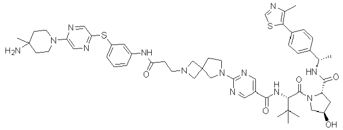 | 1030 | |
| **65** | 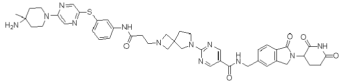 | 859 | |
| **66** | 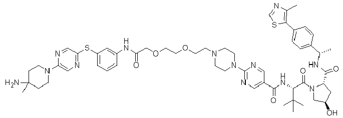 | 1078 | |
| **67** | 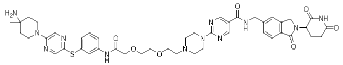 | 907 | |
| **69** | 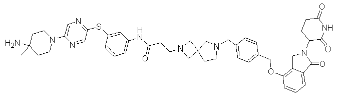 | 844 | |
| **70** | 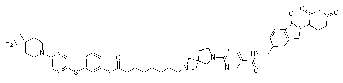 | 929 | |
| **71** | 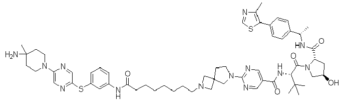 | 1100 | |
| **72** | 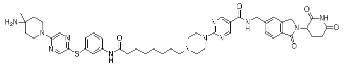 | 903 | |
| **73** | 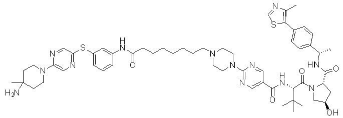 | 1074 | |
| **74** | 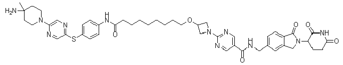 | 904 | |
| **75** | 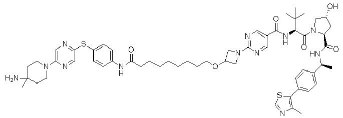 | 1075 | |
| **76** | 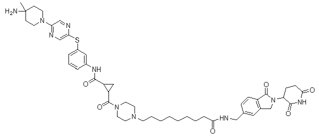 | 907 | |
| **77** | 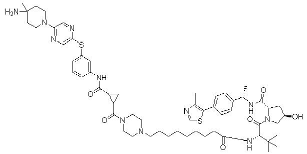 | 1078 | |
| **78** | 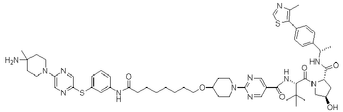 | 1089 | |
| **81** | 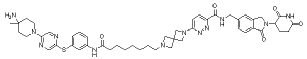 | 915 | ¹H NMR (400 MHz, DMSO-*d*₆) δ 10.99 (s, 1H), 9.93 (s, 1H), 9.47 (s, 1H), 8.37 (d, *J* = 1.5 Hz, 1H), 8.17 (d, *J* = 1.4 Hz, 1H), 7.84 (d, *J* = 9.2 Hz, 1H), 7.67 (d, *J* = 7.9 Hz, 1H), 7.59 (t, *J* = 1.9 Hz, 1H), 7.52 (s, 1H), 7.45 (d, *J* = 9.1 Hz, 2H), 7.23 (t, *J* = 8.0 Hz, 1H), 6.89 (d, *J* = 8.6 Hz, 1H), 6.85 (d, *J* = 9.3 Hz, 1H), 5.10 (dd, *J* = 13.3, 5.1 Hz, 1H), 4.59 (t, *J* = 5.1 Hz, 2H), 4.47 - 4.25 (m, 2H), 4.19 (s, 4H), 3.99 (d, *J* = 14.3 Hz, 2H), 3.49 (s, 1H), 3.45 - 3.22 (m, 3H), 2.95 - 2.82 (m, 1H), 2.69 - 2.53 (m, 1H), 2.35 (s, 3H), 2.25 (q, *J* = 6.8, 6.3 Hz, 3H), 2.04 - 1.93 (m, 2H), 1.70 (t, *J* = 5.8 Hz, 3H), 1.54 (s, 3H), 1.36 - 1.19 (m, 13H). |
| **82** | 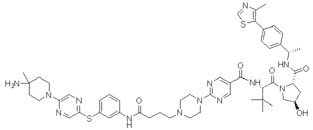 | 1018 | |
| **83** | 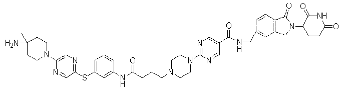 | 847 | |
| **84** | 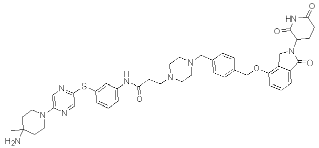 | 818 | |
| **85** | 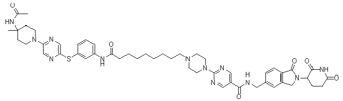 | 959 | |
| **86** | 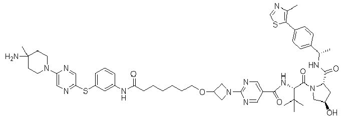 | 1047 | |
| **87** | 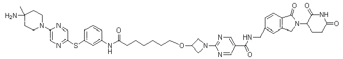 | 876 | |
| **88** | 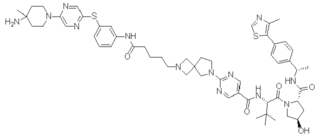 | 1058 | |
| **89** | 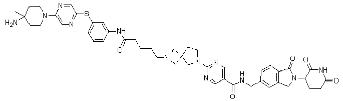 | 887 | |
| **90** | 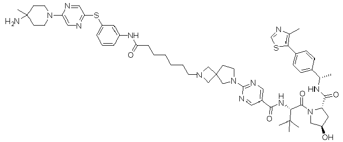 | 1086 | |
| **91** | 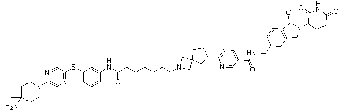 | 915 | |
| **92** | 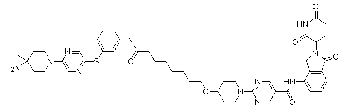 | 904 | |
| **93** | 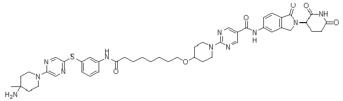 | 904 | |
| **94** | 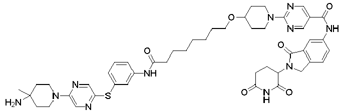 | 904 | |
| 95 | 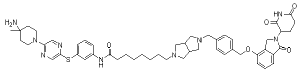 | 914 | ¹H NMR (400 MHz, DMSO-*d*₆) δ 10.98 (s, 1H), 9.99 (s, 1H), 8.37 (d, *J* = 1.5 Hz, 1H), 8.16 (d, *J* = 1.4 Hz, 1H), 7.58 (t, *J* = 2.0 Hz, 1H), 7.49 - 7.41 (m, 4H), 7.33 (p, *J* = 5.9 Hz, 4H), 7.22 (t, *J* = 8.0 Hz, 1H), 6.91 - 6.87 (m, 1H), 5.22 (d, *J* = 2.7 Hz, 2H), 5.11 (dd, *J* = 13.3, 5.1 Hz, 1H), 4.44 - 4.22 (m, 2H), |
| | | | 4.09 - 3.98 (m, 2H), 3.63 - 3.29 (m,4H), 2.96 - 2.85 (m, 1H), 2.78 - 2.63 (m, 4H), 2.61 - 2.52 (m, 2H), 2.47 - 2.40 (m, 4H), 2.30 - 2.23 (m, 3H), 2.03 - 1.94 (m, 2H), 1.77 - 1.68 (m, 4H), 1.57 - 1.46 (m, 4H), 1.37 (s, 3H), 1.32 - 1.16 (m, 10H). |
| **96** | 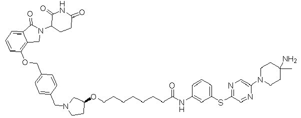 | 889 | ¹H NMR (400 MHz, DMSO-*d*₆) δ 9.88 (s, 1H), 8.33 (d, *J* = 1.5 Hz, 1H), 8.14 (d, *J* = 1.3 Hz, 1H), 7.52 - 7.39 (m, 5H), 7.31 (dd, *J* = 8.1, 5.6 Hz, 4H), 7.21 (t, *J* = 8.0 Hz, 1H), 6.88 (d, *J* = 7.8 Hz, 1H), 5.21 (s, 2H), 5.11 (dd, *J* = 13.3, 5.1 Hz, 1H), 4.45 - 4.20 (m, 2H), 3.98 - 3.86 (m, 1H), 3.80 - 3.69 (m, 2H), 3.60 - 3.46 (m, 4H), 3.30 - 3.20 (m, 3H), 2.97 - 2.84 (m, 1H), 2.71 - 2.61 (m, 1H), 2.58 (d, *J* = 3.4 Hz, 1H), 2.44 - 2.33 (m, 3H), 2.29 - 2.20 (m, 3H), 2.03 - 1.91 (m, 2H), 1.64 - 1.49 (m, 3H), 1.47 - 1.37 (m, 6H), 1.30 - 1.17 (m, 7H), 1.08 (s, 3H). |
| **97** | 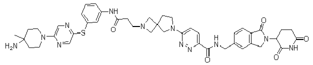 | 859 | ¹H NMR (400 MHz, DMSO-*d*₆) δ 10.99 (s, 1H), 10.10 (s, 1H), 9.49 (t, *J* = 6.3 Hz, 1H), 8.38 (d, *J* = 1.4 Hz, 1H), 8.18 (d, *J* = 1.3 Hz, 1H), 7.82 (d, *J* = 9.4 Hz, 1H), 7.67 (d, *J* = 7.8 Hz, 1H), 7.57 (d, *J* = 2.0 Hz, 1H), 7.53 (s, 1H), 7.49 - 7.41 (m, 2H), 7.24 (t, *J* = 8.0 Hz, 1H), 6.93 (dd, *J* = 15.0, 8.6 Hz, 2H), 5.10 (dd, *J* = 13.3, 5.1 Hz, 1H), 4.59 (d, *J* = 6.3 Hz, 2H), 4.48 - 4.24 (m, 2H), 4.14 (s, 1H), 4.06 - 3.92 (m, 2H), 3.69 - 3.47 (m, 3H), 3.47 - 3.39 (m, 2H), 3.17 (s, 6H), 2.97 - 2.84 (m, 1H), 2.69 (t, *J* = 6.9 Hz, 2H), 2.64 - 2.54 (m, 1H), 2.43 - 2.26 (m, 3H), 2.14 (t, *J* = 6.9 Hz, 2H), 2.03 - 1.93 (m, 1H), 1.78 - 1.63 (m, 4H), 1.34 (s, 3H). |
| **98** | 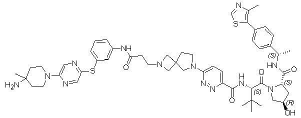 | 1030 | ¹H NMR (400 MHz, DMSO-*d*₆) δ 10.11 (s, 1H), 8.98 (d, *J* = 3.3 Hz, 1H), 8.50 (d, *J* = 7.6 Hz, 1H), 8.37 (d, *J* = 1.5 Hz, 1H), 8.33 (d, *J* = 9.7 Hz, 1H), 8.17 (d, *J* = 1.4 Hz, 1H), 7.83 (d, *J* = 9.4 Hz, 1H), 7.56 (t, *J* = 1.9 Hz, 1H), 7.49 - 7.33 (m, 5H), 7.24 (t, *J* = 7.9 Hz, 1H), 6.97 (d, *J* = 9.5 Hz, 1H), 6.94 - 6.89 (m, 1H), 5.20 (s, 1H), 4.98 - 4.87 (m, 1H), 4.71 (d, *J* = 9.8 Hz, 1H), 4.46 (t, *J* = 8.2 Hz, 1H), 4.30 (s, 1H), 4.00 - 3.87 (m, |
| | | | 2H), 3.69 - 3.27 (m, 11H), 3.21 - 3.09 (m, 5H), 2.68 (t, *J* = 6.9 Hz, 2H), 2.45 (s, 3H), 2.30 (t, *J* = 6.9 Hz, 2H), 2.19 - 2.04 (m, 3H), 1.82 - 1.73 (m, 1H), 1.70 - 1.63 (m, 3H), 1.38 (d, *J* = 7.0 Hz, 2H), 1.30 (s, 3H), 0.99 (s, 9H). |
| **99** | 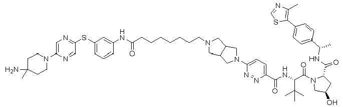 | 1100 | |
| **100** | 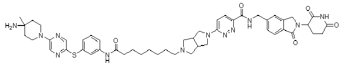 | 929 | |
| **101** | 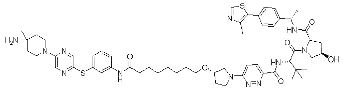 | 1075 | |
| **102** | 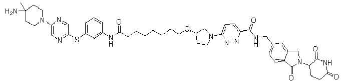 | 904 | |
| **103** | 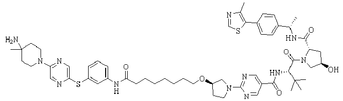 | 1075 | |
| **104** | 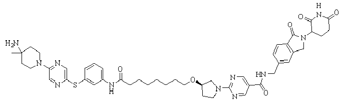 | 904 | |
| **105** | 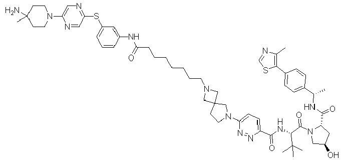 | 1100 | |
| **106** | 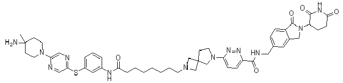 | 929 | |
| **107** | 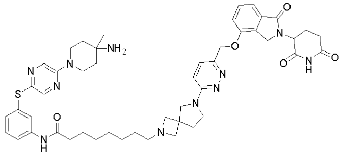 | 902 | |
| **108** | 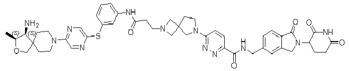 | 915 | ¹H NMR (400 MHz, DMSO-*d*₆) δ 10.99 (s, 1H), 10.14 (s, 1H), 9.50 (t, *J* = 6.4 Hz, 1H), 8.38 (d, *J* = 1.6 Hz, 1H), 8.20 - 8.14 (m, 1H), 7.83 (d, *J* = 9.4 Hz, 1H), 7.67 (d, *J* = 7.8 Hz, 1H), 7.58 - 7.51 (m, 2H), 7.50 - 7.42 (m, 2H), 7.24 (t, *J* = 8.0 Hz, 1H), 6.94 (d, *J* = 9.4 Hz, 1H), 6.90 (dd, *J* = 7.6, 1.9 Hz, 1H), 5.10 (dd, *J* = 13.3, 5.1 Hz, 1H), 4.59 (d, *J* = 6.3 Hz, 2H), 4.45 - 4.26 (m, 2H), 4.21 - 4.01 (m, 3H), 3.83 (d, *J* = 9.0 Hz, 1H), 3.72 - 3.61 (m, 3H), 3.54 (s, 3H), 3.33 - 3.26 (m, 4H), 3.22 2.97 (m, 4H), 2.95 - 2.76 (m, 3H), 2.61 (s, 1H), 2.41 - 2.30 (m, 3H), 2.17 (t, *J* = 6.9 Hz, 2H), 2.04 - 1.93 (m, 1H), 1.79 - 1.50 (m, 4H), 1.18 (d, *J* = 6.6 Hz, 3H). |
| **109** | 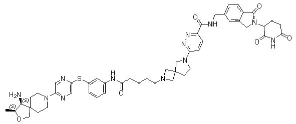 | 943 | ¹H NMR (400 MHz, DMSO-*d*₆) δ 10.99 (s, 1H), 10.14 (s, 1H), 9.50 (t, *J* = 6.4 Hz, 1H), 8.38 (d, *J* = 1.6 Hz, 1H), 8.20 - 8.14 (m, 1H), 7.83 (d, *J* = 9.4 Hz, 1H), 7.67 (d, *J* = 7.8 Hz, 1H), 7.58 - 7.51 (m, 2H), 7.50 - 7.42 (m, 2H), 7.24 (t, *J* = 8.0 Hz, 1H), 6.94 (d, *J* = 9.4 Hz, 1H), 6.90 (dd, *J* = 7.6, 1.9 Hz, 1H), 5.10 (dd, *J* = 13.3, 5.1 Hz, 1H), 4.59 (d, *J* = 6.3 Hz, 2H), 4.45 - 4.26 (m, 2H), 4.21 - 4.01 (m, 3H), 3.83 (d, *J* = 9.0 Hz, 1H), 3.72 - 3.61 (m, 3H), 3.54 (s, 3H), 3.33 - 3.26 (m, 4H), 3.22 - 2.97 (m, 4H), 2.95 - 2.76 (m, 3H), 2.61 (s, 1H), 2.41 - 2.30 (m, 3H), 2.17 (t, *J* = 6.9 Hz, 2H), 2.04 - 1.93 (m, 1H), 1.79 - 1.50 (m, 4H), 1.38 - 1.28 (m, 2H), 1.29 - 1.20 (m, 2H), 1.16 (d, J = 6.5 Hz, 3H). |
| 110 | 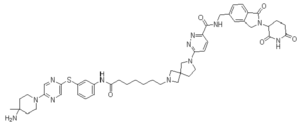 | 915 | ¹H NMR (400 MHz, DMSO-*d*₆) δ 10.99 (s, 1H), 9.99 (s, 1H), 9.50 (t, *J* = 6.4 Hz, 1H), 8.38 (d, *J* = 1.5 Hz, 1H), 8.17 (d, *J* = 1.4 Hz, 1H), 7.86 (d, *J* = 9.4 Hz, 1H), 7.68 (d, *J* = 7.8 Hz, 1H), 7.59 (t, *J* = 2.0 Hz, 1H), 7.53 (s, 1H), 7.47 (d, *J* = 7.9 Hz, 2H), 7.23 (t, *J* = 8.0 Hz, 1H), 6.94 (d, *J* = 9.4 Hz, 1H), 6.89 (d, *J* = 8.1 Hz, 1H), 5.10 (dd, *J* = 13.3, 5.1 Hz, 1H), 4.59 (d, *J* = 6.4 Hz, 2H), 4.47 - 4.25 (m, 2H), 4.13 (q, *J* = 5.3 Hz, 1H), 4.10 - 3.99 (m, 2H), 3.78 (s, 2H), 3.54 (s, 3H), 3.42 - 3.35 (m, 1H), 3.10 - 2.84 (m, 3H), 2.64 - 2.53 (m, 1H), 2.44 - 2.21 (m, 5H), 2.05 - 1.93 (m, 2H), 1.80 - 1.66 (m, 4H), 1.54 (s, 2H), 1.47 - 1.34 (m, 5H), 1.32 - 1.18 (m, 8H). |
| **111** | 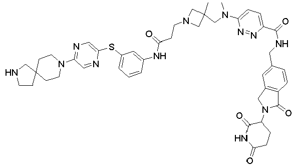 | 885 | |
| **112** | 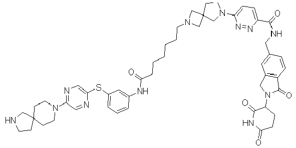 | 941 | |
| **113** | 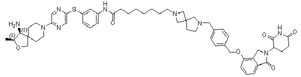 | 970 | ¹H NMR (400 MHz, DMSO-*d*₆) δ 11.00 (s, 1H), 9.91 (d, *J* = 4.9 Hz, 1H), 8.34 (s, 1H), 8.15 (s, 1H), 7.56 - 7.39 (m, 5H), 7.31 (dd, *J* = 10.1, 7.0 Hz, 4H), 7.21 (t, *J* = 8.0 Hz, 1H), 6.88 (d, *J* = 7.8 Hz, 1H), 5.22 (s, 2H), 5.11 (dd, *J* = 13.3, 5.1 Hz, 1H), 4.45 - 4.21 (m, 2H), 4.10 - 4.01 (m, 1H), 3.89 - 3.81 (m, 1H), 3.66 (d, *J* = 8.4 Hz, 1H), 3.52 (s, 2H), 3.47 (d, *J* = 8.5 Hz, 1H), 3.39 - 3.35 (m, 5H), 3.01 (d, *J* = 6.5 Hz, 2H), 2.98 - 2.84 (m, 4H), 2.61 - 2.52 (m, 3H), 2.42 (t, *J* = 7.0 Hz, 2H), 2.29 - 2.21 (m, 4H), 2.02 - 1.93 (m, 1H), 1.86 (t, *J* = 6.9 Hz, 2H), 1.80 - 1.69 (m, 1H), 1.68 - 1.59 (m, 1H), 1.58 - 1.43 (m, 4H), 1.41 - 1.33 (m, 1H), 1.24 - 1.16 (m, 8H), 1.07 (d, *J* = 6.4 Hz, 3H). |
| **115** | 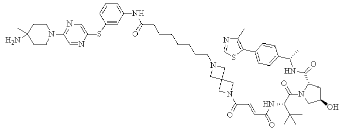 | 1062 | |
| **116** | 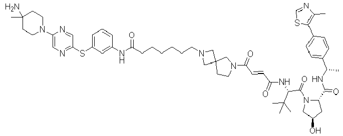 | 1062 | |
| **117** | 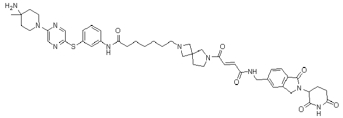 | 891 | |
| **118** | 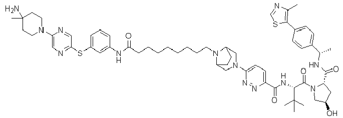 | 1114 | |
| **119** | 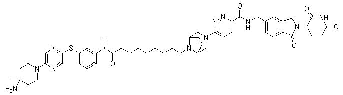 | 943 | |
| **120** | 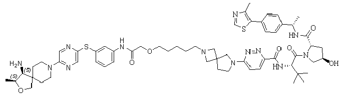 | 1158 | |
| **121** | 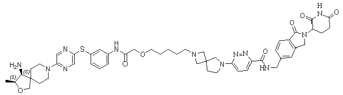 | 987 | |
| **122** | 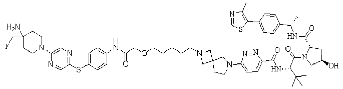 | 1120 | |
| **123** | 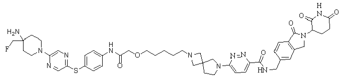 | 949 | |
| **124** | 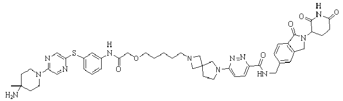 | 931 | |
| **125** | 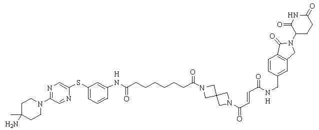 | 905 | |
| **126** | 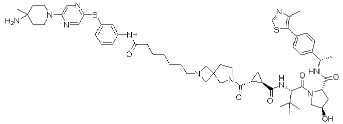 | 1076 | |
| **127** | 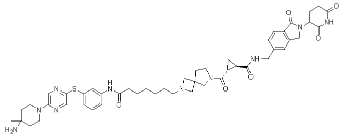 | 905 | |
| **128** | 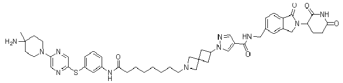 | 902 | |
| **129** | 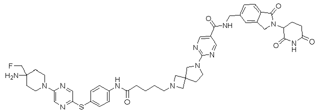 | 905 | ¹H NMR (400 MHz, DMSO-*d*₆) δ 11.00 (s, 1H), 9.98 (s, 1H), 903 (t, *J* = 6.0 Hz, 1H), 8.82 (s, 2H), 8.42 (d, *J* = 1.4 Hz, 1H), 8.18 (d, *J* = 1.4 Hz, 1H), 7.69 (d, *J* = 7.8 Hz, 1H), 7.60 - 7.51 (m, 2H), 7.46 (d, *J* = 7.9 Hz, 2H), 7.24 (t, *J* = 7.9 Hz, 1H), 6.91 (d, *J* = 7.9 Hz, 1H), 5.11 (dd, *J* = 13.3, 5.1 Hz, 1H), 4.57 (d, *J* = 5.7 Hz, 2H), 4.44 (d, *J* = 17.4 Hz, 1H), 4.30 (d, *J* = 17.3 Hz, 1H), 4.25 (s, 1H), 4.22 (s, 1H), 4.12 (s, 3H), 3.71 (s, 2H), 3.62 - 3.50 (m, 5H), 3.23 (t, *J* = 11.7 Hz, 2H), 3.08 (d, *J* = 20.7 Hz, 2H), 2.97 - 2.86 (m, 1H), 2.62 (dd, *J* = 21.6, 17.9 Hz, 1H), 2.40 (d, *J* = 4.3 Hz, 1H), 2.35 (dd, *J* = 12.7, 3.3 Hz, 1H), 2.29 (t, *J* = 7.3 Hz, 2H), 2.18 (t*, J* = 7.0 Hz, 2H), 2.00 (d, *J* = 7.0 Hz, 2H), 1.91 (d, *J* = 11.4 Hz, 1H), 1.81 (d, *J* = 11.4 Hz, 1H), 1.70 (s, 1H), 1.62 - 1.52 (m, 2H), 1.41 - 1.32 (m, 3H). |
| **130** | 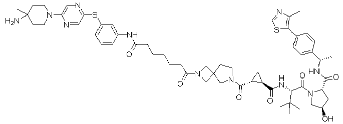 | 1090 | |
| **131** | 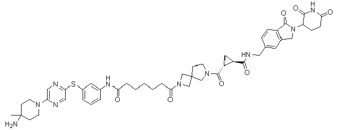 | 919 | |
| **132** | 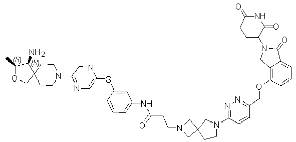 | 888 | ¹H NMR (400 MHz, DMSO-*d*₆) δ 10.99 (s, 1H), 10.11 (s, 1H), 8.40 - 8.34 (m, 1H), 8.17 (s, 1H), 7.56 - 7.42 (m, 4H), 7.39 (d, *J* = 8.1 Hz, 1H), 7.33 (d, *J* = 7.4 Hz, 1H), 7.24 (t, *J* = 8.0 Hz, 1H), 6.89 (t, *J* = 9.0 Hz, 2H), 5.32 (s, 2H), 5.10 (dd, *J* = 13.3, 5.1 Hz, 1H), 4.44 - 4.20 (m, 2H), 4.19 - 4.09 (m, 1H), 4.07 - 3.91 (m, 2H), 3.77 (d, *J* = 9.0 Hz, 1H), 3.60 - 3.54 (m, 3H), 3.50 - 3.43 (m, 3H), 3.27 - 3.13 (m, 6H), 3.08 - 2.98 (m, 1H), 2.96 - 2.86 (m, 1H), 2.72 (t, *J* = 6.6 Hz, 2H), 2.61 - 2.54 (m, 1H), 2.45 - 2.39 (m, 1H), 2.32 (t, *J* = 6.9 Hz, 2H), 2.12 (t, *J* = 6.8 Hz, 2H), 2.03 - 1.94 (m, 1H), 1.78 - 1.58 (m, 3H), 1.55 -1.44 (m, 2H), 1.14 (d, *J* = 6.5 Hz, 3H). |
| **133** | 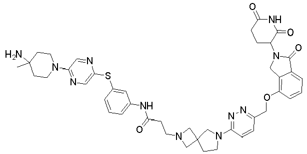 | 832 | ¹H NMR (400 MHz, DMSO-*d*₆) δ 10.07 (s, 1H), 8.33 (d, *J =* 1.5 Hz, 1H), 8.15 (d, *J* = 1.4 Hz, 1H), 7.53 - 7.42 (m, 4H), 7.38 (d, *J* = 8.1 Hz, 1H), 7.32 (d, *J* = 7.4 Hz, 1H), 7.22 (t, *J* = 8.0 Hz, 1H), 6.92 - 6.85 (m, 2H), 5.31 (s, 2H), 5.10 (dd, *J =* 13.4, 5.1 Hz, 1H), 4.42 - 4.19 (m, 2H), 3.80 - 3.70 (m, 2H), 3.60 - 3.52 (m, 4H), 3.48 - 3.43 (m, 2H), 3.13 (q, *J* = 7.2 Hz, 4H), 2.67 (t, *J* = 6.9 Hz, 2H), 2.61 - 2.53 (m, 1H), 2.46 - 2.39 (m, 1H), 2.29 (t, *J* = 6.9 Hz, 2H), 2.11 (t, *J* = 6.8 Hz, 2H), 2.03 - 1.93 (m, 2H), 1.47 - 1.39 (m, 4H), 1.28 - 1.22 (m, 2H), 1.08 (s, 3H). |
| **134** | 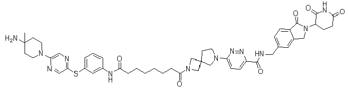 | 943 | |
| **135** | 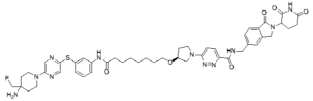 | 922 | ¹H NMR (400 MHz, DMSO-*d*₆) δ 9.92 (s, 1H), 9.49 (t, *J* = 6.4 Hz, 1H), 8.41 (d, *J* = 1.5 Hz, 1H), 8.17 (d, *J* = 1.4 Hz, 1H), 7.83 (d, *J* = 9.4 Hz, 1H), 7.68 (d, *J* = 7.8 Hz, 1H), 7.57 - 7.51 (m, 2H), 7.46 (t, *J* = 7.8 Hz, 2H), 7.23 (t, *J* = 8.0 Hz, 1H), 6.97 (d, *J* = 9.4 Hz, 1H), 6.93 - 6.87 (m, 1H), 5.10 (dd, *J* = 13.3, 5.1 Hz, 1H), 4.60 (d, *J* = 6.3 Hz, 2H), 4.43 (d, *J* = 17.4 Hz, 1H), 4.29 (d, *J* = 17.4 Hz, 1H), 4.20 (d, *J* = 3.7 Hz, 2H), 4.12 (d, *J* = 5.5 Hz, 2H), 3.61 (s, 2H), 3.43 (q, *J* = 6.8 Hz, 2H), 3.22 (t, *J* = 11.6 Hz, 2H), 3.17 (s, 2H), 2.96 - 2.88 (m, 1H), 2.87 (s, 1H), 2.63 - 2.55 (m, 1H), 2.40 (dd, *J* = 13.1, 4.5 Hz, 1H), 2.34 (dd, *J* = 9.8, 3.4 Hz, 1H), 2.24 (t, *J* = 7.3 Hz, 2H), 2.09 (d, *J* = 5.1 Hz, 2H), 2.02 - 1.93 (m, 2H), 1.87 (t, *J* = 12.3 Hz, 2H), 1.80 - 1.72 (m, 1H), 1.71 - 1.61 (m, 1H), 1.48 (dq, *J* = 13.6, 6.7 Hz, 5H), 1.26 (s, 5H). |
| **136** | 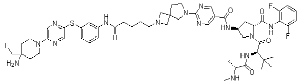 | 1071 | ¹H NMR (400 MHz, DMSO-*d*₆) δ 10.03 (d, *J* = 5.4 Hz, 1H), 8.75 (d, *J* = 9.9 Hz, 2H), 8.56 (d, *J* = 7.3 Hz, 1H), 8.48 (d, *J* = 7.5 Hz, 1H), 8.44 - 8.39 (m, 1H), 8.20 - 8.13 (m, 1H), 7.57 (d, *J* = 17.5 Hz, 1H), 7.47 (d, *J* = 8.2 Hz, 1H), 7.35 (d, *J* = 7.8 Hz, 1H), 7.24 (t, *J* = 8.0 Hz, 1H), 7.16 (t, *J* = 8.1 Hz, 2H), 6.91 (d, *J* = 7.8 Hz, 1H), 4.70 - 4.44 (m, 3H), 4.24 (d, *J* = 16.1 Hz, 2H), 3.76 (d, *J* = 13.6 Hz, 5H), 3.56 (s, 2H), 3.23 (t, *J* = 12.2 Hz, 3H), 3.12 (d, *J* = 20.7 Hz, 1H), 2.93 (s, 1H), 2.44 (s, 2H), 2.34 (s, 1H), 2.30 (d, *J* = 7.0 Hz, 2H), 2.21 (t, *J* = 6.7 Hz, 2H), 2.08 (s, 10H), 1.95 (dd, *J* = 24.2, 9.5 Hz, 2 H), 1.82 (d, *J* = 11.4 Hz, 1H), 1.73 (d, *J* = 16.6 Hz, 1H), 1.57 (d, *J* = 8.4 Hz, 2H), 1.42 (s, 2H), 1.30 (d, *J* = 6.8 Hz, 2H), 1.25 (s, 2H), 0.97 (d, *J* = 2.7 Hz, 9H). |
| **137** | 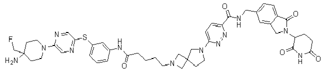 | 905 | ¹H NMR (400 MHz, DMSO-*d*₆) δ 10.07 (s, 1H), 9.51 (t, *J =* 6.3 Hz, 1H), 8.42 (d, *J* = 1.5 Hz, 1H), 8.17 (d, *J* = 1.4 Hz, 1H), 7.84 (d, *J =* 9.4 Hz, 1H), 7.69 (d, *J* = 7.9 Hz, 1H), 7.61 - 7.52 (m, 2H), 7.51 - 7.44 (m, 2H), 7.24 (t, *J* = 8.0 Hz, 1H), 6.98 - 6.88 (m, 2H), 5.11 (dd, *J* = 13.3, 5.1 Hz, 1H), 4.61 (d, *J* = 6.4 Hz, 2H), 4.44 (d, *J* = 17.5 Hz, 1H), 4.31 (d, *J* = 17.3 Hz, 1H), 4.24 (d, *J* = 13.6 Hz,1H), 3.68 (s, 2H), 3.52 (d, *J* = 15.1 Hz, 5H), 3.38 (s, 2H), 3.23 (t, *J* = 11.6 Hz, 2H), 3.04 (d, *J* = 20.5 Hz, 2H), 2.98 - 2.85 (m, 1H), 2.65 - 2.56 (m, 3H), 2.38 (dd, *J* = 13.2, 4.5 Hz, 1H), 2.30 (t, *J* = 7.3 Hz, 2H), 2.19 (t, *J* = 6.8 Hz, 2H), 2.05 - 1.95 (m, 2H), 1.91 (d, *J* = 11.1 Hz, 2H), 1.85 - 1.76 (m, 1H), 1.75 - 1.66 (m, 1H), 1.57 (q, *J* = 7.5 Hz, 2H), 1.33 (d, *J* = 8.0 Hz, 3H). |
| **138** | 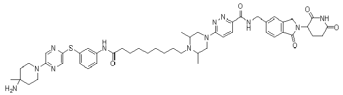 | 945 | ¹H NMR (400 MHz, DMSO-d6) δ (s, 1H), 9.93 (s, 1H), 9.54 (s, 2H), 8.38 (s, 1H), 8.17 (s, 1H), 8.00 (s, 3H), 7.72 - 7.38 (m, 6H), 7.23 (t, J = 7.9 Hz, 1H), 6.89 (d, J = 7.8 Hz, 1H), 5.32 (s, 1H), 5.10 (dd, J = 13.3, 5.1 Hz, 1H), 4.61 (d, J = 6.4 Hz, 2H), 4.43 (d, J = 17.3 Hz, 2H), 4.29 (d, J = 17.5 Hz, 2H), 4.05 (d, J = 14.0 Hz, 2H), 2.61 (s, 2H), 2.26 (t, J = 7.2 Hz, 4H), 1.99 (d, J = 8.8 Hz, 2H), 1.72 (s, 3H), 1.55 (s, 3H), 1.41 - 1.13 (m, 21H). |
| **139** | 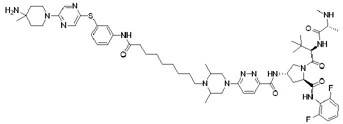 | 1111 | ¹H NMR (400 MHz, DMSO-d6) δ 10.01 - 9.89 (m, 2H), 9.16 (dd, J = 15.7, 8.5 Hz, 1H), 8.38 (d, J = 1.5 Hz, 1H), 8.17 (d, J = 1.3 Hz, 1H), 7.81 (d, J = 9.5 Hz, 1H), 7.59 (d, J = 2.0 Hz, 1H), 7.44 (d, J = 7.8 Hz, 1H), 7.38 - 7.28 (m, 2H), 7.23 (t, J = 8.0 Hz, 1H), 7.14 (t, J = 8.3 Hz, 2H), 6.90 (d, J = 7.6 Hz, 1H), 6.67 (s, 1H), 5.32 (t, J = 4.8 Hz, 1H), 4.62 (dd, J = 15.0, 7.5 Hz, 3H), 4.47 (d, J = 8.7 Hz, 1H), 4.29 (d, J = 11.9 Hz, 2H), 4.16 - 3.99 (m, 3H), 3.70 - 3.57 (m, 2H), 2.75 - 2.55 (m, 6H), 2.37 - 2.23 (m, 5H), 2.04 - 1.93 (m, 3H), 1.71 (d, J = 5.8 Hz, 3H), 1.50 (d, J = 37.6 Hz, 4H), 1.36 (s, 2H), 1.25 (d, J = 12.0 Hz, 18H), 1.06 (d, J = 5.9 Hz, 5H), 0.96 (d, J = 10.3 Hz, 7H). |
| **140** | 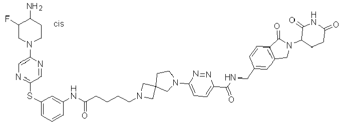 | 891 | ¹H NMR (400 MHz, DMSO-d6) δ 10.99 (s, 1H), 10.02 (s, 1H), 9.50 (t, J = 6.4 Hz, 1H), 8.41 (s, 1H), 8.16 (d, J = 1.4 Hz, 1H), 7.85 (d, J = 9.3 Hz, 1H), 7.68 (d, J = 7.8 Hz, 1H), 7.58 (d, J = 2.0 Hz, 1H), 7.53 (s, 1H), 7.50 - 7.44 (m, 2H), 7.24 (t, J = 8.0 Hz, 1H), 6.92 (dd, J = 19.0, 8.6 Hz, 2H), 5.32 (t, J = 4.9 Hz, 1H), 5.14 - 5.06 (m, 2H), 4.97 (s, 1H), 4.59 (d, J = 6.3 Hz, 2H), 4.45 (dd, J = 16.0, 11.9 Hz, 3H), 4.29 (d, J = 17.4 Hz, 2H), 3.04 - 2.86 (m, 4H), 2.61 (s, 4H), 2.33 - 2.22 (m, 5H), 1.99 (p, J = 6.9, 6.3 Hz, 5H), 1.58 (t, J = 7.5 Hz, 3H), 1.44 (s, 5H). |
| **141** | 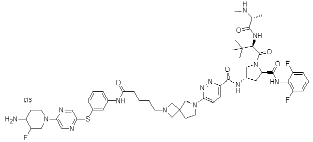 | 1057 | |
| **142** | 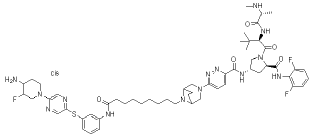 | 1113 | |
| **143** | 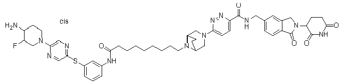 | 947 | |
| **144** | 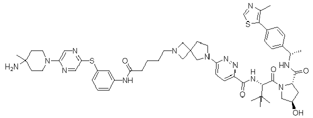 | 1058 | |
| **145** | 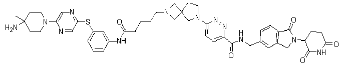 | 887 | |
| **146** | 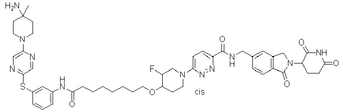 | 936 | ¹H NMR (400 MHz, DMSO-d6) δ 10.99 (s, 1H), 9.94 (s, 1H), 9.56 (t, J = 6.4 Hz, 1H), 8.38 (d, J = 1.5 Hz, 1H), 8.17 (d, J = 1.4 Hz, 1H), 8.08 (s, 2H), 7.84 (d, J = 9.5 Hz, 1H), 7.67 (d, J = 7.8 Hz, 1H), 7.58 (t, J = 2.0 Hz, 1H), 7.53 (s, 1H), 7.50 - 7.40 (m, 3H), 7.23 (t, J = 7.9 Hz, 1H), 6.93 - 6.87 (m, 1H), 5.32 (t, J = 4.9 Hz, 1H), 5.10 (dd, J = 13.3, 5.1 Hz, 1H), 4.60 (d, J = 6.3 Hz, 3H), 4.43 (d, J = 17.4 Hz, 1H), 4.29 (d, J = 17.3 Hz, 1H), 4.04 (d, J = 13.9 Hz, 2H), 3.74 (dt, J = 14.0, 7.5 Hz, 1H), 3.59 - 3.51 (m, 2H), 3.18 - 3.09 (m, 2H), 2.90 (ddd, J = 17.8, 13.4, 5.5 Hz, 1H), 2.63 - 2.55 (m, 1H), 2.43 - 2.31 (m, 2H), 2.26 (t, J = 7.4 Hz, 2H), 1.99 (q, J = 7.3, 6.5 Hz, 3H), 1.78 - 1.68 (m, 3H), 1.54 (d, J = 11.1 Hz, 5H), 1.28 - 1.23 (m, 11H). |
| **147** | 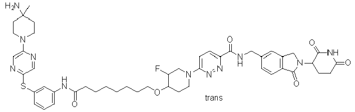 | 936 | |
| **148** | 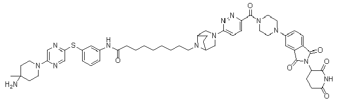 | 1012 | |
| **149** | 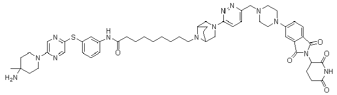 | 998 | |
| **150** | 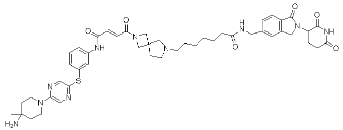 | 891 | |
| **151** | 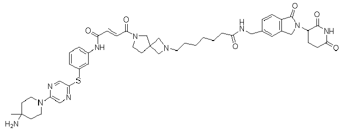 | 891 | |
| **152** | 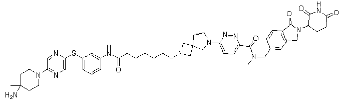 | 929 | |
| **154** | 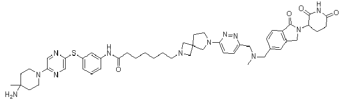 | 915 | |
| **155** | 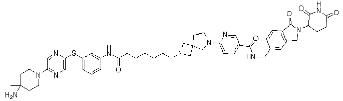 | 914 | |
| **156** | 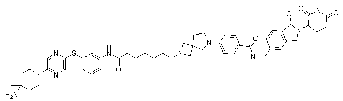 | 913 | |
| **153** | 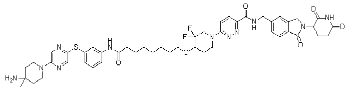 | 954 | ¹H NMR (400 MHz, DMSO-*d*₆) δ 9.91 (s, 1H), 9.58 (t, *J* = 6.4 Hz, 1H), 8.32 (d, *J* = 1.5 Hz, 1H), 8.14 (d, *J* = 1.4 Hz, 1H), 7.87 (d, *J* = 9.5 Hz, 1H), 7.67 (d, *J* = 7.9 Hz, 1H), 7.55 - 7.43 (m, 5H), 7.21 (t, *J* = 8.0 Hz, 1H), 6.87 (d, *J* = 8.0 Hz, 1H), 5.09 (dd, *J* = 13.3, 5.1 Hz, 1H), 4.60 (d, *J* = 6.2 Hz, 2H), 4.46 - 4.20 (m, 2H), 4.13 - 3.99 (m, 2H), 3.97 - 3.82 (m, 2H), 3.81 - 3.73 (m, 2H), 3.68 (s, 1H), 3.62 - 3.57 (m, 2H), 3.56 - 3.47 (m, 3H), 3.18 - 3.14 (m, 1H), 2.96 - 2.84 (m, 1H), 2.62 - 2.55 (m, 1H), 2.42 - 2.31 (m, 1H), 2.26 (t, *J =* 7.4 Hz, 2H), 2.01 - 1.93 (m, 2H), 1.80 - 1.69 (m, 1H), 1.60 - 1.49 (m, 4H), 1.45 - 1.39 (m, 4H), 1.34 - 1.26 (m, 6H), 1.07 (s, 3H). |
| **159 (158 isom er)** | 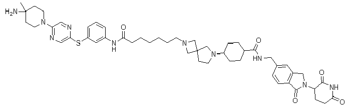 | 909 | ¹H NMR (400 MHz, DMSO-d6) δ 11.01 (d, J = 6.9 Hz, 1H), 9.97 (s, 1H), 8.46 (s, 1H), 8.38 (d, J = 1.5 Hz, 1H), 8.18 (d, J = 1.4 Hz, 3H), 7.67 (d, J = 8.1 Hz, 1H), 7.59 (d, J = 1.9 Hz, 1H), 7.48 -7.41 (m, 2H), 7.36 (d, J = 8.1 Hz, 1H), 7.23 (t, J = 7.9 Hz, 1H), 6.89 (d, J = 7.9 Hz, 1H), 5.35 - 5.06 (m, 2H), 4.47 - 4.39 (m, 1H), 4.35 (d, J = 6.2 Hz, 2H), 4.29 (d, J = 17.4 Hz, 1H), 4.17 (d, J = 4.6 Hz, 1H), 4.05 (d, J = 13.6 Hz, 1H), 3.90 (s, 2H), 3.09 (q, J = 7.3 Hz, 3H), 3.04 - 2.94 (m, 2H), 2.90 (d, J = 10.3 Hz, 2H), 2.45 - 2.32 (m, 2H), 2.26 (q, J = 7.2 Hz, 3H), 2.16 (s, 3H), 2.09 - 1.94 (m, 5H), 1.85 (d, J = 10.8 Hz, 2H), 1.74 (q, J = 6.8, 5.8 Hz, 4H), 1.55 (s, 2 H), 1.39 (d, J = 19.7 Hz, 8H), 1.27 (s, 6H). |
| **160** | 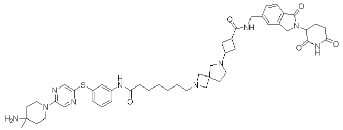 | 891 | ¹H NMR (400 MHz, DMSO-d6) δ 11.00 (s, 1H), 9.96 (s, 1H), 8.38 (d, J = 1.5 Hz, 1H), 8.18 (d, J = 1.4 Hz, 1H), 8.04 (s, 2H), 7.69 - 7.58 (m, 2H), 7.49 - 7.41 (m, 2H), 7.35 (t, J = 9.0 Hz, 1H), 7.27 - 7.16 (m, 2H), 6.92 - 6.86 (m, 1H), 5.32 (t, J = 4.8 Hz, 1H), 5.11 (dd, J = 13.2, 5.0 Hz, 1H), 4.73 (dd, J = 10.4, 4.6 Hz, 1H), 4.58 (d, J = 17.6 Hz, 1H), 4.45 (s, 1H), 4.42 - 4.30 (m, 3H), 4.05 (d, J = 14.0 Hz, 3H), 3.64 (s, 1H), 3.50 (s, 2H), 3.16 (d, J = 4.6 Hz, 2H), 2.27 (q, J = 8.1, 7.7 Hz, 5H), 2.09 - 1.90 (m, 5H), 1.78 - 1.67 (m, 4H), 1.55 (s, 2H), 1.37 (s, 4H), 1.30 - 1.13 (m, 12H) |
| **161** | 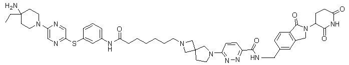 | 929 | |
| **162** | 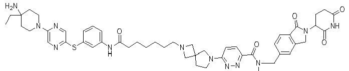 | 943 | |
| **163** | 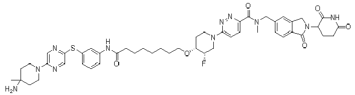 | 950 | |
| **164** | 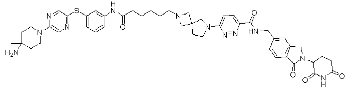 | 901 | |
| **165** | 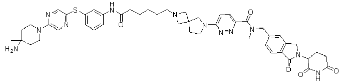 | 915 | |
| **166** | 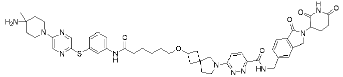 | 916 | |
| **167** | 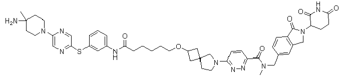 | 930 | |
| **168** | 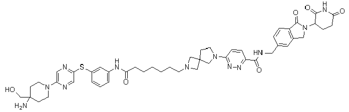 | 931 | ¹H NMR (400 MHz, DMSO-d6) δ 11.00 (s, 1H), 10.02 (s, 1H), 9.50 (s, 1H), 8.37 (d, J = 1.5 Hz, 1H), 8.17 (d, J = 1.4 Hz, 1H), 8.02 (s, 2H), 7.86 (d, J = 9.4 Hz, 1H), 7.66 (dd, J = 14.6, 7.8 Hz, 1H), 7.59 (t, J = 1.9 Hz, 1H), 7.53 (s, 1H), 7.47 (d, J = 8.2 Hz, 2H), 7.23 (t, J = 8.0 Hz, 1H), 6.91 (dd, J = 20.2, 8.3 Hz, 2H), 5.65 (s, 1H), 5.32 (t, J = 4.9 Hz, 1H), 5.10 (dd, J = 13.3, 5.1 Hz, 1H), 4.47 - 4.39 (m, 2H), 4.29 (d, J = 17.4 Hz, 1H), 4.12 (dd, J = 18.9, 7.0 Hz, 4H), 3.91 (dd, J = 13.5, 7.5 Hz, 4H), 3.77 (s, 2H), 2.90 (ddd, J = 17.8, 12.6, 5.4 Hz, 1H), 2.28 (t, J = 7.2 Hz, 4H), 1.98 (t, J = 7.9 Hz, 2H), 1.82 (d, J = 14.1 Hz, 2H), 1.71 - 1.65 (m, 2H), 1.50 (d, J = 38.4 Hz, 6H), 1.26 (d, J = 24.6 Hz, 9H). |
| **169** | 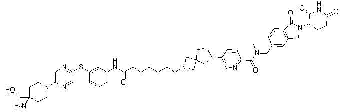 | 945 | ¹H NMR (400 MHz, DMSO-d6) δ 11.01 (s, 1H), 9.97 (s, 1H), 8.37 (d, J = 1.5 Hz, 1H), 8.17 (d, J = 1.3 Hz, 1H), 7.84 - 7.63 (m, 3H), 7.63 - 7.55 (m, 2H), 7.54 - 7.40 (m, 3H), 7.23 (t, J = 7.9 Hz, 1H), 6.91 (dd, J = 22.5, 8.1 Hz, 2H), 5.61 (s, 1H), 5.32 (t, J = 4.8 Hz, 1H), 5.12 (d, J = 13.1 Hz, 1H), 4.46 (dd, J = 17.5, 11.4 Hz, 1H), 4.32 (dd, J = 17.4, 10.9 Hz, 1H), 4.12 (d, J = 5.4 Hz, 1H), 3.94 (s, 3H), 3.76 (s, 2H), 3.60 (s, 2H), 2.91 (d, J = 19.8 Hz, 5H), 2.75 - 2.55 (m, 2H), 2.28 (d, J = 11.1 Hz, 3H), 2.06 - 1.91 (m, 3H), 1.81 (d, J = 14.0 Hz, 2H), 1.68 (d, J = 9.1 Hz, 2H), 1.49 (d, J = 49.9 Hz, 5H), 1.27 - 1.18 (m, 10H). |
| **170** | 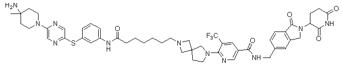 | 982 | |
| **171** | 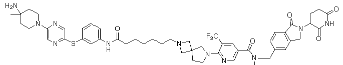 | 996 | |
| **173** | 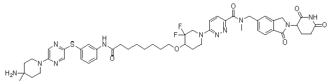 | 968 | ¹H NMR (400 MHz, DMSO-*d*₆) δ 9.91 (s, 1H), 8.32 (d, *J* = 1.5 Hz, 1H), 8.14 (d, *J* = 1.4 Hz, 1H), 7.87 (d, *J* = 9.5 Hz, 1H), 7.67 (d, *J* = 7.9 Hz, 1H), 7.55 - 7.43 (m, 5H), 7.21 (t, *J* = 8.0 Hz, 1H), 6.87 (d, *J* = 8.0 Hz, 1H), 5.09 (dd, *J* = 13.3, 5.1 Hz, 1H), 4.60 (d, *J* = 6.2 Hz, 2H), 4.46 - 4.20 (m, 2H), 4.13 - 3.99 (m, 2H), 3.97 - 3.82 (m, 2H), 3.81 - 3.73 (m, 2H), 3.68 (s, 1H), 3.62 - 3.57 (m, 2H), 3.56 - 3.47 (m, 3H), 3.18 - 3.14 (m, 1H), 2.96 - 2.84 (m, 4H), 2.62 |
| | | | - 2.55 (m, 1H), 2.42 - 2.31 (m, 1H), 2.26 (t, *J* = 7.4 Hz, 2H), 2.01 - 1.93 (m, 2H), 1.80 - 1.69 (m, 1H), 1.60 - 1.49 (m, 4H), 1.45 - 1.39 (m, 4H), 1.34 - 1.26 (m, 6H), 1.07 (s, 3H). |
| **174** | 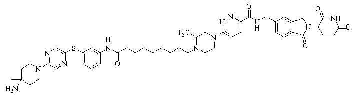 | 985 | |
| **175** | 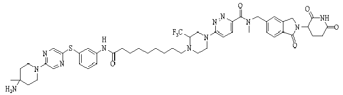 | 999 | |
| **176** | 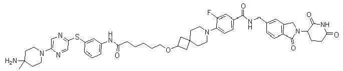 | 946 | ¹H NMR (400 MHz, DMSO-d6) δ 10.99 (s, 1H), 9.96 (s, 1H), 9.07 (t, J = 6.0 Hz, 1H), 8.38 (d, J = 1.5 Hz, 1H), 8.18 - 8.09 (m, 3H), 7.70 - 7.65 (m, 3H), 7.64 - 7.57 (m, 1H), 7.51 (s, 1H), 7.45 (t, J = 8.6 Hz, 2H), 7.23 (t, J = 8.0 Hz, 1H), 7.06 (t, J = 8.7 Hz, 1H), 6.90 (dt, J = 7.8, 1.4 Hz, 1H), 5.10 (dd, J = 13.3, 5.1 Hz, 1H), 4.56 (d, J = 5.8 Hz, 2H), 4.43 (d, J = 17.4 Hz, 1H), 4.29 (d, J = 17.3 Hz, 1H), 4.13 (s, 1H), 4.04 (d, J = 14.1 Hz, 2H), 3.93 (p, J = 7.1 Hz, 1H), 3.62 (td, J = 6.6, 3.9 Hz, 1H), 3.25 (t, J = 6.4 Hz, 2H), 3.05 - 2.85 (m, 5H), 2.59 (d, J = 17.0 Hz, 1H), 2.39 (td, J = 13.2, 4.4 Hz, 1H), 2.27 (t, J = 7.3 Hz, 2H), 2.19 - 2.10 (m, 2H), 2.03 - 1.92 (m, 2H), 1.72 (d, J = 5.5 Hz, 3H), 1.55 (dd, J = 14.9, 7.6 Hz, 3H), 1.48 (t, J = 7.4 Hz, 2H), 1.31 (d, J = 7.2 Hz, 2H), 1.28 - 1.21 (m, 8H). |
| **177** | 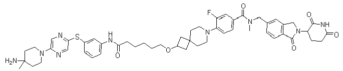 | 960 | |
| **178** | 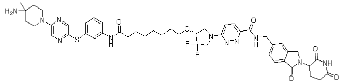 | 940 | |
| **179** | 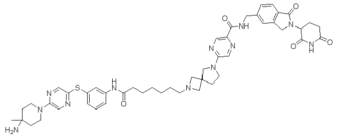 | 915 | |
| **180** | 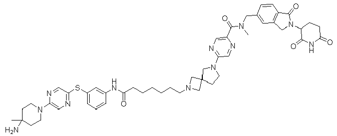 | 929 | |
| **181** | 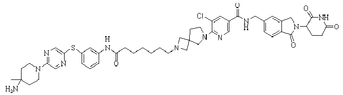 | 948 | |
| **182** | 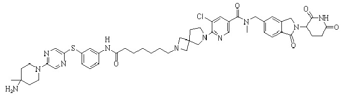 | 962 | |
| **183** | 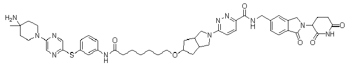 | 930 | ¹H NMR (400 MHz, DMSO-*d*₆) δ 9.89 (s, 1H), 9.47 (t, *J* = 6.2 Hz, 1H), 8.32 (d, *J* = 1.5 Hz, 1H), 8.14 (d, *J* = 1.4 Hz, 1H), 7.81 (d, *J* = 9.4 Hz, 1H), 7.67 (d, *J* = 7.8 Hz, 1H), 7.53 - 7.48 (m, 2H), 7.48 - 7.43 (m, 2H), 7.20 (t, *J* = 8.0 Hz, 1H), 6.93 (d, *J* = 9.4 Hz, 1H), 6.87 (dt, *J* = 7.9, 1.4 Hz, 1H), 5.09 (dd, *J* = 13.3, 5.1 Hz, 1H), 4.59 (d, *J* = 6.3 Hz, 2H), 4.45 - 4.24 (m, 2H), 3.95 - 3.87 (m, 1H), 3.82 - 3.69 (m, 4H), 3.56 - 3.43 (m, 4H), 3.27 (t, *J* = 6.3 Hz, 2H), 2.96 - 2.84 (m, 1H), 2.78 (s, 2H), 2.62 - 2.54 (m, 1H), 2.41 - 2.29 (m, 1H), 2.27 - 2.14 (m, 3H), 2.13 - 2.04 (m, 2H), 2.02 - 1.93 (m, 2H), 1.61 - 1.51 (m, 2H), 1.46 - 1.33 (m, 8H), 1.20 - 1.12 (m, 4H), 1.06 (s, 3H). |
| **184** | 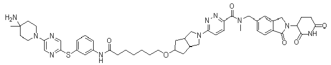 | 944 | ¹H NMR (400 MHz, DMSO-*d*₆) δ 9.89 (s, 1H), 8.31 (d, *J* = 1.5 Hz, 1H), 8.14 (d, *J* = 1.5 Hz, 1H), 7.72 (dd, *J* = 11.2, 7.9 Hz, 1H), 7.61 (dd, *J* = 9.4, 6.2 Hz, 1H), 7.55 - 7.39 (m, 4H), 7.21 (t, *J* = 8.0 Hz, 1H), 6.97 - 6.90 (m, 1H), 6.89 - 6.84 (m, 1H), 5.16 - 5.05 (m, 1H), 4.95 - 4.78 (m, 2H), 4.50 - 4.25 (m, 2H), 3.96 - 3.83 (m, 1H), 3.82 - 3.61 (m, 4H), 3.57 - 3.40 (m, 4H), 3.31 - 3.22 (m, 2H), 2.97 - 2.85 (m, 4H), 2.82 - 2.69 (m, 2H), 2.59 (d, *J* = 17.0 Hz, 1H), 2.43 - 2.31 (m, 1H), 2.29 - 2.14 (m, 3H), 2.14 - 1.94 (m, 4H), 1.60 - 1.50 (m, 2H), 1.46 - 1.31 (m, 8H), 1.20 - 1.12 (m, 4H), 1.06 (s, 3H). |
| **185** | 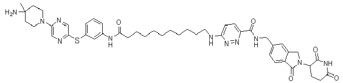 | 876 | |
| **186** | 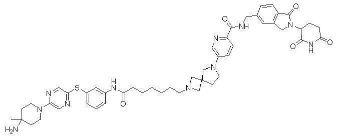 | 914 | |
| **187** | 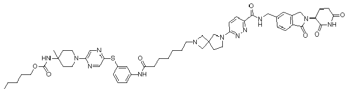 | 1029 | ¹H NMR (400 MHz, DMSO-d6) δ 10.99 (s, 1H), 9.93 (s, 1H), 9.50 (t, J = 6.4 Hz, 1H), 8.34 (d, J = 1.5 Hz, 1H), 8.15 (d, J = 1.4 Hz, 1H), 7.82 (dd, J = 9.3, 3.4 Hz, 1H), 7.68 (d, J = 7.8 Hz, 1H), 7.55 - 7.43 (m, 4H), 7.21 (t, J = 8.0 Hz, 1H), 7.02 - 6.85 (m, 3H), 5.10 (dd, J = 13.3, 5.1 Hz, 1H), 4.59 (d, J = 6.2 Hz, 2H), 4.43 (d, J = 17.4 Hz, 1H), 4.29 (d, J = 17.4 Hz, 1H), 3.90 (dd, J = 9.6, 5.2 Hz, 4H), 3.59 (d, J = 53.4 Hz, 5H), 3.25 (t, J = 11.7 Hz, 4H), 3.16 (d, J = 4.6 Hz, 1H), 2.91 (ddd, J = 18.5, 13.8, 5.3 Hz, 1H), 2.59 (d, J = 16.9 Hz, 2H), 2.37 (dd, J = 13.2, 4.4 Hz, 2H), 2.26 (t, J = 7.4 Hz, 2H), 2.18 (s, 2H), 2.09 (d, J = 13.3 Hz, 2H), 1.99 (q, J = 7.6 Hz, 2H), 1.54 (q, J = 6.7, 6.3 Hz, 4H), 1.44 (d, J = 9.8 Hz, 2H), 1.27 (dt, J = 15.2, 5.8 Hz, 15H). |
| **190** | 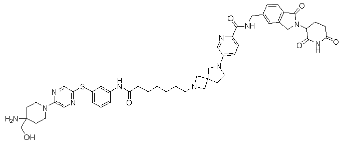 | 930 | |
| **191** | 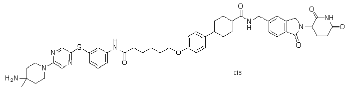 | 887 | ¹H NMR (400 MHz, DMSO-d6) δ 11.00 (s, 1H), 9.97 (s, 1H), 8.42 - 8.34 (m, 2H), 8.18 (d, J = 1.3 Hz, 1H), 8.12 (s, 2H), 7.68 (d, J = 7.8 Hz, 1H), 7.58 (t, J = 1.9 Hz, 1H), 7.46 (q, J = 2.8, 2.0 Hz, 2H), 7.43 - 7.38 (m, 1H), 7.23 (t, J = 8.0 Hz, 1H), 7.08 - 7.01 (m, 2H), 6.91 (dt, J = 7.9, 1.4 Hz, 1H), 6.84 - 6.77 (m, 2H), 5.11 (dd, J = 13.3, 5.1 Hz, 1H), 4.43 (d, J = 17.5 Hz, 1H), 4.39 (d, J = 5.8 Hz, 2H), 4.29 (d, J = 17.3 Hz, 1H), 4.04 (dd, J = 11.5, 6.9 Hz, 2H), 3.90 (t, J = 6.4 Hz, 2H), 3.65 - 3.57 (m, 1H), 2.97 - 2.85 (m, 1H), 2.40 (td, J = 13.1, 4.5 Hz, 1H), 2.30 (t, J = 7.4 Hz, 2H), 2.04 - 1.95 (m, 3H), 1.77 - 1.67 (m, 7H), 1.60 (dd, J = 15.1, 8.5 Hz, 5H), 1.42 (q, J = 8.0 Hz, 2H), 1.37 (s, 3H), 1.28 (s, 1H), 1.26 (s, 3H), 1.23 (s, 2H). |
| **192** | 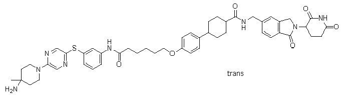 | 887 | |
| **194** | 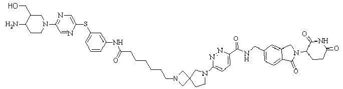 | 931 | |
| **195** | 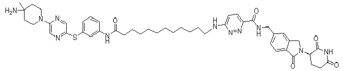 | 890 | |
| **196** | 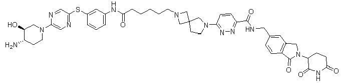 | 903 | |
| **197** | 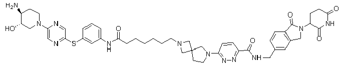 | 917 | |
| **198** | 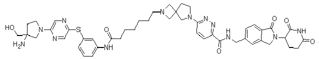 | 917 | ¹H NMR (400 MHz, DMSO-*d*₆) δ 9.91 (s, 1H), 9.49 (t, *J* = 6.2 Hz, 1H), 8.20 (d, *J* = 1.4 Hz, 1H), 8.00 (d, *J* = 1.5 Hz, 1H), 7.83 (d, *J* = 9.4 Hz, 1H), 7.68 (d, *J* = 7.9 Hz, 1H), 7.53 (s, 2H), 7.49 - 7.42 (m, 2H), 7.21 (t, *J* = 8.0 Hz, 1H), 6.95 (d, *J* = 9.5 Hz, 1H), 6.86 - 6.82 (m, 1H), 5.10 (dd, *J* = 13.2, 5.1 Hz, 1H), 4.60 (d, *J* = 6.6 Hz, 2H), 4.43 (d, *J* = 17.3 Hz, 1H), 4.29 (d, *J* = 17.2 Hz, 1H), 3.69 - 3.49 (m, 9H), 3.42 (d, *J* = 11.7 Hz, 2H), 3.27 (s, 3H), 2.96 - 2.85 (m, 1H), 2.69 - 2.59 (m, 1H), 2.57 (s, 1H), 2.44 - 2.35 (m, 2H), 2.34 - 2.22 (m, 3H), 2.17 (s, 2H), 2.00 (q, *J* = 6.8, 6.2 Hz, 3H), 1.54 (s, 2H), 1.28 (s, 6H), 1.20 - 1.12 (m, 2H). |
| **199** | 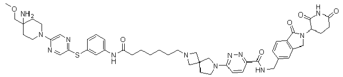 | 945 | |
| **200** | 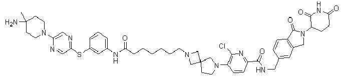 | 948 | |
| **201** | 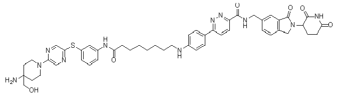 | 926 | |
| **202** | 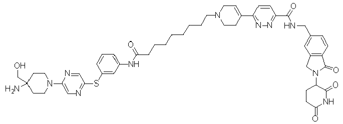 | 930 | |
| **203** | 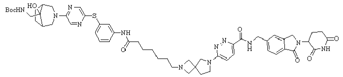 | 1057 | |
| **204** | 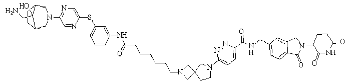 | 957 | |
| **205** | 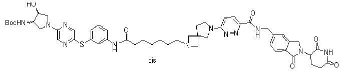 | 1003 | |
| **206** | 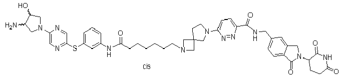 | 903 | |
| **207** | 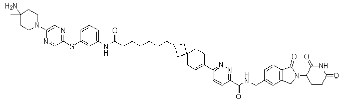 | 926 | |
| **208** | 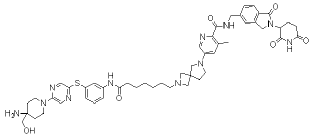 | 944 | |
| **209** | 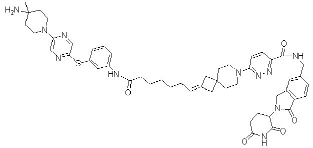 | 926 | |
| **210** | 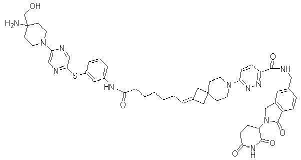 | 942 | |
| **211** | 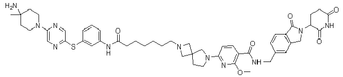 | 944 | |
| **212** | 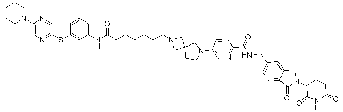 | 886 | |
| **213** | 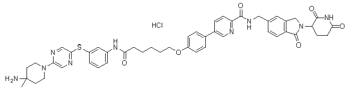 | 882 | |
| **214** | 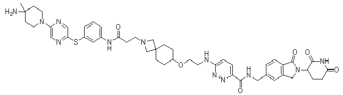 | 931 | |
| **215** | 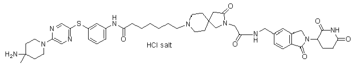 | 893 | |
| **216** | 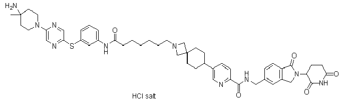 | 927 | |
| **217** | 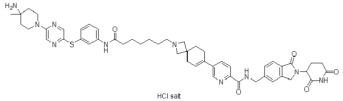 | 925 | |
| **218** | 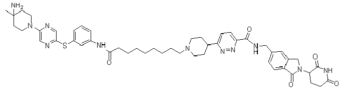 | 916 | |
| **219** | 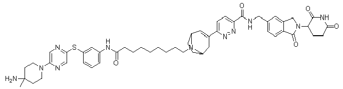 | 940 | |
| **220** | 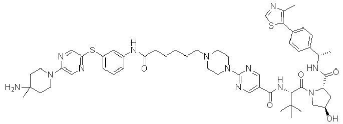 | 1046 | |
| **221** | 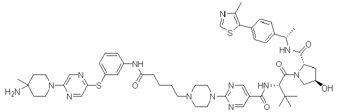 | 1032 | |
| **222** | 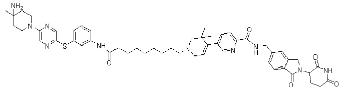 | 941 | |
| **223** | 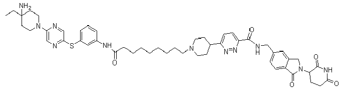 | 930 | |
| **224** | 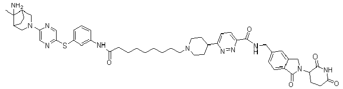 | 942 | |
| **225** | 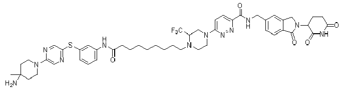 | 985 | |
| **226** | 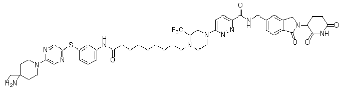 | 999 | |
| **227** | 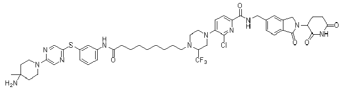 | 1018 | |
| **228** | 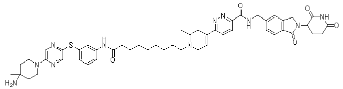 | 928 | |
| **229** | 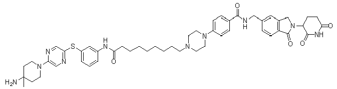 | 915 | |
| **230** | 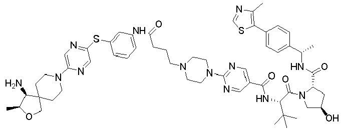 | 1074 | |
| **231** | 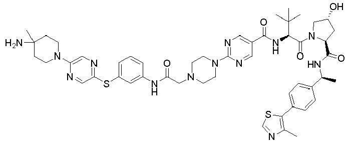 | 990 | |
| **232** | 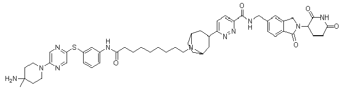 | 942 | Isomer 1 |
| **233** | 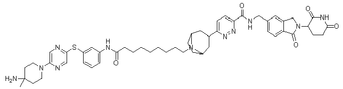 | 942 | Isomer 2 |
| **234** | 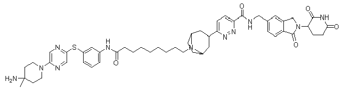 | 942 | Isomer 3 |
| **235** | 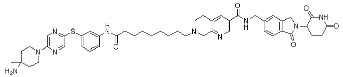 | 887 | |
| **236** | 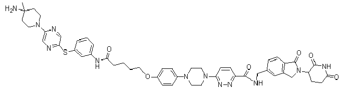 | 953 | |
| **237** | 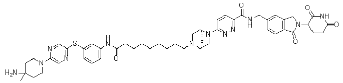 | 929 | |
| **238** | 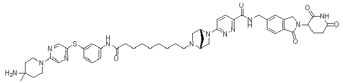 | 929 | |
| **239** | 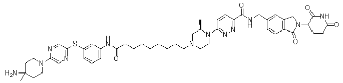 | 931 | |
| **240** | 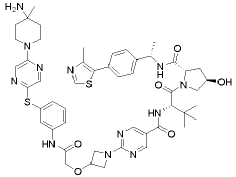 | 977 | |
| **241** | 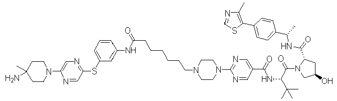 | 1060 | |
| **242** | 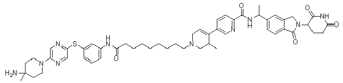 | 941 | |
| **243** | 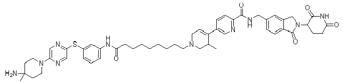 | 927 | |
| **244** | 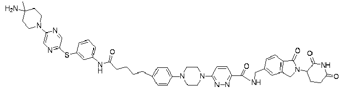 | 937 | |
| **245** | 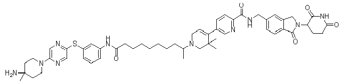 | 955 | |
| **246** | 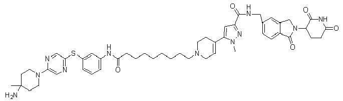 | 916 | |
| **247** | 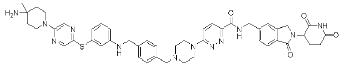 | 881 | |
| **248** | 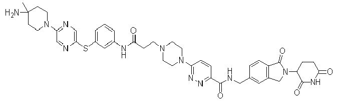 | 833 | |
| **249** | 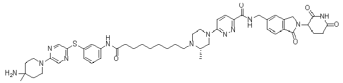 | 931 | |
| **250** | 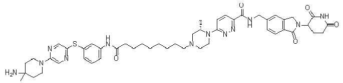 | 931 | |
| **251** | 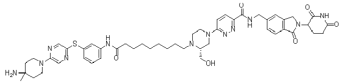 | 947 | |
| **252** | 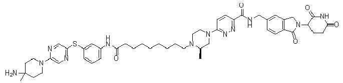 | 931 | |
| **253** | 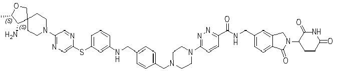 | 937 | |
| **254** | 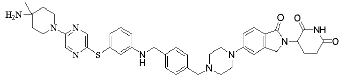 | 746 | |
| **255** | 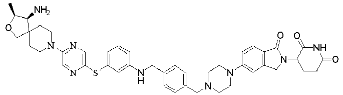 | 802 | |
| **256** | 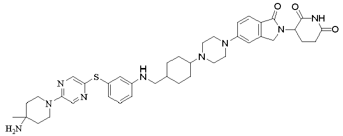 | 738 | |
| **257** | 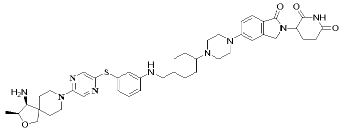 | 974 | |
| **258** | 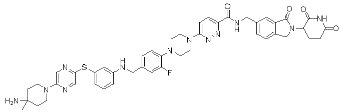 | 885 | |
| **259** | 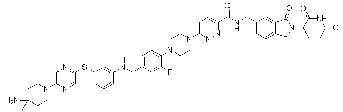 | 941 | |
| **260** | 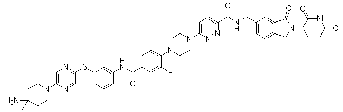 | 899 | |
| **261** | 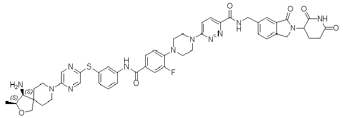 | 955 | |
| **262** | 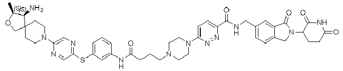 | 903 | |
| **263** | 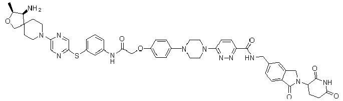 | 967 | |
| **265** | 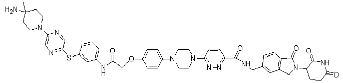 | 911 | |
| **266** | 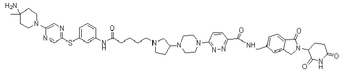 | 930 | |
| **267** | 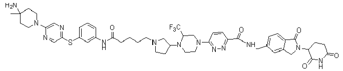 | 998 | |
| **268** | 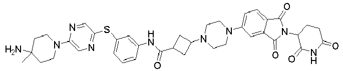 | 738 | |
| **269** | 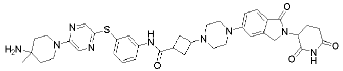 | 724 | |
| **270** | 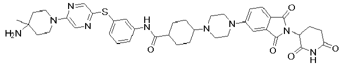 | 766 | |
| **271** | 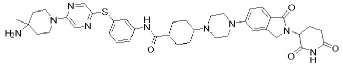 | 752 | |
| **272** | 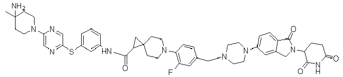 | 887 | |
| **273** | 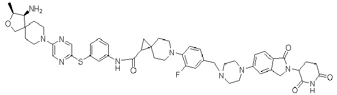 | 943 | |
| **274** | 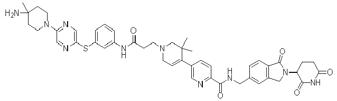 | 857 | |
| **275** | 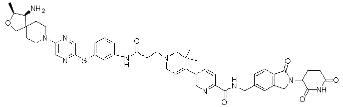 | 913 | |
| **276** | 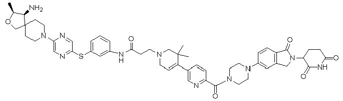 | 968 | |
| **277** | 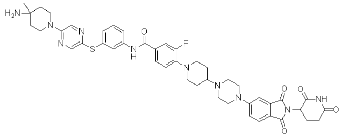 | 861 | |
| **278** | 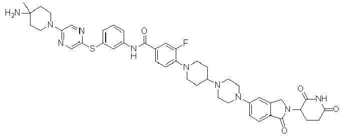 | 847 | |
| **279** | 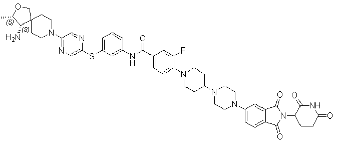 | 917 | |
| **280** | 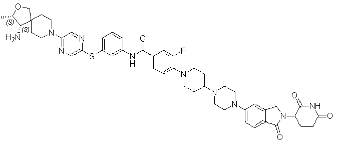 | 903 | |
| **281** | 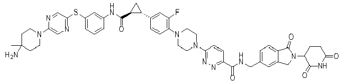 | 939 | |
| **282** | 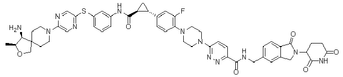 | 995 | |
| **283** | 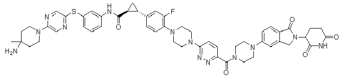 | 994 | |
| **284** | 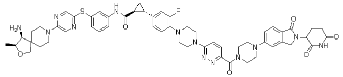 | 1050 | |
| **285** | 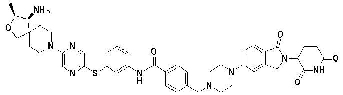 | 816 | |
| **286** | 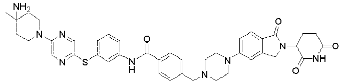 | 760 | |
| **287** | 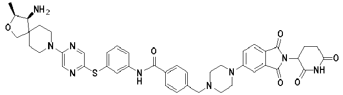 | 830 | |
| **288** | 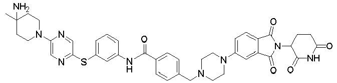 | 775 | |
| **289** | 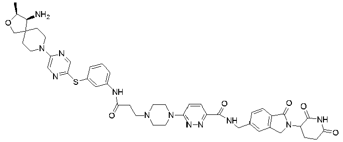 | 889 | |
| **290** | 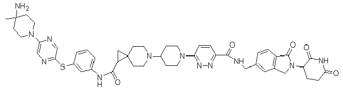 | 913 | |
| **291** | 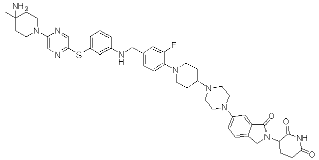 | 833 | |
| **296** | 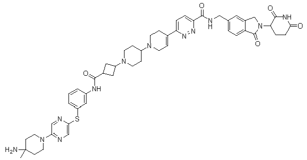 | 939 | |
| **297** | 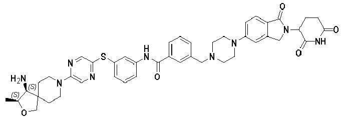 | 816 | |
| **298** | 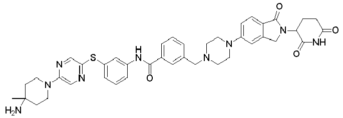 | 760 | |
| **299** | 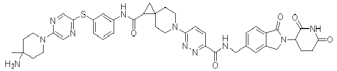 | 830 | |
| **300** | 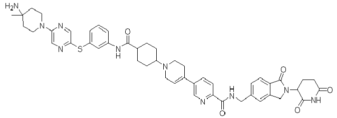 | 883 | isomer 1 |
| **301** | 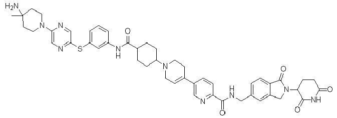 | 883 | isomer 2 |
| **302** | 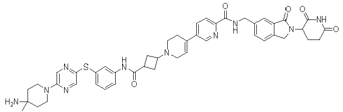 | 855 | |
| **303** | 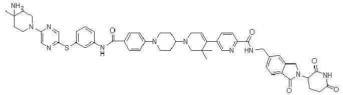 | 988 | |
| 304 | 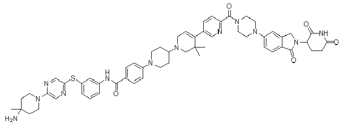 | 1043 | |
| **305** | 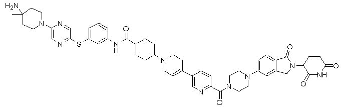 | 938 | |
| **306** | 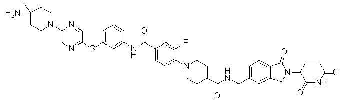 | 820 | |
| **307** | 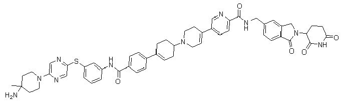 | 957 | |
| **308** | 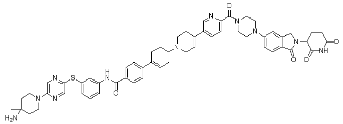 | 1012 | |
| **309** | 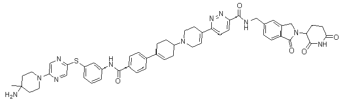 | 958 | |
| **310** | 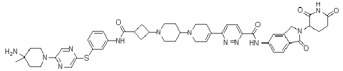 | 925 | |
| **311** | 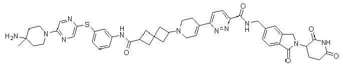 | 896 | |
| **313** | 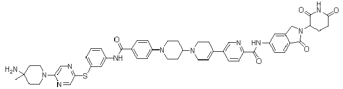 | 946 | |
| **314** | 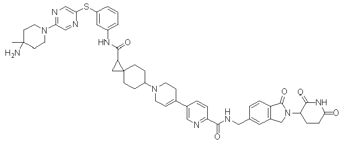 | 909 | isomer 1 |
| **315** | 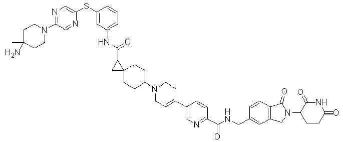 | 909 | isomer 2 |
| **316** | 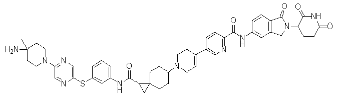 | 895 | isomer 1 |
| **317** | 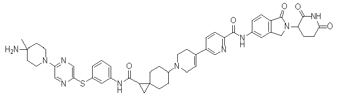 | 895 | isomer 2 |
| **318** | 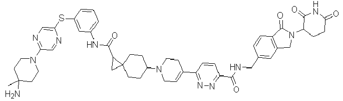 | 910 | isomer 1 |
| **319** | 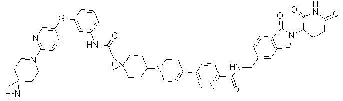 | 910 | isomer 2 |
| **320** | 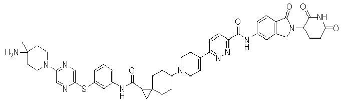 | 897 | isomer 1 |
| **321** | 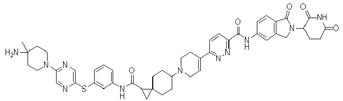 | 897 | isomer 2 |
| **322** | 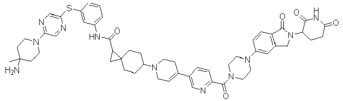 | 965 | isomer 1 |
| **323** | 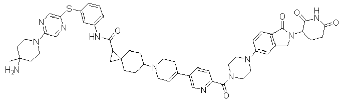 | 965 | isomer 2 |
| **324** | 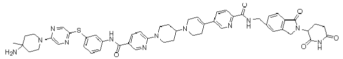 | 961 | |
| **325** | 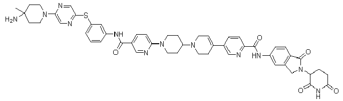 | 947 | |
| **326** | 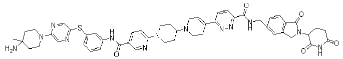 | 962 | |
| **327** | 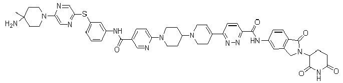 | 948 | |
| **328** | 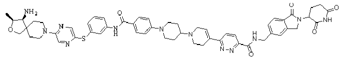 | 1017 | |
| **329** | 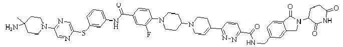 | 979 | |
| **330** | 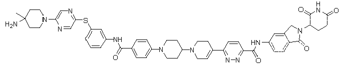 | 947 | |
| **331** | 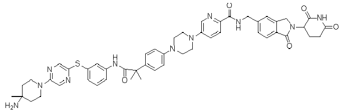 | 923 | |
| **332** | 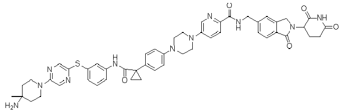 | 920 | |
| **333** | 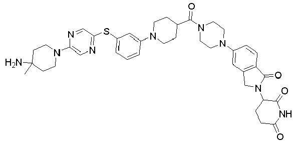 | 738 | |
| 334 | 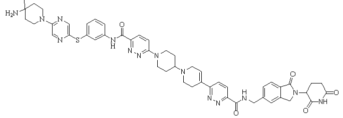 | 962.4 | |
| **335** | 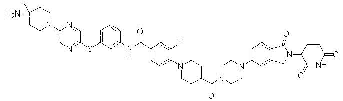 | 875 | |
| **336** | 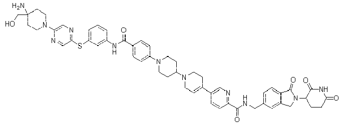 | 976 | |
| **337** | 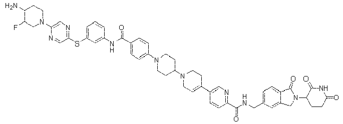 | 964 | |
| **338** | 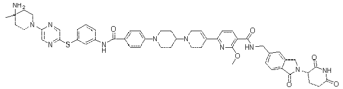 | 990 | |
| **339** | 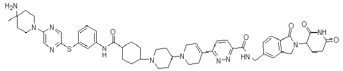 | 967 | |
| **340** | 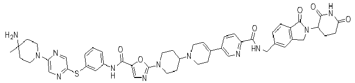 | 951 | |
| **341** | 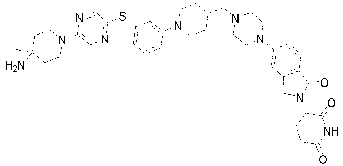 | | |
| **353** | 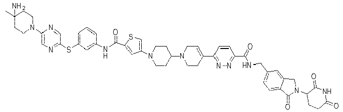 | 967 | |
| **354** | 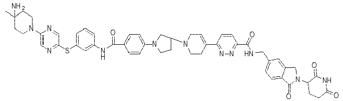 | 947 | |
| **355** | 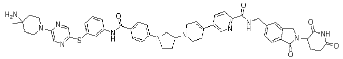 | 946 | |
| **357** | 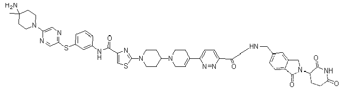 | 968 | |
| **358** | 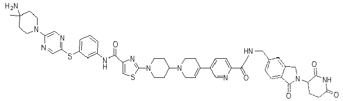 | 967 | |
| **359** | 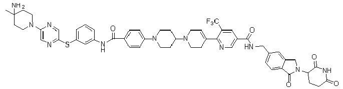 | 1028 | |
| **360** | 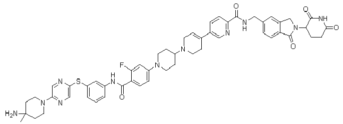 | 978 | |
| **365** | 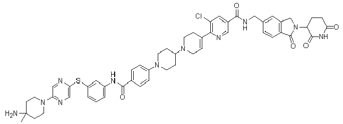 | 994 | |
| **368** | 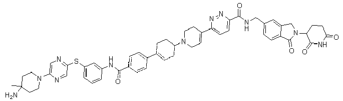 | 958 | |
| **370** | 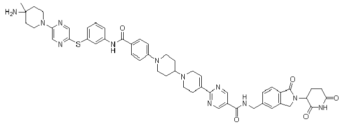 | 961 | |
| **371** | 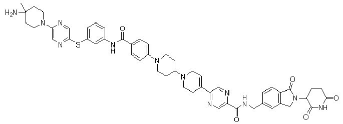 | 961 | |
| **372** | 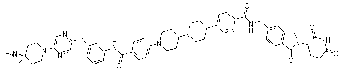 | 962 | |
| **373** | 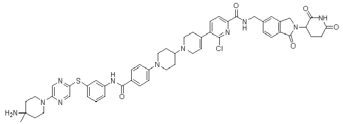 | 994 | |
| **374** | 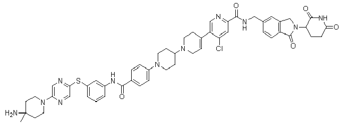 | 994 | |
| **375** | 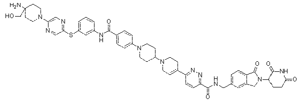 | 977 | |
| **376** | 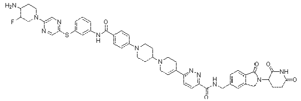 | 965 | |
| **377** | 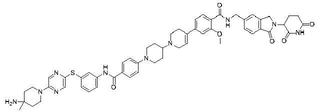 | 989 | |
| **378** | 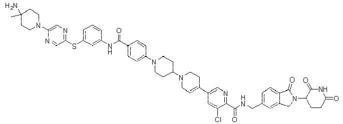 | 994 | |
| **379** | 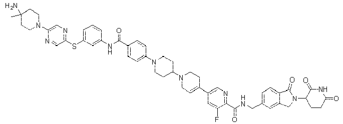 | 978 | |
| **383** | 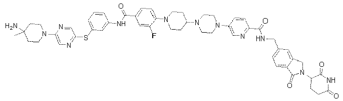 | 981.5 | ¹H NMR (400 MHz, DMSO-d₆): δ 11.74 (s, 1 H), 10.97 (s,1 H), 10.22 (s,1 H), 9.32 (s, 1 H), 8.38 (s, 5 H), 8.19 (s, 1 H), 8.03-7.99 (m, 1 H), 7.80-7.60 (m, 6 H), 7.53-7.45 (m, 2 H), 7.32-7.28 (m, 1 H), 7.18-7.14 (m, 1 H), 7.00-6.98 (m, 1 H), 5.11-5.08 (m, 1 H), 4.60-4.59 (m, 2 H), 4.45-4.41 (m, 1 H), 4.32-4.27 (m, 1 H), 3.68-3.66 (m, 5 H), 3.51-3.39 (m, 5 H), 3.23-3.20 (m, 2 H), 2.94-2.78 (m, 3 H), 2.61-2.57 (m, 1 H), 2.39-2.27 (m, 3 H), 1.96-1.72 (m, 8 H), 1.38 (s, 3 H), 1.23 (s, 1 H). |
| 385 | 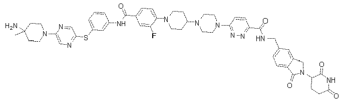 | 982 | ¹H NMR (400 MHz, DMSO-*d₆*): δ 11.77-11.76 (m, 1 H), 10.97(s,1 H), 10.21(s,1 H), 9.61-9.58 (m, 1 H), 8.39-8.33 (m, 4 H), 8.20 (s,1 H), 7.97-7.95 (m, 1H), 7.80-7.67 (m, 5H), 7.54-7.46 (m, 3 H),7.18-7.14 (m, 1 H), 7.01-6.99 (m, 1H), 6.98-6.96 (m, 1 H), 5.12-5.07 (m, 1 H), 4.66-4.61 (m, 4 H), 4.46-4.41(m, 1 H), 4.32-4.27 (m, 1 H), 3.68-3.62 (m, 8 H), 3.43-3.39 (m, 4 H),3.21-3.19 (m, 2H), 2.91-2.84 (m, 4H), 2.61-2.51 (m, 1H), 2.40-2.36 (m,3H), 2.02-1.75 (m,3H),1.38 (s, 3 H), 1.23-1.19 (m,2H). |
| 388 | 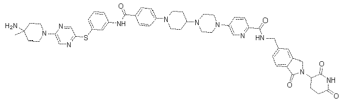 | 963 | ¹H NMR (400 MHz, DMSO-d₆): δ 10.97 (s, 1 H), 9.99 (s,1 H), 9.17 (s,1 H), 8.38 (s, 2 H), 8.19-8.18 (m, 1 H), 8.04 (s, 3 H), 7.91-7.81 (m, 4 H), 7.68-7.66 (m, 2 H), 7.51-7.44 (m, 3 H), 7.30-7.26 (m, 1 H), 7.06-7.04 (m, 2 H), 6.96-6.94 (m, 1 H), 5.11-5.07 (m, 1 H), 4.59-4.57 (m, 2 H), 4.45-4.27 (m, 3 H), 4.06-4.02 (m, 5 H), 3.70-3.59 (m, 3 H), 2.95-2.81 (m, 4 H), 2.67-2.56 (m, 2 H), 2.40-2.32 (m, 1 H), 2.22-2.17 (m, 1 H), 2.00-1.95 (m, 2 H), 1.74-1.72 (m, 6 H), 1.37 (s, 3 H), 1.23-1.22, (m, 1 H). |
| **389** | 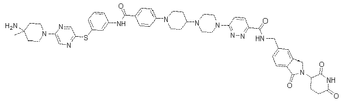 | 964 | 1H NMR (400 MHz, DMSO-*d₆*): δ 11.79 (s, 1 H), 10.97 (s,1 H), 10.05 (s,1 H), 9.58 (t, *J*=6.4Hz,1 H), 8.38-8.37 (m, 4 H), 8.19 (d, *J*=1.2Hz,1 H), 7.96 (d, *J*=9.6Hz,1 H), 7.88 (d, *J*=9.2Hz,2 H), 7.77-7.76 (m, 1 H),7.72-7.67 (m, 2 H), 7.54-7.46 (m, 3H), 7.28 (t, *J*=8.2Hz,1 H), 7.09 (d, *J*=8.8Hz,2 H), 6.96 (d, *J*=8Hz,1 H), 5.12-5.07 (m, 1 H), 4.64-4.61(m, 4 H), 4.46-4.27 (m, 2 H), 4.09-4.02 (m, 4 H), 3.65-3.64 (m, 4 H),3.44-3.38 (m, 3H), 3.20-3.17 (m, 2H), 2.90-2.83 (m, 3H), 2.61-2.57 (m, 1H), 2.43-2.24 (m, 4H),2.01-1.97 (m, 1 H), 1.88-1.71 (m, 6H), 1.38 (s, 3 H). |
| **392** | 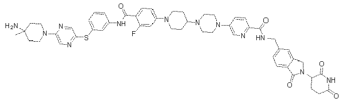 | MS( M/2+ H+) 491.3 | ¹H NMR (400 MHz, DMSO-d6) δ 11.56 (s, 1H), 11.00 (s, 1H), 10.05 (d, J = 1.9 Hz, 1H), 9.25 (t, J = 6.4 Hz, 1H), 8.39 (d, J = 1.4 Hz, 2H), 8.31 (s, 3H), 8.20 (d, J = 1.3 Hz, 1H), 7.94 (d, J = 8.8 Hz, 1H), 7.68 (d, J = 7.4 Hz, 2H), 7.64 - 7.49 (m, 4H), 7.46 (d, J = 7.9 Hz, 1H), 7.28 (t, J = 7.9 Hz, 1H), 6.96 (d, J = 7.8 Hz, 1H), 6.93 - 6.86 (m, 2H), 5.10 (dd, J = 13.3, 5.1 Hz, 1H), 4.59 (d, J = 6.3 Hz, 2H), 4.48 - 4.24 (m, 2H), 3.62 (d, J = 11.4 Hz, 2H), 3.42 (tt, J = 9.7, 5.4 Hz, 6H), 3.19 (d, J = 10.9 Hz, 3H), 2.98 - 2.79 (m, 4H), 2.64 - 2.55 (m, 1H), 2.44 - 2.30 (m, 1H), 2.23 (d, J = 11.5 Hz, 2H), 2.05 - 1.94 (m, 2H), 1.76 (ddd, J = 23.7, 11.5, 6.8 Hz, 6H), 1.37 (s, 3H). |
| **393** | 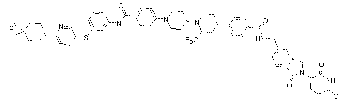 | 1032 | |
| **394** | 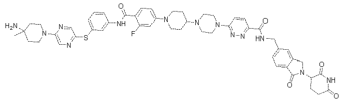 | 491.8 (M/2+ 1) | ¹H NMR (400 MHz, DMSO-d6) δ 11.88 (s, 1H), 10.99 (s, 1H), 10.06 (d, J = 1.8 Hz, 1H), 9.62 (t, J = 6.4 Hz, 1H), 8.47 - 8.36 (m, 4H), 8.19 (d, J = 1.3 Hz, 1H), 7.97 (d, J = 9.5 Hz, 1H), 7.71 - 7.64 (m, 2H), 7.62 - 7.49 (m, 4H), 7.47 (d, J = 7.8 Hz, 1H), 7.27 (t, J = 8.0 Hz, 1H), 6.96 (dd, J = 8.2, 1.8 Hz, 1H), 6.92 - 6.84 (m, 2H), 5.10 (dd, J = 13.3, 5.1 Hz, 1H), 4.61 (d, J = 5.8 Hz, 3H), 4.48 - 4.23 (m, 2H), 4.13 - 3.98 (m, 4H), 3.66 - 3.57 (m, 5H), 3.43 - 3.36 (m, |
| | | | 2H), 3.17 (d, J = 11.6 Hz, 3H), 2.86 (q, J = 12.4 Hz, 3H), 2.63 - 2.54 (m, 1H), 2.37 (qd, J = 13.4, 4.6 Hz, 1H), 2.22 (d, J= 11.6 Hz, 2H), 1.98 (ddd, J = 9.0, 6.7, 4.4 Hz, 1H), 1.80 (dq, J = 9.5, 5.4, 4.7 Hz, 3H), 1.74 (tq, J = 4.8, 2.8, 2.3 Hz, 3H), 1.37 (s, 3H). |
| **395** | 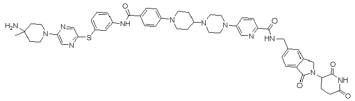 | MS( M/2+ H+) 482.2 | ¹H NMR (400 MHz, DMSO-d68 11.75 (s, 1H),δ 10.99 (s, 1H), 10.00 (s, 1H), 9.18 (s, 1H), 8.39-8.28 (m, 3H), 8.20 (d, J = 1.4 Hz, 1H), 8.05 (s, 3H), 7.93 - 7.78 (m, 4H), 7.67 (dd, J = 7.9, 2.8 Hz, 2H), 7.51 (s, 1H), 7.45 (d, J = 7.9 Hz, 1H), 7.28 (t, J = 8.0 Hz, 1H), 7.05 (s, 2H), 6.95 (dd, J = 7.7, 1.6 Hz, 1H), 5.10 (dd, J = 13.2, 5.1 Hz, 1H), 4.58 (d, J = 6.4 Hz, 2H), 4.45 - 4.25 (m, 2H), 4.05 (d, J = 15.1 Hz, 5H), 3.64-3.35 (m, 8H), 2.87 (dt, J = 28.0, 12.5 Hz, 4H), 2.59 (d, J = 17.2 Hz, 3H), 2.44 - 2.29 (m, 1H), 2.21 (s, 1H), 2.06 - 1.89 (m, 2H), 1.73 (s, 5H), 1.37 (s, 3H). |
| **396** | 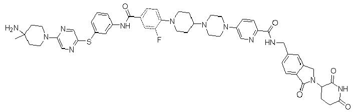 | 981.5 | ¹H NMR (400 MHz, DMSO-d₆): δ 11.74 (s, 1 H), 10.97 (s,1 H), 10.22 (s,1 H), 9.32 (s, 1 H), 8.38 (s, 5 H), 8.19 (s, 1 H), 8.03-7.99 (m, 1 H), 7.80-7.60 (m, 6 H), 7.53-7.45 (m, 2 H), 7.32-7.28 (m, 1 H), 7.18-7.14 (m, 1 H), 7.00-6.98 (m, 1 H), 5.11-5.08 (m, 1 H), 4.60-4.59 (m, 2 H), 4.45-4.41 (m, 1 H), 4.32-4.27 (m, 1 H), 3.68-3.66 (m, 5 H), 3.51-3.39 (m, 5 H), 3.23-3.20 (m, 2 H), 2.94-2.78 (m, 3 H), 2.61-2.57 (m, 1 H), 2.39-2.27 (m, 3 H), 1.96-1.72 (m, 8 H), 1.38 (s, 3 H), 1.23 (s, 1 H). |
| 397 | 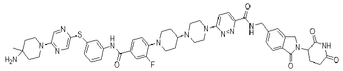 | 491.7 MS( M/2+ H+) | 1H NMR (400 MHz, DMSO-d6) δ 11.86 (s, 1H), 10.99 (s, 1H), 10.23 (s, 1H), 9.62 (t, J = 6.4 Hz, 1H), 8.48 - 8.32 (m, 4H), 8.19 (d, J = 1.3 Hz, 1H), 7.96 (d, J = 9.4 Hz, 1H), 7.81-7.66 (m, 5H), 7.55 (d, J = 8.0 Hz, 2H), 7.50 - 7.41 (m, 1H), 7.29 (t, J = 8.0 Hz, 1H), 7.16 (t, J = 8.7 Hz, 1H), 7.03 - 6.90 (m, 1H), 5.10 (dd, J = 13.3, 5.1 Hz, 1H), 4.63 (t, J = 11.6 Hz, 4H), 4.49 - 4.23 (m, 2H), 4.04 (dt, J = 14.0, 4.9 Hz, 2H), 3.67 (d, J = 12.2 Hz, 5H), 3.48 - 3.36 (m, 3H), 3.28 - 3.08 (m, 2H), 2.98 - 2.71 (m, 3H), 2.65 - 2.54 (m, 1H), 2.45 - 2.19 (m, 3H), 2.05 - 1.65 (m, 8H), |
| | | | 1.38 (s, 3H). |
| **398** | 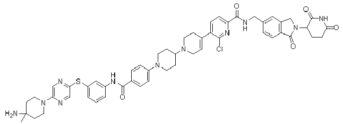 | 994 | ¹H NMR (400 MHz, DMSO-*d*₆) δ 11.19 (s, 1H), 10.99 (s, 1H), 10.05 (s, 1H), 9.42 (t, J = 6.4 Hz, 1H), 8.47-8.19 (m, 5H), 8.07 (d, J = 7.7 Hz, 1H), 7.98-7.67 (m, 6H), 7.59-7.41 (m, 2H), 7.28 (t, J = 8.0 Hz, 1H), 7.16-6.92 (m, 3H), 5.95 (d, J = 2.8 Hz, 1H), 5.10 (dd, J = 13.3, 5.1 Hz, 1H), 4.71-4.25 (m, 4H), 4.19-3.87 (m, 6H), 3.48-3.35 (m, 3H), 3.21 (d, J = 10.8 Hz, 1H), 3.11-2.80 (m, 4H), 2.61 (s, 2H), 2.43-2.19 (m, 3H), 2.07-1.69 (m, 6H), 1.37 (s, 3H). |
| **399** | 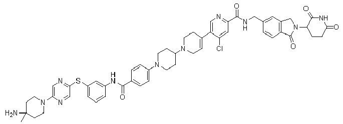 | 994 | ¹H NMR (400 MHz, DMSO-*d*₆) δ 11.19 (s, 1H), 10.99 (s, 1H), 10.05 (s, 1H), 9.42 (t, *J* = 6.4 Hz, 1H), 8.46 -8.17 (m, 5H), 8.07 (d, *J* = 7.7 Hz, 1H), 8.00-7.64 (m, 6H), 7.62-7.38 (m, 2H), 7.28 (t, *J* = 8.0 Hz, 1H), 7.16-6.92 (m, 3H), 5.95 (d, *J* = 2.8 Hz, 1H), 5.10 (dd, *J* = 13.3, 5.1 Hz, 1H), 4.60 (d, *J* = 6.4 Hz, 2H), 4.52-4.23 (m, 2H), 4.20-3.88 (m, 6H), 3.77 (s, 1H), 3.45-3.35 (m, 3H), 3.21 (d, *J* = 10.8 Hz, 1H), 3.08-2.81 (m, 4H), 2.59 (d, *J* = 17.7 Hz, 2H), 2.44-2.17 (m, 3H), 1.99 (ddd, *J* = 11.3, 6.6, 4.1 Hz, 1H), 1.78 (dtd, *J* = 24.5, 13.7, 11.9, 6.6 Hz, 6H), 1.37 (s, 3H). |
| **400** | 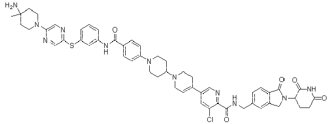 | 994 | |
| **401** | 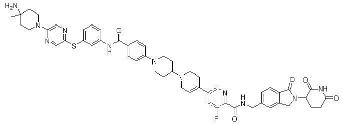 | 978 | ¹H NMR (400 MHz, DMSO-d6) δ 11.05 (s, 1H), 10.99 (s, 1H), 10.03 (s, 1H), 9.38 (t, J = 6.3 Hz, 1H), 8.67 (s, 1H), 8.39 (d, J = 1.5 Hz, 1H), 8.27 (s, 2H), 8.19 (d, J = 1.4 Hz, 1H), 8.04 (dd, J = 12.1, 1.9 Hz, 1H), 7.87 (d, J = 8.6 Hz, 2H), 7.78 (t, J = 2.0 Hz, 1H), 7.72 - 7.64 (m, 2H), 7.55 (s, 1H), 7.48 (d, J = 8.1 Hz, 1H), 7.28 (t, J = 8.0 Hz, 1H), 7.07 (d, J = 8.9 Hz, 2H), 6.99 - 6.92 (m, 1H), 6.61 (s, 1H), 5.11 (dd, J = 13.3, 5.0 Hz, 1H), 4.58 (d, J = 6.3 Hz, 2H), 4.45 (d, J = 17.4 Hz, 2H), 4.30 (d, J = 17.3 Hz, 1H), 3.86 (s, 3H), 3.76 (s, 1H), 3.57 - 3.48 (m, 1H), 3.45 - 3.35 (m, 2H), 3.19 (d, J = 23.7 Hz, 2H), 3.00 - 2.80 (m, 5H), 2.75 (d, J = 21.3 Hz, 1H), 2.59 (d, J = 16.6 Hz, 1H), 2.42 - 2.13 (m, |
| | | | 4H), 2.05 - 1.93 (m, 2H), 1.78 (dd, J = 22.2, 13.5 Hz, 5H), 1.37 (s, 3H). |
| **402** | 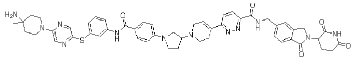 | 947 | |
| **403** | 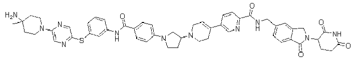 | 946 | |
| **404** | 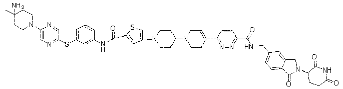 | 967 | |
| **405** | 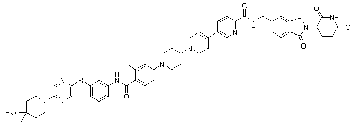 | 978 | |
| **406** | 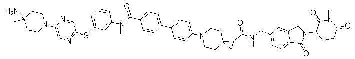 | 904.3 | 1H NMR (400 MHz, DMSO-d6): δ 10.97 (d, J = 6.8 Hz, 1 H), 10.30-10.29 (m, 1 H), 8.79-8.75 (m, 1 H), 8.40-8.39 (m, 1 H), 8.22-8.21 (m, 1 H), 8.09-7.99 (m,5 H), 7.82-7.66 (m, 7 H), 7.48-7.41 (m, 1 H),7.42-7.40 (m, 1 H), 7.34-7.32 (m, 1 H), 7.30-7.22 (m, 1 H), 7.02-6.99 (m, 1 H), 5.09-5.07 (m, 1 H), 4.50-4.44 (m, 1 H), 4.39-4.29 (m, 3 H), 4.07-4.02 (m, 3 H),3.41-3.35 (m, 5 H), 3.07-3.01(m, 1 H),2.99-2.85 (m,1 H), 2.57-2.51 (m, 1 H), 2.35-2.30 (m, 2 H), 1.96-1.88 (m, 3 H), 1.76-1.67 (m, 6 H), 1.38 (s, 3 H), 1.07-1.07 (m, 1 H) ,0.89-0.85 (m, 1 H). |
| **407** | 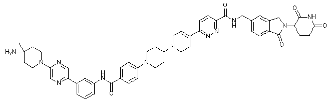 | 929 | |
| **408** | 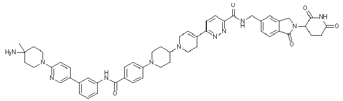 | 928 | |
| **409** | 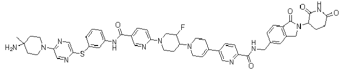 | 979 | ¹H NMR (400 MHz, DMSO-*d*₆) δ 11.44 (s, 1H), 10.99 (s, 1H), 10.31 (s, 1H), 9.50 (t, J = 6.4 Hz, 1H), 8.76 (dd, J = 33.2, 2.3 Hz, 2H), 8.51-8.04 (m, 9H), 7.81-7.41 (m, 6H), 7.37-7.17 (m, 2H), 6.98 (d, J = 7.8 Hz, 1H), 6.51 (s, 1H), 5.65 (dd, J = 48.9, 17.1 Hz, 1H), |
| | | | 5.17-4.95 (m, 2H), 4.68 (dd, J = 58.6, 9.8 Hz, 4H), 4.43 (d, J = 17.4 Hz, 2H), 3.79 (s, 1H), 3.40 (dt, J = 13.3, 4.8 Hz, 4H), 3.22-2.83 (m, 4H), 2.67-2.54 (m, 1H), 2.46-2.19 (m, 3H), 2.19 -1.68 (m, 7H), 1.38 (s, 3H). |
| **410** | 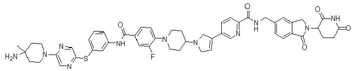 | 482.8 (M/2+ 1) | ¹H NMR (400 MHz, DMSO-d6) δ 12.37 (s, 1H), 10.99 (s, 1H), 10.22 (s, 1H), 9.53 (t, J = 6.4 Hz, 1H), 8.86 (d, J = 2.1 Hz, 1H), 8.39 (d, J = 1.5 Hz, 1H), 8.32 (s, 3H), 8.24 - 8.15 (m, 2H), 8.09 (d, J = 8.2 Hz, 1H), 7.83 -7.74 (m, 3H), 7.69 (t, J = 7.9 Hz, 2H), 7.55 (s, 1H), 7.48 (d, J = 7.9 Hz, 1H), 7.30 (t, J = 8.0 Hz, 1H), 7.17 (t, J = 8.8 Hz, 1H), 6.98 (d, J = 8.2, 1.7 Hz, 1H), 6.84 (s, 1H), 5.10 (dd, J = 13.3, 5.1 Hz, 1H), 4.89 - 4.52 (m, 4H), 4.49 - 4.23 (m, 4H), 4.16 - 3.97 (m, 2H), 3.77 - 3.62 (m, 2H), 3.56 (s, 1H), 3.47 - 3.34 (m, 2H), 2.99 - 2.72 (m, 3H), 2.59 (d, J = 17.5 Hz, 1H), 2.44 - 2.34 (m, 1H), 2.33 - 2.24 (m, 1H), 2.20 (s, 1H), 2.10 - 1.92 (m, 3H), 1.88 - 1.63 (m, 4H), 1.37 (s, 3H). |
| **411** | 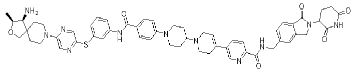 | 508.7 (M/2+ 1) | ¹H NMR (400 MHz, DMSO-d6) δ 11.28 (s, 1H), 10.99 (s, 1H), 10.06 (s, 1H), 9.50 (t, J = 6.4 Hz, 1H), 8.80 (d, J = 2.2 Hz, 1H), 8.39 (d, J = 1.5 Hz, 1H), 8.28 - 8.20 (m, 3H), 8.19 (d, J = 1.4 Hz, 1H), 8.11 (dd, J = 8.3, 2.2 Hz, 1H), 8.06 (d, J = 8.2 Hz, 1H), 7.88 (d, J = 8.6 Hz, 2H), 7.77 (d, J = 2.0 Hz, 1H), 7.69 (dd, J = 10.2, 7.8 Hz, 2H), 7.54 (s, 1H), 7.47 (d, J = 7.9 Hz, 1H), 7.28 (t, J = 8.0 Hz, 1H), 7.09 (d, J = 8.7 Hz, 2H), 6.95 (d, J = 7.7 Hz, 1H), 6.50 (s, 1H), 5.10 (dd, J = 13.3, 5.1 Hz, 1H), 4.61 (d, J = 6.4 Hz, 2H), 4.43 (d, J = 17.4 Hz, 2H),4.33-4.04(m, 5H), 3.96 (s, 2H), 3.92 (d, J = 9.0 Hz, 1H), 3.75 (s, 1H), 3.65 (d, J = 9.1 Hz, 1H), 3.53 (s, 1H), 3.37 - 3.33 (m, 1H), 3.23 (d, J = 10.4 Hz, 1H), 3.13 - 2.80 (m, 8H), 2.63 - 2.55 (m, 1H), 2.37 (dd, J = 13.1, 4.6 Hz, 1H), 2.34 - 2.27 (m, 1H), 2.23 (d, J = 11.5 Hz, 1H), 2.01 - 1.96 (m, 1H), 1.90 - 1.81 (m, 2H), 1.77 (d, J = 12.3 Hz, 2H), 1.67 (d, J = 14.0 Hz, 1H), 1.58 (d, J = 13.0 Hz, 1H), 1.23 (d, J = 4.4 Hz, 3H) |
| **412** | 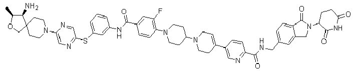 | 517.6 (M/2+ 1) | ¹H NMR (400 MHz, DMSO-d6) δ 11.14 (s, 1H), 10.99 (s, 1H), 10.21 (s, 1H), 9.50 (t, J = 6.4 Hz, 1H), 8.81 (d, J = 2.1 Hz, 1H), 8.40 (d, J = 1.4 Hz, 1H), 8.28 - 8.00 (m, 6H), 7.87 - 7.62 (m, 5H), 7.59 - 7.42 (m, 2H), 7.30 (t, J = 8.0 Hz, 1H), 7.17 (t, J = 8.7 Hz, 1H), 6.98 (d, J = 7.8 Hz, 1H), 6.52 (s, 1H), 5.10 (dd, J = 13.2, 5.0 Hz, 1H), 4.61 (d, J = 6.3 Hz, 2H), 4.48 - 4.26 (m, 2H), 4.19 (d, J = 107.0 Hz, 2H), 4.00 (s, 2H), 3.91 (d, J = 9.1 Hz, 1H), 3.70 - 3.61 (m, 1H), 3.37 (s, 1H), 3.25 (d, J = 10.9 Hz, 1H), 3.15 - 2.97 (m, 2H), 2.92 - 2.77 (m, 3H), 2.59 (d, J = 16.8 Hz, 1H), 2.45 - 2.20 (m, 2H), 2.05 - 1.86 (m, 4H), 1.84 - 1.50 (m, 4H), 1.33 - 1.16 (m, 8H). |
| **413** | 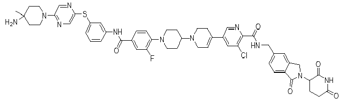 | MS( M/2+ H+) 506.6 | ¹H NMR (400 MHz, DMSO-d6) δ 11.02 (s, 1H), 10.21 (s, 1H), 9.43 ((t, J = 6.4 Hz, 1H), 8.73 (s, 1H), 8.4 (s, 1H),8.49 - 8.35 (m, 3H), 8.19- 8.08 (m, 2H), 7.85 - 7.64 (m, 5H), 7.53 (s, 1H), 7.29 (t, J = 8.0 Hz, 1H), 7.13 (d, J = 8.3 Hz, 1H), 7.00 - 6.93 (m, 1H), 6.6 (s, 1H), 5.12 (dd, J = 13.3, 5.1 Hz, 1H), 4.61 (d, J = 6.4 Hz, 2H), 4.46 - 4.26 (m, 2H), 4.09 - 3.94 (m, 4H), 3.54 - 3.19 (m, 6H), 3.10 (t, J = 12.9 Hz, 1H), 2.76 (t, J = 11.6 Hz, 2H), , 2.44 - 2.21 (m, 6H), 2.10 - 1.94 (m, 4H), 1.89 - 1.68 (m, 4H), 1.38 (s, 3H). |
| **414** | 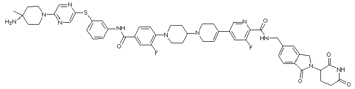 | 498.6 (M/2+ 1) | ¹H NMR (400 MHz, DMSO-d6) δ 11.19 (s, 1H), 11.00 (s, 1H), 10.21 (s, 1H), 9.38 (d, J = 6.6 Hz, 1H), 8.68 (s, 1H), 8.39 (d, J = 1.4 Hz, 1H), 8.30 (s, 3H), 8.20 (d, J = 1.3 Hz, 1H), 8.10 - 8.02 (m, 1H), 7.83 - 7.64 (m, 5H), 7.55 (s, 1H), 7.48 (d, J = 8.3 Hz, 1H), 7.38 - 7.22 (m, 2H), 7.16 (dd, J = 18.9, 10.0 Hz, 1H), 7.03 - 6.93 (m, 1H), 6.63 (s, 1H), 5.11 (dd, J = 13.3, 5.0 Hz, 1H), 4.66 - 4.25 (m, 4H), 4.13 - 3.75 (m, 2H), 3.73 - 3.60 (m, 2H), 3.55 - 3.34 (m, 2H), 3.23 (d, J = 10.5 Hz, 1H), 3.06 - 2.76 (m, 5H), 2.59 (d, J = 17.0 Hz, 1H), 2.42 - 2.11 (m, 2H), 1.97 (d, J = 10.5 Hz, 3H), 1.85 - 1.65 (m, 4H), 1.37 (s, 3H), 1.24 (d, J = 9.3 Hz, 1H). |
| **415** | 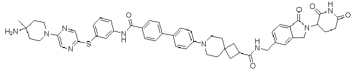 | MS( M/2+ H+) 459.7 | 1H NMR (400 MHz, DMSO-d6) δ 11.00 (s, 1H), 10.39 (s, 1H), 8.45 (t, J= 6.1 Hz, 1H), 8.40 (d, J = 1.5 Hz, 1H), 8.36 - 8.25 (m, 3H), 8.21 (d, J = 1.4 Hz, 1H), 8.04 (d, J = 8.2 Hz, 2H), 7.94 - 7.76 (m, 6H), 7.76 - 7.71 (m, 1H), 7.68 (d, J = 7.8 Hz, 1H), 7.46 (s, 1H), 7.39 (d, J = 7.9 Hz, 1H), 7.32 (t, J = 8.0 Hz, 1H), 7.00 (dd, J = 8.2, 1.8 Hz, 1H), 5.11 (dd, J = 13.3, 5.1 Hz, 1H), 4.48 - 4.25 (m,4H), 4.05 (dt, J = 14.1, 4.9 Hz, 2H), 3.53 - 3.27 (m, 6H), 3.09 (p, J = 8.4 Hz, 1H), 2.99 - 2.79 (m, 1H), 2.65 - 2.55 (m, 1H), 2.44 - 2.28 (m, 1H), 2.23 - 1.87 (m, 9H), 1.86 - 1.65 (m, 5H), 1.36 (d, J = 9.0 Hz, 3H). |
| **416** | 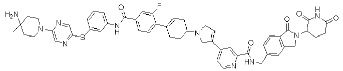 | 961 | |
| **417** | 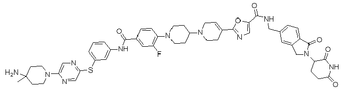 | 968 | |
| **418** | 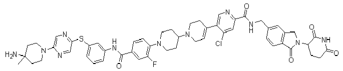 | 506.8 (M/2+ 1) | 1H NMR (400 MHz, DMSO-d6) δ 11.15 (s, 1H), 10.99 (s, 1H), 10.20 (s, 1H), 9.62 (t, J = 6.3 Hz, 1H), 8.53 (s, 1H), 8.39 (d, J = 1.5 Hz, 1H), 8.32 - 8.19 (m, 4H), 8.09 (s, 1H), 7.85 - 7.74 (m, 3H), 7.72 - 7.65 (m, 2H), 7.53 (s, 1H), 7.46 (d, J = 7.9 Hz, 1H), 7.30 (t, J = 8.0 Hz, 1H), 7.18 (t, J = 8.7 Hz, 1H), 7.02 - 6.97 (m, 1H), 6.03 (s, 1H), 5.10 (dd, J = 13.3, 5.1 Hz, 1H), 4.61 (d, J = 6.5 Hz, 2H), 4.48 - 4.25 (m, 2H), 4.10 -3.95 (m, 4H), 3.51 (s, 1H), 3.45 -3.36 (m, 2H), 3.25 (d, J = 10.2 Hz, 1H), 3.04 (s, 1H), 2.96-2.77 (m, 4H), 2.71 -2.55 (m, 2H), 2.44 - 2.21 (m, 4H), 2.06 - 1.68 (m, 8H), 1.37 (s, 3H). |
| **419** | 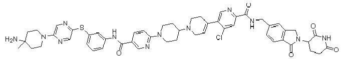 | 995 | |
| **420** | 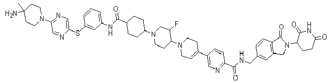 | 984 | ¹H NMR (400 MHz, DMSO-*d*₆) δ 11.85 (s, 1H), 10.99 (s, 1H), 10.44 (s, 1H), 10.13 (d, *J* = 37.0 Hz, 1H), 9.50 (t, *J* = 6.4 Hz, 1H), 8.81 (d, *J* = 2.4 Hz, 1H), 8.49 - 8.30 (m, 4H), 8.19 - 8.15 (m, 1H), 8.15 - 8.04 (m, 2H), 7.68 (d, *J* = 7.8 Hz, 1H), 7.62 - 7.51 (m, 3H), 7.47 (d, *J* = 7.8 Hz, 1H), 7.24 (td, *J* = 8.1, 2.5 Hz, 1H), 6.95 - 6.86 (m, 1H), 6.50 (s, 1H), 5.93 - 5.62 (m, 2H), 5.10 |
| | | | (dd, *J* = 13.2, 5.1 Hz, 1H), 4.61 (d, *J* = 6.4 Hz, 2H), 4.43 (d, *J* = 17.4 Hz, 2H), 4.29 (d, *J* = 17.5 Hz, 2H), 4.10 - 3.83 (m, 6H), 3.74 (s, 1H), 3.54 (s, 1H), 3.48 - 3.35 (m, 3H), 3.26 (d, *J* = 13.7 Hz, 2H), 3.04 (d, *J* = 24.0 Hz, 1H), 2.91 (ddd, *J* = 17.8, 13.7, 5.6 Hz, 2H), 2.70 (s, 1H), 2.64 - 2.54 (m, 1H), 2.37 (dd, *J* = 13.0, 4.6 Hz, 1H), 2.15 (s, 2H), 2.04 - 1.92 (m, 3H), 1.89 - 1.68 (m, 5H), 1.53 (d, *J* = 40.2 Hz, 3H), 1.38 (s, 3H). |
| **421** | 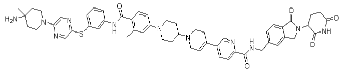 | 487.7 (M/2+ 1) | ¹H NMR (400 MHz, DMSO-d6) δ 11.32 (s, 1H), 10.99 (s, 1H), 10.18 (s, 1H), 9.50 (t, J = 6.4 Hz, 1H), 8.80 (d, J = 2.2 Hz, 1H), 8.41 - 8.31 (m, 4H), 8.19 (d, J = 1.4 Hz, 1H), 8.12 (dd, J = 8.3, 2.2 Hz, 1H), 8.06 (d, J = 8.2 Hz, 1H), 7.70 - 7.66 (m, 2H), 7.66 - 7.61 (m, 1H), 7.54 (s, 1H), 7.47 (d, J = 7.9 Hz, 1H), 7.41 (d, J = 8.3 Hz, 1H), 7.27 (t, J = 8.0 Hz, 1H), 7.04 - 6.91 (m, 3H), 6.51 (s, 1H), 5.10 (dd, J = 13.3, 5.1 Hz, 1H), 4.67 - 4.24(m , 6H), 4.08 - 3.92 (m, 4H), 3.74 (s, 1H), 3.54 (s, 1H), 3.39 (d, J = 8.3 Hz, 3H), 3.24 (d, J = 11.2 Hz, 1H), 3.10 - 2.82 (m, 5H), 2.59 (d, J = 17.4 Hz, 1H), 2.37 (s, 3H), 2.33 (d, J = 9.4 Hz, 1H), 2.24 (d, J = 11.1 Hz, 1H), 2.03 - 1.92 (m, 3H), 1.80 (td, J = 11.3, 9.1, 3.9 Hz, 2H), 1.72 (d, J = 14.4 Hz, 2H), 1.23 (s, 3H). |
| **422** | 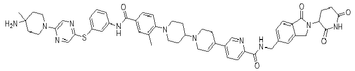 | 487.7 (M/2+ 1) | ¹H NMR (400 MHz, DMSO-d6) δ 11.17 (s, 1H), 10.99 (s, 1H), 10.16 (s, 1H), 9.54 - 9.47 (m, 1H), 8.82 (d, J = 2.1 Hz, 1H), 8.39 (d, J = 1.5 Hz, 1H), 8.30 (s, 3H), 8.20 (d, J = 1.4 Hz, 1H), 8.13 (dd, J = 8.2, 2.3 Hz, 1H), 8.07 (d, J = 8.2 Hz, 1H), 7.82 - 7.74 (m, 3H), 7.69 (dd, J = 8.4, 5.0 Hz, 2H), 7.54 (s, 1H), 7.47 (d, J = 7.9 Hz, 1H), 7.29 (t, J = 8.0 Hz, 1H), 7.12 (d, J= 8.3 Hz, 1H), 7.00 - 6.95 (m, 1H), 6.53 (s, 1H),5.10 (dd, J = 13.3, 5.1 Hz, 1H), 4.68 - 4.26 (m,6H), 4.07 - 3.99 (m, 4H), 3.80 (s, 1H), 3.53 - 3.36 (m, 2H), 3.32 (d, J = 10.9 Hz, 3H), 3.03 (s, 1H), 2.92 - 2.84 (m, 2H), 2.73 (t, J = 11.7 Hz, 2H), 2.59 (d, J = 17.0 Hz, 1H), 2.41 - 2.35 (m, 1H), 2.33 (s, 3H), 2.23 (s, 1H), 1.99 (d, J = 13.6 Hz, 3H), 1.79 (d, J = 9.4 Hz, |
| | | | 2H), 1.72 (d, J = 14.1 Hz, 2H), 1.23 (s, 3H). |
| **423** | 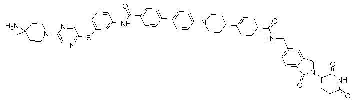 | 479.8 (M/2+ 1) | ¹H NMR (400 MHz, DMSO-d6) δ 11.00 (s, 1H), 10.41 (s, 1H), 8.51 (t, J = 6.2 Hz, 1H), 8.42 - 8.29 (m, 4H), 8.21 (d, J = 1.3 Hz, 1H), 8.10 - 8.01 (m, 2H), 7.96 - 7.89 (m, 3H), 7.87 (d, J = 8.2 Hz, 2H), 7.78 (d, J = 2.0 Hz, 1H), 7.74 (dd, J = 7.8, 2.1 Hz, 1H), 7.68 (d, J = 7.8 Hz, 1H), 7.46 (s, 1H), 7.39 (dd, J = 8.7, 4.4 Hz, 2H), 7.35 - 7.28 (m, 1H), 7.03 - 6.97 (m, 1H), 5.11 (dd, J = 13.3, 5.1 Hz, 1H), 4.49 - 4.26 (m, 5H), 4.05 (dt, J = 13.9, 4.9 Hz, 2H), 3.61 (s, 5H), 3.41 (ddd, J = 13.6, 9.4, 3.6 Hz, 2H), 2.91 (ddd, J = 17.8, 13.7, 5.3 Hz, 1H), 2.71 - 2.53 (m, 1H), 2.41 (td, J = 12.1, 4.0 Hz, 2H), 2.26 (d, J = 9.3 Hz, 1H), 2.17 (s, 2H), 2.09 - 2.03 (m, 5H), 1.88 (q, J = 5.8 Hz, 3H), 1.80 (dd, J = 9.3, 4.0 Hz, 1H), 1.73 (d, J = 13.8 Hz, 2H), 1.59 (dt, J = 13.3, 9.1 Hz, 1H), 1.37 (s, 3H). |
| **424** | 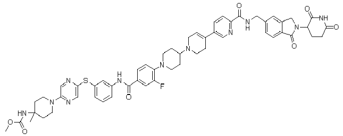 | 1036 | |
| **425** | 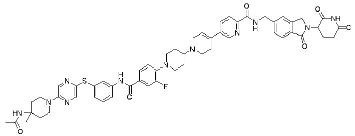 | 510.6 (M/2+ 1) | 1H NMR (400 MHz, DMSO-d6) δ 10.13 (d, J = 23.7 Hz, 1H), 9.34 (s, 0H), 8.81 - 8.63 (m, 2H), 8.36 (d, J = 1.8 Hz, 1H), 8.18 (t, J = 1.7 Hz, 1H), 8.00 (s, 1H), 7.70 (dt, J = 11.7, 8.3 Hz, 5H), 7.55 - 7.41 (m, 3H), 7.35 - 7.10 (m, 3H), 6.96 (d, J = 7.9 Hz, 1H), 5.09 (dd, J = 13.3, 4.7 Hz, 2H), 4.61 (t, J = 10.3 Hz, 4H), 4.50 - 4.23 (m, 3H), 3.94 (d, J = 13.7 Hz, 4H), 3.83 - 3.49 (m, 2H), 2.78 (d, J = 8.4 Hz, 2H), 2.63 (d, J = 23.5 Hz, 1H), 2.44 - 2.29 (m, 3H), 2.15 (d, J = 13.9 Hz, 4H), 1.96 (t, J = 16.4 Hz, 3H), 1.81 (s, 5H), 1.51 - 1.39 (m, 3H), 1.28 (s, 4H). |
| **426** | 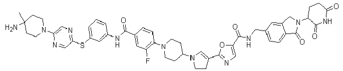 | 954 | |
| **427** | 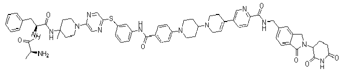 | 1178. 5 | |
| **428** | 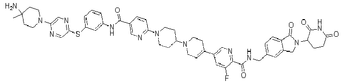 | 979 | ¹H NMR (400 MHz, DMSO-d6) δ 11.16 (s, 1H), 11.00 (s, 1H), 10.27 (s, 1H), 9.38 (t, J = 6.3 Hz, 1H), 8.67 (s, 1H), 8.39 (d, J = 1.5 Hz, 1H), 8.28 (s, |
| | | | 2H), 8.20 (d, J = 1.4 Hz, 1H), 8.07 - 8.00 (m, 1H), 7.75 (t, J = 2.0 Hz, 1H), 7.70 (d, J = 7.9 Hz, 2H), 7.55 (s, 1H), 7.48 (d, J = 7.9 Hz, 1H), 7.30 (t, J = 8.0 Hz, 1H), 7.18 (d, J = 8.9 Hz, 1H), 7.01 - 6.94 (m, 1H), 6.61 (s, 1H), 5.11 (dd, J= 13.4, 5.1 Hz, 1H), 4.67 (d, J = 13.1 Hz, 2H), 4.58 (d, J = 6.4 Hz, 2H),4.44(d,1H), 4.32 (s, 2H), 4.05(m,3H),3.97 (s, 2H), 3.74 (s, 1H), 3.64 (s, 1H), 3.40 (t, J = 10.8 Hz, 2H), 3.22 (s, 1H), 3.10 - 2.81 (m, 5H), 2.57(m,1H),2.45 - 2.14 (m, 4H), 2.05 - 1.93 (m, 2H), 1.78 (dd, J = 20.9, 12.9 Hz, 5H), 1.37 (s, 3H). |
| **429** | 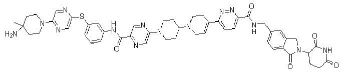 | 963 | |
| **430** | 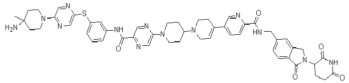 | 481.8 (M/2+ 1) | ¹H NMR (400 MHz, DMSO-d6) δ 11.16 (s, 1H), 10.99 (s, 1H), 10.31 (s, 1H), 9.49 (t, J = 6.4 Hz, 1H), 8.80 (d, J = 2.1 Hz, 1H), 8.73 (d, J = 1.1 Hz, 1H), 8.45 - 8.35 (m, 2H), 8.27 (s, 3H), 8.20 (d, J = 1.3 Hz, 1H), 8.14 - 8.09 (m, 1H), 8.06 (d, J = 8.2 Hz, 1H), 7.91 (t, J = 1.9 Hz, 1H), 7.80 - 7.64 (m, 2H), 7.58 - 7.43 (m, 2H), 7.29 (t, J = 8.0 Hz, 1H), 7.00 - 6.93 (m, 1H), 6.51 (s, 1H), 5.10 (dd, J = 13.3, 5.0 Hz, 1H), 4.73 (d, J = 12.8 Hz, 2H), 4.61 (d, J = 6.4 Hz, 2H), 4.43 (d, J = 17.3 Hz, 1H), 4.29 (d, J = 17.4 Hz, 1H), 3.96 (s, 2H), 3.71 - 3.54 (m, 3H), 3.40 (t, J = 10.8 Hz, 2H), 3.23 (d, J = 10.2 Hz, 1H), 3.16 - 2.97 (m, 5H), 2.92 - 2.80 (m, 2H), 2.40 - 2.22 (m, 3H), 2.06 - 1.95 (m, 1H), 1.89 - 1.69 (m, 6H), 1.38 (s, 3H). |
| **431** | 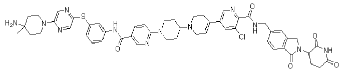 | 995 | |
| **432** | 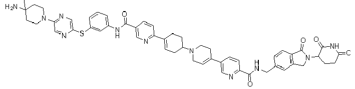 | 958 | ¹H NMR (400 MHz, DMSO-*d*₆) δ 10.99 (s, 1H), 10.87 (s, 1H), 10.50 (s, 1H), 9.50 (t, J = 6.4 Hz, 1H), 9.06 (d, J = 2.3 Hz, 1H), 8.83 (s, 1H), 8.47 - 8.03 (m, 10H), 7.83 - 7.68 (m, 4H), 7.61 - 7.30 (m, 3H), 7.08 - 6.83 (m, 2H), 6.54 (s, 1H), 5.32 (t, J = 4.8 Hz, 1H), 5.10 (dd, J = 13.2, 5.1 Hz, 1H), 4.73 - 4.56 (m, 3H), 4.51 - 4.25 (m, 3H), 4.05 (d, J = 14.5 Hz, 6H), 3.38 (s, 4H), 3.11 - 2.56 (m, 3H), 2.15 - 1.68 (m, 7H), 1.23 |
| | | | (s, 3H). |
| **433** | 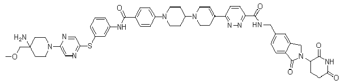 | MS( M/2+ H+) 496.2 | ¹H NMR (400 MHz, DMSO-d6) δ 11.80 (s, 1H), 11.00 (s, 1H), 10.10-10.01 (m, 2H), 8.51 (m, 1H), 8.20-7.75 (m, 6H), 7.70-7.55 (m, 4H), 7.47- 7.12 (m, 4H), 6.99 - 6.95 (m, 1H), 5.11 (dd, J = 13.3, 5.1 Hz, 1H), 4.59 (d, J = 5.8 Hz, 2H), 4.49 - 4.27 (m, 2H), 4.08 - 3.94 (m, 4H), 3.77 (s, 1H),3.40 (s,3H), 3.46 - 3.21 (m, 6H), 3.07 - 2.97 (m, 1H), 2.91 - 2.69 (m, 4H), 2.64 - 2.55 (m, 1H), 2.44 - 2.20 (m, 6H), 2.06 - 1.93 (m, 3H), 1.87 - 1.69 (m, 4H) |
| **434** | 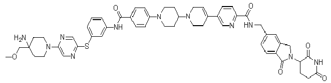 | 990 | |
| **435** | 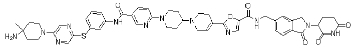 | 951 | |
| 436 | 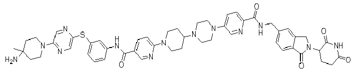 | MS( M/2+ H+) 482.7 | ¹H NMR (400 MHz, DMSO-d6) δ 11.83 (s, 1H), 10.99 (s, 1H), 10.51 (s, 1H), 9.33 (s, 1H), 8.72 (d, J = 2.4 Hz, 1H), 8.46 - 8.25 (m, 6H), 8.20 (d, J = 1.3 Hz, 1H), 8.00 (d, J = 8.4 Hz, 1H), 7.77 - 7.55 (m, 4H), 7.52 (s, 1H), 7.49 - 7.40 (m, 1H), 7.33 (dt, J = 16.3, 8.9 Hz, 2H), 6.99 (dt, J = 7.7, 1.5 Hz, 1H), 5.10 (dd, J = 13.3, 5.1 Hz, 1H), 4.68 (d, J = 13.1 Hz, 2H), 4.59 (d, J = 6.2 Hz, 2H), 4.49 - 4.22 (m, 2H), 4.19 - 3.95 (m, 4H), 3.60 (d, J = 11.8 Hz, 3H), 3.44 (p, J = 11.5, 11.0 Hz, 4H), 3.19 (t, J = 14.9 Hz, 4H), 2.90 (ddd, J = 17.3, 13.7, 5.3 Hz, 1H), 2.59 (d, J = 16.9 Hz, 1H), 2.37 (ddd, J = 28.1, 13.9, 5.6 Hz, 3H), 2.06 - 1.93 (m, 1H), 1.93 - 1.66 (m, 6H), 1.38 (s, 3H). |
| **437** | 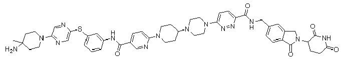 | MS( M/2+ H⁺) 483.2 | ¹H NMR (400 MHz, DMSO-d6) δ 11.77 (s, 1H), 10.99 (s, 1H), 10.38 (s, 1H), 9.61 (t, J = 6.5 Hz, 1H), 8.72 (d, J = 2.4 Hz, 1H), 8.43 - 8.24 (m, 5H), 8.20 (d, J = 1.3 Hz, 1H), 7.95 (d, J = 9.5 Hz, 1H), 7.79 - 7.62 (m, 3H), 7.58 - 7.43 (m, 3H), 7.30 (t, J = 8.0 Hz, 2H), 6.99 (d, J = 7.7 Hz, 1H), 5.10 (dd, J = 13.3, 5.0 Hz, 1H), 4.71 - 4.57 (m, 2H), 4.45 - 4.27 (m, 2H), 4.12 - 3.98 (m, 2H), 3.62 (s, 6H), 3.41 (t, J = 10.0 Hz, 2H), 3.30 - 2.99 (m, 5H), 2.91 (ddd, J = 17.1, 13.7, 5.4 Hz, 1H), 2.69 - 2.55 (m, 1H), 2.45 - 2.18 (m, 4H), 2.05 - |
| | | | 1.92 (m, 1H), 1.76 (dd, J = 30.2, 11.7 Hz, 7H), 1.38 (s, 3H). |
| **438** | 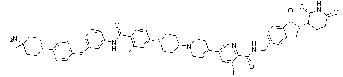 | 992 | ¹H NMR (400 MHz, DMSO-d6) δ 11.00 (s, 1H), 10.90 (s, 1H), 10.13 (s, 1H), 9.39 (d, J = 6.4 Hz, 1H), 8.68 (s, 1H), 8.39 (d, J = 1.5 Hz, 1H), 8.19 (d, J = 1.3 Hz, 2H), 8.05 (d, J = 12.1 Hz, 1H), 7.72 (dd, J = 4.7, 2.7 Hz, 1H), 7.67 (d, J = 15.5 Hz, 1H), 7.62 (d, J= 8.5 Hz, 1H), 7.55 (s, 1H), 7.48 (d, J = 8.3 Hz, 1H), 7.38 (d, J = 8.3 Hz, 1H), 7.27 (t, J = 8.0 Hz, 1H), 6.91 (q, J = 10.6, 9.3 Hz, 3H), 6.61 (s, 1H), 5.11 (dd, J = 13.2, 5.0 Hz, 1H), 4.58 (d, J = 6.3 Hz, 2H), 4.45 (d, J = 17.4 Hz, 2H), 4.30 (d, J = 17.4 Hz, 1H), 4.06 (s, 3H), 3.78 (s, 2H), 3.49 (s, 1H), 3.44 - 3.34 (m, 2H), 3.20 (d, J = 26.6 Hz, 2H), 2.87 (dd, J = 38.2, 18.7 Hz, 5H), 2.59 (d, J = 16.8 Hz, 1H), 2.37 (s, 3H), 2.21 (d, J = 33.1 Hz, 3H), 2.04 - 1.94 (m, 2H), 1.76 (d, J = 14.8 Hz, 6H), 1.36 (s, 3H). |
| **439** | 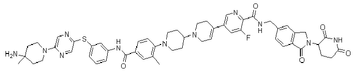 | 496.8 (M/2+ 1) | ¹H NMR (400 MHz, DMSO-d6) δ 11.33 (s, 1H), 11.00 (s, 1H), 10.18 (s, 1H), 9.45 - 9.35 (m, 1H), 8.68 (s, 1H), 8.48 - 8.28 (m, 4H), 8.20 (d, J = 1.3 Hz, 1H), 8.05 (dd, J = 12.2, 1.7 Hz, 1H), 7.85 - 7.74 (m, 3H), 7.70 (dd, J = 7.9, 2.5 Hz, 2H), 7.62 - 7.45 (m, 2H), 7.29 (t, J = 8.0 Hz, 1H), 7.11 (d, J = 8.4 Hz, 1H), 7.03 - 6.94 (m, 1H), 6.64 (s, 1H), 5.11 (dd, J= 13.3, 5.1 Hz, 1H), 4.59 (d, J = 6.3 Hz, 2H), 4.51 - 3.92 (m, 9H), 3.79 (s, 1H), 3.53 - 3.18 (m, 4H), 3.10 - 2.82 (m, 2H), 2.73 (t, J = 11.7 Hz, 2H), 2.64 - 2.56 (m, 1H), 2.44 - 2.21 (m, 5H), 2.08 - 1.92 (m, 4H), 1.87 - 1.67 (m, 4H), 1.37 (s, 3H). |
| **440** | 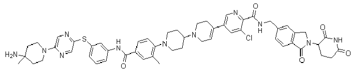 | 504.8 (M/2+ 1) | ¹H NMR (400 MHz, DMSO-d6) δ 11.30 (s, 1H), 11.00 (s, 1H), 10.17 (s, 1H), 9.33 (t, J = 7.3, 6.8 Hz, 1H), 8.75 (d, J = 1.9 Hz, 1H), 8.46 - 8.29 (m, 4H), 8.23 - 8.15 (m, 2H), 7.86 - 7.74 (m, 3H), 7.75 - 7.67 (m, 2H), 7.61 - 7.45 (m, 2H), 7.29 (t, J = 8.0 Hz, 1H), 7.12 (d, J= 8.4 Hz, 1H), 7.01 - 6.93 (m, 1H), 6.59 (s, 1H), 5.11 (dd, J = 13.3, 5.1 Hz, 1H), 4.59 (d, J = 6.2 Hz, 2H), 4.50 - 4.29 (m, 5H), 4.10 - 3.94 (m, 4H), 3.84 - 3.74 (m, 1H), 3.55 - 3.20 (m, 5H), |
| | | | 2.94 - 2.88 (m, 1H), 2.73 (t, J = 11.6 Hz, 2H), 2.64 - 2.55 (m, 1H), 2.44 - 2.20 (m, 5H), 2.09 - 1.68 (m, 8H), 1.37 (s, 3H). |
| **441** | 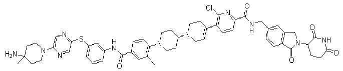 | 1008 | ¹H NMR (400 MHz, DMSO-*d*₆) δ 11.48 (s, 1H), 10.99 (s, 1H), 10.19 (s, 1H), 9.43 (t, J = 6.3 Hz, 1H), 8.51-8.31 (m, 4H), 8.26-7.90 (m, 3H), 7.85-7.67 (m, 5H), 7.59-7.41 (m, 2H), 7.29 (t, J = 8.0 Hz, 1H), 7.20-6.91 (m, 2H), 5.96 (s, 1H), 5.10 (dd, J = 13.3, 5.1 Hz, 1H), 4.61 (d, J = 6.3 Hz, 2H), 4.48-4.26 (m, 2H), 4.09-3.91 (m, 5H), 3.75 (s, 1H), 3.58-3.17 (m, 5H), 3.13 -2.82 (m, 2H), 2.80-2.55 (m, 4H), 2.44-2.21 (m, 6H), 2.07-1.69 (m, 7H), 1.38 (s, 3H). |
| **442** | 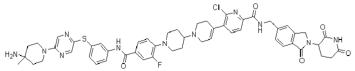 | 1012 | ¹H NMR (400 MHz, DMSO-*d*₆) δ 11.48 (s, 1H), 10.99 (s, 1H), 10.24 (s, 1H), 9.42 (t, J = 6.3 Hz, 1H), 8.54-8.29 (m, 4H), 8.27-7.85 (m, 3H), 7.84-7.64 (m, 5H), 7.61-7.40 (m, 2H), 7.23 (dt, J = 52.9, 8.3 Hz, 2H), 7.04-6.95 (m, 1H), 5.96 (s, 1H), 5.10 (dd, J = 13.3, 5.1 Hz, 1H), 4.61 (d, J = 6.3 Hz, 2H), 4.52-4.23 (m, 2H), 4.03-3.92 (m, 4H), 3.87-3.63 (m, 3H), 3.59-3.36 (m, 3H), 3.29-2.76 (m, 5H), 2.61 (t, J = 8.4 Hz, 2H), 2.45-2.20 (m, 3H), 2.13-1.68 (m, 7H), 1.38 (s, 3H). |
| **443** | 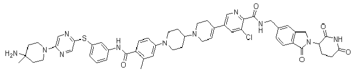 | 504.8 (M/2+ 1) | ¹H NMR (400 MHz, DMSO-d6) δ 11.41 (s, 1H), 11.00 (s, 1H), 10.20 (s, 1H), 9.34 (s, 1H), 8.74 (d, J = 1.9 Hz, 1H), 8.51 - 8.31 (m, 4H), 8.17 (dd, J = 11.1, 1.6 Hz, 2H), 7.74 - 7.61 (m, 3H), 7.57 (s, 1H), 7.50 (d, J = 7.9 Hz, 1H), 7.42 (d, J = 8.4 Hz, 1H), 7.27 (t, J = 8.0 Hz, 1H), 7.05 (s, 2H), 6.95 (s, 0H), 6.57 (s, 1H), 5.11 (dd, J = 13.3, 5.1 Hz, 1H), 4.59 (d, J = 6.2 Hz, 2H), 4.46 (d, J = 17.5 Hz, 1H), 4.31 (d, J = 17.4 Hz, 1H), 4.10 - 3.90 (m, 4H), 3.84 - 3.48 (m, 2H), 3.46 - 3.34 (m, 2H), 3.29 - 3.15 (m, 1H), 3.06 - 2.80 (m, 2H), 2.61 (d, J = 3.8 Hz, 0H), 2.46 - 2.20 (m, 5H), 1.99 (dt, J = 12.3, 4.8 Hz, 2H), 1.86 - 1.68 (m, 4H), 1.37 (s, 3H), 1.22 (d, J = 3.9 Hz, 5H). |
| **444** | 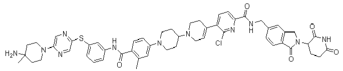 | 504.8 (M/2+ 1) | ¹H NMR (400 MHz, DMSO-d6) δ 11.41 (s, 1H), 10.99 (s, 1H), 10.18 (s, 1H), 9.42 (t, J = 6.4 Hz, 1H), 8.41 - |
| | | | 8.31 (m, 4H), 8.19 (d, J = 1.4 Hz, 1 H), 8.07 (d, J = 7.8 Hz, 1H), 7.92 (d, J = 7.7 Hz, 1H), 7.73 - 7.66 (m, 2H), 7.63 (dd, J = 7.7, 2.1 Hz, 1H), 7.54 (s, 1H), 7.43 (dd, J = 22.5, 8.1 Hz, 2H), 7.27 (t, J = 7.9 Hz, 1H), 6.99 (d, J = 12.1 Hz, 2H), 6.95 - 6.91 (m, 1H), 5.95 (s, 1H), 5.10 (dd, J = 13.3, 5.1 Hz, 1H), 4.60 (d, J = 6.3 Hz, 2H), 4.43 (d, J = 17.5 Hz, 1H), 4.32 (s, 1H), 4.07 - 3.97 (m, 4H), 3.71 (s, 1H), 3.54 (s, 2H), 3.45 - 3.35 (m, 3H), 3.28 - 3.18 (m, 1H), 3.05 (d, J = 14.6 Hz, 1H), 2.90 (dq, J = 13.4, 7.4, 5.3 Hz, 3H), 2.59 (d, J = 17.3 Hz, 2H), 2.43 - 2.31 (m, 4H), 2.02 - 1.94 (m, 2H), 1.81 (ddd, J = 13.1, 9.1, 4.0 Hz, 2H), 1.72 (d, J = 14.1 Hz, 2H), 1.37 (s, 3H), 1.22 (d, J = 2.3 Hz, 3H). |
| **445** | 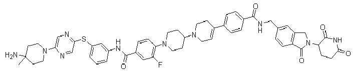 | 977 | |
| **446** | 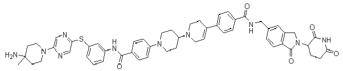 | 959 | |
| **447** | 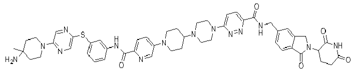 | MS( M/2+ H⁺) 483.5 | ¹H NMR (400 MHz, DMSO-d6) δ 11.81 (brs, 1H), 10.99 (s, 1H), 10.50 (s, 1H), 9.70-9.68 (m, 1H), 8.41 - 8.29 (m, 4H), 8.18 (s, 1H), 8.10 - 7.90 (m, 3H), 7.80-7.70 (m, 2H), 7.60-7.47 (m, 4H), 7.29-7.27 (m, 1H), 7.00 - 6.95 (m, 1H), 5.12 (dd, J = 13.3, 5.1 Hz, 1H), 4.62 (d, J = 6.4 Hz, 2H), 4.26 - 4.11 (m, 3H), 3.96 - 3.85 (m, 3H), 3.77 - 3.61 (m, 4H),3.28 - 3.17 (m, 1H), 3.12 - 2.98 (m, 4H), 2.96 - 2.78 (m, 3H), 2.66 - 2.54 (m, 4H), 2.44 - 2.23 (m, 3H), 2.06 - 1.91 (m, 3H), 1.86 - 1.75 (m, 2H), 1.70 - 1.56 (m, 2H), 1.38 (s, 3H). |
| **448** | 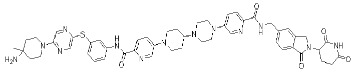 | MS( M/2+ H⁺) 483.5 | ¹H NMR (400 MHz, DMSO-d6) δ 11.80 (brs, 1H), 11.00 (s, 1H), 10.50 (s, 1H), 9.40-9.38 (m, 1H), 8.50-8.40 (m, 5H), 8.23 (s, 1H), 8.15-8.01 (m, 2H), 7.91 (s, 1H), 7.85 - 7.61 (m, 4H), 7.51 (s, 1H), 7.48-7.46 (m, 1H), 7.31-7.27 (m, 1H), 7.02 - 6.93 (m, 1H), 6.49 (s, 1H), 5.12 (dd, J = 13.3, 5.1 Hz, 1H), 4.58 (d, J = 6.3 Hz, 2H), 4.49 - 4.10 (m, 5H), 4.03 - 3.90 (m, 3H), 3.82 - 3.73 (m, 1H), 3.72 - 3.61 (m, 3H), 3.54 - 3.42 (m, 1H), 3.35 (t, J = 5.6 Hz, 1H), 3.29 - 3.17 (m, 1H), 3.13 - 2.97 (m, |
| | | | 3H), 2.97 - 2.77 (m, 4H), 2.64 - 2.54 (m, 4H), 2.45 - 2.21 (m, 3H), 2.05 - 1.90 (m, 3H), 1.87 - 1.74 (m, 2H), 1.71 - 1.55 (m, 2H), 1.38 (s, 3H). |
| **449** | 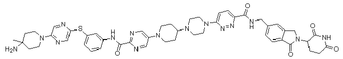 | 966.3 | ¹H NMR (400 MHz, DMSO-*d₆*): δ 11.71-11.70 (m, 1 H), 10.97 (s, 1 H), 10.52 (s,1 H), 9.59 (t, *J* = 6.2 Hz, 1 H), 8.65(s, 2H), 8.40 (s,1 H), 8.29 (s,3 H), 8.21 (d, *J* = 0.8 Hz, 1 H), 7.95 (d, *J* = 9.6 Hz, 1 H),7.87 (s, 1 H), 7.72-7.67 (m, 2 H), 7.54-7.46 (m, 3 H), 7.30 (t, *J* = 8 Hz, 1 H), 6.99 (d, *J* = 7.6 Hz, 1 H), 5.12-5.07 (m, 1 H), 4.65-4.60 (m, 4 H), 4.45-4.23 (m, 5 H), 3.63-3.50 (m, 6 H), 3.43-3.38 (m,2 H), 3.20-3.18(m,2H), 2.98-2.87 (m, 3 H), 2.57-2.56 (m,1 H), 2.43-2.26 (m, 4 H), 2.00-1.97 (m,1 H) 1.86-1.72 (m, 7 H),1.39 (s, 3 H). |
| **450** | 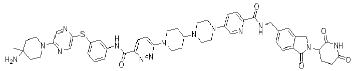 | 965.3 | ¹H NMR (400 MHz, DMSO-*d₆*): δ 11.70-11.67 (m, 1 H), 10.97 (s, 1 H), 10.77 (s,1 H), 9.27 (t, *J* = 6.2 Hz, 1 H), 8.39-8.32(m, 5H), 8.21 (d, *J* = 1.2 Hz, 1 H), 7.98-7.95 (m, 2 H), 7.91-7.89 (m, 1 H), 7.80 (d, *J* = 8 Hz, 1 H), 7.67 (d, *J* = 7.6 Hz, 1 H), 7.60-7.51(m, 3 H), 7.45 (d, *J* = 8Hz, 1 H), 7.30 (t, *J* = 8.2Hz, 1 H), 6.99 (d, *J* = 8Hz, 1 H), 5.12-5.07 (m, 2 H), 4.72 (d, *J* = 13.2Hz, 2 H), 4.59 (d, *J* = 6Hz, 2 H), 4.45-4.27 (m, 2 H), 4.12-4.03 (m, 4H), 3.62-3.59 (m, 3 H), 3.47-3.39 (m, 4 H), 3.25-3.17 (m, 2H), 3.10-3.04(m,2 H), 2.95-2.86 (m,1 H), 2.61-2.57 (m, 1 H), 2.42-2.30 (m,4 H), 2.00-1.96(m, 1H), 1.84-1.72 (m,6 H), 1.38 (s, 3 H). |
| **451** | 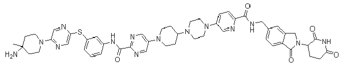 | 965.5 | ¹H NMR (400 MHz, DMSO-*d₆*): δ 11.29-11.27 (m, 1 H), 10.97 (s, 1 H), 10.52 (s,1 H), 9.19 (t, *J* = 6.6 Hz, 1H), 8.65(s, 2H), 8.40-8.38 (m,2 H), 8.22-8.17 (m,3 H), 7.94-7.89 (m, 2 H), 7.71-7.66 (m, 2 H), 7.55-7.52 (m, 2 H), 7.45 (d, *J* = 8.4 Hz, 1 H), 7.30 (t, *J* = 7.8 Hz, 1 H), 7.17 (s, 1 H), 7.04-6.98(m, 1H), 5.12-5.07 (m, 1 H), 4.58 (d, *J* = 6 Hz, 2 H ), 4.45-4.23 (m, 4 H), 4.12-4.03 (m, 4H), 3.41-3.35 (m, 6H), 3.27-3.18 (m,2 H), 2.98-2.86 (m, 3 H), 2.67-2.57 (m,2 H), 2.39-2.24 (m, 3 H), 2.00-1.97 (m,1 H), 1.85-1.74 (m, 6 H),1.38 (s, 3 H). |
| **452** | 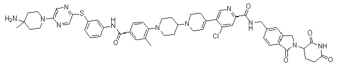 | 504.8 (M/2+ 1) | ¹H NMR (400 MHz, DMSO-d6) δ 11.56 (s, 1H), 11.00 (s, 1H), 10.21 (s, 1H), 9.63 (t, J = 6.4 Hz, 1H), 8.53 (s, 1H), 8.49 - 8.35 (m, 4H), 8.19 (d, J = 1.3 Hz, 1H), 8.08 (s, 1H), 7.85 - 7.64 (m, 5H), 7.53 (s, 1H), 7.46 (d, J = 7.9 Hz, 1H), 7.29 (t, J = 8.0 Hz, 1H), 7.13 (d, J = 8.3 Hz, 1H), 7.00 - 6.93 (m, 1H), 6.02 (s, 1H), 5.10 (dd, J = 13.3, 5.1 Hz, 1H), 4.61 (d, J = 6.4 Hz, 2H), 4.46 - 4.26 (m, 2H), 4.09 - 3.94 (m, 4H), 3.75 (s, 1H), 3.54 - 3.19 (m, 6H), 3.10 (t, J = 12.9 Hz, 1H), 2.90 (ddd, J = 17.1, 13.5, 5.4 Hz, 1H), 2.76 (t, J = 11.6 Hz, 2H), 2.61 (t, J = 17.1 Hz, 2H), 2.44 - 2.21 (m, 6H), 2.10 - 1.94 (m, 3H), 1.89 - 1.68 (m, 4H), 1.38 (s, 3H). |
| **453** | 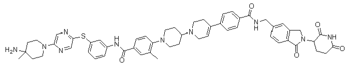 | 487.3 (M/2+ 1) | ¹H NMR (400 MHz, DMSO-d6) δ 11.19 (s, 1H), 11.00 (s, 1H), 10.19 (s, 1H), 9.29 (t, J = 5.9 Hz, 1H), 8.41 - 8.34 (m, 4H), 8.20 (d, J = 1.3 Hz, 1H), 7.96 (d, J = 8.1 Hz, 2H), 7.81 - 7.75 (m, 3H), 7.70 (dd, J = 8.6, 5.7 Hz, 2H), 7.63 (d, J = 8.2 Hz, 2H), 7.55 (s, 1H), 7.47 (d, J = 7.9 Hz, 1H), 7.29 (t, J = 8.0 Hz, 1H), 7.12 (d, J = 8.3 Hz, 1H), 6.99 - 6.95 (m, 1H), 6.38 (s, 1H), 5.11 (dd, J = 13.3, 5.1 Hz, 1H), 4.59 (d, J = 5.8 Hz, 2H), 4.49 - 4.27 (m, 2H), 4.08 - 3.94 (m, 4H), 3.77 (s, 1H), 3.46 - 3.21 (m, 6H), 3.07 - 2.97 (m, 1H), 2.91 (ddd, J = 18.1, 13.6, 5.3 Hz, 1H), 2.85 - 2.69 (m, 3H), 2.64 - 2.55 (m, 1H), 2.44 - 2.20 (m, 6H), 2.06 - 1.93 (m, 3H), 1.87 - 1.69 (m, 4H), 1.37 (s, 3H). |
| **454** | 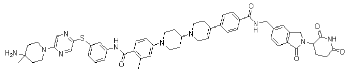 | MS( M/2+ H+) 487.2 | ¹H NMR (400 MHz, DMSO-d6) δ 11.02 (s, 1H), 10.21 (s, 1H), 9.43 ((t, J = 6.4 Hz, 1H), 8.73 (s, 1H), 8.4 (s, 1H),8.49 - 8.35 (m, 3H), 8.19- 8.08 (m, 2H), 7.85 - 7.64 (m, 5H), 7.53 (s, 1H), 7.29 (t, J = 8.0 Hz, 1H), 7.13 (d, J = 8.3 Hz, 1H), 7.00 - 6.93 (m, 1H), 6.6 (s, 1H), 5.12 (dd, J = 13.3, 5.1 Hz, 1H), 4.62 (d, J = 6.4 Hz, 2H), 4.26 - 4.11 (m, 3H), 3.96 - 3.85 (m, 3H), 3.77 - 3.61 (m, 4H),3.28 - 3.17 (m, 1H), 3.12 - 2.98 (m, 4H), 2.96 - 2.78 (m, 3H), 2.66 - 2.54 (m, 4H), 2.44 - 2.23 (m, 3H), 2.06 - 1.91 (m, 3H), 1.86 - 1.75 (m, 2H), 1.70 - 1.56 (m, 2H), 1.38 (s, 3H). |
| **455** | 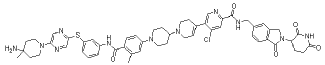 | MS( M/2+ H+) 501.2 | ¹H NMR (400 MHz, DMSO-d6) δ 11.02 (s, 1H), 10.21 (s, 1H), 9.43 ((t, J = 6.4 Hz, 1H), 8.73 (s, 1H), 8.4 (s, 1H),8.49 - 8.35 (m, 3H), 8.19- 8.08 (m, 2H), 7.85 - 7.64 (m, 5H), 7.53 (s, 1H), 7.29 (t, J = 8.0 Hz, 1H), 7.13 (d, J = 8.3 Hz, 1H), 7.00 - 6.93 (m, 1H), 6.6 (s, 1H), 5.12 (dd, J = 13.3, 5.1 Hz, 1H), 4.61 (d, J = 6.4 Hz, 2H), 4.46 - 4.26 (m, 2H), 4.09 - 3.94 (m, 4H), 3.54 - 3.19 (m, 6H), 3.10 (t, J = 12.9 Hz, 1H), 2.76 (t, J = 11.6 Hz, 2H), 2.50 (s,3H) , 2.44 - 2.21 (m, 6H), 2.10 - 1.94 (m, 4H), 1.89 - 1.68 (m, 4H), 1.38 (s, 3H). |
| **456** | 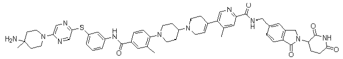 | 988 | |
| **457** | 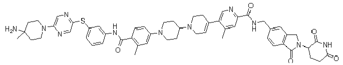 | 988 | |
| 458 | 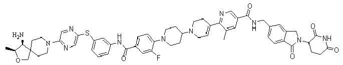 | 524.9 (M/2+ 1) | ¹H NMR (400 MHz, DMSO-d6) δ 11.27 (s, 1H), 11.00 (s, 1H), 10.21 (s, 1H), 9.49 (d, J = 5.6 Hz, 1H), 8.94 (d, J = 2.0 Hz, 1H), 8.40 (d, J = 1.5 Hz, 1H), 8.28 (s, 1H), 8.23 - 8.11 (m, 3H), 7.76 (dd, J = 4.2, 2.3 Hz, 2H), 7.70 (dd, J = 8.1, 3.6 Hz, 2H), 7.56 (s, 1H), 7.48 (d, J = 8.0 Hz, 1H), 7.30 (t, J = 8.0 Hz, 1H), 7.18 (t, J = 8.6 Hz, 1H), 7.01 - 6.95 (m, 1H), 6.04 (s, 1H), 5.11 (dd, J = 13.3, 5.1 Hz, 1H), 4.62 (d, J = 5.8 Hz, 2H), 4.44 (d, J = 17.4 Hz, 1H), 4.33 (s, 1H), 4.26 - 4.10 (m, 2H), 3.98 (s, 2H), 3.91 (d, J = 9.0 Hz, 1H), 3.78 (s, 1H), 3.67 (t, J = 10.8 Hz, 3H), 3.51 (s, 2H), 3.36 (d, J = 5.7 Hz, 1H), 3.24 (d, J = 10.8 Hz, 1H), 3.16 - 3.03 (m, 2H), 2.98 - 2.90 (m, 1H), 2.83 (q, J = 16.1, 14.4 Hz, 3H), 2.59 (d, J = 17.0 Hz, 1H), 2.47 (s, 3H), 2.41 - 2.30 (m, 2H), 2.26 (d, J = 11.5 Hz, 1H), 2.04 - 1.88 (m, 3H), 1.75 (d, J = 11.4 Hz, 2H), 1.68 (d, J = 13.1 Hz, 1H), 1.58 (d, J = 12.9 Hz, 1H), 1.29 - 1.19 (m, 6H). |
| **459** | 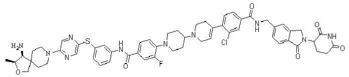 | 534.3 (M/2+ 1) | ¹H NMR (400 MHz, DMSO-d6) δ 11.48 (s, 1H), 10.99 (s, 1H), 10.25 (s, 1H), 9.44 (t, J = 5.9 Hz, 1H), 8.39 (d, J = 1.4 Hz, 1 H), 8.30 (s, 2H), 8.19 (d, J = 1.3 Hz, 1H), 8.04 (d, J = 1.6 Hz, 1H), 7.92 (dd, J = 8.0, 1.7 Hz, 1H), 7.86 - 7.64 (m, 5H), 7.54 (s, 1H), 7.44 (dd, J = 24.7, 7.9 Hz, 2H), 7.29 (t, J = 7.9 Hz, |
| | | | 1H), 7.17 (t, J = 8.7 Hz, 1H), 7.03 - 6.93 (m, 1H), 5.84 (s, 1H), 5.14 - 5.04 (m, 1H), 4.59 (d, J = 5.7 Hz, 2H), 4.48 - 4.27 (m, 2H), 4.19 (dt, J = 21.4, 14.3 Hz, 2H), 3.92 (d, J = 7.9 Hz, 3H), 3.66 (t, J = 11.9 Hz, 4H), 3.35 (t, J = 5.6 Hz, 1H), 3.20 (d, J = 30.0 Hz, 1H), 3.13 - 3.00 (m, 2H), 2.87 (dt, J = 29.4, 12.7 Hz, 2H), 2.58 (d, J = 17.4 Hz, 2H), 2.42 - 2.20 (m, 3H), 1.99 (d, J = 12.7 Hz, 3H), 1.80 (t, J = 12.2 Hz, 1H), 1.59 (s, 3H), 1.24 (d, J = 6.8 Hz, 4H), 1.10 (d, J = 2.4 Hz, 1H). |
| **460** | 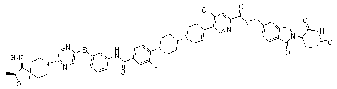 | 534.8 (M/2+ 1) | ¹H NMR (400 MHz, DMSO-d6) δ 11.43 (s, 1H), 10.99 (s, 1H), 10.23 (s, 1H), 9.63 (t, J = 6.3 Hz, 1H), 8.53 (s, 1H), 8.40 (d, J = 1.5 Hz, 1H), 8.24 (s, 3H), 8.19 (d, J = 1.3 Hz, 1H), 8.08 (s, 1H), 7.83 - 7.74 (m, 3H), 7.69 (dd, J = 10.3, 7.8 Hz, 2H), 7.56 - 7.43 (m, 2H), 7.30 (t, J = 8.0 Hz, 1H), 7.18 (t, J = 8.7 Hz, 1H), 7.02 - 6.94 (m, 1H), 6.03 (s, 1H), 5.10 (dd, J = 13.3, 5.1 Hz, 1H), 4.61 (d, J = 6.3 Hz, 2H), 4.33 - 4.10 (m, 4H), 3.97 (s, 2H), 3.92 (d, J = 9.0 Hz, 1H), 3.74 (d, J = 8.6 Hz, 1H), 3.67 (t, J = 11.3 Hz, 3H), 3.50 (s, 1H), 3.41 - 3.18 (m, 2H), 3.08 (d, J = 6.1 Hz, 1H), 2.98 - 2.77 (m, 4H), 2.61 (dd, J = 20.2, 15.8 Hz, 2H), 2.44 - 2.22 (m, 4H), 2.07 - 1.88 (m, 4H), 1.84 - 1.55 (m, 4H), 1.23 (d, J = 6.3 Hz, 3H). |
| **461** | 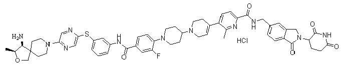 | 524.8 (M/2+ 1) | ¹H NMR (400 MHz, DMSO-d6) δ 11.81 (s, 1H), 10.99 (s, 1H), 10.27 (s, 1H), 9.49 - 9.42 (m, 1H), 8.41 - 8.29 (m, 4H), 8.18 (d, J = 1.4 Hz, 1H), 7.94 (d, J = 7.9 Hz, 1H), 7.83 - 7.66 (m, 6H), 7.54 (s, 1H), 7.47 (d, J = 7.6 Hz, 1H), 7.29 (t, J = 8.0 Hz, 1H), 7.17 (t, J = 8.7 Hz, 1H), 7.00 - 6.95 (m, 1H), 5.79 (s, 1H), 5.10 (dd, J = 13.3, 5.1 Hz, 1H), 4.62 (d, J = 6.4 Hz, 2H), 4.47 - 4.26 (m, 2H), 4.26 - 4.11 (m, 3H), 3.96 - 3.85 (m, 3H), 3.77 - 3.61 (m, 4H), 3.49 (s, 1H), 3.34 (t, J = 5.6 Hz, 1H), 3.28 - 3.17 (m, 1H), 3.12 - 2.98 (m, 4H), 2.96 - 2.78 (m, 3H), 2.66 - 2.54 (m, 4H), 2.44 - 2.23 (m, 3H), 2.06 - 1.91 (m, 3H), 1.86 - 1.75 (m, 2H), 1.70 - 1.56 (m, 2H), 1.24 (d, J = 6.5 Hz, 3H). |
| **462** | 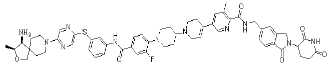 | 524.8 (M/2+ 1) | ¹H NMR (400 MHz, DMSO-d6) δ 11.50 (s, 1H), 11.00 (s, 1H), 10.26 (s, 1H), 9.36 (t, J = 6.3 Hz, 1H), 8.63 (d, J = 2.0 Hz, 1H), 8.43 - 8.25 (m, 4H), 8.19 (d, J = 1.3 Hz, 1H), 7.91 (d, J = 2.2 Hz, 1H), 7.85 - 7.66 (m, 5H), 7.55 (s, 1H), 7.48 (d, J = 7.8 Hz, 1H), 7.29 (t, J = 8.0 Hz, 1H), 7.16 (t, J = 8.6 Hz, 1H), 7.02 - 6.93 (m, 1H), 6.49 (s, 1H), 5.10 (dd, J = 13.3, 5.1 Hz, 1H), 4.58 (d, J = 6.3 Hz, 2H), 4.49 - 4.10 (m, 5H), 4.03 - 3.90 (m, 3H), 3.82 - 3.73 (m, 1H), 3.72 - 3.61 (m, 3H), 3.54 - 3.42 (m, 1H), 3.35 (t, J = 5.6 Hz, 1H), 3.29 - 3.17 (m, 1H), 3.13 - 2.97 (m, 3H), 2.97 - 2.77 (m, 4H), 2.64 - 2.54 (m, 4H), 2.45 - 2.21 (m, 3H), 2.05 - 1.90 (m, 3H), 1.87 - 1.74 (m, 2H), 1.71 - 1.55 (m, 2H), 1.24 (d, J = 6.5 Hz, 3H). |
| **463** | 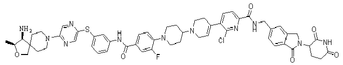 | 1068 | ¹H NMR (400 MHz, DMSO-d6) δ 11.63 (s, 1H), 10.99 (s, 1H), 10.26 (s, 1H), 9.42 (d, J = 6.3 Hz, 1H), 8.35 (d, J = 31.8 Hz, 3H), 8.19 (d, J = 1.2 Hz, 1H), 8.07 (d, J = 7.7 Hz, 1H), 7.92 (d, J = 7.7 Hz, 1H), 7.83 - 7.76 (m, 2H), 7.70 (t, J = 1.7 Hz, 3H), 7.54 (s, 1H), 7.46 (d, J = 8.2 Hz, 1H), 7.29 (t, J = 8.0 Hz, 1H), 7.17 (t, J = 8.6 Hz, 1H), 6.97 (d, J = 7.7 Hz, 1H), 5.95 (s, 1H), 5.10 (dd, J = 13.2, 5.0 Hz, 2H), 4.44 (d, J = 17.2 Hz, 2H), 4.32 (s, 1H), 4.19 (dt, J = 20.4, 14.3 Hz, 3H), 3.94 (s, 2H), 3.92 (s, 1H), 3.64 (d, J = 9.2 Hz, 4H), 3.50 (s, 1H), 3.35 (s, 1H), 3.22 (d, J = 9.6 Hz, 1H), 3.07 (s, 2H), 2.98 - 2.74 (m, 4H), 2.45 - 2.20 (m, 4H), 1.97 (d, J = 13.3 Hz, 4H), 1.85 - 1.75 (m, 2H), 1.67 (d, J = 13.4 Hz, 1H), 1.59 (d, J = 12.8 Hz, 1H), 1.25 (s, 3H). |
| **464** | 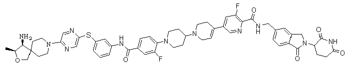 | 1052 | ¹H NMR (400 MHz, DMSO-d6) δ 11.39 (s, 1H), 10.99 (s, 1H), 10.23 (s, 1H), 9.42 - 9.35 (m, 1H), 8.68 (s, 1H), 8.40 (d, J = 1.5 Hz, 1H), 8.28 - 8.18 (m, 3H), 8.04 (dd, J = 12.2, 1.8 Hz, 1H), 7.83 - 7.65 (m, 5H), 7.55 (s, 1H), 7.48 (d, J = 8.0 Hz, 1H), 7.33 - 7.26 (m, 1H), 7.21 - 7.13 (m, 1H), 7.00 - 6.95 (m, 1H), 6.63 (s, 1H), 5.11 (dd, J = 13.3, 5.1 Hz, 1H), 4.45 (d, J = 17.3 Hz, 2H), 4.33 (s, 1H), 4.26 - 4.14 (m, 3H), 4.00 (s, |
| | | | 2H), 3.92 (d, J = 9.1 Hz, 1H), 3.78 (s, 1H), 3.66 (t, J = 10.1 Hz, 3H), 3.49 (s, 1H), 3.36 (d, J = 5.9 Hz, 1H), 3.23 (d, J = 11.4 Hz, 1H), 3.06 (d, J = 14.7 Hz, 2H), 2.93 - 2.77 (m, 4H), 2.41 - 2.21 (m, 4H), 2.06 - 1.89 (m, 4H), 1.78 (dd, J = 13.9, 9.4 Hz, 2H), 1.67 (d, J = 13.4 Hz, 1H), 1.58 (d, J = 12.9 Hz, 1H), 1.24 (s, 3H). |
| **465** | 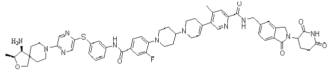 | 1048 | |
| **466** | 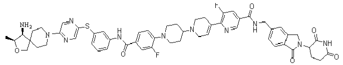 | 1052 | |
| **467** | 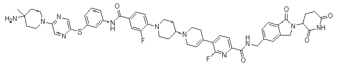 | 498.7 (M/2+ 1) | ¹H NMR (400 MHz, DMSO-d6) δ 11.12 (s, 1H), 11.00 (s, 1H), 10.20 (s, 1H), 9.46 (t, J = 6.4 Hz, 1H), 8.39 (d, J = 1.5 Hz, 1H), 8.27 (s, 3H), 8.22 - 8.15 (m, 2H), 8.02 (dd, J = 7.7, 1.8 Hz, 1H), 7.82 - 7.78 (m, 1H), 7.76 (d, J = 2.5 Hz, 2H), 7.69 (dd, J = 8.2, 4.0 Hz, 2H), 7.54 (s, 1H), 7.46 (d, J = 7.9 Hz, 1H), 7.30 (t, J = 8.0 Hz, 1H), 7.17 (t, J = 8.7 Hz, 1H), 7.02 - 6.96 (m, 1H), 6.34 (s, 1H), 5.10 (dd, J = 13.3, 5.0 Hz, 1H), 4.59 (d, J = 6.3 Hz, 2H), 4.44 (d, J = 17.3 Hz, 2H), 4.29 (d, J = 17.4 Hz, 2H), 3.77 (s, 1H), 3.68 (d, J = 11.2 Hz, 2H), 3.50 (s, 1H), 3.44 - 3.36 (m, 2H), 3.23 (d, J = 10.8 Hz, 1H), 3.06 (s, 1H), 2.91 (s, 1H), 2.83 (t, J = 11.8 Hz, 2H), 2.73 (d, J = 16.6 Hz, 1H), 2.59 (d, J = 16.9 Hz, 2H), 2.40 - 2.20 (m, 4H), 2.01 - 1.95 (m, 3H), 1.79 (s, 1H), 1.77 (d, J = 3.9 Hz, 1H), 1.75 (d, J = 5.5 Hz, 2H), 1.23 (s, 3H). |
| **468** | 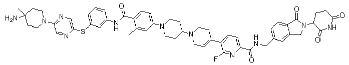 | 992 | ¹H NMR (400 MHz, DMSO-d6) δ 10.99 (s, 1H), 10.93 (s, 1H), 10.13 (s, 1H), 9.45 (t, J = 6.3 Hz, 1H), 8.39 (d, J = 1.5 Hz, 1H), 8.26 - 8.14 (m, 4H), 8.02 (dd, J = 7.7, 1.9 Hz, 1H), 7.74 - 7.66 (m, 2H), 7.62 (d, J = 8.6 Hz, 1H), 7.53 (s, 1H), 7.46 (d, J = 7.9 Hz, 1H), 7.38 (d, J = 8.4 Hz, 1H), 7.27 (t, J = 8.0 Hz, 1H), 6.97 - 6.86 (m, 3H), 6.33 (s, 1H), 5.10 (dd, J = 13.2, 5.1 Hz, 1H), 4.58 (d, J = 6.3 Hz, 2H), 4.43 (d, J = 17.4 Hz, 1H), 4.29 (d, J = 17.4 Hz, 1H), 4.03 (s, 2H), 3.54 (d, J = 21.6 Hz, 2H), 3.44 - 3.34 (m, 2H), 3.25 - 3.15 (m, |
| | | | 1H), 3.03 (s, 1H), 2.93 - 2.85 (m, 1H), 2.81 (t, J = 12.2 Hz, 2H), 2.73 (d, J = 5.2 Hz, 1H), 2.70 (s, 1H), 2.61 (s, 1H), 2.57 (s, 1H), 2.37 (s, 4H), 2.22 (d, J = 31.6 Hz, 3H), 1.99 (dt, J = 12.3, 6.6 Hz, 3H), 1.77 (q, J = 9.6, 5.3 Hz, 6H), 1.37 (s, 3H). |
| **469** | 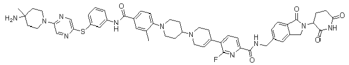 | 496.6 (M/2+ 1) | ¹H NMR (400 MHz, DMSO-d6) δ 11.33 (s, 1H), 10.99 (s, 1H), 10.17 (s, 1H), 9.46 (t, J = 6.4 Hz, 1H), 8.45 - 8.30 (m, 4H), 8.23 - 7.97 (m, 3H), 7.87 - 7.64 (m, 5H), 7.58 - 7.43 (m, 2H), 7.29 (t, J = 8.0 Hz, 1H), 7.12 (d, J = 8.4 Hz, 1H), 7.01 - 6.94 (m, 1H), 6.35 (s, 1H), 5.10 (dd, J = 13.3, 5.0 Hz, 1H), 4.59 (d, J = 6.4 Hz, 2H), 4.46 (s, 1H), 4.41 (s, 1H), 4.29 (d, J = 17.4 Hz, 1H), 4.01 (s, 3H), 3.77 (s, 1H), 3.52 - 3.24 (m, 3H), 3.25 - 3.03 (m, 2H), 2.91 (ddd, J = 17.8, 13.5, 5.4 Hz, 1H), 2.73 (s, 2H), 2.64 - 2.55 (m, 1H), 2.44 - 2.18 (m, 5H), 2.07 - 1.91 (m, 3H), 1.87 - 1.66 (m, 3H), 1.37 (s, 3H), 1.25 (d, J = 18.4 Hz, 3H). |
| **470** | 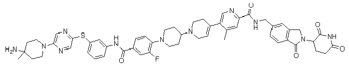 | 992 | |
| **471** | 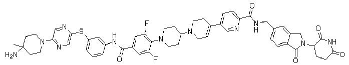 | 996 | |
| **472** | 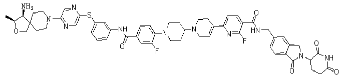 | 526.8 (M/2+ 1) | ¹H NMR (400 MHz, DMSO-d6) δ 11.36 (s, 1H), 11.00 (s, 1H), 10.22 (s, 1H), 9.25 - 9.15 (m, 1H), 8.40 (d, J = 1.5 Hz, 1H), 8.32 - 8.16 (m, 5H), 7.84 - 7.67 (m, 6H), 7.57 (s, 1H), 7.50 (d, J = 7.9 Hz, 1H), 7.30 (t, J = 8.0 Hz, 1H), 7.17 (t, J = 8.7 Hz, 1H), 7.02 - 6.96 (m, 1H), 6.88 (s, 1H), 5.11 (dd, J = 13.3, 5.1 Hz, 1H), 4.60 (d, J = 5.9 Hz, 2H), 4.51 - 4.28 (m, 2H), 4.28 - 4.11 (m, 4H), 4.07 - 3.85 (m, 2H), 3.79 (d, J = 10.8 Hz, 1H), 3.66 (t, J = 9.9 Hz, 3H), 3.49 (s, 1H), 3.37 (q, J = 5.5 Hz, 1H), 3.20 (d, J = 10.0 Hz, 1H), 3.14 - 3.02 (m, 2H), 2.99 - 2.78 (m, 5H), 2.66 - 2.56 (m, 1H), 2.45 - 2.20 (m, 3H), 2.06 - 1.88 (m, 3H), 1.84 - 1.54 (m, 4H), 1.23 (d, J = 6.4 Hz, 3H). |
| **473** | 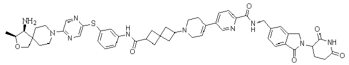 | 476.2 (M/2+ 1) | ¹H NMR (400 MHz, DMSO-d6) δ 11.41 (s, 1H), 10.99 (s, 1H), 9.99 (s, 1H), 9.50 (t, J = 6.3 Hz, 1H), 8.78 (s, |
| | | | 1H), 8.39 (d, J = 1.4 Hz, 1H), 8.24 (s, 3H), 8.17 (d, J = 1.3 Hz, 1H), 8.12 - 8.03 (m, 2H), 7.68 (d, J = 7.8 Hz, 1H), 7.53 (t, J = 7.3 Hz, 3H), 7.47 (d, J = 7.8 Hz, 1H), 7.23 (t, J = 7.9 Hz, 1H), 6.90 (d, J = 7.7 Hz, 1H), 6.45 (s, 1H), 5.10 (dd, J = 13.3, 5.1 Hz, 1H),4.69 - 4.55 (m, 2H), 4.43 (d, J = 17.4 Hz, 2H), 4.29 (d, J = 17.5 Hz, 2H), 4.25 - 4.19 (m, 2H), 4.19 - 4.11 (m, 2H), 3.92 (d, J = 9.0 Hz, 2H), 3.71 (d, J = 7.9 Hz, 1H), 3.65 (d, J = 9.0 Hz, 2H), 3.47 (s, 1H), 3.36 (s, 1H), 3.17 - 3.02 (m, 4H), 2.90 (ddd, J = 18.2, 13.7, 5.5 Hz, 2H), 2.81 (d, J = 17.2 Hz, 1H), 2.59 (d, J = 16.8 Hz, 1H), 2.40 (d, J = 10.0 Hz, 2H), 2.25 (d, J = 7.8 Hz, 3H), 2.12 (t, J = 10.1 Hz, 1H), 2.00 - 1.96 (m, 1H), 1.77 (d, J = 11.5 Hz, 2H), 1.67 (d, J = 13.4 Hz, 1H), 1.61 - 1.54 (m, 1H), 1.23 (d, J = 6.1 Hz, 3H). |
| **474** | 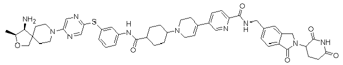 | MS( M/2+ H+) 470.2 | ¹H NMR (400 MHz, DMSO-d6) δ 11.01 (s, 1H), 10.2-10.1(m, 1H), 9.49 (m, 1H), 8.80 (m,1H), 8.41 - 8.21 (m, 5H), 8.15 (s, 1H),8.10- 7.94 (m, 2H), 7.70 (m, 1H), 7.64-7.42 (m, 3H), 7.29-7.17 (m, 1H), 7.00 - 6.95 (m, 1H), 6.57 (s, 1H), 5.12 (dd, J = 13.3, 5.1 Hz, 1H), 4.62 (d, J = 6.4 Hz, 2H), 4.47 - 4.26 (m, 2H), 4.26 - 4.11 (m, 3H), 3.96 - 3.85 (m,2H), 3.77 - 3.61 (m, 4H), 3.49 (s, 1H), 3.34(m, 3H), 3.12 - 2.98 (m, 4H), 2.96 - 2.78 (m, 3H), 2.66 - 2.54 (m, 4H), 2.44 - 2.23 (m, 3H), 1.86 - 1.75 (m, 2H), 1.70 - 1.56 (m, 2H), 1.24 (d, J = 6.5 Hz, 3H). |
| **475** | 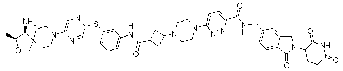 | 915 | |
| **476** | 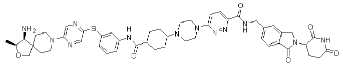 | 943 | |
| **477** | 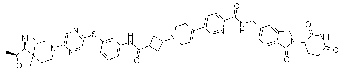 | 911 | ¹H NMR (400 MHz, DMSO-d6) δ 11.00 (s, 1H), 10.94 (s, 1H), 10.26 (s, 1H), 9.50 (t, J = 6.4 Hz, 1H), 8.79 (d, J = 2.2 Hz, 1H), 8.40 (d, J = 1.5 Hz, 1H), 8.28 - 8.17 (m, 3H), 8.15 - 8.03 (m, 2H), 7.68 (d, J = 7.8 Hz, 1H), 7.61 - 7.52 (m, 3H), 7.47 (d, J = 7.9 Hz, 1H), 7.25 (t, J = 8.0 Hz, 1H), 6.92 (d, J = 7.6 Hz, 1H), 6.46 (s, 1H), 5.10 (dd, J = |
| | | | 13.3, 5.1 Hz, 1H), 4.62 (s, 2H), 4.45 (s, 1H), 4.41 (s, 1H), 4.31 (s, 1H), 4.26 (d, J = 5.9 Hz, 1H), 4.24 - 4.12 (m, 3H), 4.02 - 3.90 (m, 2H), 3.88 - 3.77 (m, 1H), 3.68 (dd, J = 28.4, 12.6 Hz, 2H), 3.54 (s, 1H), 3.35 (d, J = 6.0 Hz, 1H), 3.20 - 2.80 (m, 8H), 2.61 (s, 1H), 2.55 (s, 1H), 2.37 (dd, J = 13.2, 4.5 Hz, 1H), 1.98 (dt, J = 11.0, 4.8 Hz, 2H), 1.78 (s, 2H), 1.67 (d, J = 13.2 Hz, 1H), 1.58 (d, J = 12.8 Hz, 1H), 1.23 (s, 3H). |
| **478** | 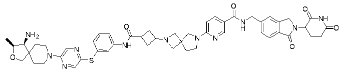 | 970 | ¹H NMR (400 MHz, DMSO-d6) δ 11.58 (s, 1H), 11.00 (s, 1H), 10.19 (s, 1H), 9.41 (s, 1H), 8.61 (s, 1H), 8.39 (d, J = 1.5 Hz, 1H), 8.30 (s, 1H), 8.22 - 8.17 (m, 2H), 7.69 (t, J = 7.6 Hz, 1H), 7.55 (t, J = 10.2 Hz, 3H), 7.47 (d, J = 7.9 Hz, 1H), 7.25 (t, J = 8.0 Hz, 1H), 6.92 (d, J = 8.1 Hz, 1H), 5.11 (dd, J = 13.3, 5.1 Hz, 1H), 4.57 (s, 2H), 4.44 (d, J = 17.4 Hz, 2H), 4.32 (s, 1H), 4.28 (s, 1H), 4.21 (d, J = 5.8 Hz, 2H), 4.15 (d, J = 5.0 Hz, 3H), 4.08 (s, 1H), 4.00 (d, J = 9.4 Hz, 1H), 3.92 (d, J = 8.0 Hz, 2H), 3.82 (s, 1H), 3.66 (d, J = 9.0 Hz, 2H), 3.60 (s, 1H), 3.37 (d, J = 6.4 Hz, 1H), 3.08 (d, J = 26.1 Hz, 2H), 3.02 - 2.94 (m, 1H), 2.93 - 2.86 (m, 1H), 2.61 (s, 1H), 2.57 (s, 1H), 2.40 (t, J = 6.4 Hz, 3H), 2.37 - 2.29 (m, 3H), 2.00 (t, J = 7.4 Hz, 2H), 1.79 (d, J = 10.7 Hz, 2H), 1.68 (d, J = 13.4 Hz, 1H), 1.58 (d, J = 12.9 Hz, 1H), 1.33 (t, J = 6.4 Hz, 1H), 1.23 (d, J = 3.1 Hz, 3H). |
| **479** | 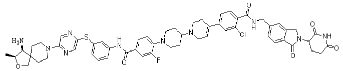 | 1067 | |
| **480** | 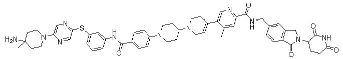 | 974 | |
| **481** | 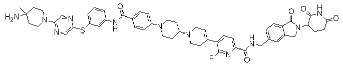 | 489.6 (M/2+ 1) | ¹H NMR (400 MHz, DMSO-d6) δ 11.33 (s, 1H), 10.99 (s, 1H), 10.06 (s, 1H), 9.45 (t, J = 6.4 Hz, 1H), 8.44 - 8.30 (m, 4H), 8.24 - 8.10 (m, 2H), 8.01 (dd, J = 7.7, 1.8 Hz, 1H), 7.88 (d, J = 8.6 Hz, 2H), 7.77 (t, J = 2.0 Hz, 1H), 7.69 (dd, J = 11.3, 8.0 Hz, 2H), 7.53 (s, 1H), 7.46 (d, J = 7.9 Hz, 1H), 7.28 (t, J = 8.0 Hz, 1H), 7.09 (d, J = 8.7 Hz, 2H), 6.95 (dd, J = 8.2, 1.8 Hz, 1H), 6.32 (s, 1H), 5.10 (dd, J = 13.3, 5.1 Hz, 1H), |
| | | | 4.58 (d, J = 6.3 Hz, 2H), 4.29 (d, J = 17.4 Hz, 1H), 4.15 - 4.00 (m, 4H), 3.96 (s, 2H), 3.73 (d, J = 11.0 Hz, 1H), 3.51 (d, J = 16.9 Hz, 1H), 3.41 (ddd, J = 13.2, 9.4, 3.8 Hz, 2H), 3.27 - 3.00 (m, 2H), 2.90 (ddt, J = 20.9, 12.7, 6.7 Hz, 3H), 2.76 - 2.54 (m, 2H), 2.44 - 2.13 (m, 3H), 2.07 - 1.94 (m, 1H), 1.78 (dtd, J = 33.9, 13.9, 11.8, 6.6 Hz, 4H), 1.37 (s, 3H), 1.22 (d, J = 3.6 Hz, 3H). |
| **482** | 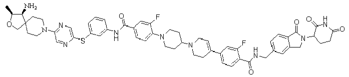 | 526.1 (M/2+ 1) | ¹H NMR (400 MHz, DMSO-d6) δ 11.41 (s, 1H), 11.01 (s, 1H), 10.24 (s, 1H), 9.30 (t, J = 5.9 Hz, 1H), 8.64 (s, 1H), 8.39 (s, 1H), 8.23 (d, J = 32.7 Hz, 3H), 7.75 (ddd, J = 23.1, 13.2, 7.3 Hz, 6H), 7.59 (s, 1H), 7.51 (d, J = 7.8 Hz, 1H), 7.29 (t, J = 8.0 Hz, 1H), 7.17 (t, J = 8.6 Hz, 1H), 6.98 (d, J = 7.6 Hz, 1H), 6.87 (s, 1H), 5.11 (dd, J = 13.3, 5.0 Hz, 1H), 4.59 (d, J = 5.8 Hz, 2H), 4.51 - 4.29 (m, 6H), 4.02 (s, 2H), 3.92 (d, J = 9.0 Hz, 1H), 3.66 (t, J = 10.6 Hz, 3H), 3.49 (s, 1H), 3.36 (q, J = 5.9 Hz, 1H), 3.18 (d, J = 18.7 Hz, 1H), 3.13 - 2.91 (m, 3H), 2.89 - 2.77 (m, 2H), 2.60 (d, J = 17.1 Hz, 1H), 2.46 (s, 3H), 2.42 - 2.20 (m, 2H), 1.99 (ddt, J = 19.9, 13.8, 6.7 Hz, 3H), 1.86 - 1.73 (m, 2H), 1.63 (dd, J = 32.4, 13.0 Hz, 2H), 1.23 (d, J = 6.6 Hz, 5H). |
| 483 | 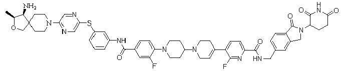 | 987 | ¹H NMR (400 MHz, DMSO-d6) δ 11.92 (s, 1H), 10.99 (s, 1H), 10.29 (s, 1H), 9.51 (t, J = 6.4 Hz, 1H), 8.80 (d, J = 2.1 Hz, 1H), 8.40 (s, 1H), 8.22 (d, J = 19.0 Hz, 3H), 8.14 - 8.05 (m, 2H), 7.93 (d, J = 8.1 Hz, 2H), 7.78 - 7.60 (m, 6H), 7.54 (s, 1H), 7.47 (d, J = 8.1 Hz, 1H), 7.30 (t, J = 8.0 Hz, 1H), 6.98 (d, J = 7.7 Hz, 1H), 6.50 (s, 1H), 5.10 (dd, J = 13.3, 5.1 Hz, 1H), 4.61 (d, J = 6.4 Hz, 2H), 4.46 (s, 1H), 4.41 (s, 1H), 4.31 (s, 1H), 4.21 - 4.16 (m, 3H), 3.98 - 3.90 (m, 2H), 3.86 (s, 1H), 3.76 - 3.69 (m, 1H), 3.64 (d, J = 9.0 Hz, 1H), 3.56 (d, J = 9.8 Hz, 1H), 3.35 (t, J = 5.6 Hz, 1H), 3.16 (s, 1H), 3.06 (d, J = 13.5 Hz, 2H), 2.94 (s, 1H), 2.92 - 2.81 (m, 2H), 2.75 - 2.66 (m, 3H), 2.62 - 2.55 (m, 1H), 2.40 - 2.31 (m, 1H), 2.04 - 1.94 (m, 2H), 1.77 (d, J = 7.1 Hz, 2H), 1.67 (d, J |
| | | | = 13.6 Hz, 1H), 1.62 - 1.54 (m, 1H), 1.24 (s, 3H). |
| **484** | 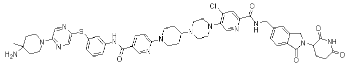 | MS( M/2+ H+) 499.7 | ¹H NMR (400 MHz, DMSO-d6) δ 11.73 (s, 1H), 10.99 (s, 1H), 10.39 (s, 1H), 9.45 (t, J = 6.4 Hz, 1H), 8.72 (d, J = 2.3 Hz, 1H), 8.48 - 8.25 (m, 6H), 8.20 (d, J = 1.3 Hz, 1H), 8.02 (s, 1H), 7.78 - 7.60 (m, 3H), 7.51 (s, 1H), 7.47 - 7.40 (m, 1H), 7.29 (q, J = 8.3 Hz, 2H), 7.03 - 6.94 (m, 1H), 5.09 (dd, J = 13.3, 5.1 Hz, 1H), 4.66 (d, J = 13.1 Hz, 2H), 4.58 (d, J = 6.2 Hz, 2H), 4.47 - 4.20 (m, 2H), 4.19 - 3.95 (m, 4H), 3.60 (d, J = 11.8 Hz, 3H), 3.44 (p, J= 11.5, 11.0 Hz, 4H), 3.19 (t, J = 14.9 Hz, 4H), 2.90 (ddd, J = 17.3, 13.7, 5.3 Hz, 1H), 2.59 (d, J = 16.9 Hz, 1H), 2.35 (ddd, J = 28.1, 13.9, 5.6 Hz, 3H), 2.03 - 1.91 (m, 1H), 1.90 - 1.62 (m, 6H), 1.38 (s, 3H). |
| **485** | 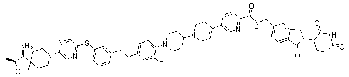 | 610.8 (M/2+ 1) | ¹H NMR (400 MHz, DMSO-d6) δ 11.32 (s, 1H), 10.99 (s, 1H), 9.51 (t, J= 6.4 Hz, 1H), 8.81 (d, J = 2.2 Hz, 1H), 8.36 (d, J = 1.5 Hz, 1H), 8.27 (s, 3H), 8.16 - 8.04 (m, 3H), 7.68 (d, J = 7.8 Hz, 1H), 7.54 (s, 1H), 7.47 (d, J = 7.9 Hz, 1H), 7.21 - 6.98 (m, 4H), 6.73 - 6.45 (m, 4H), 5.10 (dd, J = 13.3, 5.1 Hz, 1H), 4.81 - 4.49 (m, 2H), 4.47 - 4.26 (m, 2H), 4.26 - 4.11 (m, 5H), 4.02 - 3.90 (m, 3H), 3.82 - 3.73 (m, 1H), 3.65 (d, J = 9.1 Hz, 1H), 3.53 - 3.41 (m, 3H), 3.36 (t, J = 5.4 Hz, 1H), 3.23 (d, J = 10.6 Hz, 1H), 3.15 - 2.97 (m, 3H), 2.97 - 2.80 (m, 2H), 2.71 (t, J = 11.8 Hz, 2H), 2.63 - 2.55 (m, 1H), 2.43 - 2.27 (m, 3H), 2.23 (d, J = 11.2 Hz, 1H), 2.05 - 1.88 (m, 3H), 1.85 - 1.56 (m, 4H), 1.24 (d, J = 6.1 Hz, 3H). |
| **486** | 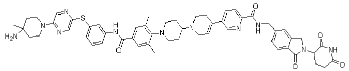 | 988 | |
| **487** | 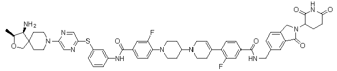 | 1051 | |
| **488** | 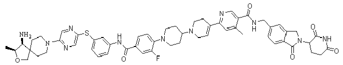 | 524.9 (M/2+ 1) | ¹H NMR (400 MHz, DMSO-d6) δ 11.41 (s, 1H), 11.00 (s, 1H), 10.24 (s, 1H), 9.30 (t, J = 5.9 Hz, 1H), 8.64 (s, 1H), 8.39 (s, 1H), 8.28 (s, 2H), 8.19 (s, 1H), 7.82 - 7.74 (m, 3H), 7.74 - 7.67 (m, 3H), 7.59 (s, 1H), 7.51 (d, J = 7.8 Hz, 1H), 7.29 (t, J = 8.0 Hz, 1H), 7.18 |
| | | | (d, J = 8.6 Hz, 1H), 6.98 (d, J = 7.7 Hz, 1H), 6.87 (s, 1H), 5.11 (dd, J = 13.3, 5.0 Hz, 1H), 4.59 (d, J = 5.8 Hz, 2H), 4.47 (d, J = 17.4 Hz, 1H), 4.33 (d, J = 17.4 Hz, 1H), 4.02 (s, 2H), 3.92 (d, J = 9.0 Hz, 1H), 3.79 (s, 1H), 3.66 (t, J = 10.7 Hz, 3H), 3.48 (s, 2H), 3.35 (t, J = 5.6 Hz, 1H), 3.18 (d, J = 18.6 Hz, 1H), 3.08 (dt, J = 15.5, 7.6 Hz, 2H), 2.97 (d, J = 5.5 Hz, 1H), 2.83 (t, J = 11.7 Hz, 3H), 2.64 - 2.55 (m, 1H), 2.46 (s, 3H), 2.44 - 2.22 (m, 3H), 1.99 (tt, J = 13.9, 8.3 Hz, 5H), 1.83 - 1.74 (m, 2H), 1.71 - 1.56 (m, 1H), 1.23 (d, J = 6.6 Hz, 6H). |
| **489** | 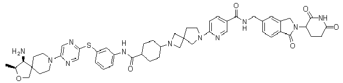 | 968 | |
| **490** | 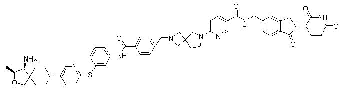 | MS( M/2+ H+) 488.7 | ¹H NMR (400 MHz, DMSO-d6) δ 11.01 (s, 1H), 10.2-10.1(m, 1H), 9.49 (m, 1H), 8.80 (m,1H), 8.41 - 8.21 (m, 5H), 8.15 (s, 1H),8.10- 7.94 (m, 2H), 7.70 (m, 1H), 7.64-7.42 (m, 3H), 7.29-7.17 (m, 1H), 7.00 - 6.95 (m, 1H), 6.57 (s, 1H), 5.12 (dd, J = 13.3, 5.1 Hz, 1H), 4.62 (d, J = 6.4 Hz, 2H), 4.47 - 4.26 (m, 2H), 4.26 - 4.11 (m, 3H), 3.96 - 3.85 (m, 3H), 3.77 - 3.61 (m, 4H), 3.49 (s, 1H), 3.28 - 3.17 (m, 1H), 3.12 - 2.98 (m, 4H), 2.96 - 2.78 (m, 3H), 2.66 - 2.54 (m, 4H), 2.44 -2.23 (m, 3H), 1.86 - 1.75 (m, 2H), 1.70 - 1.56 (m, 2H), 1.24 (d, J = 6.5 Hz, 3H). |
| **491** | 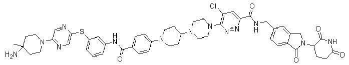 | MS( M/2+ H+) 499.7 | ¹H NMR (400 MHz, DMSO-d6) δ 11.81 (s, 1H), 10.99 (s, 1H), 10.07 (d, J = 4.0 Hz, 1H), 9.90 - 9.70 (m, 1H), 8.47 - 8.26 (m, 4H), 8.17 (d, J = 11.6 Hz, 1H), 7.87 (dd, J = 8.9, 4.5 Hz, 2H), 7.76 (q, J = 1.8 Hz, 1H), 7.69 (tq, J = 8.7, 4.4, 3.8 Hz, 2H), 7.54 (s, 1H), 7.49 - 7.43 (m, 1H), 7.32 - 7.25 (m, 1H), 7.10 (d, J = 8.4 Hz, 2H), 6.95 (d, J = 8.1 Hz, 1H), 5.10 (dd, J = 13.3, 5.0 Hz, 1H), 4.46 - 4.25 (m, 2H), 4.18 (d, J = 13.0 Hz, 2H), 4.04 (dt, J = 12.6, 4.2 Hz, 2H), 3.75 - 3.63 (m, 4H), 3.53 - 3.40 (m, 4H), 3.24 (d, J = 10.8 Hz, 2H), 2.88 (d, J = 17.9 Hz, 3H), 2.59 (d, J = 17.4 Hz, 1H), 2.43 - 2.19 (m, 4H), 2.02 - 1.68 (m, 9H), 1.37 (s, 3H). |
| **492** | 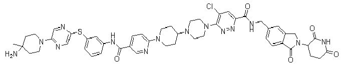 | MS( M/2+ H+) 500.2 | ¹H NMR (400 MHz, DMSO-d6) δ 11.94 (s, 1H), 10.99 (s, 1H), 10.50 (s, 1H), 9.84 (t, J = 6.5 Hz, 1H), 8.72 (d, J = 2.4 Hz, 1H), 8.43 - 8.31 (m, 5H), 8.22 - 8.13 (m, 2H), 7.77 - 7.64 (m, 3H), 7.54 (s, 1H), 7.50 - 7.42 (m, 1H), 7.33 (dt, J = 15.8, 8.7 Hz, 2H), 6.99 (dt, J = 7.7, 1.4 Hz, 1H), 5.10 (dd, J = 13.3, 5.0 Hz, 1H), 4.29 (d, J = 17.4 Hz,2H), 4.18 (d, J = 13.3 Hz, 2H), 4.09 - 3.98 (m, 2H), 3.76 - 3.56 (m, 6H), 3.41 (q, J = 7.0, 5.9 Hz, 2H), 3.33 - 3.07 (m, 5H), 2.90 (ddd, J = 18.4, 13.7, 5.3 Hz, 1H), 2.64 - 2.54 (m, 1H), 2.43 - 2.26 (m, 3H), 2.04 - 1.66 (m, 9H), 1.38 (s, 3H). |
| **493** | 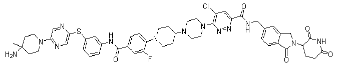 | MS( M/2+ H+) 508.7 | ¹H NMR (400 MHz, DMSO-d6) δ 11.92 (s, 1H), 10.99 (s, 1H), 10.23 (s, 1H), 9.85 (t, J = 6.2 Hz, 1H), 8.39 (s, 4H), 8.18 (d, J = 13.4 Hz, 2H), 7.80 - 7.64 (m, 5H), 7.54 (s, 1H), 7.47 (d, J = 7.8 Hz, 1H), 7.29 (t, J = 8.0 Hz, 1H), 7.16 (t, J = 9.1 Hz, 1H), 6.98 (d, J = 7.8 Hz, 1H), 5.10 (dd, J = 13.2, 5.0 Hz, 1H), 4.65 - 4.57 (m, 2H), 4.46 - 4.28 (m, 2H), 4.22 - 4.00 (m, 4H), 3.75 - 3.61 (m, 6H), 3.44 - 3.39 (m, 2H), 3.26 (d, J = 11.1 Hz, 2H), 2.84 (dt, J = 34.9, 12.3 Hz, 3H), 2.59 (d, J = 17.3 Hz, 1H), 2.27 (s, 3H), 2.00 - 1.70 (m, 8H), 1.38 (s, 3H). |
| **494** | 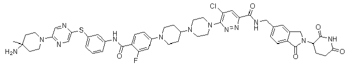 | 508.8 (M/2+ 1) | ¹H NMR (400 MHz, DMSO-d6) δ 11.08 (s, 1H), 11.00 (s, 1H), 10.04 (s, 1H), 9.83 (t, J = 6.2 Hz, 1H), 8.39 (d, J = 1.5 Hz, 1H), 8.23 - 8.15 (m, 2H), 8.10 (s, 3H), 7.77 - 7.64 (m, 2H), 7.55 (q, J = 10.4, 9.5 Hz, 3H), 7.47 (d, J = 8.1 Hz, 1H), 7.28 (t, J = 8.0 Hz, 1H), 7.03 - 6.93 (m, 1H), 6.93 - 6.84 (m, 2H), 5.10 (dd, J = 13.2, 5.0 Hz, 1H), 4.63 (d, J = 6.1 Hz, 2H), 4.43 (d, J = 17.4 Hz, 1H), 4.29 (d, J = 17.4 Hz, 1H), 4.19 (d, J = 13.5 Hz, 2H), 4.08 (dd, J = 20.1, 14.3 Hz, 5H), 3.63 (dt, J = 29.9, 12.3 Hz, 3H), 3.38 (s, 1H), 3.25 (d, J = 11.0 Hz, 2H), 2.88 (dt, J = 20.3, 13.0 Hz, 3H), 2.70 - 2.54 (m, 1H), 2.18 (s, 1H), 2.05 - 1.90 (m, 2H), 1.73 (s, 6H), 1.37 (s, 3H), 1.23 (s, 3H). |
| **495** | 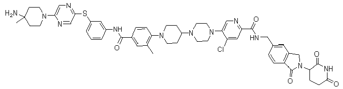 | 506.3 (M/2+ 1) | ¹H NMR (400 MHz, DMSO-d6) δ 11.78 (s, 1H), 10.99 (s, 1H), 10.19 (s, 1H), 9.46 (t, J = 6.4 Hz, 1H), 8.45 (s, 1H), 8.42 - 8.34 (m, 4H), 8.19 (d, J = 1.3 Hz, 1H), 8.03 (s, 1H), 7.80 (d, J = 2.1 Hz, 1H), 7.76 (q, J = 2.1 Hz, 2H), 7.72 - 7.65 (m, 2H), 7.52 (s, 1H), 7.45 (d, J = 7.7 Hz, 1H), 7.29 (t, J = 8.0 Hz, 1H), 7.12 (d, J = 8.4 Hz, 1H), 6.99 - 6.93 (m, 1H), 5.10 (dd, J = 13.3, 5.1 Hz, 1H), 4.59 (d, J = 6.2 Hz, 2H), 4.47 - 4.24 (m, 2H), 4.04 (dt, J = 14.0, 4.8 Hz, 2H), 3.76 - 3.62 (m, 4H), 3.54 (t, J = 11.7 Hz, 2H), 3.47 - 3.39 (m, 3H), 3.29 (dd, J = 25.9, 10.3 Hz, 4H), 2.90 (ddd, J = 17.9, 13.5, 5.3 Hz, 1H), 2.72 (t, J = 11.6 Hz, 3H), 2.63 - 2.54 (m, 1H), 2.33 (s, 4H), 2.05 - 1.94 (m, 4H), 1.82 (ddd, J = 13.3, 9.2, 4.0 Hz, 2H), 1.76 - 1.69 (m, 2H), 1.37 (s, 3H). |
| **496** | 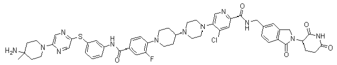 | 508.3 (M/2+ 1) | ¹H NMR (400 MHz, DMSO-d6) δ 11.73 (s, 1H), 10.99 (s, 1H), 10.21 (s, 1H), 9.46 (t, J = 6.4 Hz, 1H), 8.44 (s, 1H), 8.42 - 8.31 (m, 4H), 8.19 (d, J = 1.3 Hz, 1H), 8.02 (s, 1H), 7.82 - 7.73 (m, 3H), 7.72 - 7.65 (m, 2H), 7.51 (s, 1H), 7.44 (d, J = 7.9 Hz, 1H), 7.29 (t, J = 8.0 Hz, 1H), 7.16 (t, J = 8.7 Hz, 1H), 7.04 - 6.92 (m, 1H), 5.09 (dd, J = 13.3, 5.1 Hz, 1H), 4.42 (d, J = 17.4 Hz, 1H), 4.28 (d, J = 17.4 Hz, 1H), 4.04 (dt, J = 13.9, 4.6 Hz, 2H), 3.67 (d, J = 10.3 Hz, 7H), 3.56 - 3.45 (m, 2H), 3.45 - 3.39 (m, 2H), 3.24 (d, J = 11.0 Hz, 2H), 2.90 (ddd, J = 17.7, 13.6, 5.1 Hz, 1H), 2.80 (t, J = 11.8 Hz, 2H), 2.58 (d, J = 17.0 Hz, 1H), 2.38 (td, J = 13.1, 4.5 Hz, 1H), 2.33 - 2.25 (m, 3H), 2.03 - 1.90 (m, 4H), 1.85 - 1.65 (m, 4H), 1.37 (s, 3H). |
| **497** | 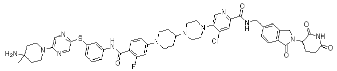 | 508.3 (M/2+ 1) | ¹H NMR (400 MHz, DMSO-d6) δ 11.70 (s, 1H), 10.99 (s, 1H), 10.05 (d, J = 1.9 Hz, 1H), 9.45 (t, J = 6.3 Hz, 1H), 8.44 (s, 1H), 8.40 - 8.34 (m, 4H), 8.19 (d, J = 1.4 Hz, 1H), 8.02 (s, 1H), 7.68 (d, J = 2.2 Hz, 1H), 7.59 (dd, J = 7.9, 2.1 Hz, 1H), 7.51 (s, 1H), 7.46 - 7.40 (m, 2H), 7.31 - 7.23 (m, 2H), 6.96 (d, J = 7.9 Hz, 1H), 6.91 (d, J = 4.5 Hz, 1H), 6.87 (s, 1H), 5.09 (dd, J = 13.2, 5.2 Hz, 1H), 4.59 (d, J = 6.1 Hz, 2H), 4.28 (d, J |
| | | | = 17.4 Hz, 1H), 3.66 (t, J = 14.1 Hz, 6H), 3.50 (t, J = 11.6 Hz, 4H), 3.41 (ddd, J = 13.4, 9.3, 3.5 Hz, 2H), 3.21 (d, J = 11.4 Hz, 3H), 2.84 (t, J = 11.5 Hz, 3H), 2.63 - 2.54 (m, 1H), 2.27 - 2.22 (m, 4H), 1.99 (dd, J = 12.4, 6.1 Hz, 1H), 1.83 - 1.77 (m, 5H), 1.73 (d, J = 5.0 Hz, 1H), 1.37 (s, 3H). |
| **498** | 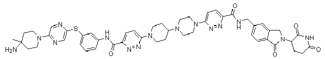 | MS( M/2+ H+) 483.7 | ¹H NMR (400 MHz, DMSO-d6) δ 11.90 (s, 1H), 10.99 (s, 1H), 10.79 (s, 1H), 9.61 (t, J = 6.4 Hz, 1H), 8.49 - 8.31 (m, 4H), 8.20 (d, J = 1.3 Hz, 1H), 7.96 (dd, J = 9.5, 4.2 Hz, 2H), 7.87 (t, J = 2.0 Hz, 1H), 7.80 (dd, J = 7.9, 2.1 Hz, 1H), 7.68 (d, J = 7.8 Hz, 1H), 7.59 - 7.51 (m, 3H), 7.47 (d, J = 7.9 Hz, 1H), 7.30 (t, J = 7.9 Hz, 1H), 7.02 - 6.94 (m, 1H), 5.10 (dd, J = 13.3, 5.1 Hz, 1H), 4.64-4.60 (m, 2H), 4.47 - 4.26 (m, 3H), 4.05 (dt, J = 13.9, 4.9 Hz, 2H), 3.63 (d, J = 11.9 Hz, 6H), 3.42 (ddd, J = 13.7, 9.5, 3.7 Hz, 2H), 3.26 - 3.00 (m, 5H), 2.90 (ddd, J = 17.7, 13.7, 5.3 Hz, 1H), 2.64 - 2.54 (m, 1H), 2.40 - 2.23 (m, 3H), 2.04 - 1.69 (m, 8H), 1.38 (s, 3H). |
| **499** | 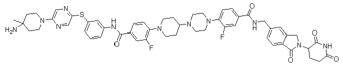 | MS( M/2+ H+) 499.7 | ¹H NMR (400 MHz, DMSO-d6) δ 11.53 (brs, 1H), 10.99 (s, 1H), 10.22 (s, 1H), 9.20 (m, 1H), 8.43 (s, 1H), 8.30 (s, 2H), 8.20 (s, 1H),7.84 - 7.66 (m, 6H), 7.52(s, 1H), 7.48 - 7.45 (m, 1H), 7.21-7.05 (m, 3H), 7.01 - 6.95 (m, 1H), 5.12 (dd, J = 13.3, 5.1 Hz, 1H), 4.61 (d, J = 6.4 Hz, 2H), 4.46 - 4.26 (m, 2H), 4.09 - 3.94 (m, 4H), 3.54 - 3.19 (m, 5H), 3.10 (t, J = 12.9 Hz, 1H), 2.76 (t, J = 11.6 Hz, 2H), 2.50 (s,3H) , 2.44 - 2.21 (m, 6H), 2.10 -1.94 (m, 4H), 1.89 - 1.68 (m, 4H), 1.38 (s, 3H). |
| **500** | 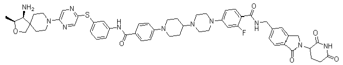 | 518.8 (M/2+ 1) | ¹H NMR (400 MHz, DMSO-d6) δ 11.52 (s, 1H), 11.00 (s, 1H), 10.06 (s, 1H), 8.63 (q, J = 5.7 Hz, 1H), 8.39 (d, J = 1.5 Hz, 1H), 8.21 (d, J = 16.5 Hz, 4H), 7.87 (d, J = 8.5 Hz, 2H), 7.76 (d, J = 2.0 Hz, 1H), 7.73 - 7.61 (m, 3H), 7.53 (s, 1H), 7.46 (d, J = 7.9 Hz, 1H), 7.28 (t, J = 8.0 Hz, 1H), 7.08 (d, J = 8.6 Hz, 2H), 6.99 - 6.84 (m, 3H), 5.11 (dd, J = 13.3, 5.1 Hz, 1H), 4.55 (d, J = 5.9 Hz, 2H), 4.49 - 4.27 (m, 2H), 4.26 - 4.14 (m, 2H), 4.14 - 3.88 (m, 6H), 3.62 |
| | | | (dd, J = 24.9, 10.2 Hz, 3H), 3.52 - 3.30 (m, 4H), 3.21 - 3.02 (m, 4H), 2.98 - 2.78 (m, 3H), 2.64 - 2.55 (m, 1H), 2.38 (qd, J = 13.2, 4.4 Hz, 1H), 2.29 - 2.19 (m, 2H), 2.03 - 1.95 (m, 1H), 1.88 - 1.72 (m, 4H), 1.71 - 1.54 (m, 2H), 1.23 (d, J = 6.4 Hz, 3H). |
| **501** | 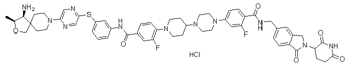 | 1054 | |
| **502** | 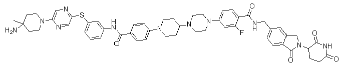 | 980 | ¹H NMR (400 MHz, DMSO-*d*₆) δ 11.53 (s, 1H), 11.00 (s, 1H), 10.07 (s, 1H), 8.63 (q, J = 5.7 Hz, 1H), 8.48-8.29 (m, 4H), 8.19 (d, J = 1.3 Hz, 1H), 8.00-7.61 (m, 6H), 7.56-7.44 (m, 2H), 7.28 (t, J = 8.0 Hz, 1H), 7.19-6.81 (m, 5H), 5.20-5.09 (m, 1H), 4.56 (d, J = 5.9 Hz, 3H), 4.13-3.98 (m, 6H), 3.66-3.30 (m, 8H), 3.25-3.05 (m, 2H), 2.90 (ddd, J = 25.9, 13.4, 6.9 Hz, 3H), 2.70-2.56 (m, 1H), 2.43-2.22 (m, 3H), 2.09-1.69 (m, 7H), 1.38 (s, 3H). |
| **503** | 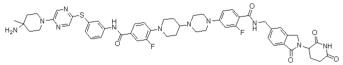 | 998 | ¹H NMR (400 MHz, DMSO-*d*₆) δ 11.66 (s, 1H), 11.00 (s, 1H), 10.22 (s, 1H), 8.63 (q, J = 5.7 Hz, 1H), 8.47-8.28 (m, 4H), 8.20 (d, J = 1.4 Hz, 1H), 7.86-7.61 (m, 6H), 7.60-7.43 (m, 2H), 7.23 (dt, J = 56.6, 8.3 Hz, 2H), 7.04 -6.85 (m, 3H), 5.11 (dd, J = 13.3, 5.1 Hz, 1H), 4.65-4.43 (m, 3H), 4.04 (dt, J = 13.6, 4.6 Hz, 5H), 3.64 (dd, J= 19.3, 11.7 Hz, 4H), 3.48-3.34 (m, 5H), 3.16 (d, J = 12.3 Hz, 2H), 3.01-2.75 (m, 3H), 2.69-2.56 (m, 1H), 2.47-2.18 (m, 3H), 2.11-1.66 (m, 7H), 1.38 (s, 3H). |
| **504** | 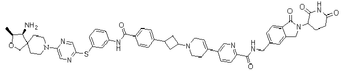 | 987 | |
| **505** | 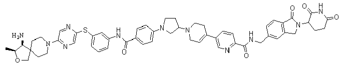 | 519.7. (M/2+ 1) | ¹H NMR (400 MHz, DMSO-d₆) *δ* 11.91 (s, 1H), 10.99 (s, 1H), 9.98 (s, 1H), 9.50 (t, J = 6.4 Hz, 1H), 8.82 (d, J = 2.2 Hz, 1H), 8.39 (d, J = 1.5 Hz, 1H), 8.27 - 8.04 (m, 6H), 7.90 (d, J = 8.4 Hz, 2H), 7.77 (d, J = 2.0 Hz, 1H), 7.69 (dd, J = 8.5, 5.6 Hz, 2H), 7.54 (s, 1H), 7.47 (d, J = 7.9 Hz, 1H), 7.28 (t, J = 8.0 Hz, 1H), 6.94 (d, J = 7.7 Hz, 1H), 6.74 - 6.65 (m, 2H), 6.51 (s, 1H), 5.10 (dd, J = 13.3, 5.1 Hz, 1H), 4.61 (d, J = 6.4 Hz, 2H), 4.43 (d, J = 17.4 Hz, 2H), 4.33 - |
| | | | 4.10 (m, 7H), 4.03 - 3.88 (m, 2H), 3.76 (d, J = 9.3 Hz, 2H), 3.64 (t, J = 9.9 Hz, 2H), 3.37 (s, 1H), 3.08 (d, J = 7.9 Hz, 3H), 2.91 (t, J = 12.9 Hz, 2H), 2.59 (d, J = 17.1 Hz, 2H), 2.43 (d, J = 7.9 Hz, 1H), 2.37 (dd, J = 13.1, 4.5 Hz, 1H), 2.14 (p, J = 8.0 Hz, 1H), 2.00 - 1.96 (m, 1H), 1.82 - 1.72 (m, 2H), 1.67 (d, J = 13.3 Hz, 1H), 1.58 (d, J = 12.9 Hz, 1H), 1.23 (d, J = 2.8 Hz, 3H). |
| **506** | 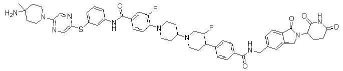 | 499.1 (M/2+ 1) | ¹H NMR (400 MHz, DMSO-d₆) *δ* 12.03 (s, 1H), 10.99 (s, 1H), 10.22 (s, 1H), 9.32 - 9.04 (m, 1H), 8.42 - 8.29 (m, 3H), 8.20 (d, J = 1.3 Hz, 1H), 7.94 (d, J = 8.0 Hz, 2H), 7.83 - 7.64 (m, 4H), 7.54 (s, 1H), 7.44 (dd, J = 22.7, 8.0 Hz, 3H), 7.30 (t, J = 8.0 Hz, 1H), 7.17 (t, J = 8.7 Hz, 1H), 6.98 (d, J = 7.8 Hz, 1H), 5.41 (d, J = 48.4 Hz, 1H), 5.10 (dd, J = 13.2, 5.1 Hz, 1H), 4.60 (d, J = 5.7 Hz, 2H), 4.47 - 4.40 (m, 2H), 4.32 (d, 2H), 4.04 (dd, J = 13.8, 5.2 Hz, 2H), 3.86 (s, 1H), 3.67 (d, J = 10.8 Hz, 2H), 3.63 - 3.47 (m, 1H), 3.40 (td, J = 10.1, 9.5, 5.0 Hz, 1H), 3.31 - 3.05 (m, 2H), 2.98 - 2.74 (m, 3H), 2.65 - 2.54 (m, 1H), 2.35 (ddd, J = 28.7, 13.6, 8.7 Hz, 3H), 2.02 - 1.87 (m, 2H), 1.86 - 1.66 (m, 3H), 1.38 (s, 3H), 1.32 -1.19 (m, 5H). |
| **507** | 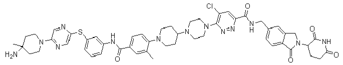 | 1012. 2 | ¹H NMR (400 MHz, DMSO-*d₆*): *δ* 11.52-11.46 (m, 1 H), 10.97 (s, 1 H), 10.13 (s, 1 H), 9.84 (t, *J* = 1.2Hz, 1 H), 9.39 (d, *J* = 1.2Hz, 1 H), 8.27-8.17 (m, 5 H), 7.78-7.77 (m, 3 H), 7.70-7.69 (m, 2 H),7.55-7.47 (m,2 H), 7.31-7.27 (m, 1 H), 7.12-7.10 (m, 1 H), 6.98-6.96 (m, 1 H), 5.11-5.09 (m, 1 H), 4.4.64-4.63(m, 2 H), 4.44-4.41 (m, 1 H), 4.32-4.23 (m, 4 H), 3.72-3.64 (m,5 H), 3.42-3.39 (m, 3 H), 3.37-3.30 (m,4 H), 2.94-2.91 (m,1 H), 2.71-2.51 (m,4 H), 2.48-2.25 (m, 7 H) ,1.99-1.96 (m,3 H), 1.79-1.73 (m,5 H), 1.38 (s, 3 H). |
| **508** | 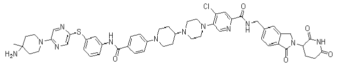 | 996.8 | ¹H NMR (400 MHz, DMSO-*d₆*): *δ* 11.61-11.58 (m, 1 H), 10.97 (s, 1 H), 10.04 (s,1 H), 9.43 (t, *J* = 6.4 Hz, 1 H), 8.44 (s, 1 H), 8.38 (d, *J* = 1.2 Hz, 1 H), 8.31(s, 3H), 8.19 (d, *J* = 1.2 Hz, 1 H), 8.02 (s, 1H), 7.87 (d, *J* = 8.8 Hz, 2 H), 7.78-7.76 (m, 1 H), 7.71-7.66(m,2H), |
| | | | 7.52 (s, 1 H), 7.45(d, *J* = 8.4Hz, 1 H), 7.28 (t, *J* = 8 Hz, 1 H), 7.08 (d, *J* = 8.8Hz, 1 H), 6.96 (d, *J* = 7.2 Hz, 1 H), 5.12-5.07 (m, 1 H), 4.59 (d, *J* = 6Hz, 2 H), 4.45-4.26 (m, 2H), 4.11-4.02 (m, 4 H), 3.70-3.63 (m, 4 H), 3.52-3.37 (m, 5 H), 3.26-3.19 (m,2 H), 2.95-2.81 (m, 3 H), 2.61-2.57 (m, 1 H), 2.42-2.25(m, 3 H), 2.01-1.96 (m, 1 H), 1.89-1.71 (m,6 H), 1.37 (s, 3 H). |
| **509** | 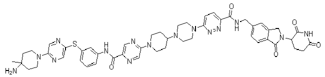 | 965.9 | ¹H NMR (400 MHz, DMSO-*d*₆) δ 11.96 (s, 1H), 10.99 (s, 1H), 10.31 (s, 1H), 9.62 (t, *J* = 6.4 Hz, 1H), 8.72 (d, *J* = 1.2 Hz, 1H), 8.45 - 8.37 (m, 4H), 8.19 (d, *J* = 1.3 Hz, 1H), 7.96 (d, *J* = 9.5 Hz, 1H), 7.88 (t, *J* = 2.0 Hz, 1H), 7.78 - 7.71 (m, 1H), 7.68 (d, *J* = 7.8 Hz, 1H), 7.55 (d, *J* = 10.3 Hz, 2H), 7.50 - 7.43 (m, 1H), 7.28 (t, *J* = 8.0 Hz, 1H), 6.96 (dd, *J* = 7.8, 1.6 Hz, 1H), 5.10 (dd, *J* = 13.3, 5.1 Hz, 1H), 4.71 (d, *J* = 12.9 Hz, 2H), 4.62 (t, *J* = 10.0 Hz, 4H), 4.43 (d, *J* = 17.4 Hz, 1H), 4.29 (d, *J* = 17.5 Hz, 1H), 4.03 (dd, *J* = 16.2, 11.0 Hz, 2H), 3.71 - 3.51 (m, 5H), 3.51 - 3.38 (m, 3H), 3.19 (d, *J* = 11.3 Hz, 2H), 3.02 (t, *J =* 12.7 Hz, 2H), 2.90 (ddd, *J =* 17.8, 13.6, 5.3 Hz, 1H), 2.59 (d, *J* = 17.0 Hz, 1H), 2.45 - 2.25 (m, 3H), 2.03 - 1.94 (m, 1H), 1.78 (q, *J* = 10.6 Hz, 6H), 1.38 (s, 3H). |
| **510** | 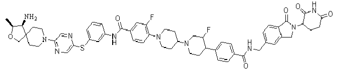 | 527.2 (M/2+ 1) | ¹H NMR (400 MHz, DMSO-d₆) *δ* 12.01 (s, 1H), 11.00 (s, 1H), 10.22 (s, 1H), 9.23 (t, J = 5.9 Hz, 1H), 8.40 (d, J = 1.4 Hz, 1H), 8.28 - 8.15 (m, 4H), 7.94 (d, J = 8.0 Hz, 2H), 7.82 - 7.73 (m, 3H), 7.69 (dd, J = 8.5, 5.2 Hz, 2H), 7.54 (s, 1H), 7.47 (d, J = 8.1 Hz, 1H), 7.41 (d, J = 8.1 Hz, 2H), 7.30 (t, J = 8.1 Hz, 1H), 7.17 (t, J = 8.9 Hz, 1H), 6.98 (d, J = 7.8 Hz, 1H), 5.10 (dd, J = 13.3, 5.0 Hz, 1H), 4.60 (d, J = 5.8 Hz, 2H), 4.49 - 4.40 (m, 2H), 4.30 (d, J = 17.4 Hz, 2H), 4.27 - 4.16 (m, 3H), 3.90 (t, J = 11.3 Hz, 3H), 3.67 (t, J = 10.0 Hz, 3H), 3.55 (dd, J = 27.0, 8.6 Hz, 2H), 3.36 (p, J = 5.5, 4.8 Hz, 1H), 3.20 - 3.04 (m, 3H), 2.87 (dt, J = 29.6, 12.7 Hz, 3H), 2.64 - 2.55 (m, 1H), 2.42 - 2.34 (m, 2H), 2.30 (d, J = 11.5 Hz, 2H), 2.07 (d, |
| | | | J = 13.5 Hz, 1H), 2.02 - 1.91 (m, 3H), 1.82 - 1.73 (m, 2H), 1.67 (d, J = 13.5 Hz, 1H), 1.58 (d, J = 13.0 Hz, 1H), 1.23 (d, J = 4.4 Hz, 3H). |
| **511** | 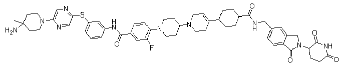 | 492.2 (M/2+ 1) | ¹H NMR (400 MHz, DMSO-d₆) *δ* 11.00 (s, 1H), 10.29 (s, 1H), 10.18 (s, 1H), 8.45 - 8.37 (m, 2H), 8.28 - 8.17 (m, 4H), 7.80 - 7.74 (m, 3H), 7.68 (dd, J = 7.9, 3.0 Hz, 2H), 7.45 (s, 1H), 7.38 (d, J = 7.8 Hz, 1H), 7.30 (t, J = 8.0 Hz, 1H), 7.15 (t, J = 8.7 Hz, 1H), 6.98 (d, J = 7.7 Hz, 1H), 5.40 (s, 1H), 5.11 (dd, J = 13.3, 5.1 Hz, 1H), 4.44 (d, J = 17.3 Hz, 2H), 4.37 (d, J = 5.8 Hz, 2H), 4.29 (d, J = 17.3 Hz, 2H), 4.05 (d, J = 14.3 Hz, 2H), 3.72 (s, 4H), 3.44 - 3.34 (m, 4H), 3.06 - 2.86 (m, 3H), 2.81 (t, J = 11.7 Hz, 2H), 2.60 (d, J = 16.6 Hz, 2H), 2.45 (s, 2H), 2.40 (dd, J = 13.1, 4.6 Hz, 1H), 2.22 (s, 2H), 2.16 (s, 2H), 2.04 - 1.81 (m, 5H), 1.80 - 1.70 (m, 4H), 1.61 (s, 2H), 1.23 (s, 3H). |
| **512** | 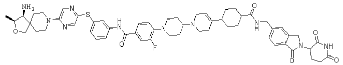 | 1039 | |
| **513** | 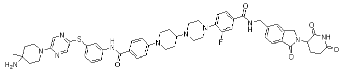 | MS( M/2+ H+) 490.8 | ¹H NMR (400 MHz, DMSO-d₆) *δ* 11.53 (brs, 1H), 10.99 (s, 1H), 10.22 (s, 1H), 9.20 (m, 1H), 8.43 (s, 1H), 8.30 (s, 2H), 8.20 (s, 1H),7.84 - 7.66 (m, 6H), 7.52(s, 1H), 7.48 - 7.45 (m, 1H), 7.21-7.05 (m, 4H), 7.01 - 6.95 (m, 1H), 5.12 (dd, J = 13.3, 5.1 Hz, 1H), 4.61 (d, J = 6.4 Hz, 2H), 4.46 - 4.26 (m, 2H), 4.09 - 3.94 (m, 4H), 3.54 - 3.19 (m, 5H), 3.10 (t, J = 12.9 Hz, 1H), 2.76 (t, J = 11.6 Hz, 2H), 2.50 (s,3H) , 2.44 - 2.21 (m, 6H), 2.10 -1.94 (m, 4H), 1.89 - 1.68 (m, 4H), 1.38 (s, 3H). |
| **514** | 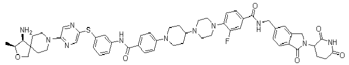 | 1036 | |
| **515** | 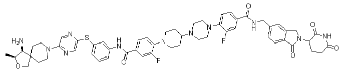 | 527.7 (M/2+ 1) | ¹H NMR (400 MHz, DMSO-d₆) *δ* 11.49 (s, 1H), 11.00 (s, 1H), 10.20 (s, 1H), 9.19 (t, J = 6.0 Hz, 1H), 8.40 (d, J = 1.5 Hz, 1H), 8.24 - 8.11 (m, 4H), 7.81 - 7.72 (m, 5H), 7.69 (dd, J = 7.9, 2.4 Hz, 2H), 7.53 (s, 1H), 7.45 (d, J = 8.0 Hz, 1H), 7.30 (t, J = 8.0 Hz, 1H), 7.18 (q, J = 9.4 Hz, 2H), 7.00 - 6.95 (m, 1H), 5.10 (dd, J = 13.3, 5.1 Hz, 1H), 4.57 (d, J = 5.7 Hz, 2H), 4.44 (d, J = 17.4 Hz, |
| | | | 2H), 4.35 - 4.09 (m, 5H), 3.91 (d, J = 9.1 Hz, 1H), 3.68 - 3.62 (m, 5H), 3.38 (d, J = 2.8 Hz, 4H), 3.25 (d, J = 10.5 Hz, 2H), 3.15 - 3.04 (m, 2H), 2.91 (ddd, J = 18.0, 13.5, 5.4 Hz, 1H), 2.81 (t, J = 12.0 Hz, 2H), 2.59 (d, J = 16.8 Hz, 1H), 2.39 (td, J = 13.2, 4.5 Hz, 1H), 2.28 (d, J = 11.5 Hz, 2H), 2.02 - 1.91 (m, 3H), 1.75 (d, J = 10.8 Hz, 2H), 1.68 (d, J = 13.1 Hz, 1H), 1.58 (d, J = 13.0 Hz, 1H), 1.23 (d, J = 5.0 Hz, 3H). |
| **516** | 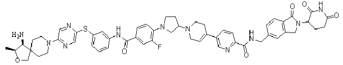 | 1020 | |
| **517** | 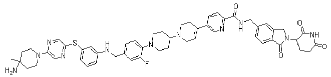 | 482.8 (M/2+ 1) | ¹H NMR (400 MHz, DMSO-d₆) *δ* 11.31 (s, 1H), 10.99 (s, 1H), 9.51 (t, J = 6.4 Hz, 1H), 8.81 (d, J = 2.2 Hz, 1H), 8.48 - 8.32 (m, 3H), 8.17 - 8.03 (m, 3H), 7.68 (d, J = 7.8 Hz, 1H), 7.54 (s, 1H), 7.47 (d, J = 7.9 Hz, 1H), 7.22 - 6.99 (m, 4H), 6.76 - 6.59 (m, 3H), 6.51 (s, 1H), 5.09 (dd, J = 13.3, 5.0 Hz, 1H), 4.61 (d, J = 6.4 Hz, 2H), 4.47 - 4.26 (m, 2H), 4.26 - 4.18 (m, 2H), 4.11 - 3.94 (m, 4H), 3.77 (s, 2H), 3.55 - 3.37 (m, 5H), 3.30 - 3.18 (m, 1H), 3.07 - 2.98 (m, 1H), 2.97 - 2.81 (m, 2H), 2.78 - 2.65 (m, 2H), 2.63 - 2.55 (m, 1 H), 2.44 - 2.19 (m, 3H), 2.05 - 1.89 (m, 4H), 1.89 - 1.69 (m, 4H), 1.38 (s, 3H). |
| **518** | 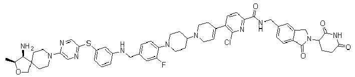 | 527.8 (M/2+ 1) | ¹H NMR (400 MHz, DMSO-d₆) *δ* 11.37 (s, 1H), 10.99 (s, 1H), 9.42 (t, *J* = 6.3 Hz, 1H), 8.36 (d, *J* = 1.5 Hz, 1H), 8.24 (d, *J* = 5.4 Hz, 3H), 8.12 - 8.04 (m, 2H), 7.92 (d, *J* = 7.8 Hz, 1H), 7.69 (d, *J* = 7.9 Hz, 1H), 7.54 (s, 1H), 7.46 (d, *J* = 7.9 Hz, 1H), 7.19 - 6.98 (m, 4H), 6.65 - 6.47 (m, 3H), 5.95 (s, 1H), 5.10 (dd, *J* = 13.3, 5.1 Hz, 1H), 4.60 (d, *J* = 6.3 Hz, 2H), 4.47 - 4.06 (m, 6H), 3.93 (d, *J* = 9.6 Hz, 4H), 3.74 (s, 1H), 3.66 (d, *J* = 9.0 Hz, 1H), 3.48 (d, *J* = 11.3 Hz, 3H), 3.37 (t, *J* = 5.6 Hz, 1H), 3.22 (d, *J =* 10.7 Hz, 1H), 3.14 - 2.98 (m, 3H), 2.97 - 2.85 (m, 1H), 2.71 (t, *J =* 11.7 Hz, 2H), 2.65 - 2.55 (m, 2H), 2.44 - 2.19 (m, 4H), 1.96 (q, *J =* 14.2, 9.9 Hz, 3H), 1.85 - 1.74 (m, 2H), 1.68 (d, *J* = 13.5 Hz, 1H), 1.60 (d, *J* = 12.8 Hz, 1H), 1.23 (d, *J* = 6.2 Hz, 3H). |
| **519** | 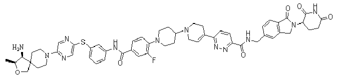 | 518.3 (M/2+ 1) | ¹H NMR (400 MHz, DMSO-d₆) *δ* 11.73 (s, 1H), 10.99 (s, 1H), 10.28 (s, 1H), 9.98 (t, *J* = 6.3 Hz, 1H), 8.43 - 8.30 (m, 4H), 8.27 - 8.17 (m, 3H), 7.84 - 7.66 (m, 5H), 7.57 (s, 1H), 7.53 - 7.48 (m, 1H), 7.29 (t, *J* = 7.9 Hz, 1H), 7.16 (t, *J* = 8.7 Hz, 1H), 7.01 - 6.95 (m, 2H), 5.10 (dd, *J* = 13.3, 5.1 Hz, 1H), 4.66 (d, *J* = 6.3 Hz, 2H), 4.47 - 4.26 (m, 2H), 4.26 -4.02 (m, 5H), 3.93 (d, *J* = 9.0 Hz, 1H), 3.88 - 3.79 (m, 1H), 3.71 - 3.61 (m, 3H), 3.55 - 3.45 (m, 1H), 3.34 (t, *J* = 5.6 Hz, 1H), 3.29 - 3.18 (m, 1H), 3.17 - 3.02 (m, 4H), 2.96 - 2.77 (m, 3H), 2.63 - 2.54 (m, 1H), 2.44 - 2.22 (m, 3H), 2.07 - 1.91 (m, 3H), 1.87 - 1.74 (m, 2H), 1.70 - 1.56 (m, 2H), 1.24 (d, *J* = 6.5 Hz, 3H). |
| **520** | 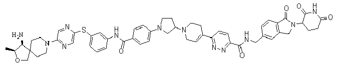 | 502.2 (M/2+ 1) | ¹H NMR (400 MHz, DMSO-d₆) *δ* 11.93 (s, 1H), 10.99 (s, 1H), 9.98 (d, *J* = 4.9 Hz, 2H), 8.32 - 8.06 (m, 6H), 7.91 (d, *J* = 8.5 Hz, 2H), 7.78 (s, 1H), 7.69 (d, *J* = 7.9 Hz, 2H), 7.58 (s, 1H), 7.51 (d, *J* = 7.9 Hz, 1H), 7.28 (t, *J* = 8.0 Hz, 1H), 7.02 - 6.92 (m, 2H), 6.70 (d, *J* = 8.5 Hz, 2H), 5.10 (dd, *J* = 13.3, 5.1 Hz, 1H), 4.66 (d, *J* = 6.2 Hz, 2H), 4.44 (d, *J* = 17.3 Hz, 2H), 4.34 - 4.11 (m, 7H), 3.94 - 3.74 (m, 4 H), 3.65 (t, *J* = 9.2 Hz, 2H), 3.36 (s, 1H), 3.21 - 3.02 (m, 4H), 2.91 (ddd, *J* = 18.4, 13.6, 5.4 Hz, 1H), 2.59 (d, *J* = 17.1 Hz, 2H), 2.44 - 2.37 (m, 1H), 2.37 - 2.30 (m, 1H), 2.00 (q, *J* = 7.3 Hz, 2H), 1.75 (d, *J* = 12.6 Hz, 2H), 1.68 (d, *J* = 13.3 Hz, 1H), 1.57 (d, *J* = 13.3 Hz, 1H), 1.23 (d, *J* = 2.9 Hz, 3H). |

The beneficial effects of the present invention were demonstrated by specific Experimental Examples.

### Experimental example 1. Biological assays of the degradation activity of compounds according to the present invention on SHP2 protein

### Experimental materials:

MV-411 cell line (COBIER, CBP-60522)
FBS (GEMINI, Cat. No. 900-108)
0.01M PBS (Biosharp, Cat. No. 162262)
IMDM (Hyclone, Cat. No. SH30228.01)
Penicillin-Streptomycin (Gibco, Cat. No. 15140122)
DMSO (Sigma, Cat. No. D5879)
Centrifuge Tube, 15 ml (Excell Bio, Cat. No. CS015-0001)
Cell Culture Dish, (WHB, Cat. No. CS016-0128)
12-well cell culture cluster (Corning, Cat. No. 3513)
RIPA lysate buffer (Thermo, Cat. No.89900)
Protein Loding Buffer (Beyotime, Cat. No. P0015L)
BCAProtein Assay Kit (EpiZyme, Cat. No. ZJ102)
SDS-PAGE Fast Preparation Kit (EpiZyme, Cat. No. PG112)
Anti-GAPDH(14C10) Rabbit mAb (CST, Cat. No. 2118L)
Anti-SHP2 rabbit mAb (CST, Cat. No. 3752S)
Peroxidase Affinipure(HRP) Goat Anti-Rabbit IgG (Zen Bioscience, Cat. No. 511203) TBST (Biosharp, Cat. No. BL601A)
ECL chemiluminescence kit (4A Biotech, Cat. No. 4AW011-200)

### Experimental method:

1. Preparation of buffer
   (1) Cell culture medium: IMDM medium + 10% FBS + 1% Pen Strep;
   (2) PBS buffer: PBS powder was dissolved in 2 L of ultrapure water and sterilized;
   (3) Cell lysate: RIPA lysate buffer is added with protease inhibitors at a ratio of 1:1000 before use.
2. Experimental procedures:
   (1) MV-411 cells were cultured in the cell culture medium, and then well-growing cells were selected and inoculated in a 12-well plate, with 1 mL/well and 1 × 10⁶ cells/well. The plate was incubated overnight in a 5% CO₂ cell incubator at 37 °C.
   (2) 10 mM storage solution of each drug was prepared using dimethylsulfoxide (DMSO).
      The stock solution was diluted gradually with DMSO before use, and then 1 µL of the compound solution was added to the cell culture well (to ensure that the DMSO concentration in the culture system was 0.1%), so that the final drug concentration is 100 nM, 10 nM, 3 nM, 1 nM, 0.3 nM, and 0.1 nM. Two wells were set for each concentration. The plate was gently shaken and mixed. Additionally, negative control wells (containing an equal amount of DMSO) and positive control wells were included.
   (3) After 24 hours of cultivation, the cells were lysed using RIPA cell lysate, and the proteins were extracted. The protein concentration was measured using a BCA assay kit. 5× concentrated protein buffer was loaded, heated at 100 °C for 5 min, and then the sample was stored at -20 °C.
   (4) For each well, 30 µg of protein was loaded to polyacrylamide gel for electrophoresis.
   (5) The protein was transferred from polyacrylamide gel to PVDF membrane, and sealed at room temperature with 5% skimmed milk for 1 h, which was then incubated with the primary antibodies (Anti-SHP2 rabbit mAb and Anti-GAPDH rabbit mAb) at 4 °C overnight. The membrane was washed three times with TBST solution, 10 minutes for each time. After that, the membrane was incubated with the secondary antibodies (horseradish peroxidase labeled goat anti-rabbit IgG) at room temperature for 2 h, and then washed three times with TBST solution, 10 minutes for each time, followed by exposure.
   (6) The images were analyzed with Image J grayscale, and the degradation ratio of protein bands in the test group was calculated, based on the negative control group (DMSO) (100%).
   (7) Using the Dose-response-inhibition equation in the data processing software GraphPad Prism 8, the DC50 value was obtained (which represents the drug concentration corresponding to 50% degradation of the target protein).
3. Results:
   Finally, ECL detection solution was added for color development, and photos were taken with an automatic chemiluminescence instrument, so as to collect images and analyze them.
   Using similar methods and different cell lines, the degradation activity of the compound according to the present invention on SHP2 protein of different cell lines was tested under suitable culture conditions.

The results are shown in Table 2: (++++: DC50 > 1 µM; +++: 0.1 µM < DC50 < 1 µM; ++: DC50 < 0.1 µM)

**Table 2. The degradation activity of the compound according to the present invention on SHP2 protein of different cell lines.**

| | Degradation activity for differenct cell lines | | |
|---|---|---|---|
| Compound No. | MV41 1 | NCI-H358 | MIA Paca-2 |
| **1** | ++++ | | |
| **2** | +++ | | |
| **3** | +++ | | |
| **4** | +++ | | |
| **5** | ++++ | | |
| **7** | +++ | | |
| **8** | ++++ | | |
| **9** | ++++ | | |
| **10** | ++++ | | |
| **11** | ++++ | | |
| **12** | ++ | | |
| **13** | ++ | | |
| **14** | ++ | | |
| **15** | ++ | | |
| **16** | ++ | | |
| **17** | ++++ | | |
| **18** | ++++ | | |
| **19** | ++++ | | |
| **20** | ++++ | | |
| **21** | ++++ | | |
| **22** | ++++ | | |
| **24** | ++++ | | |
| **25** | ++++ | | |
| **26** | ++++ | | |
| **27** | ++ | | |
| **28** | ++ | | |
| **29** | ++++ | | |
| **30** | ++++ | | |
| **31** | ++++ | | |
| **34** | ++ | | |
| **35** | ++++ | | |
| **36** | ++++ | | |
| **37** | ++++ | | |
| **38** | ++ | | |
| **39** | ++ | | |
| **40** | ++ | | |
| **41** | ++++ | | |
| **42** | ++++ | | |
| **43** | ++++ | | |
| **44** | +++ | | |
| **45** | +++ | | |
| **46** | ++ | ++++ | |
| **47** | ++ | | |
| **48** | ++ | | ++ |
| **49** | ++ | | |
| **50** | ++ | | |
| **51** | ++ | | |
| **52** | ++ | | |
| **53** | ++++ | | |
| **54** | ++ | | |
| **55** | ++ | | ++++ |
| **56** | ++++ | | |
| **57** | ++++ | | |
| **58** | +++ | | |
| **59** | ++++ | | |
| **60** | ++++ | | |
| **61** | ++++ | | |
| **62** | +++ | | |
| **63** | ++ | ++ | ++ |
| **64** | ++ | +++ | +++ |
| **65** | ++ | | |
| **66** | ++ | | |
| **67** | ++++ | | |
| **68** | ++ | | |
| **69** | ++++ | | |
| **70** | ++ | | |
| **71** | ++ | | |
| **72** | ++ | | |
| **73** | ++ | | |
| **74** | ++ | | +++ |
| **75** | ++ | ++ | ++ |
| **77** | ++ | | |
| **78** | ++ | ++ | ++ |
| **79** | ++ | ++ | +++ |
| **80** | ++ | | |
| **81** | ++ | | |
| **82** | ++ | | |
| **83** | ++++ | | |
| **84** | ++++ | | |
| **85** | ++++ | | |
| **86** | ++ | ++ | ++ |
| **87** | ++ | | |
| **88** | ++ | | |
| **89** | ++ | | |
| **90** | ++ | | ++ |
| **91** | ++ | | +++ |
| **92** | ++++ | | |
| **93** | ++ | | |
| **94** | ++++ | | |
| **95** | ++ | | |
| **96** | ++ | | |
| **97** | ++++ | | |
| **98** | ++ | | +++ |
| **99** | ++ | | |
| **100** | ++ | ++ | ++ |
| **101** | ++ | | |
| **102** | ++ | | ++ |
| **103** | ++ | | |
| **104** | ++ | | ++++ |
| **105** | ++ | | +++ |
| **106** | ++ | | |
| **107** | ++ | | ++++ |
| **108** | ++ | | |
| **109** | ++ | | |
| **110** | ++ | +++ | +++ |
| **111** | ++++ | | |
| **112** | ++ | | ++++ |
| **113** | ++++ | | |
| **114** | ++++ | | |
| **115** | ++++ | | |
| **116** | ++++ | | |
| **117** | ++++ | | |
| **118** | +++ | | |
| **119** | ++ | ++ | ++ |
| **120** | ++ | | |
| **121** | ++ | | |
| **122** | ++ | | |
| **123** | +++ | | |
| **124** | ++ | | |
| **125** | ++++ | | |
| **126** | +++ | | |
| **127** | +++ | | |
| **128** | ++ | | |
| **129** | ++++ | | |
| **130** | ++ | | |
| **131** | +++ | | |
| **132** | ++ | | |
| **133** | ++++ | | |
| **134** | +++ | | |
| **135** | ++ | | |
| **136** | ++++ | | |
| **137** | ++++ | | |
| **138** | ++ | ++ | ++ |
| **139** | ++++ | | |
| **140** | ++++ | | ++++ |
| **142** | ++++ | | |
| **143** | ++ | | |
| **144** | +++ | | |
| **145** | ++ | | |
| **146** | ++ | | ++ |
| **147** | ++ | ++ | ++ |
| **148** | ++ | | |
| **149** | ++ | | |
| **150** | ++++ | | |
| **151** | ++++ | | |
| **152** | ++ | | |
| **153** | ++ | | |
| **155** | ++ | | ++ |
| **157** | ++ | | ++++ |
| **158** | +++ | | |
| **159** | ++ | | |
| **160** | +++ | | ++++ |
| **161** | ++ | +++ | ++ |
| **162** | ++ | | |
| **163** | ++ | | |
| **164** | ++ | | |
| **165** | ++ | | |
| **166** | ++ | | ++ |
| **167** | ++ | | |
| **168** | ++ | +++ | ++ |
| **169** | ++ | | |
| **170** | ++ | | +++ |
| **171** | ++ | | |
| **172** | ++ | | |
| **173** | ++ | | |
| **174** | ++ | ++ | ++ |
| **175** | ++ | | |
| **176** | ++ | +++ | ++ |
| **178** | ++ | | ++ |
| **179** | ++ | | ++++ |
| **180** | ++ | | |
| **181** | ++ | ++ | ++ |
| **182** | ++ | | |
| **183** | ++ | | ++++ |
| **184** | ++ | | |
| **185** | ++ | | +++ |
| **186** | ++ | ++ | ++ |
| **187** | ++++ | | |
| **188** | ++ | ++ | ++ |
| **189** | ++ | | ++++ |
| **190** | +++ | | ++++ |
| **191** | ++ | | ++++ |
| **192** | ++ | | ++++ |
| **193** | ++++ | | |
| **194** | ++ | | |
| **195** | +++ | | |
| **196** | ++++ | | |
| **197** | ++++ | | |
| **198** | ++++ | | |
| **199** | ++ | | ++ |
| **200** | ++ | ++ | ++ |
| **201** | +++ | | |
| **202** | ++ | | |
| **203** | ++++ | | |
| **204** | ++++ | | |
| **205** | ++++ | | |
| **206** | ++++ | | |
| **207** | ++ | ++ | ++ |
| **208** | ++ | | ++++ |
| **209** | ++ | | ++++ |
| **210** | ++ | | |
| **211** | ++ | | ++ |
| **212** | | | ++++ |
| **213** | | | ++++ |
| **214** | | | ++++ |
| **215** | | | ++++ |
| **216** | | ++ | ++ |
| **217** | | ++ | ++ |
| **218** | | ++ | ++ |
| **219** | | ++ | ++ |
| **220** | | ++ | ++ |
| **221** | | ++ | ++ |
| **222** | | ++ | ++ |
| **223** | | ++ | ++ |
| **224** | | ++ | ++ |
| **225** | | ++ | ++ |
| **226** | | ++ | ++ |
| **227** | | ++ | ++ |
| **228** | | ++ | ++ |
| **229** | | ++ | ++ |
| **230** | | ++ | ++ |
| **231** | | ++++ | ++++ |
| **232** | | ++ | ++ |
| **233** | | ++ | ++ |
| **234** | | ++ | ++ |
| **235** | | ++ | |
| **236** | | ++ | |
| **237** | | ++ | ++ |
| **238** | | ++ | ++ |
| **239** | | ++ | ++ |
| **240** | | ++++ | |
| **241** | | ++ | ++ |
| **242** | | ++ | ++ |
| **243** | | ++ | ++ |
| **244** | | ++ | ++ |
| **245** | | ++ | ++ |
| **246** | | ++ | ++ |
| **247** | | +++ | ++++ |
| **248** | | ++ | ++ |
| **249** | | ++ | ++ |
| **250** | | ++ | ++ |
| **251** | | ++ | ++ |
| **252** | | ++ | ++ |
| **253** | | | ++++ |
| **254** | | | ++++ |
| **255** | | | ++++ |
| **256** | | | ++++ |
| **257** | | | ++++ |
| **258** | | | ++++ |
| **259** | | | ++++ |
| **260** | | | ++++ |
| **261** | | | ++ |
| **262** | | | ++++ |
| **263** | | | ++ |
| **264** | | ++ | ++ |
| **265** | | | ++ |
| **266** | | +++ | ++++ |
| **267** | | | ++ |
| **268** | | | ++++ |
| **269** | | | ++++ |
| **270** | | | ++++ |
| **271** | | | ++++ |
| **272** | | | ++++ |
| **273** | | | ++++ |
| **274** | | | ++++ |
| **275** | | | ++++ |
| **276** | | | ++++ |
| **277** | | | ++++ |
| **278** | | | ++++ |
| **279** | | | ++++ |
| **280** | | | ++++ |
| **281** | | | ++++ |
| **282** | | | ++++ |
| **283** | | | ++++ |
| **284** | | | ++ |
| **285** | | | ++++ |
| **286** | | | ++++ |
| **287** | | | ++++ |
| **288** | | | ++++ |
| **289** | | | ++++ |
| **290** | | | ++++ |
| **291** | | | ++++ |
| **294** | | | ++ |
| **295** | | | ++ |
| **296** | | | ++ |
| **297** | | | ++++ |
| **298** | | | ++++ |
| **299** | | | ++++ |
| **300** | | | ++++ |
| **301** | | | ++++ |
| **302** | | | ++++ |
| **303** | | | ++ |
| **304** | | | ++ |
| **305** | | | ++++ |
| **306** | | | ++++ |
| **307** | | | ++ |
| **308** | | | ++ |
| **309** | | | ++ |
| **310** | | | ++ |
| **311** | | | ++++ |
| **312** | | | ++ |
| **313** | | | ++ |
| **314** | | | ++++ |
| **315** | | | ++++ |
| **316** | | | ++++ |
| **318** | | | ++++ |
| **319** | | | ++++ |
| **320** | | | ++++ |
| **321** | | | ++ |
| **322** | | | ++++ |
| **323** | | | ++++ |
| **324** | | | ++ |
| **325** | | | ++ |
| **326** | | | ++ |
| **327** | | | ++ |
| **328** | | | ++ |
| **329** | | | ++ |
| **330** | | | ++ |
| **331** | | | ++++ |
| **332** | | | ++++ |
| **333** | | | ++++ |
| **335** | | | ++++ |
| **336** | | | ++ |
| **337** | | | ++ |
| **338** | | | ++ |
| **339** | | | ++ |
| **340** | | | ++++ |
| **343** | | ++++ | ++ |
| **344** | | ++++ | ++ |
| **357** | | ++ | ++ |
| **358** | | | ++++ |
| **370** | | ++ | +++ |
| **371** | | ++ | +++ |
| **365** | | ++ | +++ |
| **375** | | ++ | ++++ |
| **377** | | ++ | ++ |
| **341** | | | ++++ |
| **359** | | | ++ |
| **376** | | | ++++ |
| **372** | | | ++ |
| **392** | | | ++ |
| **368** | | | ++ |
| **389** | | | ++ |
| **395** | | ++ | ++ |
| **396** | | ++ | ++ |
| **397** | | ++ | ++ |
| **398** | | ++ | ++ |
| **399** | | ++ | ++ |
| **400** | | ++ | ++ |
| **401** | | ++ | ++ |
| **402** | | | ++ |
| **403** | | | ++ |
| **404** | | | ++++ |
| **405** | | ++ | +++ |
| **406** | | ++++ | ++++ |
| **407** | | | ++++ |
| **408** | | | ++++ |
| **409** | | ++ | ++ |
| **410** | | ++ | ++ |
| **411** | | | ++ |
| **412** | | | ++ |
| **413** | | | ++ |
| **414** | | | ++ |
| **415** | | | ++++ |
| **416** | | | ++++ |
| **417** | | | ++++ |
| **418** | | ++ | ++ |
| **419** | | ++ | ++ |
| **420** | | | ++ |
| **421** | | ++ | ++ |
| **422** | | ++ | ++ |
| **423** | | | ++++ |
| **424** | | | ++++ |
| **425** | | | ++++ |
| **426** | | | ++++ |
| **427** | | | ++++ |
| **428** | | | +++ |
| **429** | | | +++ |
| **430** | | | +++ |
| **431** | | | +++ |
| **432** | | | ++++ |
| **433** | | | ++++ |
| **434** | | | ++ |
| **436** | | | +++ |
| **437** | | | ++ |
| **438** | | | ++ |
| **439** | | ++ | ++ |
| **440** | | | ++++ |
| **441** | | | ++++ |
| **442** | | +++ | +++ |
| **443** | | ++ | +++ |
| **444** | | | ++ |
| **445** | | ++ | +++ |
| **446** | | ++ | ++ |
| **447** | | | ++++ |
| **448** | | ++ | ++ |
| **449** | | | ++ |
| **450** | | | ++ |
| **451** | | ++ | ++ |
| **452** | | | ++ |
| **453** | | | +++ |
| **454** | | | +++ |
| **455** | | | ++ |
| **456** | | | +++ |
| **457** | | ++ | ++ |
| **458** | | | ++++ |
| **459** | | | ++ |
| **460** | | | ++ |
| **461** | | | ++ |
| **462** | | | ++++ |
| **463** | | | ++++ |
| **464** | | | ++ |
| **465** | | ++ | ++ |
| **466** | | | ++ |
| **467** | | +++ | ++ |
| **468** | | | ++ |
| **469** | | | +++ |
| **470** | | | +++ |
| **471** | | | +++ |
| **472** | | | +++ |
| **473** | | | ++ |
| **474** | | | ++ |
| **475** | | | ++++ |
| **476** | | | ++++ |
| **477** | | | ++ |
| **478** | | | ++ |
| **479** | | | +++ |
| **480** | | | ++ |
| **481** | | | ++ |
| **482** | | | ++ |
| **483** | | | ++ |
| **484** | | | ++ |
| **485** | | | ++ |
| **486** | | | ++ |
| **488** | | | ++ |
| **489** | | | ++++ |
| **490** | | | +++ |
| **491** | | | ++++ |
| **492** | | | ++ |
| **493** | | | ++ |
| **494** | | | ++ |
| **495** | | | ++ |
| **496** | | | ++ |
| **497** | | | ++ |
| **498** | | | +++ |
| **499** | | | ++ |
| **500** | | | ++++ |
| **501** | | | ++ |
| **502** | | | ++ |
| **503** | | | ++ |
| **504** | | | ++ |
| **505** | | | ++ |
| **506** | | | ++ |
| **507** | | | ++ |
| **508** | | | ++ |
| **509** | | | ++ |
| **510** | | | ++ |
| **511** | | | ++ |
| **512** | | | +++ |
| **513** | | | ++ |
| **514** | | | ++++ |
| **515** | | | ++ |
| **516** | | | ++ |
| **518** | | | +++ |
| **519** | | | ++ |
| **520** | | | ++ |
| **999** | ++ | ++ | ++ |

The results in Table 2 above indicated that the compound of the present invention had a good degradation effect on SHP2 protein in different cells.

### Experiment Example 2: Biological assay of the inhibitory effect of the compound according the present invention on cell proliferation

### Experimental materials

MV-411 cell line (COBIER, CBP-60522)
FBS (GEMINI, Cat. No. 900-108)
0.01M PBS (Biosharp, Cat. No. 162262)
INMM (Hyclone, Cat. No. SH30228.01)
Penicillin-Streptomycin (Gibco, Cat. No. 15140122)
DMSO (Sigma, Cat. No. D5879)
Cell counting kit-8(Signalway Antibody, Cat. No. CP002)
Centrifuge Tube, 15 ml (Excell Bio, Cat. No. CS015-0001)
Cell Culture Dish, (WHB, Cat. No. CS016-0128)
96-well cell culture cluster (Corning, Cat. No. 3599)

### Expermental methods

1. Preparation of buffer
   (1) Cell culture medium: IMDM medium + 10% FBS + 1% Pen Strep;
   (2) PBS buffer: PBS powder was dissolved in 2 L of ultrapure water and sterilized;
2. Experimental procedures:
   (1) MV-411 cells were cultured in the cell culture medium, and then well-growing cells were selected and inoculated in a 96-well plate at 80 µL/well, with 2 × 10⁴ cells/well. The plate was incubated overnight in a 5% CO₂ cell incubator at 37 °C.
   (2) 10 mM storage solution of each drug was prepared using dimethylsulfoxide (DMSO).
      The stock solution was diluted in a ratio of 1:3 with DMSO before use, and then the solution was serially diluted in a ratio of 1:3, to obtain 9 gradient concentrations. Each concentration was futher diluted with the medium in a ratio of 1:200 (to ensure that the DMSO concentration in the culture system was 0.1%). Two wells were set for each concentration. 20 µL of each compound solution was added to the cell culture well (with a final concentration of 10 µM, 3.3 µM, 1.1 µM...). The plate was gently shaken and mixed. Additionally, 3 negative control wells (only containing cells) and 3 blank control wells (only containing the medium) were included (6 wells were each added with 20 µL of DMSO diluted with the medium in a ratio of 1:200).
3. Results:
   (1) After 5 days of cultivation, 10 µL of CCK-8 was added to each well, and then the plate was further cultured for 3 h at 37 °C in a 5% CO₂ cell incubator.
   (2) The absorbance (OD value) was measured at 450 nm with a multifunctional microplate reader.
   (3) The data were analyzed using the Dose-response-inhibition equation in GraphPad Prism8 software to obtain the IC₅₀ value.

Using a similar method, the IC₅₀ value (nM) for the inhibitory activity of the compound according to the present invention against different cell lines was obtained, and the results are shown in Table 3 (++++: IC₅₀ > 5 µM; +++: 1 µM <IC₅₀ < 5 µM; ++: IC₅₀ <1 µM).

**Table 3. The inhibitory effect of the compound according to the present invention on the proliferation activity of different cell lines.**

| ID | Inhibition activity on the proliferation of different cell lines | | | | |
|---|---|---|---|---|---|
| | MV 411 | NCI-H358 | MIA Paca-2 | NCI-H1975 | KYSE 520 |
| 63 | ++ | ++ | +++ | | |
| 96 | | ++ | ++ | | |
| **110** | | ++++ | ++++ | | |
| **111** | ++ | +++ | +++ | | |
| **119** | ++ | ++ | +++ | | |
| **138** | ++ | | | | |
| **161** | ++ | +++ | +++ | | |
| **168** | ++ | +++ | +++ | | |
| **175** | | | +++ | | |
| **181** | | | +++ | | |
| **174** | +++ | +++ | +++ | | |
| **403** | | | | +++ | |
| **412** | +++ | | | ++ | +++ |
| **414** | ++++ | | | | +++ |
| **421** | ++ | | | | +++ |
| **422** | +++ | | | | ++++ |
| **482** | | | | ++ | |
| **485** | | | | ++ | |
| **500** | ++ | | | | +++ |
| **501** | ++ | | | +++ | +++ |
| **514** | ++ | | | | +++ |
| **999** | ++ | +++ | +++ | | +++ |
| **519** | ++ | | | | +++ |

The results in Table 3 above indicated that compounds of the present invention, such as **63, 96,** etc., exhibited excellent anti-proliferative activity against different cell lines.

In summary, the compound of the present invention had a good inhibitory effect on both hematomas and solid tumor cell lines. It had strong inhibitory effects on the proliferation of acute leukemia, esophageal cancer, KRAS mutant non-small cell lung cancer and pancreatic cancer cell lines. Moreover, when it was combined with other anti-tumor medicaments, a significant synergistic effect was demonstrated. In addition, the compound of the present invention had a rather different mechanism of action compared to traditional small-molecule targeting drugs or macromolecular drugs such as antibodies, and had good application prospects. The compound of the present invention could be used as a phosphatase degrader, especially as a SHP2 protein degrader, so that it could be used in the manufacturer of medicaments for treating diseases such as cancer, and had good application prospects.

## Claims

1. A compound represented by formula I, or a salt thereof, or a deuterated compound thereof, or a stereoisomer thereof, or a solvate thereof, or a hydrate thereof, or a prodrug thereof: 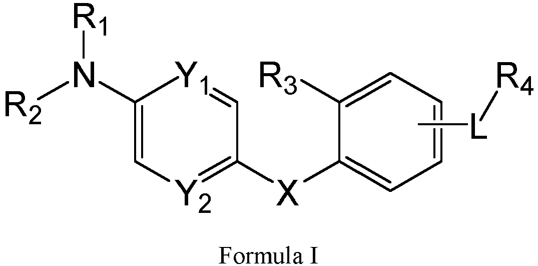 wherein,
R₁ and R₂, together with the N atom to which they are attached, form a 5-10 membered heterocyclic group substituted with 0-5 R₅;
each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
each R₆ is independently selected from the group consisting of hydroxyl, amino, halogen, carboxyl, nitro, cyano, C₁-C₈ alkoxy, and -N(H)R₇;
R₇ is selected from the group consisting of C₁-C₈ alkyl substituted with 0-5 R₉, C₁-C₈ alkoxy, and tert-butoxycarbonyl;
Rs is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy;
Y₁ and Y₂ are each independently selected from -N- or -CH-; and at least one of Y₁ and Y₂ is selected from -N-;
X is selected from -S- or absence;
R₃ is selected from the group consisting of hydrogen, C₁-C₈ alkyl, C₁-C₈ alkoxy, halogen, hydroxyl, carboxyl, amino, nitro, and cyano;
R₄ is selected from the group consisting of 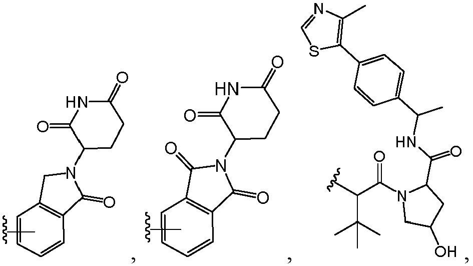 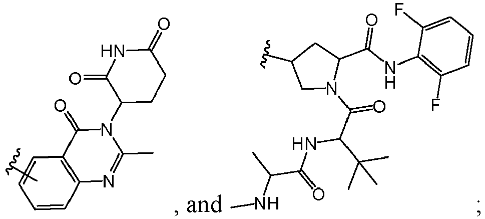
L is connected to phenyl ring at any position, and selected from the group consisting of 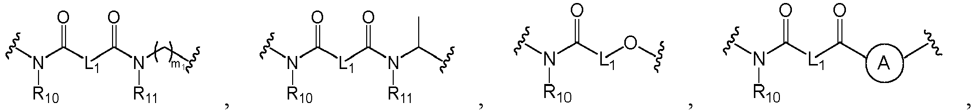 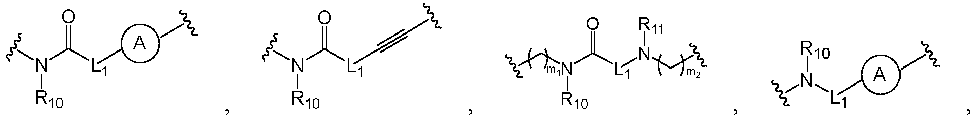 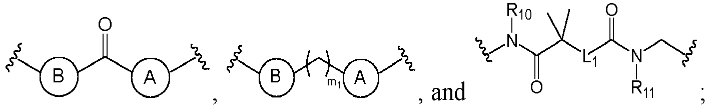
m₁ is an integer from 0 to 15;
m₂ is an integer from 0 to 15;
R₁₀ and R₁₁ are each independently selected from Hand C₁-C₈ alkyl;
L₁ is selected from the group consisting of 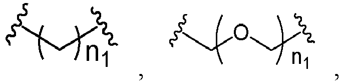 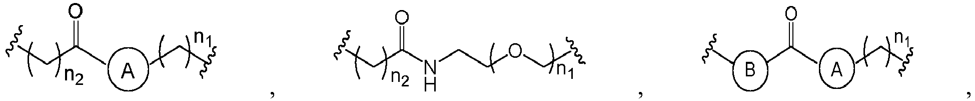 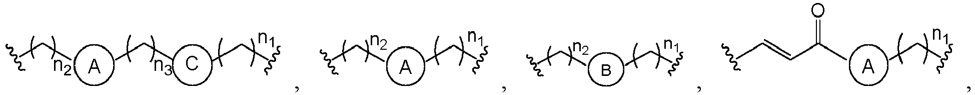 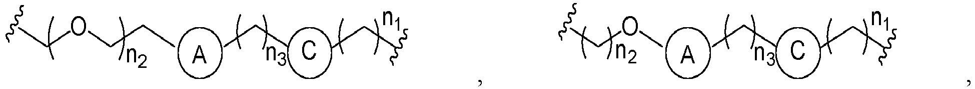 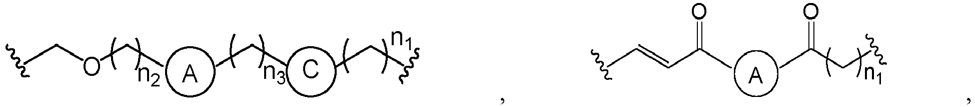 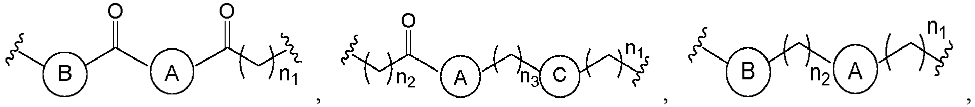 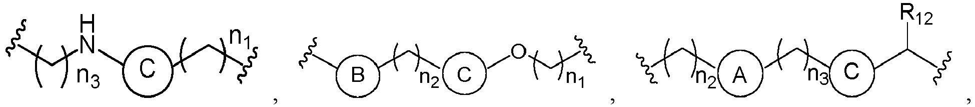 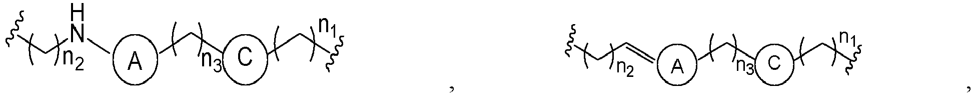 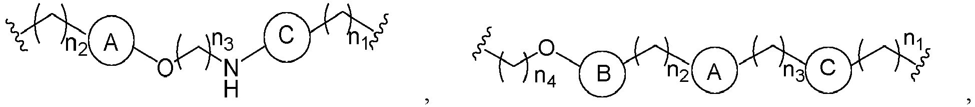 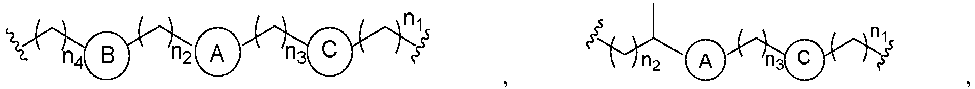 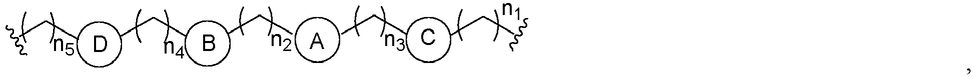 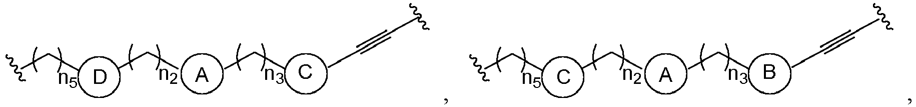 and 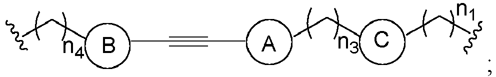
n₁ is an integer from 0 to 15;
n₂ is an integer from 0 to 15;
n₃ is an integer from 0 to 15;
n₄ is an integer from 0 to 15;
n₅ is an integer from 0 to 15;
R₁₂ is selected from C₁-C₈ alkyl and trifluoromethyl.
Ring A is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
Ring B is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
Ring C is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
Ring D is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
Each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
Each R₁₄ is independently selected from the group consisting of hydroxyl, carboxyl, halogen, and amino.

2. The compound according to claim 1, or a salt thereof, or a deuterated compound thereof, or a stereoisomer thereof, or a solvate thereof, or a hydrate thereof, or a prodrug thereof, **characterized in that**:
R₁ and R₂, together with the N atom to which they are attached, form piperidyl substituted with 0-2 R₅, 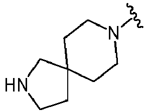 substituted with 0-2 R₅, 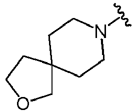 substituted with 0-2 R₅, 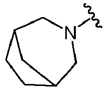 substituted with 0-2 R₅, 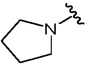 substituted with 0-2 R₅, and 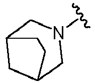 substituted with 0-2 R₅;
each R₅ is independently selected from the group consisting of C₁-C₈ alkyl substituted with 0-2 R₆, C₁-C₈ alkoxy, carboxyl, nitro, cyano, amino, halogen, hydroxyl, -N(H)C(O)R₇, -N(H)R₇, and -C(O)Rs;
each R₆ is independently selected from the group consisting of hydroxyl, amino, halogen, carboxyl, nitro, cyano, C₁-C₈ alkoxy, and -N(H)R₇;
R₇ is selected from the group consisting of C₁-C₈ alkyl substituted with 0-5 R₉, C₁-C₈ alkoxy, and tert-butoxycarbonyl;
Rs is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy;
Y₁ and Y₂ are each independently selected from -N- or -CH-; and at least one of Y₁ and Y₂ is selected from -N-;
X is selected from -S- or absence;
R₃ is selected from the group consisting of hydrogen, C₁-C₈ alkyl, C₁-C₈ alkoxy, halogen, hydroxyl, carboxyl, amino, nitro, and cyano;
R₄ is selected from the group consisting of 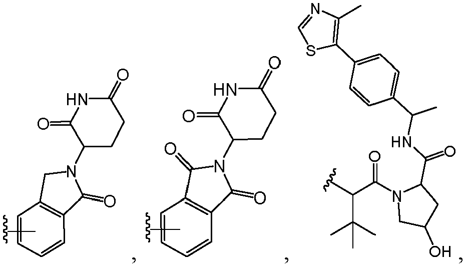 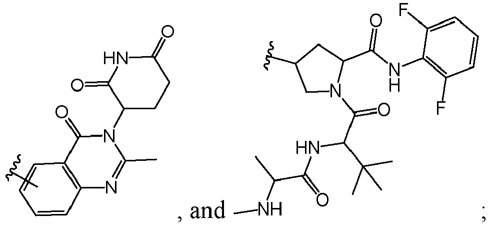
L is connected to phenyl ring at any position, and selected from the group consisting of 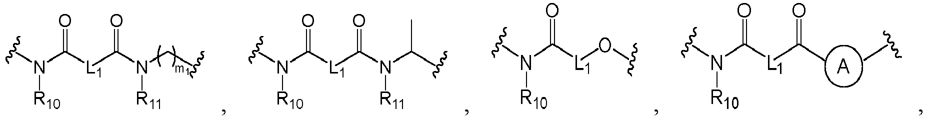 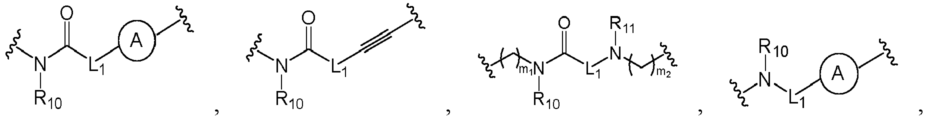 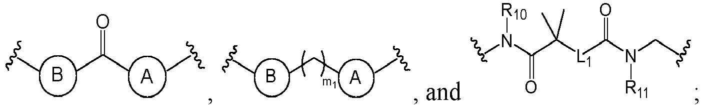
m₁ is an integer from 0 to 15;
m₂ is an integer from 0 to 15;
R₁₀ and R₁₁ are each independently selected from Hand C₁-C₈ alkyl;
L₁ is selected from the group consisting of 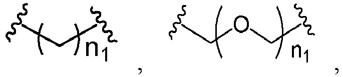 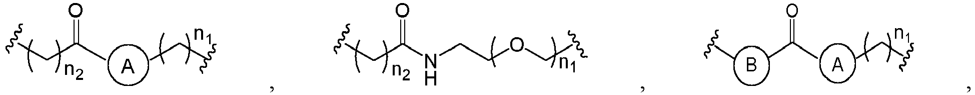 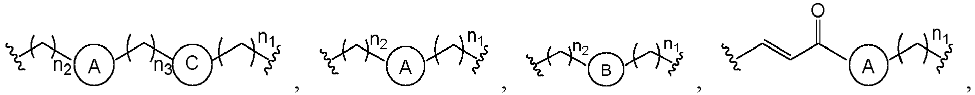 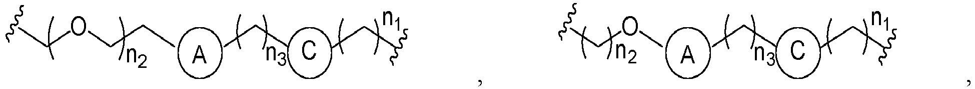 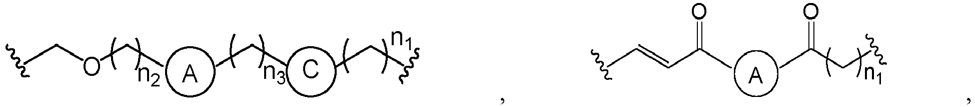 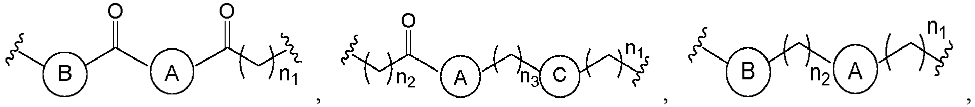 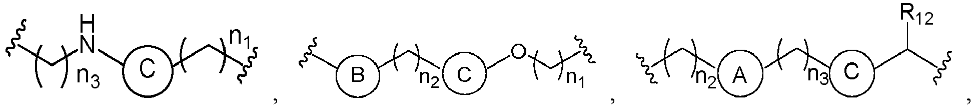 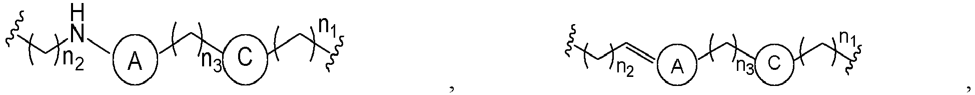 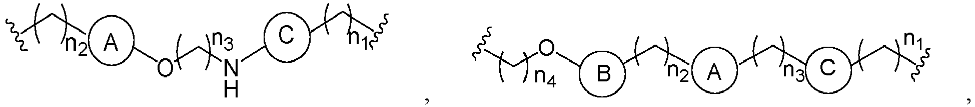 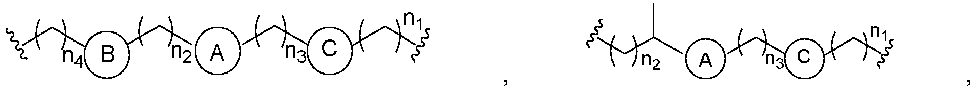 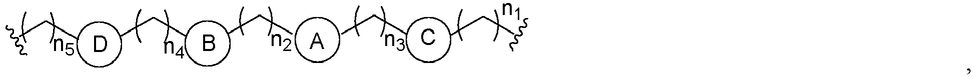 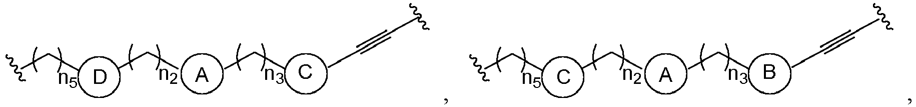 and 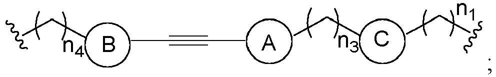
n₁ is an integer from 0 to 15;
n₂ is an integer from 0 to 15;
n₃ is an integer from 0 to 15;
n₄ is an integer from 0 to 15;
n₅ is an integer from 0 to 15;
R₁₂ is selected from C₁-C₈ alkyl and trifluoromethyl.
Each ring A is independently selected from the group consisting of 3-6 membered cycloalkyl substituted with 0-3 R₁₃, piperazinyl substituted with 0-3 R₁₃, piperidyl substituted with 0-3 R₁₃, azetidinyl substituted with 0-3 R₁₃, pyrrolidinyl substituted with 0-3 R₁₃, 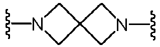 substituted with 0-3 R₁₃, 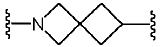 substituted with 0-3 R₁₃, 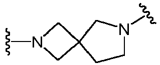 substituted with 0-3 R₁₃, 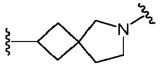 substituted with 0-3 R₁₃, 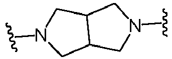 substituted with 0-3 R₁₃, 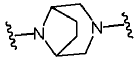 substituted with 0-3 R₁₃, 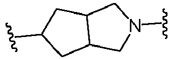 substituted with 0-3 R₁₃, 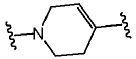 substituted with 0-3 R₁₃, 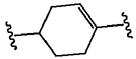 substituted with 0-3 R₁₃, phenyl substituted with 0-3 R₁₃, 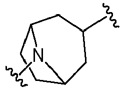 substituted with 0-3 R₁₃, 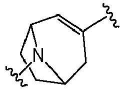 substituted with 0-3 R₁₃, 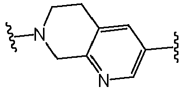 substituted with 0-3 R₁₃, 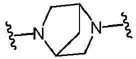 substituted with 0-3 R₁₃, 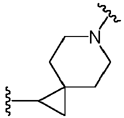 substituted with 0-3 R13, 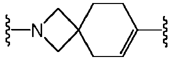 substituted with 0-3 R₁₃, 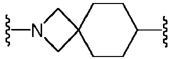 substituted with 0-3 R₁₃, 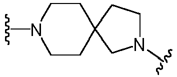 substituted with 0-3 R₁₃, and 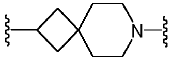 substituted with 0-3 R₁₃;
Each ring B is independently selected from the group consisting of 3-6 membered cycloalkyl substituted with 0-3 R₁₃, phenyl substituted with 0-3 R₁₃, piperidyl substituted with 0-3 R₁₃, pyrrolidinyl substituted with 0-3 R₁₃, piperazinyl substituted with 0-3 R₁₃, 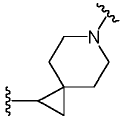 substituted with 0-3 R₁₃, 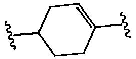 substituted with 0-3 R₁₃, 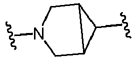 substituted with 0-3 R₁₃, 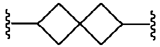 substituted with 0-3 R₁₃, 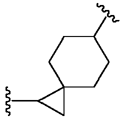 substituted with 0-3 R₁₃, and 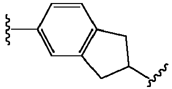 substituted with 0-3 R₁₃;
Each ring C is independently selected from the group consisting of phenyl substituted with 0-3 R₁₃, pyrimidinyl substituted with 0-3 R₁₃, pyridazinyl substituted with 0-3 R₁₃, pyrazolyl substituted with 0-3 R₁₃, and pyrazinyl substituted with 0-3 R₁₃;
Each ring D is independently selected from the group consisting of phenyl substituted with 0-3 R₁₃, thienyl substituted with 0-3 R₁₃, cycloalkyl substituted with 0-3 R₁₃, pyridyl substituted with 0-3 R₁₃, pyridazinyl substituted with 0-3 R₁₃, 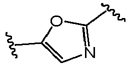 substituted with 0-3 R₁₃, 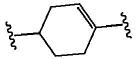 substituted with 0-3 R₁₃, 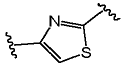 substituted with 0-3 R₁₃;
Each R₁₃ is independently selected from the group consisting of C₁-C₈ alkyl substituted with 0-3 R₁₄, halogen, trifluoromethyl, and C₁-C₈ alkoxy; or two R₁₃ attached to the same carbon atom form =O;
Each R₁₄ is independently selected from the group consisting of hydroxyl, carboxyl, halogen, and amino.

3. The compound according to claim 1, or a salt thereof, or a deuterated compound thereof, or a stereoisomer thereof, or a solvate thereof, or a hydrate thereof, or a prodrug thereof, **characterized in that** the compound is as represented by formula II: 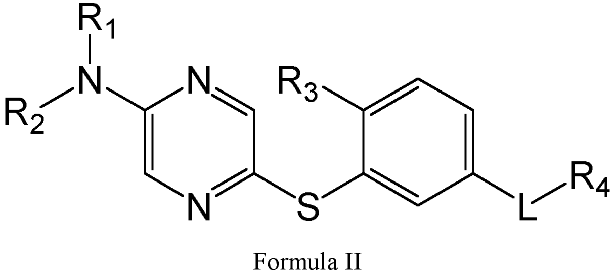 wherein,
R₁ and R₂, together with the N atom to which they are attached, form a 5-10 membered heterocyclic group substituted with 0-5 R₅;
each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
each R₆ is independently selected from the group consisting of hydroxyl, amino, halogen, carboxyl, nitro, cyano, C₁-C₈ alkoxy, and -N(H)R₇;
R₇ is independently selected from the group consisting of C₁-C₈ alkyl substituted with 0-5 R₉, C₁-C₈ alkoxy, and tert-butoxycarbonyl;
Rs is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy;
R₃ is selected from the group consisting of hydrogen, C₁-C₈ alkyl, C₁-C₈ alkoxy, halogen, hydroxyl, carboxyl, amino, nitro, and cyano;
R₄ is selected from the group consisting of 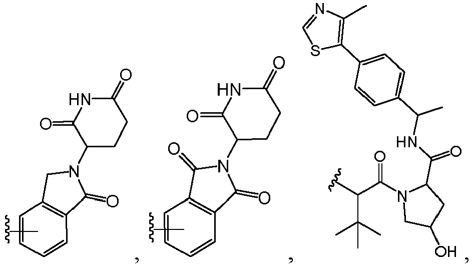 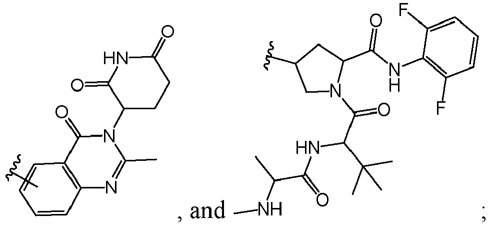
L is connected to phenyl ring at any position, and selected from the group consisting of 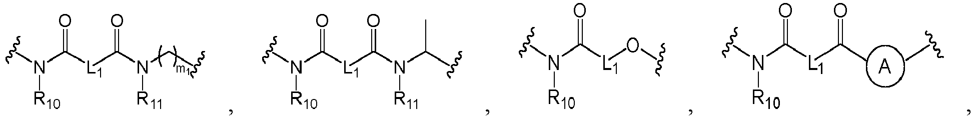 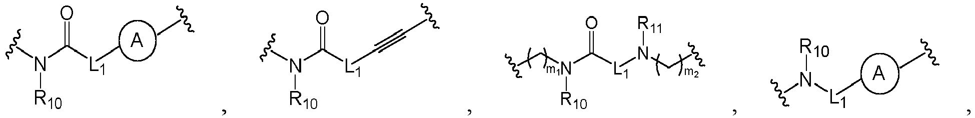 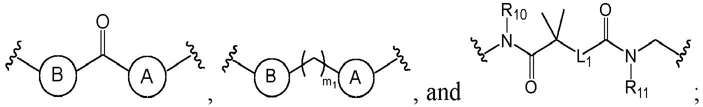
m₁ is an integer from 0 to 15;
m₂ is an integer from 0 to 15;
R₁₀ and R₁₁ are each independently selected from Hand C₁-C₈ alkyl;
L₁ is selected from the group consisting of 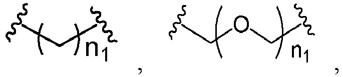 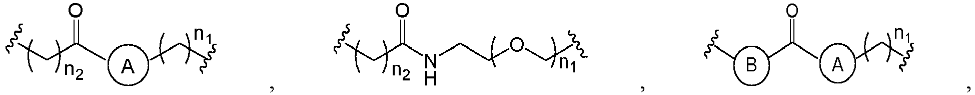 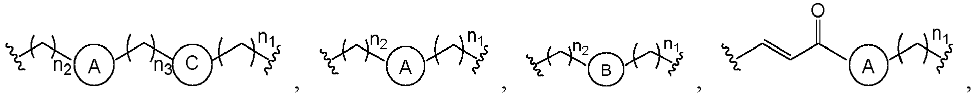 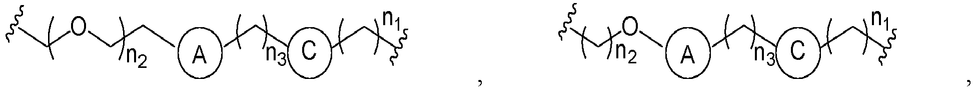 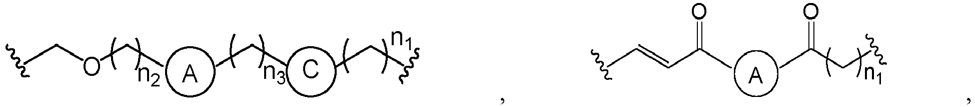 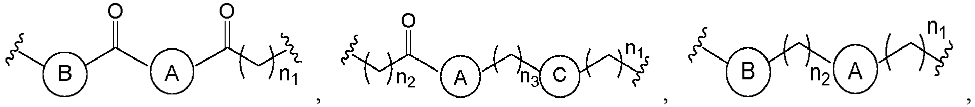 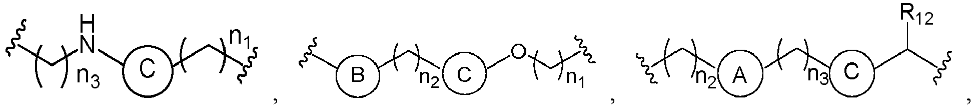 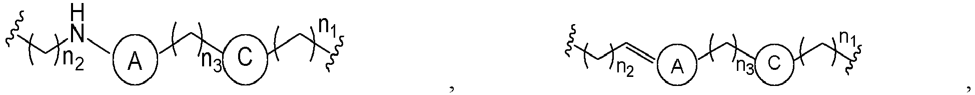 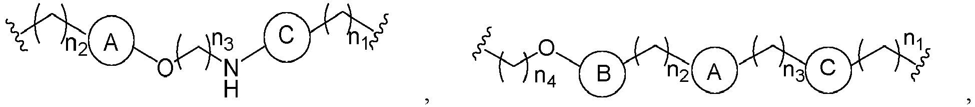 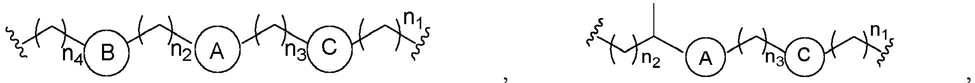 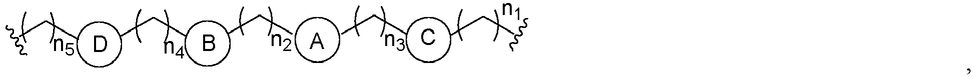 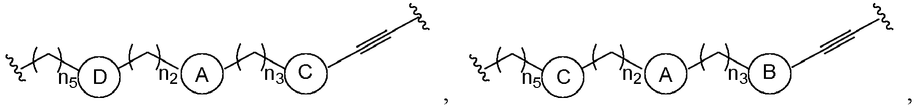 and 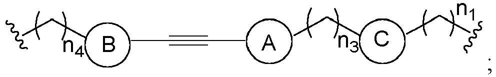
n₁ is an integer from 0 to 15;
n₂ is an integer from 0 to 15;
n₃ is an integer from 0 to 15;
n₄ is an integer from 0 to 15;
n₅ is an integer from 0 to 15;
R₁₂ is selected from C₁-C₈ alkyl and trifluoromethyl;
ring A is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring B is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring C is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring D is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino;
preferably,
R₁, R₂, R₃, R₄, and L are as defined in claim 2.

4. The compound according to claim 1, or a salt thereof, or a deuterated compound thereof, or a stereoisomer thereof, or a solvate thereof, or a hydrate thereof, or a prodrug thereof, **characterized in that** the compound is as represented by formula III: 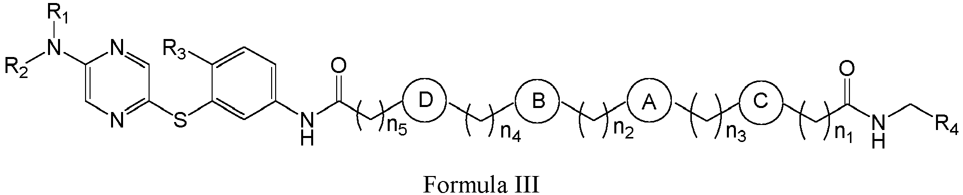 wherein,
R₁ and R₂, together with the N atom to which they are attached, form a 5-10 membered heterocyclic group substituted with 0-5 R₅;
each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
each R₆ is independently selected from the group consisting of hydroxyl, amino, halogen, carboxyl, nitro, cyano, C₁-C₈ alkoxy, and -N(H)R₇;
R₇ is independently selected from the group consisting of C₁-C₈ alkyl substituted with 0-5 R₉, C₁-C₈ alkoxy, and tert-butoxycarbonyl;
Rs is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy;
R₃ is selected from the group consisting of hydrogen, C₁-C₈ alkyl, C₁-C₈ alkoxy, halogen, hydroxyl, carboxyl, amino, nitro, and cyano;
R₄ is selected from the group consisting of 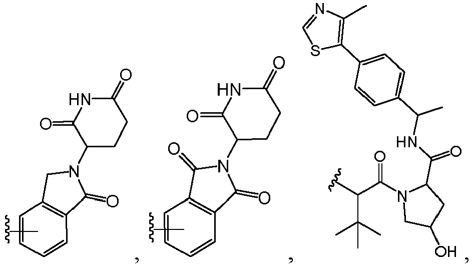 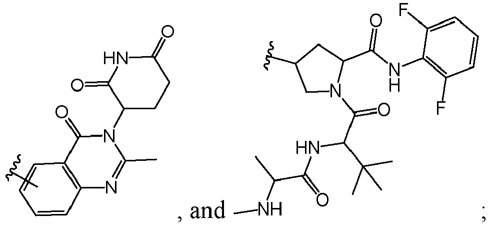
n₁ is an integer from 0 to 15;
n₂ is an integer from 0 to 15;
n₃ is an integer from 0 to 15;
n₄ is an integer from 0 to 15;
n₅ is an integer from 0 to 15;
ring A is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring B is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring C is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring D is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino;
preferably,
R₁, R₂, R₃, R₄, n₁, n₂, n₃, n₄, n₅, ring A, ring B, ring C, and ring D are as defined in claim 2.

5. The compound according to claim 1, or a salt thereof, or a deuterated compound thereof, or a stereoisomer thereof, or a solvate thereof, or a hydrate thereof, or a prodrug thereof, **characterized in that** the compound is as represented by formula IV: 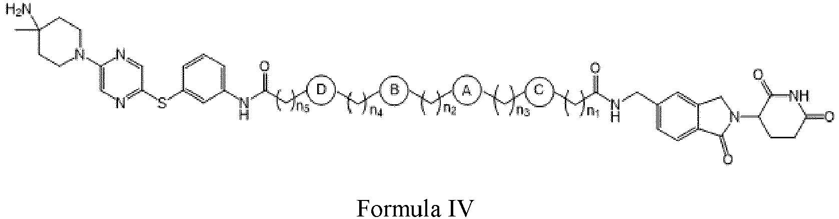 wherein,
n₁ is an integer from 0 to 15;
n₂ is an integer from 0 to 10;
n₃ is an integer from 0 to 10;
n₄ is an integer from 0 to 10;
n₅ is an integer from 0 to 10;
ring A is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring B is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring C is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring D is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino;
preferably,
n₁, n₂, n₃, n₄, n₅, ring A, ring B, ring C, and ring D are as defined in claim 2.

6. The compound according to claim 1, or a salt thereof, or a deuterated compound thereof, or a stereoisomer thereof, or a solvate thereof, or a hydrate thereof, or a prodrug thereof, **characterized in that** the compound is as represented by formula V: 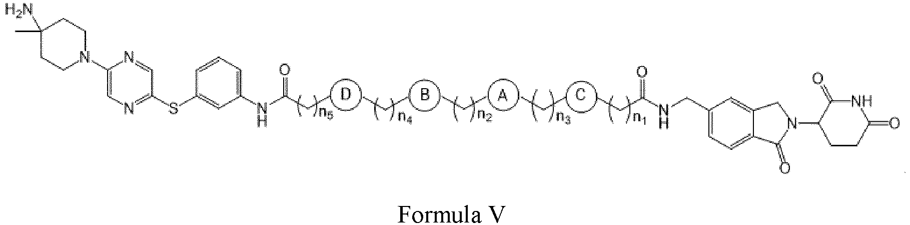 wherein,
ring A is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring B is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring C is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
ring D is selected from the group consisting of 4-10 membered heterocyclic group substituted with 0-5 R₁₃, 3-10 membered cycloalkyl substituted with 0-5 R₁₃, 5-10 membered aryl substituted with 0-5 R₁₃, and 5-10 membered heteroaryl substituted wtih 0-5 R₁₃;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino;
preferably,
ring A, ring B, ring C, and ring D are as defined in claim 2.

7. The compound according to claim 1, or a salt thereof, or a deuterated compound thereof, or a stereoisomer thereof, or a solvate thereof, or a hydrate thereof, or a prodrug thereof, **characterized in that**:
rings A and B are selected from 4-10 membered heterocyclic group substituted with 0-5 R₁₃, and 3-10 membered cycloalkyl substituted with 0-5 R₁₃;
rings C and D are selected from 5-10 membered aryl substituted with 0-5 R₁₃, and 5-10 membered heteroaryl substituted wtih 0-5 R₁₃;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino;
preferably,
each group is as defined in claim 2.

8. The compound according to claim 7, or a salt thereof, or a deuterated compound thereof, or a stereoisomer thereof, or a solvate thereof, or a hydrate thereof, or a prodrug thereof, **characterized in that** the compound is as represented by formula VI: 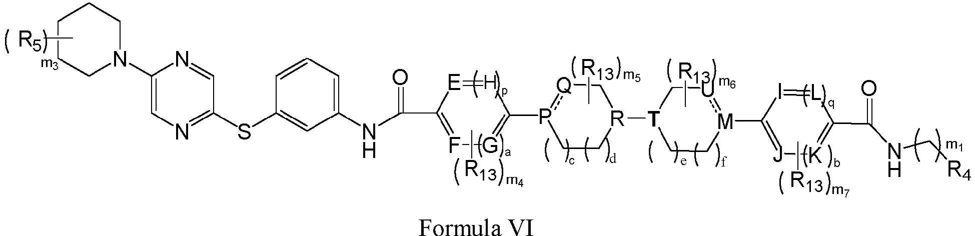 wherein,
Rs is a substituent in ring at any position, and each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
m₃ is an integer from 0 to 5;
each R₆ is independently selected from the group consisting of hydroxyl, amino, halogen, carboxyl, nitro, cyano, C₁-C₈ alkoxy, and -N(H)R₇;
R₇ is independently selected from the group consisting of C₁-C₈ alkyl substituted with 0-5 R₉, C₁-C₈ alkoxy, and tert-butoxycarbonyl;
Rs is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy;
R₄ is selected from the group consisting of 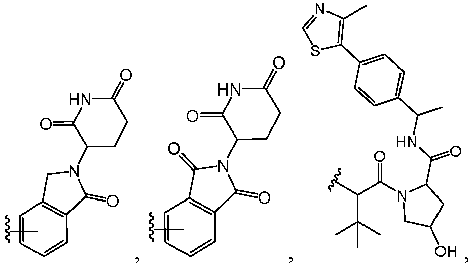 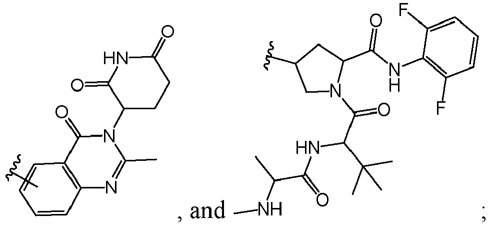
E, F, G, H, I, J, K, L, M, U, T, P, Q, and Rare C or N atoms;
the bond between U and M is a single or double bond;
the bond between Q and P is a single or double bond;
a, b, c, d, e, f, p, and q are each independently selected from an integer of 0 to 1;
m₄ is an integer from 0 to 5;
m₅ is an integer from 0 to 5;
m₆ is an integer from 0 to 5;
m₇ is an integer from 0 to 5;
m₁ is an integer from 0 to 15;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino.

9. The compound according to claim 8, or a salt thereof, or a deuterated compound thereof, or a stereoisomer thereof, or a solvate thereof, or a hydrate thereof, or a prodrug thereof, **characterized in that** the compound is as represented by formula VII: 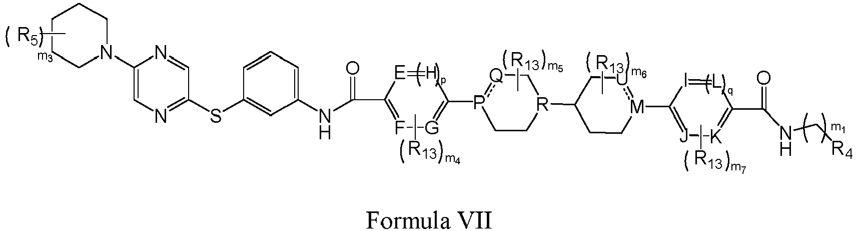 wherein,
R₅ is a substituent in ring at any position, and each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
m₃ is an integer from 0 to 5;
each R₆ is independently selected from the group consisting of hydroxyl, amino, halogen, carboxyl, nitro, cyano, C₁-C₈ alkoxy, and -N(H)R₇;
R₇ is independently selected from the group consisting of C₁-C₈ alkyl substituted with 0-5 R₉, C₁-C₈ alkoxy, and tert-butoxycarbonyl;
R₈ is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy;
R₄ is selected from the group consisting of 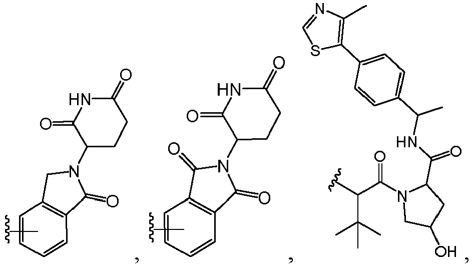 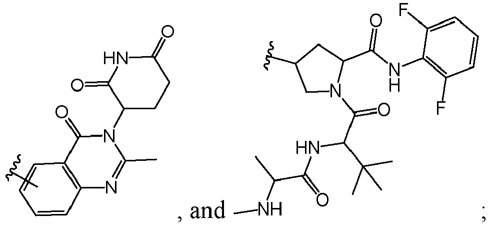
E, F, G, H, I, J, K, L, M, U, P, Q, and Rare C or N atom;
the bond between O and M is a single or double bond;
the bond between Q and P is a single or double bond;
p and q are each independently selected from an integer of 0 to 1;
m₄ is an integer from 0 to 5;
m₅ is an integer from 0 to 5;
m₆ is an integer from 0 to 5;
m₇ is an integer from 0 to 5;
m₁ is an integer from 0 to 15;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino.

10. The compound according to claim 1, or a salt thereof, or a deuterated compound thereof, or a stereoisomer thereof, or a solvate thereof, or a hydrate thereof, or a prodrug thereof, **characterized in that** the compound is as represented by formula VIII: 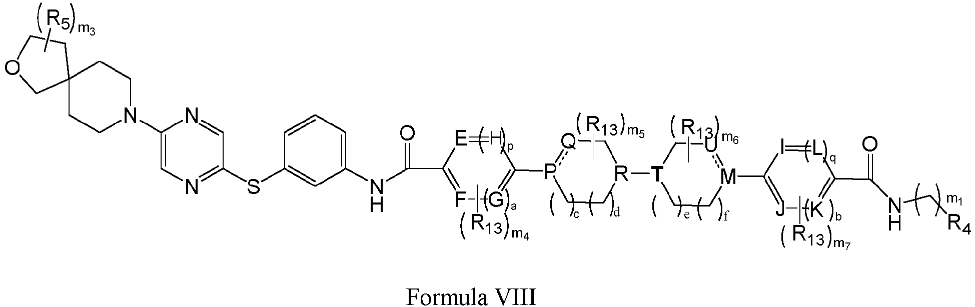 wherein,
R₅ is a substituent in ring at any position, and each R₅ is independently selected from the group consisting of C₁-C₈ alkyl substituted with 0-5 R₆, C₁-C₈ alkoxy, carboxyl, nitro, cyano, amino, halogen, hydroxyl, -N(H)C(O)R₇, -N(H)R₇, and -C(O)Rs;
m₃ is an integer from 0 to 2;
each R₆ is independently selected from the group consisting of hydroxyl, amino, halogen, carboxyl, nitro, cyano, C₁-C₈ alkoxy, and -N(H)R₇;
R₇ is independently selected from the group consisting of C₁-C₈ alkyl substituted with 0-5 R₉, C₁-C₈ alkoxy, and tert-butoxycarbonyl;
R₈ is selected from the group consisting of C₁-C₈ alkyl, amino, C₁-C₈ alkoxy, and tert-butoxycarbonyl;
each R₉ is independently selected from the group consisting of amino, hydroxyl, halogen, carboxyl, nitro, cyano, and C₁-C₈ alkoxy;
R₄ is selected from the group consisting of 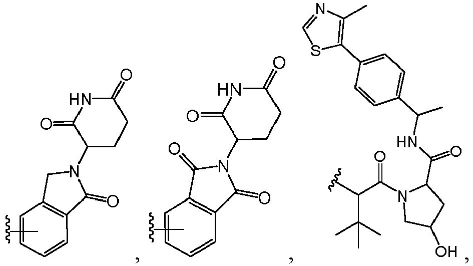 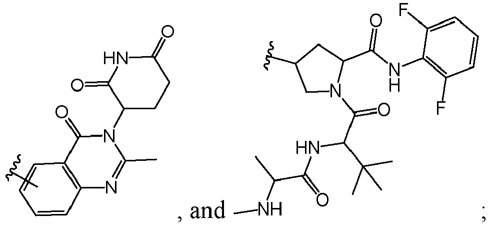
E, F, G, H, I, J, K, L, M, U, T, P, Q, and Rare C or N atom;
the bond between U and M is a single or double bond;
the bond between Q and P is a single or double bond;
a, b, c, d, e, f, p and q are each independently selected from an integer of 0 to 1;
m₄ is an integer from 0 to 5;
m₅ is an integer from 0 to 5;
m₆ is an integer from 0 to 5;
m₇ is an integer from 0 to 5;
m₁ is an integer from 0 to 15;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino.

11. The compound according to claim 10, or a salt thereof, or a deuterated compound thereof, or a stereoisomer thereof, or a solvate thereof, or a hydrate thereof, or a prodrug thereof, **characterized in that** the compound is as represented by formula IX: 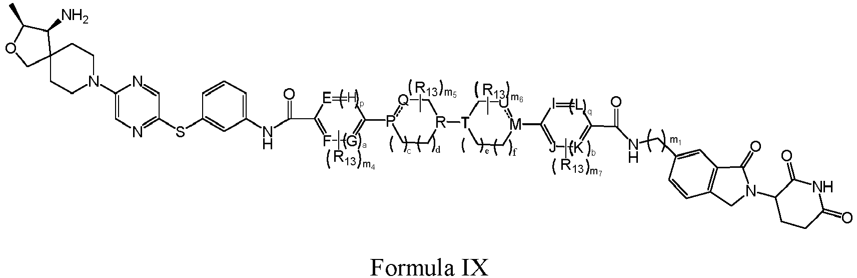 wherein,
E, F, G, H, I, J, K, L, M, U, T, P, Q, and Rare C or N atom;
the bond between U and M is a single or double bond;
the bond between Q and P is a single or double bond;
a, b, c, d, e, f, p and q are each independently selected from an integer of 0 to 1;
m₄ is an integer from 0 to 5;
m₅ is an integer from 0 to 5;
m₆ is an integer from 0 to 5;
m₇ is an integer from 0 to 5;
m₁ is an integer from 0 to 15;
each R₁₃ is independently selected from the group consisting of C₁-C₈ alkyl substituted with 0-5 R₁₄, halogen, trifluoromethyl, and C₁-C₈ alkoxy; or two R₁₃ attached to the same carbon atom form =O;
each R₁₄ is independently selected from the group consisting of hydroxyl, carboxyl, halogen, and amino.

12. The compound according to any one of claims 1 to 11, or a deuterated compound thereof, or a salt thereof, or a stereoisomer thereof, or a solvate thereof, or a hydrate thereof, or a prodrug thereof, **characterized in that** the compound is selected from the group consisting of:

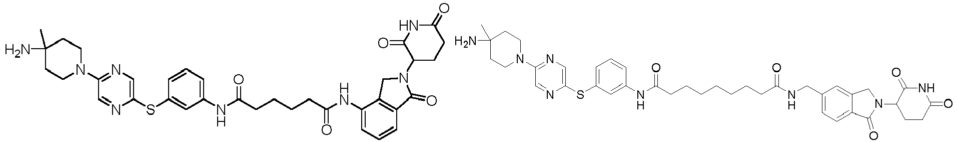

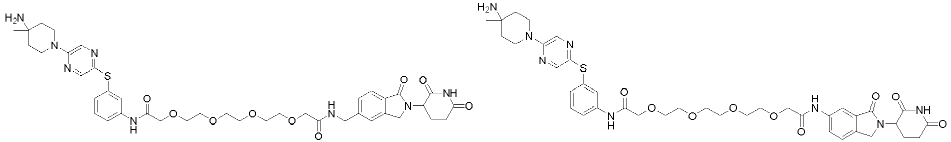

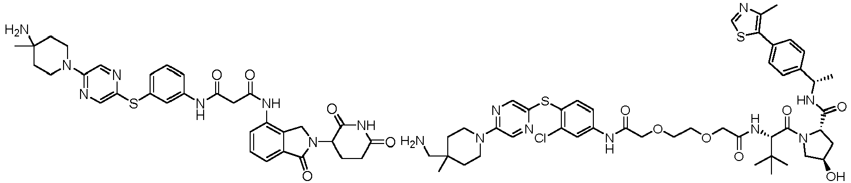

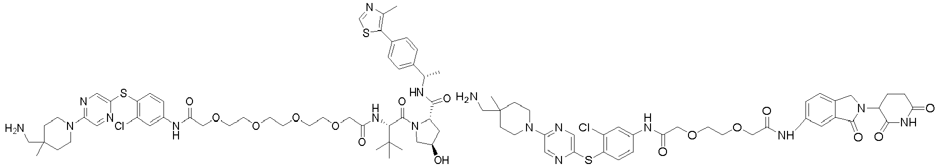

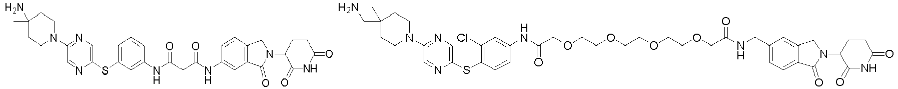

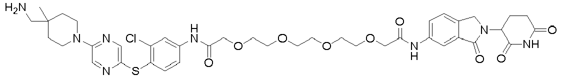

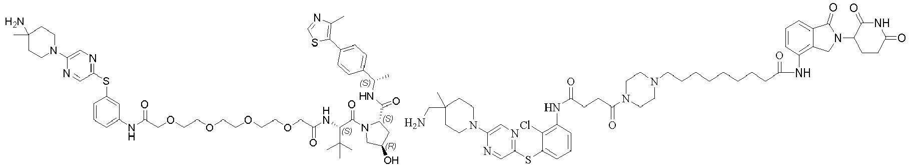

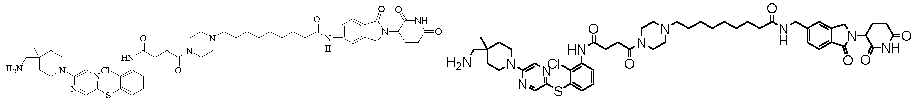

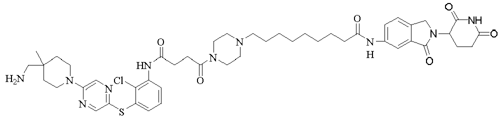

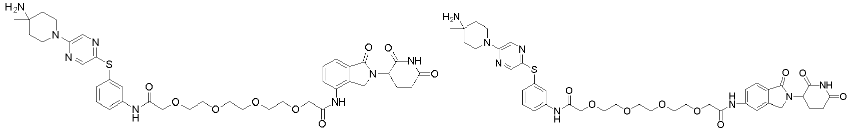

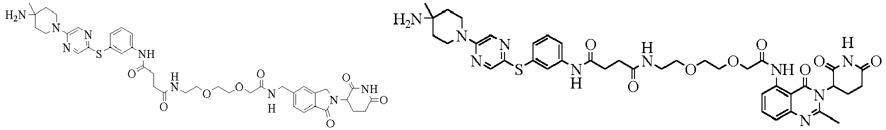

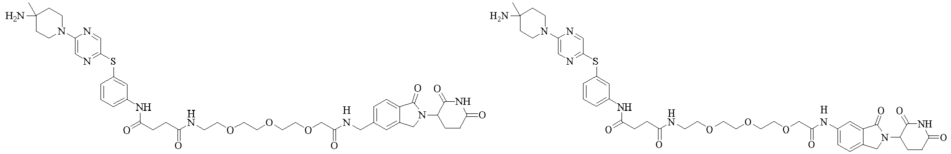

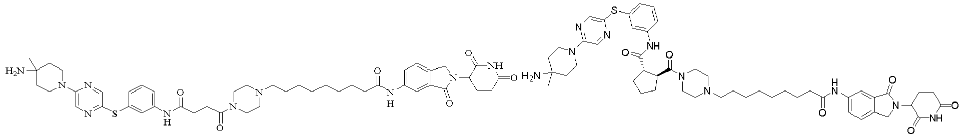

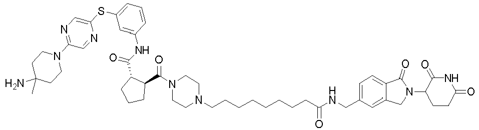

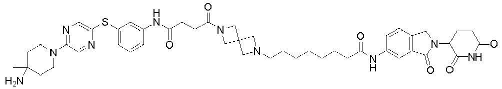

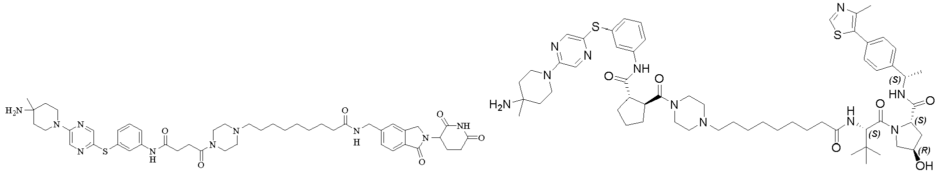

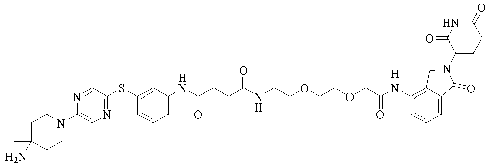

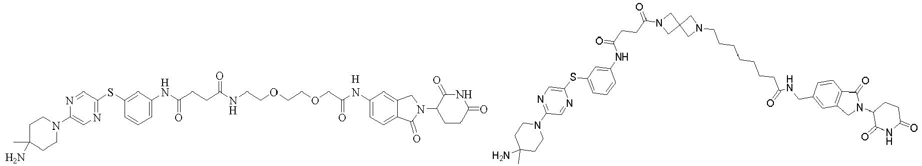

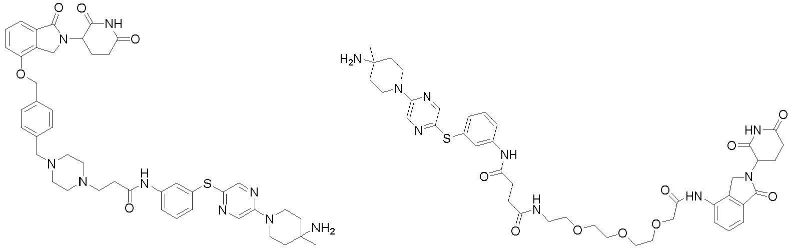

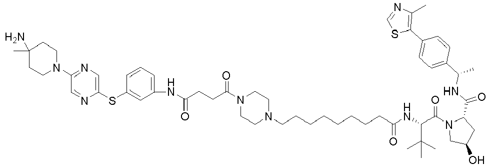

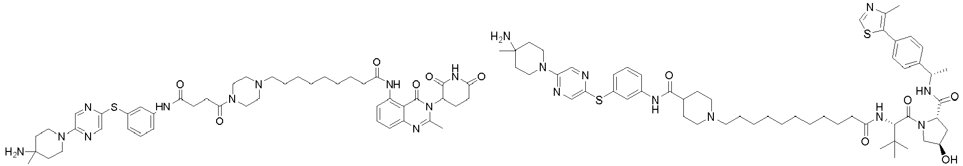

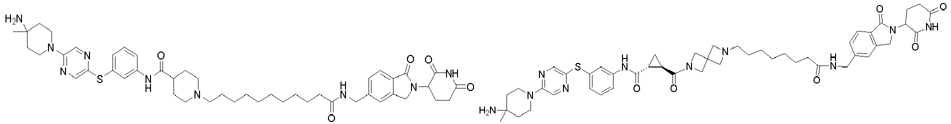

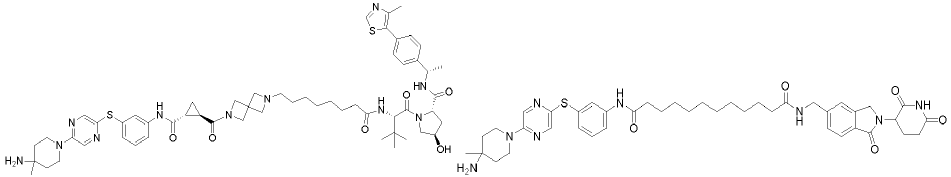

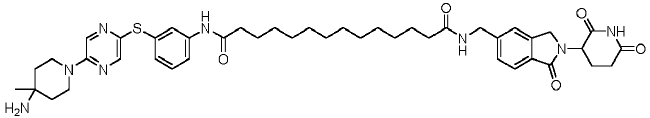

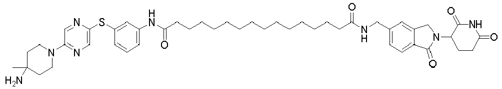

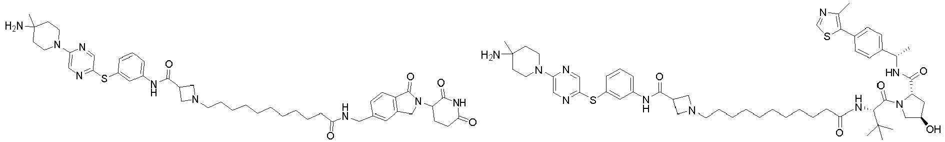

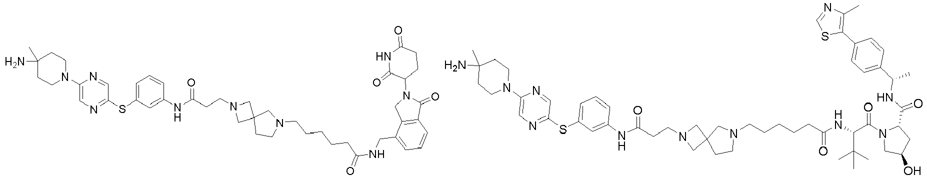

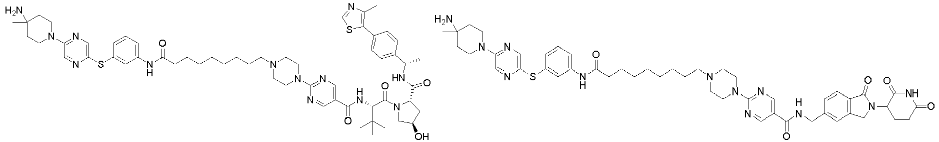

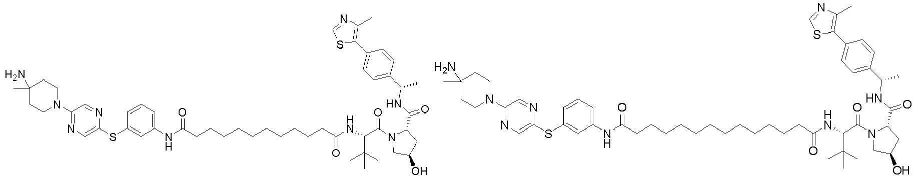

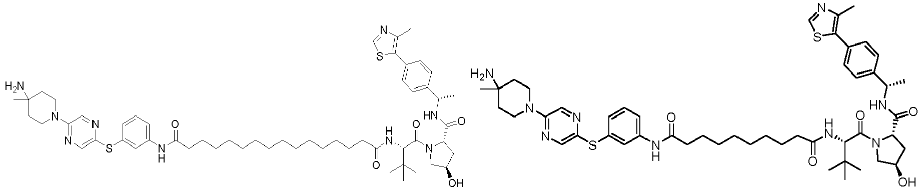

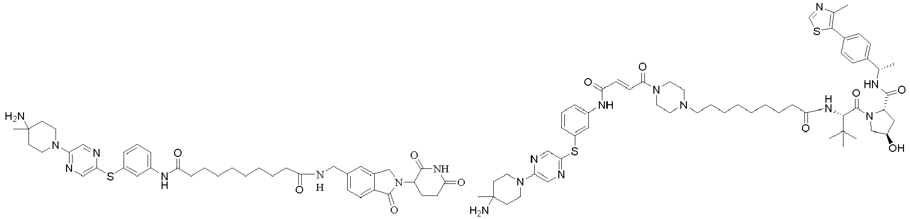

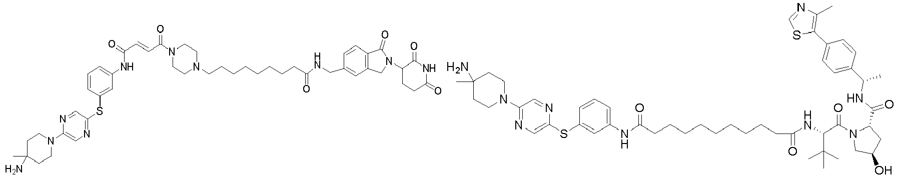

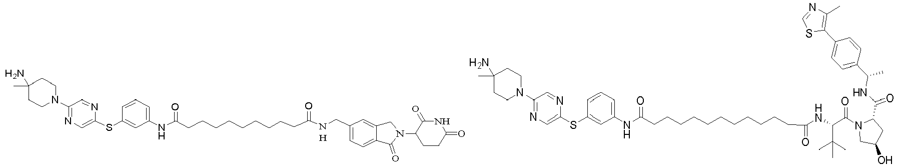

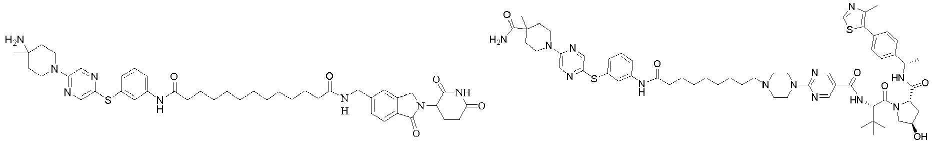

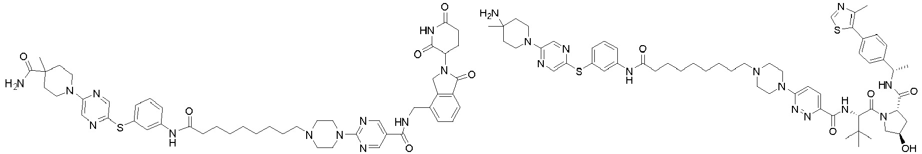

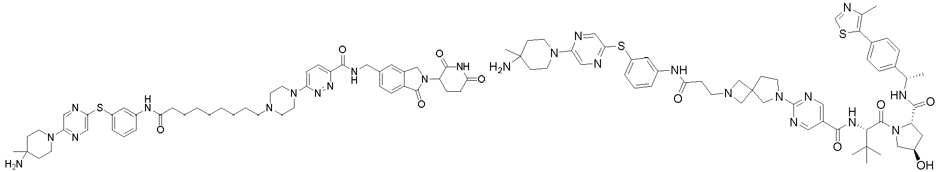

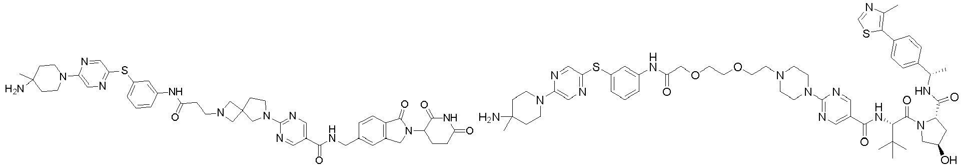

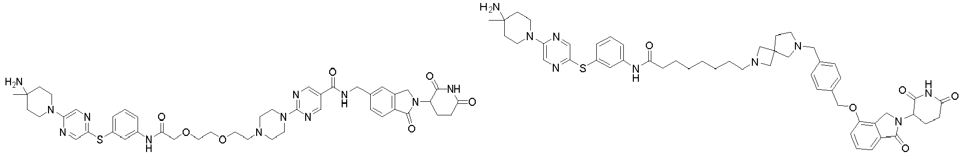

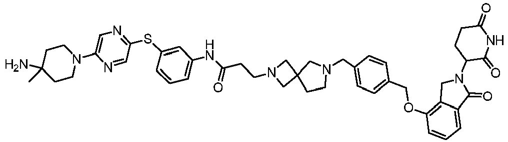

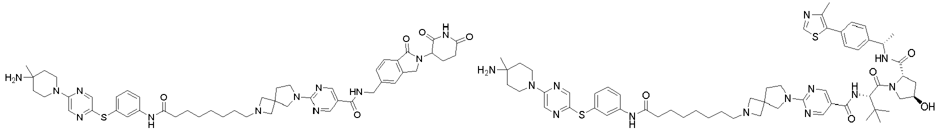

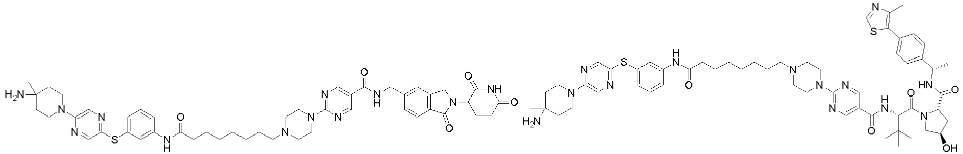

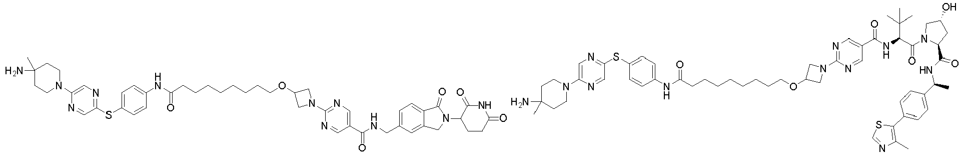

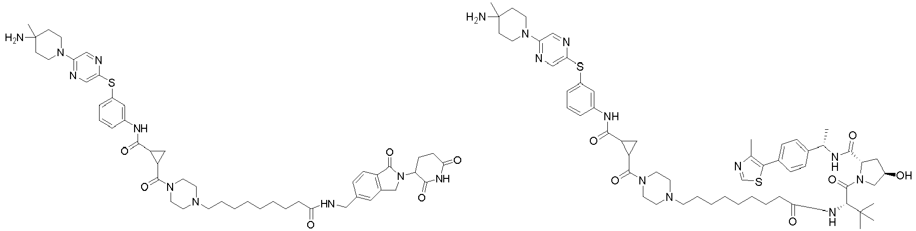

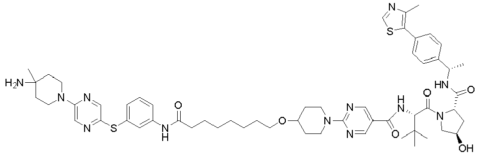

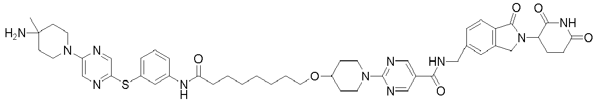

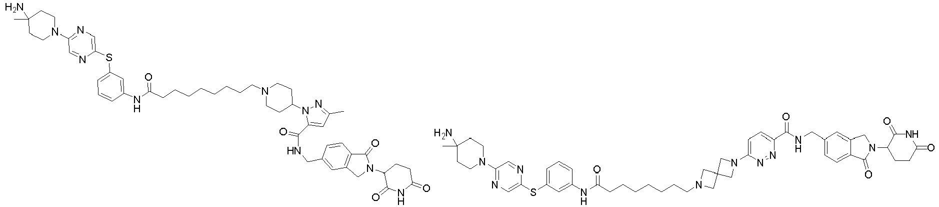

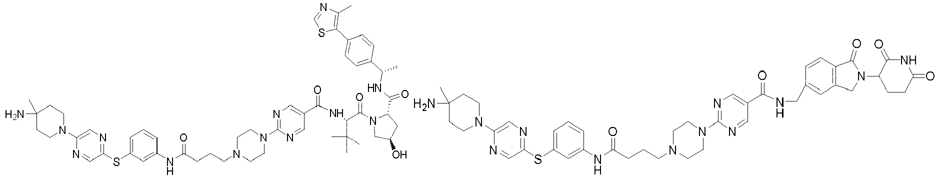

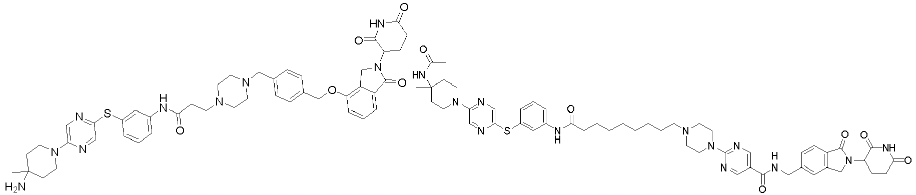

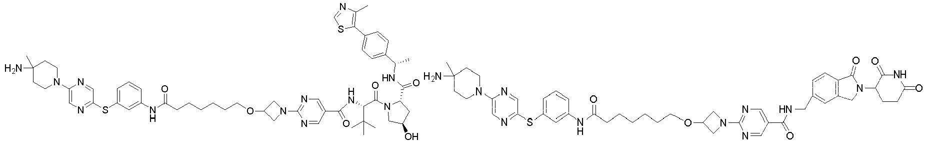

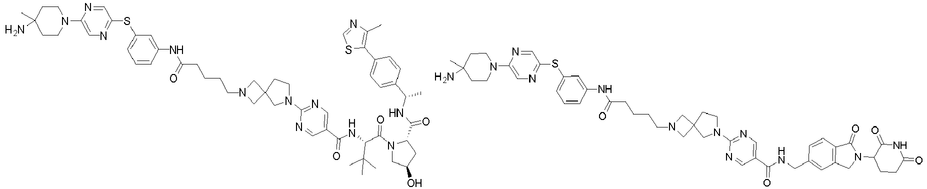

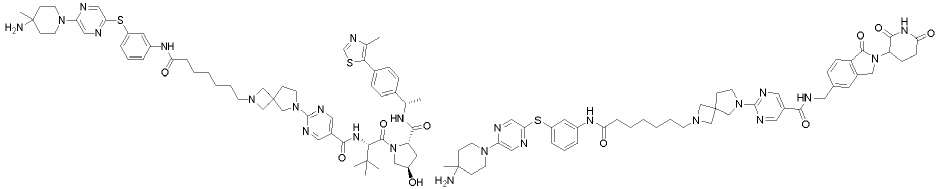

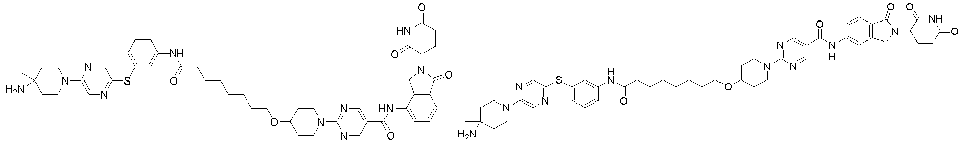

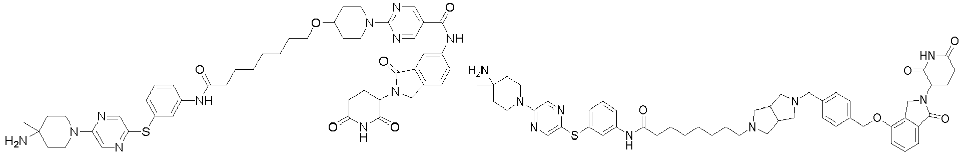

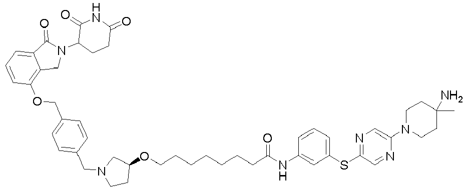

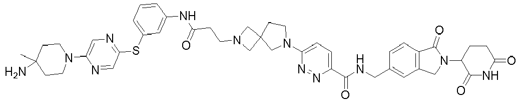

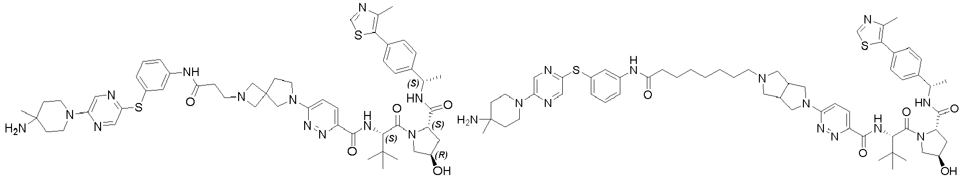

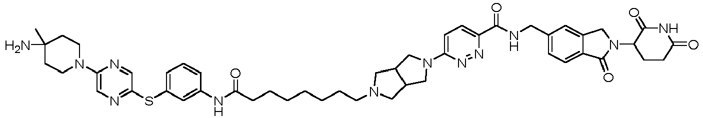

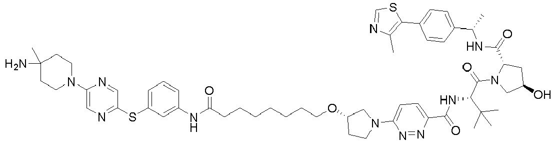

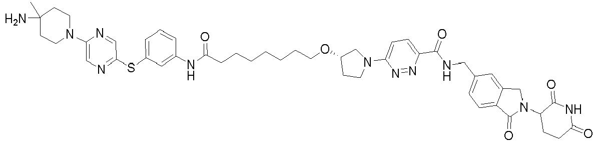

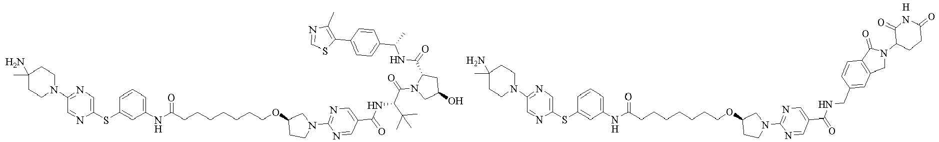

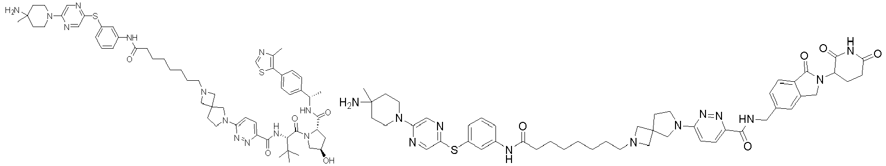

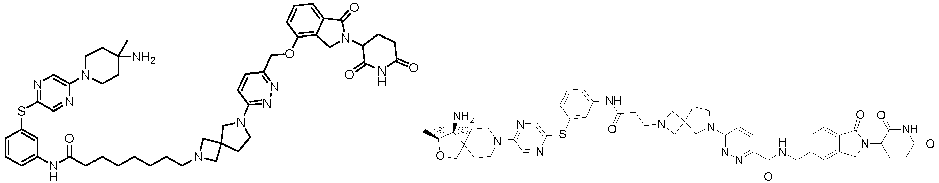

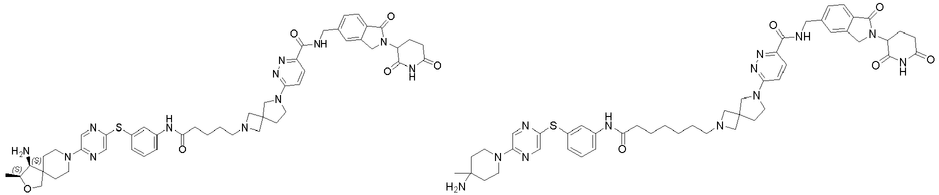

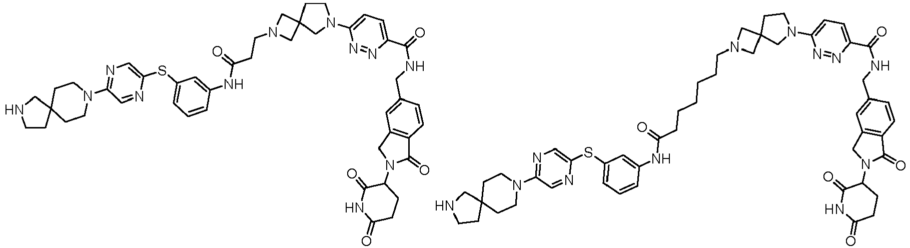

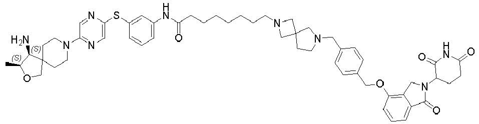

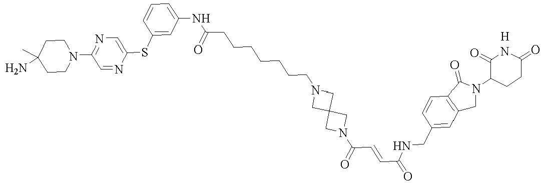

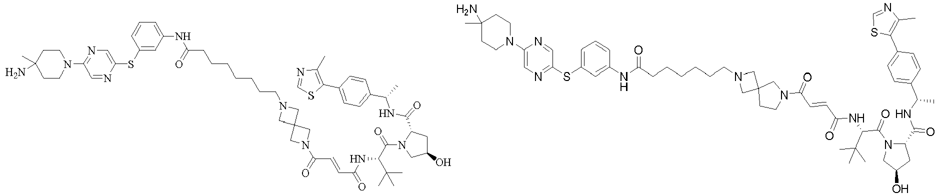

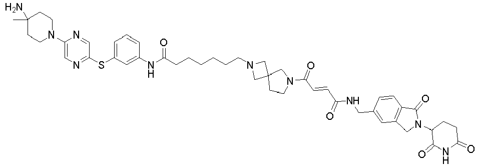

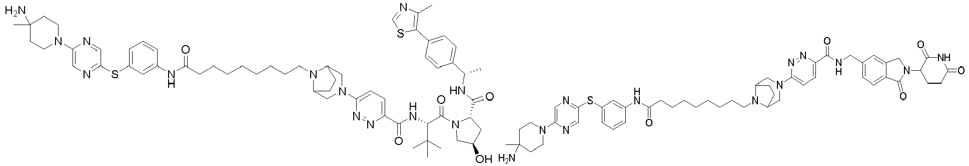

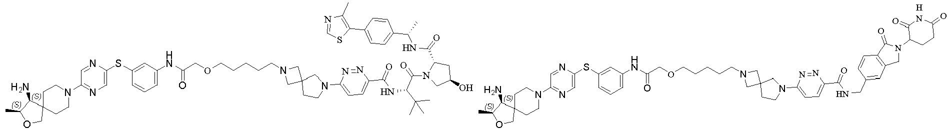

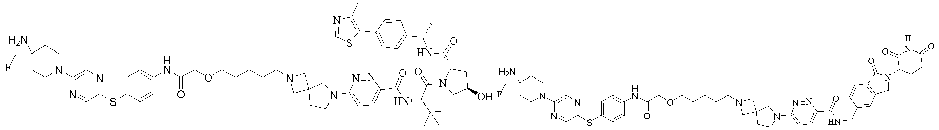

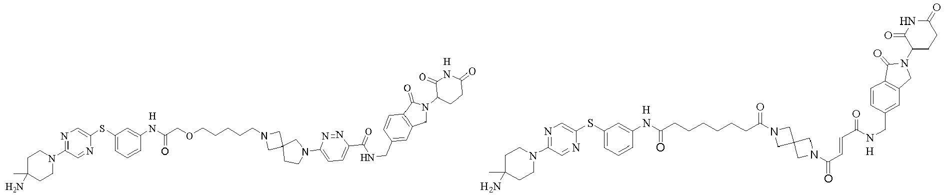

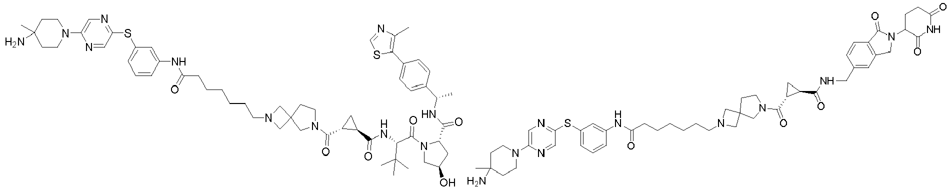

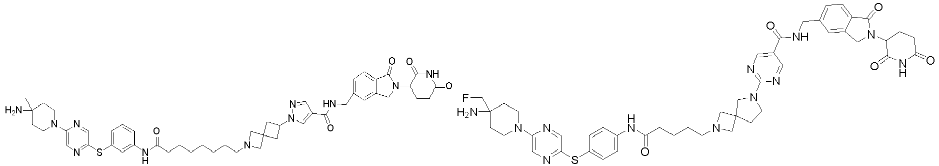

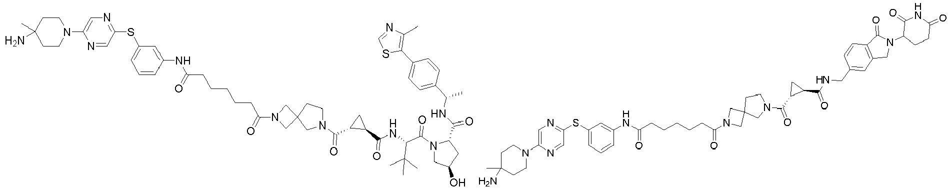

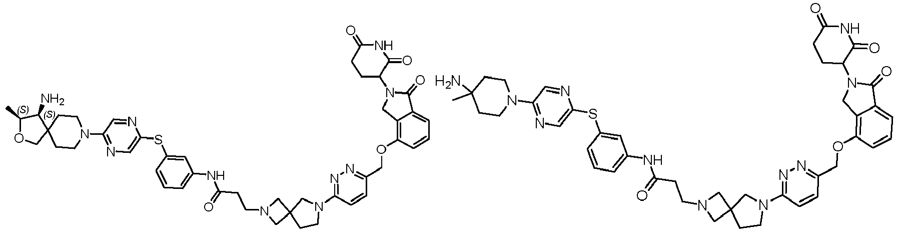

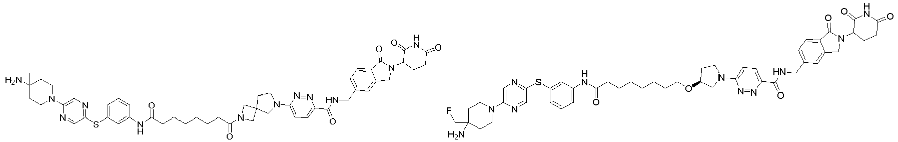

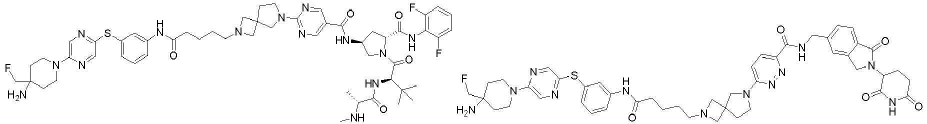

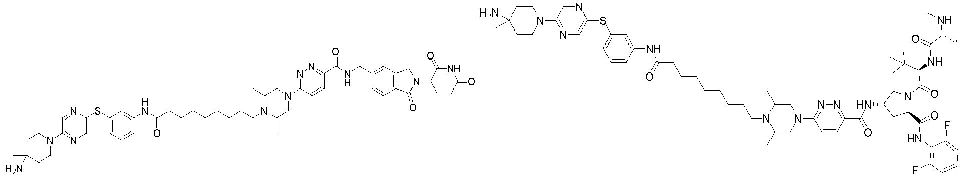

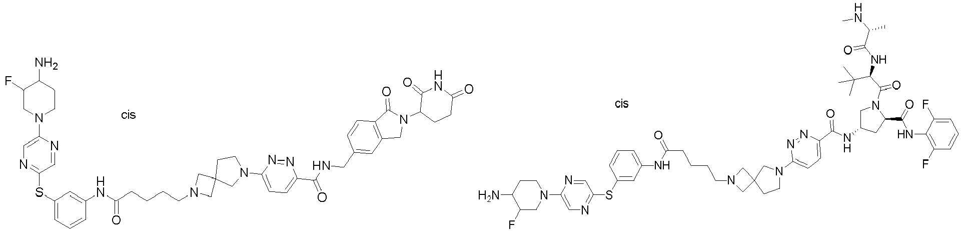

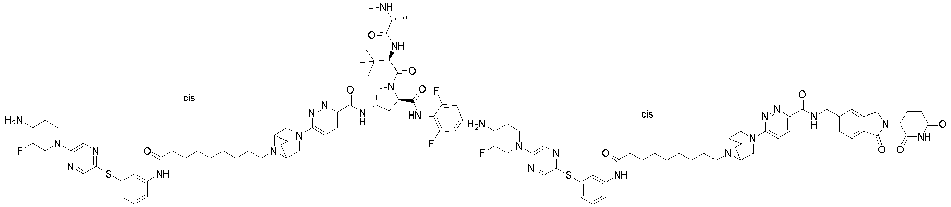

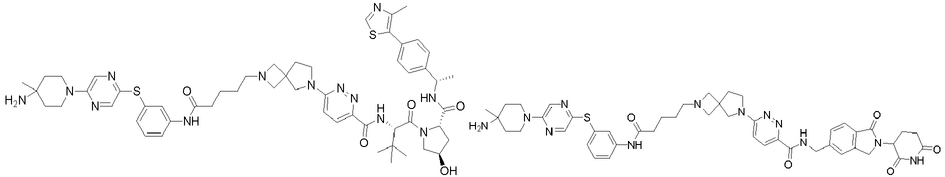

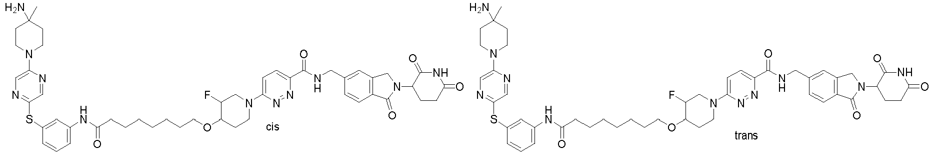

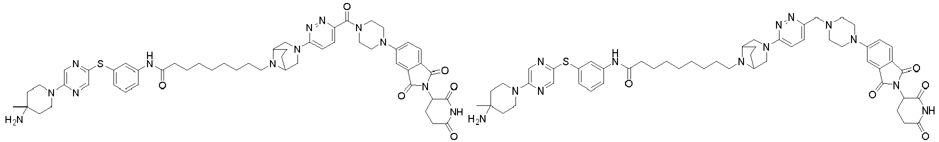

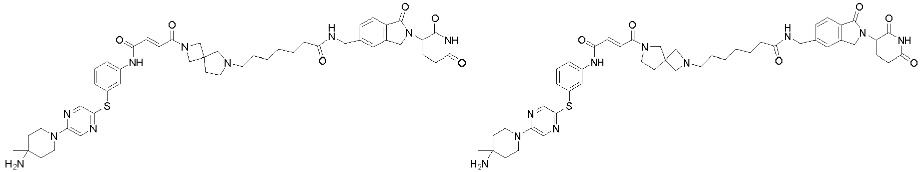

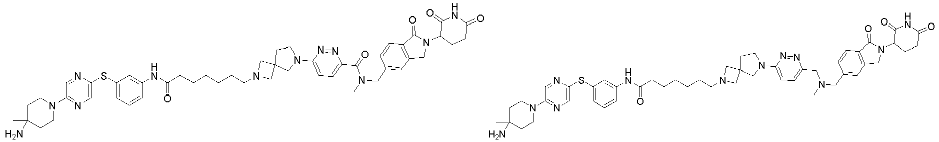

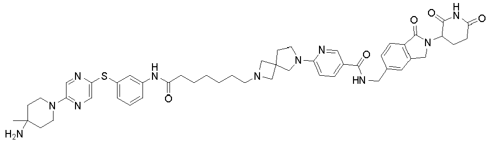

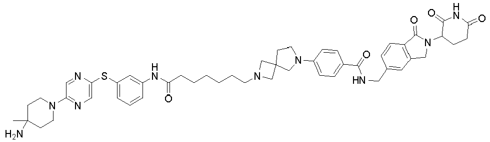

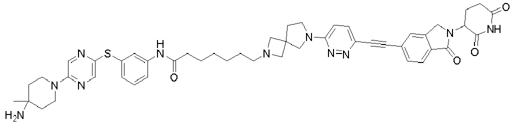

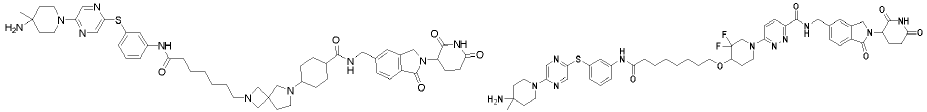

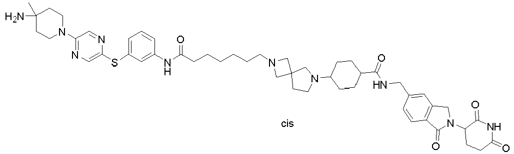

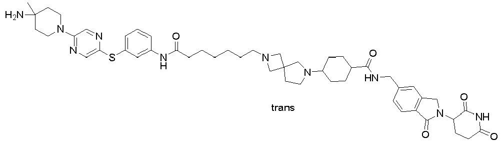

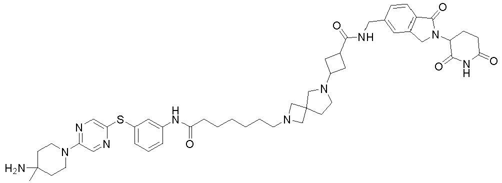

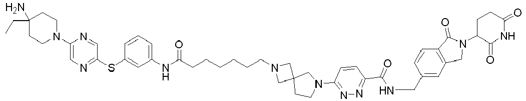

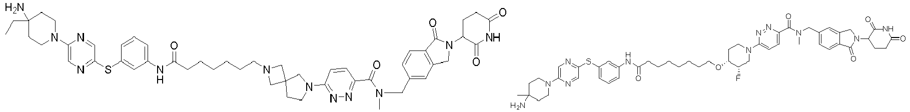

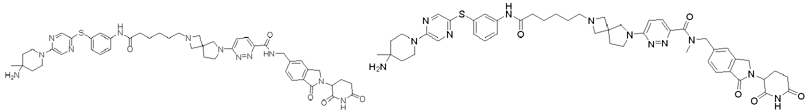

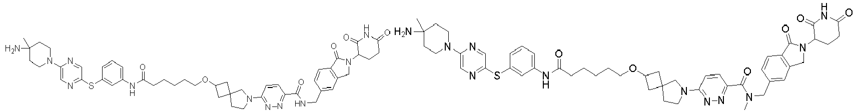

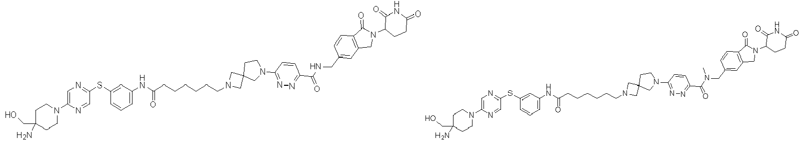

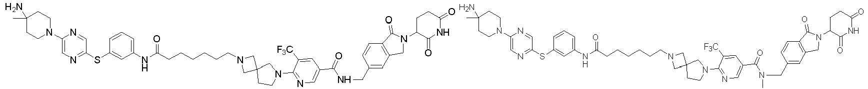

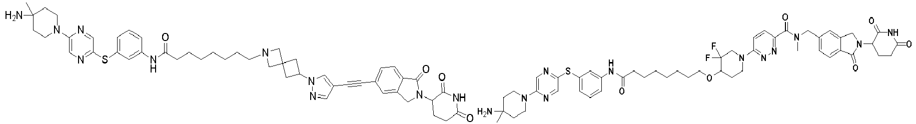

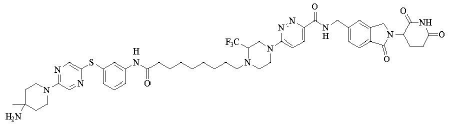

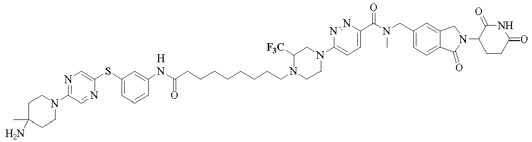

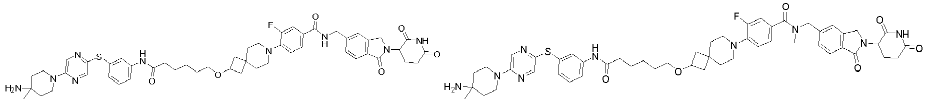

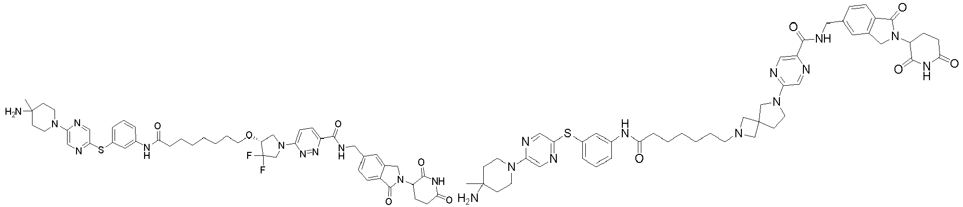

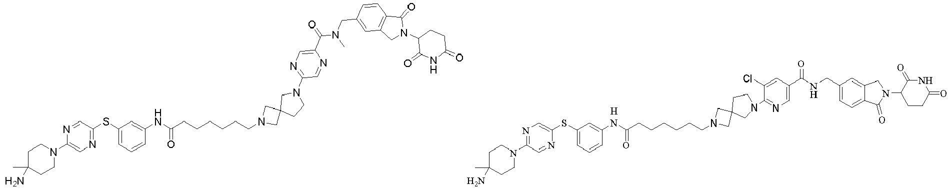

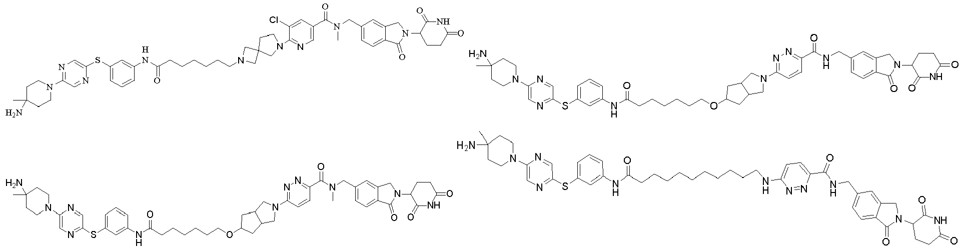

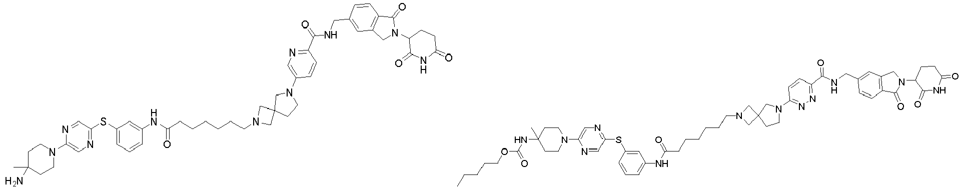

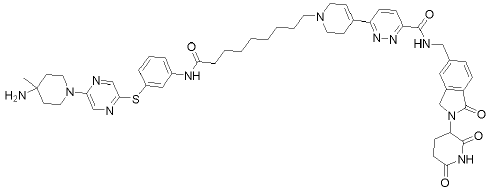

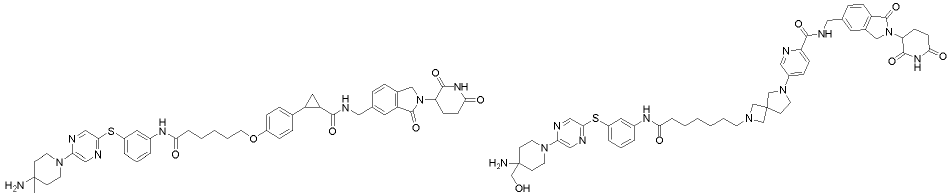

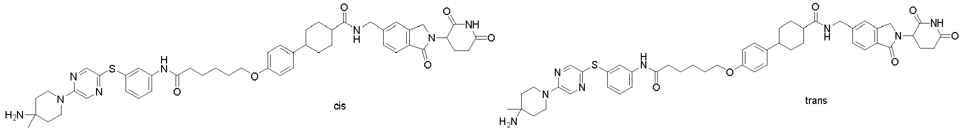

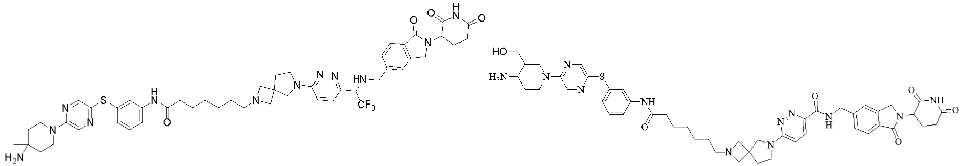

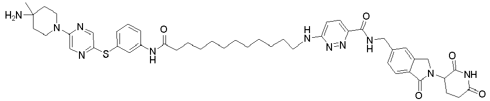

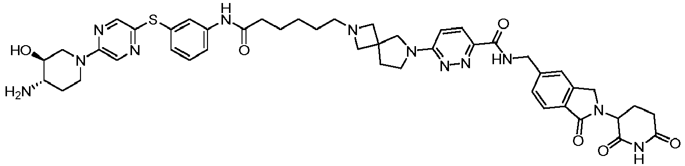

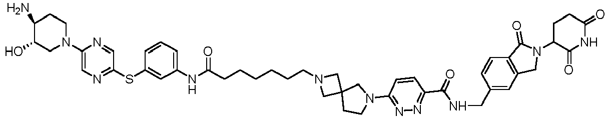

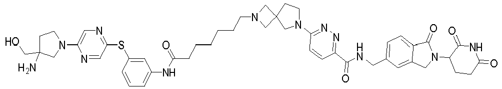

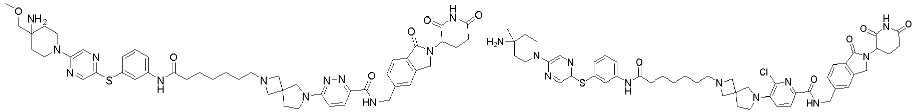

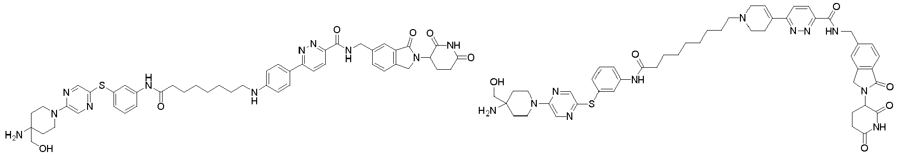

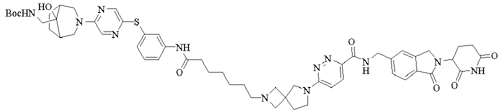

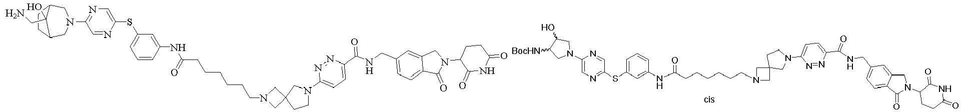

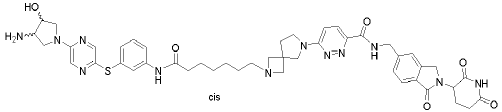

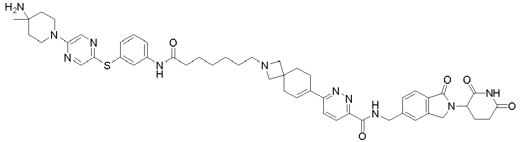

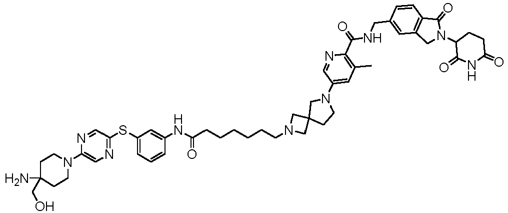

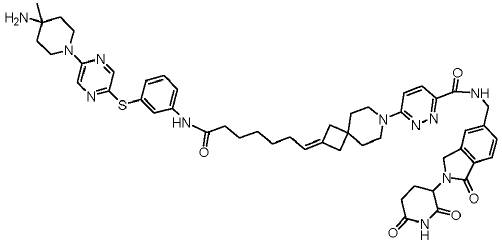

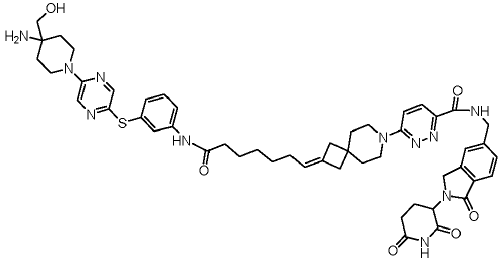

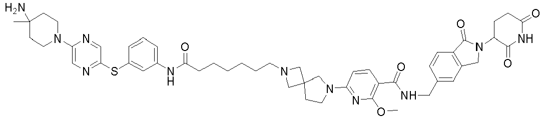

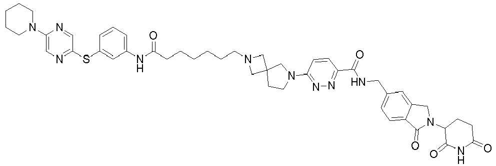

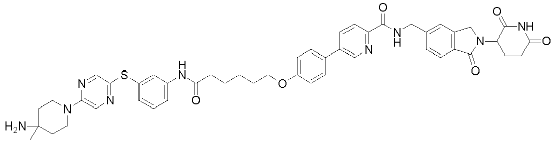

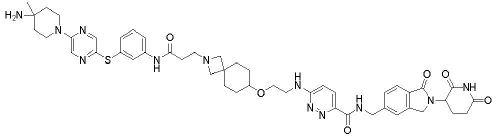

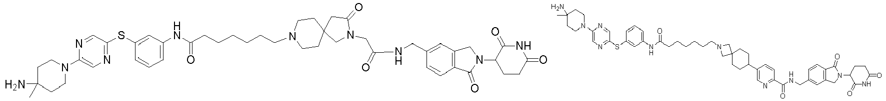

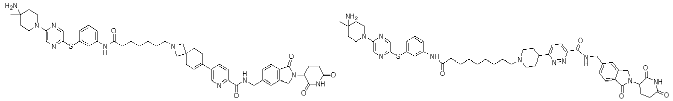

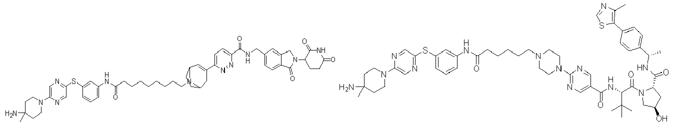

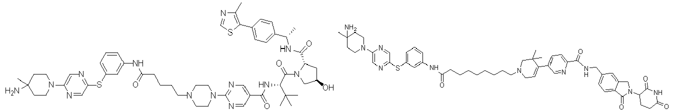

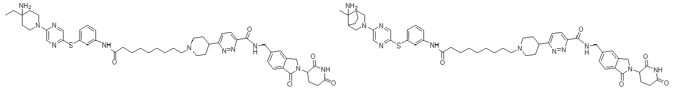

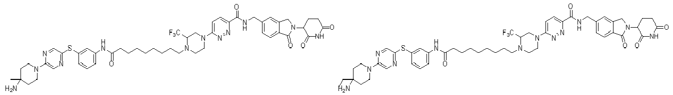

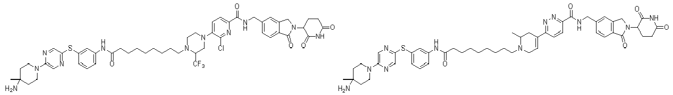

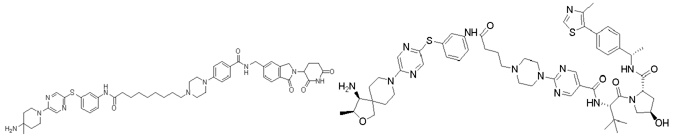

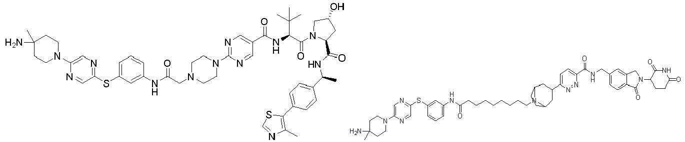

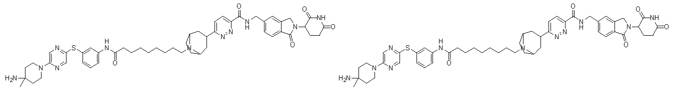

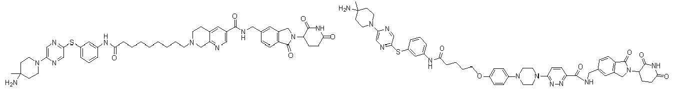

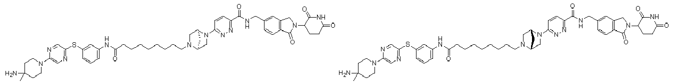

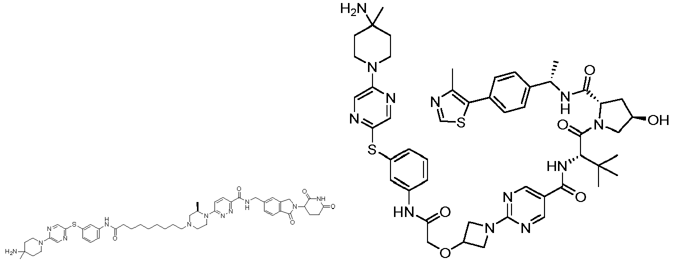

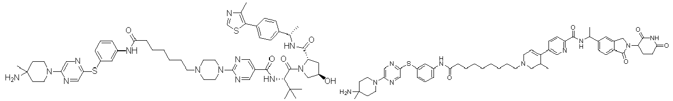

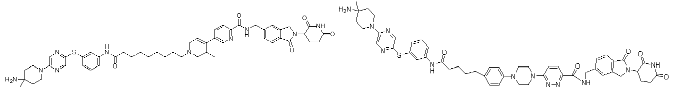

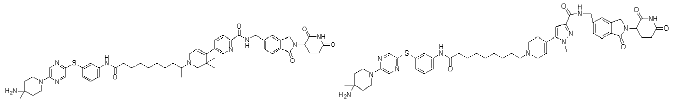

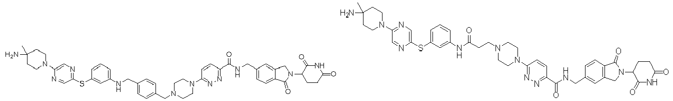

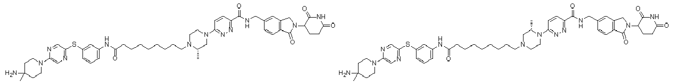

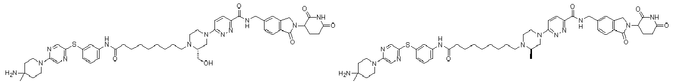

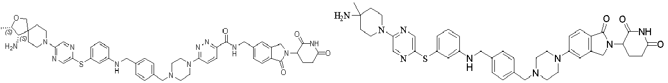

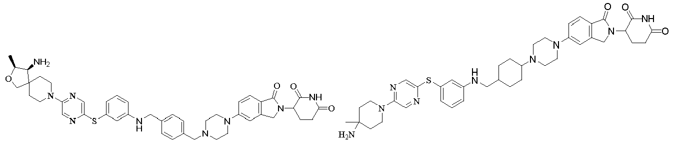

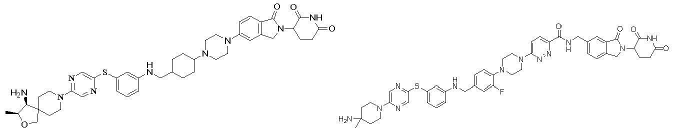

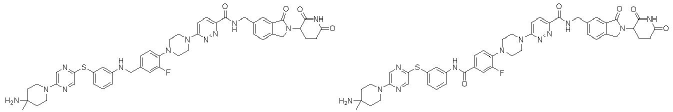

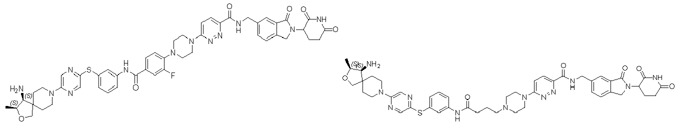

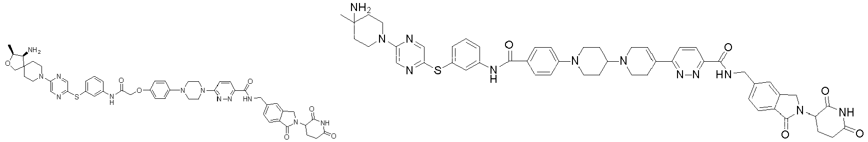

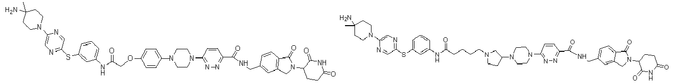

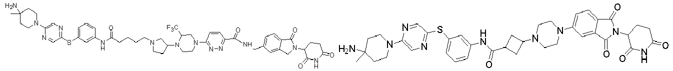

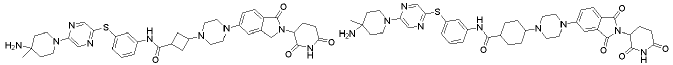

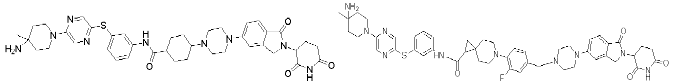

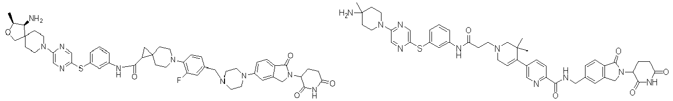

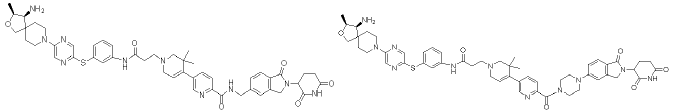

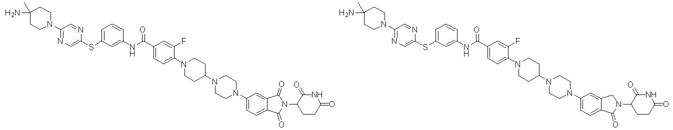

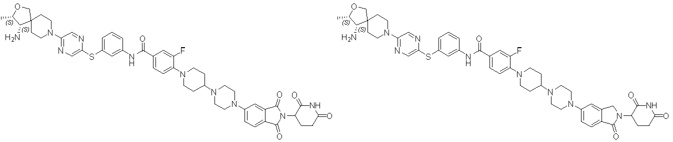

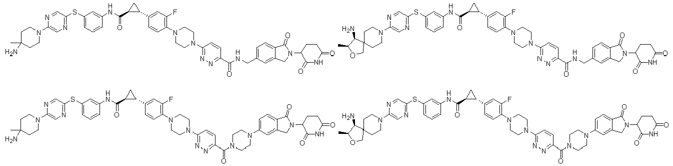

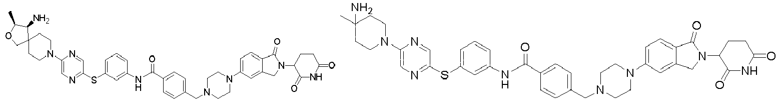

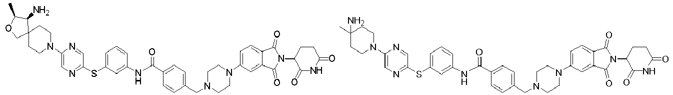

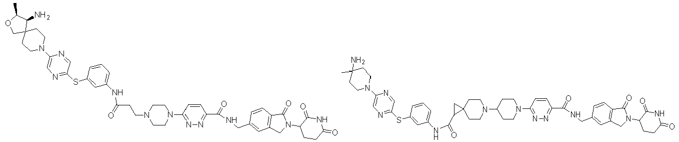

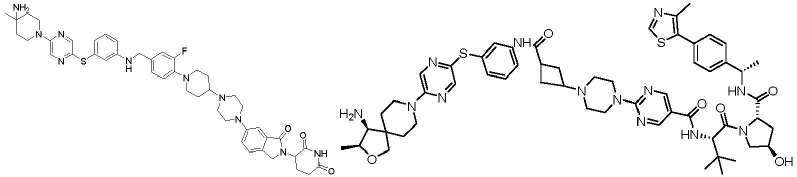

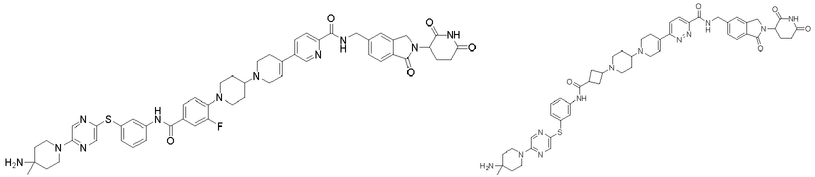

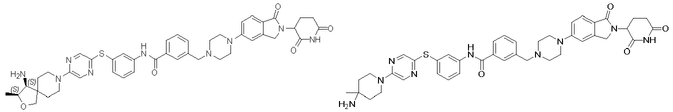

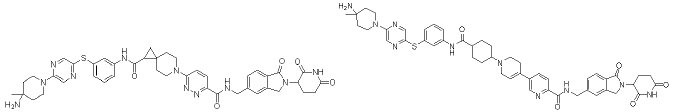

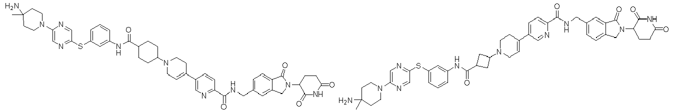

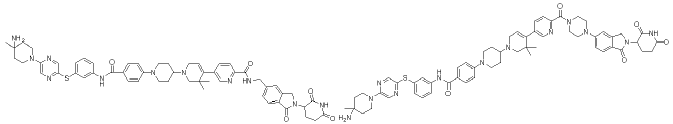

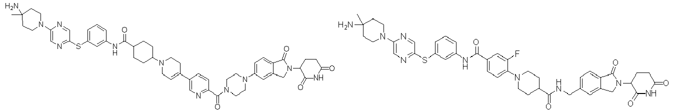

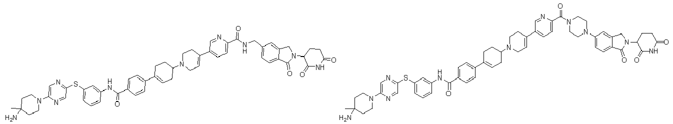

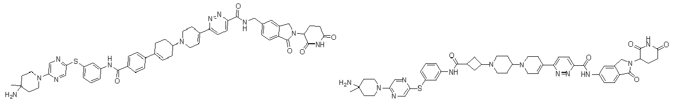

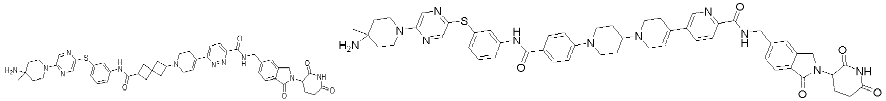

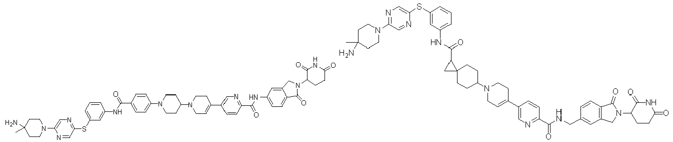

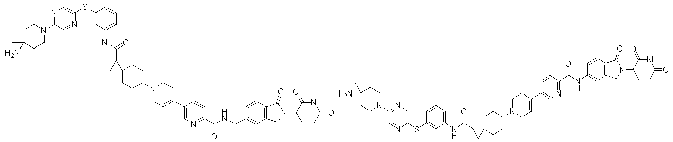

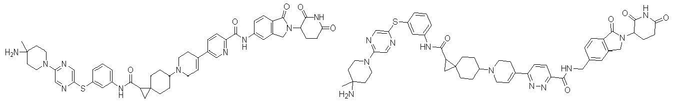

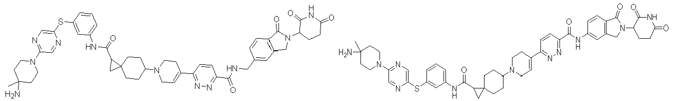

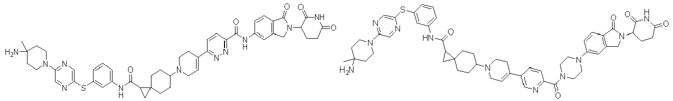

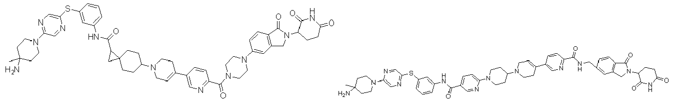

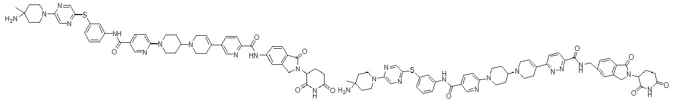

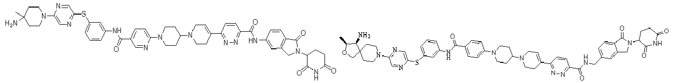

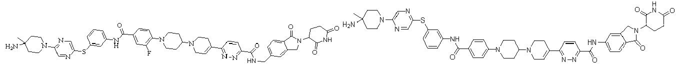

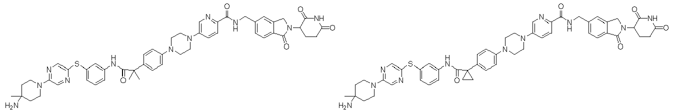

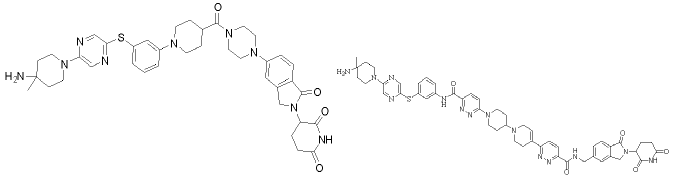

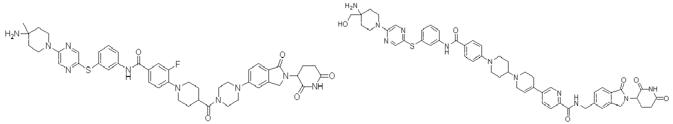

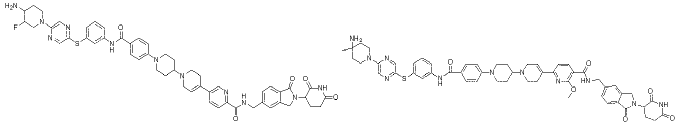

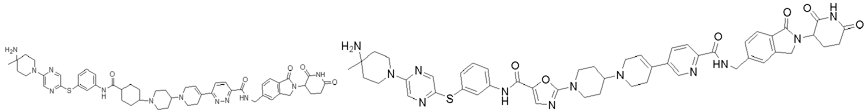

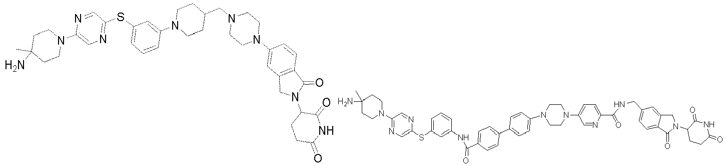

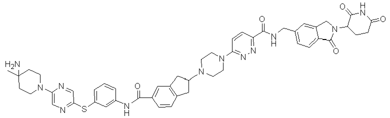

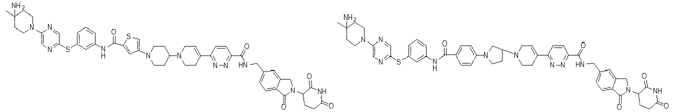

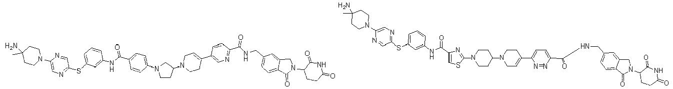

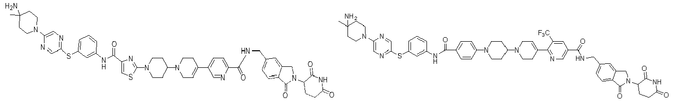

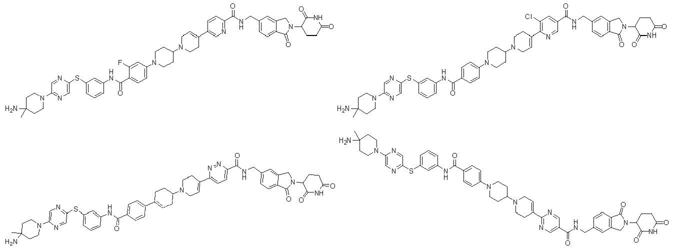

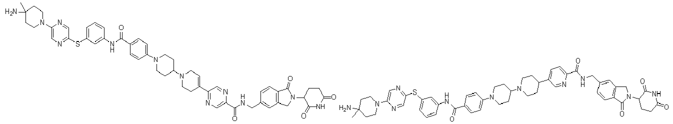

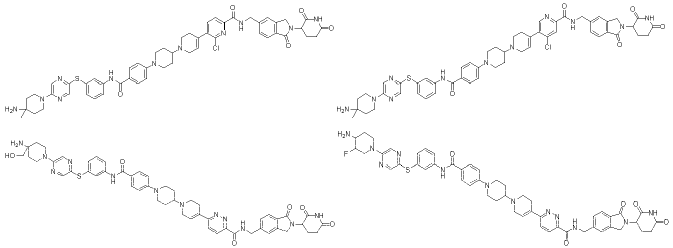

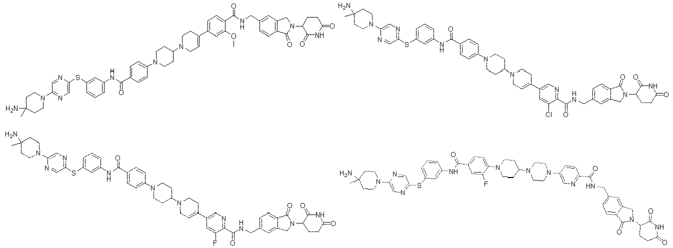

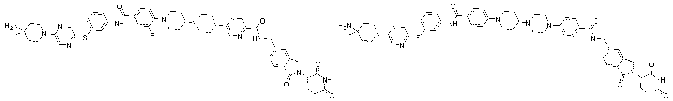

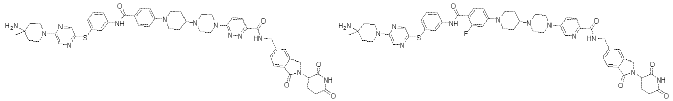

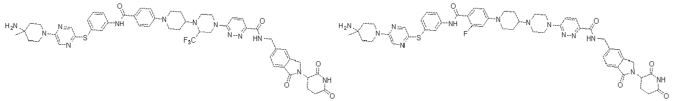

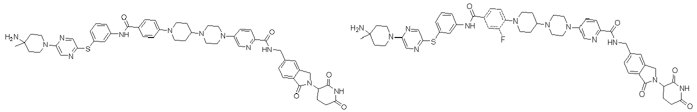

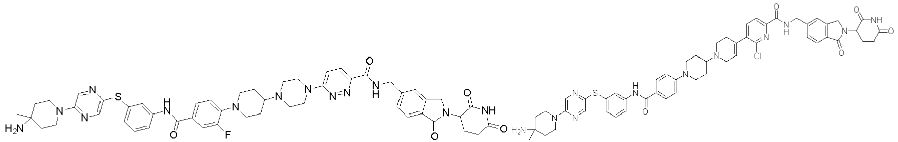

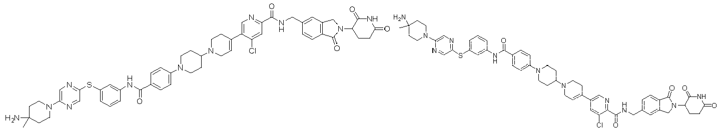

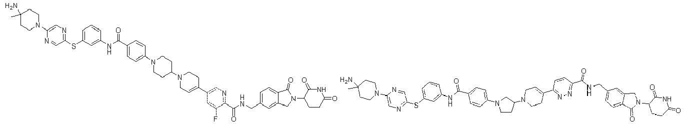

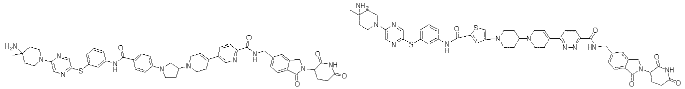

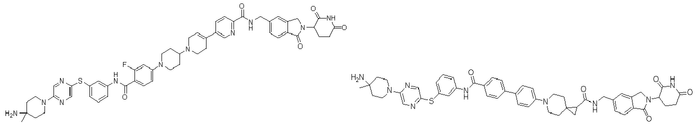

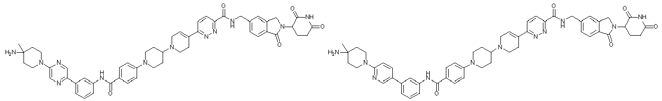

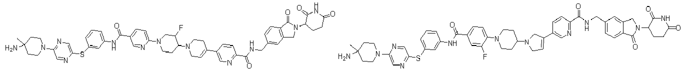

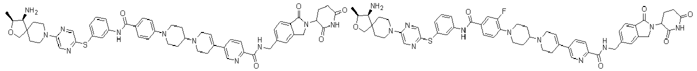

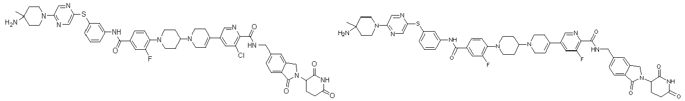

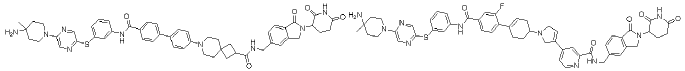

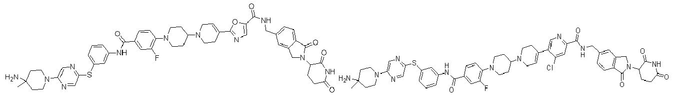

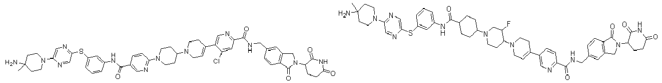

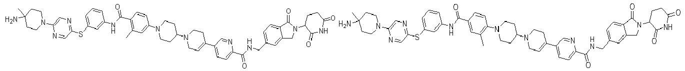

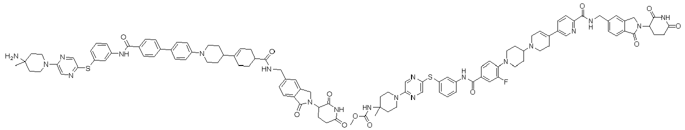

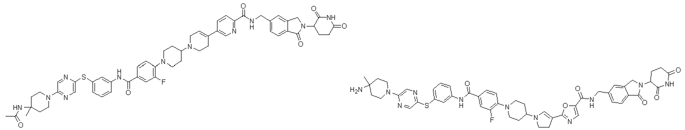

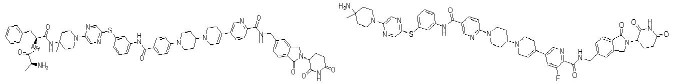

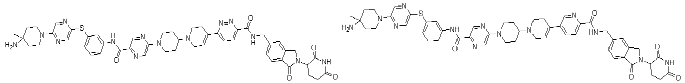

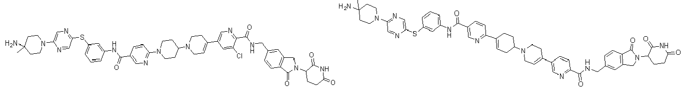

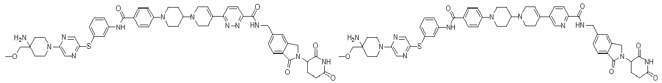

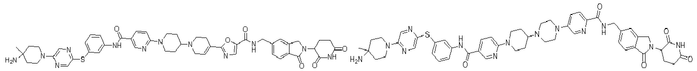

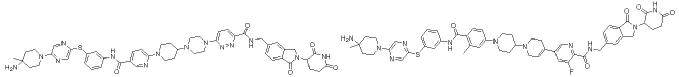

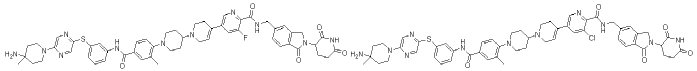

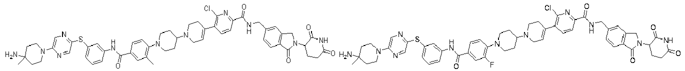

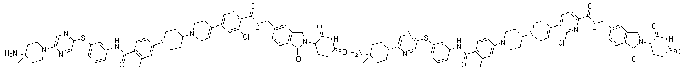

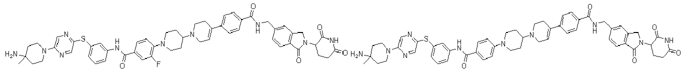

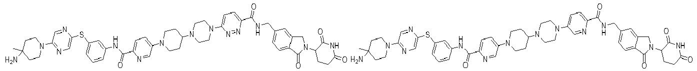

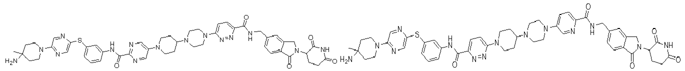

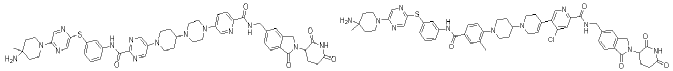

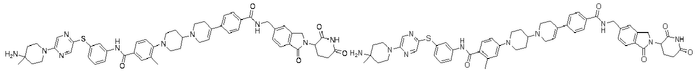

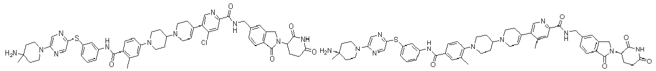

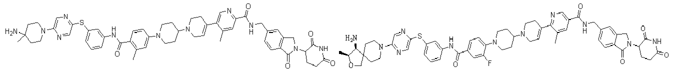

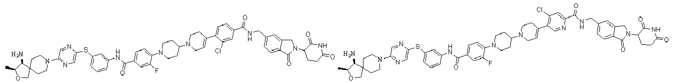

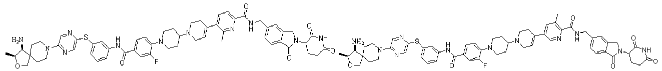

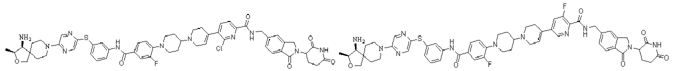

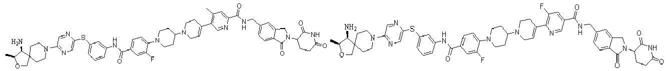

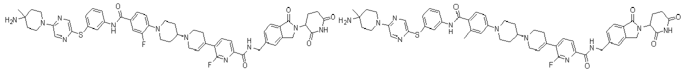

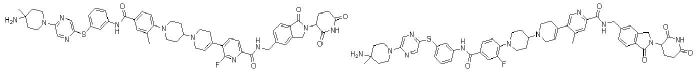

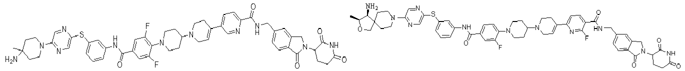

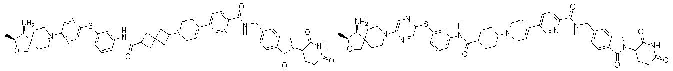

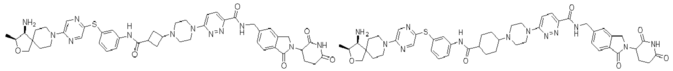

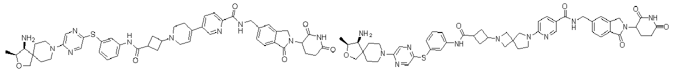

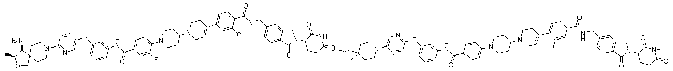

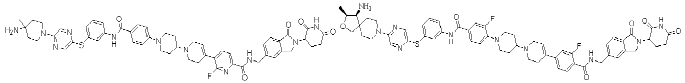

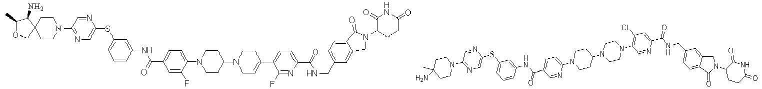

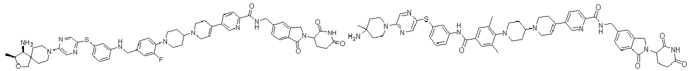

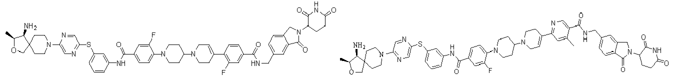

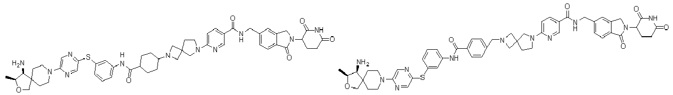

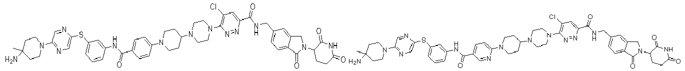

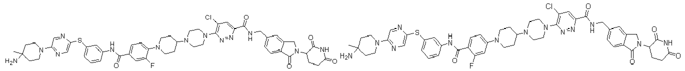

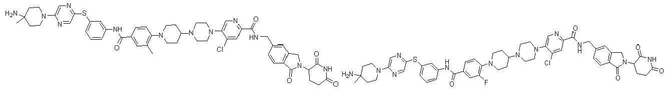

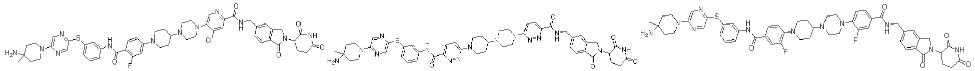

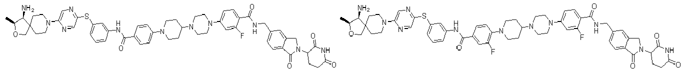

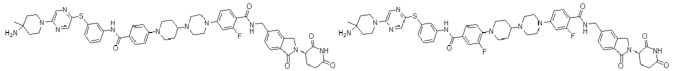

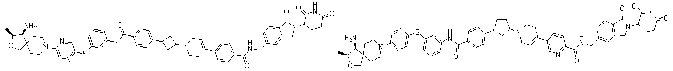

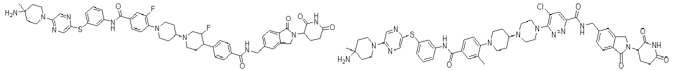

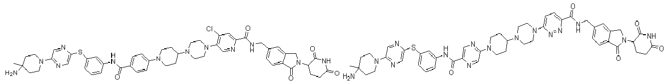

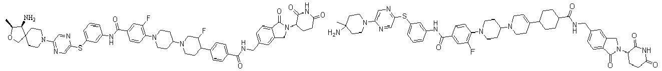

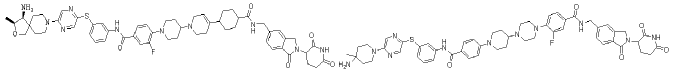

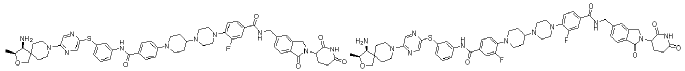

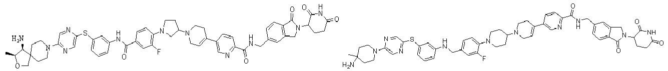

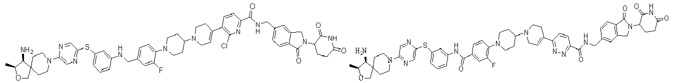

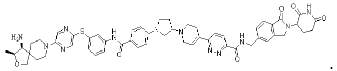

13. The compound according to any one of claims 1 to 12, or a salt thereof, or a deuterated compound thereof, or a stereoisomer thereof, or a solvate thereof, or a hydrate thereof, or a prodrug thereof for use in the manufacturer of phosphatase degraders.

14. The compound according to any one of claims 1 to 12, or a salt thereof, or a deuterated compound thereof, or a stereoisomer thereof, or a solvate thereof, or a hydrate thereof, or a prodrug thereof for use in the manufacturer of SHP2 protein degraders.

15. The compound according to any one of claims 1 to 12, or a salt thereof, or a deuterated compound thereof, or a stereoisomer thereof, or a solvate thereof, or a hydrate thereof, or a prodrug thereof for use in the manufacturer of medicaments for treatment of cancer, Noonan syndrome, Leopard syndrome, juvenile myelomonocytic leukemia, and myelodysplastic syndrome.

16. The use according to claim 15, **characterized in that** the medicament is used to treat lung cancer, colon cancer, rectal cancer, melanoma, neuroblastoma, pancreatic cancer, liver cancer, esophageal cancer, prostate cancer, breast cancer, bile duct cancer, hematoma, and acute leukemia.

17. A medicament, **characterized in that** it is a preparation formed by the compound according to any one of claims 1 to 12, or a salt thereof, or a deuterated compound thereof, or a stereoisomer thereof, or a solvate thereof, or a hydrate thereof, or a prodrug thereof as active ingredient, in combination with pharmaceutically acceptable excipients or adjuvant ingredients.

18. A drug combination, **characterized in that** it comprises the compound according to any one of claims 1 to 12, or a salt thereof, or a deuterated compound thereof, or a stereoisomer thereof, or a solvate thereof, or a hydrate thereof, or a prodrug thereof and other anti-tumor drugs at the same or different specifications, which are administered simultaneously or separately, in combination with pharmaceutically acceptable carriers.
